# EUROPEAN PATENT SPECIFICATION

(11) **EP 1 836 163 B1**
(45) Date of publication and mention of the grant of the patent: **22.09.2010**
(21) Application number: 05825434.3
(22) Date of filing: 21.12.2005
(51) Int. Cl.: C07D 207/09, C07D 207/14, C07D 401/12, C07D 405/12, C07D 403/12, C07D 409/12, C07D 207/08, C07D 413/12, C07D 417/12, C07D 491/10, A61K 31/40, A61K 31/41, A61K 31/435, A61K 31/54, A61P 9/00

(54) **PYRROLIDINE DERIVATIVES FOR THE TREATMENT OF A DISEASE DEPENDING ON THE ACTIVITY OF RENIN**
PYRROLIDINDERIVATE ZUR BEHANDLUNG EINER VON DER RENINAKTIVITÄT ABHÄNGIGEN KRANKHEIT
DERIVES DE PYRROLIDINE POUR LE TRAITEMENT DES MALADIES QUI DEPENDENT DE L'ACTIVITE DE RENINE

(30) Priority: 23.12.2004 GB 0428250
(43) Date of publication of application: 26.09.2007
(73) Proprietor: Novartis AG, 4056 Basel (CH)
(72) Inventor: BREITENSTEIN, Werner, CH-4054 Basel (CH); COTTENS, Sylvain, CH-4108 Witterswil (CH); EHRHARDT, Claus, 79541 Lörrach (DE); JACOBY, Edgar, CH-4059 Basel (CH); LORTHIOIS, Edwige, Liliane, Jeanne, F-68680 Niffer (FR); MAIBAUM, Juergen, Klaus, 79576 Weil-Haltingen (DE); OSTERMANN, Nils, 79589 Binzen (DE); SELLNER, Holger, CH-4106 Therwil (CH); SIMIC, Oliver, CH-4053 Basel (CH)
(74) Representative: Dietel, Anja
(86) International application number: PCT/EP2005/013786
(87) International publication number: WO 2006/066896

(56) References cited:
- EP-A- 0 900 793
- EP-A- 1 341 533
- EP-A- 1 486 500
- WO-A-96/17842
- WO-A-03/099767
- WO-A-2004/113335
- WO-A-2005/066139
- WO-A-2005/118531
- US-A- 4 971 966
- SCHROEDER ET AL.: "Synthesis of the Four Stereoisomers of Several 3-(1-Aminoethyl)pyrrolidines. important Intermediates in the Preparation of quinolone Antibacterials" J. HETEROCYCLIC CHEM., vol. 29, no. 6, October 1992 (1992-10), - November 1992 (1992-11) pages 1481-1498, XP002376378
- KIELY ET AL.: "New "Ofloxacin" Type Antibacterial Agents. Incorporation of the Spiro Cyclopropyl Group at N-1" J. MED. CHEM., vol. 31, 1988, pages 2004-2008, XP002376379

## Description

The invention relates to (3-mono-, 3,4-di or 3,4,4-tri-)substituted pyrrolidine compounds, these compounds for use in the diagnostic and therapeutic treatment of a warm-blooded animal, especially for the treatment of a disease (= disorder) that depends on activity of renin; the use of a compound of that class for the preparation of a pharmaceutical formulation for the treatment of a disease that depends on activity of renin; pharmaceutical formulations comprising said substituted pyrrolidine compound, and a method for the manufacture of said substituted pyrrolidine compound.

EP 1 341 533 discloses pharmaceutical combinations comprising a renin inhibitor for cardiovascular diseases. WO 03/099 767 discloses amide derivatives as renin inhibitors.

The present invention provides especially compounds of the formula I 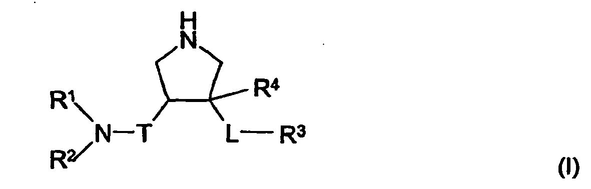 wherein
R¹ is unsubstituted or substituted aryl, unsubstituted or substituted mono- or bicyclic heterocyclyl, unsubstituted or substituted cycloalkyl, unsubstituted or substituted aryl-alkyl, unsubstituted or substituted mono- or bicyclic heterocyclyl-alkyl, unsubstituted or substituted cycloalkyl-alkyl, or acyl;
R² is unsubstituted or substituted alkyl, unsubstituted or substituted aryl, unsubstituted or substituted mono- or bicyclic heterocyclyl, unsubstituted or substituted cycloalkyl, unsubstituted or substituted aryl-alkyl, unsubstituted or substituted mono- or bicyclic heterocyclyl-alkyl or unsubstituted or substituted cycloalkyl-alkyl, with the proviso that if L is methylene (-CH₂-), oxy (-O-), thio (-S-) or unsubstituted (-NH-)or substituted imino, R² is selected from one of these mentioned groups and (as additional alternative) from hydrogen; R³ is unsubstituted or substituted alkyl, substituted or unsubstituted aryl, unsubstituted or substituted heterocyclyl, unsubstituted or substituted cycloalkyl, unsubstituted or substituted aryl-alkyl, unsubstituted or substituted heterocyclyl-alkyl, unsubstituted or substituted cycloalkyl-alkyl, or, if L is oxy, thio or unsubstituted or substituted imino, has one of the meanings just mentioned or is unsubstituted or substituted alkylcarbonyl, unsubstituted or substituted arylcarbonyl (aroyl), unsubstituted or substituted heterocyclylcarbonyl (heterocycloyl), unsubstituted or substituted cycloalkylcarbonyl, etherified carboxy, carbamoyl, N-mono- or N,N-di-substituted amino-carbonyl, substituted or unsubstituted alkylsulfonyl, substituted or unsubstituted arylsulfonyl, substituted or unsubstituted heterocyclylsulfonyl or substituted or unsubstituted cycloalkylsulfonyl, sulfamoyl or N-mono- or N,N-di-substituted amino-sulfonyl;
R⁴ is hydrogen or hydroxy;
L is a bond, methylene (-CH₂-), oxy (-O-), thio (-S-) or unsubstituted (-NH-)or substituted imino, with the proviso that if L is a bond then R³ is one of the moieties mentioned for R³ other than substituted alkyl;
or R³ and R⁴ which then is -0- together with L which then is methylene and the carbon to which R³-L- and R⁴ are bound form a substituted or unsubstituted ring annealed to an unsubstituted or substituted aryl, unsubstituted or substituted heterocyclyl or unsubstituted or substituted cycloalkyl, thus forming a spiro compound of the formula I, or
R³ and R⁴ together with L form oxo (=O), thioxo (=S) or unsubstituted or substituted imino (=NH);
and
T is methylene or methylene monosubstituted by alkyl, carbonyl (-C(=O)-) or thiocarbonyl (-C(=S)-); wherein the optionally substituted groups are as defined below; or a salt thereof.

The compounds of the present invention exhibit inhibitory activity on the natural enzyme renin. Thus, compounds of formula I may be employed for the treatment (this term also including prophylaxis) of one or more disorders or diseases selected from, *inter alia,* hypertension, atherosclerosis, unstable coronary syndrome, congestive heart failure, cardiac hypertrophy, cardiac fibrosis, cardiomyopathy postinfarction, unstable coronary syndrome, diastolic dysfunction, chronic kidney disease, hepatic fibrosis, complications resuming from diabetes, such as nephropathy, vasculopathy and neuropathy, diseases of the coronary vessels, restenosis following angioplasty, raised intra-ocular pressure, glaucoma, abnormal vascular growth, hyperaldosteronism, cognitive impairment, alzheimers, dementia, anxiety states and cognitive disorders.

Listed below are definitions of various terms used to describe the compounds of the present invention as well as their use and synthesis, starting materials and intermediates and the like. These definitions, either by replacing one, more than one or all general expressions or symbols used in the present disclosure and thus yielding preferred embodiments of the invention, preferably apply to the terms as they are used throughout the specification unless they are otherwise limited in specific instances either individually or as part of a larger group.

The term "lower" or "C₁-C₇-" defines a moiety with up to and including maximally 7, especially up to and including maximally 4, carbon atoms, said moiety being branched (one or more times) or straight-chained and bound via a terminal or a non-terminal carbon. Lower or C₁-C-₇alkyl, for example, is n-pentyl, n-hexyl or n-heptyl or preferably C₁-C₄-alkyl, especially as methyl, ethyl, n-propyl, sec-propyl, n-butyl, isobutyl, sec-butyl, tert-butyl.

Halo or halogen is preferably fluoro, chloro, bromo or iodo, most preferably fluoro, chloro or bromo. If not explicitely or implicitely stated otherwise, halo can also stand for more than one halogen substituted in moieties such as alkyl, alkanoyl and the like (e.g. in trifluoromethyl, trifiuoroacetyl).

Unsubstituted or substituted aryl is a is mono- or polycyclic aryl with 6 to 22 carbon atoms, and is unsubstituted or substituted by one or more, especially one to three, moieties, independently selected from the group consisting of
a substitutent of the formula -(C₀-C₇-alkylene)-(x)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, especially selected from C₁-C₇-alkyl, phenyl, naphthyl, phenyl or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₁-alkyl, C₁-C₇-alkoxy-C-₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇ alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl;
from C₂-C₇-alkenyl, C₂-C₇-alkinyl, phenyl, naphtyl, heterocyclyl, especially as defined below for heterocyclyl, preferably selected from pyrrolyl, furanyl, thienyl, pyrimidine-2,4-dione-1-, -3- or -5-yl and benzo[1,3]-dioxolyl, phenyl- or naphthyl- or heterocyclyl-C₁-C₇-alkyl wherein heterocyclyl is as defined below, preferably selected from pyrrolyl, furanyl, thienyl and benzo[1,3]-dioxolyl; such as benzyl or naphthylmethyl, halo-C₁-C₇-alkyl, such as trifluoromethyl, phenyloxy- or naphthyloxy-C₁-C₇-alkyl, phenyl-C₁-C₇-alkoxy- or naphthyl-C₁-C₇-alkoxy-C₁-C₇-alkyl, di-(naphthyl- or phenyl)-amino-C₁-C₇-alkyl, di-(naphthyl- or phenyl-C₁-C₇-alkyl)-amino-C₁-C₇-alkyl, benzoyl- or naphthoylamino-C₁-C₇-alkyl, phenyl- or naphthylsuffonylamino-C₁-C₇-alkyl wherein phenyl or naphthyl is unsubstituted or substituted by one or more, especially one to three, C₁-C₇-alkyl moieties, phenyl- or naphthyl-C₁-C₇-alkylsulfonylamino-C₁-C₇-alkyl, carboxy-C₁-C₇-alkyl, halo, hydroxy, phenyl-C₁-C₇-alkoxy wherein phenyl is unsubstituted or substituted by C₁-C₇-alkoxy and/or halo, halo-C₁-C₇-alkoxy, such as trifluoromethoxy, phenyl- or naphthyloxy, phenyl- or naphthyl-C₁-C₇-alkyloxy, benzoyl- or naphthoyloxy, halo-C₁-C₇-alkylthio, such as trifluoromethylthio, phenyl- or naphthylthio, phenyl- or naphthyl-C₁-C₇-alkylthio, benzoyl- or naphthoylthio, nitro, amino, di-(naphthyl- or phenyl-C₁-C₇-alkyl)-amino, benzoyl- or naphthoylamino, phenyl- or naphthylsulfonylamino wherein phenyl or naphthyl is unsubstituted or substituted by one or more, especially one to three, C₁-C₇-alkyl moieties, phenyl- or naphthyl-C₁-C₇-alkylsulfonylamino, carboxyl, C₁-C₇-alkyl-carbonyl, halo-C₁-C₇-alkylcarbonyl, hydroxy-C₁-C₇-alkylcarbonyl, C₁-C₇-alkoxy-C₁-C₇-alkylcarbonyl, amino-C₁-C₇-alkylcarbonyl, (N-) mono- or (N,N-) di-(C₁-C₇-alkyl)-amino-C₁-C₇-alkylcarbonyl, C₁-C₇-alkanoylamino-C₁-C₇-alkylcarbonyl, halo-C₁-C₇-alkoxycarbonyl, phenyl- or naphthyloxycarbonyl, phenyl- or naphthyl-C₁-C₇-alkoxycarbonyl, (N,N-) di-(C₁-C7-alkyl)-amino-C₁-C₇-alkoxycarbonyl, carbamoyl, , N-mono or N,N-di-(naphthyl- or phenyl-)-aminocarbonyl, N-mono- or N,N-di-(naphthyl- or phenyl-C₁-C₇-alkyl)-aminocarbonyl, cyano, C₁-C₇-alkylene which is unsubstituted or substituted by up to four C₁-C₇-alkyl substituents and bound to two adjacent ring atoms of the aryl moiety, C₂-C₇-alkenylene or -alkinylene which are bound to two adjacent ring atoms of the aryl moiety, sulfenyl, sulfinyl, C₁-C₇-alkylsulfinyl, phenyl- or naphthylsulfinyl wherein phenyl or naphthyl is unsubstituted or substituted by one or more, especially one to three, C₁-C₇-alkyl moieties, phenyl- or naphthyl-C₁-C₇-alkylsulfinyl, sulfonyl, C₁-C₇-alkylsulfonyl, halo-C₁-C₇-alkylsulfonyl, hydroxy-C₁-C₇-alkylsulfonyl, C₁-C₇-alkoxy-C₁-C₇-alkylsulfonyl, amino-C₁-C₇-alkysulfonyl, (N,N-) di-(C₁-C₇-alkyl)-amino-C₁-C₇-alkylsulfonyl, C₁-C₇-alkanoylamino-C₁-C₇-alkylsuifonyl, phenyl- or naphthylsulfonyl wherein phenyl or naphthyl is unsubstituted or substituted by one or more, especially one to three, C₁-C₇-alkyl moieties, phenyl- or naphthyl-C₁-C₇-alkylsulfonyl, sulfamoyl and N-mono or N,N-di-(C₁-C₇-alkyl, phenyl-, naphthyl, phenyl-C₁-C₇-alkyl and/or naphthyl-C₁-C₇-alkyl)-aminosulfonyl.

Unsubstituted or substituted heterocyclyl is a mono- or bicyclic or if not part of a substituent R¹ or R² or if not a substituent R¹ and R² further polycyclic heterocyclic moiety (meaning that in cases where unsubstituted or substituted heterocyclyl is part of a substituent R¹ and R² (e.g. in heterocyclylalkyl) or itself is a moiety R¹ or R², it comprises not more than two rings annelated to each other, while in the case of substitutents R³ comprising or consisting of unsubstituted or substituted heterocyclyl it may comprise more than two rings annelated to each other), preferably a mono- or bicyclic or, if not part of a substituent R¹ or R² or if not a substituent R¹ and R², mono-, bi- or further tricyclic-, (in all cases mono-cyclic or annelated systems mentioned so far) unsaturated, partially saturated or saturated ring system with 3 to 22 (preferably 3 to 14) ring atoms and with one or more, preferably one to four, heteroatoms independently selected from nitrogen (=N-, -NH- or substituted -NH-), oxygen, sulfur (-S-, S(=O)- or S-(=O)₂-) which is unsubstituted or substituted by one or more, e.g. up to three, substitutents independently selected from the subsitutents mentioned above for aryl and from oxo. Preferably, unsubstituted or substituted heterocyclyl is selected from the following moieties: 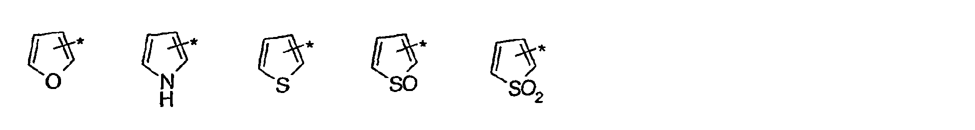 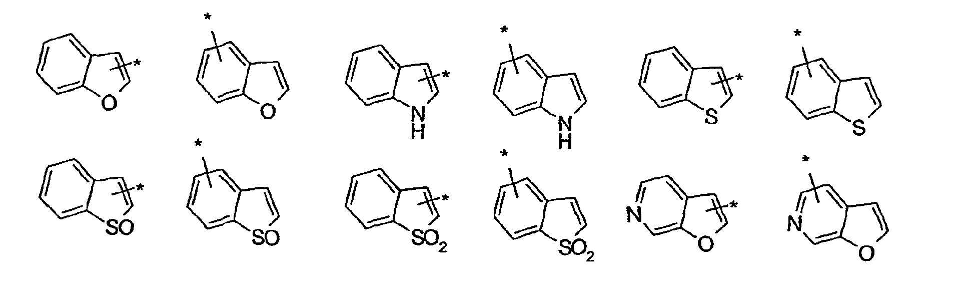 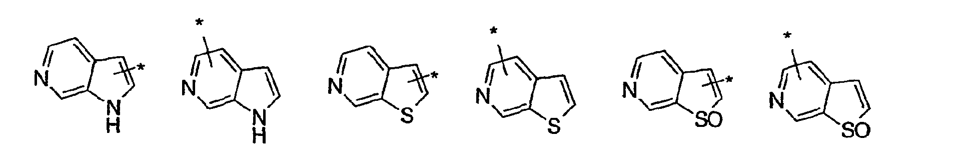 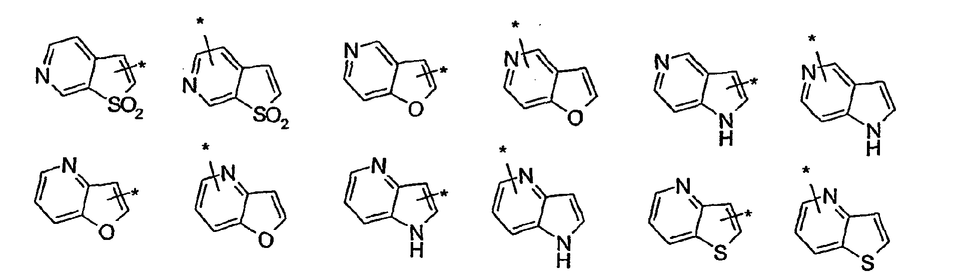 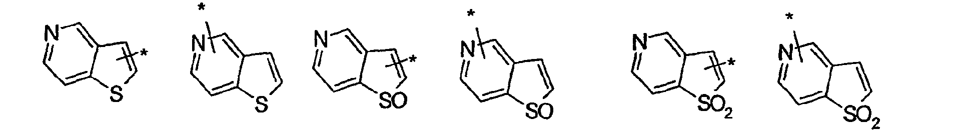 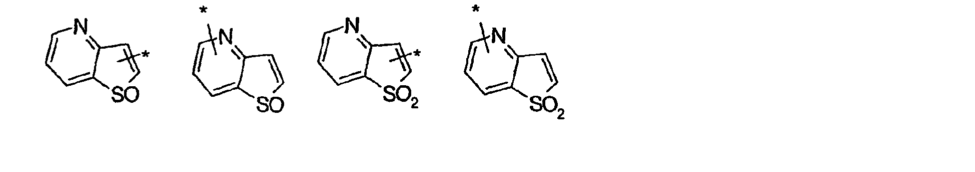 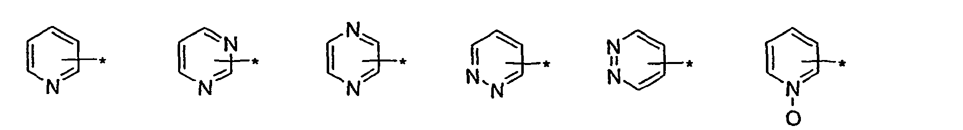 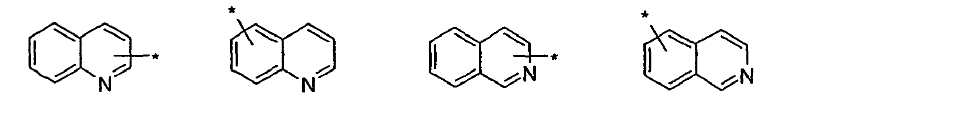 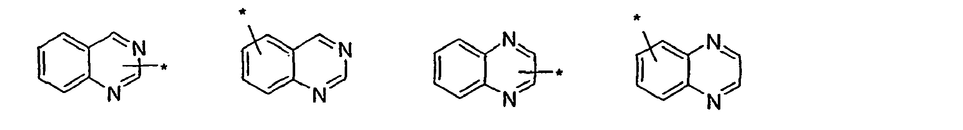 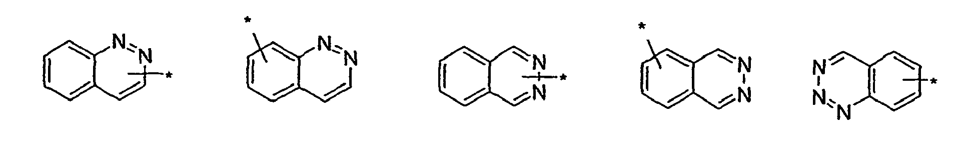 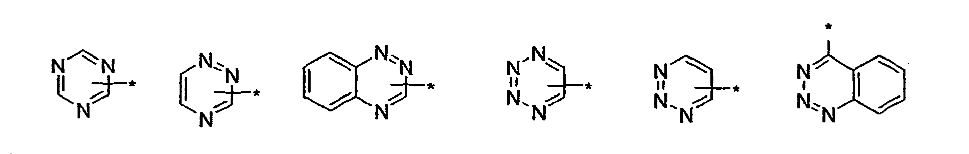 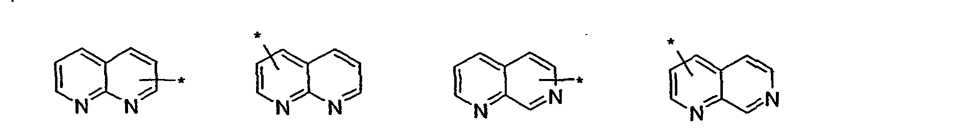 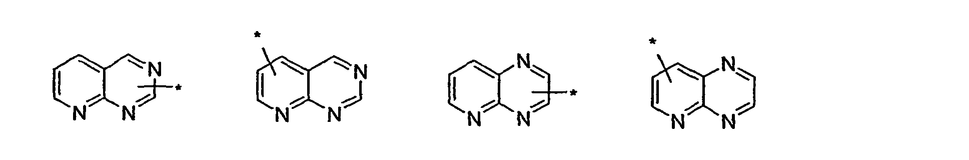 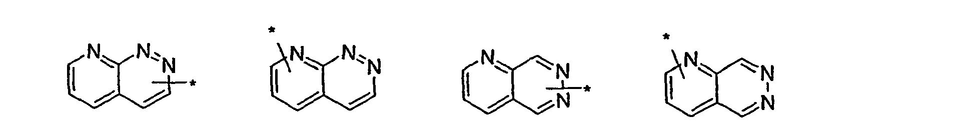 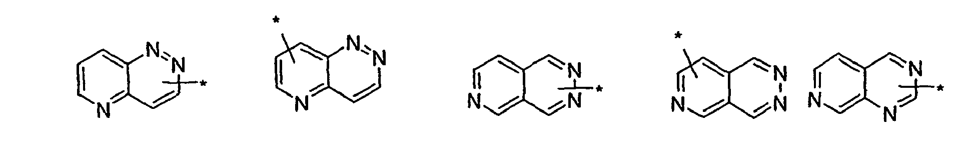 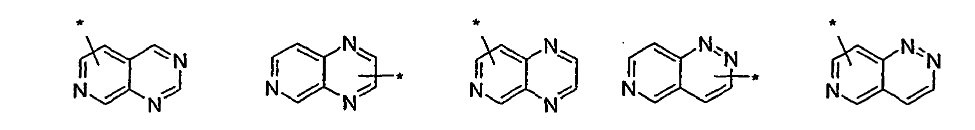 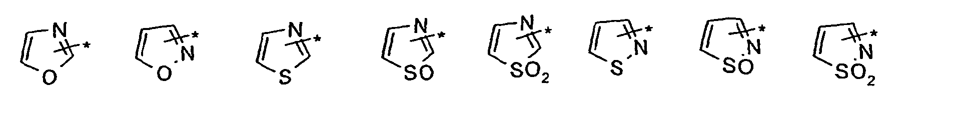 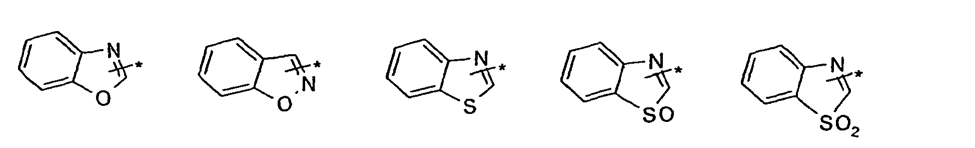 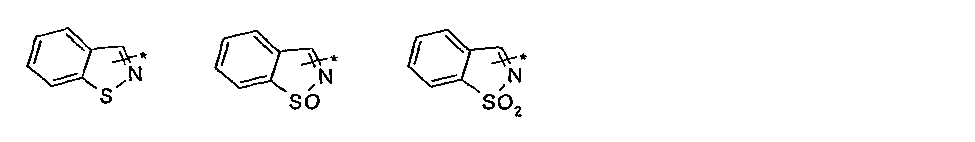 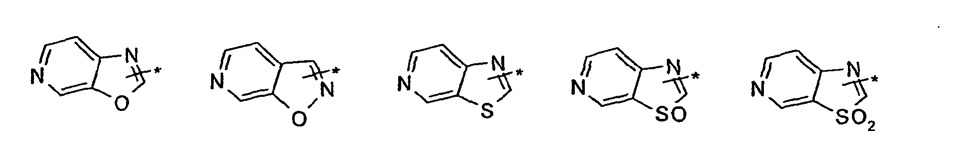 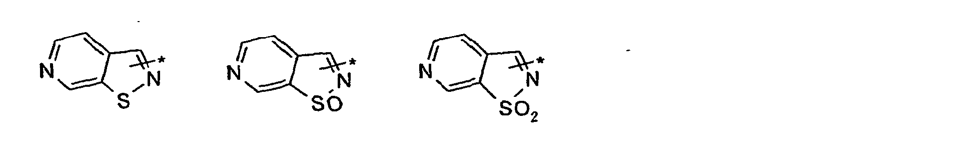 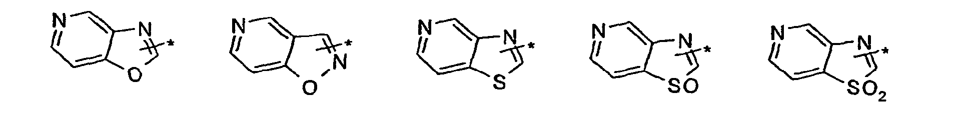 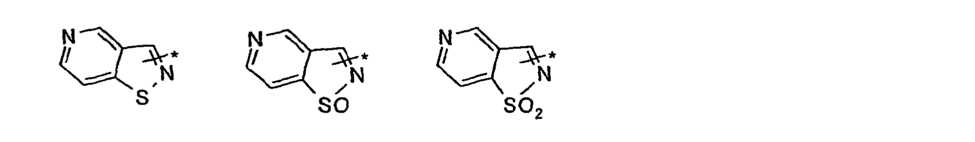 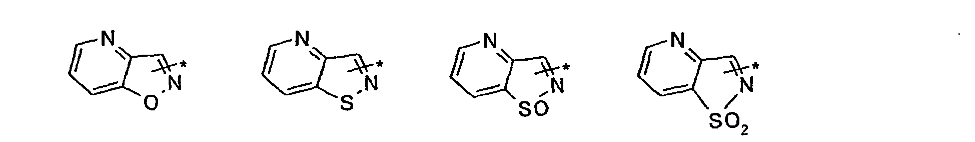 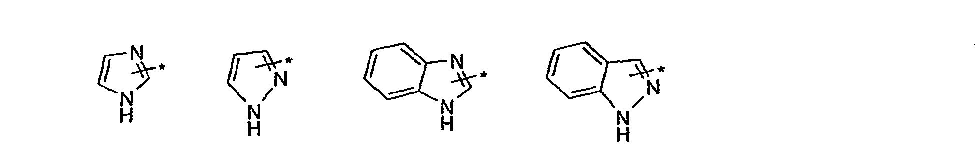 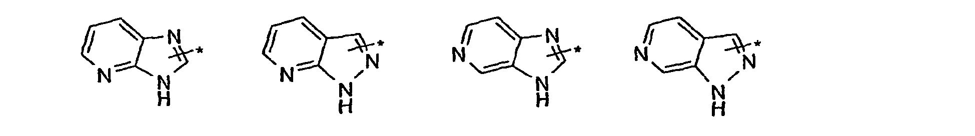 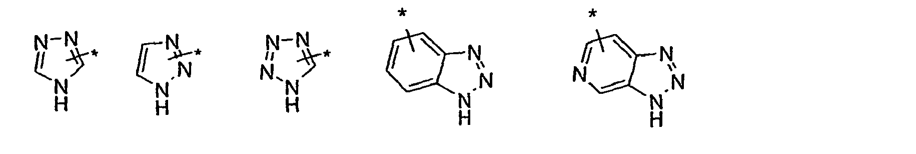 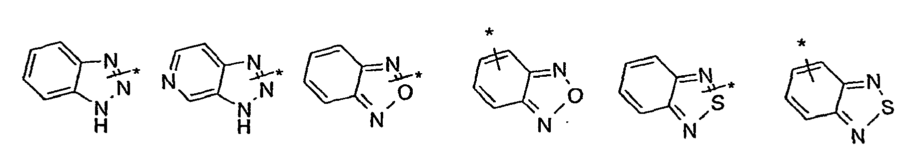 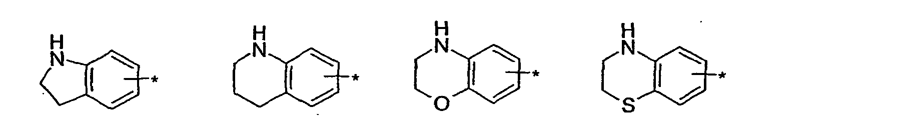 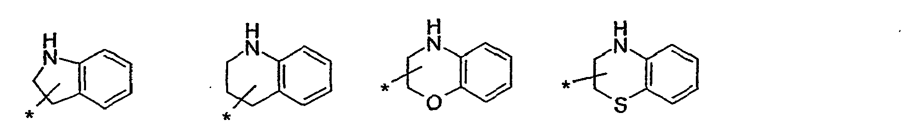 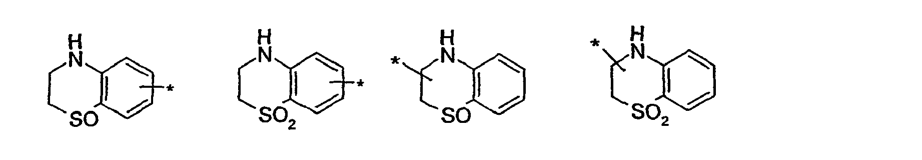 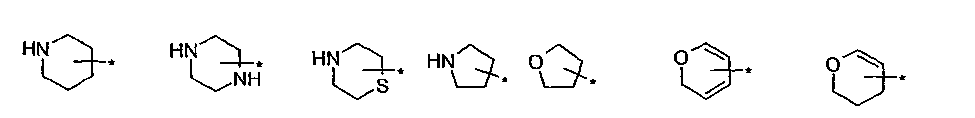 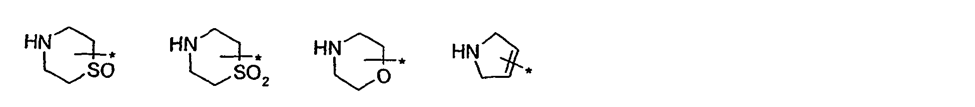 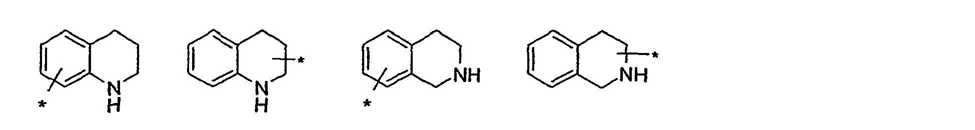 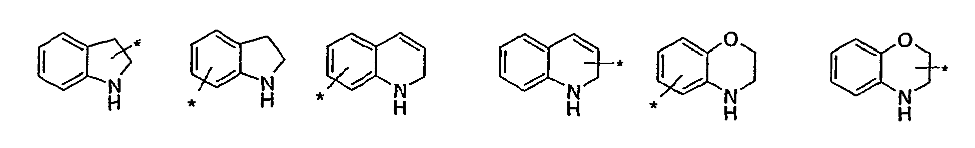 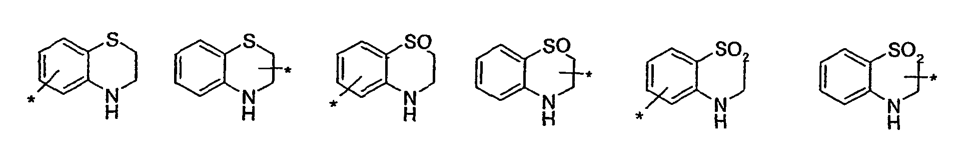 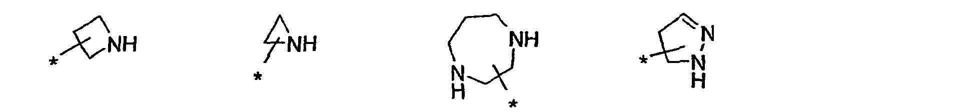 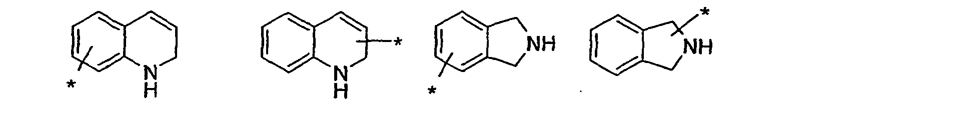 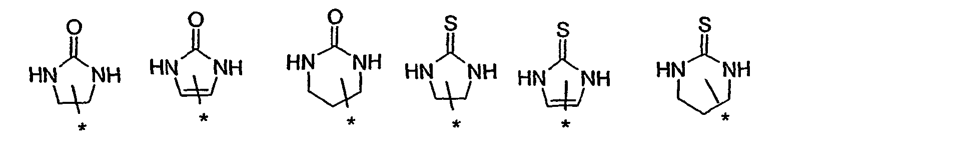 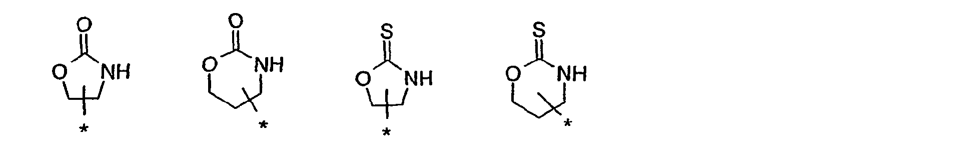 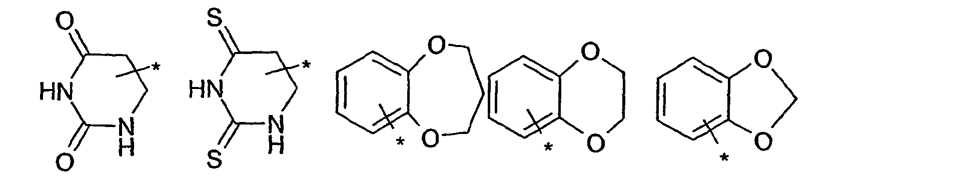 or in the case of where heterocyclyl is present in R₃ defined as unsubstituted or substituted heterocyclyl, unsubstituted or substituted heterocyclyl-alkyl or substituted or unsubstituted heterocyclylsulfonyl in addition selected from 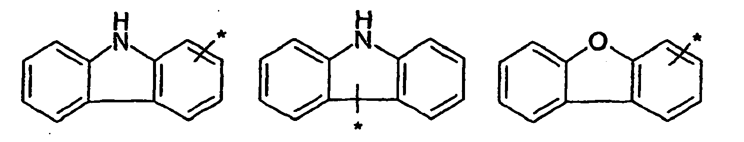 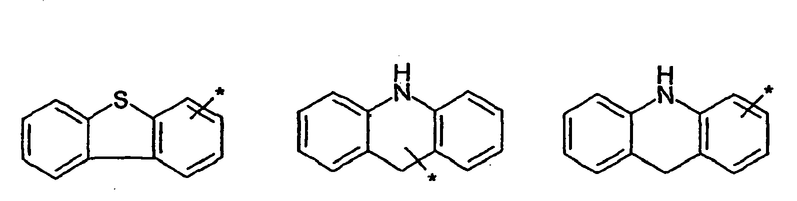 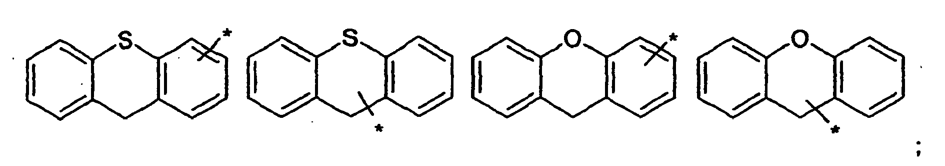 where in each case where an NH is present the bond with the asterisk connecting the respective heterocyclyl moiety to the rest of the molecule the H may be replaced with said bond and/or the H may be replaced by a substituent, and one or more substituents may be present as just described.

Whenever anunsubstituted or substituted heterocyclyl moiety is present as part of R¹ and R² or is such a substitutent, this heterocyclyl is mono- or bicyclic, that is, it does not have more than two annelated rings (while more rings bound via single bonds which are not annelated, such as aryl substituents or the like, are possible).

Unsubstituted or substituted cycloalkyl is mono- or polycyclic, preferably monocyclic, C₃-C₁₀-cycloalkyl which may include one or more double (e.g. in cycloalkenyl) and/or triple bonds (e.g. in cycloalkinyl), and is unsubstituted or substituted by one or more, e.g. one to three substitutents independently selected from those mentioned above as substituents for aryl.

In unsubstituted or substituted aryl-alkyl, aryl (which is preferably unsubstituted or substituted by one or more substituents, e.g. one to three substituents independently selected from those mentioned above as substituents for aryl) is preferably as described above for aryl and is bound to alkyl, preferably C₁-C₇-alkyl, either terminally or at any other carbon in the alkyl chain, e.g. at the 1-carbon.

In unsubstituted or substituted heterocyclyl-alkyl, heterocyclyl is as described above and is unsubstituted or substituted by one or more, e.g. up to three, substitutents independently selected from those mentioned above for substituted aryl, and heterocyclyl is bound to alkyl, preferably C₁-C₇-alkyl, either terminally or at any other carbon in the alkyl chain, e.g. at the 1-carbon.

In unsubstituted or substituted cycloalkyl-alkyl, cycloalkyl is as described above and is unsubstituted or substituted by one or more, e.g. up to three, substitutents independently selected from those mentioned above for substituted aryl, and cycloalkyl is bound to alkyl, preferably C₁-C₇-alkyl, either terminally or at any other carbon in the alkyl chain, e.g. at the 1-carbon.

Acyl is preferably unsubstituted or substituted aryl-carbonyl or -sulfonyl, unsubstituted or substituted heterocyclylcarbonyl or -sulfonyl, unsubstituted or substituted cycloalkylcarbonyl or -sulfonyl, formyl or unsubstituted or substituted alkylcarbonyl or -sulfonyl, wherein unsubstituted or substituted aryl, unsubstituted or substituted heterocyclyl and unsubstituted or substituted cycloalkyl are as defined above and unsubstituted or substituted alkyl is as described below.

Unsubstituted or substituted alkyl is C₁-C₂₀-alkyl, preferably C₁-C₇-alkyl, that is straight-chained or branched (one or, where appropriate, more times), which is unsubstituted or substituted by one or more, e.g. up to three moieties selected from unsubstituted or substituted aryl as described above, especially phenyl or naphthyl each of which is unsubstituted or substituted as described above for unsubstituted or substituted aryl, unsubstituted or substituted heterocycyclyl as described above, especially pyrrolyl, furanyl, thienyl, pyrimidine-2,4-dione-1-, -2-, -3- or -5-yl or benzo[1,3]dioxolyl, which heterocyclyl is unsubstituted or substituted as described above for unsubstituted or substituted heterocyclyl; unsubstituted or substituted cycloalkyl as described above, especially cyclopropyl, cyclobutyl, cyclopentyl or cyclohexyl each of which is unsubstituted or substituted as described above for unsubstituted or substituted cycloalkyl; C₂-C₇-alkenyl, C₂-C₇-alkinyl, halo, hydroxy, C₁-C₇-alkoxy, halo-C₁-C₇-alkoxy, such as trifluoromethoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, benzoyl- or naphthoyloxy, C₁-C₇-alkylthio, halo-C₁-C₇-alkthio, such as trifluoromethylthio, hydroxy-C₁-C₇-alkylthio, C₁-C₇-alkoxy-C₁-C₇-alkylthio, phenyl- or naphthylthio, phenyl- or naphthyl-C₁-C₇-alkylthio, C₁-C₇-alkanoylthio, benzoyl- or naphthoylthio, nitro, amino, mono-or di-(C₁-C₇-alkyl, hydroxy-C₁-C₇-alkyl and/or C₁-C₇-alkoxy-C₁-C₇-alkyl)-amino, mono- or di-(naphthyl- or phenyl-C₁-C₇-alkyl)-amino, C₁-C₇-alkanoylamino, benzoyl- or naphthoylamino, C₁-C₇-alkylsulfonylamino, phenyl- or naphthylsulfonylamino wherein phenyl or naphthyl is unsubstituted or substituted by one or more, especially one to three, C₁-C₇-alkyl moieties, phenyl- or naphthyl-C₁-C₇-alkylsulfonylamino, carboxyl, C₁-C₇-alkyl-carbonyl, C₁-C₇-alkoxycarbonyl, phenyl- or naphthyloxycarbonyl, phenyl- or naphthyl-C₁-C₇-alkoxycarbonyl, carbamoyl, N- mono- or N,N-di-(C₁-C₇-alkyl)-aminocarbonyl, N-mono- or N,N-di-(naphthyl- or phenyl-C₁-C₇-alkyl)-aminocarbonyl, cyano, C₁-C₇-alkenylene or -alkinylene, C₁-C₇-al-kylenedioxy, sulfenyl, (-S-OH) sulfonyl (-S(=O)-OH), C₁-C₇-alkyleulfinyl (C₁-C₇-alkyl-S(=O)-), phenyl- or naphthylsulfinyl wherein phenyl or naphthyl is unsubstituted or substituted by one or more, especially one to three, C₁-C₇-alkyl moieties, phenyl- or naphthyl-C₁-C₇-alkylsulfinyl, sulfonyl, C₁-C₇-alkylsulfonyl, phenyl- or naphthylsulfonyl wherein phenyl or naphthyl is unsubstituted or substituted by one or more, especially one to three, C₁-C₇-alkyl moieties, phenyl- or naphthyl-C₁-C₇-alkylsulfonyl, sulfamoyl, N-mono or N,N-di-(C₁-C₇-alkyl, phenyl-, naphthyl, phenyl-C₁-C₇-alkyl or naphthyl-C₁-C₇-alkyl)-aminosulfonyl, N-mono-, N'-mono-, N,N-di- or N,N,N'-tri-(C₁-C₇-alkyl, hydroxy-C₁-C₇-alkyl and/or C₁-C₇-alkoxy-C₁-C₇-alkyl)-aminocarbonylamino and N-mono-, N'-mono-, N,N-di- or N,N,N'-tri-(C₁-C₇-alkyl, hydroxy-C₁-C₇-alkyl and/or C₁-C₇-alkoxy-C₁-C₇-alkyl) aminosulfonylamino. In cases where unsubstituted or substituted heterocyclyl-alkyl, unsubstituted or substituted aryl-alkyl or unsubstituted or substituted cycloalkyl-alkyl-moieties are mentioned as substituents, the definition of unsubstituted or substituted alkyl relates to such moieties which, in addition to unsubstituted or substituted heterocyclyl, aryl or cycloalkyl comprise at least one further and different moiety (especially from those mentioned in this paragraph) as alkyl substituent.

In substituted or unsubstituted alkylsulfonyl, substituted or unsubstituted alkyl is as defined above for unsubstituted or substituted alkyl.

In substituted or unsubstituted arylsulfonyl, substituted or unsubstituted aryl is as defined above for unsubstituted or substituted aryl.

In substituted or unsubstituted heterocyclylsulfonyl, substituted or unsubstituted heterocyclyl is as defined above for unsubstituted or substituted heterocyclyl.

In substituted or unsubstituted cycloalkylsulfonyl, unsubstituted or substituted cycloalkyl is as defined above for unsubstituted or substituted cycloalkyl.

When R³ and R⁴ which then is -O- together with L which then is methylene and the carbon to which R³-L- and R⁴ are bound form a substituted or unsubstituted ring (with one or more, e.g. up to 3, substituents independently selected from those mentioned above for aryl, preferably without substituent) annealed to an unsubstituted or substituted aryl, unsubstituted or substituted heterocyclyl or unsubstituted or substituted cycloalkyl, each of which is as defined above, thus forming a spiro compound of the formula I; preferred is an unsubstituted ring with five ring atoms one of which is the carbon in the central 3,4-substituted pyrrolidinyl ring in formula I, the second methylene L, the third -0- (R⁴) and two of which belong to an annealed unsubstituted (preferred) or substituted benzo wherein the substituents are one or more, especially up to three, substituents independently selected from those mentioned above for substituted aryl.

In unsubstituted or substituted imino (=NH or -NH-) (as well as where substituted NH-groups are present in -heterocycles), the substituents are selected from the group consisting of
a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-, -CO-NV-; -NV-SO₂-, -SO₂-NV; -NV-CO-NV-, -NV-CO-O-, -O-CO-NV-, -NV-SO₂-NV-, where preferably if r is 1 and X is -O-, -NV-, -S-, -O-CO-, NV-CO-, -NV-SO₂-, -NV-CO-NV- or O-CO-NV-, -NV-SO₂-NV-, the substituent has the formula -(C₁-C₇-alkylene)-(X)ᵣ-(C₁-C₇-alkylene)-(Y)ₛ-(C₀-C₇-alkylene)-H; wherein V is hydrogen or unsubstituted or substituted alkyl as defined above, especially selected from C₁-C₇-alkyl, phenyl, naphthyl, phenyl- or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇-alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl;
C₂-C₇-alkenyl, C₂-C₇-alkinyl, phenyl or, naphthyl, heterocyclyl, especially as defined below for heterocyclyl, preferably selected from pyrrolyl, furanyl, thienyl, pyrimidine-2,4-dione-1-, -3- or -5-yl and benzo[1,3]-dioxolyl, phenyl- or naphthyl-C₁-C₇-alkyl, such as benzyl or naphthylmethyl, heterocyclyl-C₁-C₇-alkyl wherein heterocyclyl is especially as defined below for heterocyclyl, preferably selected from pyrrolyl, furanyl, thienyl, pyrimidine-2,4-dione-1-, -3- or -5-yl and benzo[1,3]-dioxolyl, halo-C₁-C₇-alkyl, such as trifluoromethylmethyl, hydroxy-C₁-C₇-alkyl, C₁-C₇-alkoxy-C₁-C₇-alkyl, such as 3-methoxypropyl or 2-methoxyethyl, phenyloxy- or naphthyloxy-C₁-C₇-alkyl, phenyl-C₁-C₇-alkoxy- or naphthyl-C₁-C₇-alkoxy-C₁-C₇-alkyl, amino-C₂-C₇-alkyl, (N-) mono- or (N,N-) di-(C₁-C₇-alkyl)-amino-C₁-C₇-alkyl, mono- or , di-(naphthyl or phenyl)-amino-C₁-C₁-alkyl, di-(naphthyl- or phenyl-C₁-C₇-alkyl)-amino-C₁-C₇-alkyl, C₁-C₇-alkanoylamino-C₁-C₇-alkyl, benzoyl- or naphthoytamino-C₁-C₇-alkyl, C₁-C₇-alkylsulfonylamino-C₁-C₇-alkyl, phenyl- or naphthylsulfonylamino-C₁-C₇-alkyl wherein phenyl or naphthyl is unsubstituted or substituted by one or more, especially one to three, C₁-C₇-alkyl moieties, phenyl- or naphthyl-C₁-C₇-alkylsulfonylamino-C₁-C₇-alkyl, C₁-C₇-alkylcarbonyl, halo-C₁-C₇-alkylcarbonyl, hydroxy-C₁-C₇-alkylcarbonyl, C₁-C₇-alkoxy-C₁-C₇-alkylcarbonyl, amino-C₁-C₇- alkylcarbonyl, (N-) mono- or (N,N-) di-(C₁-C₇-alkyl)-amino-C₁-C₇-alkylcarbonyl, C₁-C₇-alkanoylamino-C₁-C₇- alkylcarbonyl, C₁-C₇-alkoxy-carbonyl, halo-C₁-C₇-alkoxycarbony, hydroxy-C₁-C₇-alkoxycarbonyl, C₁-C₇-alkoxy-C₁-C₇-alkoxycarbonyl, amino-C₁-C₇-alkoxycarbonyl, (N-) mono- or (N,N-) di-(C₁-C₇-alkyl)-amino-C₁-C₇-alkoxycarbonyl, C₁-C₇-alkanoylamino-C₁-C₇-alkoxycarbonyl, halo-C₁-C₇-alkoxycarbonyl, phenyl- or naphthyloxycarbonyl, phenyl- or naphthyl-C₁-C₇-alkoxycarbonyl, C₁-C₇-alkylsulfinyl, phenyl- or naphthylsulfinyl wherein phenyl or naphthyl is unsubstituted or substituted by one or more, especially one to three, C₁-C₇-alkyl moieties, phenyl- or naphthyl-C₁-C₇-alkylsulfinyl, sulfonyl, C₁-C₇-alkylsulfonyl, halo-C₁-C₇-alkylsulfonyl , hydroxy-C₁-C₇-alkylsulfonyl, C₁-C₇alkoxy-C₁-C₇-alkylsulfonyl, amino-C₁-C₇-alkylsulfonyl, (N,N-) di-(C₁-C₇-alkyl)-amino-C₁-C₇-alkylsulfonyl, C₁-C₇-alkanoylamino-C₁-C₇-alkylsulfonyl, phenyl- or naphthylsulfonyl wherein phenyl or naphthyl is unsubstituted or substituted by one or more, especially one to three, C₁-C₇-alkyl moieties, and phenyl- or naphthyl-C₁-C₇-alkylsulfonyl.

In unsubstituted or substituted alkylcarbonyl, unsubstituted or substituted alkyl is as defined above. An example is C₁-C₇-alkanoyl.

In unsubstituted or substituted arylcarbonyl, unsubstituted or substituted heterocyclylcarbonyl and unsubstituted or substituted cycloalkylcarbonyl, the unsubstituted or substituted aryl, heterocyclyl and cycloalkyl moieties, respectively, are as described for the corresponding unsubstituted or substituted aryl, heterocyclyl and cycloalkyl moieties, respectively.

Etherified carboxy (-C(=O)-O-bound group) is carbonyl (preferably bound to L = oxy or especially imino) to which a moiety selected from unsubstituted or substituted alkyloxy, unsubstituted or substituted aryloxy, unsubstituted or substituted heterocyclyloxy or unsubstituted or substituted cycloalkyloxy, in each of which the unsubstituted or substituted alkyl, aryl, heterocyclyl or cycloalkyl moieties are defined as above, is bound as bound group; especially preferred is unsubstituted or substituted alkoxycarbonyl (unsubstituted or substituted alkyl-O-C(=O)-), especially C₁-C₇-alkoxycarbonyl, bound to L = imino.

N-mono- or N,N-di-substituted aminocarbonyl is aminocarbonyl (-C(=OFNH₂) (preferably bound to L = imino or especially oxy) that is mono- or di-substituted at the nitrogen preferably by one or more moieties selected from unsubstituted or substituted alkyl, unsubstituted or substituted aryl, unsubstituted or substituted heterocyclyl or unsubstituted or substituted cycloalkyl, each of which is defined as above; a preferred example is aryl-C₁-C₇-alkylaminocarbonyl (= aryl-C₁-C₇-NH-C(=O)-), such as benzylaminocarbonyl, bound to L = oxy or further imino.

N-mono- or N,N-di-substituted aminosulfonyl is sulfamoyl (-S(=O)₂-NH₂) (preferably bound to L = imino or especially oxy) that is mono- or di-substituted at the nitrogen preferably by one or more moieties selected from unsubstituted or substituted alkyl, unsubstituted or substituted aryl, unsubstituted or substituted heterocyclyl or unsubstituted or substituted cycloalkyl, each of which is defined as above; a preferred example is aryl-C₁-C₇-alkylaminosuffonyl (= aryl-C₁-C₇-NH-S(=O)₂-), such as benzylaminosulfonyl, bound to L = oxy or further imino.

In all definitions above it goes without saying that only stable compounds the person having skill in the art will, without undue experimentation or considerations, be able to recognize are important (e.g. those that are sufficiently stable for the manufacture of pharmaceuticals, e.g. having a half-life of more than 30 seconds) and thus are preferably encompassed by the present claims and that only chemically feasible bonds and substitutions are encompassed, as well as tautomeric forms where present.

Salts are especially the pharmaceutically acceptable salts of compounds of formula I. They can be formed where salt forming groups, such as basic or acidic groups, are present that can exist in dissociated form at least partially, e.g. in a pH range from 4 to 10 in aqueous solutions, or can be isolated especially in solid form.

Such salts are formed, for example, as acid addition salts, preferably with organic or inorganic adds, from compounds of formula I with a basic nitrogen atom (e.g. imino or amino), especially the pharmaceutically acceptable salts. Suitable inorganic adds are, for example, halogen acids, such as hydrochloric add, sulfuric acid, or phosphoric acid. Suitable organic acids are, for example, carboxylic, phosphonic, sulfonic or sulfamic acids, for example acetic acid, propionic acid, lactic add, fumaric acid, succinic acid, citric acid, amino acids, such as glutamic acid or aspartic acid, maleic acid, hydroxymaleic acid, methyl maleic acid, benzoic acid, methane- or ethane-sulfonic add, ethane-1,2-disulfonic acid, benzenesulfonic acid, 2-naphthalenesulfonic acid, 1,5-naphthalene-disulfonic acid, N-cyclohexylsulfamic acid, N-methyl-, N-ethyl- or N-propyl-sulfamic acid, or other organic protonic acids, such as ascorbic acid.

In the presence of negatively charged radicals, such as carboxy or sulfo, salts may also be formed with bases, e.g. metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri(2-hydroxyethyl)amine, or heterocyclic bases, for example N-ethyl-piperidine or N,N'-dimethylpiperazine.

When a basic group and an acid group are present in the same molecule, a compound of formula I may also form internal salts.

For isolation or purification purposes it is also possible to use pharmaceutically unacceptable salts, for example picrates or perchlorates. For therapeutic use, only pharmaceutically acceptable salts or free compounds are employed (where applicable comprised in pharmaceutical preparations), and these are therefore preferred.

In view of the close relationship between the compounds in free form and in the form of their salts, including those salts that can be used as intermediates, for example in the purification or identification of the compounds or salts thereof, any reference to "compounds" and "intermediates" hereinbefore and hereinafter, especially to the compound(s) of the formula I, is to be understood as referring also to one or more salts thereof or a mixture of a free compound and one or more salts thereof, each of which is intended to include also any solvate of the compound of formula I, or salt of any one or more of these, as appropriate and expedient and if not explicitly mentioned otherwise. Different crystal forms may be obtainable and then are also included.

Where the plural form is used for compounds, salts, pharmaceutical preparations, diseases, disorders and the like, this is intended to mean one (preferred) or more single compound(s), salt(s), pharmaceutical preparation(s), disease(s), disorder(s) or the like, where the singular or the indefinite article ("a", "an") is used, this is intended to include the plural or preferably the singular.

The compounds of the present invention possess two or more asymmetric centers depending on the choice of the substituents. The preferred absolute configuration at the C-3 and C-4 asymmetric centers is maintained throughout the specification and the appended claims as indicated herein-above. However, any possible diastereoisomers, enantiomers and geometric isomers, and mixtures thereof, e.g., racemates, are encompassed by the present invention.

As described herein above, the present invention provides 3,4-disubstituted pyrrolidine derivatives of formula I, these compounds for use in the (prophylactic and/or therapeutic) treatment of a disease (= condition, disorder) in a warm-blooded animal, especially a human, preferably of a disease dependent on (especially inappropriate) renin activity, a pharmaceutical composition comprising a compound of the formula I, methods for preparing said compound or pharmaceutical preparation, and methods of treating conditions dependent on (especially inappropriate) renin activity by administration of a therapeutically effective amount of a compound of the formula I, or a pharmaceutical composition thereof.

"Inappropriate" renin activity preferably relates to a state of a warm-blooded animal, especially a human, where renin shows a renin activity that is too high in the given situation (e.g. due to one or more of misregulation, overexpression e.g. due to gene amplification or chromosome rearrangement or infection by microorganisms such as virus that express an aberrant gene, abnormal activity e.g. leading to an erroneous substrate specificity or a hyperactive renin e.g. produced in normal amounts, too low activity of renin activity product removing pathways, high substrate concentration, other circumstances that make the activity of renin relatively too high, such as other mechanisms leading to blood pressure increase, and/or the like) and/or leads to or supports a renin dependent disease or disorder as mentioned above and below, e.g. by renin activity the reduction of which has beneficial effects in the given disease. Such inappropriate renin activity may, for example, comprise a higher than normal activity, or further an activity in the normal or even below the normal range which, however, due to preceding, parallel and or subsequent processes, e.g. signaling, regulatory effect on other processes, higher substrate or product concentration and the like, leads to direct or indirect support or maintenance of a disease or disorder, and/or an activity that supports the outbreak and/ or presence of a disease or disorder in any other way. The inappropriate activity of renin may or may not be dependent on parallel other mechanisms supporting the disorder or disease, and/or the prophylactic or therapeutic effect may or may include other mechanisms in addition to inhibition of renin. Therefore "dependent" has to be read as "dependent inter alia", (especially in cases where a disease or disorder is really exclusively dependent only on renin) preferably as "dependent mainly", more preferably as "dependent essentially only".

Where a disease or disorder dependent on inappropriate activity of a renin is mentioned (such in the definition of "use" in the following paragraph and also especially where a compound of the formula I is mentioned for use in the diagnostic or therapeutic treatment which is preferably the treatment of a disease or disorder dependent on inappropriate renin activity, this refers preferably to any one or more diseases or disorders that depend on inappropriate activity of natural renin and/or one or more altered or mutated forms (including alleles or single nuclear polymorphism forms thereof).

Where subsequently or above the term "use" is mentioned (as verb or noun) (relating to the use of a compound of the formula I or of a pharmaceutically acceptable salt thereof), this (if not indicated differently or to be read differently in the context) includes any one or more of the following embodiments of the invention, respectively (if not stated otherwise): the use for the manufacture of pharmaceutical compositions for use in the treatment of a disease or disorder that depends on (especially inappropriate) activity of renin; a pharmaceutical preparation comprising one or more compounds of the formula I for the treatment of a disease or disorder that depends on (especially inappropriate) activity of renin; and one or more compounds of the formula I for use in the treatment of a disease or disorder in a warm-blooded animal, especially a human, preferably a disease that depends on (especially inappropriate) activity of renin; as appropriate and expedient, if not stated otherwise.

For a compound of the formula I wherein R¹ is hydrogen, R² is 1-N-(carbamoylmethyl)-carbamoyl-1-(2-methyl-n-propyl), R³ is phenyl, T is carbonyl and L is methylen, or a salt thereof, the use as mentioned above is claimed only.

The terms "treat", "treatment" or "therapy" refer to the prophylactic (e.g. delaying or preventing the onset of a disease or disorder) or preferably therapeutic (including but not limited to preventive, delay of onset and/or progression, palliative, curing, symptom-alleviating, symptom-reducing, patient condition ameliorating, renin-modulating and/or renin-inhibiting) treatment of said disease(s) or disorder(s), especially of the one or more disease or disorder mentioned above or below.

Preferred embodiments according to the invention

The groups of preferred embodiments of the invention mentioned below are not to be regarded as exclusive, rather, e.g., in order to replace general expressions or symbols with more specific definitions, parts of those groups of compounds can be interchanged or exchanged using the definitions given above, or omitted, as appropriate.

Highly preferred is a compound of the formula IA with the following configuration:

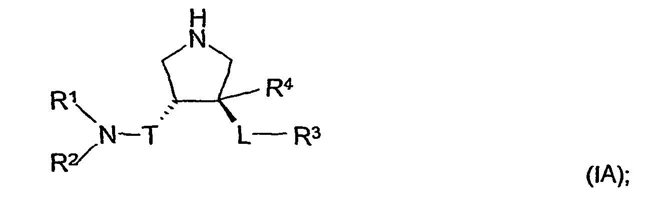

Preferred is a compound of the formula IB with the following configuration:

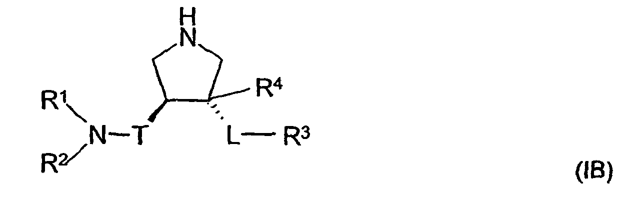

Preferred is also a compound of the formula IC with the following configuration:

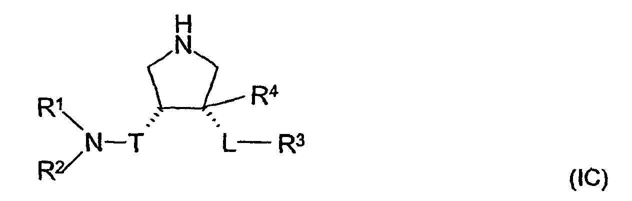

Preferred is also a compound of the formula ID with the following configuration:

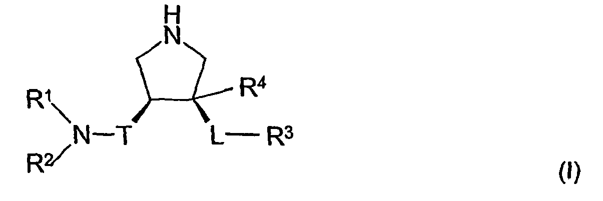

In each of the formulae IA, IB, IC and ID, the moieties R¹, R², R³, R⁴, L and T are as defined hereinbefore or preferably hereinafter.

The formula IA, IB, IC or ID can replace formula I wherever a compound of the formula I (including a salt thereof) is mentioned hereinbefore or hereinafter; also, the corresponding intermediates are preferred.

A preferred embodiment of the invention relates to a compound of the formula I, wherein
R¹ is phenyl or naphthyl, each of which is unsubstituted or substituted by one or more, e.g. up to three, substitutents selected from the group consisting of C₁-C₇-alkyl, phenyl, naphthyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylamino-C₁-C₇-alkyl, halo, hydroxy, C₁-C₇-alkoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl and/or (phenyl- or naphthyl)-C₁-C₇-alkyl)-carbamoyl, C₁-C₇-alkylsulfonyl, unsubstituted or C₁-C₇-alkyl-substituted phenyl- or naphthylsulfonyl, N-mono- or N,N-di-(C₁-C₇-alkyl and/or (phenyl- or naphthyl)-C₁-C₇-alkyl)-sulfamoyl and cyano;
   phenyl- or naphthyl-C₁-C₇-alkyl, wherein each of phenyl or naphthyl is unsubstituted or substituted by one or more, e.g. up to three, substitutents selected from the group consisting of the substitutents just mentioned for substituted phenyl or naphthyl, pyrrolyl, furanyl, thienyl, pyrimidine-2,4-dione-1-, -2-, -3- or -5-yl and benzo[1,3]dioxalyl , each if which is unsubstituted or substituted by one or more, e.g. up to three, substituents independently selected from those mentioned for substituted phenyl or naphthyl R¹ above, especially C₁-C₇-alkyl, halo-C₁-C₇-alkyl, hydroxy-C₁-C₇-alkoxy, C₁-C₇-alkoxy-C₁-C₇-alkyl and C₁-C₇-alkyloxy; pyrrolyl-C₁-C₇-alkyl, furanyl-C₁-C₇-alkyl, thienyl-C₁-C₇-alkyl, pyrimidine-2,4-dione-1-, -2-, -3- or -5-yl-C₁-C₇-alkyl, indolyl-C₁-C₇-alkyl, benzofuranyl-C₁-C₇-alkyl, benzimidazolyl-C₁-C₇-alkyl, benzopyrazolyl-C₁-C₇-alkyl, quinolinyl-C₁-C₇-alkyl, isoquinolyl-C₁-C₇-alkyl or benzo[1,2,5]oxadiazolyl-C₁-C₇-alkyl, each if which is unsubstituted or substituted by one or more, e.g. up to three, substituents independently selected from the substitutents mentioned above for substituted phenyl or naphthyl R¹, especially C₁-C₇-alkyl, halo-C₁-C₇-alkyl, hydroxy-C₁-C₇-alkoxy, C₁-C₇-alkoxy-C₁-C₇-alkyl and C₁-C₇-alkyloxy;
   C₃-C₁₀-cycloalkyl which is unsubstituted or substituted by one or more, e.g. up to three, substituents independently selected from the substitutents mentioned above for substituted phenyl or naphthyl R¹, especially by C₁-C₇-alkyl, phenyl, naphthyl, phenyl-C₁-C₇-alkyl or naphthyl-C₁-C₇-alkyl;
   C₃-C₁₀-cycloalkyl-C₁-C₇-alkyl wherein cycloalkyl is unsubstituted or substituted by one or more, e.g. up to three, substituents independently selected from the substitutents mentioned above for substituted phenyl or naphthyl R¹, especially by C₁-C₇-alkyl, phenyl, naphthyl, phenyl-C₁-C₇-alkyl or naphthyl-C₁-C₇alkyl;
   phenyl- or naphthyl-carbonyl or phenyl- or naphthyl-C₁-C₇-alkylcarbonyl, wherein each phenyl or naphthyl is unsubstituted or substituted by one or more, e.g. up to three, substitutents selected from the group consisting of
   - a substitutent of the formula -(C₀-C₇-alkylene)-(X)ᵣ-(C₁-C₇-alkylene)-(Y)ₛ(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, especially selected from C₁-C₇-alkyl, phenyl, naphthyl, phenyl- or naphthyl-C₁-C₇-alkyl and halo-C₁-C₇-alkyl; e.g. C₁-C₇alkyl, such as methyl, ethyl, n-propyl, isopropyl, n-butyl, isobutyl, sec-butyl or tert-butyl, hydroxy-C₁-C₇alkyl; C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇-alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
   - from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, phenyl- or naphthyl-C₁-C₇-alkoxycarbonyl, halo-C₁-C₇-alkoxycarbonyl, C₁-C₇-alkylsulfonyl, carbamoyl and cyano;
   pyrrolylcarbonyl, furanylcarbonyl, thienylcarbonyl, pyrimidine-2,4-dione-1-, -2-, -3- or -5-yl-carbonyl, indolyl- carbonyl, benzimidazolyl-carbonyl, benzopyrazolyl-carbonyl benzofuranyl-carbonyl, quinolinyl-carbonyl or benzo[1,2,5]oxadiazolyl-carbonyl, each if which is unsubstituted or substituted by one or more, e.g. up to three, substituents independently selected from a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, especially selected from C₁-C₇-alkyl, phenyl, naphthyl, phenyl- or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
   from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, phenyl- or naphthyl-C₁-C₇-alkoxycarbonyl, halo-C₁-C₇-alkoxycarbonyl, C₁-C₇-alkylsulfonyl, carbamoyl and cyano;
   or phenyl- or naphthyl-sulfonyl, wherein each phenyl or naphthyl is unsubstituted or substituted by one or more, e.g. up to three, substitutents selected from those mentioned above for substituted phenyl or naphthyl R¹, preferably from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, halo, hydroxy, C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl and cyano;
R² is
   C₁-C₇-alkyl that is unsubstituted or substituted by one or more, e.g. up to three, substitutents selected from the group consisting of halo, phenyl- or naphthyl, hydroxy, C₁-C₇-alkoxy, amino, mono- or di-(C₁-C₇-alkyl)-amino, C₁-C₇-alkanoylamino, C₁-C₇-alkyl-sulfonylamino, phenyl- or napthylsulfonylamino, phenyl- or naphthyl-C₁-C₇-alkylsulfonylamino, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl; and cyano;
   phenyl or naphthyl, each of which is unsubstituted or substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkylsulfonylamino-C₁-C₇-alkyl, halo, hydroxy, C₁-C₇-alkoxy, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, carboxyl, C₁-C₇-alkoxy-carbonyl, halo-C₁-C₇-alkoxycarbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl and cyano;
   phenyl- or naphthyl-C₁-C₇-alkyl, wherein each of phenyl or naphthyl is unsubstituted or substituted by one or more, e.g. up to three, substitutents selected from the group just mentioned for substituted phenyl or naphthyl R²;
   C₃-C₁₀-cycloalkyl which is unsubstituted or substituted by one or more, e.g. up to three, substitutents selected from the group just mentioned for substituted phenyl or naphthyl R², especially by C₁-C₇-alkyl, phenyl, naphthyl, phenyl-C₁-C₇-alkyl or naphthylC₁-C₇alkyl; C₃-C₇cycloalkyl-C₁-C₇-alkyl wherein cycloalkyl is unsubstituted or substituted by one or more, e.g. up to three, substitutents selected from the group just mentioned for substituted phenyl or naphthyl R², especially by C₁-C₇-alkyl, phenyl, naphthyl, phenyl-C₁-C₇-alkyl or naphthyl-C₁-C₇alkyl;
   or pyrrolyl, furanyl or thienyl,
   or, if L is methylene, oxy, thio or imino, R² is selected from one of the groups of moieties R² just mentioned and from hydrogen;
R³ is
   carbamoyl or N-mono- or N,N-di-(C₃-C₈-cloalkyl-, C₁-C₇-alkyl-, phenyl-C₁-C₇-alkyl- and/or naphthyl-C₁-C₇-alkyl-)aminocarbono-C₁-C₇-alkyl;
   phenyl, naphthyl, phenyl-C₁-C₇alkyl or naphthyl-C₁-C₇-alkyl, wherein each phenyl or naphthyl is unsubstituted or substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-Cₗ-C₇-alkyl, C₁-C₇-alkanoylamino-C₁-C₇-alkyl, C₁-C₇-alkyl-sulfon4ylamino-C₁-C₇-alkyl, phenyl, naphthyl, mono- or di-(C₁-C₇-alkoxy)-phenyl or -naphthyl, C₁-C₇-alkylendioxy-phenyl where the oxy atoms are bound to adjacent phenyl ring atoms, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, (unsubstituted or mono-, di- or tri-C₁-C₇-alkyl substituted)-phenyl- or-naphthyl-C₁-C₇-alkyloxy, C₁-C₇-alkanoyloxy, nitro, amino, mono- or di-(C₁-C₇-alkyl)-amino, C₁-C₇-alkanoylamino C₁-C₇-alkylsulfonylylamino, carboxyl, C₁-C₇-alkoxy-carbonyl, phenyl- or naphthyl-C₁-C₇-alkoxycarbonyl, carbamoyl and cyano, C₁-C₇-alkylsulfonyl, C₁-C₇-alkylene which is unsubstituted or substituted by up to four C₁-C₇-alkyl substituents and bound to two adjacent ring atoms of the phenyl or naphthyl moiety; pyrrolyl, furanyl and thienyl;
   phenyl- or naphthyl-sulfonyl or phenyl-C₁-C₇-alkyl- or naphthyl-C₁-C₇-alkyl-sulfonyl, wherein each phenyl or naphthyl is unsubstituted or substituted by one or more, e.g. up to three, substitutents selected from the group just described for substituted phenyl- or naphthyl R³, C₃-C₁₀-cycloalkyl or C₃-C₁₀-cyclyoalkyl-C₁-C₇-alkyl, in both of which cycloalkyl is unsubstituted or substituted by one or more of the substitutents just mentioned for substituted phenyl or naphthyl R³, especially by C₁-C₇-alkyl, phenyl, naphthyl, phenyl-C₁-C₇-alkyl or naphthyl-C₁-C₇alkyl;
   or heterocyclyl or heterocyclyl-C₁-C₇-alkyl wherein heterocyclyl is selected from pyrrolyl, furanyl, thienyl, pyrimidine-2,4-dione-1-, -3- or -5-yl, indolyl, benzofuranyl, benzimidazolyl, benzopyrazolyl, quinolinyl, isoquinolinyl, methylene-dioxy-phenyl, ethylene-1,2-dioxy-phenyl or trimethylen-1,3-dioxyphenyl wherein the oxy groups are bound to adjacent ring atoms of the phenyl ring, where each of the heterocyclyl moieties is unsubstituted or substituted as mentioned above for substituted phenyl R³;
   or, if L is imino, oxy or thio, can alternatively be phenyl- or naphthylcarbonyl, C₁-C₇-alkoxycarbonyl (meaning C₁-C₇-alkyl-O-C(=O)-), phenyloxycarbonyl, naphthyloxycarbonyl, phenyl-C₁-C₇-alkyloxycarbonyl, naphthyl-C₁-C₇-alkyloxycarbonyl or N-mono- or N,N-di-(C₁-C₇-alkyl, phenyl, naphthyl, phenyl-C₁-C₇-alkyl and/or naphthyl-C₁-C₇-alkyl)-aminocarbonyl, where in each case the phenyl or naphthyl rings are unsubstituted or substituted as mentioned above for substituted phenyl or naphthyl R³;
R⁴ is hydrogen or hydroxy; and
   L is a bond, methylene (-CH₂-), oxy (-O-) or imino (-NH-);
   or R³ and R⁴ which then is -0- together with L which then is methylene and the carbon to which R³-L- and R⁴ are bound form a substituted or unsubstituted 5-membered ring annealed to benzo where benzo is substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, halo, hydroxy, C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl and cyano, or unsubstituted, thus forming a spiro compound of the formula I, or
R³ and R⁴ together with L form oxo (=O);
   and T is methylene, carbonyl or thiocarbonyl;
   or a pharmaceutically acceptable salt thereof.

A further preferred embodiment of the invention relates to a compound of the formula I, wherein
R¹ is
   phenyl- or naphthyl-carbonyl or phenyl- or naphthyl-C₁-C₇-alkylcarbonyl, wherein each phenyl or naphthyl is unsubstituted or substituted by one or more, e.g. up to three, substitutents selected from the group consisting of
   - a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, especially selected from C₁-C₇-alkyl, phenyl, naphthyl, phenyl- or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, halo-C₁-C₇-alkoxycarbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N-mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
   - from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, halo-C₁-C₇-alkoxycarbonyl, phenyl- or naphthyl-C₁-C₇-alkoxycarbonyl, C₁-C₇-alkylsulfonyl, carbamoyl and cyano;
   pyrrolylcarbonyl, furanylcarbonyl, thienylcarbonyl, pyrimidine-2,4-dione-1-, -2-, -3- or -5-yl-carbonyl, indolyl- carbonyl, benzimidazolyl-carbonyl, benzopyrazolyl-carbonyl benzofuranyl-carbonyl, quinolinyl-carbonyl or benzo[1,2,5]oxadiazolyl-carbonyl, each if which is unsubstituted or substituted by one or more, e.g. up to three, substituents independently selected from a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, especially selected from C₁-C₇-alkyl, phenyl, naphthyl, phenyl- or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl;
   from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, halo-C₁-C₇-alkoxycarbonyl, phenyl- or naphthyl-C₁-C₇-alkoxycarbonyl, C₁-C₇-alkylsulfonyl, carbamoyl and cyano;
R² is
   C₁-C₇-alkyl that is unsubstituted or substituted by one or more, e.g. up to three, substitutents selected from the group consisting of halo, phenyl- or naphthyl, hydroxy, C₁-C₇-alkoxy, amino, mono- or di-(C1-C₇-alkyl)-amino, C₁-C₇-alkanoylamino, C₁-C₇-alkyl-sulfonylamino, phenyl- or napthylsulfonylamino, phenyl- or naphthyl-C₁-C₇-alkylsulfonylamino, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇--alkyl- or naphthyl-C₁-C₇-alkyl-)sulfamoyl; and cyano;
   and R³ and R⁴ which then is -O- together with L which then is methylene and the carbon to which R³-L- and R⁴ are bound form a substituted or unsubstituted 5-membered ring annealed to benzo where benzo is substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, halo, hydroxy, C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl and cyano, or unsubstituted, thus forming a spiro compound of the formula I;
   and T is carbonyl or thiocarbonyl or preferably methylene;
   or a pharmaceutically acceptable salt thereof.

Still another preferred embodiment of the inventionr relates to a compound of the formula I, wherein
R¹ is
   phenyl- or naphthyl-carbonyl or phenyl- or naphthyl-C₁-C₇-alkylcarbonyl, wherein each phenyl or naphthyl is unsubstituted or substituted by one or more, e.g. up to three, substitutents selected from the group consisting of
   - a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂NV- wherein V is hydrogen or unsubstituted or substituted alkyl as defined below, specially selected from C₁-C₇-alkyl, phenyl, naphthyl, phenyl- or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
   - from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, halo-C₁-C₇-alkoxycarbonyl, phenyl- or naphthyl-C₁-C₇-alkoxycarbonyl, C₁-C₇-alkylsulfonyl, carbamoyl and cyano;
   or pyrrolylcarbonyl, furanylcarbonyl, thienylcarbonyl, pyrimidine-2,4-dione-1-, -2-, -3- or -5-yl-carbonyl, indolyl- carbonyl, benzimidazolyl-carbonyl, benzopyrazolyl-carbonyl benzofuranyl-carbonyl, quinolinyl-carbonyl or benzo[1,2,5]oxadiazolyl-carbonyl, each if which is unsubstituted or substituted by one or more, e.g. up to three, substituents independently selected from a substitutent of the formula -(C₀-C₇-alkylene)-(X)ᵣ(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, especially selected from C₁-C₇-alkyl, phenyl, naphthyl, phenyl- or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
   from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, phenyl- or naphthyl-C₁-C₇-alkoxycarbonyl, halo-C₁-C₇-alkoxycarbonyl, C₁-C₇-alkylsulfonyl, carbamoyl and cyano;
R² is
   C₁-C₇-alkyl that is unsubstituted or substituted by one or more, e.g. up to three, substitutents selected from the group consisting of halo, phenyl- or naphthyl, hydroxy, C₁-C₇-alkoxy, amino, mono- or di-(C₁-C₇-alkyl)-amino, C₁-C₇-alkanoylamino, C₁-C₇-alkyl-sulfonylamino, phenyl- or napthylsulfonylamino, phenyl- or naphthyl-C₁-C₇-alkylsulfonylamino, C₁-C₇alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl; and cyano, C₃-C₁₀-cycloalkyl which is unsubstituted or substituted by one or more, e.g. up to three, substituents independently selected from the substitutents mentioned above for substituted phenyl or naphthyl R¹, especially by C₁-C₇-alkyl, phenyl, naphthyl, phenyl-C₁-C₇-alkyl or naphthyl-C₁-C₇-alkyl or C₃-C₁₀-cycloalkyl-C₁-C₇-alkyl wherein cycloalkyl is unsubstituted or substituted by one or more, e.g. up to three, substituents independently selected from the substitutents mentioned above for substituted phenyl or naphthyl R¹, especially by C₁-C₇-alkyl, phenyl, naphthyl, phenyl-C₁-C₇-alkyl or naphthyl-C₁-C₇-alkyl;
R³ is phenyl, naphthyl, phenyl-C₁-C₇-alkyl or naphthyl-C₁-C₇alkyl, wherein each phenyl or naphthyl is unsubstituted or substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylylamino-C₁-C₇-alkyl, phenyl, naphthyl, mono- or di-(C₁-C₇-alkoxy)-phenyl or -naphthyl, C₁-C₇-alkylendioxy-phenyl where the oxy atoms are bound to adjacent phenyl ring atoms, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, (unsubstituted or mono-, di- or tri-C₁-C₇-alkyl substituted)-phenyl- or - naphthyl-C₁-C₇-alkyloxy, C₁-C₇-alkanoyloxy, nitro, amino, mono- or di-(C₁-C₇-alkyl)-amino, C₁-C₇-alkanoylamino C₁-C₇-alkylsulfonylylamino, carboxyl, C₁-C₇-alkoxy-carbonyl, phenyl- or naphthyl-C₁-C₇-alkoxycarbonyl, carbamoyl and cyano, C₁-C₇-alkylsulfonyl, C₁-C₇-alkylene which is unsubstituted or substituted by up to four C₁-C₇-alkyl substituents and bound to two adjacent ring atoms of the phenyl or naphthyl moiety; pyrrolyl, furanyl and thienyl;
R⁴ is hydroxy;
   L is methylene;
   and T is carbonyl, thiocarbonyl or preferably methylene;
   or a pharmaceutically acceptable salt thereof.

Another preferred embodiment of the invention, which is in fact a very highly preferred embodiment, relates to a compound of the formula I,
wherein
R¹ is phenyl or naphthyl, each of which is unsubstituted or substituted by one or more, e.g. up to three, substitutents selected from the group consisting of C₁-C₇-alkyl, phenyl, naphthyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylamino-C₁-C₇-alkyl, halo, hydroxy, C₁-C₇-alkoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl and/or (phenyl- or naphthyl)-C₁-C₇-alkyl)-carbamoyl, C₁-C₇-alkylsulfonyl, unsubstituted or C₁-C₇-alkyl-substituted phenyl- or naphthylsulfonyl, N-mono- or N,N-di-(C₁-C₇-alkyl and/or (phenyl- or naphthyl)-C₁-C₇-alkyl)-sulfamoyl and cyano;
   phenyl- or naphthyl-C₁-C₇-alkyl, wherein each of phenyl or naphthyl is unsubstituted or substituted by one or more, e.g. up to three, substitutents selected from the group consisting of the substitutents just mentioned for substituted phenyl or naphthyl, pyrrolyl, furanyl, thienyl, pyrimidine-2,4-dione-1-, -2-, -3- or -5-yl and benzo[1,3]dioxolyl, each if which is unsubstituted or substituted by one or more, e.g. up to three, substituents independently selected from those mentioned for substituted phenyl or naphthyl R¹ above, especially C₁-C₇-alkyl, halo-C₁-C₇-alkyl, hydroxy-C₁-C₇-alkoxy, C₁-C₇-alkoxy-C₁-C₇-alkyl and C₁-C₇-alkyloxy; pyrrolyl-C₁-C₇-alkyl, furanyl-C₁-C₇-alkyl, thienyl-C₁-C₇-alkyl, pyrimidine-2,4-dione-1-, -2-, -3- or -5-yl-C₁-C₇-alkyl, indolyl-C₁-C₇-alkyl, benzofuranyl-C₁-C₇-alkyl, benzimidazolyl-C₁-C₇-alkyl, benzopyrazolyl-C₁-C₇-alkyl, quinolinyl-C₁-C₇-alkyl, isoquinolyl-C₁-C₇-alkyl or benzo[1,2,5]oxadiazolyl-C₁-C₇-alkyl, each if which is unsubstituted or substituted by one or more, e.g. up to three, substituents independently selected from the substitutents mentioned above for substituted phenyl or naphthyl R¹, especially C₁-C₇-alkyl, halo-C₁-C₇-alkyl, hydroxy-C₁-C₇-alkoxy, C₁-C₇-alkoxy-C₁-C₇-alkyl and C₁-C₇-alkyloxy,
   C₃-C₁₀-cycloalkyl which is unsubstituted or substituted by one or more, e.g. up to three, substituents independently selected from the substitutents mentioned above for substituted phenyl or naphthyl R¹, especially by C₁-C₇-alkyl, phenyl, naphthyl, phenyl-C₁-C₇-alkyl or naphthyl-C₁-C₇alkyl;
   C₃-C₁₀-cycloalkyl-C₁-C₇-alkyl wherein cycloalkyl is unsubstituted or substituted by one or more, e.g. up to three, substituents independently selected from the substitutents mentioned above for substituted phenyl or naphthyl R¹, especially by C₁-C₇-alkyl, phenyl, naphthyl, phenyl-C₁-C₇-alkyl or naphthyl-C₁-C₇alkyl;
   phenyl- or naphthyl-carbonyl or phenyl- or naphthyl-C₁-C₇-alkylcarbonyl, wherein each phenyl or naphthyl is unsubstituted or substituted by one or more, e.g. up to three, substitutents selected from the group consisting of
   a substitutent of the formula -(C₀-C₇-alkylene)-(X)ᵣ(C₁-C₇-alkylene)-(Y)ₛ(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, especially selected from C₁-C₇-alkyl, phenyl, naphthyl, phenyl- or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamirto, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
   from phenyl- or naphthyl-C₁-C₇-alkyl, hato-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, halo-C₁-C₇-alkoxycarbonyl, phenyl- or naphthyl-C₁-C₇-alkoxycarbonyl, C₁-C₇-alkylsulfonyl, carbamoyl and cyano;
   pyrrolylcarbonyl, furanylcarbonyl, thienylcarbonyl, pyrimidine-2,4-dione-1-, -2-, -3- or -5-yl-carbonyl, indolyl- carbonyl, benzimidazolyl-carbonyl, benzopyrazolyl-carbonyl benzofuranyl-carbonyl, quinolinyl-carbonyl or benzo[1,2,5]oxadiazolyl-carbonyl, each if which is unsubstituted or substituted by one or more, e.g. up to three, substituents independently selected from a substitutent of the formula -(C₀-C₇-alkylene)-(X)ᵣ(C₁-C₇-alkylene)-(Y)ₛ-(C₀₋C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, especially selected from C₁-C₇-alkyl, phenyl, naphthyl, phenyl- or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
   from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, halo-C₁-C₇-alkoxycarbonyl, phenyl- or naphthyl-C₁-C₇-alkoxycarbonyl, C₁-C₇-alkylsulfonyl, carbamoyl and cyano;
   or phenyl- or naphthyl-sulfonyl, wherein each phenyl or naphthyl is unsubstituted or substituted by one or more, e.g. up to three, substitutents selected from those mentioned above for substituted phenyl or naphthyl R¹, preferably from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, halo, hydroxy, C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl and cyano;
R² is
   C₁-C₇-alkyl that is unsubstituted or substituted by one or more, e.g. up to three, substitutents selected from the group consisting of halo, phenyl- or naphthyl, hydroxy, C₁-C₇-alkoxy, amino, mono- or di-(C₁-C₇-alkyl)-amino, C₁-C₇-alkanoylamino, C₁-C₇-alkyl-sulfonylamino, phenyl- or napthylsulfonylamino, phenyl- or naphthyl-C₁-C₇-alkylsulfonylamino, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl; and cyano;
   phenyl or naphthyl, each of which is unsubstituted or substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkylsulfonylamino-C₁-C₇-alkyl, halo, hydroxy, C₁-C₇-alkoxy, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl and cyano;
   phenyl- or naphthyl-C₁-C₇-alkyl, wherein each of phenyl or naphthyl is unsubstituted or substituted by one or more, e.g. up to three, substitutents selected from the group just mentioned for substituted phenyl or naphthyl R²;
   C₃-C₁₀-cycloalkyl which is unsubstituted or substituted by one or more, e.g. up to three, substitutents selected from the group just mentioned for substituted phenyl or naphthyl R², especially by C₁-C₇-alkyl, phenyl, naphthyl, phenyl-C₁-C₇-alkyl or naphthyl-C₁-C₇alkyl; C₃-C₁₀-cycloalkyl-C₁-C₇-alkyl wherein cycloalkyl is unsubstituted or substituted by one or more, e.g. up to three, substitutents selected from the group just mentioned for substituted phenyl or naphthyl R², especially by C₁-C₇-alkyl, phenyl, naphthyl, phenyl-C₁-C₇-alkyl or naphthyl-C₁-C₇alkyl;
   or pyrrolyl, furanyl or thienyl,
   or, if L is methylene, oxy, thio or imino, R² is selected from one of the groups of moieties R² just mentioned and from hydrogen;
R³ is
   carbamoyl, N-mono- or N,N-di-(C₃-C₈-cycloalkyl-, C₁-C₇-alkyl-, phenyl-C₁-C₇-alkyl- and/or naphthyl-C₁-C₇alkyl-)aminocarbonyl-C₁-C₇-alkyl;
   phenyl, naphthyl, phenyl-C₁-C₇-alkyl or naphthyl-C₁-C₇alkyl, wherein each phenyl or naphthyl is unsubstituted or substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylylamino-C₁-C₇-alkyl, phenyl, naphthyl, mono- or di-(C₁-C₇-alkoxy)-phenyl or -naphthyl, C₁-C₇-alkylendioxy-phenyl where the oxy atoms are bound to adjacent phenyl ring atoms, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, (unsubstituted or mono-, di- or tri-C₁-C₇-alkyl substituted)-phenyl- or -naphthyl-C₁-C₇-alkyloxy, C₁-C₇-alkanoyloxy, nitro, amino, mono- or di-(C₁-C₇-alkyl)-amino, C₁-C₇-alkanoylamino C₁-C₇-alkylsulfonylylamino, carboxyl, C₁-C₇-alkoxy-carbonyl, phenyl- or naphthyl-C₁-C₇-alkoxycarbonyl, carbamoyl and cyano, C₁-C₇-alkylsulfonyl, C₁-C₇-alkylene which is unsubstituted or substituted by up to four C₁-C₇-alkyl substituents and bound to two adjacent ring atoms of the phenyl or naphthyl moiety; pyrrolyl, furanyl and thienyl;
   phenyl- or naphthyl-sulfonyl or phenyl-C₁-C₇-alkyl- or naphthyl-C₁-C₇-alkyl-sulfonyl, wherein each phenyl or naphthyl is unsubstituted or substituted by one or more, e.g. up to three, substitutents selected from the group just described for substituted phenyl- or naphthyl R³, C₃-C₁₀-cycloalkyl or C₃-C₁₀-cyclyoalkyl-C₁-C₇-alkyl, in both of which cycloalkyl is unsubstituted or substituted by one or more of the substitutents just mentioned for substituted phenyl or naphthyl R³, especially by C₁-C₇-alkyl, phenyl, naphthyl, phenyl-C₁-C₇-alkyl or naphthyl-C₁-C₇alkyl;
   or heterocyclyl or heterocyclyl-C₁-C₇-alkyl wherein heterocyclyl is selected from pyrrolyl, furanyl, thienyl, pyrimidine-2,4-dione-1-, -3- or -5-yl, indolyl, benzofuranyl, benzimidazolyl, benzopyrazolyl, quinolinyl, isoquinolinyl, methylene-dioxy-phenyl, ethylene-1,2-dioxy-phenyl or trimethylen-1,3-dioxyphenyl wherein the oxy groups are bound to adjacent ring atoms of the phenyl ring, where each of the heterocyclyl moieties is unsubstituted or substituted as mentioned above for substituted phenyl R³;
   or, if L is imino, oxy or thio, can alternatively be phenyl- or naphthylcarbonyl, C₁-C₇-alkoxycarbonyl (meaning C₁-C₇-alkyl-O-C(=O)-), phenyloxycarbonyl, naphthyloxycarbonyl, phenyl-C₁-C₇-alkyloxycarbonyl, naphthyl-C₁-C₇-alkyloxycarbonyl or N-mono- or N,N-di-(C₁-C₇-alkyl, phenyl, naphthyl, phenyl-C₁-C₇-alkyl and/or naphthyl-C₁-C₇-alkyl)-aminocarbonyl, where in each case the phenyl or naphthyl rings are unsubstituted or substituted as mentioned above for substituted phenyl or naphthyl R³;
R⁴ is hydrogen;
   L is methylene (-CH₂-), oxy (-O-) or imino (-NH-);
   and T is carbonyl, thiocarbonyl or preferably methylene;
   or a pharmaceutically acceptable salt thereof.

Another preferred embodiment relates to a compound of the formula I, wherein
R¹ is
   phenyl- or naphthyl-carbonyl or phenyl- or naphthyl-C₁-C₇-alkylcarbonyl, wherein each phenyl or naphthyl is unsubstituted or substituted by one or more, e.g. up to three, substitutents selected from the group consisting of a substitutent of the formula -(C₀-C₇-alkylene)-(X)₁-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, especially selected from C₁-C₇-alkyl, phenyl, naphthyl, phenyl- or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
   from phenyl- or naphthyl-C₁-C₇alkyl, halo-C₁-C₇alkyl, halo, hydroxy, phenyl- or naphthyloxy, phenyl- or naphthyl-C₁-C₇-alkyloxy, nitro, amino, amino-C₁-C₇-alkyl, carboxyl, halo-C₁-C₇-alkoxycarbonyl, phenyl- or naphthyl-C₁-C₇-alkoxycarbonyl, C₁-C₇-alkylsulfonyl, carbamoyl and cyano;
R² is
   C₁-C₇-alkyl that is unsubstituted or substituted by one or more, e.g. up to three, substitutents selected from the group consisting of halo, phenyl- or naphthyl, hydroxy, C₁-C₇-alkoxy, amino, mono- or di-(C₁-C₇-alkyl)-amino, C₁-C₇-alkanoylamino, C₁-C₇-alkyl-sulfonylamino, phenyl- or napthylsulfonylamino, phenyl- or naphthyl-C₁-C₇-alkylsulfonylamino, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl; and cyano;
R³ is
   phenyl or naphthyl, wherein each phenyl or naphthyl is unsubstituted or substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylylamino-C₁-C₇-alkyl, phenyl, naphthyl, mono- or di-(C₁-C₇-alkoxy)-phenyl or -naphthyl, C₁-C₇-alkylendioxy-phenyl where the oxy atoms are bound to adjacent phenyl ring atoms, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, (unsubstituted or mono-, di- or tri-C₁-C₇-alkyl substituted)-phenyl- or -naphthyl-C₁-C₇-alkyloxy, C₁-C₇-alkanoyloxy, nitro, amino, mono- or di-(C₁-C₇-alkyl)-amino, C₁-C₇-alkanoylamino C₁-C₇-alkylsulfonylylamino, carboxyl, C₁-C₇-alkoxy-carbonyl, phenyl- or naphthyl-C₁-C₇-alkoxycarbonyl, carbamoyl and cyano, C₁-C₇-alkylsulfonyl, C₁-C₇-alkylene which is unsubstituted or substituted by up to four C₁-C₇-alkyl substituents and bound to two adjacent ring atoms of the phenyl or naphthyl moiety; pyrrolyl, furanyl and thienyl;
R⁴ is hydrogen;
   L is a bond;
   and T is carbonyl, thiocarbonyl or preferably methylene;
   or a pharmaceutically acceptable salt thereof.

Yet another preferred embodiment of the invention relates to a compound of the formula I, wherein
R¹ is phenylmethyl or naphthylmethyl, where each phenyl or naphthyl is unsubstituted or substituted by one or more, e.g. up to three, substitutents selected from the group consisting of C₁-C₇-alkyl, phenyl, naphthyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylamino-C₁-C₇-alkyl, halo, hydroxy, C₁-C₇-alkoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl and/or (phenyl- or naphthyl)-C₁-C₇-alkyl)-carbamoyl, C₁-C₇-alkylsulfonyl, unsubstituted or C₁-C₇-alkyl-substituted phenyl- or naphthylsulfonyl, N-mono- or N,N-di-(C₁-C₇-alkyl and/or (phenyl- or naphthyl)-C₁-C₇-alkyl)-sulfamoyl and cyano;
R² is phenyl that is unsubstituted or substituted by one or more, e.g. up to three, substitutents selected from the group consisting of C₁-C₇-alkyl, phenyl, naphthyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkylsulfonylamino-C₁-C₇-alkyl, halo, hydroxy, C₁-C₇-alkoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl and/or (phenyl- or naphthyl)-C₁-C₇-alkyl)-carbamoyl, C₁-C₇-alkylsulfonyl, unsubstituted or C₁-C₇-alkyl-substituted phenyl- or naphthylsulfonyl, N-mono- or N,N-di-(C₁-C₇-alkyl and/or (phenyl- or naphthyl)-C₁-C₇-alkyl)-sulfamoyl and cyano;
R³ is phenyl, naphthyl, phenyl-C₁-C₇-alkyl or naphthyl-C₁-C₇-alkyl, wherein each phenyl or naphthyl is unsubstituted or substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylylamino-C₁-C₇-alkyl, phenyl, naphthyl, mono- or di-(C₁-C₇-alkoxy)-phenyl or -naphthyl, C₁-C₇-alkylendioxy-phenyl where the oxy atoms are bound to adjacent phenyl ring atoms, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, (unsubstituted or mono-, di- or tri-C₁-C₇-alkyl substituted)-phenyl- or - naphthyl-C₁-C₇-alkyloxy, C₁-C₇-alkanoyloxy, nitro, amino, mono- or di-(C₁-C₇-alkyl)-amino, C₁-C₇-alkanoylamino C₁-C₇-alkylsulfonylylamino, carboxyl, C₁-C₇-alkoxy-carbonyl, phenyl- or naphthyl-C₁-C₇-alkoxycarbonyl, carbamoyl and cyano, C₁-C₇-alkylsulfonyl, C₁-C₇-alkylene which is unsubstituted or substituted by up to four C₁-C₇-alkyl substituents and bound to two adjacent ring atoms of the phenyl or naphthyl moiety; pyrrolyl, furanyl and thienyl;
   phenyl- or naphthyl-sulfonyl or phenyl-C₁-C₇-alkyl- or naphthyl-C₁-C₇-alkyl-sulfonyl, wherein each phenyl or naphthyl is unsubstituted or substituted by one or more, e.g. up to three, substitutents selected from the group just described for substituted phenyl- or naphthyl R³, C₃-C₁₀-cycloalkyl or C₃-C₁₀-cyclyoalkyl-C₁-C₇-alkyl, in both of which cycloalkyl is unsubstituted or substituted by one or more of the substitutents just mentioned for substituted phenyl or naphthyl R³, especially by C₁-C₇-alkyl, phenyl, naphthyl, phenyl-C₁-C₇-alkyl or naphthyl-C₁-C₇alkyl;
   or heterocyclyl or heterocyclyl-C₁-C₇-alkyl wherein heterocyclyl is selected from pyrrolyl, furanyl, thienyl, pyrimidine-2,4-dione-1-, -3- or -5-yl, indolyl, benzofuranyl, benzimidazolyl, benzopyrazolyl, quinolinyl, isoquinolinyl, methylene-dioxy-phenyl, ethylene-1,2-dioxy-phenyl or trimethylen-1,3-dioxyphenyl wherein the oxy groups are bound to adjacent ring atoms of the phenyl ring, where each of the heterocyclyl moieties is unsubstituted or substituted as mentioned above for substituted phenyl R³;
R⁴ is hydrogen;
   L is methylene;
   and T is methylene;
   or a pharmaceutically acceptable salt thereof.

A preferred embodiment of the invention relates to a compound of the formula I,
wherein
R¹ is unsubstituted or substituted aryl, unsubstituted or substituted aryl-alkyl, or acyl;
R² is unsubstituted or substituted alkyl, unsubstituted or substituted aryl, unsubstituted or substituted cycloalkyl, unsubstituted or substituted aryl-alkyl, unsubstituted or substituted mono- or bicyclic heterocyclyl-alkyl, unsubstituted or substituted cycloalkyl-alkyl, with the proviso that if L is methylene (-CH₂-), oxy (-O-), thio (-S-) or unsubstituted (-NH-)or substituted imino, R² is selected from one of the mentioned groups and from hydrogen; R³ is unsubstituted or substituted alkyl, substituted or unsubstituted aryl, unsubstituted or substituted heterocyclyl, unsubstituted or substituted cycloalkyl, unsubstituted or substituted aryl-alkyl, unsubstituted or substituted heterocyclyl-alkyl, unsubstituted or substituted cycloalkyl-alkyl or, if L is oxy or unsubstituted or substituted imino, has one of the meanings just mentioned or is unsubstituted or substituted alkylcarbonyl, unsubstituted or substituted arylcarbonyl, unsubstituted or substituted heterocyclylcarbonyl, unsubstituted or substituted cycloalkylcarbonyl, unsubstituted or substituted aryl-alkylcarbonyl, unsubstituted or substituted heterocyclyl-alkyl carbonyl, unsubstituted or substituted cycloalkyl-alkyl carbonyl, etherified carboxy, carbamoyl or N-mono- or N,N-di-substituted amino-carbonyl; substituted or unsubstituted alkylsulfonyl, substituted or unsubstituted arylsulfonyl; substituted or unsubstituted aryl-alkyl sulfonyl N-mono- or N,N-di-substituted amino-sulfonyl;
R⁴ is hydrogen or hydroxy; with the proviso that when R³ is hydrogen, then R⁴ is hydroxyl;
L is a bond, methylene (-CH₂-), oxy (-O-), or unsubstituted (-NH-)or substituted imino, with the proviso that if L is a bond then R³ is one of the moieties mentioned for R³ other than substituted alkyl;
   or R³ and R⁴ which then is -O- together with L which then is methylene and the carbon to which R³-L- and R⁴ are bound form a substituted or unsubstituted ring annealed to an unsubstituted or substituted aryl, thus forming a spiro compound of the formula I, or R³ and R⁴ together with L form oxo (=O);
   and
   T is methylene;
   or a salt thereof.

### Preferred definitions for R¹

R¹ is as defined in the claims, preferably in a first embodiment R¹ is unsubstituted or substituted aryl such as phenyl or naphthyl, preferably phenyl, or unsubstituted or substituted aryl-alkyl, such as phenyl-C₁-C₄-alkyl or naphthyl-C₁-C₄-alkyl, preferably phenyl-C₁-C₄-alkyl, such as benzyl. When the aryl moiety is substituted, it is preferably mono- or di-substituted. Suitable substituents are as defined herein, preferably C₁-C₇-alkyl, -O-C₁-C₇-alkyl, halo-C₁-C₇-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano.

When R¹ has this definition, then one or more, preferably all of the following substituents have the following definition:
T is methylene,
L is CH₂ or O, preferably CH₂,
R³ is aryl, such as phenyl, or aryl-alkyl, such as phenyl-C₁-C₄-alkyl, which are each unsubstituted or substituted by a suitable substituent such as C₁-C₇-alkyl, -O-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, and cyano; preferably each are unsubstituted, and/or
R⁴ is hydrogen.

Preferably in a second embodiment R¹ is acyl. Acyl is preferably unsubstituted or substituted aryl-carbonyl or -sulfonyl, unsubstituted or substituted heterocyclylcarbonyl, unsubstituted or substituted cycloalkylcarbonyl, or unsubstituted or substituted alkylcarbonyl or -sulfonyl, wherein unsubstituted or substituted aryl, unsubstituted or substituted heterocyclyl, unsubstituted or substituted alkyl and unsubstituted or substituted cycloalkyl are preferably as defined herein.
Preferred examples for the aryl moiety of the acyl substituent are phenyl and naphthyl. When the aryl moiety is substituted, it is preferably mono- or di-substituted. Naphthyl is preferably mono-substituted and phenyl is preferably mono- or di-substituted, more preferably di-substituted. Suitable substituents for the aryl moiety are as defined herein, preferably -(C₀-C₇-alkylene)-(X)ᵣ-(C₁-C₇-alkylene)-(Y)ₛ-(C₀-C₇-alkylene)-H, wherein r and s are 0 or 1 and Y and X are independently O , NH or NH-CO-O-, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, and cyano. Preferred examples of -(C₀-C₇-alkylene)-(X)ᵣ-(C₁-C₇-alkylene)-(Y)ₛ-(C₀-C₇-alkylene)-H include -(O or NH)-C₁-C₇-alkyl, -C₁-C₇-alkyl, -(O or NH)-C₁-C₇-alkylene-(O or NH)-C₁-C₇-alkyl, -(O or NH)-C₁-C₇-alkylene-(O or NH)-H, -C₁-C₇-alkylene-(O or NH)-C₁-C₇-alkylene-(O or NH)-C₁-C₇-alkyl, -C₁-C₇-alkylene-(O or NH)-C₁-C₇-alkyl, or -C₁-C₇-alkylene-NH-CO-O-C₁-C₇-alkyl, most preferably -OMe, -OC₃H₆OMe, -NH-butyl, methyl, ethyl, -C₂H₄-NH-CO-OMe, -CH₂OC₂H₄OMe, -OC₂H₄OC₂H₄, -OC₃H₆OH, -C₂H₄OMe, -C₃H₆OMe and -NH-C₃H₆OMe. Most preferably the aryl moiety is unsubstituted or substituted with OMe and/or - OC₃H₆OMe.

Preferred examples for the heterocyclyl moiety of the acyl substituent are mono- or bicyclic rings. Preferred are aromatic ring systems, or in particular if a bicyclic moiety is contemplated, partially saturated ring systems, in particular whereby one of the rings is aromatic and the other is saturated. The heterocyclyl moiety has preferably 1, 2 or 3, more preferably 1 or 2 heteroatoms selected from O, N or S, more preferably O or N. Particularly preferred examples include pyrrolylcarbonyl, furanylcarbonyl, thienylcarbonyl, pyridylcarbonyl, pyrimidine-2,4-dione-1-, -2-, -3- or -5-yl-carbonyl, indolyl-carbonyl, benzimidazolyl-carbonyl, benzopyrazolyl-carbonyl, benzofuranyl-carbonyl, quinolinyl-carbonyl, benzo[1,2,5]oxadiazolyl-carbonyl, and 3,4-dihydro-2H-benzo[1,4]oxazinyl carbonyl, more preferably pyridylcarbonyl, indolyl-carbonyl, benzimidazolyl-carbonyl, benzofuranyl-carbonyl, quinolinyl-carbonyl, and 3,4-dihydro-2H-benzo[1,4]oxazinyl carbonyl. When the heterocyclyl moiety is substituted, it is preferably mono-substituted. Suitable substituents for the heterocyclyl moiety are as defined herein, preferably -(C₀-C₇-alkylene)-(X)ᵣ-(C₁-C₇-alkylene)-(Y)ₛ-(C₀-C₇-alkylene)-H, wherein r and s are 0 or 1 and Y and X are independently O , NH or NH-CO-O-, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, and cyano. Preferred examples of -(C₀-C₇-alkylene)-(X)ᵣ-(C₁-C₇-alkylene)-(Y)ₛ-(C₀-C₇-alkylene)-H include -(O or NH)-C₁-C₇-alkyl, -C₁-C₇-alkyl, -(O or NH)-C₁-C₇-alkylene-(O or NH)-C₁-C₇-alkyl, -(O or NH)-C₁-C₇-alkylene-(O or NH)-H, -C₁-C₇-alkylene-(O or NH)-C₁-C₇-alkylene-(O or NH)-C₁-C₇-alkyl, -C₁-C₇-alkylene-(O or NH)-C₁-C₇-alkyl, or -C₁-C₇-alkylene-NH-CO-O-C₁-C₇-alkyl, more preferably -OMe, -OC₂H₄OMe, -NH-butyl, methyl, ethyl, - C₂H₄-NH-CO-OMe, - CH₂OC₂H₄OMe, -OC₂H₄OC₂H₄, -OC₃H₆OH, -C₂H₄OMe, -C₃H₆OMe and -NH-C₃H₆OMe, yet more preferably -NH-propyl, -C₂H₄OMe and -C₃H₆OMe. Most preferably the heterocyclyl moiety is unsubstituted or substituted -NH-butyl, Me, -C₂H₄OMe or -C₃H₆OMe.

Preferred examples for the cycloalkyl moiety of the acyl substituent are monocyclic rings, preferably C₃-C₇-cycloalkyl, more preferably C₃, C₄, C₅and C₆-cycloalkyl, most preferably cyclopropyl. The cycloalkyl moiety may be substituted or unsubstituted. When the cycloalkyl moiety is substituted, it is preferably mono-substituted. Suitable substituents for the cycloalkyl moiety are as defined herein, preferably O-C₁-C₄-alkyl, halo, hydroxy, unsubstituted or substituted, preferably unsubstituted, phenyl, unsubstituted or substitute, preferably unsubstituted, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano, most preferably phenyl or naphthyl.

Preferred examples for the alkyl moiety of the acyl substituent is branched or straight chain C₁-C₇-alkyl which may be substituted or unsubstituted. When the alkyl moiety is substituted, it is preferably mono-substituted. Suitable substituents for the alkyl moiety are as defined herein, preferably O-C₁-C₄-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano. Preferably the alkyl moiety is substituted.

### Preferred definitions for R²

R² is as defined in the claims, preferably in a first embodiment R² is unsubstituted or substituted aryl such as phenyl or naphthyl, preferably phenyl. When the aryl moiety is substituted, it is preferably mono- or di-substituted. Suitable substituents are as defined herein, preferably C₁-C₇-alkyl, -O-C₁-C₇-alkyl, halo-C₁-C₇-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano.

Preferably in a second embodiment R² is unsubstituted or substituted aryl-alkyl, such as phenyl-C₁-C₄-alkyl or naphthyl-C₁-C₄-alkyl, preferably phenyl-C₁-C₄-alkyl, such as benzyl, phenethyl, phenyl-CH₂CH₂CH₂, phenyl-CH₂CH₂CH₂CH₂, phenyl-CH(CH₃), naphthyl-CH₂, most preferably benzyl or naphthyl-CH₂. When the aryl moiety is substituted, it is preferably mono- or di-substituted. Suitable substituents are as defined herein, preferably -(C₀-C₇-alkylene)-(X)ᵣ-(C₁-C₇-alkylene)-(Y)ₛ-(C₀-C₇-alkylene)-H, wherein r and s are 0 or 1 and Y and X are independently O , NH or -NH-CO-O-, -CO-NH-, NHCO, N(C₁-C₇-alkyl), halo-C₁-C₇-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, N(mono or di- CO-C₁-C₇-alkyl or formyl) amino, amino-C₁-C₇-alkyl, carboxyl, and cyano. Preferred examples of -(C₀-C₇-alkylene)-(X)ᵣ-(C₁-C₇-alkylene)-(Y)ₛ-(C₀-C₇-alkylene)-H include -(O or NH)-C₁-C₇-alkyl, -CO-NH₂, -C₁-C₇-alkyl, -NHCO-C₁-C₇-alkyl, - (O or NH)-C₁-C₇-alkylene-(O or NH)-C₁-C₇-alkyl, -(O or NH)-C₁-C₇-alkylene-(O or NH)-H, -C₁-C₇-alkylene-(O or NH)-C₁-C₇-alkylene-(O or NH)-C₁-C₇-alkyl, -C₁-C₇-alkylene-(O, NH-CO-O, NHCO or NH)-C₁-C₇-alkyl, -C₁-C₇-alkylene-(O, NHCO or NH)-H, -C₁-C₇-alkylene-N(C₁-C₇-alkyl)-C₁-C₇-alkyl, -C₁-C₇-alkylene-(O or NH)-C₁-C₇-alkylene-(O or NH)-H or -C₁-C₇-alkylene-NH-CO-O-C₁-C₇-alkyl, most preferably -OMe, -CH₂NH₂, -CONH₂, -CH₂N(Me)₂, - CH₂NHCOMe, -CH₂NHCO-H, -CH₂NHC₂H₄OH, NHCOMe, -OC₂H₄OMe, NHCOMe, or - OC₃H₆OMe. Most preferably the aryl moiety is unsubstituted or substituted with halo, OMe and/or CN.

When R² is aryl or aryl-alkyl, then one or more, preferably all of the following substituents have the following definition:
T is methylene,
L is CH₂ or O, preferably CH₂,
R³ is aryl such as phenyl or aryl-alkyl, such as phenyl-C₁-C₄-alkyl, which are each unsubstituted or substituted by a suitable substituent such as C₁-C₇-alkyl, -O-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, and cyano; preferably each are unsubstituted, and/or
R⁴ is hydrogen.

Preferably in a third embodiment R² is cycloalkyl or cycloalkyl alkyl such as cycloalkyl-C₁₋₄ alkyl-, in particular cycloalkyl-CH₂- Preferred examples for the cycloalkyl moiety are in each case monocyclic rings, preferably C₃-C₇-cycloalkyl, more preferably C₃, C₄, C₅ and C₆-cycloalkyl. The cycloalkyl moiety may be substituted or unsubstituted. When the cycloalkyl moiety is substituted, it is preferably mono-substituted. Suitable substituents for the cycloalkyl moiety are as defined herein, preferably O-C₁-C₄-alkyl, halo, hydroxy, unsubstituted or substituted phenyl, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano, most preferably phenyl or naphthyl. Most preferably, the cycloalkyl moiety is unsubstituted.

Preferably in a third embodiment R² is unsubstituted or substituted heterocyclyl-alkyl. Preferred examples for the heterocyclyl moiety are mono- or bicyclic rings. Preferred are aromatic ring systems, or in particular if a bicyclic moiety is contemplated, partially saturated ring systems, in particular whereby one of the rings is aromatic and the other is saturated or partially saturated. The heterocyclyl moiety has preferably 1, 2 or 3, more preferably 1 or 2 heteroatoms selected from O, N or S, more preferably O or N. Particularly preferred examples include pyrrolyl, furanyl, thienyl, pyridyl, pyrimidine-2,4-dione-1-, -2-, -3- or -5-yl, indolyl, benzimidazolyl, benzopyrazolyl, benzofuranyl, quinolinyl, benzo[1,2,5]oxadiazolyl, and 3,4-dihydro-2H-benzo[1,4]oxazinyl, more preferably thienyl, pyrimidine-2,4-dione-1-, -2-, -3- or -5-yl, and benzo[1,2,5]oxadiazolyl. When the heterocyclyl moiety is substituted, it is preferably mono-substituted. Suitable substituents for the heterocyclyl moiety are as defined herein, preferably -(C₀-C₇-alkylene)-(X)ᵣ-(C₁-C₇-alkylene)-(Y)ₛ-(C₀-C₇-alkylene)-H, wherein r and s are 0 or 1 and Y and X are independently O , NH or NH-CO-O-, halo-C₁-C₇-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano. Preferred examples of -(C₀-C₇-alkylene)-(X)ᵣ-(C₁-C₇-alkylene)-(Y)ₛ-(C₀-C₇-alkylene)-H include -(O or NH)-C₁-C₇-alkyl, -C₁-C₇-alkyl, -(O or NH)-C₁-C₇-alkylene-(O or NH)-C₁-C₇-alkyl, -(O or NH)-C₁-C₇-alkylene-(O or NH)-H, -C₁-C₇-alkylene-(O or NH)-C₁-C₇-alkylene-(O or NH)-C₁-C₇-alkyl, -C₁-C₇-alkylene-(O or NH)-C₁-C₇-alkyl, or -C₁-C₇-alkylene-NH-CO-O-C₁-C₇-alkyl, more preferably -OMe, - OC₂H₄OMe, -NH-propyl, methyl, ethyl, -C₂H₄-NH-CO-OMe, -CH₂OC₂H₄OMe, -OC₂H₄OC₂H₄, - OC₃H₆OH, -C₂H₄OMe, -C₃H₆OMe and -NH-C₃H₆OMe, yet more preferably -NH-propyl, - C₂H₄OMe and -C₃H₆OMe. Most preferably the heterocyclyl moiety is unsubstituted.

When R² is cycloalkyl, cycloalkyl alkyl or heterocyclyl-alkyl, then one or more, preferably all of the following substituents have the following definition:
R¹ is acyl or aryl, preferably aryl carbonyl,
T is methylene,
L is CH₂ or O, preferably CH₂,
R³ is aryl such as phenyl or aryl-alkyl, such as phenyl-C₁-C₄-alkyl, which are each unsubstituted or substituted by a suitable substituent such as C₁-C₇-alkyl, -O-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, and cyano; preferably each are unsubstituted, most preferably R³ is phenyl,and/or
R⁴ is hydrogen.

Preferably in a fourth embodiment R² is unsubstituted or substituted alkyl. Preferred examples for the alkyl moiety of the acyl substituent is branched or straight chain C₁-C₇-alkyl which may be substituted or unsubstituted. In one embodiment, R² is branched alkyl such as isopropyl, isobutyl, sec-butyl or tert-butyl, isopentyl, 1-ethylpropyl, and 1,2-dimethyl-propyl, most preferably isopropyl. Branched alkyl is preferably unsubstituted. In another embodiment R² is straight chain alkyl such as methyl, ethyl, n-propyl, n-butyl or n-pentyl, preferably methyl, ethyl or n-propyl. Straight chain alkyl is preferably substituted. When the alkyl moiety is substituted, it is preferably mono-, di- or tri-substituted, more preferably mono-substituted. Suitable substituents for the alkyl moiety are as defined herein, preferably O-C₁-C₄-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano.

In one embodiment, both R¹ and R² are unsubstituted or substituted aryl as defined above. In one embodiment, both R¹ and R² are unsubstituted or substituted aryl alkyl as defined above. In another embodiment, R¹ is unsubstituted or substituted aryl as defined above and R² is unsubstituted or substituted aryl alkyl as defined above. In another embodiment, R¹ is unsubstituted or substituted acyl as defined above and R² is unsubstituted or substituted aryl as defined above. In another embodiment, R¹ is unsubstituted or substituted acyl as defined above and R² is unsubstituted or substituted alkyl as defined above. In another embodiment, R¹ is unsubstituted or substituted acyl as defined above and R² is unsubstituted or substituted cycloalkyl as defined above. In another embodiment, R¹ is unsubstituted or substituted acyl as defined above and R² is unsubstituted or substituted heterocyclyl alkyl as defined above. Most preferably, R¹ is unsubstituted or substituted acyl as defined above, preferably substituted aryl carbonyl and R² is unsubstituted or substituted alkyl as defined above, preferably branched alkyl.

### Preferred definitions for T

T is as defined in the claims, preferably in a first embodiment T is methylene. Preferably, in a second embodiment T is carbonyl. Most preferably, T is methylene.

### Preferred definitions for L and R³ and R⁴

L is as defined in the claims, preferably in a first embodiment L is methylene.
In this embodiment R³ has preferably one of the following definitions (a) or (b):
(a) R³ is preferably unsubstituted or substituted aryl as defined below, more preferably unsubstituted aryl, such as phenyl or naphthyl, more preferably phenyl.
(b) R³ is preferably substituted alkyl. Preferred examples for alkyl are branched or straight chain C₁-C₇-alkyl which may be substituted or unsubstituted. Preferred examples include methyl, ethyl, isopropyl, n-propyl, n-butyl, sec-butyl or tert-butyl, more preferably methyl, ethyl or isopropyl, most preferably methyl. The alkyl moiety is is preferably mono-, di- or tri-substituted, more preferably mono-substituted. Suitable substituents for the alkyl moiety are as defined herein, preferably O-C₁-C₄-alkyl, halo, hydroxy, unsubstituted or substituted, preferably unsubstituted, phenyl, unsubstituted or substituted, preferably unsubstituted, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, unsubstituted or substituted, preferably unsubstituted, cycloalkyl, nitro, amino, amino-C₁-C₇-alkyl, N-mono- or N,N-di-substituted aminocarbonyl, carboxyl, and cyano. A preferred example is N-mono- or N,N-di-substituted aminocarbonyl which will be described in more detail below with respect to preferred substituents:
   (i) Aminocarbonyl is preferably N-substituted by cycloalkyl. Preferred examples for the cycloalkyl moiety are monocyclic rings, preferably C₃-C₇-cycloalkyl, more preferably C₃, C₄, C₅ and C₆-cycloalkyl, in particular C₃-cycloalkyl. The cycloalkyl moiety may be substituted or unsubstituted. When the cycloalkyl moiety is substituted, it is preferably mono-substituted. Suitable substituents for the cycloalkyl moiety are as defined herein, preferably O-C₁-C₄-alkyl, halo, hydroxy, unsubstituted or substituted phenyl, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano, most preferably phenyl or naphthyl. Most preferably, the cycloalkyl moiety is unsubstituted.
   (ii) Aminocarbonyl is preferably N-substituted by unsubstituted alkyl. Preferred examples for the alkyl moiety are straight chain or branched alkyl, preferably straight chain alkyl, more preferably methyl or ethyl.
   (iii) Aminocarbonyl is preferably N-substituted by substituted alkyl. Preferred examples for the alkyl moiety are as defined for the unsubstituted alkyl moiety under item (ii). Particularly preferred is methyl.. The alkyl moiety is typically mono- or di-substituted, preferably mono-substituted. Suitable substituents for the cycloalkyl moiety are as defined herein, preferably O-C₁-C₄-alkyl, halo, hydroxy, unsubstituted or substituted, preferably unsubstituted, phenyl, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, unsubstituted or substituted, preferably unsubstituted, C₃-C₇-cycloalkyl, such as C₃, C₄, C₅ and C₆-cycloalkyl, in particular C₆ or C₃-cycloalkyl; substituted, preferably unsubstituted, heterocyclyl, such as five-or six-membered rings, preferably fully saturated, preferably containing one heteroatom selected from O or N, such as tetrahydropyranyl or piperidinyl; nitro, amino, amino-C₁-C₇-alkyl, carboxyl, and cyano, most preferably phenyl, heterocyclyl or cycloalkyl.
   (iv) Aminocarbonyl is preferably N-substituted by heterocyclyl. Preferred examples for the heterocyclyl moiety are mono- or bicyclic rings, more preferably mnocyclic rings such as 5- or 6-membered rings. Preferred are saturated ring systems or aromatic ring systems, in particular saturated ring systems. The heterocyclyl moiety has preferably 1, 2 or 3, more preferably 1 or 2 heteroatoms selected from O, N or S, more preferably O or N. Particularly preferred examples include 5- or 6-membered rings preferably containing an oxygen atom, in particular tetrahydropyranyl or tetrahydrofuranyl. When the heterocyclyl moiety is substituted, it is preferably mono-substituted. Suitable substituents for the heterocyclyl moiety are as defined herein, preferably halo, hydroxy, nitro, amino, amino-C₁-C₇-alkyl, carboxyl, and cyano, more preferably phenyl-C₁-C₇-alkyl,. Suitable phenyl substituents include C₁-C₇-alkyl, -O-C₁-C₇-alkyl, halo-C₁-C₇-alkyl, halo, hydroxyl and amino. Most preferably the heterocyclyl moiety is unsubstituted.
   (v) Aminocarbonyl is preferably N-substituted by aryl. Preferred examples of aryl include phenyl or naphthyl, more preferably phenyl. When the aryl moiety is substituted, it is preferably mono- or di-substituted. In particular, phenyl is preferably mono-substituted. Most preferably aryl is unsubstituted. Suitable substituents are as defined herein, preferably C₁-C₇-alkyl, -O-C₁-C₇-alkyl, halo-C₁-C₇-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano.

The above substituents apply to both the N-mono-substituted and the N-di-substituted aminocarbonyl. Preferably in the case of-di-substituted aminocarbonyl, the first substituent is selected from one of the above and the other is preferably C₁-C₄-alkyl, such as methyl, ethyl, isopropyl or cyclopropyl.

Alternatively, N-di-substituted aminocarbonyl can be a ring formed by the N and the two substituents such as a pyrrolidine or piperidine ring.

According to the first embodiment R⁴ is preferably hydrogen or OH, more preferably hydrogen.

Preferably in a second embodiment L is O.

Preferably in this embodiment, R³ is one of the following (a) to (f):
(a) Preferably R³ is unsubstituted or substituted aryl.
   Preferred examples of aryl include phenyl or naphthyl, more preferably phenyl. When the aryl moiety is substituted, it is preferably mono- or di-substituted. In particular, phenyl is preferably mono-substituted. Most preferably aryl is mono-substituted. Suitable substituents are as defined herein, preferably C₁-C₇-alkyl, -O-C₁-C₇-alkyl, halo-C₁-C₇-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano, most preferably haloalkyl such as CF₃.
(b) Preferably R³ is unsubstituted or substituted aryl alkyl.
   Preferred examples of the alkyl moiety include C₁-C₄-alkyl, in particular CH₂.
   Preferred examples of the aryl moiety include phenyl, naphthyl or 1,2,3,4-tetrahydronaphthyl, more preferably phenyl. When the aryl moiety is substituted, it is preferably mono- or di-substituted. In particular, phenyl is preferably unsubstituted, mono- or di-substituted. Naphthyl is preferably unsubstituted or mono-substituted. 1,2,3,4-Tetrahydronaphthyl is preferably tetra-substituted, in particular by alkyl. Suitable substituents are as defined herein, preferably C₁-C₇-alkyl, -O-C₁-C₇-alkyl, halo-C₁-C₇-alkyl, halo-Cl-C7-alkyl-O-, halo, hydroxy, unsubstituted or substituted, preferably substituted phenyl, unsubstituted or substituted, preferably unsubstituted, naphthyl, unsubstituted or substituted, preferably substituted, phenyl- or naphthyloxy, unsubstituted or substituted, preferably unsubstituted, phenyl- or naphthyl-C₁-C₇-alkyloxy, unsubstituted or substituted, preferably unsubstituted, heterocyclyl, nitro, amino, amino-C₁-C₇-alkyl, carboxyl, and cyano. In this context, if phenyl, naphthyl, phenyl- or naphthyloxy, phenyl- or naphthyl-C₁-C₇-alkyloxy, heterocyclyl are substituted, they are preferably mono-or di-substituted. Preferred substituents include C₁-C₇-alkyl, -O-C₁-C₇-alkyl, halo-C₁-C₇-alkyl, halo, hydroxy, amino, amino-C₁-C₇-alkyl, acylamino, heterocyclyl, such as aromatic heterocyclyl, in particular pyrrolyl and benzo[1,3]dioxole, and cyano.
(c) Preferably R³ is unsubstituted or substituted heterocyclyl-alkyl.
   Preferred examples of the alkyl moiety include C₁-C₄-alkyl, in particular CH₂.
   Preferred examples for the heterocyclyl moiety are mono- or bicyclic rings. Preferred are aromatic ring systems, or in particular if a bicyclic moiety is contemplated, partially saturated ring systems, in particular whereby one of the rings is aromatic and the other is saturated or partially saturated. The heterocyclyl moiety has preferably 1, 2 or 3, more preferably 1 or 2 heteroatoms selected from O, N or S, more preferably O or N. Particularly preferred examples include 5-membered rings preferably containing a nitrogen atom, in particular oxadiazolyl, oxazolyl, isoxazolyl or pyrrolyl; or bicyclic ring systems preferably containing an oxygen atom, in particular 2,3-dihydro-benzo[1,4]dioxinyl, 3,4-dihydro-2H-benzo[b][1,4]dioxepinyl, benzofuranyl, benzo[1,2,5]oxadiazolyl or 3,4-dihydro-2H-benzo[1,4]oxazinyl, more preferably oxadiazolyl, oxazolyl, isoxazolyl, 2,3-dihydro-benzo[1,4]dioxinyl, 3,4-dihydro-2H-benzo[b][1,4]dioxepinyl, or benzofuranyl. When the heterocyclyl moiety is substituted, it is preferably mono-substituted. Suitable substituents for the heterocyclyl moiety are as defined herein, preferably halo, hydroxy, unsubstituted or substituted phenyl, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano, more preferably unsubstituted or mono-substituted phenyl. Suitable phenyl substituents include C₁-C₇-alkyl, - O-C₁-C₇-alkyl, halo-C₁-C₇-alkyl, halo, hydroxyl and amino. Most preferably the heterocyclyl moiety is unsubstituted.
(d) Preferably R³ is unsubstituted or substituted alkyl.
   Preferred examples for alkyl are branched or straight chain C₁-C₇-alkyl which may be substituted or unsubstituted. Preferred examples include methyl, ethyl, isopropyl, n-propyl, n-butyl, sec-butyl or tert-butyl, more preferably methyl, ethyl or isopropyl, most preferably methyl. The alkyl moiety is preferably substituted. When the alkyl moiety is substituted, it is preferably mono-, di- or tri-substituted, more preferably mono-substituted. Suitable substituents for the alkyl moiety are as defined herein, preferably O-C₁-C₄-alkyl, halo, hydroxy, unsubstituted or substituted, preferably unsubstituted, phenyl, unsubstituted or substituted, preferably unsubstituted, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, unsubstituted or substituted, preferably unsubstituted, cycloalkyl, nitro, amino, amino-C₁-C₇-alkyl, N-mono- or N,N-di-substituted aminocarbonyl, carboxyl, and cyano. A preferred example is N-mono- or N,N-di-substituted aminocarbonyl which will be described in more detail below with respect to preferred substituents:
   (i) Aminocarbonyl is preferably N-substituted by cycloalkyl. Preferred examples for the cycloalkyl moiety are monocyclic rings, preferably C₃-C₇-cycloalkyl, more preferably C₃, C₄, C₅ and C₆-cycloalkyl, in particular C₃-cycloalkyl. The cycloalkyl moiety may be substituted or unsubstituted. When the cycloalkyl moiety is substituted, it is preferably mono-substituted. Suitable substituents for the cycloalkyl moiety are as defined herein, preferably O-C₁-C₄-alkyl, halo, hydroxy, unsubstituted or substituted phenyl, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano, most preferably phenyl or naphthyl. Most preferably, the cycloalkyl moiety is unsubstituted.
   (ii) Aminocarbonyl is preferably N-substituted by unsubstituted alkyl. Preferred examples for the alkyl moiety are straight chain or branched alkyl, preferably straight chain alkyl, more preferably methyl or ethyl.
   (iii) Aminocarbonyl is preferably N-substituted by substituted alkyl. Preferred examples for the alkyl moiety are as defined for the unsubstituted alkyl moiety under item (ii). Particularly preferred is methyl.. The alkyl moiety is typically mono- or di-substituted, preferably mono-substituted. Suitable substituents for the cycloalkyl moiety are as defined herein, preferably O-C₁-C₄-alkyl, halo, hydroxy, unsubstituted or substituted, preferably unsubstituted, phenyl, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, unsubstituted or substituted, preferably unsubstituted, C₃-C₇-cycloalkyl, such as C₃, C₄, C₅ and C₆-cycloalkyl, in particular C₆ or C₃-cycloalkyl; substituted, preferably unsubstituted, heterocyclyl, such as five-or six-membered rings, preferably fully saturated, preferably containing one heteroatom selected from O or N, such as tetrahydropyranyl or piperidinyl; nitro, unsubstituted or substituted, preferably unsubstituted, amino, unsubstituted or substituted, preferably unsubstituted, amino-C₁-C₇-alkyl, whereby the amino moiety can be substituted by -C₁-C₇-alkyl, cycloalkyl, phenyl or heterocyclyl; carboxyl, and cyano, most preferably phenyl, heterocyclyl or cycloalkyl.
   (iv) Aminocarbonyl is preferably N-substituted by heterocyclyl. Preferred examples for the heterocyclyl moiety are mono- or bicyclic rings, more preferably mnocyclic rings such as 5- or 6-membered rings. Preferred are saturated ring systems or aromatic ring systems, in particular saturated ring systems. The heterocyclyl moiety has preferably 1, 2 or 3, more preferably 1 or 2 heteroatoms selected from O, N or S, more preferably O or N. Particularly preferred examples include 5- or 6-membered rings preferably containing an oxygen atom, in particular tetrahydropyranyl or tetrahydrofuranyl. When the heterocyclyl moiety is substituted, it is preferably mono-substituted. Suitable substituents for the heterocyclyl moiety are as defined herein, preferably halo, hydroxy, nitro, amino, amino-C₁-C₇-alkyl, carboxyl, and cyano, more preferably phenyl-C₁-C₇-alkyl,. Suitable phenyl substituents include C₁-C₇-alkyl, -O-C₁-C₇-alkyl, halo-C₁-C₇-alkyl, halo, hydroxyl and amino. Most preferably the heterocyclyl moiety is unsubstituted.

The above substituents apply to both the N-mono-substituted and the N-di-substituted aminocarbonyl. Preferably in the case of-di-substituted aminocarbonyl, the first substituent is selected from one of the above and the other is preferably C₁-C₄-alkyl, such as methyl, ethyl, isopropyl or cyclopropyl.

Alternatively, N-di-substituted aminocarbonyl can be a ring formed by the N and the two substituents such as a pyrrolidine or piperidine ring.
(e) Preferably R³ is unsubstituted or substituted aminocarbonyl.
   Aminocarbonyl is preferably N-mono- or N,N-di-substituted aminocarbonyl, that is mono- or di-substituted at the nitrogen by one or more moieties selected from unsubstituted or substituted, preferably substituted, alkyl, unsubstituted or substituted, preferably substituted, aryl, or unsubstituted or substituted, preferably substituted, cycloalkyl.
   Preferred examples for the alkyl moiety of the substituted aminocarbonyl substituent are branched or straight chain C₁-C₇-alkyl which may be substituted or unsubstituted. Preferred examples include methyl, ethyl or isopropyl, most preferably methyl. The alkyl moiety is preferably substituted. When the alkyl moiety is substituted, it is preferably mono-, di- or tri-substituted, more preferably mono-substituted. Suitable substituents for the alkyl moiety are as defined herein. More preferred examples of alkyl substituents of the substituted aminocarbonyl substituent are as follows:
   (i) aryl, preferably unsubstituted or substituted phenyl or naphthyl. Aryl may be unsubstituted or further substituted such as mono- or di-substituted. Suitable substituents are as described herein, preferably O-C₁-C₄-alkyl, halo, hydroxy, unsubstituted or substituted, preferably unsubstituted, heterocyclyl, such as 5- or 6-membered, preferably nitrogen containing aromatic or saturated rings, preferably pyrrolyl, morpholinyl, piperidyl and pyrrolidinyl, nitro, amino, acylamino, amino-C₁-C₇-alkyl, carboxyl, and cyano.
   (ii) heterocyclyl, preferably unsubstituted or substituted mono- or bicyclic ring systems. Preferred are, in particular if a bicyclic moiety is contemplated, aromatic ring systems or fully saturated ring systems, or, in particular if a bicyclic moiety is contemplated, aromatic ring systems or partially saturated ring systems, in particular whereby one of the rings is aromatic and the other is saturated or partially saturated. The heterocyclyl moiety has preferably 1, 2 or 3, more preferably 1 or 2 heteroatoms selected from O, N or S, more preferably O or N. Particularly preferred examples include 5- or 6 membered rings membered rings preferably containing a nitrogen, or oxygen atom, in particular pyrrolyl, furanyl, pyridyl, imidazolyl, thiazoyl, oxazolyl, pyrrolidinyl, tetrahydrofuranyl, or bicyclic ring systems containing 5- or 6 membered rings preferably containing a nitrogen or oxygen atom, in particular quinilinyl, isoquinolinyl, benzofuranyl, indolyl, benzoimidazolyl, benzothiazolyl, 2,3-dihydrobenzofuranyl, benzo[1,3]dioxolyl, or 2,3-dihydro-benzo[1,4]dioxinyl. When the heterocyclyl moiety is substituted, it is preferably mono-substituted. Suitable substituents for the heterocyclyl moiety are as defined herein, preferably C₁-C₇-alkyl, halo, hydroxy, nitro, unsubstituted or substituted, preferably unsubstituted, amino, unsubstituted or substituted, preferably unsubstituted, amino-C₁-C₇-alkyl, whereby the amino moiety can be substituted by -C₁-C₇-alkyl, cycloalkyl, phenyl or heterocyclyl; carboxyl, and cyano, more preferably C₁-C₄-alkyl such as methyl. Most preferably the heterocyclyl moiety is unsubstituted or , if present, N-substituted.
   (iii) halo, hydroxy, nitro, amino, amino-C₁-C₇-alkyl, carboxyl, and cyano.

Preferred examples for the aryl moiety of the substituted aminocarbonyl substituent are phenyl or naphthyl. Aryl may be unsubstituted or further substituted such as mono- or di-substituted. Suitable substituents are as described herein, preferably O-C₁-C₄-alkyl, halo, hydroxy, nitro, amino, acylamino, amino-C₁-C₇-alkyl, carboxyl, and cyano.

Preferred examples for the cycloalkyl moiety of the substituted aminocarbonyl substituent are monocyclic rings, preferably C₃-C₇-cycloalkyl, more preferably C₃, C₄, C₅ and C₆-cyctoalkyl, yet more preferably C₅ and C₆-cycloalkyl. The cycloalkyl moiety may be substituted or unsubstituted. When the cycloalkyl moiety is substituted, it is preferably mono-substituted. Suitable substituents for the cycloalkyl moiety are as defined herein, preferably O-C₁-C₄-alkyl, halo, hydroxy, nitro, amino, amino-C₁-C₇-alkyl, carboxyl, and cyano, most preferably phenyl or naphthyl. Most preferably, O-C₁-C₄-alkyl or hydroxyl.

Typically aminocarbonyl is N-mono-substituted. If aminocarbonyl is N-di-substituted, the first substituent is selected from one of the above and the other is preferably C₁-C₄-alkyl, such as methyl, ethyl or isopropyl.
(f) Preferably R³ is unsubstituted or substituted heterocyclyl carbonyl.
   Preferred examples for heterocyclyl moiety of the heterocyclyl carbonyl are monocyclic rings, preferably 5 or 6-memberedrings. Preferably these rings are fully saturated. Preferably the rings contain one or two, more preferably 1 heteroatom selected from O or N, more preferably N. Most preferred is pyrrolidinyl. The heterocyclyl moiety may be substituted or unsubstituted. Preferred substituents include C₁-C₇-alkyl, O-C₁-C₇-alkyl, halo-C₁-C₇-alkyl, halo, hydroxy, unsubstituted or substituted, preferably substituted, phenyl or naphthyl, unsubstituted or substituted, preferably substituted, phenyl- or naphthyloxy, unsubstituted or substituted, preferably substituted, phenyl- or naphthyl-C₁-C₇-alkyloxy, nitro, amino, amino-C₁-C₇-alkyl, carboxyl, and cyano, most preferably substituted phenyl whereby the substituent is preferably C₁-C₇-alkyl, O-C₁-C₇-alkyl, halo-C₁-C₇-alkyl, halo, hydroxy, nitro, amino, amino-C₁-C₇-alkyl, carboxyl, and cyano, most preferably halo.

According to the second embodiment R⁴ is preferably hydrogen or OH, more preferably hydrogen.

Preferably in a third embodiment L is NH or substituted NH. In this instance substituted NH means preferably substituted with cycloalkyl alkyl, alkyl or with N-mono- or N,N-di-substituted aminocarbonyl substituted alkyl. Cycloalkyl alkyl is preferably cycloalkyl-C₁₋₄ alkyl-, in particular cycloalkyl-CH₂-. Preferred examples for the cycloalkyl moiety are monocyclic rings, preferably C₃-C₇-cycloalkyl, more preferably C₃, C₄, C₅ and C₆-cycloalkyl, in particular C₅-cycloalkyl. Preferred examples for alkyl substituent of substituted NH are branched or straight chain C₁-C₇-alkyl which may be substituted or unsubstituted. Preferred examples include methyl, ethyl, isopropyl, n-propyl, n-butyl, sec-butyl or tert-butyl, more preferably methyl, ethyl or isopropyl, most preferably methyl. With N-mono- or N,N-di-substituted aminocarbonyl substituted alkyl is preferably the same as defined below under item (e), in particular (e) (iii), namely N-mono- or N,N-di-substituted aminocarbonyl substituted with substituted alkyl such as alkyl substituted with phenyl or cycloalkyl, in particular phenyl.

Preferably in this third embodiment, R³ is one of the following (a) to (m):
(a) Preferably R³ is unsubstituted or substituted aryl-alkyl.
   unsubstituted or substituted aryl-alkyl, such as phenyl-C₁-C₄-alkyl or naphthyl-C₁-C₄-alkyl, preferably phenyl-C₁-C₄-alkyl, such as benzyl, phenethyl, phenyl-CH₂CH₂CH₂, phenyl-CH₂CH(OH)CH₂, phenyl-CH₂CH₂CH₂CH₂, phenyl-CH(CH₃), naphthyl-CH₂, most preferably benzyl or naphthyl-CH₂. When the aryl moiety is substituted, it is preferably mono- or di-substituted. Suitable substituents are as defined herein, preferably -(C₀-C₇-alkylene)-(X)ᵣ-(C₁-C₇-alkylene)-(Y)ₛ-(C₀-C₇-alkylene)-H, wherein r and s are 0 or 1 and Y and X are independently O, NH or -NH-CO-O-, -CO-NH-, NHCO, N(C₁-C₇-alkyl), halo-C₁-C₇-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, N(mono or di- CO-C₁-C₇-alkyl or formyl) amino, amino-C₁-C₇-alkyl, carboxyl, and cyano. Preferred examples of -(C₀-C₇-alkylene)-(X)ᵣ-(C₁-C₇-alkylene)-(Y)ₛ-(C₀-C₇-alkylene)-H include -(O or NH)-C₁-C₇-alkyl, -CO-NH₂, -C₁-C₇-alkyl, -NHCO-C₁-C₇-alkyl, -(O or NH)-C₁-C₇-alkylene-(O or NH)-C₁-C₇-alkyl, -(O or NH)-C₁-C₇-alkylene-(O or NH)-H, -C₁-C₇-alkylene-(O or NH)-C₁-C₇-alkylene-(O or NH)-C₁-C₇-alkyl, -C₁-C₇-alkylene-(O. NH-CO-O, NHCO or NH)-C₁-C₇-alkyl, -C₁-C₇-alkylene-(O, NHCO or NH)-H, -C₁-C₇-alkylene-N(C₁-C₇-alkyl)-C₁-C₇-alkyl, -C₁-C₇-alkylene-(O or NH)-C₁-C₇-alkylene-(O or NH)-H or -C₁-C₇-alkylene-NH-CO-O-C₁-C₇-alkyl, most preferably -OMe, -CH₂NH₂, -CONH₂, -CH₂N(Me)₂,-CH₂NHCOMe, -CH₂NHCO-H, -CH₂NHC₂H₄OH, NHCOMe, -OC₂H₄OMe, NHCOMe, or-OC₃H₆OMe. Most preferably the aryl moiety is unsubstituted or substituted with halo, OMe and/or CN.
(b) Preferably R³ is unsubstituted or substituted heterocyclyl or unsubstituted or substituted heterocyclyl-alkyl.
   Preferably heterocyclyl alkyl is heterocyclyl-C₁₋₄ alkyl-, in particular heterocyclyl-CH₂-. Preferred examples for the heterocyclyl moiety are mono- or bicyclic rings. Preferred are saturated ring systems, or in particular if a bicyclic moiety is contemplated, aromatic or partially saturated ring systems, in particular whereby one of the rings is aromatic and the other is saturated or partially saturated. The heterocyclyl moiety has preferably 1, 2 or 3, more preferably 1 or 2 heteroatoms selected from O, N or S, more preferably O or N. Particularly preferred examples include 5-membered rings preferably containing a nitrogen atom, in particular pyrrolidinyl or tetrahydrofuranyl; or bicyclic ring systems preferably containing a nitrogen or oxygen atom, in particular 2,3-dihydro-benzo[1,4]dioxinyl, 3,4-dihydro-2H-benzo[b][1,4]dioxepinyl, benzofuranyl, benzo[1,2,5]oxadiazolyl, benzimidazolyl or 3,4-dihydro-2H-benzo[1,4]oxazinyl, more preferably pyrrolidinyl, benzimidazolyl or 3,4-dihydro-2H-benzo[1,4]oxazinyl. When the heterocyclyl moiety is substituted, it is preferably mono-substituted. Suitable substituents for the heterocyclyl moiety are as defined herein, preferably -C₁-C₇-alkyl, halo, hydroxy, unsubstituted or substituted phenyl, unsubstituted or substituted, preferably unsubstituted, phenyl- or naphthyloxy, unsubstituted or substituted, preferably unsubstituted, phenyl- or naphthyl-C₁-C₇-alkyl, nitro, amino, amino-C₁-C₇-alkyl, carboxyl, and cyano, more preferably phenyl-C₁-C₇-alkyl,. Suitable phenyl substituents include C₁-C₇-alkyl, -O-C₁-C₇-alkyl, halo-C₁-C₇-alkyl, halo, hydroxyl and amino. Most preferably the heterocyclyl moiety is unsubstituted.
(c) Preferably R³ is unsubstituted or substituted cycloalkyl.
   Preferred examples for the cycloalkyl moiety are monocyclic rings, preferably C₃-C₇-cycloalkyl, more preferably C₃, C₄, C₅ and C₆-cycloalkyl. The cycloalkyl moiety may be substituted or unsubstituted. When the cycloalkyl moiety is substituted, it is preferably mono-substituted. Suitable substituents for the cycloalkyl moiety are as defined herein, preferably O-C₁-C₄-alkyl, halo, hydroxy, unsubstituted or substituted phenyl, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano, most preferably phenyl or naphthyl. Most preferably, the cycloalkyl moiety is unsubstituted.
(d) Preferably R³ is unsubstituted or substituted cycloalkyl-alkyl.
   Preferably cycloalkyl alkyl is cycloalkyl-C₁₋₄ alkyl-, in particular cycloalkyl-CH₂-. Preferred examples for the cycloalkyl moiety are monocyclic rings, preferably C₃-C₇-cycloalkyl, more preferably C₃, C₄, C₅ and C₆-cycloalkyl, in particular C₅-cycloalkyl. The cycloalkyl moiety may be substituted or unsubstituted. When the cycloalkyl moiety is substituted, it is preferably mono-substituted. Suitable substituents for the cycloalkyl moiety are as defined herein, preferably O-C₁-C₄-alkyl, halo, hydroxy, unsubstituted or substituted phenyl, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano, most preferably phenyl or naphthyl. Most preferably, the cycloalkyl moiety is unsubstituted.
(e) Preferably R³ is unsubstituted or substituted alkyl.
   Preferred examples for alkyl are branched or straight chain C₁-C₇-alkyl which may be substituted or unsubstituted. Preferred examples include methyl, ethyl, isopropyl, n-propyl, n-butyl, sec-butyl or tert-butyl, more preferably methyl, ethyl or isopropyl, most preferably methyl. The alkyl moiety is preferably substituted. When the alkyl moiety is substituted, it is preferably mono-, di- or tri-substituted, more preferably mono-substituted. Suitable substituents for the alkyl moiety are as defined herein, preferably O-C₁-C₄-alkyl, halo, hydroxy, unsubstituted or substituted, preferably unsubstituted, phenyl, unsubstituted or substituted, preferably unsubstituted, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, unsubstituted or substituted, preferably unsubstituted, cycloalkyl, nitro, amino, amino-C₁-C₇-alkyl, N-mono- or N,N-di-substituted aminocarbonyl, carboxyl, and cyano. A preferred example is N-mono- or N,N-di-substituted aminocarbonyl which will be described in more detail below with respect to preferred substituents: :
   (i) Aminocarbonyl is preferably N-substituted by cycloalkyl. Preferred examples for the cycloalkyl moiety are monocyclic rings, preferably C₃-C₇-cycloalkyl, more preferably C₃, C₄, C₅ and C₆-cycloalkyl, in particular C₃-cycloalkyl. The cycloalkyl moiety may be substituted or unsubstituted. When the cycloalkyl moiety is substituted, it is preferably mono-substituted. Suitable substituents for the cycloalkyl moiety are as defined herein, preferably O-C₁-C₄-alkyl, halo, hydroxy, unsubstituted or substituted phenyl, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano, most preferably phenyl or naphthyl. Most preferably, the cycloalkyl moiety is unsubstituted.
   (ii) Aminocarbonyl is preferably N-substituted by unsubstituted alkyl. Preferred examples for the alkyl moiety are straight chain or branched alkyl, preferably straight chain alkyl, more preferably methyl or ethyl.
   (iii) Aminocarbonyl is preferably N-substituted by substituted alkyl. Preferred examples for the alkyl moiety are as defined for the unsubstituted alkyl moiety under item (ii). Particularly preferred is methyl.. The alkyl moiety is typically mono- or di-substituted, preferably mono-substituted. Suitable substituents for the alkyl moiety are as defined herein, preferably O-C₁-C₄-alkyl, halo, hydroxy, unsubstituted or substituted, preferably unsubstituted, phenyl, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, unsubstituted or substituted, preferably unsubstituted, C₃-C₇-cycloalkyl, such as C₃, C₄, C₅ and C₆-cycloalkyl, in particular C₆ or C₃-cycloalkyl; substituted, preferably unsubstituted, heterocyclyl, such as five-or six-membered rings, preferably fully saturated, preferably containing one heteroatom selected from O or N, such as tetrahydropyranyl or piperidinyl; nitro, unsubstituted or substituted, preferably unsubstituted, amino, unsubstituted or substituted, preferably unsubstituted, amino-C₁-C₇-alkyl, whereby the amino moiety can be substituted by -C₁-C₇-alkyl, cycloalkyl, phenyl or heterocyclyl; carboxyl, and cyano, most preferably phenyl, heterocyclyl or cycloalkyl.
   (iv) Aminocarbonyl is preferably N-substituted by heterocyclyl. Preferred examples for the heterocyclyl moiety are mono- or bicyclic rings, more preferably mnocyclic rings such as 5- or 6-membered rings. Preferred are saturated ring systems or aromatic ring systems, in particular saturated ring systems. The heterocyclyl moiety has preferably 1, 2 or 3, more preferably 1 or 2 heteroatoms selected from O, N or S, more preferably O or N. Particularly preferred examples include 5- or 6-membered rings preferably containing an oxygen atom, in particular tetrahydropyranyl or tetrahydrofuranyl. When the heterocyclyl moiety is substituted, it is preferably mono-substituted. Suitable substituents for the heterocyclyl moiety are as defined herein, preferably halo, hydroxy, nitro, amino, amino-C₁-C₇-alkyl, carboxyl, and cyano, more preferably phenyl-C₁-C₇-alkyl,. Suitable phenyl substituents include C₁-C₇-alkyl, -O-C₁-C₇-alkyl, halo-C₁-C₇-alkyl, halo, hydroxyl and amino. Most preferably the heterocyclyl moiety is unsubstituted.
   (v) Aminocarbonyl is preferably N-substituted by aryl. Preferred examples of aryl include phenyl or naphthyl, more preferably phenyl. When the aryl moiety is substituted, it is preferably mono- or di-substituted. In particular, phenyl is preferably mono-substituted. Most preferably aryl is unsubstituted. Suitable substituents are as defined herein, preferably C₁-C₇-alkyl, -O-C₁-C₇-alkyl, halo-C₁-C₇-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano.

The above substituents apply to both the N-mono-substituted and the N-di-substituted aminocarbonyl. Preferably in the case of-di-substituted aminocarbonyl, the first substituent is selected from one of the above and the other is preferably C₁-C₄-alkyl, such as methyl, ethyl, isopropyl or cyclopropyl.

Alternatively, N-di-substituted aminocarbonyl can be a ring formed by the N and the two substituents such as a pyrrolidine or piperidine ring.
(f) Preferably R³ is unsubstituted or substituted arylcarbonyl.
   Preferred examples for the aryl moiety of the arylcarbonyl substituent are phenyl or naphthyl. Aryl may be unsubstituted or further substituted. When the aryl moiety is substituted, it is preferably mono- or di-substituted. Suitable substituents are as defined herein, preferably - (C₀-C₇-alkylene)-(X)ᵣ-(C₁-C₇-alkylene)-(Y)ₛ-(C₀-C₇-alkylene)-H, wherein r and s are 0 or 1 and Y and X are independently O, NH or -NH-CO-O-, -CO-NH-, NHCO, N(C₁-C₇-alkyl), halo-C₁-C₇-alkyl, halo, hydroxy, unsubstituted or substituted, preferably unsubstituted, phenyl- or naphthyloxy, unsubstituted or substituted, preferably unsubstituted, phenyl- or naphthyl-C₁-C₇-alkyloxy, nitro, unsubstituted or substituted, preferably unsubstituted, amino, whereby the amino moiety can be substituted by -C₁-C₇-alkyl, cycloalkyl, phenyl or heterocyclyl; N(mono or di- CO-C₁-C₇-alkyl or formyl) amino, amino-C₁-C₇-alkyl, carboxyl, and cyano. Preferred examples of -(C₀-C₇-alkylene)-(X)ᵣ-(C₁-C₇-alkylene)-(Y)ₛ-(C₀-C₇-alkylene)-H include -(O or NH)-C₁-C₇-alkyl, -CO-NH₂, -C₁-C₇-alkyl, -NHCO-C₁-C₇-alkyl, -(O or NH)-C₁-C₇-alkylene-(O or NH)-C₁-C₇-alkyl, -(O or NH)-C₁-C₇-alkylene-(O or NH)-H, -C₁-C₇-alkylene-(O or NH)-C₁-C₇-alkylene-(O or NH)-C₁-C₇-alkyl, -C₁-C₇-alkylene-(O, NH-CO-O, NHCO or NH)-C₁-C₇-alkyl, - C₁-C₇-alkylene-(O, NHCO or NH)-H, -C₁-C₇-alkylene-N(C₁-C₇-alkyl)-C₁-C₇-alkyl, -C₁-C₇-alkylene-(O or NH)-C₁-C₇-alkylene-(O or NH)-H or -C₁-C₇-alkylene-NH-CO-O-C₁-C₇-alkyl, most preferably -OMe, -CH₂NH₂, -CONH₂, -CH₂N(Me)₂, -CH₂NHCOMe. -CH₂NHCO-H, - CH₂NHC₂H₄OH, NHCOMe, -OC₂H₄OMe, NHCOMe, or -OC₃H₆OMe. Most preferably the aryl moiety is unsubstituted or substituted with halo, OMe and/or CN.
(g) Preferably R³ is unsubstituted or substituted alkylcarbonyl.
   Preferred examples for the alkyl moiety of the alkylcarbonyl substituent is branched or straight chain C₁-C₇-alkyl, more preferably C₁-C₄-alkyl, most preferably methyl or ethyl, which may be substituted or unsubstituted. When the alkyl moiety is substituted, it is preferably mono-substituted. Preferably the alkyl moiety is substituted. Suitable substituents for the alkyl moiety are as defined herein, preferably O-C₁-C₄-alkyl, halo, hydroxy, unsubstituted or substituted, preferably unsubstituted, phenyl or naphthyl, unsubstituted or substituted, preferably unsubstituted, C₃-C₇-cycloalkyl, or substituted, preferably unsubstituted, heterocyclyl, such as 5-or six-membered rings, preferably fully saturated, preferably containing one heteroatom selected from O or N, such as tetrahydropyranyl, nitro, amino, amino-C₁-C₇-alkyl, carboxyl, and cyano, more preferably phenyl, heterocyclyl, cycloalkyl and/or OH.
(h) Preferably R³ is unsubstituted or substituted cycloalkyl-carbonyl.
   Preferred examples for the cycloalkyl moiety are monocyclic rings, preferably C₃-C₇-cycloalkyl, more preferably C₃, C₄, C₅ and C₆-cycloalkyl, in particular C₆-cycloalkyl. The cycloalkyl moiety may be substituted or unsubstituted. When the cycloalkyl moiety is substituted, it is preferably mono-substituted. Suitable substituents for the cycloalkyl moiety are as defined herein, preferably -C₁-C₇-alkyl, O-C₁-C₄-alkyl, halo, hydroxy, unsubstituted or substituted phenyl, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, nitro, amino, amino-C₁-C₇-alkyl, carboxyl, and cyano, most preferably phenyl or naphthyl. Most preferably, the cycloalkyl moiety is unsubstituted.
(i) Preferably R³ is unsubstituted or substituted heterocyclyl-carbonyl
   Preferred examples for the heterocyclyl moiety are mono- or bicyclic rings. Preferred are saturated ring systems, or in particular if a bicyclic moiety is contemplated, aromatic or partially saturated ring systems, in particular whereby one of the rings is aromatic and the other is saturated or partially saturated. The heterocyclyl moiety has preferably 1, 2 or 3, more preferably 1 or 2 heteroatoms selected from O, N or S, more preferably O or N. Particularly preferred examples include 6-membered rings preferably containing an oxygen atom atom, in particular motpholinyl or tetrahydropyranyl; or bicyclic ring systems preferably containing a nitrogen or oxygen atom, in particular 2,3-dihydro-benzo[1,4]dioxinyl, 3,4-dihydro-2H-benzo[b][1,4]dioxepinyl, benzofuranyl, benzo[1,2,5]oxadiazolyl, benzimidazolyl or 3,4-dihydro-2H-benzo[1,4]oxazinyl, more preferably tetrahydropyranyl. When the heterocyclyl moiety is substituted, it is preferably mono-substituted. Suitable substituents for the heterocyclyl moiety are as defined herein, preferably halo, hydroxy, unsubstituted or substituted phenyl, unsubstituted or substituted, preferably unsubstituted, phenyl- or naphthyloxy, unsubstituted or substituted, preferably unsubstituted, phenyl- or naphthyl-C₁-C₇-alkyl, nitro, amino, amino-C₁-C₇-alkyl, carboxyl, and cyano, more preferably phenyl-C₁-C₇-alkyl,. Suitable phenyl substituents include C₁-C₇-alkyl, -O-C₁-C₇-alkyl, halo-C₁-C₇-alkyl, halo, hydroxyl and amino. Most preferably the heterocyclyl moiety is unsubstituted.
(j) Preferably R³ is unsubstituted or substituted etherified carboxy. Preferred examples of the etherified carboxy include a carbonyl group to which one of the following groups are bound:
   (i) O-alkyl, whereby preferred examples for the alkyl moiety of the etherified carboxy substituent are branched or straight chain C₁-C₇-alkyl which may be substituted or unsubstituted. Preferred examples include methyl, ethyl, isopropyl, n-propyl, n-butyl, sec-butyl or tert-butyl, more preferably methyl, ethyl or isopropyl, most preferably methyl. The alkyl moiety is preferably substituted. When the alkyl moiety is substituted, it is preferably mono-, di- or tri-substituted, more preferably mono-substituted. Suitable substituents for the alkyl moiety are as defined herein, preferably O-C₁-C₄-alkyl, halo, hydroxy, unsubstituted or substituted, preferably unsubstituted, phenyl, unsubstituted or substituted, preferably unsubstituted, naphthyl, unsubstituted or substituted, preferably unsubstituted, phenyl- or naphthyloxy, unsubstituted or substituted, preferably unsubstituted, phenyl- or naphthyl-C₁-C₇-alkyloxy, unsubstituted or substituted, preferably unsubstituted, cycloalkyl, nitro, amino, amino-C₁-C₇-alkyl, N-mono- or N,N-di-substituted aminocarbonyl, such as monosubstituted aminocarbonyl with e.g. alkyl or cycloalkyl, carboxyl, and cyano, most preferably the substituent is phenyl, preferably unsubstituted phenyl, cycloalkyl, preferably cyclohexyl, and N-mono- or N,N-di-substituted aminocarbonyl, preferably monosubstituted with cycloalkyl such as cyclopropyl.
   (ii) O-aryl, preferably unsubstituted or substituted phenyl or naphthyl, more preferably phenyl. Aryl may be unsubstituted or further substituted such as mono- or di-substituted. Suitable substituents are as described herein, preferably C₁-C₇-alkyl, O-C₁-C₄-alkyl, halo-C₁-C₇-alkyl, halo, hydroxy, unsubstituted or substituted, preferably unsubstituted, amino, acylamino, unsubstituted or substituted, preferably unsubstituted, amino-C₁-C₇-alkyl , whereby the amino moiety can be substituted by -C₁-C₇-alkyl, cycloalkyl, phenyl or heterocyclyl; carboxyl, and cyano. Preferably aryl is unsubstituted.
   (iii) O-cycloalkyl, preferably monocyclic rings, more preferably C₃-C₇-cycloalkyl, yet more preferably C₃, C₄, C₅ and C₆-cycloalkyl, still more preferably C₅ and C₆-cycloalkyl. The cycloalkyl moiety may be substituted or unsubstituted. When the cycloalkyl moiety is substituted, it is preferably mono-substituted. Suitable substituents for the cycloalkyl moiety are as defined herein, preferably C₁-C₇-alkyl, O-C₁-C₄-alkyl, halo, hydroxy, nitro, unsubstituted or substituted, preferably unsubstituted, amino, unsubstituted or substituted, preferably unsubstituted, amino-C₁-C₇-alkyl , whereby the amino moiety can be substituted by -C₁-C₇-alkyl, cycloalkyl, phenyl or heterocyclyl; carboxyl, and cyano. Most preferably cyloalkyl is unsubstituted.
(k) Preferably R³ is unsubstituted or substituted aminocarbonyl. Aminocarbonyl is preferably N-mono- or N,N-di-substituted aminocarbonyl, that is mono- or di-substituted at the nitrogen by one or more moieties selected from unsubstituted or substituted, preferably substituted, alkyl, unsubstituted or substituted, preferably substituted, aryl, or unsubstituted or substituted, preferably substituted, cycloalkyl.
   Preferred examples for the alkyl moiety of the substituted aminocarbonyl substituent are branched or straight chain C₁-C₇-alkyl which may be substituted or unsubstituted. Preferred examples include methyl, ethyl or isopropyl, most preferably methyl or ethyl. The alkyl moiety is preferably substituted. When the alkyl moiety is substituted, it is preferably mono-, di- or tri-substituted, more preferably mono-substituted. Suitable substituents for the alkyl moiety are as defined herein. A preferred example of an alkyl substituent of the substituted aminocarbonyl substituent is aryl, preferably unsubstituted or substituted phenyl or naphthyl. Aryl may be unsubstituted or further substituted such as mono- or di-substituted. Suitable substituents are as described herein, preferably C₁-C₇-alkyl, O-C₁-C₄-alkyl, halo-C₁-C₇-alkyl, halo, hydroxy, nitro, amino, acylamino, amino-C₁-C₇-alkyl, carboxyl, and cyano.

Typically aminocarbonyl is N-mono-substituted. If aminocarbonyl is N-di-substituted, the first substituent is selected from one of the above and the other is preferably C₁-C₄-alkyl, such as methyl, ethyl or isopropyl.
(I) Preferably R³ is unsubstituted or substituted arylsufonyl.
   Preferred examples of the aryl moiety of arylsufonyl include unsubstituted or substituted phenyl or naphthyl. Aryl may be unsubstituted or further substituted such as mono- or di-substituted. Suitable substituents are as described herein, preferably C₁-C₇-alkyl, O-C₁-C₄-alkyl, halo-C₁-C₇-alkyl, halo, hydroxy, unsubstituted or substituted, preferably unsubstituted, phenyl, unsubstituted or substituted, preferably unsubstituted, phenyl- or naphthyloxy, unsubstituted or substituted, preferably unsubstituted; phenyl- or naphthyl-C₁-C₇-alkyl, amino, acylamino, amino-C₁-C₇-alkyl, carboxyl, and cyano. Preferably aryl is unsubstituted or substituted with C₁-C₇-alkyl, such as methyl, unsubstituted phenyloxy, or O-C₁-C₄-alkyl, such as OMe or O-isopropyl.
(m) Preferably R³ is unsubstituted or substituted alkylsufonyl.
   Preferred examples for the alkyl moiety of the alkylsulfonyl substituent is branched or straight chain C₁-C₇-alkyl, more preferably C₁-C₄-alkyl, most preferably methyl or ethyl, which may be substituted or unsubstituted. When the alkyl moiety is substituted, it is preferably mono-substituted. Preferably the alkyl moiety is substituted. Suitable substituents for the alkyl moiety are as defined herein, preferably O-C₁-C₄-alkyl, halo, hydroxy, unsubstituted or substituted, preferably unsubstituted, phenyl or naphthyl, such as with C₁-C₇-alkyl, O-C₁-C₄-alkyl, halo-C₁-C₇-alkyl, halo-C1-C7-alkyl-O-, halo, hydroxy, amino, acylamino, amino-C₁-C₇-alkyl, carboxyl, and cyano mono-substituted phenyl; unsubstituted or substituted, preferably unsubstituted, phenyl- or naphthyloxy, unsubstituted or substituted, preferably unsubstituted, phenyl- or naphthyl-C₁-C₇-alkyl, unsubstituted or substituted, preferably unsubstituted, C₃-C₇-cycloalkyl, such as C₅ and C₆-cycloalkyl; or substituted, preferably unsubstituted, heterocyclyl, such as 5-or six-membered rings, preferably fully saturated, preferably containing one heteroatom selected from O or N, such as tetrahydropyranyl; nitro, amino, amino-C₁-C₇-alkyl, carboxyl, and cyano, more preferably phenyl, heterocyclyl, cycloalkyl and/or OH. A most preferred example of a substituted alkylsulfonyl is and unsubstituted benzylsulfonyl.

According to the third embodiment R⁴ is preferably hydrogen or OH, more preferably hydrogen.

Preferably in a fourth embodiment L is a bond. According to the fourth embodiment R⁴ is preferably OH.

Preferably in a fifth embodiment R³ and R⁴ together with L form oxo (=O).

Preferably in a sixth embodiment R³ and R⁴ which then is -0- together with L which then is methylene and the carbon to which R³-L- and R⁴ are bound form a substituted or unsubstituted, preferably unsubstituted, 5-7 membered, preferably 5-membered, ring annealed to an unsubstituted or substituted aryl, unsubstituted or substituted heterocyclyl or unsubstituted or substituted cycloalkyl, preferred a unsubstituted or substituted aryl, in particular phenyl, which may be unsubstituted or further substituted such as mono- or di-substituted. Suitable substituents are as described herein, preferably C₁-C₇-alkyl, O-C₁-C₄-alkyl, halo-C₁-C₇-alkyl, halo, hydroxy, amino, acylamino, amino-C₁-C₇-alkyl, carboxyl, and cyano. Preferably aryl is unsubstituted.

It is most preferred that R⁴ is H independently of the other definitions of the substituents.

Independently of L and R³, R⁴ can be OH. In this embodiment it is preferred that
L is methylene and R³ is aryl, in particular phenyl, which may be unsubstituted or further substituted such as mono- or di-substituted. Suitable substituents are as described herein, preferably C₁-C₇-alkyl, O-C₁-C₄-alkyl, halo-C₁-C₇-alkyl, halo, hydroxy, amino, acylamino, amino-C₁-C₇-alkyl, carboxyl, and cyano. Preferably aryl is unsubstituted or substituted by halo.

Particular embodiments of the invention, especially of compounds of the formula I and/or salts thereof, are provided in the Examples - the invention thus, in a very preferred embodiment, relates to a compound of the formula I. or a salt thereof, selected from the compounds given in the Examples, as well as their use.

### Process of Manufacture

A compound of formula I, or a salt thereof, is prepared analogously to methods that, for other compounds, are in principle known in the art, so that for the novel compounds of the formula I the process is novel at least as analogy process, especially as described or in analogy to methods described herein in the illustrative Examples, or modifications thereof, preferably in general by
A) for the synthesis of a compound of the formula I wherein T is methylene, carbonyl or thiocarbonyl and R¹, R². R³, R⁴ and T have the meanings given above or below for a compound of the formula I, reacting an acid of the formula II, 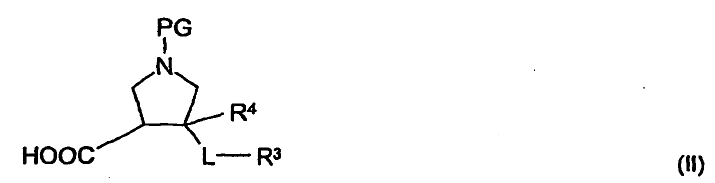 or a reactive derivative thereof, wherein R³, R⁴ and L are as defined for a compound of the formula I an PG is a protecting group, with
   (i) an amino compound of the formula III,

      R¹R²NH (III)

      wherein R¹ and R² are as defined for a compound of the formula I, under condensation conditions and
      (a) to obtain a compound of the formula I wherein T is carbonyl and wherein R¹, R², R³, R⁴, L and PG are as defined for compounds of the formula I, removing protecting groups or
      (b) , if desired, reducing the carbonyl group in the obtainable compound of the formula IV (a special compound of the formula I), 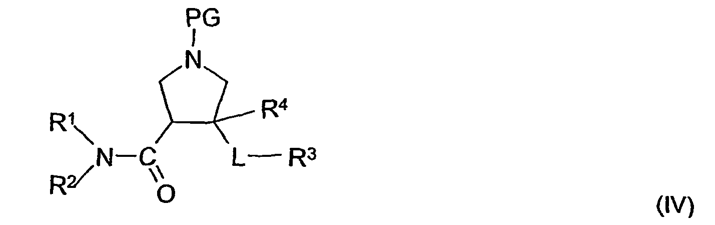 wherein R¹, R², R³, R⁴, L and PG are as defined for compounds of the formula II and III, to a methylene group, and, to obtain a compound of the formula I wherein R¹, R², R³, R⁴, L and PG are as defined for compounds of the formula I and T is methylene, removing protecting groups;
      or
   (ii) with an amino compound of the formula V,

      R²-NH₂ (V)

      wherein R¹ is as defined for a compound of the formula I, to give a compound of the formula VI, 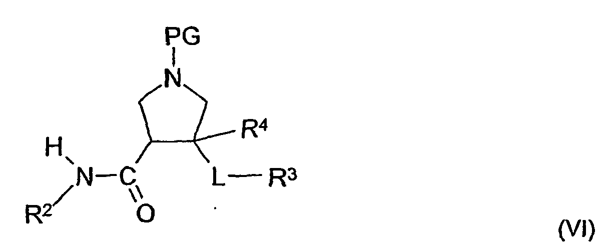 wherein R², R³, R⁴ and L are as defined for a compound for the formula I and PG is a protecting group, and either
      (a) reducing the carbonyl group whereby a compound of the formula VII 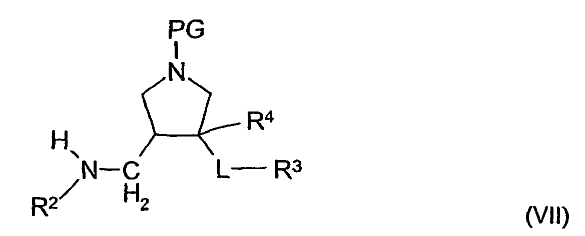 is obtained wherein R², R³, R⁴, L and PG are as defined for a compound of the formula VI, and reacting the compound of the formula VII with a compound of the formula VIII,

         R¹-Z (VIII)

         wherein R¹ is as defined for a compound of the formula I and Z is a leaving group,
         and, to obtain a compound of the formula I wherein T is methylene and R¹, R², R³, R⁴ and L are as defined for a corresponding compound of the formula I, removing protecting groups;
         or
      (b) reacting the compound of the formula VI with a compound of the formula VIII as defined above and, to obtain a compound of the formula I wherein T is carbonyl and R¹, R², R³, R⁴ and L are as defined for a compound of the formula I, removing protecting groups;
B) for the synthesis of a compound of the formula I wherein T is methylene and R¹, R², R³, R⁴ and T have the meanings given above or below for a compound of the formula I, reacting an aldehyde of the formula IX, 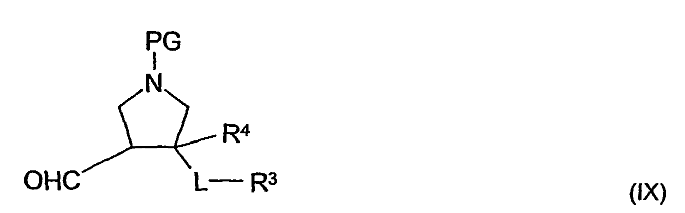 wherein R³, R⁴ and L are as defined for a compound of the formula I and PG is a protecting group, either
   (i) with an amino compound of the formula III as defined above under the conditions for reductive amination and, to obtain a compound of the formula I wherein R¹, R², R³, R⁴ and L are as defined for a compound of the formula I and T is methylene, removing protecting groups;
      or
   (ii) with an amino compound of the formula V as defined above whereby a compound of the formula X 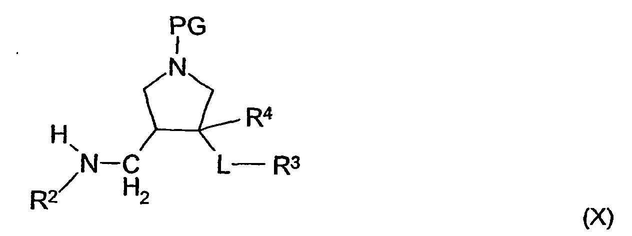 is obtained wherein R², R³, R⁴ and L are as defined for a compound of the formula I and PG is a protecting group, under conditions for reductive amination and then reacting the compound of the formula X
      (I) with a compound of the formula VIII as defined above or
      (II) for introduction of a moiety R¹ bound vial a methylene group that is part of said R¹, with an aldehyde of the formula VIII*

         R¹*-CHO (VIII*)

         wherein R¹* is a moiety complementing the moiety R¹*-CH₂- thus obtainable to a corresponding moiety R¹ (that is bound via a methylene) in the resulting compound, under conditions of reductive amination,
      and, to obtain a compound of the formula I wherein T is methylene and R¹, R², R³, R⁴ and L are as defined for a compound of the formula I, removing protecting groups;
C) for the synthesis of a compound of the formula I wherein R¹, R² and T are as defined for a compound of the formula I, R³ is unsubstituted or substituted alkyl, substituted or unsubstituted aryl, unsubstituted or substituted heterocyclyl, unsubstituted or substituted unsubstituted or substituted cycloalkyl, unsubstituted or substituted aryl-alkyl, unsubstituted or substituted heterocyclyl-alkyl, unsubstituted or substituted cycloalkyl-alkyl, substituted or unsubstituted alkylsulfonyl, substituted or unsubstituted arylsulfonyl, substituted or unsubstituted heterocyclylsulfonyl, substituted or unsubstituted cycloalkylsulfonyl, unsubstituted or substituted alkylcarbonyl, unsubstituted or substituted arylcarbonyl, unsubstituted or substituted heterocyclylcarbonyl, etherified carboxy or (less preferably) N-moo- or N,N-disubstituted amino-sulfonyl, R⁴ is hydrogen and L is oxy, thio or unsubstituted or substituted imino, a compound of the formula XI, 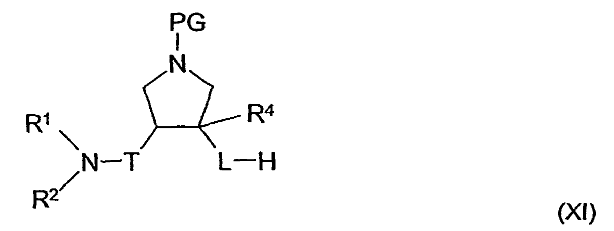 wherein R¹, R², R⁴ and T are as just defined, PG is a protecting group and L is oxy, thio or unsubstituted or substituted imino, is reacted
   (i) with a compound of the formula XII,

      R³-Z (XII)

      wherein Z is a leaving group and R³ is as just defined,
      or
   (ii) in the case where L is imino or monosubstituted imino, under the conditions of reductive amination with an aldehyde of the formula XIIA

      R³*-CHO (XIIA)

      wherein R³* is a moiety completing a moiety R³*-CH₂ thus obtainable to a corresponding moiety R³ in the resulting compound,
   and, to obtain a corresponding compound of the formula I, removing protecting groups;
D) for the preparation of a compound of the formula I wherein R¹, R² and T are as defined under formula I and R³ and R⁴ together with L form oxo, thioxo or unsubstituted or substituted imino, oxidising a compound of the formula XI as defined above but wherein L is oxy (so that -L-H is -OH) to a corresponding oxo compound of the formula XIII, 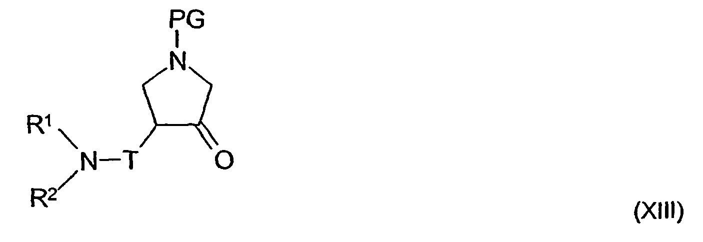 wherein R¹, R² and T are as defined under formula I and, if desired, converting the oxo group to a thioxo or unsubstituted or substituted imino group, and, to obtain a corresponding compound of the formula I, removing the protecting group(s);
E) for the synthesis of a compound of the formula I, wherein R¹, R², L and T are as defined for a compound of the formula I, R³ is unsubstituted or substituted alkyl, substituted or unsubstituted aryl, unsubstituted or substituted heterocyclyl, unsubstituted or substituted cycloalkyl, unsubstituted or substituted aryl-alkyl, unsubstituted or substituted heterocyclyl-alkyl or unsubstituted or substituted cycloalkyl-alkyl, and R⁴ is hydroxy, reacting a compound of the formula XIII as defined above with a metallo reagent of the formula XIV,

   R³-L-Mg-Hal (XIV)

   wherein R³ is as just defined and Hal is halo, and, to obtain a corresponding compound of the formula I, removing protecting groups;
F) for the synthesis of a spiro compound of the formula I wherein R¹, R² and T are as defined for a compound of the formula I and R³ and R⁴ which then is -O- together with L which then is methylene and the carbon to which R³-L- and R⁴ are bound form a substituted or unsubstituted ring annealed to an unsubstituted or substituted aryl, unsubstituted or substituted heterocyclyl or unsubstituted or substituted cycloalkyl, reacting a compound of the formula XV, 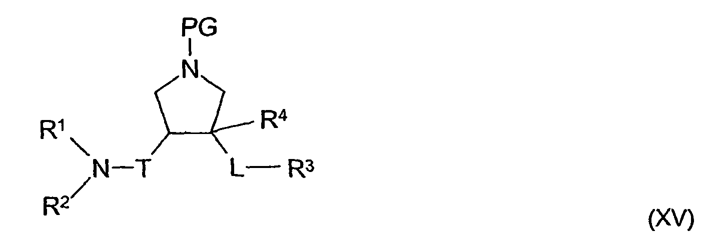 wherein R¹, R² and T are as defined for a compound of the formula I, R³ is substituted or unsubstituted aryl, unsubstituted or substituted heterocyclyl, or unsubstituted or substituted cycloalkyl, each of which carries a leaving group, L is methylene and R⁴ is hydroxy, in the presence of a strong base to obtain a corresponding spiro compound of the formula I, removing protecting groups;
G) for the synthesis of a compound of the formula I wherein R¹, R² and L are as defined for a compound of the formula I, R⁴ is hydrogen, L is oxy, thio or imino and R³ is N-mono-substituted amino-carbonyl, reacting a compound of the formula XI as shown above under C) wherein L is oxy, thio or imino and the other moieties are as described above, with an ioscyanate compound of the formula XIB,

   R3**-NCO (XIIB)

   wherein R³** is a substitutent completing the corresponding N-mono-substituted amino-carbonyl, and removing protecting groups to obtain the corresponding compound of the formula I; or
H) for the synthesis of a compound of the formula I wherein R¹, R² and T are as defined for a compound of the formula I, L is oxy, thio or unsubstituted or substituted imino and R³ is as defined above, reacting a reactive derivative of a compound of the formula XI as defined above under C), wherein instead of -L-H a leaving group is present, R⁴ is hydrogen and the other moieties are as defined under C), with a compound of the formula XIIC,

   R³-L-H (XIIC)

   wherein R³ is as defined for a compound of the formula I and L is oxy, thio or unsubstituted or substituted imino, and removing protecting groups to obtain the corresponding compound of the formula I;
and, if desired, subsequent to any one or more of the processes mentioned under (A) to (H) converting an obtainable compound of the formula I or a protected form thereof into a different compound of the formula I, converting a salt of an obtainable compound of formula I into the free compound or a different salt, converting an obtainable free compound of formula I into a salt thereof, and/or separating an obtainable mixture of isomers of a compound of formula I into individual isomers;
where in any of the starting materials (especially of the formulae II to XV), in addition to specific protecting groups mentioned, further protecting groups may be present, and any protecting groups are removed at an appropriate stage in order to obtain the corresponding compound of the formula I, or a salt thereof.

### Preferred Reaction Conditions

The preferred reaction conditions for the reactions mentioned above under A) to F), as well as for the transformations and conversions, are as follows:
The condensation reaction in A) (i) between an acid of the formula II, or a reactive derivative thereof, and an amino compound of the formula III preferably takes place under customary condensation conditions, where among the possible reactive derivatives of an acid of the formula II reactive esters (such as the hydroxybenzotriazole (HOBT), pentafluorophenyl, 4-nitrophenyl or N-hydroxysuccinimide ester), acid halogenides (such as the acid chloride or bromide) or reactive anhydrides (such as mixed anhydrides with lower alkanoic acids or symmetric anhydrides) are preferred. Reactive carbonic acid derivatives can also be formed in situ. The reaction is carried out by dissolving the compounds of formulae II and III in a suitable solvent, for example a halogenated hydrocarbon, such as methylene chloride, N,N-dimethylformamide, *N,N-*dimethylacetamide, *N*-methyl-2-pyrrolidone, methylene chloride, or a mixture of two or more such solvents, and by the addition of a suitable base, for example triethylamine or diisopropylethylamine (DIEA) and, if the reactive derivative of the acid of the formula II is formed *in situ,* a suitable coupling agent that forms a preferred reactive derivative of the carbonic acid of formula III *in situ,* for example dicyclohexylcarbodiimide/1-hydroxybenzotriazole (DCC/ HOBT); bis(2-oxo-3-oxazolidinyl)phosphinic chloride (BOPCI); O-(1,2-dihydro-2-oxo-1-pyridyl)-*N,N,N',N'*-tetramethyluronium tetrafluoroborate (TPTU); O-benzotriazol-1-yl)-N,N,N', N'-tetramethyluronium tetrafluoroborate (TBTU); (benzotriazol-1-yloxy)-tripyrrolidinophosphonium-hexafluorophosphate (PyBOP) or 1-(3-dimethylaminopropyl)-3-ethylcarbodiimide hydrochloride/hydroxybenzotriazole (EDCI/HOBT). For review of some other possible coupling agents, see e.g. Klauser; Bodansky, Synthesis 1972, 453-463. The reaction mixture is preferably stirred at a temperature of between approximately -20 and 50°C, especially between 0 °C and 30 °C, e.g. at room temperature. The reaction is preferably carried out under an inert gas, e.g. nitrogen or argon.

The subsequent removal of a protecting group under A) (i) (a), e.g. PG, such as tert-butoxycarbonyl, benzyl or 2-(trimethylsilyl)-ethoxycarbonyl, takes place under standard conditions, see also the literature mentioned below under General Process Conditions. For example, tert-butoxycarbonyl is removed in the presence of an acid, e.g. a TFA or hydrohalic acid, such as HCl, in an appropriate solvent, e.g. an ether, such as dioxane, at customary temperatures, e.g. at room temperature, the removal of benzyl can be achieved e.g. by reaction with ethylchloroformate or 2-trimethylsilylethyl-chloroformate in an appropriate solvent, e.g. toluene, at elevated temperatures, e.g. from 80 to 110 °C, and subsequent removal of the resulting ethoxycarbonyl group by hydrolysis in the presence of a base, e.g. an alkali metal hydroxide, such as potassium hydroxide, in an appropriate solvent, e.g. in an alcohol, such as ethanol, at elevated temperatures, e.g. from 80 to 120 °C, and the removal of 2-(trimethylsilyl)-ethoxycarbonyl can be achieved, for example, by reaction with a tetra-lower alkylammonium fluoride, such as tetraethylammoniumfluoride, in an appropriate solvent or solvent mixture, e.g. a halogenated hydrocarbon, such as methylene chloride, and/or a nitrile, such as acetoneitrile, preferably at elevated temperatures, e.g. under reflux conditions.

The reduction of a carbonyl group can preferably take place in the presence of an appropriate complex hydride, e.g. borane dimethylsulfide complex, in an appropriate solvent, such as an ether, e.g. tetrahydrofurane, at preferred temperatures between room temperature and the reflux temperature of the reaction mixture or at 140-150 °C, the subsequent removal of (a) protecting group(s) can be achieved as just described.

In step A) (ii), the reaction between a compound of the formula V with an acid of the formula II, or a reactive derivative thereof, preferably takes place under conditions analogous to those described above for reaction A) (i), the subsequent reduction under A) (ii) (a) preferably under the reaction conditions described under A) i) (b) before the removal of the protecting group. The reaction between a compound of the formula VII and a compound of the formula VIII under A) (ii) (a) preferably takes place under customary substitution conditions, e.g. in the case where an aryl moiety R¹ is to be coupled and Z is halo, e.g. iodo or bromo, in the presence of copper (e.g. Venus copper), sodium or potassium iodide and a base, such as potassium carbonate, in the presence or preferably absence of an appropriate solvent, e.g. at elevated temperatures in the range from, for example, 150 to 250 °C, or (especially if Z in formula VIII is bromo) in the presence of a strong base, such as an alkali metal alkoholate, e.g. sodium tert-butylate, in the presence of an appropriate catalyst, such as [Pd(µ-Br)(t-Bu₃P)]₂, in the presence of an appropriate solvent, e.g. an aromatic solvent, such as toluene, at preferred temperatures between room temperature and the reflux temperature of the mixture, or (e.g. where the moiety R¹ is unsubstituted or substituted alkyl) in the presence of a base, such as an alkali metal carbonate, such as potassium carbonate, if useful in the presence of an alkali metal halogenide, e.g. sodium or potassium iodide, in an appropriate solvent, such as dimethyl formamide, at preferably elevated temperatures, e.g. between 50 ° C and the reflux temperature of the mixture, or, where R¹ is to be bound via a carbonyl or sulfonyl group, under condensation conditions e.g. as described above under A) (i) a); the reactions can preferably take place under a protective gas, such as nitrogen or argon. The subsequent removal of (a) protecting group(s) takes place as described above under A) (i) (a).

The reaction under B) (i) between an aldehyde compound of the formula IX with an amino compound of the formula III preferably takes place under customary conditions for reductive amination, e.g. in the presence of an appropriate reducing (e.g. hydrogenation) agent, such as hydrogen in the presence of a catalyst or a complex hydride, e.g. sodium triacetoxyborohydride or sodium cyanoborhydride, in an appropriate solvent, such as a halogenated hydrocarbon, e.g. methylene chloride or 1,2,-dichloroethane, and optionally a carbonic acid, e.g. acetic acid, at preferred temperatures between -10 °C and 50 °C, e.g. from 0 °C to room temperature; the subsequent removal of protecting groups takes place e.g. as described above under A) (i) (a).

The reaction under B) (ii) between an aldehyde compound of the formula IX with an amino compound of the formula V takes place under customary conditions for reductive amination, e.g. as just described unter B) (i), the subsequent reaction under B) (ii)(1) between a thus obtainable compound of the formula X and a compound of the formula VIII under customary substitution conditions, e.g. as described above for reaction A) (ii) (b), the subsequent reaction under B) (ii) (II) under conditions as just described for reductive amination, and the removing of (a) protecting group(s) takes place e.g. as described above under A) (i) (a).

The reaction under C) (i) between a compound of the formula XI and a compound of the formula XII preferably takes place in the presence of a base, such as (especially in the case of unsubstituted or substituted alkyl, unsubstituted or substituted aryl, unsubstituted or substituted heterocyclyl, unsubstituted or substituted cycloalkyl, unsubstituted or substituted aryl-alkyl, unsubstituted or substituted heterocyclyl-alkyl or unsubstituted or substituted cycloalkyl R³) a strong base, e.g. an alkali metal hydride, such as sodium hydride, in the presence of an appropriate solvent, such as dimethylformamide, at preferred temperatures from room temperature to 90 °C, or (e.g. in the case of unsubstituted or substituted alkylsulfonyl, unsubstituted or substituted arylsulfonyl, unsubstituted or substituted heterocyclylsulfonyl or unsubstituted or substituted cycloalkylsulfonyl, unsubstituted or substituted aryl-carbonyl, unsubstituted or substituted heterocyclylcarbonyl or etherified carboxy) a tertiary nitrogen base, such as triethylamine, in the presence of an appropriate solvent, e.g. a halogenated hydrocarbon, such as methylene chloride, and/or a hydrocarbon, such as toluene, at preferred temperatures between 0 °C and 50 °C, e.g. at room temperature. Removal of protecting groups takes place preferably as described under A) (i) (a).

The reaction under C) (ii) preferably takes place under conditions analogous to those described for the reductive amination under B) above.

The oxidation under D) of a hydroxy compound of the formula XI to a corresponding oxo compound of the formula XIII preferably takes place in the presence of an appropriate oxidant, such as Dess-Martin-periodinane, in an appropriate solvent, e.g. a halogenated hydrocarbon, e.g. methylene chloride, at preferred temperatures from 0 °C to 50 °C, e.g. at room temperature. The optional subsequent conversion of an oxo group into a thioxo group (=S) can take place in the presence of Lawesson's reagent or under under customary thionation conditions, the conversion of oxo into an (unsubstituted or substituted) imino by reaction with protected ammonia (for unsubstituted imino) or a primary amine corresponding to a substituted imino to be introduced under customary Schiff base formation conditions. Removal of protecting groups takes place preferably as described under A) (i) (a).

The coupling under E) between a metallo reagent of the formula XIV and a compound of the formula XIII takes place under customary reaction conditions, e.g. under Grignard coupling conditions, in an appropriate solvent, e.g. an ether, such as diethyl ether, at preferred temperatures in the range from -100 to -50 °C, e.g. at -80 to -70 °C. Removal of protecting groups takes place preferably as described under A) (i) (a).

The synthesis of a spiro compound under F) from a compound of the formula XV preferably takes place in the presence of a strong base, such as an alkali metal hydride, e.g. sodium hydride, in an appropriate solvent, such as dimethylformamide, preferably at elevated temperatures, e.g. from 80 to 120 °C, such as 110 °C. Removal of protecting groups takes place preferably as described under A) (i) (a). That R³ in a compound of the formula carries a leaving group, means that in addition to normal substituents of R³ a leaving group, such as halogen or C₁-C₇-alkylsulfonyl or the like, is present.

The reaction under G) preferably takes place in the presence of a Lewis Acid, such as aluminium chloride, in an appropriate solvent, such as diethylether, at preferred temperatures from 0 to 50°C. Removal of protecting groups takes place as described above or below, especially as described under the general process conditions.

For reaction H), a leaving group present instead of -L-H in a compound of the formula XI, the leaving group is preferably halo or more preferably an organic sulfonyl moiety, such as C₁-C₇-alkylsulfonyl, and the reaction can, for example, take place in an appropriate solvent, such as dimethylformamide, at preferred temperatures from 0 to 50 °C. Removal of protecting groups takes place as described above or below, especially as described under the general process conditions.

### Optional Reactions and Conversions

Compounds of the formula I, or protected forms thereof directly obtained according to any one of the preceding procedures or after introducing protecting groups anew, which are included subsequently as starting materials for conversions as well even if not mentioned specifically, can be converted into different compounds of the formula I according to known procedures, where required after removal of protecting groups.

For example, a lower alkoxy (especially methoxy) group present as a substituent of an aryl moiety in a compound of the formula I (e.g. as part of R¹) can be converted into the corresponding hydroxy substituent by reaction, e.g., with boron tribromide in an appropriate solvent, e.g. a halogenated hydrocarbon, at preferred temperatures in the range from -100 to -50 °C, e.g. at -80 to -70 °C, yielding the corresponding hydroxy compound of the formula I.

In an acyl moiety R¹ of a carbonic acid bound via a carbonyl group to the nitrogen in formula I binding R¹, the carbonyl can be reduced to a methylene by treatment with a complex hydride, especially borane dimethylsulfide complex, under reaction conditions as described above for process variant A) (i), yielding a corresponding compound of the formula I.

A cyano group present as substituent on a compound of the formula I can be converted into an aminomethyl group e.g. by hydrogenation in the presence of a catalyst, such as a transition metal catalyst, e.g. Raney-Nickel, under customary conditions, e.g. in an alcohol, such as methanol, at preferred temperatures between 0 °C and 50 °C, e.g. at room temperature, to yield the corresponding amino compound of the formula I, yielding a corresponding compound of the formula I.

An amino group present as a substituent on a compound of the formula I can be converted into an acyl(especially lower-alkanoyl)-amino group e.g. by acylation with a carbonic or sulfonic acid, or a reactive derivative thereof, e.g. the corresponding acid halogenide, such as the acid chloride, or under *in situ* formation of the corresponding active derivative, under conditions analogous to those described above under A) (i), yielding the corresponding acylamino compound of the formula I.

An amino group present as a substituent on a compound of the formula I can be converted into an N,N-di-(C₁-C₇-alkyl)- or N,N-di-(phenyl- or naphthyl-C₁-C₇-alkyl)-amino group by alkylation e.g. with a corresponding N,N-di-(C₁-C₇-alkyl)- or N,N-di-(phenyl- or naphthyl-C₁-C₇-alkyl)-halogenide, e.g. -chloride or -bromide, or by reductive amination with a corresponding oxo compound (wherein one of the methylene groups in the C₁-C₇-alkyl-comprising compound used as precursor carries oxo instead of two hydrogen atoms) under conditions of reductive amination, e.g. analogous to those described under process variant B) (i) described above, yielding a corresponding compound of the formula I.

A nitro group present as substituent on a compound of the formula I can be converted into an amino group e.g. by hydrogenation in the presence of a catalyst, such as a transition metal catalyst, e.g. Raney-Nickel, under customary conditions, e.g. in an alcohol, such as methanol, at preferred temperatures between 0 °C and 50 °C, e.g. at room temperature, to yield the corresponding amino compound of the formula I, yielding a corresponding compound of the formula I.

A hydroxy group present as a substituent in a compound of the formula I can be converted into an alkylated or acylated hydroxy group, e.g. C₁-C₇-alkoxy-C₁-C₇-alkoxy, C₁-C₇-alkoxy or phenyl- or naphthyl-C₁-C₇-alkyloxy, by reaction with a corresponding alkylhalogenide or acyl-halogenide, e.g. a C₁-C₇-alkoxy-C₁-C₇-alkylchloride or -bromide, a C₁-C₇-alkylchloride or - bromide or a phenyl- or naphthyl-C₁-C₇-alkyl-chloride or -bromide, under appropriate customary substitution reaction conditions, e.g. in the presence of a base, such as an alkali metal carbonate, e.g. potassium carbonate, or a strong base, such as an alkali metal hydride, e.g. sodium hydride, in an appropriate solvent, e.g. an amide, such as dimethylformamide, at preferred temperatures from 0 to 100 °C, e.g. from room temperature to 80 °C, yielding a corresponding compound of the formula I.

An imino group in a compound of the formula I, e.g. -NH- as part of a substituent in a compound of the formula I comprising an N-heterocyclic moiety, can be transformed into a C₁-C₇-alkoxy-C₁-C₇-alkylimino group by reaction with a C₁-C₇-alkoxy-C₁-C₇-alkylhalogenide, e.g. chloride or bromide, under reaction conditions as described in the directly preceding paragraph, yielding a corresponding compound of the formula I.

An amino group L-R³ of a compound of the formula I can be converted into an unsubstituted or substituted alkylamino (e.g. C₁-C₇-alkylamino, such as isopropylamino), unsubstituted or substituted cycloalkylamino (e.g. cyclohexylamino), unsubstituted or substituted aryl-alkylamino, unsubstituted or substituted heterocyclyl-alkylamino, unsubstituted or substituted cycloalkyl-alkylamino, alkyloxycarbonylamino, alkylcarbonylamino, substituted or unsubstituted alkylsulfonylamino, substituted or unsubstituted arylsulfonylamino (such as C₁-C₇-alkyl-phenylsulfonyl, e.g. tosyl), substituted or unsubstituted heterocyclylsulfonylamino or substituted or unsubstituted cycloalkylsulfonylamino by reaction with the corresponding unsubstituted or substituted alkane, unsubstituted or substituted cycloalkane, unsubstituted or substituted aryl-alkane, unsubstituted or substituted heterocyclyl-alkane, unsubstituted or substituted cycloalkyl-alkane carrying a keto group instead of a methylene or a formyl group instead of a methyl in the alkyl part, under customary reaction conditions for reductive amination, e.g. as described above under B) (i); or by reaction with a substituted or unsubstituted alylsulfonylhalogenide, substituted or unsubstituted arylsulfonylhalogenide, substituted or unsubstituted heterocyclylsulfonylhalogenide or substituted or unsubstituted cycloalkylsulfonylhalogenide under customary reaction conditions, e.g. in the presence of a tertiary amine, such as triethylamine, in an appropriate solvent, e.g. a halogenated hydrocarbon, such as methylene chloride, at preferred temperatures from 0 °C to 50 °C, e.g. at room temperature; yielding a corresponding compound of the formula I.

Salts of compounds of formula I having at least one salt-forming group may be prepared in a manner known per se. For example, salts of compounds of formula I having acid groups may be formed, for example, by treating the compounds with metal compounds, such as alkali metal salts of suitable organic carboxylic acids, e.g. the sodium salt of 2-ethylhexanoic acid, with organic alkali metal or alkaline earth metal compounds, such as the corresponding hydroxides, carbonates or hydrogen carbonates, such as sodium or potassium hydroxide, carbonate or hydrogen carbonate, with corresponding calcium compounds or with ammonia or a suitable organic amine, stoichiometric amounts or only a small excess of the salt-forming agent preferably being used. Acid addition salts of compounds of formula I are obtained in customary manner, e.g. by treating the compounds with an acid or a suitable anion exchange reagent. Internal salts of compounds of formula I containing acid and basic salt-forming groups, e.g. a free carboxy group and a free amino group, may be formed, e.g. by the neutralisation of salts, such as acid addition salts, to the isoelectric point, e.g. with weak bases, or by treatment with ion exchangers.

A salt of a compound of the formula I can be converted in customary manner into the free compound; metal and ammonium salts can be converted, for example, by treatment with suitable acids, and acid addition salts, for example, by treatment with a suitable basic agent. In both cases, suitable ion exchangers may be used.

Stereoisomeric mixtures, e.g. mixtures of diastereomers, can be separated into their corresponding isomers in a manner known per se by means of appropriate separation methods. Diastereomeric mixtures for example may be separated into their individual diastereomers by means of fractionated crystallization, chromatography, solvent distribution, and similar procedures. This separation may take place either at the level of one of the starting compounds or in a compound of formula I itself. Enantiomers may be separated through the formation of diastereomeric salts, for example by salt formation with an enantiomer-pure chiral acid, or by chromatography, for example by HPLC, using chromatographic substrates with chiral ligands.

Intermediates and final products can be worked up and/or purified according to customary methods, e.g. using chromatographic methods, distribution methods, (re-) crystallization, and the like.

### Starting Materials

Starting Materials, including intermediates, for compounds of the formula I, such as the compounds of the formulae II, III, IV, V, VI, VII, VIII, VIII*, IX, X, XI, XII, XIIA, XIIB, XIIC, XIII, XIV and/or XV and the like, can be prepared, for example, according to methods that are known in the art, according to methods described in the examples or methods analogous to those described in the examples, and/or they are known or commercially available.

In the subsequent description of starting materials and intermediates and their synthesis, R¹, R¹*, R², R³, R⁴, T, L and PG have the meanings given above or in the Examples for the respecive starting materials or intermediates, if not indicated otherwise directly or by the context. Protecting groups, if not specifically mentioned, can be introduced and removed at appropriate steps in order to prevent functional groups, the reaction of which is not desired in the corresponding reaction step or steps, employing protecting groups, methods for their introduction and their removal are as described above or below, e.g. in the references mentioned under "General Process Conditions".

A compound of the formula II wherein L is methylene can, for example, be obtained by reacting a compound of the formula XVI,

PG-NH-CH₂-CH₂-CN (XVI)

wherein PG is a protecting group, especially benzyl, with a compound of the formula XVII,

R³-CH=CH-CH₂-Hal (XVII)

wherein Hal is halo, such as bromo, or a different leaving group, such as tosyl, in the presence of a base, such as an alkali metal hydroxide, e.g. NaOH, and e.g. benzyl-tri-(N-butyl)ammonium bromide, in an appropriate solvent, e.g. a halogenated hydrocarbon, such as methylene chloride, and/or water, preferably at a temperature from 10 to 50 °C, e.g. 40 °C, treating the resulting compound of the formula XVIII,

R³-CH=CH-CH₂-N(PG)-CH₂-CH₂-CN (XVIII)

wherein the substituents have the meanings just described in the presence of a strong base, such as sodium hydride, in an appropriate solvent, e.g. hexamethylphosphoroamide, at preferred temperatures between -10 and 40 °C, thus obtaining a compound of the formula XIX, 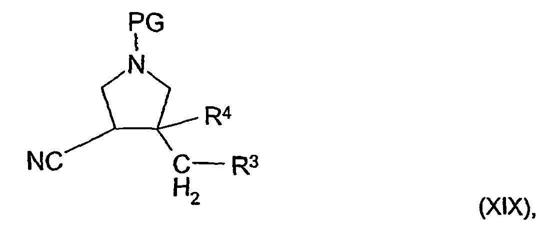 which is then hydrolyzed, e.g. in the presence of a hydrohalic acid, such as HCI, in an appropriate solvent, e.g. acetic acid, water or a mixture thereof, at elevated temperatures, e.g. under reflux, to the corresponding compound of the formula II.

A compound of the formula XII wherein instead of the group -L-H a leaving group is present and which thus has the formula XX, 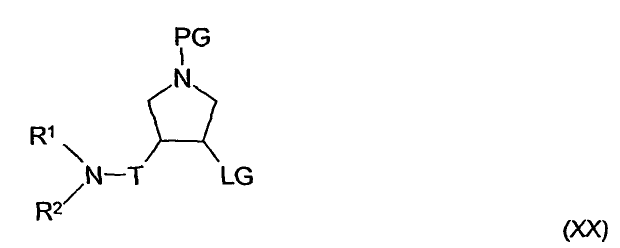 wherein LG is a leaving group, especially as described under process variant H) above, can, for example, be obtained by reacting a compound of the formula XXI, 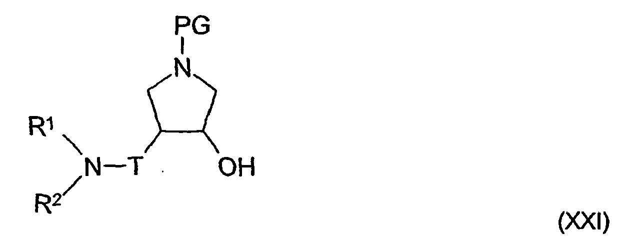 which is a special compound of the formula XII and can be obtained accordingly, in the presence of an appropriate base, e.g. a tertiary nitrogen base, such as triethylamine, in an appropriate solvent, such as a halohydrocarbon, e.g. methylene chloride, at customary temperatures, e.g. from 0 to 50 °C, with an organic sulfonylhalogenide, e.g. -chloride, to introduce an organic sulfonyl leaving group, or with a halogenating agent to introduce a halo group as leaving group, respectively.

A compound of the formula IX can be obtained from a compound of the formula II, e.g. one described in the last paragraph, by first reducing the carboxy function in the presence of an appropriate complex hydride, e.g. borane dimethylsulfide, in an appropriate solvent, e.g. tetrahydrofurane, at preferred temperatures between -20 and 40 °C, to the corresponding hydroxymethylene compound of the formula XXII, 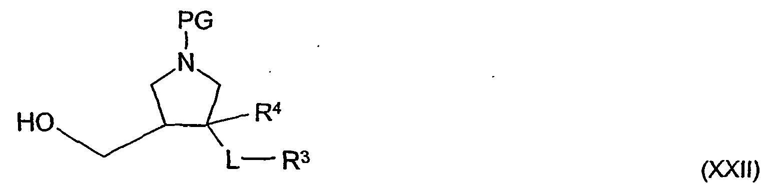 which is then oxidized to the aldehyde of the formula IX, for example in the presence of Dess Martin periodinane e.g. in methylene chloride and/or water or of 2,2,6,6,-tetramethyl-1-piperidinyloxy free radical e.g. in toluene and/or ethyl acetate in the presence of potassium bromide, water and potassium hydrogencarbonate, at preferred temperatures in the range from 0 to 50 °C.

An aldehyde of the formula VIII* wherein R¹* is aryl that is substituted by C₁-C₇-alkyloxy-C₁-C₇-alkyloxy (and possibly other substituents are present) is, for example, obtained by reacting a corresponding hydroxy substituted aryl with a C₁-C₇-alkyloxy-C₁-C₇-alkanol in the presence of triphenylphosphine and a solvent, e.g. tetrahydrofurane, and diethyl diethyl azodicarboxylate at preferred temperatures between 0 and 50 °C.

A compound of the formula XI can, for example, be prepared as follows: A compound of the formula XXIII, 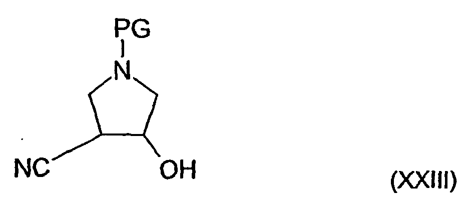 obtainable e.g. as described in the literature (see e.g. J. Med. Chem. 1997, 40, 3584) is first protected, e.g. by introduction of a t-butyldimethylsilyl (TBDMS) protecting group in the presence of imidazole and dimethylformamide at preferred temperatures between 0 and 50 °C.

The obtainable O-protected compound is then treated to reduce the cyano group into a formyl group, e.g. with diisobutylaluminium hydride in toluene at low temperatures, e.g. from - 90 to -70 °C, to give a corresponding compound of the formula XXIV, 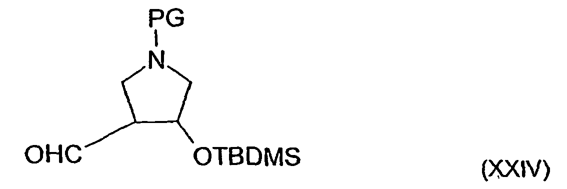 which is then treated first with a compound of the formula V as described above under reaction conditions as described under process B) (ii) above or analogous conditions, then a compound of the formula VIII as described above, under reaction conditions as described for the reaction described under B) (ii) above or analogous conditions, to give a compound of the formula XXV, 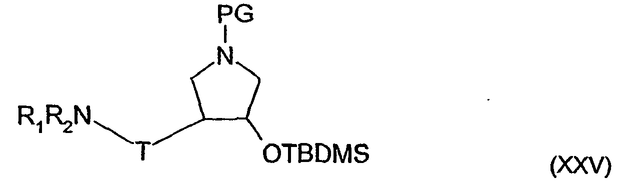 from which then the TBDMS group is removed, e.g. by reaction with tetra-butylammonium fluoride e.g. in tetrahydrofurane at 0 to 50 °C to give the compound of the formula XI.

A starting material of the formula II wherein R³ is unsubstituted or substituted aryl or aryl-alkyl, unsubstituted or substituted heterocyclyl or heterocyclyl-alkyl, unsubstituted or substituted cycloalkyl or cycloalkyl-alkyl, or unsubstituted or substituted alkyl, preferably as defined above, and L is absent or methylene, can be obtained by reacting a compound of the formula XXVI,

R³-L-CHO (XXVI)

wherein R³ and L are as just defined, with a compound of the formula XXVII, 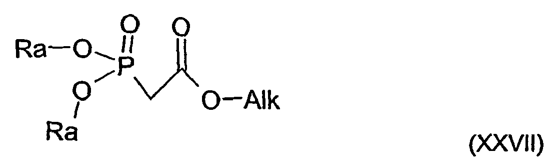 wherein Ra is ethyl or 2,2,2-trifluoroethyl and Alk is lower alkyl, in the presence of a strong base, e.g. sodium hydride e.g. in tetrahydrofurane at preferred temperatures in the range from -10 to 40 °C, or in the presence of potassium hexamethyldisiliazane and a crown ether, e.g. 18-crown-6, e.g. in tetrahydrofurane and/or toluene at low temperatures, e.g. from -90 to -70 °C, to give a compound of the formula XXVIII,

R³-(CH₂)_{0 or 1}-CH=CH-COOAlk (XXVIII)

wherein R³ and Alk are as just defined, which alternatively (especially if L is absent) can also be obtained by reaction of a compound of the formula R³-Hal, wherein R³ is as defined and Hal is halogen, with an ester of acrylic acid, e.g. the methyl ester, in the presence of an appropriate base, e.g. triethylamine, and a catalyst, such as Pd(Oac)₂, in an appropriate solvent, such as dimethylformamide;
which compound of the formula XVIII is then reacted with a compound of the formula XXIX,

(H₃C)₃Si-CH₂-N(PG)-CH₂-O-CH₃ (XXIX)

wherein PG is a protecting group as defined e.g. for a compound of the formula II, in the presence of an acid, e.g. trifluoroacetic acid, in an appropriate solvent, e.g. toluene, at preferred temperatures between -10 and 40 °C, to give a compound of the formula XXX, 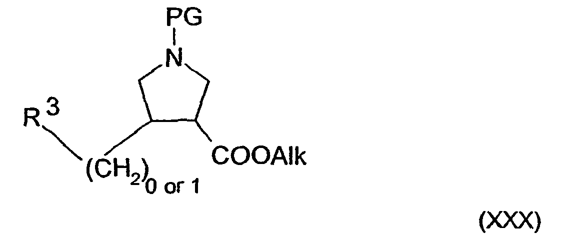 wherein R³ and Alk are as just defined (if desired, the protecting group PG may be replaced by a different protecting group, e.g. benzyl by tert-butoxycarbonyl), and then hydrolysis to remove the Alk-goup to give the corresponding free acid of the formula II or reduction, e.g. with lithium aluminium chloride in tentrahydrofurane and followd by oxidation under Dess-Martin-conditions to the corresponding aldehyde of the formula IX which can thus also be obatined.

A corresponding compound of the formula IX can be obtained by reducing the carboxy function in a compound of the formula II as obtained in the preceding paragraph, e.g. in the presence of borane dimethylsulfide complex in e.g. tetrahydrofurane at from -20 °C to 40 °C, to the corresponding hydroxymethyl function and oxidation of this to the corresponding formyl function, e.g. with Dess-Martin periodinane e.g. in wet methylenechloride at temperatures from 0 to 50 °C.

A compound of the formula IX wherein wherein L is O and R³ is as defined for compounds of the formula IX, especially unsubstituted or substituted aryl, can be obtained by reacting a compound of the formula XXXI,

HO-R³ (XXXI)

wherein R³ is as just defined, with a compound of the formula XXXII,

AlkO-C(=O)-C≡CH (XXXII)

in the presence of a tertiary nitrogen base, e.g. N-methylmorpholine, in an appropriate solvent, e.g. tetrahydrofurane, at preferred temperatures from -10 to 50 °C, to give a compound of the formula XXXIII,

AlkO-C(=O)-CH=CH-O-R³ (XXXIII)

wherein Alk and R³ are as defined; which is then, under reaction conditions as described above for the reaction of a compound of the formula XXVIII and one of the formula XXIX, reacted with a compound of the formula XXVIII as defined above to a corresponding pyrrolidine of the formula XXX*, 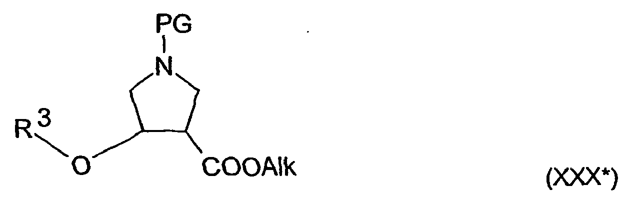 wherein Alk is as just defined and R³ is as defined above, which (after optional replacement of the protecting group PG by a different protecting group PG, e.g. of benzyl by tert-butoxycarbonyl), which is a compound of the formula II that can thus be obtained, which can then be reduced to the corresponding compound with a hydroxymethylene instead of the group -COOAlk which can then be subjected to oxidation to the corresponding formyl function, e.g. with Dess-Martin periodinane e.g. in wet methylenechloride at temperatures from 0 to 50 °C, giving a corresponding compound of the formula IX.

A compound of the formula XI wherein L is NH can, for example, be prepared by reacting a compound of the formula XXIX, as defined above, e.g. in the presence of an acid, such as trifluoroacetic acid, in an appropriate solvent, e.g. methylene chloride, at preferred temperatures between -10 and 50 °C, with a carbonic acid of the formula XXXIV,

Alk-O-CO-CH=CH-COOH (XXXIV)

wherein Alk is e.g. lower alkyl, to a corresponding pyrrolidine of the formula XXXV, 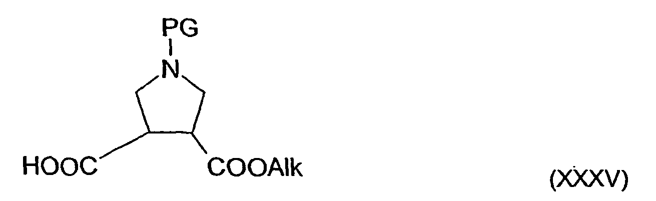 which can then, e.g. by treatment with diphenylphosphorus azide e.g. in dioxane and in the presence of a tertiary nitrogen base, e.g. triethylamine, at elevated temperatures, e.g. under reflux, and a tri-lower alkylsilyl ethanol, e.g. trimethylsilyl ethanol, to give the corresponding protected amino compound of the formula XXXVI, 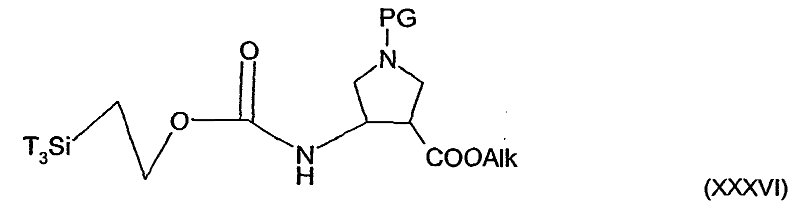 in which the COOAlk group is subsequently hydrolyzed, e.g. with an alkali metal hydroxide, such as sodium hydroxide or lithium hydroxide, in an appropriate solvent, e.g. an alcohol, such as methanol, at preferred temperatures from 0 to 50 °C, to give the corresponding free carboxy group which is then reduced to hydroxymethylene, e.g. with a complex hydride such as borane dimethyl sulphide complex, e.g. in THF and at -20 to 50 °C, which is then oxidised to formyl (-CHO), e.g. with Dess-Martin periodinane e.g. in wet methylene chloride at preferred temperatures from 0 to 50 °C to give a compound of the formula XXXVII, 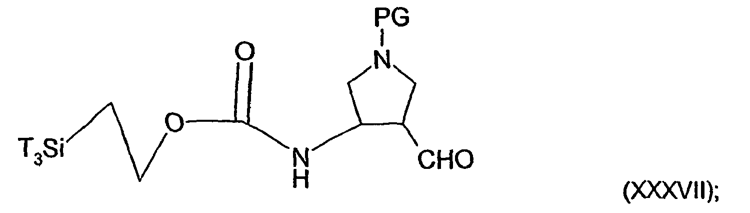 this compound can then be reacted with a compound of the formula V and then a compound of the formula VIII as defined above under reaction conditions such as those described above under process variant B) (ii) and subsequent removal of the tri-lower alkylsilylethoxy group e.g. with tetraethylammonium fluoride in a solvent, e.g. methylene chloride and/or acetoneitrile, at elevated temperatures, e.g. under reflux, to give an amino compound of the formula XXXVIII, 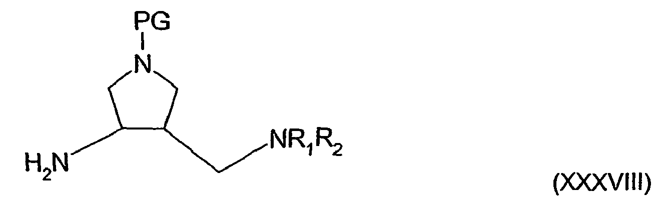 which is a compound of the formula XI wherein L is NH.

In all formulae above where present, the central pyrrolidine and its substituents at positions 3 and 4 may be present in any one ore more of the following configurations, and/or mixtures of the corresponding isomers may be formed and/or separated into the individual isomers at appropriate stages: 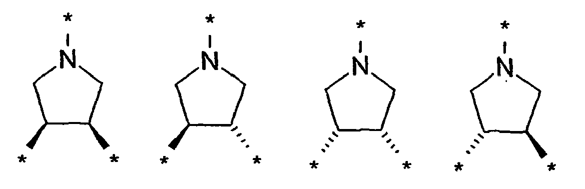 wherein the left lower bond is also on the left side in any of the formulae intermediates or starting materials as shown above or final products of the formula I, the right lower bond on the right side.

### General Process Conditions

The following applies in general to all processes mentioned hereinbefore and hereinafter, while reaction conditions specifically mentioned above or below are preferred:
In any of the reactions mentioned hereinbefore and hereinafter, protecting groups may be used where appropriate or desired, even if this is not mentioned specifically, to protect functional groups that are not intended to take part in a given reaction, and they can be introduced and/or removed at appropriate or desired stages. Reactions comprising the use of protecting groups are therefore included as possible wherever reactions without specific mentioning of protection and/or deprotection are described in this specification.

Within the scope of this disclosure only a readily removable group that is not a constituent of the particular desired end product of formula I is designated a "protecting group", unless the context indicates otherwise. The protection of functional groups by such protecting groups, the protectting groups themselves, and the reactions appropriate for their introduction and removal are described for example in standard reference works, such as J. F. W. McOmie, "Protective Groups in Organic Chemistry", Plenum Press, London and New York 1973, in T. W. Greene and P. G. M. Wuts, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of Organic Chemistry), Houben Weyl, 4th edition, Volume 15/l, Georg Thieme Verlag, Stuttgart 1974, in H.-D. Jakubke and H. Jeschkeit, "Aminosäuren, Peptide, Proteine" (Amino acids, Peptides, Proteins), Verlag Chemie, Weinheim, Deerfield Beach, and Basel 1982, and in Jochen Lehmann, "Chemie der Kohlenhydrate: Monosaccharide und Derivate" (Chemistry of Carbohydrates: Monosaccharides and Derivatives), Georg Thieme Verlag, Stuttgart 1974. A characteristic of protecting groups is that they can be removed readily (i.e. without the occurrence of undesired secondary reactions) for example by solvolysis, reduction, photolysis or alternatively under physiological conditions (e.g. by enzymatic cleavage).

All the above-mentioned process steps can be carried out under reaction conditions that are known per se, preferably those mentioned specifically, in the absence or, customarily, in the presence of solvents or diluents, preferably solvents or diluents that are inert towards the reagents used and dissolve them, in the absence or presence of catalysts, condensation or neutralizing agents, for example ion exchangers, such as cation exchangers, e.g. in the H⁺ form, depending on the nature of the reaction and/or of the reactants at reduced, normal or elevated temperature, for example in a temperature range of from about -100 °C to about 190°C, preferably from approximately -80°C to approximately 150°C, for example at from -80 to -60°C, at room temperature, at from -20 to 40 °C or at reflux temperature, under atmospheric pressure or in a closed vessel, where appropriate under pressure, and/or in an inert atmosphere, for example under an argon or nitrogen atmosphere.

The solvents from which those solvents that are suitable for any particular reaction may be selected include those mentioned specifically or, for example, water, esters, such as lower alkyl-lower alkanoates, for example ethyl acetate, ethers, such as aliphatic ethers, for example diethyl ether, or cyclic ethers, for example tetrahydrofurane or dioxane, liquid aromatic hydrocarbons, such as benzene or toluene, alcohols, such as methanol, ethanol or 1- or 2-propanol, nitriles, such as acetoneitrile, halogenated hydrocarbons, e.g. as methylene chloride or chloroform, acid amides, such as dimethylformamide or dimethyl acetamide, bases, such as heterocyclic nitrogen bases, for example pyridine or N-methylpyrrolidin-2-one, carboxylic acid anhydrides, such as lower alkanoic acid anhydrides, for example acetic anhydride, cyclic, linear or branched hydrocarbons, such as cyclohexane, hexane or isopentane, or mixtures of these, for example aqueous solutions, unless otherwise indicated in the description of the processes. Such solvent mixtures may also be used in working up, for example by chromatography or partitioning.

The invention relates also to those forms of the process in which a compound obtainable as intermediate at any stage of the process is used as starting material and the remaining process steps are carried out, or in which a starting material is formed under the reaction conditions or is used in the form of a derivative, for example in protected form or in the form of a salt, or a compound obtainable by the process according to the invention is produced under the process conditions and processed further in situ. In the process of the present invention those starting materials are preferably used which result in compounds of formula I described as being preferred. Special preference is given to reaction conditions that are identical or analogous to those mentioned in the Examples.

### Pharmaceutical use, pharmaceutical preparations and methods

As described above, the compounds of the present invention are inhibitors of renin activity and, thus, may be employed for the treatment of hypertension, atherosclerosis, unstable coronary syndrome, congestive heart failure, cardiac hypertrophy, cardiac fibrosis, cardiomyopathy postinfarction, unstable coronary syndrome, diastolic dysfunction, chronic kidney disease, hepatic fibrosis, complications resulting from diabetes, such as nephropathy, vasculopathy and neuropathy, diseases of the coronary vessels, restenosis following angioplasty, raised intra-ocular pressure, glaucoma, abnormal vascular growth, hyperaldosteronism, cognitive impairment, alzheimers, dementia, anxiety states and cognitive disorders, and the like.

The present invention further provides pharmaceutical compositions comprising a therapeutically effective amount of a pharmacologically active compound of the instant invention, alone or in combination with one or more pharmaceutically acceptable carriers.

The pharmaceutical compositions according to the present invention are those suitable for enteral, such as oral or rectal, transdermal and parenteral administration to mammals, including man, to inhibit renin activity, and for the treatment of conditions associated with (especially inappropriate) renin activity. Such conditions include hypertension, atherosclerosis, unstable coronary syndrome, congestive heart failure, cardiac hypertrophy, cardiac fibrosis, cardiomyopathy postinfarction, unstable coronary syndrome, diastolic dysfunction, chronic kidney disease, hepatic fibrosis, complications resulting from diabetes, such as nephropathy, vasculopathy and neuropathy, diseases of the coronary vessels, restenosis following angioplasty, raised intra-ocular pressure, glaucoma, abnormal vascular growth, hyperaldosteronism, cognitive impairment, alzheimers, dementia, anxiety states and cognitive disorders and the like.

Thus, the pharmacologically active compounds of the invention may be employed in the manufacture of pharmaceutical compositions comprising an effective amount thereof in conjunction or admixture with excipients or carriers suitable for either enteral or parenteral administration. Preferred are tablets and gelatin capsules comprising the active ingredient together with:
a) diluents, e.g., lactose, dextrose, sucrose, mannitol, sorbitol, cellulose and/or glycine;
b) lubricants, e.g., silica, talcum, stearic acid, its magnesium or calcium salt and/or polyethyleneglycol; for tablets also
c) binders, e.g., magnesium aluminum silicate, starch paste, gelatin, tragacanth, methylcellulose, sodium carboxymethylcellulose and or polyvinylpyrrolidone; if desired
d) disintegrants, e.g., starches, agar, alginic acid or its sodium salt, or effervescent mixtures; and/or
e) absorbants, colorants, flavors and sweeteners.

Injectable compositions are preferably aqueous isotonic solutions or suspensions, and suppositories are advantageously prepared from fatty emulsions or suspensions.

Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, preferably about 1-50%, of the active ingredient.

Suitable formulations for transdermal application include a therapeutically effective amount of a compound of the invention with carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the compound of the skin of the host at a controlled and pre-determined rate over a prolonged period of time, and means to secure the device to the skin.

Accordingly, the present invention provides pharmaceutical compositions as described above for the treatment of conditions mediated by renin activity, preferably, hypertension, atherosclerosis, unstable coronary syndrome, congestive heart failure, cardiac hypertrophy, cardiac fibrosis, cardiomyopathy postinfarction, unstable coronary syndrome, diastolic dysfunction, chronic kidney disease, hepatic fibrosis, complications resulting from diabetes, such as nephropathy, vasculopathy and neuropathy, diseases of the coronary vessels, restenosis following angioplasty, raised intra-ocular pressure, glaucoma, abnormal vascular growth, hyperaldosteronism, cognitive impairment, alzheimers, dementia, anxiety states and cognitive disorders, as well as methods of their use.

The pharmaceutical compositions may contain a therapeutically effective amount of a compound of the formula I as defined herein, either alone or in a combination with another therapeutic agent, e.g., each at an effective therapeutic dose as reported in the art. Such therapeutic agents include:
a) antidiabetic agents such as insulin, insulin derivatives and mimetics; insulin secretagogues such as the sulfonylureas, e.g., Glipizide, glyburide and Amaryl; insulinotropic sulfonylurea receptor ligands such as meglitinides, e.g., nateglinide and repaglinide; peroxisome proliferator-activated receptor (PPAR) ligands; protein tyrosine phosphatase-1B (PTP-1 B) inhibitors such as PTP-112; GSK3 (glycogen synthase kinase-3) inhibitors such as SB-517955, SB-4195052, SB-216763, NN-57-05441 and NN-57-05445; RXR ligands such as GW-0791 and AGN-194204; sodium-dependent glucose cotransporter inhibitors such as T-1095; glycogen phosphorylase A inhibitors such as BAY R3401; biguanides such as metformin; alpha-glucosidase inhibitors such as acarbose; GLP-1 (glucagon like peptide-1), GLP-1 analogs such as Exendin-4 and GLP-1 mimetics; and DPPIV (dipeptidyl peptidase IV) inhibitors such as LAF237;
b) hypolipidemic agents such as 3-hydroxy-3-methyl-glutaryl coenzyme A (HMG-CoA) reductase inhibitors, e.g., lovastatin, pitavastatin, simvastatin, pravastatin, cerivastatin, mevastatin, velostatin, fluvastatin, dalvastatin, atorvastatin, rosuvastatin and rivastatin; squalene synthase inhibitors; FXR (famesoid X receptor) and LXR (liver X receptor) ligands; cholestyramine; fibrates; nicotinic acid and aspirin;
c) anti-obesity agents such as orlistat; and
d) anti-hypertensive agents, e.g., loop diuretics such as ethacrynic acid, furosemide and torsemide; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase (NEP) inhibittors; ACE/NEP inhibitors such as omapatrilat, sampatrilat and fasidotril; angiotensin II antagonists such as candesartan, eprosartan, irbesartan, losartan, telmisartan and valsartan, in particular valsartan; β-adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amLodipine, bepridil, diltiazem, felodipine, nicardipine, nimodipine, nifedipine, nisoldipine and verapamil; aldosterone receptor antagonists; and aldosterone synthase inhibitors.

Other specific anti-diabetic compounds are described by Patel Mona in Expert Opin Investig Drugs, 2003, 12(4), 623-633, in the figures 1 to 7, which are herein incorporated by reference. A compound of the present invention may be administered either simultaneously, before or after the other active ingredient, either separately by the same or different route of administration or together in the same pharmaceutical formulation.

The structure of the therapeutic agents identified by code numbers, generic or trade names may be taken from the actual edition of the standard compendium "The Merck Index" or from databases, e.g., Patents International (e.g. IMS World Publications). The corresponding content thereof is hereby incorporated by reference.

Accordingly, the present invention provides pharmaceutical compositions comprising a therapeutically effective amount of a compound of the invention alone or in combination with a therapeutically effective amount of another therapeutic agent, preferably selected from antidiabetics, hypolipidemic agents, anti-obesity agents or anti-hypertensive agents, most preferably from antidiabetics, anti-hypertensive agents or hypolipidemic agents as described above.

The present invention further relates to pharmaceutical compositions as described above for use as a medicament.

The present invention further relates to use of pharmaceutical compositions or combinations as described above for the preparation of a medicament for the treatment of conditions mediated by (especially inappropriate) renin activity, preferably, hypertension, atherosclerosis, unstable coronary syndrome, congestive heart failure, cardiac hypertrophy, cardiac fibrosis, cardiomyopathy postinfarction, unstable coronary syndrome, diastolic dysfunction, chronic kidney disease, hepatic fibrosis, complications resulting from diabetes, such as nephropathy, vasculopathy and neuropathy, diseases of the coronary vessels, restenosis following angioplasty, raised intra-ocular pressure, glaucoma, abnormal vascular growth, hyperaldosteronism, cognitive impairment, alzheimers, dementia, anxiety states and cognitive disorders, and the like.

Thus, the present invention also relates to a compound of formula I for use as a medicament, to the use of a compound of formula I for the preparation of a pharmaceutical composition for the prevention and/or treatment of conditions mediated by (especially inappropriate) renin activity, and to a pharmaceutical composition for use in conditions mediated by (especially inappropriate) renin activity comprising a compound of formula I, or a pharmaceutically acceptable salt thereof, in association with a pharmaceutically acceptable diluent or carrier material therefore.

The present invention further considers a method for the prevention and/or treatment of conditions mediated by (especially inappropriate) renin activity, which comprises administering a therapeutically effective amount of a compound of the present invention to a warm-blooded animal, especially a human, in need of such treatment.

A unit dosage for a mammal of about 50-70 kg may contain between about 1 mg and 1000 mg, advantageously between about 5-600 mg of the active ingredient. The therapeutically effective dosage of active compound is dependent on the species of warm-blooded animal (especially mammal, more especially human), the body weight, age and individual condition, on the form of administration, and on the compound involved.

In accordance with the foregoing the present invention also provides a therapeutic combination, e.g., a kit, kit of parts, e.g., for use in any method as defined herein, comprising a compound of formula I, or a pharmaceutically acceptable salt thereof, to be used concomitantly or in sequence with at least one pharmaceutical composition comprising at least another therapeutic agent, preferably selected from anti-diabetic agents, hypolipidemic agents, anti-obesity agents or anti-hypertensive agents. The kit may comprise instructions for its administration.

Similarly, the present invention provides a kit of parts comprising: (i) a pharmaceutical composition comprising a compound of the formula I according to the invention; and (ii) a pharmaceutical composition comprising a compound selected from an anti-diabetic, a hypolipidemic agent, an anti-obesity agent, an anti-hypertensive agent, or a pharmaceutically acceptable salt thereof, in the form of two separate units of the components (i) to (ii).

Likewise, the present invention considers a method as defined above comprising co-administration, e.g., concomitantly or in sequence, of a therapeutically effective amount of a compound of formula I, or a pharmaceutically acceptable salt thereof, and at least a second drug substance, said second drug substance preferably being an anti-diabetic, a hypolipidemic agent, an anti-obesity agent or an anti-hypertensive agent, e.g., as indicated above.

Preferably, a compound of the invention is administered to a mammal in need thereof.

Preferably, a compound of the invention is used for the treatment of a disease which responds to a modulation of (especially inappropriate) renin activity.

Preferably, the condition associated with (especially inappropriate) renin activity is selected from hypertension, atherosclerosis, unstable coronary syndrome, congestive heart failure, cardiac hypertrophy, cardiac fibrosis, cardiomyopathy postinfarction, unstable coronary syndrome, diastolic dysfunction, chronic kidney disease, hepatic fibrosis, complications resulting from diabetes, such as nephropathy, vasculopathy and neuropathy, diseases of the coronary vessels, restenosis following angioplasty, raised intra-ocular pressure, glaucoma, abnormal vascular growth, hyperaldosteronism, cognitive impairment, alzheimers, dementia, anxiety states and cognitive disorders.

Finally, the present invention considers a method or use which comprises administering a compound of formula I in combination with a therapeutically effective amount of an anti-diabetic agent, a hypolipidemic agent, an anti-obesity agent or an anti-hypertensive agent.

Ultimately, the present invention provides a method or use which comprises administering a compound of formula I in the form of a pharmaceutical composition as described herein.

The above-cited properties are demonstrable *in vitro* and *in vivo* tests using advantageously mammals, e.g., mice, rats, rabbits, dogs, monkeys or isolated organs, tissues and preparations thereof. Said compounds can be applied *in vitro* in the form of solutions, e.g., preferably aqueous solutions, and *in vivo* either enterally, parenterally, advantageously intravenously, e.g., as a suspension or in aqueous solution. The concentration level *in vitro* may range between about 10⁻³ molar and 10⁻¹⁰ molar concentrations. A therapeutically effective amount *in vivo* may range depending on the route of administration, between about 0.001 and 500 mg/kg, preferably between about 0.1 and 100 mg/kg.

As described above, the compounds of the present invention have enzyme-inhibiting properties. In particular, they inhibit the action of the natural enzyme renin. Renin passes from the kidneys into the blood where it effects the cleavage of angiotensinogen, releasing the deca-peptide angiotensin I which is then cleaved in the lungs, the kidneys and other organs to form the octapeptide angiotensin II. The octapeptide increases blood pressure both directly by arterial vasoconstriction and indirectly by liberating from the adrenal glands the sodium-ion-retaining hormone aldosterone, accompanied by an increase in extracellular fluid volume which increase can be attributed to the action of angiotensin II. Inhibitors of the enzymatic activity of renin lead to a reduction in the formation of angiotensin I, and consequently a smaller amount of angiotensin II is produced. The reduced concentration of that active peptide hormone is a direct cause of the hypotensive effect of renin inhibitors.

The action of renin inhibitors may be demonstrated *inter alia* experimentally by means of *in vitro* tests, the reduction in the formation of angiotensin I being measured in various systems (human plasma, purified human renin together with synthetic or natural renin substrate).

*Inter alia* the following *in vitro* tests may be used:
Recombinant human renin (expressed in Chinese Hamster Ovary cells and purified using standard methods) at 7.5 nM concentration is incubated with test compound at various concentrations for 1 h at RT in 0.1 M Tris-HCl buffer, pH 7.4, containing 0.05 M NaCl, 0.5 mM EDTA and 0.05 % CHAPS. Synthetic peptide substrate Arg-Glu(EDANS)-Ile-His-Pro-Phe-His-Leu-Val-Ile-His_Thr-Lys(DABCYL)-Arg9 is added to a final concentration of 2 µM and increase in fluorescence is recorded at an excitation wave-length of 350 nm and at an emission wave-length of 500 nm in a microplate spectro-fluorimeter. IC50 values are calculated from percentage of inhibition of renin activity as a function of test compound concentration (Fluorescence Resonance Energy Transfer, FRET, assay). Compounds of the formula I, in this assay, preferably show IC₅₀ values in the range from 10 nM to 20 µM

Alternatively, recombinant human renin (expressed in Chinese Hamster Ovary cells and purified using standard methods) at 0.5 nM concentration is incubated with test compound at various concentrations for 2 h at 37°C in 0.1 M Tris-HCl buffer, pH 7.4, containing 0.05 M NaCl, 0.5 mM EDTA and 0.05 % CHAPS. Synthetic peptide substrate Arg-Glu(EDANS)-Ile-His-Pro-Phe-His-Leu-Val-Ile_His_Thr-Lys(DABCYL)-Arg9 is added to a final concentration of 4 µM and increase in fluorescence is recorded at an excitation wave-length of 340 nm and at an emission wave-length of 485 nm in a microplate spectro-fluorimeter. IC50 values are calculated from percentage of inhibition of renin activity as a function of test compound concentration (Fluorescence Resonance Energy Transfer, FRET, assay). Compounds of the formula I, in this assay, preferably show IC₅₀ values in the range from 10 nM to 20 µM.

In another assay, human plasma spiked with recombinant human renin (expressed in Chinese Hamster Ovary cells and purified using standard methods) at 0.8 nM concentration is incubated with test compound at various concentrations for 2 h at 37°C in 0.1 M Tris/HCl pH 7.4 containing 0.05 M NaCl, 0.5 mM EDTA and 0.025% (w/v) CHAPS. Synthetic peptide substrate Ac-Ile-His-Pro-Phe-His-Leu-Val-Ile-His-Asn-Lys-[DY-505-X5] is added to a final concentration of 2.5 µM. The enzyme reaction is stopped by adding an excess of a blocking inhibitor. The product of the reaction is separated by capillary electrophoresis and quantified by spectrophotometric measurement at 505 nM wave-length. IC50 values are calculated from percentage of inhibition of renin activity as a function of test compound concentration. Compounds of the formula I, in this assay, preferably show IC₅₀ values in the range from 10 nM to 20 µM.

In another assay, recombinant human renin (expressed in Chinese Hamster Ovary cells and purified using standard methods) at 0.8 nM concentration is incubated with test compound at various concentrations for 2 h at 37°C in 0.1 M Tris/HCl pH 7.4 containing 0.05 M NaCl, 0.5 mM EDTA and 0.025% (w/v) CHAPS. Synthetic peptide substrate Ac-Ile-His-Pro-Phe-His-Leu-Val-Ile-His-Asn-Lys-[DY-505-X5] is added to a final concentration of 2.5 µM. The enzyme reaction is stopped by adding an excess of a blocking inhibitor. The product of the reaction is separated by capillary electrophoresis and quantified by spectrophotometric measurement at 505 nM wave-length. IC50 values are calculated from percentage of inhibition of renin activity as a function of test compound concentration. Compounds of the formula I, in this assay, preferably show IC₅₀ values in the range from 10 nM to 20 µM.

In animals deficient in salt, renin inhibitors bring about a reduction in blood pressure. Human renin may differ from the renin of other species. In order to test inhibitors of human renin, primates, e.g.,marmosets (Callithrix jacchus) may be used, because human renin and primate renin are substantially homologous in the enzymatically active region. *Inter alia* the following *in vivo* tests may be used:
Compounds can be tested in vivo in primates as described in the literature (see for example by Schnell CR et al. Measurement of blood pressure and heart rate by telemetry in conscious, unrestrained marmosets. Am J Physiol 264 (Heart Circ Physiol 33). 1993: 1509-1516; or Schnell CR et al. Measurement of blood pressure, heart rate, body temperature, ECG and activity by telemetry in conscious, unrestrained marmosets. Proceedings of the fifth FELASA symposium: Welfare and Science. Eds BRIGHTON. 1993.

The following Examples, while representing preferred embodiments of the invention, serve to illustrate the invention without limiting its scope.

### Abbreviations:

- abs.: Absolute
- Ac: acetyl
- AcOEt: ethyl acetate
- AcOH: acetic acid
- aq: aqueous
- Ar: phenyl (Scheme1);
left phenyl, right 4-chloro-3-methoxyphenyl (Scheme8);
3-cyanophenyl (Scheme10);
4-chlorophenyl (Scheme23)
- ARX: 4-fluorobenzotrifuloride (Scheme24)
- BINAP: 2,2'-Bis(diphenylphosphino)-1,1'-binaphthyl
- Bn: benzyl
- Bu: butyl (nBu = n-butyl, tBu = tert-butyl)
- cc: concentrated
- c-hexane: cyclohexane
- DIBAL-H: diisobutylaluminium hydride
- DMF: dimethylformamide
- DMSO: dimethylsulfoxide
- DPPA: diphenylphosphoryl azide
- EDCI: 1-(3-dimethylaminopropyl)-3-ethylcarbodiimide hydrochloride
- Ether: diethylether
- Et₃N: triethylamine
- Et₂O: diethylether
- EtOH: ethanol
- Flow: flow rate
- h: hour(s)
- HMPA: hexamethylphosphoroamide
- HOBt: 1-hydroxybenzotriazole
- HPLC: High Performance Liquid Chromatography
- iPrOH: isopropanol
- L: liter(s)
- KHMDS: potassium hexamethyldisilazane
- LC-MS: Liquid Chromatography/Mass Spectrometry
- LDA: lithium diisopropylamine
- Me: methyl
- Mel: methyl iodide
- MeOH: methanol
- Min: minute(s)
- ML: milliliter
- MS: Mass Spectrometry
- NMM: 4-methylmorpholine
- NMR: Nuclear Magnetic Resonance
- Pd/C: palladium on charcoal
- Ph: phenyl
- PyBOP: (benzotriazol-1-yloxy)-tripyrrolidinophosphonium- hexafluorophosphate
- R: benzyl (Scheme3);
left 4-chlorophenyl, right 3-cyanobenzyl (Scheme9);
4-methoxy-3-(3-methoxypropoxy)-phenyl (Scheme10);
3-(3-methoxy-propoxy)-4-methyl-phenyl (Scheme11a);
4-methoxy-3-(3-methoxy-propoxy)-phenyl (scheme 11b);
methyl (Scheme13);
4-methoxy-3-(3-methoxy-propoxy)-phenyl (Scheme16);
4-methoxy-3-(2-benzyloxy-ethoxy)-phenyl (Scheme17 in upper line);
isopropyl (Scheme23 left lane 2 from bottom), 4- methoxy-3-(3-methoxy-propoxy)-phenyl (Scheme23 right lane 2 from bottom);
hydrogen (Scheme31);
4-methoxy-3-(3-methoxy-propoxy)-phenyl (Scheme32 left);
cyclohexylidene (Scheme32 right and bottom),
isopropylidene (Scheme32 right and bottom).
- R¹, R²: each phenyl (Scheme2);
R¹ = cyclohexyl, R² = 4-chlorophenyl (Scheme7);
- R¹, R²: R¹ = isopropyl, R² = H (Scheme11)
- R₁: ratio of fronts
- RMgX: benzylmagnesium chloride (Scheme28)
- RT: room temperature
- RX: methyl iodide (Mel) (Scheme17);
3-chloro-methoxypropane (Scheme19);
benzyl bromide (Scheme23 last lane, Scheme26)
- TBAF: tetra-butylammonium fluoride
- TBDMS-CI: tert-butyldimethylsilyl chloride
- TBDMS: tert-butyldimethylsilyl
- TBME: tert-Butylmethylether
- TEA: triethylamine
- TEMPO: 2,2,6,6,-tetramethyl-1-piperidinyloxy free radical
- TFA: trifluoroacetic acid
- THF: tetrahydrofurane
- RP: reverse phase
- Prep: Preparative
- TLC: Thin Layer Chromatography
- tᵣ: retention time

### Trademarks

| | |
|---|---|
| Celite | = Celite^{®} (The Celite Corporation) = filtering aid based on diatomaceous earth |
| NH₂ Isolute | (= Isolute^{®} NH₂, Isolute^{®} is registered for Argonaut Technologies, Inc.) = ion exchange with amino groups based on silica gel |
| Nucleosil | = Nucleosil^{®}, trademark of Machery & Nagel, Düren, FRG for HPLC materials |

Temperatures are measured in degrees Celsius. Unless otherwise indicated, the reactions take place at RT.

TLC conditions: R_{f} values for TLC are measured on 5 x 10 cm TLC plates, silica gel F₂₅₄, Merck, Darmstadt, Germany.

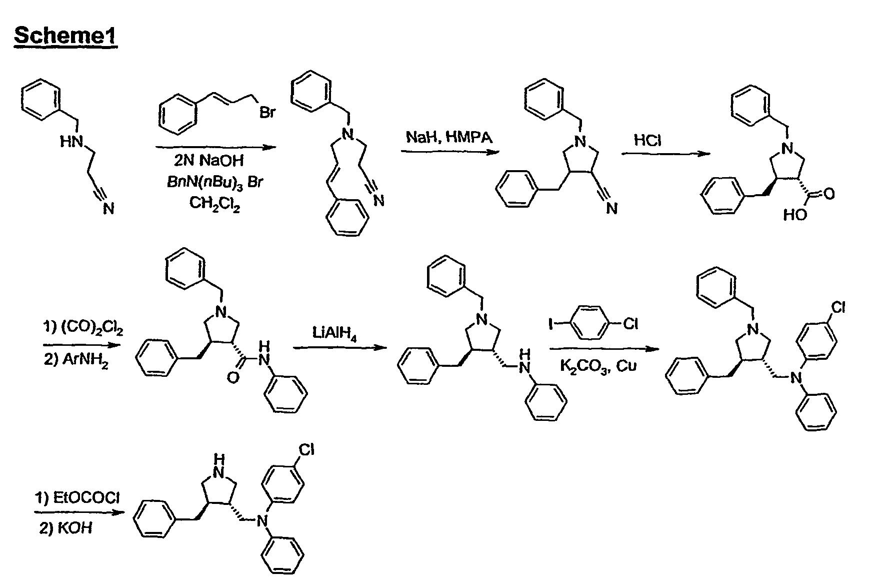

### Example 1: ((3S*,4S*)-4-Benzyl-pyrrolidin-3-ylmethyl)-(4-chloro-phenyl)-phenyl-amine

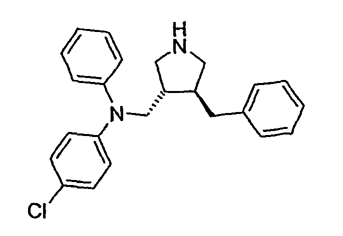

### A. 3-[Benzyl-((E)-3-phenyl-allyl)-amino]-propionitrile

To a solution of 3-benzylamino-propionitrile (4.8 kg, 30 mol) and benzyl-tri-(N-butyl) ammonium bromide (1.08 kg, 3 mol) in 15 L CH₂Cl₂, a 2 N aqueous NaOH solution (30 L) is added. The reaction mixture is stirred under N₂ atmosphere, and a solution of cinnamyl bromide (5.91 kg, 30 mol) in CH₂Cl₂ (30 L) is added dropwise. The reaction mixture is further stirred at 40°C for 5 h, diluted with 30 L of CH₂Cl₂ and poured into water (20 L). The layers are separated, and the aqueous one is extracted with CH₂Cl₂. The combined organic layers are dried over MgSO₄, filtered and concentrated under reduced pressure to give the title compound. TLC, Rf (toluene/EtOH, NH₄OH 84/15/1) = 0.75.

### B. 1,4-Dibenzyl-pyrrolidine-3-carbonitrile

To NaH (80 % in grease, 0.816 kg, 27.2 mol), HMPA (17 L) (exothermic!) is carefully added under N₂ atmosphere. The resulting suspension is stirred for 30 min and cooled to 0°C, before dropwise addition of a solution of 3-[benzyl-((E)-3-phenyl-allyl)-amino]-propionitrile (5.98 kg, 18.1 mol) in HMPA (16 L) follows. The reaction mixture is allowed to reach RT overnight and AcOH (1.9 L) is added at 0°C, followed by water (27 L). The reaction mixture is extracted with toluene (3x25 L), the combined organic layers are dried over MgSO₄, filtered and concentrated under reduced pressure to give the title compound as a brown oil. To a solution of the residue in EtOH abs. (5 L), a solution of oxalic acid monohydrate (2.28 kg, 18.1 mol) in EtOH abs. (4 L) is added, and the resulting mixture is stirred at RT. 8 L of Et₂O are added, and the mixture is further stirred for 1 h at 5 C. Acetonee and Et₂O 1/1 (6 L) are added, the mixture is centrifuged, and the residue is further washed with Et₂O and filtered. The resulting material is dried under vacuum to give the desired oxalate salt. To a solution of the oxalate salt (3.3 kg) in a mixture of water (20 L) and toluene (33 L), NH₄OH (25%, 2.6 L) is added to adjust the pH to 10. The layers are separated, and the aqueous one is extracted twice with toluene (10 L). The combined organic layers are dried over MgSO₄, filtered and concentrated to give the title compound. TLC, Rf (CH₂Cl₂/benzene 75/25) = 0. 5.

### C. (3R*,4R*)-1,4-Dibenzyl-pyrrolidine-3-carboxylic acid

A solution of 1,4-dibenzyl-pyrrolidine-3-carbonitrile (0.828 kg, 3 mol), acetic acid (2.7 L), water (0.9 L) and concentrated HCl (0.9 L) is refluxed for 18 h. To the solution, charcoal is added, and the resulting mixture is further stirred at 50°C before filtration of the (still warm) mixture on a pad of Celite. The filtrate is concentrated under reduced pressure, and the residue is dissolved into a mixture of MeOH (1.5 L) and water (7.5 L) at 50°C. To the resulting solution, H₂O (7.5 L) and toluene (4.5 L) are added, the layers are separated, and the aqueous one is back-extracted with toluene (4 L). Charcoal is added to the aqueous layer, and, after filtration on a pad of Celite, the filtrate is basified to pH 6 by addition at 60°C of an aqueous ammonia solution (10%) to allow the ammonium salt to precipitate out. The mixture is vigorously stirred for 45 min and allowed to cool to RT before filtration. The resulting compound is re-crystallized in EtOH to give the title compound. TLC, Rf (CH₂Cl₂/MeOH/NH₄OH 50/45/5) = 0.45. MS (LC-MS): 296 [M+H]⁺; t_{R} (HPLC, Nucleosil C18 column, 10-100% CH₃CN/H₂O in 5 min, 100% CH₃CN/3 min, flow: 1.5 mL/min): 4.15 min.

### D. (3R*,4R*)-1,4-Dibenzyl-pyrrolidine-3-carboxylic acid phenylamide

(3R*,4R*)-1,4-Dibenzyl-pyrrolidine-3-carboxylic acid (2 g, 6.77 mmol) is suspended in CH₂Cl₂ (15 mL) under nitrogen and treated with oxalyl chloride (1.18 mL, 13.54 mmol). The resulting mixture is stirred overnight at RT and concentrated under reduced pressure. The resulting crude acid chloride is taken up in toluene (10 mL) and concentrated once more. This operation is repeated 3 times to assure the excess of oxalyl chloride is eliminated. Finally, to a solution of the crude oil in CH₂Cl₂ (15 mL) aniline (1.02 mL, 20.30 mmol) and triethylamine (1.13 mL, 8.12 mmol) are added, and the mixture is refluxed for 8 h. The crude mixture is then poured into an aqueous saturated solution of NaHCO₃, extracted with CH₂Cl₂, dried over Na₂SO₄, filtered and concentrated under reduced pressure. The crude material is purified on silica gel (eluent: hexane/AcOEt 1/2 to 1/1) to afford the desired compound. TLC, Rf (c-hexane/AcOEt 50/50) = 0.2. t_{R} (HPLC, Nucleosil C18 column, 10-100% CH₃CN in H₂O in 5 min, 100% CH₃CN for 3 min, CH₃CN and H₂O containing 0.1% TFA, flow: 1.5 mL/min): 4.75 min

### E. ((3S*,4R*)-1,4-Dibenzyl-pyrrolidin-3-ylmethyl)-phenyl-amine

To a solution of (3R*,4R*)-1,4-dibenzyl-pyrrolidine-3-carboxylic acid phenylamide (0.2 g, 0.54 mmol) in THF (3 mL), carefully LiAlH₄ (1 N in THF, 1.62 mL, 1.62 mmol) is added. The mixture is stirred at reflux for 3 h and allowed to cool to RT, before the careful addition of 0.06 mL of water followed by 0.06 mL of an aqueous solution containing 15 % of NaOH and finally 0.12 mL of water follows. The resulting mixture is stirred overnight, filtered and concentrated. Chromatography on silica gel (eluent: c-hexane/AcOEt 1/1) gives the title compound (0.19 g). TLC, Rf (c-hexane/AcOEt 50/50) = 0.3. MS (LC-MS): 357 [M+H]⁺

### F. (4-Chloro-phenyl)-((3R*,4R*)-1,4-dibenzyl-pyrrolidin-3-ylmethyl)-phenyl-amine

A mixture of ((3S*,4R*)-1,4-dibenzyl-pyrrolidin-3-ylmethyl)-phenyl-amine (10 g, 56.1 mmol), 1-bromo-4-iodobenzene (190 g, 0.796 mol), K₂CO₃ (7.75 g, 56.1 mol), Nal (0.7 g, 4.67 mmol) and Venus copper (UP 55, 20 g) is heated at 210°C for 6.5 h. The reaction mixture is taken up in CHCl₃ and filtrated over a pad of Celite. To the resulting filtrate, NaOH (2N) is added, the layers are separated, and the aqueous one is back-extracted twice with CHCl₃. The combined organic extracts are dried over Na₂SO₄, filtered and concentrated. The crude residue is dissolved in CH₂Cl₂ and HCl (1N) is added, the layers are separated and the aqueous one is basified by the addition of NaOH (2N) and extracted 3 times with CH₂Cl₂. The combined organic extracts are dried over Na₂SO₄, filtered and concentrated. Finally, the title compound is crystallized as its mono-hydrochloride salt. The salt is further dissolved in CH₂Cl₂, NaOH (2N) is added, the layers are separated, and the aqueous one is extracted 3 times with CH₂Cl₂. The combined organic extracts are dried over Na₂SO₄, filtered and concentrated to give the desired title compound as a dark oil. MS (LC-MS): 467, 468.9 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.78 min.

### G. ((3R*,4R*)-4-Benzyl-pyrrolidin-3-ylmethyl)-(4-chloro-phenyl)-phenyl-amine

A mixture of (4-chloro-phenyl)-((3R*,4R*)-1,4-dibenzyl-pyrrolidin-3-ylmethyl)-phenyl-amine (11.2 g) and ethyl chloroformate (4.42 mL) in toluene (110 mL) is heated at 90-100 °C for 7 h. The solution is quenched by the addition of NaOH (2N). Toluene (100 mL) is added, the layers are separated, and the aqueous one is extracted twice with toluene. The combined organic extracts are dried over Na₂SO₄, filtered and concentrated to give (3R*,4S*)-3-benzyl-4-{[(4-chloro-phenyl)-phenyl-amino]-methyl}-pyrrolidine-1-carboxylic acid ethyl ester. A suspension of (3R*,4S*)-3-benzyl-4-{[(4-chloro-phenyl)-phenyl-amino]-methyl}-pyrrolidine-1-carboxylic acid ethyl ester (11.16 g, 26.51 mmol) and KOH (11.16 g, in H₂O 11.2 mL) in EtOH (100 mL) is heated at 110°C for 15 h. The reaction mixture is allowed to cool to RT and concentrated. CH₂Cl₂ and water are added, the layers are separated, and the aqueous one is extracted twice with CH₂Cl₂. The combined organic layers are dried over Na₂SO₄, filtered and concentrated. The crude material is purified by flash chromatography on silica gel (eluent: CH₂Cl₂/MeOH 90/10 to 90/10 + 3 % NH₄OH) to give the title product. MS (LC-MS): 376.9, 379 [M+H]⁺; t_{R} (HPLC, Nucleosil C18 column, 10-100% CH₃CN/H₂O in 5 min, 100% CH₃CN/3 min, CH₃CN and H₂O containing 0.1% TFA, flow: 1.5 mL/min): 5.40 min.

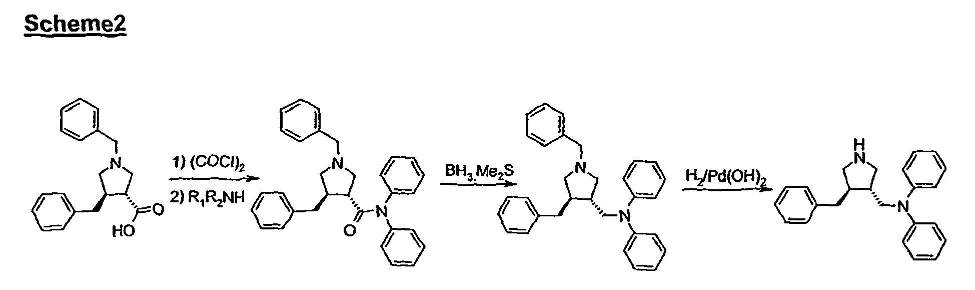

### Example 2: ((3R*,4R*)-4-Benzyl-pyrrolidin-3-ylmethyl)-diphenyl-amine

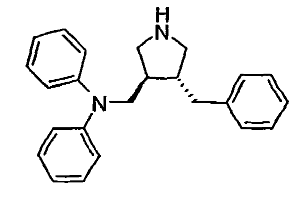

### A. (3R*,4R*)-1,4-Dibenzyl-pyrrolidine-3-carboxylic acid diphenylamide

To a suspension of (3R*,4R*)-1,4-dibenzyl-pyrrolidine-3-carboxylic acid (500 mg, 1.69 mmol) in CH₂Cl₂ (5 mL) under argon atmosphere, oxalyl chloride (0.52 mL, 6.01 mmol) is added slowly. The reaction mixture is stirred overnight and concentrated. To a suspension of the resulting crude acid chloride in CH₂Cl₂ (5 mL), diphenyl-amine (237 mg, 1.86 mmol) and triethylamine (0.26 mL, 1.86 mmol) are added at RT, the reaction mixture is further stirred for 2 h and poured into an aqueous saturated solution of NaHCO₃, extracted twice with CH₂Cl₂, dried over Na₂SO₄, filtered and concentrated. The resulting crude oil is purified by flash chromatography on silica gel (eluent: 1-4% MeOH in CH₂Cl₂) to give the title product.
MS (LC-MS): 447 [M+H]⁺; t_{R} (HPLC, Nucleosil C18 column, 10 to 90% CH₃CN in H₂O in 11 min, CH₃CN and H₂O containing 0.1 % TFA, flow: 1.5 mL/min): 5.24 min.

### B. ((3R*,4R*)-1,4-Dibenzyl-pyrrolidin-3-ylmethyl)-diphenyl-amine

To a solution of (3R*,4R*)-1,4-dibenzyl-pyrrolidine-3-carboxylic acid diphenylamide (500 mg, 1.12 mmol) in THF (3 mL), BH₃Me₂S (2N in THF, 3.36 mL, 6.72 mmol) is added. The reaction mixture is refluxed for 3 h until completion of the reaction, and concentrated. The residual oil is taken up in MeOH (3 mL) and HCl cc (3 mL), refluxed overnight and poured into an aqueous solution of NaOH (0.5 N). CH₂Cl₂ is added and the organic layer is separated. The resulting aqueous layer is extracted twice with CH₂Cl₂, and the combined organic layers are dried over anhydrous sodium sulfate, filtered and concentrated under reduced pressure. The resulting crude material is purified by flash chromatography on silica gel (eluent: hexane/AcOEt 2/1) to give the title compound. MS (LC-MS): 433.0 [M+H]⁺;
t_{R} (HPLC, Nucleosil C18 column, 10 to 90% CH₃CN in H₂O in 11 min, CH₃CN and H₂O containing 0.1% TFA, flow: 1.5 mL/min): 5.71 min.

### C. ((3R*,4R*)-4-Benzyl-pyrrolidin-3-ylmethyl)-diphenyl-amine

A mixture of ((3R*,4R*)-1,4-dibenzyl-pyrrolidin-3-ylmethyl)-diphenyl-amine (0.38 g) and Pd(OH)₂ (20%/C) (0.1 g, 50% wet) in MeOH/THF (8/1 mL) is stirred under a hydrogen atmosphere. After completion of the reaction, the crude material is filtered over a pad of Celite, dried over Na₂SO₄ and concentrated. Chromatography on silica gel (eluent CH₂Cl₂/MeOH 98/2 to 95/5 containing 1% of NH₄OH) gives the title compound. Addition of 139 µl of 4N HCl/dioxane (0.38 mmol) to a solution of the product in dioxane (3 mL) and lyophilization yields the corresponding hydrochloride salt as a white solid. MS (LC-MS): 343 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.21 min.

### Example 3: 3-[((3R*,4R*)-4-Benzyl-pyrrolidin-3-ylmethyl)-phenyl-amino]-phenol

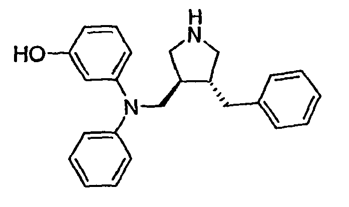

### (3R*,4R*)-1,4-Dibenzyl-pyrrolidine-3-carboxylic acid (3-methoxy-phenyl)-phenyl-amine

Title compound is prepared analogously as described for the title compound under B in Example 2 from of (3R*,4R*)-1,4-dibenzyl-pyrrolidine-3-carboxylic acid and 3-methoxy diphenyl amine. MS (LC-MS): 477 [M+H]⁺; t_{R} (HPLC, Nucleosil C18 column, 10 to 100% CH₃CN in H₂O in 5 min, then 100% CH₃CN for 3 min, CH₃CN and H₂O containing 0.1% TFA, flow: 1.5 mL/min): 5.59 min.

### 3-[((3R*,4R*)-1,4-Dibenzyl-pyrrolidin-3-ylmethyl)-phenyl-amino]-phenol

To a solution of ((3R*,4R*)-1,4-dibenzyl-pyrrolidin-3-ylmethyl)-(3-methoxy-phenyl)-phenylamine (2.16 g, 4.67 mol) (prepared in analogy to the title compound under A in Example 2 using the methoxy analogue of (3R*,4R*)-1,4-dibenzyl-pyrrolidine-3-carboxylic acid) in CH₂Cl₂ (25 mL), BBr₃ (2.70 mL, 28.0 mmol) is slowly added at -78 °C under N₂ atmosphere. The solution is allowed to warm to RT, stirred for 2 additional hours, and quenched with H₂O (25 mL). The aqueous layer is extracted twice with CH₂Cl₂, and the combined organic layers are dried over Na₂SO₄ and concentrated under reduced pressure. The residual oil is purified by flash chromatography to give title product. MS (LC-MS): 449 [M+H]⁺; t_{R} (HPLC, Nucleosil C18 column, 10 to 100% CH₃CN in H₂O in 5 min, 100% CH₃CN for 3 min, CH₃CN and H₂O containing 0.1% TFA, flow: 1.5 mL/min): 5.14 min.

### 3-[((3R*,4R*)-4-Benzyl-pyrrolidin-3-ylmethyl)-phenyl-amino]-phenol

Title compound is prepared analogously as described for the title compound under C in Example 2 from of 3-[((3R*,4R*)-1,4-dibenzyl-pyrrolidin-3-ylmethyl)-phenyl-amino]-phenol. MS (LC-MS): 359 [M+H]⁺; t_{R} (HPLC, Nucleosil C18 column, 10 to 90% CH₃CN in H₂O in 11 min, CH₃CN and H₂O containing 0.1% TFA, flow: 1.5 mUmin): 5.75 min.

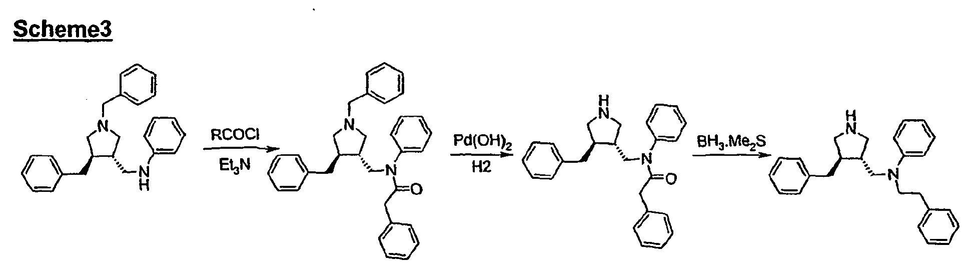

### Example 4: ((3S*,4S*)-4-Benzyl-pyrrolidin-3-ylmethyl)-phenethyl-phenyl-amine

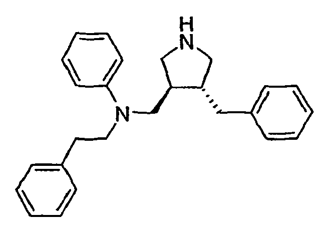

### A. N-((3R*,4R*)-1,4-Dibenzyl-pyrrolidin-3-ylmethyl)-2,N-diphenyl-acetamide

To a solution of ((3S*,4R*)-1,4-dibenzyl-pyrrolidin-3-ylmethyl)-phenyl-amine (200 mg, 0.56 mmol) in CH₂Cl₂ (2 mL) under nitrogen, phenylacetylchloride (89 µL, 0.67 mmol) and triethylamine (64 µL, 0.67 mmol) are added. The mixture is stirred for 2 h at RT and poured into an aqueous saturated solution of NaHCO₃. The organic layer is separated, and the resulting aqueous layer is extracted twice with CH₂Cl₂. The combined organic layers are dried over anhydrous sodium sulfate, filtered and concentrated under reduced pressure. The resulting crude material is purified by flash chromatography on silica gel (eluent: hexane/AcOEt 80/20) to give the title compound. MS (LC-MS): 475 [M+H]⁺; t_{R} (HPLC, Nucleosil C18 column, 10-100% CH₃CN in H₂O in 5 min, 100% CH₃CN for 3 min, CH₃CN and H₂O containing 0.1% TFA, flow: 1.5 mL/min): 5.35 min.

### B. N-((3R*,4R*)-4-Benzyl-pyrrolidin-3-ylmethyl)-2,N-diphenyl-acetamide

A mixture of N-((3R*,4R*)-1,4-dibenzyl-pyrrolidin-3-ylmethyl)-2,N-diphenyl-acetamide (0.25 mg, 0.53 mmol) and Pd(OH)₂ (20%/C) (0.08 g, 50% wet) in MeOH (8 mL) is stirred under an hydrogen atmosphere. After completion of the reaction, the crude material is filtered over a pad of Celite, dried over Na₂SO₄ and concentrated. Chromatography on silica gel (eluent: CH₂Cl₂/MeOH 98/2 to 95/5 containing 1% of NH₄OH) gives the title compound. Addition of 4N HCl/dioxane (0.1 mmol) to a solution of the product in dioxane (2 mL) and lyophilization yields the corresponding hydrochloride salt as a white solid. MS (LC-MS): 385 [M+H]⁺;
t_{R} (HPLC, Nucleosil C18 column, 10-90% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.79 min.

### C. ((3R*,4R*)-4-Benzyl-pyrrolidin-3-ylmethyl)-phenethyl-phenyl-amine

To a solution of N-((3R*,4R*)-4-benzyl-pyrrolidin-3-ylmethyl)-2,N-diphenyl-acetamide (100 mg, 0.26 mmol) in THF (1.5 mL), BH₃.Me₂S (2 N in THF, 780 µL, 1.56 mmol) is added. The reaction mixture is refluxed for 3 h until completion of the reaction and concentrated. The residual oil is taken up in MeOH (3 mL) and HCl cc (3 mL), refluxed over night and poured into an aqueous solution of NaOH (0.5 N). CH₂Cl₂ is added, and the organic layer is separated off. The resulting aqueous layer is extracted twice with CH₂Cl₂, and the combined organic layers are dried over anhydrous sodium sulfate, filtered and concentrated under reduced pressure. The resulting crude material is purified by flash chromatography on silica gel (eluent: CH₂Cl₂/MeOH 98/2 to 95/5 containing 1% of NH₄OH) to give the title compound. Addition of 139 µl of 4N HCl/dioxane (0.054 mmol) to a solution of the product in dioxane (2 mL) and lyophilization afford the corresponding hydrochloride salt as a white solid.
MS (LC-MS): 371 [M+H]⁺; t_{R} (HPLC, Nucleosil C18 column, 10 to 90% CH₃CN in H₂O in 11 min, CH₃CN and H₂O containing 0.1 % TFA, flow: 1.5 mL/min): 5.43 min.

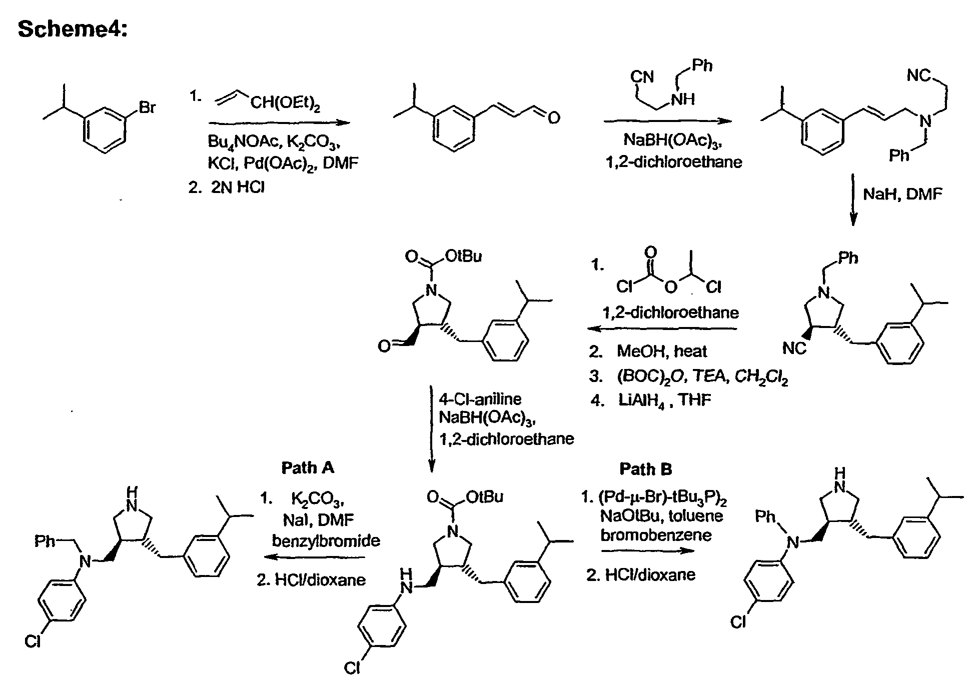

### Example 5: (3R*,4R*)-Benzyl-(4-chloro-phenyl)-[4-(3-isopropyl-benzyl)-pyrrolidin-3-ylmethyl]-amine

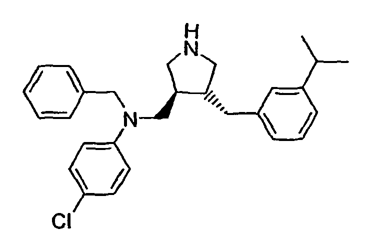

### A. (E)-3-(3-Isopropyl-phenyl)-propenal

1-Bromo-3-isopropylbenzene (14.5 g, 69 mmol), acrolein-diethylacetal (33.5 mL, 208 mmol), Bu₄NOAc (44 g, 138 mmol), K₂CO₃ (14.5 g, 104 mmol), KCl (5.2 g, 70 mmol) and Pd(OAc)₂ (0.5 g, 2 mmol) are suspended in 290 mL DMF and heated for 24 h at 90°C. The reaction mixture is cooled to ambient temperature, diluted with water (100mL) and acidified with 2N HCl. After extraction with TBME, the organic layer is washed with water, dried over Na₂SO₄, filtered and concentrated. The crude product is purified by flash chromatography on silica gel (eluent: hexane/AcOEt 19/1) to give the title compound. TLC, Rf (hexane/AcOEt 10/1) = 0.56. MS(EI+): 175 (M+1).

### B. 3-{Benzyl-[(E)-3-(3-isopropyl-phenyl)-allyl]-amino}-propionitrile

To an ice-cold solution of (E)-3-(3-isopropyl-phenyl)-propenal (8.4 g, 48 mmol) in 1,2-Dichloroethane (180 mL), NaBH(OAc)₃ (14.8 g, 63 mmol) is added in one portion. After 30 min the ice-bath is removed and stirring is continued for 60 min. The reaction is quenched at 0°C by addition of a saturated aqueous NaHCO₃ solution. After extraction with CH₂Cl₂, the organic layer is dried over Na₂SO₄, filtered and concentrated. The crude product is purified by flash chromatography on silica gel (eluent: hexane/AcOEt 10/1) to give the title compound. TLC, Rf (hexane/AcOEt 10/1) = 0.25. MS(El+): 319 (M+1)

### C. (3R*,4R*)-1-Benzyl-4-(3-isopropyl-benzyl)-pyrrolidine-3-carbonitrile

To a solution of 3-{benzyl-[(E)-3-(3-isopropyl-phenyl)-allyl]-amino}-propionitrile (17.2 g, 54 mmol) in DMF (200 mL) sodium hydride (4.7 g, 108 mmol, 55% suspension in mineral oil) is added at ambient temperature in two portions over 5 min. After stirring for 4 h the reaction is quenched at 0°C by careful addition of a saturated aqueous NaHCO₃ solution. The mixture is diluted with water and extracted with AcOEt. The organic layer is dried over Na₂SO₄, filtered and concentrated. The crude trans/cis mixture is separated by flash chromatography on silica gel (eluent: hexane/AcOEt 4/1) to give the title compound. TLC, Rf (hexane/AcOEt 4/1) = 0.48. MS(EI+): 319 (M+1).

### D. (3R*,4R*)-4-(3-Isopropyl-benzyl)-pyrrolidine-3-carbonitrile

To a solution of (3R*,4R*)-1-benzyl-4-(3-isopropyl-benzyl)-pyrrolidine-3-carbonitrile (2.0 g, 6.3 mmol) in 1,2-dichloroethane (40 mL), 1-chloroethoxycarbonyl chloride (1.4 mL, 12.6 mmol) is added at ambient temperature. After heating overnight under reflux, the mixture is cooled to ambient temperature and the solvent removed *in vacuo*. The crude product is dissolved in methanol (50 mL) and heated under reflux for 1 h. The solvent was removed in *vacuo* and the product is purified by flash chromatography on silica gel (eluent: CH₂Cl₂/MeOH 9/1) to give the title compound. TLC, Rf (CH₂Cl₂ /MeOH 9/1) = 0.48. MS(EI+): 229 (M+1).

### E. (3R*,4R*)-3-Cyano-4-(3-isopropyl-benzyl)-pyrrolidine-1-carboxylic acid tert-butyl ester

To a solution of (3R*,4R*)-4-(3-isopropyl-benzyl)-pyrrolidine-3-carbonitrile (1.5 g, 6.3 mmol) in CH₂Cl₂ (50mL) is added triethylamine (1.1 mL, 8.2 mmol). After cooling to 0°C, (BOC)₂O is added in two portions and stirring is continued for 15 min. After stirring overnight at ambient temperature, the solvent is removed *in vacuo* and the crude product is purified by flash chromatography on silica gel (eluent: hexane/AcOEt 4/1) to give the title compound. TLC, Rf (hexane/AcOEt 10/1) = 0.36. MS(EI+): 229 (M-55).

### F. (3R*,4R*)-3-Formyl-4-(3-isopropyl-benzyl)-pyrrolidine-1-carboxylic acid tert-butyl ester

To a solution of (3R*,4R*)-3-cyano-4-(3-isopropyl-benzyl)-pyrrolidine-1-carboxylic acid tert-butyl ester (1.0 g, 2.9 mmol) in toluene (30mL) is added DIBAL (4.8 mL, 5.8 mmol, 1.2 M in toluene) at -78°C. After stirring for 90 min, a 30% Rochelle-salt solution (50 mL) is added and stirring is continued for 1 h at ambient temperature. The mixture is extracted with AcOEt, the organic layer is dried over Na₂SO₄, filtered and concentrated. The crude product is purified by flash chromatography on silica gel (eluent: hexane/AcOEt 9/1) to give the title compound. TLC, Rf (hexane/AcOEt 4/1) = 0.27. t_{R} (HPLC, Nucleosil C18 column, 20-100% CH₃CN/H₂O/6 min, 100% CH₃CN/1.5 min, 100-20% CH₃CN/H₂O/0.5 min, CH₃CN and H₂O containing 0.1% TFA, flow: 1.0 mL/ min): 5.73 min. MS(EI+): 276 (M-55).

### G. (3R*,4R*)-3-[(4-Chloro-phenylamino)-methyl]-4-(3-isopropyl-benzyl)-pyrrolidine-1-carboxylic acid tert-butyl ester

To an ice-cold solution of (3R*,4R*)-3-formyl-4-(3-isopropyl-benzyl)-pyrrolidine-1-carboxylic acid tert-butyl ester (0.30 g, 0.9 mmol) and 4-chloro aniline (0.13 g, 0.9 mmol) in 1,2-dichloroethane (10 mL), NaBH(OAc)₃ (0.28 g, 1.2 mmol) is added. After 30 min, the reaction mixture is warmed up to ambient temperature and stirred for 60 min. The reaction is quenched at 0°C by addition of a saturated aqueous NaHCO₃ solution. After extraction with CH₂Cl₂, the organic layer is dried over Na₂SO₄, filtered and concentrated. The crude product is purified by flash chromatography on silica gel (eluent: hexane/AcOEt 4/1) to give the title compound. TLC, Rf (hexane/AcOEt 4/1) = 0.31. MS(EI+): 443 (M+).

### Path A

### H. (3R*,4R*)-3-{[Benzyl-(4-chloro-phenyl)-amino]-methyl}-4-(3-isopropyl-benzyl)-pyrrolidine-1-carboxylic acid tert-butyl ester

A vial is charged with (3R*,4R*)-3-[(4-chloro-phenylamino)-methyl]-4-(3-isopropyl-benzyl)-pyrrolidine-1-carboxylic acid tert-butyl ester (0.17 g, 0.22 mmol), benzylbromide (0.08 g, 0.44 mmol), K₂CO₃ (0.06 g, 0.44 mmol) and sodium iodide (0.07 g, 0.44 mmol) in air and suspended in DMF (8 mL). The vial is sealed with an Al crimp top with septum and heated for 30 min at 120°C in a microwave apparatus (PersonalChemistry). The reaction mixture is diluted with water and AcOEt , and the aqueous layer is extracted with AcOEt. The combined organic extracts are washed with brine, dried over Na₂SO₄, filtered and concentrated. The residue is purified by RP präp. HPLC (eluent; ACN/water) to give the title compound. TLC, Rf (hexane/AcOEt 4/1) = 0.43. MS(EI+): 533 (M+).

### I. (3R*,4R*)-Benzyl-(4-chloro-phenyl)-[4-(3-isopropyl-bezyl)-pyrrolidin-3-ylmethyl]-amine

To a solution of (3R*,4R*)-3-{[benzyl-(4-chloro-phenyl)-amino]-methyl}-4-(3-isopropyl-benzyl)-pyrrolidine-1-carboxylic acid tert-butyl ester (0.09 g, 0.17 mmol) in 1,4-dioxane (3 mL), 4N HCl (3 mL) in 1,4-dioxane is added. After stirring for 3.5 h, the solvent is removed in vacuo. The residue is taken up in CH₂Cl₂ and washed with a saturated K₂CO₃ solution. The organic layer is dried over Na₂SO₄, filtered and concentrated to give the title compound. TLC, Rf (CH₂Cl₂ /MeOH 9/1) = 0.40. MS(EI+): 433 (M+).

### Path B

### Example 6: (3R*,4R*)-(4-Chloro-phenyl)-[4-(3-isopropyl-benzyl)-pyrrolidin-3-ylmethyl]-phenyl-amine

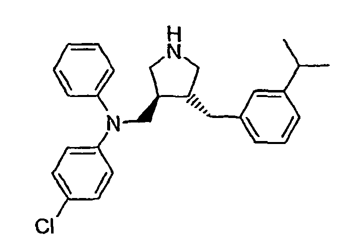

### J. (3R*,4R*)-3-{[(4-Chloro-phenyl)-phenyl-amino]-methyl}-4-(3-isopropyl-benzyl)-pyrrolidine-1-carboxylic acid tert-butyl ester

A two-necked flask, equipped with a magnetic stirring bar, septum and condenser with an argon inlet-outlet is charged with [Pd(µ-Br)(t-Bu₃P)]₂ (8 mg, 5 mol%), NaOtBu (32 mg, 0.34 mmol), (3R*,4R*)-3-[(4-chloro-phenylamino)-methyl]-4-(3-isopropyl-benzyl)-pyrrolidine-1-carboxylic acid tert-butyl ester (105 mg, 0.22 mmol) and bromobenzene (42 mg, 0.27 mmol). Abs. toluene (7 mL) are added, and the mixture is stirred for 10 min at RT and heated 24 h under a gentle reflux. After cooling to RT, the reaction is quenched with a saturated aqueous NaHCO₃ solution, extracted with AcOEt, dried over Na₂SO₄ and concentrated. The residue is purified by RP präp. HPLC (eluent; ACN/water) to give the title compound. TLC, Rf (hexane/AcOEt 4/1) = 0.49. MS(EI+): 519 (M+)

### K. (3R*,4R*)-(4-Chloro-phenyl)-[4-(3-isopropyl-benzyl)-pyrrolidin-3-ylmethyl]-phenylamine

To a solution of (3R*,4R*)- 3-{[(4-chloro-phenyl)-phenyl-amino]-methyl}-4-(3-isopropyl-benzyl)-pyrrolidine-1-carboxylic acid tert-butyl ester (0.08 g, 0.16 mmol) in 1,4-dioxane (3 mL), 4N HCl (3 mL) in 1,4-dioxane is added. After stirring for 5.5 h, the solvent is removed in *vacuo.* The residue is taken up in CH₂Cl₂ and washed with a saturated K₂CO₃ solution. The organic layer is dried over Na₂SO₄, filtered and concentrated to give the title compound. TLC, Rf (CH₂Cl₂ /MeOH 9/1) = 0.35. MS(El+): 419 (M+1).

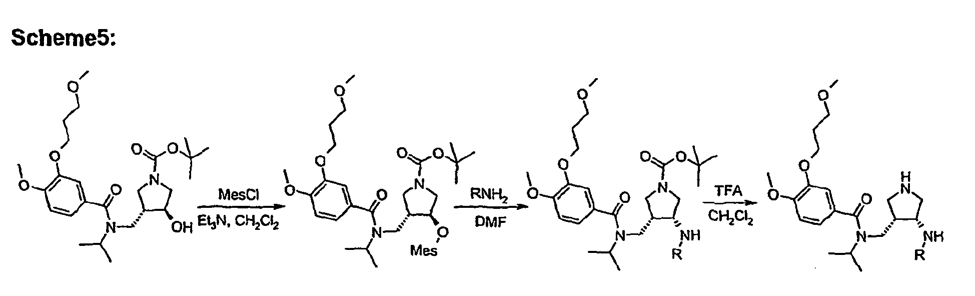

### Example 7: N-((3S*,4R*)-4-Benzylamino-pyrrolidin-3-ylmethyl)-N-isopropyl-4-methoxy-3-(3-methoxy-propoxy)-benzamide

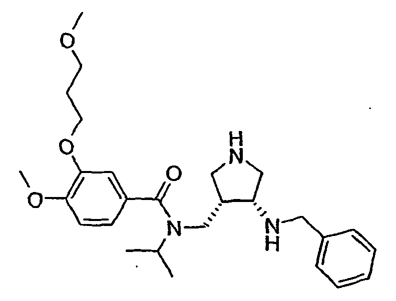

### (3R*,4S*)-3-({Isopropyl-[4-methoxy-3-(3-methoxy-propoxy)-benzoyl]-amino}-methyl)-4-methanesulfonyloxy-pyrrolidine-1-carboxylic acid tert-butylester

(3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester (250 mg, 0.522 mmol) is dissolved in 3 mL of CH₂Cl₂. The mixture is cooled to 0°C, and 45 µL (0.575 mmol) of methansulfonyl chloride is added, followed by 80 µL (0.575 mmol) of triethylamine. The mixture is stirred 5 h at RT, poured into water. CH₂Cl₂ is added, the layer are separated and the aqueous one back extracted twice with CH₂Cl₂. The combined organic extracts are dried over Na₂SO₄, filtered and concentrated. The crude material is used in the next without further purification. TLC, Rf (AcOEt) = 0.4.

### (3R*,4R*)-3-Benzylamino-4-({isopropyl-[4-methoxy-3-(3-methoxy-propoxy)-benzoyl]-amino}-methyl)-pyrrolidine-1-carboxylic acid tert-butyl ester

A mixture (3R*,4S*)-3-({isopropyl-[4-methoxy-3-(3-methoxy-propoxy)-benzoyl]-amino}-methyl)-4-methanesulfonyloxy-pyrrolidine-1-carboxylic acid tert-butylester (295 mg, 0.52 mmol), benzylamine (63 µL, 0.575 mmol) and K₂CO₃ (79 mg, 0.575 mmol) in DMF (3 mL) is stirred under Argone at RT overnight. H₂O is then added and the mixture is extracted twice with EtOAc. The combined organic extracts are dried (Na₂SO₄), filtered and concentrated. The crude material is purified by flash column chromatography on silica gel (c-hexane/EtOAc 1/1 to 0/1) to give the title compound. TLC, Rf (AcOEt) = 0.25. MS (LC-MS): 570.1 [M+H]⁺.

### N-((3S*,4R*)-4-Benzylamino-pyrrolidin-3-ylmethyl)-N-isopropyl-4-methoxy-3-(3-methoxy-propoxy)-benzamide

To a solution of (3R*,4R*)-3-benzylamino-4-({isopropyl-[4-methoxy-3-(3-methoxy-propoxy)-benzoyl]-amino}-methyl)-pyrrolidine-1-carboxylic acid tert-butyl ester (100 mg, 0.175 mmol) in 2 mL CH₂Cl₂, TFA (162 µL, 2.11 mmol) is added. The mixture is stirred at RT for 2 h and poured into a saturated solution of NaHCO₃. The layers are separated, and the aqueous one is back-extracted twice with CH₂Cl₂. The combined organic extracts are dried over Na₂SO₄, filtered and concentrated. The crude material is purified by preparative on silica gel (eluent: CH₂Cl₂/MeOH 100/0 to 90/10+1% NH₄OH) to give the title compound. To a solution of the free base (54 mg, 0.115 mmol) in dioxane (3 mL), (72 µL, 0.289 mmol of 4N HCl in dioxane is added, and the resulting solution is lyophilized to afford the corresponding hydrochloride salt as a white powder.

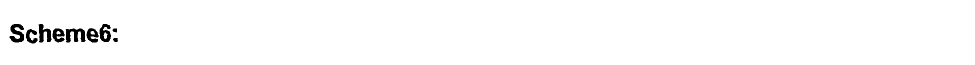

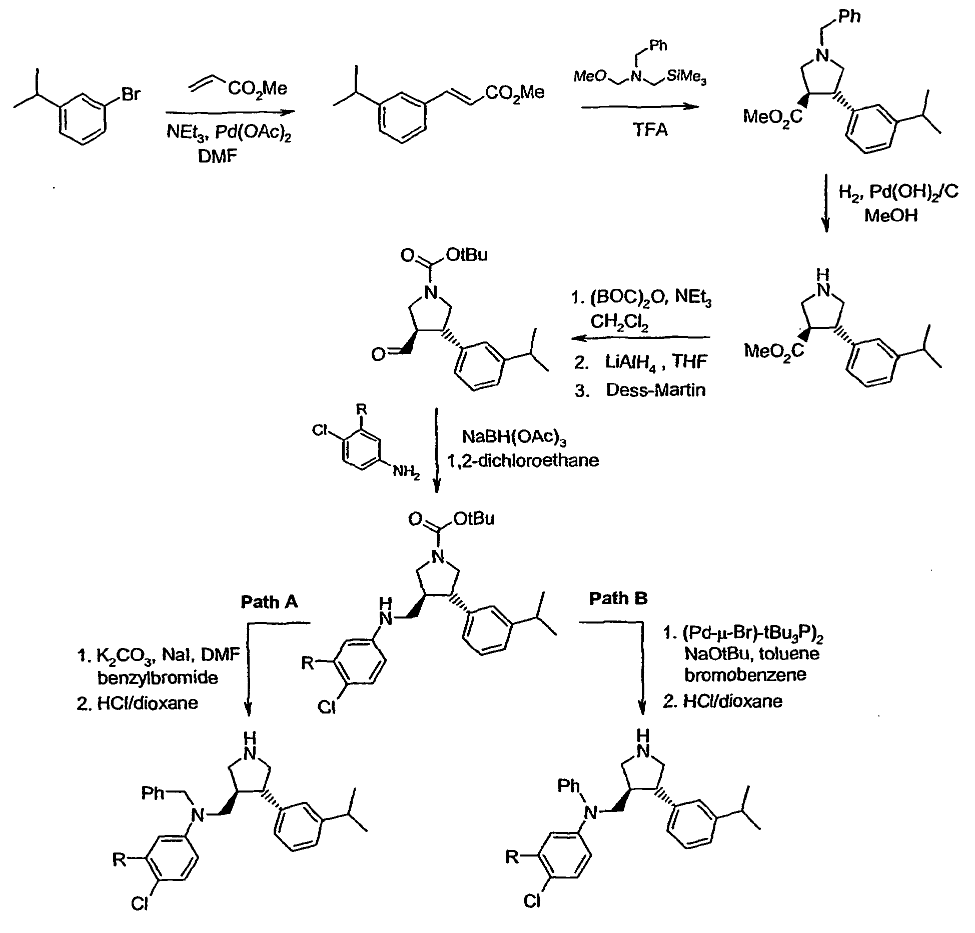

### Example 8: (3R*,4R*)-Benzyl-(4-chloro-phenyl)-[4-(3-isopropyl-phenyl)-pyrrolidin-3-ylmethyl]-amine

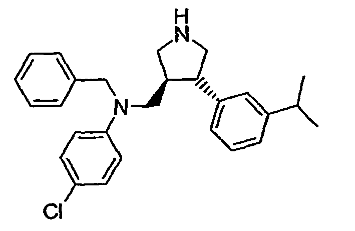

### A. (E)-3-(3-Isopropyl-phenyl)-acrylicacid methyl ester

A solution of 1-bromo-3-isopropylbenzene (5.0 g, 24 mmol), methyl acrylate (4.3 mL, 48 mmol), triethylamine (5.0 mL, 36 mmol), Pd(OAc)₂ (0.16 g, 0.7 mmol) and tri-ortho-tolylphosphine (0.45 g, 1.4 mmol) in DMF (50 mL) is heated under reflux overnight. The solvent was removed *in vacuo*, the residue is taken up in AcOEt, washed with water, dried over Na₂SO₄, filtered and concentrated. The crude product is purified by flash chromatography on silica gel (eluent: hexane/AcOEt 98/2) to give the title compound. TLC, Rf (hexane/AcOEt 19/1) = 0.25. MS(EI+): 222 (M+18).

### B. (3R*,4R*)-1-Benzyl-4-(3-isopropyl-phenyl)-pyrrolidine-3-carboxylic acid methyl ester

To an ice-cold solution of (E)-3-(3-isopropyl-phenyl)-acrylicacid methyl ester (3.6 g, 18 mmol) in toluene (65 mL), N-benzyl-N-(methoxymethyl) trimethylsilyl amine (7 mL, 26 mmol) is added, followed by trifluoroacetic acid (0.09 mL, 1.8 mmol), and the reaction mixture is stirred at ambient temperature overnight. The reaction is quenched with a saturated aqueous NaHCO₃ solution extracted with CH₂Cl₂, dried over Na₂SO₄ and concentrated. The crude product is purified by flash chromatography on silica gel (eluent : hexane/EtOAc 85:15) to give the title compound as a pale yellow oil. TLC, Rf (hexane/AcOEt 9/1) = 0.20. MS(EI+): 338 (M+1).

### C. (3R*,4R*)-4-(3-isopropyl-phenyl)-pyrrolidine-3-carboxylic acid methyl ester

A mixture of (3R*,4R*)-1-benzyl-4-(3-isopropyl-phenyl)-pyrrolidine-3-carboxylic acid methyl ester (5.9 g, 17 mmol) and Pd(OH)₂/C (0.17 g, 50% wet) in EtOH (100 mL) is stirred overnight under an hydrogen atmosphere. The crude material is filtered over a pad of Celite, washed with EtOH and concentrated. The crude product is purified by flash chromatography on silica gel (eluent: CH₂Cl₂ /MeOH 9/1) to give the title compound. TLC, Rf (CH₂Cl₂ /MeOH 9/1)=0.39. MS(EI+): 248 (M+1).

### D. (3R*,4R*)-4-(3-Isopropyl-phenyl)-pyrrolidine-1,3-dicarboxylic acid 1-tert-butyl ester 3-ethyl ester

To a solution of (3R*,4R*)-4-(3-isopropyl-phenyl)-pyrrolidine-3-carboxylic acid methyl ester (4.2 g, 16.8 mmol) in CH₂Cl₂ (100 mL) is added triethylamine (3.2 mL, 23.5 mmol) followed by (BOC)₂O (4.8 g, 22 mmol). After stirring overnight at ambient temperature, the mixture is washed with water and brine. The organic layer is dried over Na₂SO₄, filtered and concentrated. The crude product is purified by flash chromatography on silica gel (eluent: CH₂Cl₂) to give the title compound. TLC, Rf (CH₂Cl₂) = 0.31, t_{R} (HPLC, Nucleosil C18 column, 20-100% CH₃CN/H₂O/6 min, 100% CH₃CN/1.5 min, 100-20% CH₃CN/H₂O/0.5 min, CH₃CN and H₂O containing 0.1% TFA, flow: 1.0 mU min): 6.00 min.

### E. (3R*,4R*)-3-Hydroxymethyl-4-(3-isopropyl-phenyl)-pyrrolidine-1-carboxylic acid tert-butyl ester

To an ice-cold solution of (3R*,4R*)-4-(3-isopropyl-phenyl)-pyrrolidine-1,3-dicarboxylic acid 1-tert-butyl ester 3-ethyl ester (5.3 g, 15.3 mmol) in THF (250 mL), a 1M-THF solution of LiAIH₄ (15.7 mL, 16 mmol) is added. After 10 min at 0°C, the reaction mixture is carefully quenched by addition of AcOEt (10mL) and solid Na₂SO₄-decahydrate. After no more gas generation is observed, TBME is added and stirring is continued for 1 h. The suspension is filtered and washed thoroughly with TBME. The filtrate is concentrated in vacuo to give the title compound, which is used without further purification. TLC, Rf (CH₂Cl₂/MeOH 19/1) = 0.32. MS(EI+): 320 (M+1).

### F. (3R*,4R*)-3-Formyl-4-(3-isopropyl-phenyl)-pyrrolidine-1-carboxylic acid tert-butyl ester

To a well stirred mixture of (3R*,4R*)-3-hydroxymethyl-4-(3-isopropyl-phenyl)-pyrrolidine-1-carboxylic acid tert-butyl ester (0.5 g, 1.6 mmol) and Dess-Martin Periodinane (1.0 g, 2.3 mmol) in CH₂Cl₂ (15 mL), water (0.1 mL) is added. The suspension is stirred overnight, then saturated aqueous NaHCO₃ is added followed by Na₂S₂O₃. After stirring for 20 min, the aqueous layer is extracted with CH₂Cl₂. The organic extract is dried over Na₂SO₄, filtered and concentrated. The crude product is purified by flash chromatography on silica gel (eluent; CH₂Cl₂/MeOH 98/2) to give the title compound. TLC, Rf (CH₂Cl₂/MeOH 19/1) = 0.44. MS(EI+): 262 (M-55).

### G. (3R*,4R*)-3-[(4-Chloro-phenylamino)-methyl]-4-(3-isopropyl-phenyl)-pyrrolidine-1-carboxylic acid tert-butyl ester

To an ice-cold solution of (3R*,4R*)-3-formyl-4-(3-isopropyl-phenyl)-pyrrolidine-1-carboxylic acid tert-butyl ester (0.25 g, 0.8 mmol) and 4-chloroaniline (0.12 g, 0.9 mmol) in 1,2-dichloroethane (8 mL), NaBH(OAc)₃ (0.24 g, 1.0 mmol) is added. After 30 min, the reaction mixture is warmed up to ambient temperature and stirred for overnight. The reaction is quenched by addition of a saturated aqueous NaHCO₃ solution. After extraction with CH₂Cl₂, the organic layer is dried over Na₂SO₄, filtered and concentrated. The crude product is purified by flash chromatography on silica gel (eluent; CH₂Cl₂/MeOH 98/2) to give the title compound. TLC, Rf (CH₂Cl₂/MeOH 98/2) = 0.49. MS(EI+): 373 (M-55).

### Path A

### H. (3R*,4R*)-3-{[Benzyl-(4-chloro-phenyl)-amino]-methyl}-4-(3-isopropyl-phenyl)-pyrrolidine-1-carboxylic acid tert-butyl ester

A vial is charged with (3R*,4R*)-3-[(4-chloro-phenylamino)-methyl]-4-(3-isopropyl-phenyl)-pyrrolidine-1-carboxylic acid tert-butyl ester (0.15 g, 0.22 mmol), benzylbromide (0.12 g, 0.65 mmol), K₂CO₃ (0.09 g, 0.65 mmol), sodium iodide (0.10 g, 0.65 mmol) in air and suspended in DMF (12 mL). The vial is sealed with an Al crimp top with septum and heated for 30 min at 120°C in a microwave apparatus (PersonalChemistry). The reaction mixture is diluted with AcOEt, washed with brine, dried over Na₂SO₄, filtered and concentrated. The residue is purified by RP prep. HPLC (eluent; ACN/water) to give the title compound. TLC, Rf (CH₂Cl₂) = 0.37. MS(EI+): 519 (M+).

### I. (3R*,4R*)-Benzyl-(4-chloro-phenyl)-[4-(3-isopropyl-phenyl)-pyrrolidin-3-ylmethyl]-amine

To a solution of (3R*,4R*)-3-{[benzyl-(4-chloro-phenyl)-amino]-methyl}-4-(3-isopropylphenyl)-pyrrolidine-1-carboxylic acid tert-butyl ester (0.11 g, 0.22 mmol) in 1,4-dioxane (5 mL), 4N HCl (5 mL) in 1,4-dioxane is added. After stirring for 1 h, the solvent is removed in *vacuo,* and the residue is lyophilized overnight to give the title compound as a hydrochloride salt. TLC, Rf (CH₂Cl₂/MeOH 9/1) = 0.32. MS(EI+): 419 (M+)

### Path B

### Example 9: (3R*,4R*)-(4-Chloro-phenyl)-[4-(3-isopropyl-phenyl)-pyrrolidin-3-ylmethyl]-phenylamine

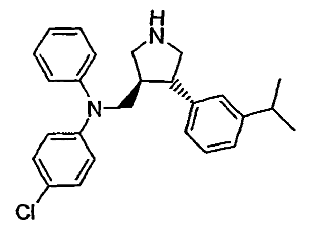

### J. (3R*,4R*)-3-{[(4-Chloro-phenyl)-phenyl-amino]-methyl}-4-(3-isopropyl-phenyl)-pyrrolidine-1-carboxylic acid tert-butyl ester

A two-necked flask, equipped with a magnetic stirring bar, septum and condenser with an argon inlet-outlet is charged with [Pd(µ-Br)(t-Bu₃P)]₂ (13 mg, 5 mol%), NaOtBu (72 mg, 0.51 mmol), (3R*,4R*)-3-[(4-chloro-phenylamino)-methyl]-4-(3-isopropyl-phenyl)-pyrrolidine-1-carboxylic acid tert-butyl ester (145 mg, 0.34 mmol) and bromobenzene (64 mg, 0.41 mmol). Abs. toluene (8 mL) is added, and the mixture is stirred for 10 min at RT and heated 24 h under a gentle reflux. After cooling to RT, the reaction is quenched with a saturated aqueous NaHCO₃ solution, extracted with AcOEt, dried over Na₂SO₄ and concentrated. The residue is purified by flash chromatography on silica gel (CH₂Cl₂) to give the title compound. TLC, Rf (CH₂Cl₂) = 0.44. MS(EI+): 505 (M+).

### K. (3R*,4R*)-(4-Chloro-phenyl)-[4-(3-isopropyl-phenyl)-pyrrolidin-3-ylmethyl]-phenylamine

To a solution of (3R*,4R*)-3-{[(4-chloro-phenyl)-phenyl-amino]-methyl}-4-(3-isopropylphenyl)-pyrrolidine-1-carboxylic acid tert-butyl ester (0.15 g, 0.29 mmol) in 1,4-dioxane (5 mL), 4N HCl (5 mL) in 1,4-dioxane is added. After stirring for 2 h, the solvent is removed *in vacuo,* and the residue is lyophilized overnight to give the title compound as a hydrochloride salt. TLC, Rf (CH₂Cl₂/MeOH 9/1) = 0.25. MS(EI+): 405 (M+1)

### Example 10: (3R*,4R*)-Benzyl-(4-chloro-3-methoxy-phenyl)-[4-(3-isopropyl-phenyl)-pyrrolidin-3-ylmethyl]-amine

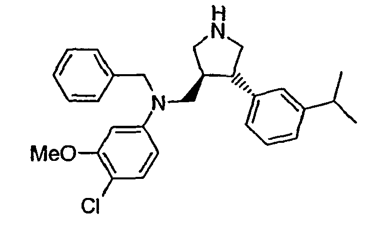

The title compound is prepared analogously as described for the title compound under I in Example 8 (Scheme6 path A) using 4-chloro-3-methoxy-phenylamine in reductive amination step G. TLC, Rf (CH₂Cl₂/MeOH 9/1) = 0.23. MS(EI+): 449 (M+1)

### Example 11: (3R*,4R*)-(4-Chloro-3-methoxy-phenyl)-[4-(3-isopropyl-phenyl)-pyrrolidin-3-ylmethyl]-phenyl-amine

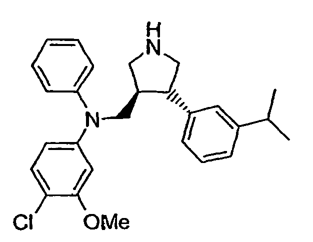

The title compound is prepared analogously as described for the title compound under I in Example 9 (Scheme6 **path B**) using 4-chloro-3-methoxy-phenylamine in reductive amination step G. TLC, Rf (CH₂Cl₂/MeOH 9/1) = 0.23. MS(EI+): 435 (M+).

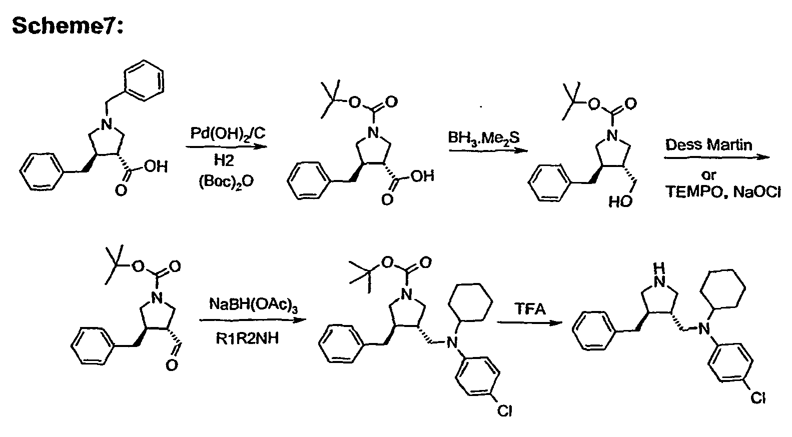

### Example 12: ((3R*,4R*)-4-Benzyl-pyrrolidin-3-ylmethyl)-(4-chloro-phenyl)-cyclohexyl-amine

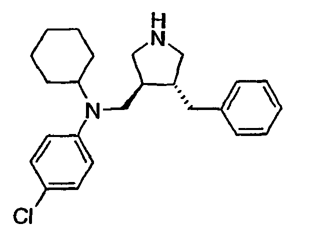

### A. (3R*,4R*)-4-Benzyl-pyrrolidine-1,3-dicarboxylic acid 1-tert-butyl ester

A mixture of (3R*,4R*)-1,4-dibenzyl-pyrrolidine-3-carboxylic acid (50 g, 0.169 mol), di-tert-butylcarbonate (37.1 g, 0.169 mol) and Pd(OH)₂/C 20% (5 g, 50% wet) in EtOH (1 L) is stirred under an hydrogen atmosphere for 6 h. The crude material is filtered over a pad of Celite, dried over Na₂SO₄, filtered and concentrated under reduced pressure to give the title compound. TLC, Rf (CH₂Cl₂/MeOH 95/5) = 0.33. MS (LC-MS): 304.2 [M+H]⁺.

### B. (3R*,4R*)-3-Benzyl-4-hydroxymethyl-pyrrolidine-1-carboxylic acid tert-butyl ester

To a solution of (3R*,4R*)-4-benzyl-pyrrolidine-1,3-dicarboxylic acid 1-tert-butyl ester (47.2 g, 0.154 mol) in THF (340 mL), a solution of borane dimethylsulfide complex (2N in THF, 123.5 mL, 0.247 mol) is slowly added at -10°C. The mixture is stirred for 80 min at -10°C then allowed to reach RT and further stirred overnight. The mixture is carefully poured into MeOH and concentrated under reduced pressure. The residue is taken up in CH₂Cl₂ and extracted with an aqueous saturated solution of NaHCO₃. The organic layer is dried over Na₂SO₄, filtered and concentrated under reduced pressure to afford the title compound. TLC, Rf (CH₂Cl₂/MeOH 90/10) = 0.6. t_{R} (HPLC, Nucleosil C18 column, 10 to 90% CH₃CN in H₂O in 11 min, CH₃CN and H₂O containing 0.1 % TFA, flow: 1.5 mL/min): 5.29 min.

### (3R,4R)-3-Benzyl-4-hydroxymethyl-pyrrolidine-1-carboxylic acid tert-butyl ester and (3S,4S)-3-Benzyl-4-hydroxymethyl-pyrrolidine-1-carboxylic acid tert-butyl ester

The two enantiomers are separated via chiral preparative HPLC (Chiracel OJ, Daicel Chemical Industries, LTD.) 10x50 cm 20 um, flow: 120 mL/min, UV = 210 nM, injection = 1.2 g) (eluent: heptane/EtOH 85/45):
(3R,4R)-3-Benzyl-4-hydroxymethyl-pyrrolidine-1-carboxylic acid tert-butyl ester, t_{R} 32.5 min.
(3S,4S)-3-Benzyl-4-hydroxymethyl-pyrrolidine-1-carboxylic acid tert-butyl ester: t_{R} 40.9 min.

### C. Alternative a) (3R*,4R*)-3-Benzyl-4-formyl-pyrrolidine-1-carboxylic acid tert-butyl ester

To a well-stirred mixture of (3R*,4R*)-3-benzyl-4-hydroxymethyl-pyrrolidine-1-carboxylic acid tert-butyl ester (10 g, 34.3 mol) and Dess-Martin periodinane (14.55 g, 34.3 mmol) in CH₂Cl₂ (200 mL), slowly wet CH₂Cl₂ (37.7 mmol, 0.68 mL of water in 50 mL of CH₂Cl₂) is added. The clear solution becomes cloudy towards the end of wet CH₂Cl₂ addition. The mixture is diluted with Et₂O and concentrated to a few mL of solvent by rotary evaporation. The residue is taken up in Et₂O, and washed with a 1/1 10% Na₂S₂O₃ / saturated aqueous solution of NaHCO₃, followed by H₂O and brine. The aqueous washings are back-extracted with Et₂O, and this organic layer is washed with H₂O and brine. The combined organic layers are dried over Na₂SO₄, filtered and concentrated under reduced pressure. The crude material is purified by flash chromatography on silica gel (eluent; c-hexane/AcOEt 2/1) to give the title compound as a slightly yellow oil. TLC, Rf (c-hexane/AcOEt 2/1) = 0.5. ¹H NMR (DMSO, 400 MHz): δ = 1.49 (s, 9H), 2.7-2.88 (m, 4H), 3.08 (dd, 1H), 3.32 (dd, 1H), 3.48-3.58 (m, 2H), 7.23 (m, 3H), 7.32 (m, 2H), 9.5 (m, 1H).

### Alternative b) (3R*,4R*)-3-Benzyl-4-formyl-pyrrolidine-1-carboxylic acid tert-butyl ester

To a mixture of (3R*,4R*)-3-benzyl-4-hydroxymethyl-pyrrolidine-1-carboxylic acid tert-butyl ester (60 g, 0.206 mol) and TEMPO (0.96 g, 0.006 mol) in toluene/AcOEt (1/1, 2L), at RT a solution of KBr (36.6 g, 0.309 mol) in water (100 mL) is added. The resulting mixture is cooled to 0°C, before the dropwise addition of a water (1 L) solution containing KHCO₃ (77.4 g, 0.773 mol) and NaOCl (57.5 g, 0.772 mol) follows. The resulting reaction mixture is further stirred for 1 h at 0°C and 3 h at RT. The layers are separated, and the aqueous one is back-extracted twice with toluene/AcOEt (1/1, 500 mL). The combined organic extracts are washed with a solution (3 L) containing water / 10% aqueous solution of Na₂S₂O₃ / 10% aqueous solution of KHSO₄ (1/1/1), dried over Na₂SO₄, filtered and concentrated under reduced pressure. The crude material is purified by flash chromatography on silica gel (eluent; c-hexane/AcOEt 2/1) to give the title compound as a slightly yellow oil.

### D. (3R*,4S*)-3-Benzyl-4-{[(4-chloro-phenyl)-cyclohexyl-amino]-methyl}-pyrrolidine-1-carboxylic acid tert-butylester

This reaction is performed as described in the literature (see Abdel-Magid A.F. et al J. Org. Chem., 1996, 61, 3849-3862): (3R*,4R*)-3-Benzyl-4-formyl-pyrrolidine-1-carboxylic acid tert-butyl ester (50 mg, 0.173 mmol) and (4-chloro-phenyl)-cyclohexyl-amine (35 mg, 0.169 mmol) are mixed in 1,2-dichloroethane (1 mL) and treated with sodium triacetoxyborohydride (51 mg, 0.24 mmol) and AcOH (9.9 µL, 0.17 mmol). The mixture is stirred at RT under nitrogen overnight, quenched by the addition of 5 mL aqueous saturated solution of NaHCO₃, extracted with CH₂Cl₂, dried over Na₂SO₄ and concentrated. The crude oil is purified by flash chromatography on silica gel (eluent: c-hexane/AcOEt 90/10) to give the title compound. TLC, Rf (hexane/AcOEt 80/20) = 0.6.

### E. ((3R*,4R*)-4-Benzyl-pyrrolidin-3-ylmethyl)-(4-chloro-phenyl)-cyclohexyl-amine

To a solution of (3R*4S*)-3-benzyl-4-{[(4-chloro-phenyl)-cyclohexyl-amino]-methyl}-pyrrolidine-1-carboxylic acid tert-butylester (161 mg, 0.334 mmol) in CH₂Cl₂ (3 mL), trifluoroacetic acid (230 µL, 3 mmol) is added. The mixture is stirred overnight at RT, concentrated and poured into a saturated aqueous solution of NaHCO₃. The organic layer is extracted, and the aqueous one is back-extracted twice with CH₂Cl₂, dried over Na₂SO₄ and concentrated under reduced pressure. The crude material is purified by flash chromatography on silica gel (eluent: CH₂Cl₂/MeOH 95/5 to 90/10 with 1% NH₄OH) to give the title compound. Addition of 83 µl of 4N HCl/dioxane (0.33 mmol) to a solution of the product in dioxane (2 mL) and lyophilization affords the corresponding hydrochloride salt as a white solid. MS (LC-MS): 383.1, 385.0 [M+H]⁺; t_{R} (HPLC, Nucleosil C18 column, 10 to 90% CH₃CN in H₂O in 11 min, CH₃CN and H₂O containing 0.1 % TFA, flow: 1.5 mL/min): 5.08 min.

### (4-chloro-phenyl)-cyclohexyl-amine

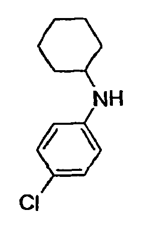

A solution of cyclohexanone (0.4 mL, 3.92 mmol) and 4-chloro-phenyl amine (0.5 g, 3.92 mmol) in dichloromethane (3.5 mL) is stirred for 15 min, followed by the addition of sodium triacetoxyborohydride (1.16 g, 5.79 mmol) and AcOH (0.22 mL, 3.92 mmol). The mixture is stirred at RT under nitrogen overnight, diluted with CH₂Cl₂, quenched by the addition of 5 mL of aqueous saturated solution of NaHCO₃, extracted with CH₂Cl₂, dried over Na₂SO₄ and concentrated under reduced pressure. The crude residue is purified by flash chromatography on silica gel (eluent: c-hexane/AcOEt 95/5 to 90/10) to give the title compound. MS (LC-MS): 210.0, 212.0 [M+H]⁺; t_{R} (HPLC, Nucleosil C18 column, 10 to 100% CH₃CN in H₂O in 5 min, 100% CH₃CN for 3 min, CH₃CN and H₂O containing 0.1% TFA, flow: 1.5 mL/min): 4.31 min.

### Example 13: ((3R*,4R*)-4-Benzyl-pyrrolidin-3-ylmethyl)-(4-chloro-phenyl)-((R)-1-phenylethyl)-amine

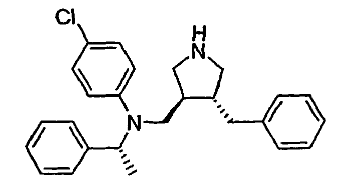

The title compound is prepared analogously as described for the title compound under E in Example 12 from (3R*,4R*)-3-benzyl-4-formyl-pyrrolidine-1-carboxylic acid tert-butyl ester and (4-chloro-phenyl)-((R)-1-phenyl-ethyl)-amine. MS (LC-MS): 405, 406 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.76 min.

### (4-Chloro-phenyl)-((R)-1-phenyl-ethyl)-amine

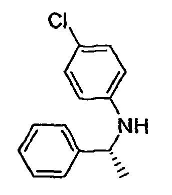

This reaction is performed as described in the literature (see Buchwald S.L.. et al J. Org. Chem., 2000, 65, 1144-1157). A flask is charged with 1-bromo-4-chlorobenzene (2.07 g, 10.8 mmol), R-(+)-1-phenylethylamin (1.51 mL, 11.88 mmol), sodium tert-butoxide (1.453 g, 15.12 mmol), tris-(dibenzylideneacetonee)dipalladium (24.7 mg, 0.027 mmol), BINAP (rac.) (50.4 mg, 0.081 mmol), and toluene (21 mL) under argon. The flask is immersed in a 80°C sand bath with stirring over the weekend. The solution is then allowed to cool to RT, taken up in ether, filtered, and concentrated under reduced pressure. The crude material is purified by flash chromatography on silica gel (eluent: c-hexane/AcOEt 95/5) to give the title compound. TLC, Rf (c-hexane/AcOEt 80/20) = 0.7. MS (LC-MS): 232.0 [M+H]⁺.

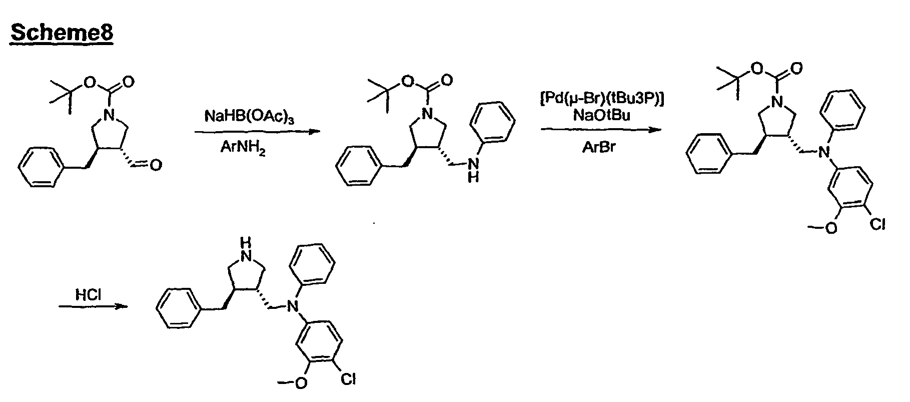

### Example 14: ((3R*,4R*)-4-Benzyl-pyrrolidin-3-ylmethyl)-(4-chloro-3-methoxy-phenyl)-phenylamine

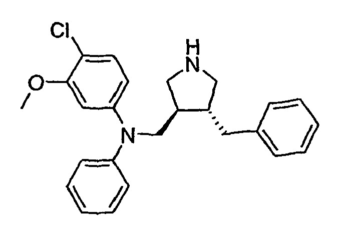

### A. (3R*,4S*)-3-Benzyl-4-phenylaminomethyl-pyrrolidine-1-carboxylic acid tert-butyl ester

This reaction is performed as described in the literature (see Abdel-Magid A.F. et al J. Org. Chem., 1996, 61, 3849-3862). (3R*,4R*)-3-Benzyl-4-formyl-pyrrolidine-1-carboxylic acid tert-butyl ester (2.4 g, 8.29 mmol) and aniline (0.75 mL, 8.29 mmol) are mixed in 1,2-dichloroethane (50 mL) and treated with sodium triacetoxyborohydride (2.46 g, 11.6 mmol) and AcOH (0.5 mL, 8.29 mmol). The mixture is stirred at RT under nitrogen for 3 h, quenched by addition of an aqueous saturated solution of NaHCO₃, extracted with CH₂Cl₂, dried over Na₂SO₄ and concentrated under reduced pressure to give the title compound. MS (LC-MS): 311.0 [M+H-tBu]⁺; t_{R} (HPLC, Nucleosil C18 column, 10-100% CH₃CN/H₂O in 5 min, 100% CH₃CN for 3 min, CH₃CN and H₂O containing 0.1% TFA, flow: 1.5 mL/min): 5.49 min.

### B. (3R*,4S*)-3-Benzyl-4-{[(4-chloro-3-methoxy-phenyl)-phenyl-amino]-methyl}-pyrrolidine-1-carboxylic acid tert-butyl ester

This reaction is performed as described in the literature (see Prashad M. et al J. Org. Chem., 2003, 68, 1163-1164). A two-necked flask, equipped with a magnetic stirring bar, septum and condenser with an argon inlet-outlet is charged with [Pd(µ-Br)(t-Bu₃P)]₂ (1.2 mg, 0.25% mol), NaOtBu (40 mg, 0.409 mmol), (3R*,4S*)-3-benzyl-4-phenylaminomethyl-pyrrolidine-1-carboxylic acid tert-butyl ester (100 mg, 0.273 mmol) and 2-chloro-5-bromo-anisol (73 mg, 0.327 mmol). Dry de-aerated toluene (3 mL) is added. The mixture is stirred at RT for 20 min under an Argon stream and then heated under a gentle reflux overnight. After 17 h, the mixture is diluted with CH₂Cl₂ and extracted twice with water, and the organic layer is dried over Na₂SO₄, filtered, concentrated and purified by flash chromatography on silica gel (eluent: CH₂Cl₂/MeOH 100/0 to 98/2) to give the title compound. t_{R} (HPLC, Nucleosil C18 column, 10-100% CH₃CN/H₂O in 5 min, 100% CH₃CN/3 min, CH₃CN and H₂O containing 0.1 % TFA, flow: 1.5 mUmin): min.

### C. ((3R*,4R*)-4-Benzyl-pyrrolidin-3-ylmethyl)-(4-chloro-3-methoxy-phenyl)-phenylamine

A solution of (3R*,4S*)-3-benzyl-4-{[(4-chloro-3-methoxy-phenyl)-phenyl-amino]-methyl}-pyrrolidine-1-carboxylic acid tert-butyl ester (250 mg, 0.493 mmol) in cc HCl/ iPrOH (1:1) (3 mL) is stirred for 1 h at RT and then concentrated under reduced pressure. The residual oil is diluted in CH₂Cl₂, washed with an aqueous saturated solution of NaHCO₃, dried over Na₂SO₄, concentrated and purified by flash chromatography on silica gel (eluent: CH₂Cl₂/MeOH 95/5 to 90/10 with 1% NH₄OH) to give the title compound. Addition of 4N HCl/dioxane (1 equivalent) to a solution of the product in dioxane (2 mL) and lyophilization affords the corresponding hydrochloride salt as a white solid. MS (LC-MS): 407, 408 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.12 min.

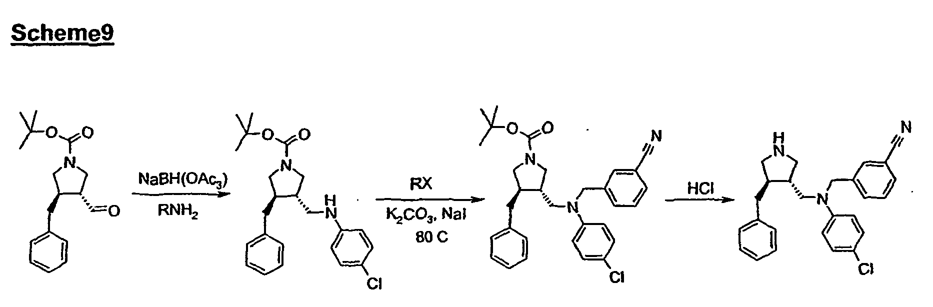

### Example 15: 3-{[((3R*,4R*)-4-Benzyl-pyrrolidin-3-ylmethyl)-(4-chloro-phenyl)-amino]-methyl}-benzonitrile

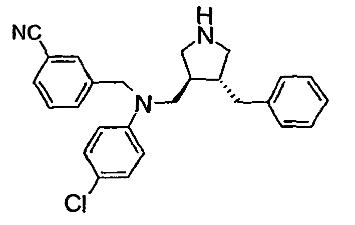

### A. (3R*,4S*)-3-Benzyl-4-[(4-chloro-phenylamino)-methyl]-pyrrolidine-1-carboxylic acid tert-butyl ester

This reaction is performed as described in the literature (see Abdel-Magid A.F. et al J. Org. Chem., 1996, 61, 3849-3862). (3R*,4R*)-3-Benzyl-4-formyl-pyrrolidine-1-carboxylic acid tert-butyl ester (2.51 g, 8.67 mmol) and 4-chloroaniline (1.084 g, 8.497 mmol) are mixed in 1,2-dichloroethane (20 mL) and treated with sodium triacetoxyborohydride (2.573 mg, 12.138 mmol). The mixture is stirred 3 h at RT under nitrogen, quenched by addition of 20 mL aqueous saturated solution of NaHCO₃, extracted twice with CH₂Cl₂, dried over Na₂SO₄ and concentrated under reduced pressure to give the title compound as a yellowish oil which is used without further purification. MS (LC-MS): 345 [M+H-tBu]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 6.55 min.

### B. (3R*,4S*)-3-Benzyl-4-{[(4-chloro-phenyl)-(3-cyano-benzyl)-amino]-methyl}-pyrrolidine-1-carboxylic acid tert-butyl ester

A mixture 3-benzyl-4-[(4-chloro-phenylamino)-methyl]-pyrrolidine-1-carboxylic acid tert-butyl ester (500 mg, 1.3 mmol), 3-bromomethyl-benzonitrile (189 mg, 1.3 mmol), K₂CO₃ (259 mg, 1.9 mmol) and Nal (25 mg, 0.17 mmol) in DMF (10 mL) is stirred under Ar at 80°C for 2 h. For workup, H₂O is added and the mixture is extracted with ethyl acetate. Drying (Na₂SO₄) of the combined extracts and evaporation of the solvent affords the crude product which is purified by flash column chromatography (80g SiO₂, c-hexane/EtOAc 4/1) to give 3-benzyl-4-{[(4-chloro-phenyl)-(3-cyano-benzyl)-amino]-methyl}-pyrrolidine-1-carboxylic acid tert-butyl ester as a yellowish oil. MS (LC-MS): 460.0 [M+H-tert-butyl]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 7.22 min.

### C. 3-{[((3R*,4R*)-4-Benzyl-pyrrolidin-3-ylmethyl)-(4-chloro-phenyl)-amino]-methyl}-benzonitrile

Concentrated HCl (1.0 mL) is added to a solution of (3R*,4S*)-3-benzyl-4-{[(4-chloro-phenyl)-(3-cyano-benzyl)-amino]-methyl}-pyrrolidine-1-carboxylic acid tert-butyl ester (100 mg, 0.2 mmol) in isopropanol (1.0 mL). After stirring at RT for 90 min, the reaction solution is concentrated under reduced pressure, diluted with CH₂Cl₂ and quenched by careful addition of a saturated solution of NaHCO₃. 1 N NaOH is added, until the aqueous layer has a basic pH. Extraction with CH₂Cl₂, drying of the combined organic extracts (Na₂SO₄) and evaporation of the solvent affords the crude product which is purified by flash column chromatography (CH₂Cl₂ → CH₂Cl₂/MeOH 95:5) to give 3-{[(4-benzyl-pyrrolidin-3-ylmethyl)-(4-chloro-phenyl)-amino]-methyl}-benzonitrile. Treatment of the product with 4N HCl/dioxane (23 µl, 0.09 mmol) and lyophilization afford the corresponding mono-hydrochloride as a white solid. MS (LC-MS): 416.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mUmin): 5.32 min.

### Example 16: Benzyl-((3R*,4R*)-4-benzyl-pyrrolidin-3-ylmethyl)-(4-chloro-phenyl)-amine

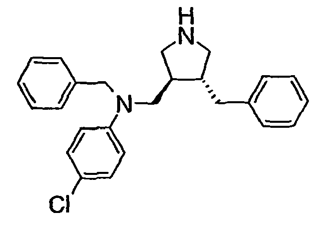

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and benzylbromide. MS (LC-MS): 391, 393 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.44 min.

### Example 17: Benzyl-((3R,4R)-4-benzyl-pyrrolidin-3-ylmethyl)-(4-chloro-phenyl)-amine

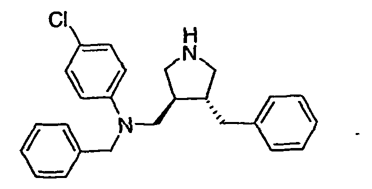

The title compound is prepared analogously as described for the title compound under C in Example 15 from (3R,4S)-3-benzyl-4-[(4-chloro-phenylamino)-methyl]-pyrrolidine-1-carboxylic acid tert-butyl ester and benzylbromide. MS (LC-MS): 391, 393 [M+H]⁺; t_{R} (HPLC, Nucleosil C18 column, 10 to 90% CH₃CN in H₂O in 11 min, CH₃CN and H₂O containing 0.1 % TFA, flow: 1.5 mUmin): 5.47 min.

### Example 18: Benzyl-((3S,4S)-4-beozyl-pyrrolidin-3-ylmethyl)-(4-chloro-phenyl)-amine

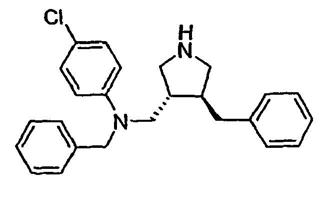

The title compound is prepared analogously as described for the title compound under C in Example 15 from (3S,4R)-3-benzyl-4-[(4-chloro-phenylamino)-methyl]-pyrrolidine-1-carboxylic acid tert-butyl ester and benzylbromide. MS (LC-MS): 391, 392.9 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.48 min.

### Example 19: ((3R*,4R*)-4-Benzyl-pyrrolidin-3-ylmethyl)-(4-chloro-phenyl)-(2-methoxybenzyl)-amine

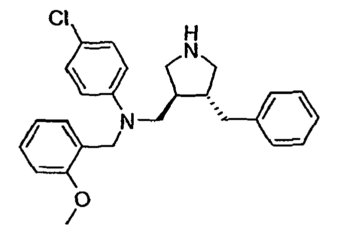

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 1-chloromethyl-2-methoxy-benzene. MS (LC-MS): 421, 423 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.53 min.

### Example 20: ((3R*,4R*)-4-Benzyl-pyrrolidin-3-ylmethyl)-(4-chloro-phenyl)-(3-methoxybenzyl)-amine

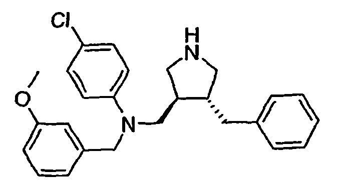

The title compound is prepared analogously as described for the title compound under C in Example 15 from (3R*,45*)-3-benzyl-4-[(4-chloro-phenylamino)-methyl]-pyrrolidine-1-carboxylic acid tert-butyl ester and 1-chloromethyl-3-methoxy-benzene. MS (LC-MS): 421, 423 [M+H]⁺; t_{R} (HPLC, Nucleosil C18 column, 10 to 90% CH₃CN in H₂O in 11 min, CH₃CN and H₂O containing 0. 1 % TFA, flow: 1.5 mL/min): 5.48 min.

### Example 21: Benzyl-((3R*,4R*)-4-benzyl-pyrrolidin-3-ylmethyl)-(4-methoxy-phenyl)-amine

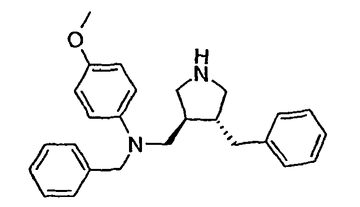

The title compound is prepared analogously as described for the title compound under C in Example 15 from (3R*,4R*)-3-benzyl-4-formyl-pyrrolidine-1-carboxylic acid tert-butyl ester and 4-methoxy-phenylamine followed by alkylation reaction using benzylbromide. MS (LC-MS): 387.1 [M+H]⁺; t_{R} (HPLC, Nucleosil C18 column, 10-90% CH₃CN/H₂O in 11 min, CH₃CN and H₂O containing 0.1% TFA, flow: 1.5 mL/min): 4.70 min.

### Example 22: Benzyl-((3R*,4R*)-4-benzyl-pyrrolidin-3-ylmethyl)-p-tolyl-amine

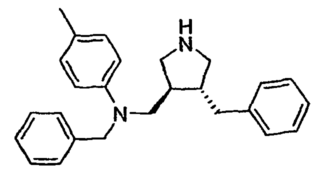

The title compound is prepared analogously as described for the title compound under C in Example 15 from (3R*,4R*)-3-Benzyl-4-formyl-pyrrolidine-1-carboxylic acid tert-butyl ester and 4-methyl-phenylamine followed by alkylation reaction using benzylbromide. MS (LC-MS): 371.1 [M+H]⁺; t_{R} (HPLC, Nucleosil C18 column, 10-90% CH₃CN/H₂O in 11 min, CH₃CN and H₂O containing 0.1% TFA, flow: 1.5 mUmin): 5.24 min.

### Example 23: Benzyl-((3R*,4R*)-4-benzyl-pyrrolidin-3-ylmethyl)-(4-fluoro-phenyl)-amine

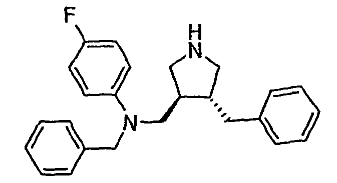

The title compound is prepared analogously as described for the title compound under C in Example 15 from (3R*,4R*)-3-benzyl-4-formyl-pyrrolidine-1-carboxylic acid tert-butyl ester and 4-fluoro-phenylamine followed by alkylation reaction using benzylbromide. MS (LC-MS): 375 [M+H]⁺; t_{R} (HPLC, Nucleosil C18 column, 10-90% CH₃CN/H₂O in 11 min, CH₃CN and H₂O containing 0.1% TFA, flow: 1.5 mL/min): 5.30 min.

### Example 24: ((3R*,4R*)-4-Benzyl-pyrrolidin-3-ylmethyl)-(4-chloro-phenyl)-naphthalen-1-ylmethyl-amine

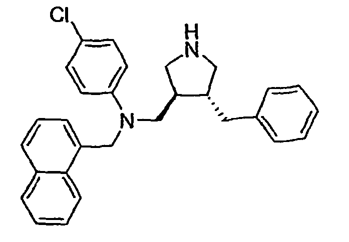

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 1-chloromethyl-naphthalene. MS (LC-MS): 442.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.87 min.

### Example 25: 3-{[((3R*,4R*)-4-Benzyl-pyrrolidin-3-ylmethyl)-(4-chloro-phenyl)-amino]-methyl}-benzamide

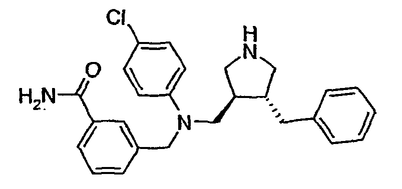

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 3-chloromethyl-benzamide. MS (LC-MS): 434.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.76 min.

### Example 26: ((3R*,4R*)-4-Benzyl-pyrrolidin-3-ylmethyl)-(4-chloro-phenyl)-naphthalen-2-ylmethyl-amine

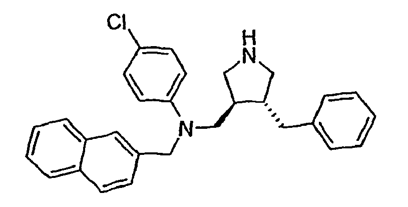

The title compound is prepared analogously as described for the title compound under C in Example 15 from (3R*,45*)-3-benzyl-4-[(4-chloro-phenylamino)-methyl]-pyrrolidine-1-carboxylic acid tert-butyl ester and 2-bromomethyl-naphthalene. MS (LC-MS): 441.9 [M+H]⁺ t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.79 min.

### Example 27: ((3R*,4R*)-4-Benzyl-pyrrolidin-3-ylmethyl)-(4-chloro-phenyl)-(4-propoxy-2-trifluoromethyl-quinolin-6-ylmethyl)-amine

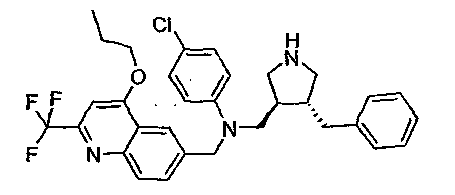

The title compound is prepared analogously as described for the title compound under C in Example 15 from {3R*,4S*)-3-benzyl-4-[(4-chloro-phenylamino)-methyl]-pyrrolidine-1-carboxylic acid tert-butyl ester and 6-bromomethyl-4-propoxy-2-trifluoromethyl-quinoline. MS (LC-MS): 583.1 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 6.27 min.

### Example 28: 7-{[((3R*,4R*)-4-Benzyl-pyrrolidin-3-ylmethyl)-(4-chloro-phenyl)-amino]-methyl}-naphthalene-2-carbonitrile

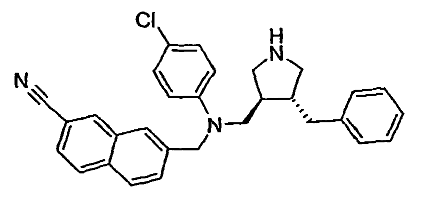

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 7-bromomethyl-naphthalene-2-carbonitrile. MS (LC-MS): 467.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mUmin): 5.64 min.

### Example 29: 7-{[((3R*,4R*)-4-Benzyl-pyrrolidin-3-ylmethyl)-(4-chloro-phenyl)-amino]-methyl}-naphthalene-1-carbonitrile

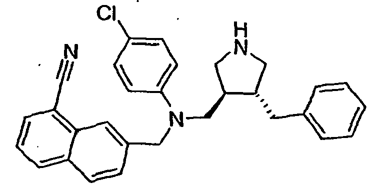

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 7-bromomethyl-naphthalene-1-carbonitrile. MS (LC-MS): 465.9 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.64 min.

### Example 30: 6-{[((3R*,4R*)-4-Benzyl-pyrrolidin-3-ylmethyl)-(4-chloro-phenyl)-amino]-methyl}-naphthalene-2-carbonitrile

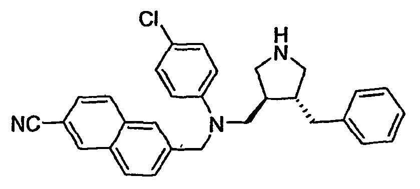

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 6-bromomethyl-naphthalene-2-carbonitrile. MS (LC-MS): 466.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.66 min.

### Example 31: 6-{[((3R*,4R*)-4-Benzyl-pyrrolidin-3-ylmethyl)-(4-chloro-phenyl)-amino]-methyl}-1H-pyrimidine-2,4-dione

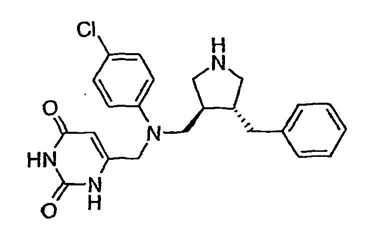

The title compound is prepared analogously as described for the title compound under C in Example 15 from (3R*,45*)-3-benzyl-4-[(4-chloro-phenylamino)-methyl]-pyrrolidine-1-carboxylic acid tert-butyl ester and 6-chloromethyl-1H-pyrimidine-2,4-dione. MS (LC-MS): 424.9 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.51 min.

### Example 32: 5-{[((3R*,4R*)-4-Benzyl-pyrrolidin-3-ylmethyl)-(4-chloro-phenyl)-amino]-methyl}-naphthalene-2-carbonitrile

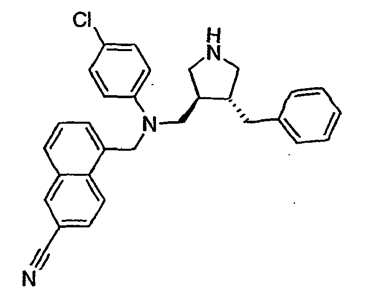

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 5-bromomethyl-naphthalene-2-carbonitrile. MS (LC-MS): 465.9 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.56 min.

### Example 33: 5-{[((3R*,4R*)-4-Benzyl-pyrrolidin-3-ylmethyl)-(4-chloro-phenyl)-amino]-methyl}-naphthalene-1-carbonitrile

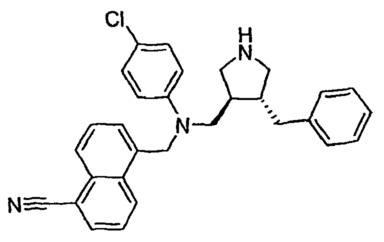

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 5-bromomethyl-naphthalene-1-carbonitrile. MS (LC-MS): 465.9 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.53 min.

### Example 34: 4-{[((3R*,4R*)-4-Benzyl-pyrrolidin-3-ylmethyl)-(4-chloro-phenyl)-amino]-methyl}-naphthalene-1-carbonitrile

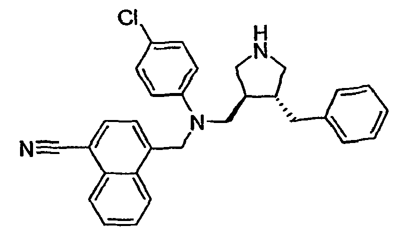

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 4-bromomethyl-naphthalene-1-carbonitrile. MS (LC-MS): 466.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.52 min.

### Example 35: 4-{[((3R*,AR*)-4-Benzyl-pyrrolidin-3-ylmethyl)-(4-chloro-phenyl)-amino]-methyl}-benzonitrile

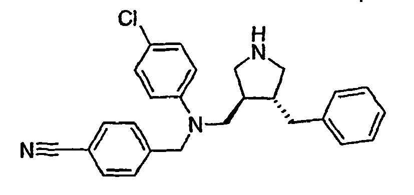

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 4-bromomethyl-benzonitrile. MS (LC-MS): 416.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.15 min.

### Example 36: 5-{[((3R*,4R*)-4-Benzyl-pyrrolidin-3-ylmethyl)-(4-chloro-phenyl)-amino]-methyl}-2-fluoro-benzonitrile

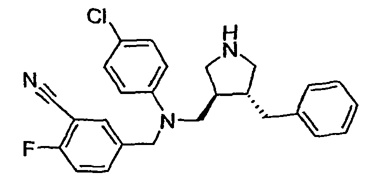

The title compound is prepared analogously as described for the title compound under C in Example 15 from (3R*,4S*)-3-benzyl-4-[(4-chloro-phenylamino)-methyl]-pyrrolidine-1-carboxylic acid tert-butyl ester and 5-bromomethyl-2-fluoro-benzonitrile. MS (LC-MS): 434.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.25 min.

### Example 37: 4-[((3R*,4R*)-4-Benzyl-pyrrolidin-3-ylmethyl)-naphthalen-2-ylmethyl-amino]-benzonitrile

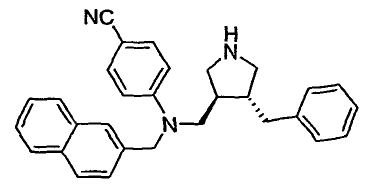

The title compound is prepared analogously as described for the title compound under C in Example 15 from (3R*,4R*)-3-benzyl-4-formyl-pyrrolidine-1-carboxylic acid tert-butyl ester and 4-cyano-phenylamine followed by alkylation reaction using 2-bromomethyl-naphthalene. MS (LC-MS): 432.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.16 min.

### Example 38: 4-{[((3R*,4R*)-4-Benzyl-pyrrolidin-3-ylmethyl)-(4-chloro-benzyl)-amino]-methyl}-benzonitrile

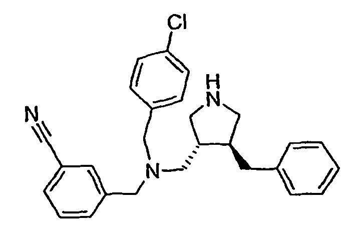

The title compound is prepared analogously as described for the title compound under C in Example 15 from (3R*,4R*)-3-benzyl-4-formyl-pyrrolidine-1-carboxylic acid tert-butyl ester and 4-chlorobenzylamine followed by alkylation reaction using 3-bromomethyl-benzonitrile. MS (LC-MS): 430.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.68 min.

### Example 39: N-(2-{[((3R*,4R*)-4-Benzyl-pyrrolidin-3-ylmethyl)-(4-chloro-phenyl)-amino]-methyl}-phenyl)-acetamide (see also Scheme15 above)

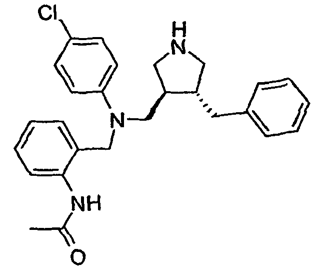

### A. (3R*,4S*)-3-Benzyl-4-{[(4-chloro-phenyl)-(2-nitro-benzyl)-amino]-methyl}-pyrrolidine-1-carboxylic acid tert-butyl ester

A vial is charged with (3R*,4R*)-3-[(4-chloro-phenylamino)-methyl]-4-(benzyl)-pyrrolidine-1-carboxylic acid tert-butyl ester (2 g, 4.99 mmol), 2-nitro-benzylchloride (2.57 g, 14.97 mmol), triethylamine (1.04 mL, 7.48 mmol) and sodium iodide (0.97 g, 6.49 mmol) in air and suspended in DMF (8 mL). The vial is sealed with an Al crimp top with septum and heated for 30 min at 120°C in a microwave apparatus (PersonalChemistry). The reaction mixture is diluted with water and AcOEt , and the aqueous layer is extracted with AcOEt. The combined organic extracts are washed with brine, dried over Na₂SO₄, filtered and concentrated. The residue is purified by flash chromatography on silica gel (eluent; c-hexane/AcOEt 90/10) to give the title compound. MS (LC-MS): 536.0 [M-H]⁺. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 7.38 min.

### B. (3S*,4R*)-3-{[(2-Amino-benryl)-(4-chloro-phenyl)-amino]-methyl}-4-benzyl-pyrrolidine-1-carboxylic acid tert-butyl ester

H₂ is bubbled through a suspension of (3R*,4S*)-3-benzyl-4-{[(4-chloro-phenyl)-(2-nitrobenzyl)-amino]-methyl}-pyrrolidine-1-carboxylic acid tert-butyl ester (0.5 g, 0.93 mmol) and Raney-Niclcel (50 mg) in MeOH (40 mL) during 20 h. The catalyst is filtered off and washed with MeOH. Concentration of the solution affords the crude material which is purified by flash chromatography on silica gel (eluent: c-hexane/AcOEt 95/5) to give the desired title product. MS (LC-MS): 507.0 [M-H]⁺. t_{R} (HPLC, Nucleosil C18 column, 10-100% CH₃CN/H₂O/5 min, 100% CH₃CN/3 min, CH₃CN and H₂O containing 0.1 % TFA, flow: 1.5 mL/min): 6.09 min.

### C. (3S*,4R*)-3-{[(2-Acetylamino-benzyl)-(4-chloro-phenyl)-amino]-methyl}-4-benzyl-pyrrolidine-1-carboxylic acid tert-butyl ester

To a solution of (3S*,4R*)-3-{[(2-amino-benzyl)-(4-chloro-phenyl)-amino]-methyl}-4-benzyl-pyrrolidine-1-carboxylic acid tert-butyl ester (120 mg, 0.237 mmol) in pyridine (1.30 mL), acetic anhydride (27 µL, 0.237 mmol) is added at 0 °C, and the mixture is stirred for 2 days at RT. The reaction mixture is concentrated under *vaccum,* the reside is diluted with CH₂Cl₂, washed with an aqueous saturated solution of NaHCO₃, dried over Na₂SO₄, filtered and concentrated. The crude product is purified by flash chromatography on silica gel (eluent c-hexan/AcOEt 2/1) to give the title product. MS (LC-MS): 548.0 [M-H]⁺. t_{R} (HPLC, Nucleosil C18 column, 10-100% CH₃CN/H₂O/5 min, 100% CH₃CN/3 min, CH₃CN and H₂O containing 0.1 % TFA, flow: 1.5 mL/min): 6.63 min.

### D. N-(2-{[((3R*,4R*)-4-Benzyl-pyrrolidin-3-ylmethyl)-(4-chloro-phenyl)-amino]-methyl}-phenyl)-acetamide

TFA (181 µL) is added to a solution of (3S*,4R*)-3-{[(2-acetylamino-benzyl)-(4-chloro-phenyl)-amino]-methyl}-4-benzyl-pyrrolidine-1-carboxylic acid tert-butyl ester (97.9 mg, 0.179 mmol) in CH₂Cl₂ (2 mL). The resulting mixture is stirred at RT for 24 h, diluted with CH₂Cl₂ and quenched with a saturated aqueous solution of NaHCO₃. The layers are separated and the aqueous one is extracted twice with CH₂Cl₂, dried over Na₂SO₄, filtered and concentrated. The crude material is purified by flash chromatography on Isolute SPE Flash NH₂ column (eluent CH₂Cl₂/MeOH 98/2 to 95/5) to give the title. MS (LC-MS): 448.0 [M-H]⁺. t_{R} (HPLC, Nucleosil C18 column, 10-100% CH₃CN/H₂O/5 min, 100% CH₃CN/3 min, CH₃CN and H₂O containing 0.1 % TFA, flow: 1.5 mL/min): 4.82 min.

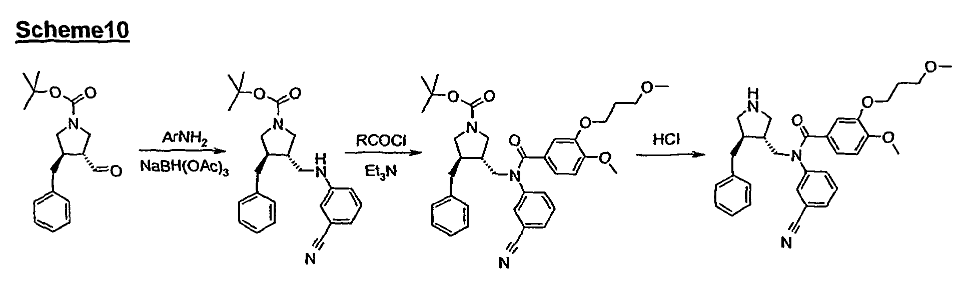

### Example 40: N-((3R*,4R*)-4-Benzyl-pyrrolidin-3-ylmethyl)-N-(3-cyano-phenyl)-4-methoxy-3-(3-methoxy-propoxy)-benzamide, hydrochloride

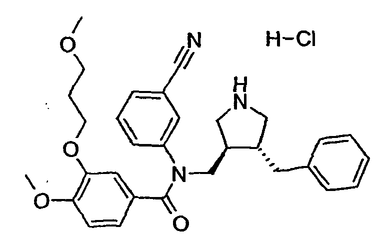

### A. ((3R*,4S*)-3-Benzyl-4-[(3-cyano-phenylamino)-methyl]-pyrrolidine-1-carboxylic acid tert-butyl ester

This reaction is performed according to a method known from the literature (see Abdel-Magid A.F. et al J. Org. Chem., 1996, 61, 3849-3862). A mixture of (3R*,4R*)-3-benzyl-4-formyl-pyrrolidine-1-carboxylic acid tert-butyl ester (150 mg, 0.52 mmol), 3-aminobenzonitrile (184.3 mg, 1.56 mmol) and 1,2-dichloroethane (5 mL) is stirred at RT for 10 min. Sodium triacetoxyborohydride (157 mg, 0.74 mmol) and AcOH (30 µl, 0.52 mmol) are added, and the mixture is stirred for 14 h at RT. The reaction is then quenched by the addition of a saturated aqueous NaHCO₃ solution. After separation of the organic layer, the aqueous phase is extracted twice with CH₂Cl₂. The combined organic extracts are dried (Na₂SO₄), and the solvent is removed *in vacuo.* The crude product is purified by preparative HPLC (C18 column 150x30 mm, 10-100% CH₃CN + 0.1%TFA / H₂O + 0.1%TFA / 30 min). The combined pure fractions are neutralized by the addition of saturated aqueous Na₂CO₃ solution, and CH₃CN is removed *in vacuo.* The remaining aqueous phase is extracted twice with CH₂Cl₂. The combined organic layers are dried (Na₂SO₄) and evaporated *in vacuo* to afford (3R*,4S*)-3-benzyl-4-[(3-cyano-phenylamino)-methyl]-pyrrolidine-1-carboxylic acid tert-butyl ester. MS: 390.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 7.63 min

### B. (3R*,4S*)-3-Benzyl-4-({(3-cyano-pheny)-[4-methoxy-3-(3-methoxy-propoxy)-benzoyl]-amino}-methyl)-pyrrolidine-1-carboxylic acid tert-butyl ester

A mixture of (3R*,4S*)-3-benzyl-4-[(3-cyano-phenylamino)methyl]-pyrrolidine-1-carboxylic acid tert-butyl ester (146 mg, 0.37 mmol), 4-methoxy-3-(3-methoxy-propoxy)-benzoyl chloride (106 mg, 0.41 mmol) and triethylamine (67 µl, 0.48 mmol) in CH₂Cl₂ (2 mL) is stirred at RT for 14 h and then quenched by the addition of aqueous NaHCO₃ solution. The organic layer is separated, and the aqueous phase is extracted three times with CH₂Cl₂. The combined organic extracts are dried (Na₂SO₄), and the solvent is removed *in vacuo.* The crude product is purified by preparative HPLC (C18 column 150x30 mm, 10-100% CH₃CN + 0.1%TFA, / H₂O + 0.1%TFA /30 min). The combined pure fractions are neutralized by the addition of saturated aqueous Na₂CO₃ solution, and CH₃CN is removed *in vacuo.* The remaining aqueous phase is extracted twice with CH₂Cl₂. The combined organic layers are dried (Na₂SO₄) and evaporated *in vacuo* to afford (3R*,4S*)-3-benzyl-4-({(3-cyano-pheny)-[4-methoxy-3-(3-methoxy-propoxy)-benzoyl]-amino}-methyl)-pyrrolidine-1-carboxylic acid tert-butyl ester. MS: 614.5 [M+H]⁺; t_{R} (HPLC, C18 column, 5-100% CH₃CN 0.1%TFA/H₂O+0.1%TFA/8 min, 100% CH₃CN+0.1%TFA/2 min, flow 1.5 mL/min): 7.26 min

### C. N-((3R*,4R*)-4-Benzyl-pyrrolidin-3-ylmethyl)-N-(3-cyano-phenyl)-4-methoxy-3-(3-methoxy-propoxy)-benzamide, hydrochloride

A mixture of (3R*,4S*)-3-benzyl-4-({(3-cyano-pheny)-[4-methoxy-3-(3-methoxy-propoxy)-benzoyl]-amino}-methyl)-pyrrolidine-1-carboxylic acid tert-butyl ester (117.2 mg, 0.216 mmol) and HCl (5M in 2-propanol, 2 mL, 10 mmol) is stirred for 2 h at RT. The reaction mixture is evaporated *in vacuo* to afford the title compound as a white solid. MS: 514.6 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 4.79 min.

### 4-Methoxy-3-(3-methoxy-propoxy)-benzoyl chloride

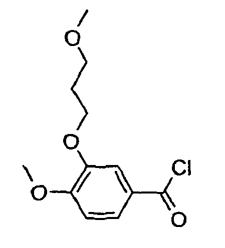

### a) 4-Methoxy-3-(3-methoxy-propoxy)-benzoic acid methyl ester

A solution of methyl-3-hydroxy-4-methoxybenzoate (89.3 g, 0.49 mol), K₂CO₃ (100.5 g, 0.727 mol) and 1-bromo-3-methoxy-propane (80 g, 0.523 mol) in CH₃CN (1100 mL) is refluxed for 6 h. After completion of the reaction, the mixture is cooled to RT and concentrated under reduced pressure. The residue is taken up into EtOAc (500 mL) and washed with water. The aqueous layer is back-extracted twice with EtOAc, and the combined organic extracts are dried over MgSO₄, filtered and concentrated to afford the title compound which is further used in the next step without purification. t_{R} (HPLC, CC 70/4 Nucleosil 3 C18HD column, 20 to 100% CH₃CN in H₂O in 2, then 4 min with 100% CH₃CN, CH₃CN and H₂O with 0.1% TFA, flow: 1.5 mUmin): 3.07 min.

### b) 4-Methoxy-3-(3-methoxy-propoxy)-benzoic acid

A solution of 4-methoxy-3-(3-methoxy-propoxy)-benzoic acid methyl ester (140 g, 0.55 mol) and NaOH (1N, 825 mL, 0.825 mol) in MeOH (840 mL) is stirred at RT for 18 h. After completion, the solvent is removed under reduced pressure, and the residue is diluted with water (200 mL) and extracted twice with EtOAc (250 mL). The aqueous layer is acidified by addition of aqueous HCl (2N, 470 mL) and extracted 3 times with EtOAc (1 L). The combined organic extracts are washed with brine, dried over Na₂SO₄, filtered and concentrated. The crude material is purified by crystallization in EtOAc to give the title compound. MS (LC-MS): 239.1 [M-H]⁻; t_{R} (HPLC, CC 70/4 Nucleosil 3 C18HD column, 20 to 100% CH₃CN in H₂O in 2, then 4 min with 100% CH₃CN, CH₃CN and H₂O with 0.1% TFA, flow: 1.5 mL/min): 2.43 min.

### c) 4-Methoxy-3-(3-methoxy-propoxy)-benzoyl chloride

4-Methoxy-3-(3-methoxy-propoxy)-benzoic acid (634 mg, 2.64 mmol) is suspended in CH₂Cl₂ (10 mL) under N₂ and treated with oxalyl chloride (818 µL, 9.37 mmol). The reaction mixture is stirred overnight at RT and concentrated under reduced pressure. The resulting crude acid chloride is taken up in toluene (10 mL) and concentrated again. This operation is repeated 3 times to ensure complete removal of the excess of oxalyl chloride. The crude oil is further applied into the next step without purification.

### Example 41: N-((3R*,4R*)-4-Benzyl-pyrrolidin-ylmethyl)-3,N-diphenyl-propionamide hydrochloride

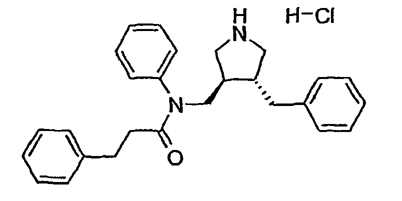

The title compound is prepared analogously as described for the title compound under C in Example 40. MS (LC-MS): 399.6 [M+H]⁺; t_{R} (HPLC, C18 column, 5-100% CH₃CN+0.1% TFA /H₂O+0.1% TFA/8 min, 100% CH₃CN+0.1%TFA/2 min, flow 1.5 mUmin): 5.57 min.

### Example 42: (1R*,2R*)-2-Phenl-cyclopropanecarbosylic acid ((3R*,4R*)-4-benzyl-pyrrolidin-3-ylmethyl)-phenyl-amide, hydrochloride

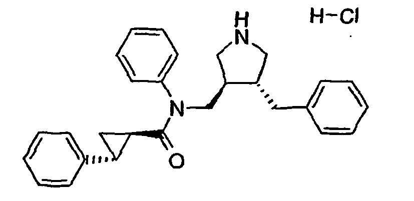

The title compound is prepared analogously as described for the title compound under C in Example 40. MS (LC-MS): 411.6 [M+H]⁺; t_{R} (HPLC, C18 column, 5-100% CH₃CN+0.1% TFA/H₂O+0.1% TFA/8 min, 100% CH₃CN+0.1%TFA/2 min, flow 1.5 mL/min): 5.61 and 5.65 min (diastereomers).

### Example 43: (1R*,2R*)-2-Phenyl-cyclopropanecarboxylic acid ((3R*,4R*)-4-benzyl-pyrrolidin-3-ylmethyl)-(4-chloro-phenyl)-amide, hydrochloride

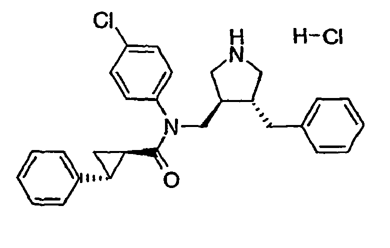

The title compound is prepared analogously as described for the title compound under C in Example 40. MS: 445.4 [M+H]⁺; t_{R} (HPLC, C18 column, 5-100% CH₃CN+0.1%TFA/H₂O+0.1% TFA/8 min, 100% CH₃CN+0.1%TFA/2 min, flow 1.5 mUmin): 6.15 min.

### Example 44: Naphthalene-2-carboxylic acid ((3R*,4R*)-4-benzyl-pyrrolidin-3-ylmethyl)-(4-chloro-phenyl)-amide, hydrochloride

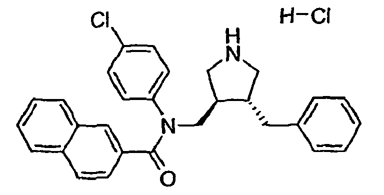

The title compound is prepared analogously as described for the title compound under C in Example 40. MS: 455.3 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 6.09 min.

### Example 45: Naphthalene-1-carboxylic acid ((3R*,4R*)-4-benzyl-pyrrolidin-3-ylmethyl)-(4-chloro-phenyl)-amide, hydrochloride

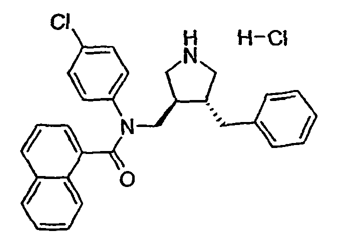

The title compound is prepared analogously as described for the title compound under C in Example 40. MS: 455.3 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mUmin): 5.98 min.

### Example 46: N-((3R*,4R*)-4-Benzyl-pyrrolidin-3-ylmethyl)-N-(4-chloro-phenyl)-4-methoxy-3-(3-methoxy-propoxy)-benzamide, hydrochloride

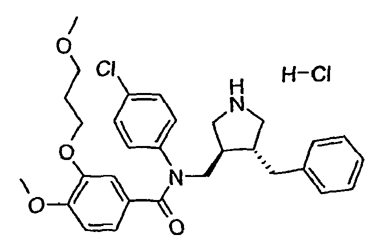

The title compound is prepared analogously as described for the title compound under C in Example 40. MS: 523.4 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 5.48 min.

### Example 47: N-((3R*,4R*)-4-Benzyl-pyrrolidin-3-ylmethyl)-4-methoxy-3-(3-methoxy-propoxy)-N-phenyl-benzamide, hydrochloride

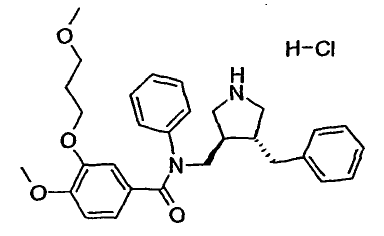

The title compound is prepared analogously as described for the title compound under C in Example 40. MS: 489.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 5.24 min.

### Example 48: N-((3R*,4R*)-4-Benzyl-pyrrolidin-3-ylmethyl)-N-(4-cyano-phenyl)-4-methoxy-3-(3-methoxy-propoxy)-benzamide, hydrochloride

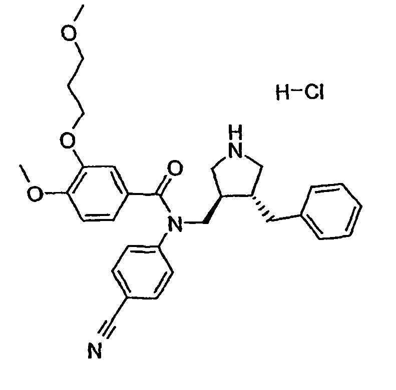

The title compound is prepared analogously as described for the title compound under C in Example 40. MS: 514.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 4.97 min.

### Example 49: Naphthalene-2-carboxylic acid ((3R*,4R*)-4-berizyl-pyrrolidin-3-ylmethyl)-(4-cyano-phenyl)-amide

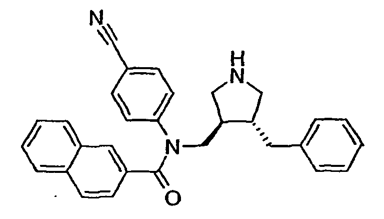

The title compound is prepared analogously as described for the title C compound in Example 40. MS (LC-MS): 446.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.02 min.

### Example 50: Benzofuran-5-carboxylic acid ((3R*,4R*)-4-benzyl-pyrrolidin-3-ylmethyl)-(4-cyano-phenyl)-amide

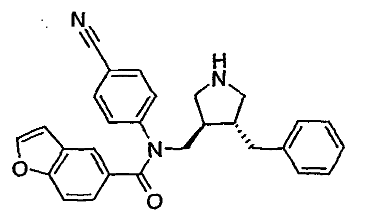

The title compound is prepared analogously as described for the title compound under C in Example 40. MS (LC-MS): 436.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.61 min.

### Example 51: Naphthalene-2-carboxylic acid ((3R*,4R*)-4-benzyl-pyrrolidin-3-ylmethyl)-(4-phenoxy-phenyl)-amide

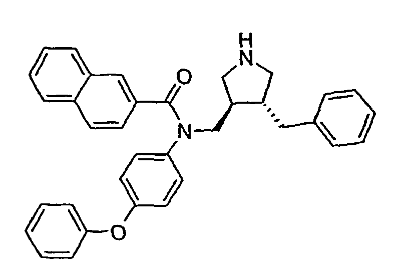

The title compound is prepared analogously as described for the title compound under C in Example 40. MS (LC-MS): 513.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.41 min.

### Example 52: Benzofuran-5-carboxylic acid ((3R*,4R*)-4-benzyl-pyrrolidin-3-ylmethyl)-(4-chloro-phenyl)-amide

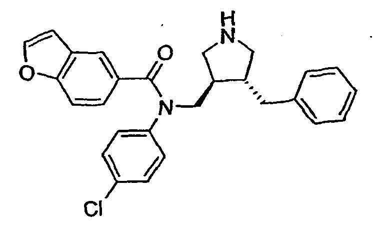

The title compound is prepared analogously as described for the title compound under C in Example 40. MS (LC-MS): 445.1 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.89 min.

### Example 53: N-((3R*,4R*)-4-Benzyl-pyrrolidin-3-ylmethyl)-N-(4-chloro-phenyl)-2-phenoxy-benzamide

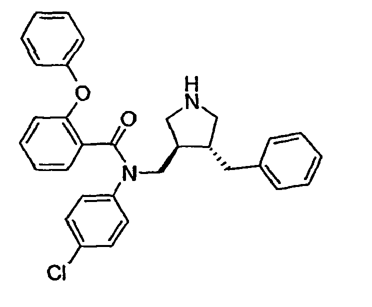

The title compound is prepared analogously as described for the title compound under C in Example 40. MS (LC-MS): 497.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.28 min.

### Example 54: N-((3R*,4R*)-4-Benzyl-pyrrolidin-3-ylmethyl)-N-(4-chloro-phenyl)-benzenesulfonamide, hydrochloride

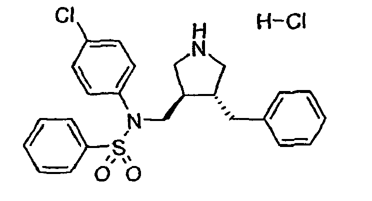

### A. (3R*,45*)-3-{[benzenesulfonyl-(4-chloro-phenyl)-amino]-methyl}-4-benzyl-pyrrolidine-1-carboxylic acid tert-butyl ester

A mixture of ((3R*,45*)-3-benzyl-4-[(4-chloro-phenylamino)-methyl]-pyrrolidine-1-carboxylic acid tert-butyl ester (compound A under Example 15) (120.3 mg, 0.3 mmol), benzenesulfonyl chloride (96.2 µl, 0.75 mmol), N-ethyldiisopropylamine (342.4 µl, 2 mmol) and 4-(dimethylamino)pyridine (20 mg, 0.16 mmol) in CH₂Cl₂ (2 mL) is stirred at RT for 16 h. The reaction mixture is diluted with CH₂Cl₂ and washed with 2N HCl and saturated aqueous NaHCO₃ solution. The organic layer is dried, (Na₂SO₄) and the solvent is removed *in vacuo.* The crude product is purified by preparative HPLC (C18 column 150x30 mm, 10-100% CH₃CN + 0.1 %TFA / H₂O + 0.1 %TFA / 30 min). The combined pure fractions are neutralized by the addition of saturated aqueous NaHCO₃ solution, and CH₃CN is removed *in vacuo.* The remaining aqueous phase is extracted twice with CH₂Cl₂. The combined organic layers are dried (Na₂SO₄) and evaporated *in vacuo* to afford (3R*,4S*)-3-{[benzenesulfonyl-(4-chloro-phenyl)-amino]-methyl}-4-benzyl-pyrrolidine-1-carboxylic acid tert-butyl ester. MS: 541.2 [M+H]⁺; t_{R} (HPLC, C18 column, 5-100% CH₃CN +0.1%TFA/H₂O+0.1%TFA/8 min, 100% CH₃CN+0.1%TFA/2 min, flow 1.5 mL/min): 8.39 min.

### B. N-((3R*,4R*)-4-Benzyl-pyrrolidin-3-ylmethyl)-N-(4-chloro-phenyl)-benzenesulfonamide, hydrochloride

A mixture of (3R*,45*)-3-{[benzenesulfonyl-(4-chloro-phenyl)-amino]-methyl}-4-benzyl-pyrrolidine-1-carboxylic acid tert-butyl ester (117.2 mg, 0.216 mmol) and HCl (5M in 2-propanol, 2 mL, 10 mmol) is stirred for 2h at RT. The reaction mixture is evaporated *in vacuo* to afford the title compound as a slightly beige amorphous solid. MS: 441.3 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 5.82 min.

### Example 55: ((3R*,4R*)-4-Benzyl-pyrrolidin-3-ylmethyl)-(4-chloro-phenyl)-phenethyl-amine

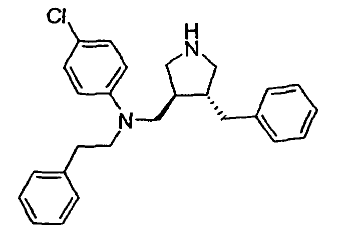

### A. (3R*,4S*)-3-Benzyl-4-{[(4-chloro-phenyl)-Phenylacetyl-amino]-methyl}-pyrrolidine-1-carboxylic acid tert-butyl ester

The title compound is prepared analogously as described for the title compound under B in Example 40.

### B. ((3R*,4R*)-4-Benzyl-pyrrolidin-3-ylmethyl)-(4-chloro-phenyl)-phenethyl-amine.

To a solution of (3R*,4S*)-3-benzyl-4-{[(4-chloro-phenyl)-phenylacetyl-amino]-methyl}-pyrrolidine-1-carboxylic acid tert-butyl ester (130 mg, 0.25 mmol) in THF (2 mL), BH₃-Me₂S (2M in THF, 0.75 mL) is added. The mixture is stirred for 2 h and concentrated under vacuum. The residual oil is refluxed overnight in a mixture of cc HCl /MeOH (1/1, 4 mL), then diluted with CH₂Cl₂ and basified with an aqueous saturated solution of NaHCO₃. The layers are separated, and the aqueous one is back-extracted twice with CH₂Cl₂. The combined organic extracts are dried over Na₂SO₄, filtered and concentrated. The crude material is purified by flash chromatography on silica gel (eluent: CH₂Cl₂/MeOH 95/5 to 90/10 +1% NH₄OH) to give the title compound. The corresponding hydrochloric salt is obtained by adding HCl 4N in dioxane (1 equivalent) to a solution of the compound in dioxane (2 mL) and lyophilization. MS (LC-MS): 405, 407 [M+H]⁺; t_{R} (HPLC, Nucleosil C18 column, 10 to 90% CH₃CN in H₂O in 11 min, CH₃CN and H₂O containing 0.1 % TFA, flow: 1.5 mL/min): 5.75 min.

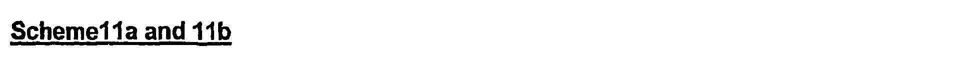

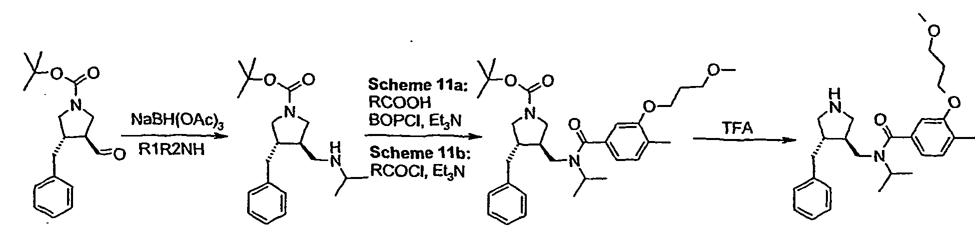

### Example 56 (Scheme11a): N-((3S*,4S*)-4-Benzyl-pyrrolidin-3-ylmethyl)-N-isopropyl-3-(3-methoxy-propoxy)-4-methyl-benzamide

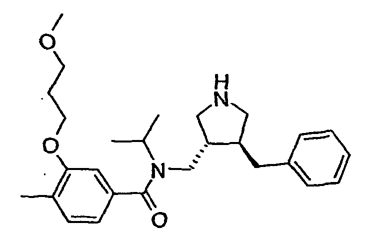

### A. (3R*,4S*)-3-Benzyl-4-(isopropylamino-methyl)-pyrrolidine-1-carboxylic acid tert-butyl ester

This reaction is performed according to known literature methods (see Abdel-Magid A.F. et al J. Org. Chem., 1996, 61, 3849-3862). A mixture of (3R*,4R*)-3-benzyl-4-formyl-pyrrolidine-1-carboxylic acid tert-butyl ester (2.64 g, 9.12 mmol), isopropyl amine (2.35 mL, 27.37 mmol) and 1,2-dichloroethane (150 mL) is stirred at RT for 10 minutes. Sodium triacetoxyborohydride (4.83 g, 22.81 mmol) is added, and the mixture is stirred for 20 h at RT. The reaction is quenched by the addition of a saturated aqueous NaHCO₃ solution. After separation of the organic layer, the aqueous phase is extracted twice with CH₂Cl₂. The combined organic extracts are dried (Na₂SO₄), and the solvent is removed *in vacuo* to afford the title compound as a colorless oil. MS: 333.1 [M+H]⁺; t_{R} (HPLC, Nucleosil C18 column, 10-100% CH₃CN+0.1%TFA in H₂O+0.1%TFA in 5 min, 100% CH₃CN+0.1%TFA 3 min, CH₃CN and H₂O containing 0.1% TFA, flow 1.5 mUmin): 4.66 min.

### B. (3S*,4R*)-3-Benzyt-4-({isopropyl-[3-(3-methoxy-propoxy)-4-methyl-benzoyl]-amino}-methyl)-pyrrolidine-1-carboxylic acid tert-butyl ester

A mixture of (3R*,4S*)-3-benzyl-4-(isopropylamino-methyl)-pyrrolidine-1-carboxylic acid tert-butyl ester (200 mg, 0.6 mmol), 3-(3-methoxy-propoxy)-4-methyl-benzoic acid (148 mg, 0.66 mmol), bis(2-oxo-3-oxazolidinyl)phosphinic chloride (168 mg, 0.66 mmol) and triethylamine (334 µL, 2.4 mmol) in CH₂Cl₂ (5 mL) is stirred at reflux for 4 h and then quenched by the addition of aqueous NaHCO₃ solution. The organic layer is separated, and the aqueous phase is extracted three times with CH₂Cl₂. The combined organic extracts are dried (Na₂SO₄), and the solvent is removed *in vacuo.* The crude product is purified by flash chromatography on silica gel (eluent : c-hexane/AcOEt 80/20 to 70/30) to give the title product. TLC, Rf (c-hexane/AcOEt 50/50) = 0.6.

### C. N-((3S*,4S*)-4-Benzyl-pyrrolidin-3-ylmethyl)-N-isopropyl-3-(3-methoxy-propoxy)-4-methyl-benzamide

TFA (800 µL) is added to a solution of (3S,4R)-3-benzyl-4-({isopropyl-[3-(3-methoxy-propoxy)-4-methyl-benzoyl]-amino}-methyl)-pyrrolidine-1-carboxylic acid tert-butyl ester (243.8 mg, 0.45 mmol) in CH₂Cl₂ (2 mL). The resulting mixture is stirred for 1 h at RT and concentrated, diluted with CH₂Cl₂ and quenched with a saturated aqueous solution of NaHCO₃. The layers are separated, and the aqueous one is extracted twice with CH₂Cl₂, dried over Na₂SO₄, filtered and concentrated. The crude material is purified by flash chromatography on NH₂-isolute (eluent: CH₂Cl₂/MeOH 100/0 to 95/5) to afford the title product. To a solution of the compound in dioxane (2 mL), (0.38 mmol,94.5 µL) of 4N HCl in dioxane is added, and the resulting solution is lyophilized to afford the corresponding hydrochloride salt as a white powder. MS (LC-MS): 439.1 [M+H]⁺; t_{R} (HPLC, C18 column, 10-100% CH₃CN/H₂O/5 min, 100% CH₃CN/3 min, CH₃CN and H₂O containing 0.1% TFA, flow: 1.5 mL/min): 4.95 min.

### 3-(3-Methoxy-propoxy)-4-methyl-benzoic acid

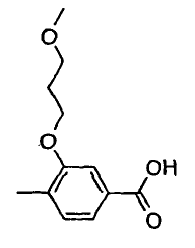

### a. 3-Hydroxy-4-methyl-benzoic acid methyl ester

To a solution of 3-hydroxy-4-methyl-benzoic acid (5 g, 32.8 mmol) in MeOH (100 mL) cc H₂SO₄ (1 mL) is added. The solution is refluxed for 14 h, then concentrated to about 30 mL and poured into water. The aqueous layer is extracted with ether (50 mL x 4) and the combined organic extracts are neutralized with a saturated aqueous solution of NaHCO₃ (50 mL x 2), washed with brine (50 mL), dried over Na₂SO₄, filtered and concentrated to give the title compound as a white powder. TLC, Rf (hexane/AcOEt 2/1 ) = 0.55. ¹H NMR (CDCl₃, 300 MHz): δ = 2.3 (s, 3H), 3.9 (s, 3H), 7.15 (d, 1H), 7. 5 (d, 1H), 7.6 (s, 1H).

### b. 3-(3-Methoxy-propoxy)-4-methyl-benzoic acid methyl ester

A solution of 3-hydroxy-4-methyl-benzoic acid methyl ester (7.7 g, 32.43 mmol), potassium carbonate (6.72 g, 48.65 mmol) and 1-iodo-3-methoxy propane (7.14 g, 35.68 mmol) in acetoneitrile (125 mL) is stirred at reflux for 26 h. The solvent is concentrated under reduced pressure, H₂O (100 mL) is added, and the aqueous layer is extracted with ether (50 mL x 4). The combined organic extracts are washed with brine, dried over anhydrous sodium sulfate and concentrated under reduced pressure to afford the title which is used without further purification in the next step. TLC, Rf (hexane/AcOEt 2/1 ) = 0.65. ¹H NMR (CDCl₃, 300 MHz): δ = 2.10 (p, 2H), 2.3 (s, 3H), 3.38 (s, 3H), 3.62 (t, 2H), 3.9 (s, 3H), 4.15 (t, 2H), 7.15 (d, 1H), 7.45 (s, 1H), 7.55 (d, 1H).

### c. 3-(3-Methoxy-propoxy)-4-methyl-benzoic acid

A solution of 3-(3-methoxy-propoxy)-4-methyl-benzoic acid methyl ester (7.12 g, 32.86 mmol) and NaOH (1 N in water, 100 mL, 100 mmol) in EtOH (100 mL) is refluxed for 1 h. The reaction mixture is allowed to reach RT, and the solvent is concentrated under reduced pressure. The residue is dissolved in water (200 mL) and washed with ether (50 mL x 3). The pH is adjusted to 2 by addition of cc HCl and the aqueous layer extracted with AcOEt (150 mL x 2). The combined organic extracts are dried over Na₂SO₄, filtered and concentrated *in vacuo*. The crude material is recrystallized in ether /hexane to afford the desired title product. TLC, Rf (hexane/AcOEt 2/1) = 0.15. MS (LC-MS): 224.0 [M-H]⁻. ¹H NMR (CDCl₃, 300 MHz): δ = 2.10 (p, 2H), 2.3 (s, 3H), 3.38 (s, 3H), 3.62 (t, 2H), 4.15 (t, 2H), 7.2 (d, 1H), 7.55 (s, 1H), 7.65 (d, 1H).

### Example 57 (Scheme11 b): N-((3S*,4S*)-4-Benzyl-pyrrolidin-3-ylmethyl)-N-isopropyl-4-methoxy-3-(3-methoxy-propoxy)-benzamide

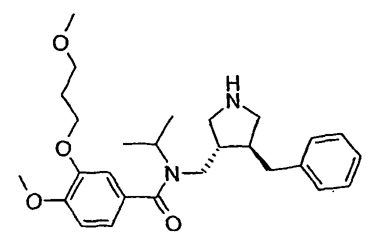

The title compound is prepared analogously as described for the title compound under C in Example 56 except that the peptidic coupling reaction is performed using an acid chloride as described in the following: A mixture of (3R*,4S*)-3-benzyl-4-(isopropylamino-methyl)-pyrrolidine-1-carboxylic acid tert-butyl ester (600 mg, 1.80 mmol), 4-methoxy-3-(3-methoxy-propoxy)-benzoyl chloride (512 mg, 1.98 mmol) and triethylamine (326 µl, 2.34 mmol) in CH₂Cl₂ (6 mL) is stirred at RT overnight and then quenched by the addition of aqueous NaHCO₃ solution. The organic layer is separated, and the aqueous phase is extracted three times with CH₂Cl₂. The combined organic extracts are dried (Na₂SO₄), and the solvent is removed *in vacuo*. The crude product is purified by flash chromatography on silica gel (eluent: c-hexane/AcOEt 2/1) to give the title compound. MS (LC-MS): 455 [M+H]⁺; t_{R} (HPLC, Nucleosil C18 column, 10 to 90% CH₃CN in H₂O in 11, CH₃CN and H₂O containing 0.1% TFA, flow: 1.5 mL/min): 4.71 min.

### Example 58: N-((3S,4S)-4-Benzyl-pyrrolidin-3-ylmethyl)-N-isopropyl-4-methoxy-3-(3-methoxy-propoxy)-benzamide

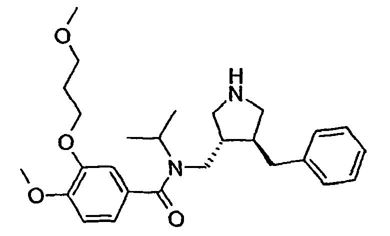

The title compound is prepared analogously as described for the title compound under C in Example 57 according to Scheme11b starting from (3S,4S)-3-benzyl-4-hydroxymethyl-pyrrolidine-1-carboxylic acid tert-butyl ester (prepared according to Scheme7). MS (LC-MS): 455 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.83 min.

### Example 59: N-((3R,4R)-4-Benzyl-pyrrolidin-3-ylmethyl)-N-isoproyl-4-methoxy-3-(3-methoxy-propoxy)-benzamide

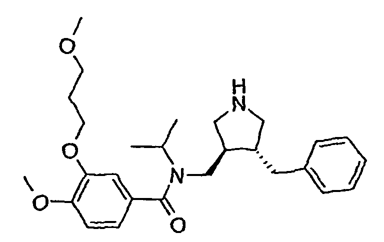

The title compound is prepared analogously as described for the title compound under C in Example 57 according to Scheme11b starting from (3R,4R)-3-benzyl-4-hydroxymethyl-pyrrolidine-1-carboxylic acid tert-butyl ester (prepared according to Scheme7). MS (LC-MS): 455 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.83 min.

### Example 60: N-((3R*,4R*)-4-Benzyl-pyrrolidin-3-ylmethyl)4-ethyl-N-isopropyl-3-(3-methoxy-propoxy)-benzamide

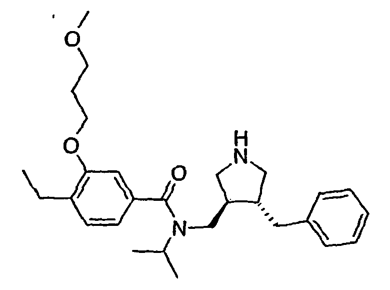

The title compound is prepared analogously as described for the title compound under C in Example 56 using Scheme11a. MS (LC-MS): 453.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 6.31 min.

### 4-Ethyl-3-(3-methoxy-propoxy)-benzoic acid

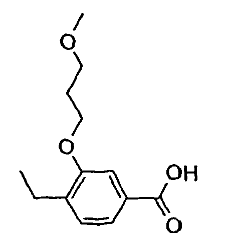

### a. 4-Bromo-3-hydroxy-benzoic acid methyl ester

To a solution of 4-bromo-3-hydroxy-benzoic acid (prepared according to J. Amer. Chem Soc. 1946, 68, 574) (5 g, 32.8 mmol) in MeOH (100 mL) , cc H₂SO₄ (1 mL) is added. The solution is refluxed for 14 h, then concentrated to about 30 mL and poured into a water. The aqueous layer is extracted with ether (50 mL x 4) and the combined organic extracts are neutralized with a saturated aqueous solution of NaHCO₃ (50 mL x 2), washed with brine (50 mL), dried over Na₂SO₄, filtered and concentrated to give the title compound as a white powder. TLC, Rf (AcOEt) = 0.9. ¹H NMR (CDCl₃, 300 MHz): δ = 5.8 (bs, 1H), 7.45 (d, 1H), 7.55 (d, 1H), 7.7 (s, 1H).

### b. 4-Bromo-3-(3-methoxy-propoxy)-benzoic acid methyl ester

A solution of 4-bromo-3-hydroxy-benzoic acid methyl ester (12 g, 51.9 mmol), potassium carbonate (10.77 g, 77.9 mmol) and 1-iodo-3-methoxy propane (11.42 g, 57.1 mmol) in acetoneitrile (250 mL) is stirred at reflux for 16 h. The solvent is concentrated under reduced pressure, H₂O (100 mL) is added, and the aqueous layer extracted with ether (50 mL x 4). The combined organic extracts are washed with brine, dried over anhydrous sodium sulfate and concentrated under reduced pressure to afford the title which was used without further purification in the next step. TLC, Rf (hexane/AcOEt 2/1) = 0.65. ¹H NMR (CDCl₃, 300 MHz): δ = 2.12 (p, 2H), 3.38 (s, 3H), 3.63 (t, 2H), 3.9 (s, 3H), 4.2 (t, 2H), 7.5 (d, 1H), 7.55 (m, 1H), 7.6 (d, 1H).

### c. 3-(3-Methoxy-propoxy)-4-trimethylsilanylethynyl-benzoic acid methyl ester

To a stirred solution of 4-bromo-3-(3-methoxy-propoxy)-benzoic acid methyl ester (5 g, 16.49 mmol) and trimethylsilyl actetylene (2.74 mL, 19.8 mmol) in triethylamine (60 mL), Cl₂Pd(PPh₃)₂ (2.31 g, 3.29 mmol) and Cul (0.314 g, 1.65 mmol) are added. The resulting mixture is stirred at RT for 15 h and concentrated under reduced pressure. The crude residue is purified by flash chromatography on silica gel (eluent hexane/EtOAc 10/1 to 5/1) to give the desired title product as a brown oil. ¹H NMR (CDCl₃, 300 MHz): δ = 0.25 (s, 9H), 2.12 (p, 2H), 3.38 (s, 3H), 3.65 (t, 2H), 3.9 (s, 3H), 4.18 (t, 2H), 7.45 (d, 1H), 7.52 (s, 1H), 7.58 (d, 1H).

### d. 4-Ethynyl-3-(3-methoxy-propoxy)-benzoic acid

To a solution of 3-(3-methoxy-propoxy)-4-trimethylsilanylethynyl-benzoic acid methyl ester (16.49 mmol) in MeOH (40 mL) is added KOH (1 N, 24.7 mL, 24.7 mmol). The resulting mixture is stirred at RT for 15 h and concentrated under reduced pressure. The residue was taken up in HCl (2 N, 100 mL) and extracted with AcOEt (100 mL x 3). The combined organic extracts are washed with brine, dried over anhydrous sodium sulfate and concentrated under reduced pressure to afford the title as a yellow oil which is used without further purification in the next step. MS (FAB): 235.0 [M+H]⁺. ¹H NMR (CDCl₃, 300 MHz): δ = 2.15 (p, 2H), 3.38 (s, 3H), 3.41 (s, 1H), 3.62 (t, 2H), 4.22 (t, 2H), 7.5 (d, 1H), 7.65 (s, 1H), 7.68 (d, 1H).

### e. 4-Ethyl-3-(3-methoxy-propoxy)-benzoic acid

To a solution of 4-ethynyl-3-(3-methoxy-propoxy)-benzoic acid (1 g, 4.11 mmol) in EtOH (20 mL), Pd(OH)₂ (0.1 g) is added. The resulting mixture is stirred under an hydrogen atmosphere for 15 min, then filtered and concentrated. The crude material is purified by flash chromatography on silica gel (eluent hexane/AcOEt 2/1) to give, the title compound as white powder. ¹H NMR (CDCl₃, 300 MHz): δ = 1.2 (t, 3H), 2.15 (p, 2H), 2.7 (q, 2H), 3.38 (s, 3H), 3.62 (t, 2H), 4.15 (t, 2H), 7.25 (d, 1H), 7.55 (s, 1H), 7.68 (d, 1H).

### Example 61: N-((3R*,4R*)-4-Benzyl-pyrrolidin-3-ylmethyl)-N-isopropyl-3-(3-methoxy-propoxy)-benzamide

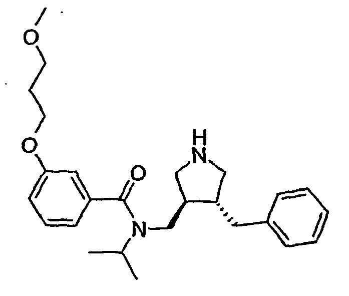

The title compound is prepared analogously as described for the title compound under C in Example 56 using Scheme11a. MS (LC-MS): 425 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.64 min.

### 3-(3-Methoxy-propoxy)-henzoic acid

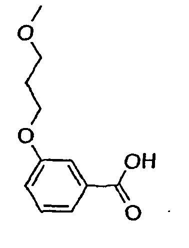

### a. 3-(3-Methoxy-propoxy)-benzoic acid ethyl ester

A solution of 3-hydroxy-benzoic acid ethyl ester (2.04 g, 13.4 mmol) and NaH (80 % in oil, 0.386 g, 16.08 mmol) in THF (50 mL) is stirred under nitrogen for 20 min before the addition of 1-bromo-3-methoxy propane (3.07 g, 20.1 mmol) follows. The resulting mixture is further stirred for 24 h at reflux, then poured into an ice/water mixture and extracted twice with CH₂Cl₂. The combined organic extracts are washed with brine, dried over anhydrous sodium sulfate and concentrated. The crude material is purified by flash chromatography on silica gel (eluent hexane/AcOEt 9/1) to afford the title compound as a yellow oil. ¹H NMR (CDCl₃, 300 MHz): δ = 2.15 (p, 2H), 3.35 (s, 3H), 3.55 (t, 2H), 3.9 (s, 3H), 4.12 (t, 2H), 7.15 (dd, 1H), 7.32 (t, 1H), 7.55 (bs, 1H), 7.65 (d, 1H).

### b. 3-(3-Methoxy-propoxy)-benzoic acid

A solution of 3-(3-methoxy-propoxy)-benzoic acid ethyl ester (2 g) and NaOH (1 N in water, 13.4 mL, 13.4 mmol) in EtOH (10 mL) and water (5 mL) is refluxed for 2 h. The reaction mixture is allowed to reach RT, and the solvent is concentrated under reduced pressure. The residue is dissolved in water (200 mL) and washed with ether (50 mL x 3). The pH is adjusted to 2 by addition of cc HCl and the aqueous layer extracted with AcOEt (50 mL x 2). The combined organic extracts are dried over Na₂SO₄, filtered and concentrated to afford the desired title product. TLC, Rf (hexane/AcOEt 3/1) = 0.17.

### Example 62: 1-(3-Methoxy-propyl)-1H-indole-2-carboxylic acid ((3R*,4R*)-4-benzyl-pyrrolidin-3-ylmethyl)-isopropyl-amide

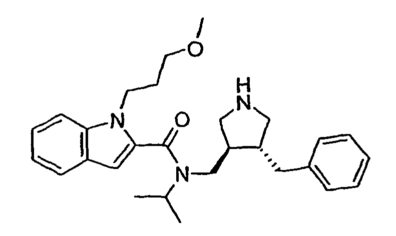

The title compound is prepared analogously as described for the title compound under C in Example 56 (Scheme11a). MS (LC-MS): 448 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.79 min.

### 1-(3-Methoxy-propyl)-1H-indole-3-carboxylic acid

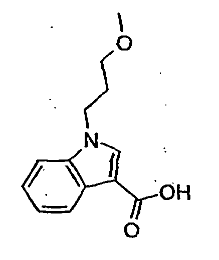

### a. 1H-Indole-3-carboxylic acid ethyl ester

To a solution of 1H-indole-3-carboxylic acid (3.23 g, 0.02 mmol) in MeOH (85 mL) and water (8.5 mL), cesium carbonate (20% in water, 23 mL) is added. The resulting mixture is stirred at 45°C, then filtered and dried under vacuum. The resulting carboxylate is dissolved in DMF (30 mL), ethyl iodide (1.75 mL, 0.022 mmol) is added, and the resulting mixture is stirred at RT for 4 h. The mixture is concentrated under reduced pressure and the crude product is purified by flash chromatography on silica gel (eluent: hexane/AcOEt 3/1) to give the desired title compound. TLC, Rf (hexane/AcOEt 5/1) = 0.2. ¹H NMR (CDCl₃, 300 MHz): δ = 1.45 (t, 3H), 4.45 (q, 2H), 7.3 (m, 2H), 7.45 (m, 1H), 8.15 (d, 1H), 8.4 (m, 1H), 8.6 (m, NH).

### b. 1-(3-Methoxy-propyl)-1H-indole-3-carboxylic acid ethyl ester

To a solution of 1H-Indole-3-carboxylic acid ethyl ester (2.21 g, 13.71 mmol) in DMF (50 mL), sodium hydride (80% in mineral oil, 0.598 g, 20 mmol) is added, and the solution is stirred for 20 min. 1-lodo-3-methoxy propane (3 g, 19.6 mmol) is added and the mixture is stirred for 3 h. The reaction mixture is then poured into an ice/water solution and extracted with CH₂Cl₂ (20 mL x 3). The combined organic extracts are washed with brine, dried over anhydrous sodium sulfate and concentrated under reduced pressure to afford the title compound which is used without further purification in the next step. TLC, Rf (CH₂Cl₂/MeOH 97/3 ) = 0.7. ¹H NMR (CDCl₃, 300 MHz): δ = 1.42 (t, 3H), 2.09 (p, 2H), 3.29 (t, 2H), 3.32 (s, 3H), 4.28 (t, 2H), 4.39 (q, 2H), 7.23 (m, 2H), 7.4 (m, 1H), 7.81 (s, 1H), 8.18 (m, 1H).

### c. 1-(3-Methoxy-propyl)-1H-indole-3-carboxylic acid

A solution of 1-(3-methoxy-propyl)-1H-indole-3-carboxylic acid ethyl ester (3.18 g, 12.17 mmol) and NaOH (1 N in water, 12.2 mL, 12.24 mmol) in EtOH (24 mL) and water (12 mL) is refluxed for 3 h. The reaction mixture is allowed to reach RT, and the solvent is concentrated under reduced pressure. The residue is dissolved in CH₂Cl₂ (250 mL) and the pH is adjusted to 2 by addition of 1 N KHSO₄. The layers are separated, and the aqueous phase is back-extracted with CH₂Cl₂ (50 mL x 2). The combined organic extracts are dried over Na₂SO₄, filtered and concentrated. The crude material is purified by flash chromatography on silica gel (eluent: hexane/EtOAc/AcOH 50/50/1) to afford the desired title product TLC, Rf (hexane/AcOEt/AcOH 50/50/1) = 0.5. ¹H NMR (CDCl₃, 300 MHz): δ = 2.12 (p, 2H)_{;} 3.3 (t, 2H), 3.32 (s, 3H), 4.31 (t, 2H), 7.3 (m, 2H), 7.42 (m, 1H), 7.9 (s, 1H), 8.22 (m, 1H).

### Example 63: N-((3R*,4R*)-4-Benzyl-pyrrolidin-3-ylmethyl)-N-isopropyl-4-methoxy-3-(4

### methoxy-butyl)-benzamide

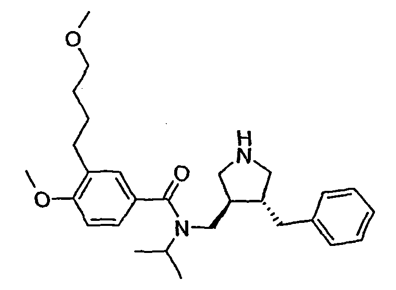

The title compound is prepared analogously as described for the title compound under C in Example 56 using Scheme11a. MS (LC-MS): 453.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.96 min.

### 4-Methoxy-3-(4-methoxy-butyl)-benzoic acid

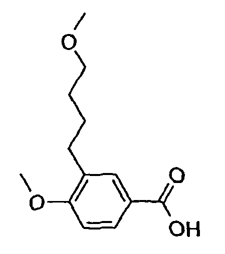

### a. (3-Methoxy-propyl)-triphenyl-phosphonium bromide

A solution of PPh₃ (42.8 g, 163.2 mmol) and 1-bromo-3-methoxypropane (25 g, 163.3 mmol) in toluene (70 mL) is heated at 150°C in an autoclave for 44 h. After completion of the reaction, the mixture is filtered and the precipitate washed with toluene and dried under high vacuum for 4 h affording the title compound as a white powder (64.8 g). Rt (HPLC, Nucleosil C18, 10:90-100:0 CH₃CN/H₂O + 0.1% TFA within 5 min, then 100% CH₃CN + 0.1 % TFA): 5.06 min.

### b. 4-Bromo-1-methoxy-2-(-4-methoxy-but-1-enyl)-benzene

To a stirred solution of NaHMDS (26.69 g, 153.81 mmol) in THF (175 mL) under nitrogen atmosphere is added dropwise at 0°C a THF solution (175 mL) of (3-methoxy-propyl)-triphenyl-phosphonium bromide (63.88 g, 153.8 mmol). The resulting mixture is stirred for 1 h at 0°C before the addition of a THF solution (310 mL) of 5-bromo-2-methoxy-benzaldehyde (27.56 g, 128.18 mmol). The reaction mixture is further stirred for 1 h at room temperature and poured into a saturated NH₄Cl aqueous solution, the aqueous layer is extracted twice with EtOAc. The combined organic extracts are dried over anhydrous magnesium sulfate and concentrated under reduced pressure. The residue is taken up into ether and the triphenylphosphine oxide precipitate is filtered off through a pad of celite. The filtrate is concentrate and the residual material purified by flash column chromatography on silica gel (hexane/EtOAc 4/1) to afford the title compound (as a mixture Z and E stereoisomers) as a yellow oil. MS (LC-MS): 288.0, 289.8 [M+18]⁺; t_{R} (HPLC, Nucleosil C18, 10:90-100:0 CH₃CN/H₂O + 0.1% TFA within 5 min, then 100% CH₃CN + 0.1% TFA): 6.18 min.

### c. 4-Bromo-1-methoxy 2-(4-methoxy-butyl)-benzene

A suspension of 4-bromo-1-methoxy-2-(-4-methoxy-but-1-enyl)-benzene (30.7 g, 113.21 mmol) and Pd/c 5% (3.1 g) in THF (620 mL) is shaked under an hydrogen atmosphere. After completion of the reaction, the mixture is filtered through a pad of celite, the solvent is evaporated under reduced pressure and the residue purified by flash chromatography on silica gel (hexane/EtOAc 6/1) to give the title compound as a colorless oil. MS 260.1, 261.9 [M+18]. MS (LC-MS): 289.8, 291.0 [M+18]⁺; t_{R} (HPLC, Nucleosil C18, 10:90-100:0 CH₃CN/H₂O + 0.1% TFA within 5 min, then 100% CH₃CN + 0.1% TFA): 6.49 min.

### d. 4-Methoxy-3-(4-methoxy-butyl)-benzaldehyde

To a stirred solution of 4-bromo-1-methoxy-2-(4-methoxy-butyl)-benzene (26.2 g, 95.91 mmol) in 525 mL of THF is added dropwise n-butyl lithium (105.5 mmol, 1.6 M solution in hexane) over 30 min at -78°C. The reaction mixture is further stirred 5 min at -78°C before the addition of a THF solution (85 mL) of DMF (16.27 mL, 211 mmol) over 30 min. The reaction mixture is further stirred for 15 min at -78°C, 1 h at room and poured into aqueous 1 M HCl solution. The aqueous layer is extracted twice with ether (200 mL x 3), and the combined organic extracts are dried over anhydrous sodium sulfate and concentrated under reduced pressure. The residue is purified by flash column chromatography on silica gel (hexane/EtOAc 4/1) to afford the title compound as a yellow oil. MS (LC/MS) : 222.9 [M+H]⁺. Rt (HPLC, Nucleosil C18, 10:90-100:0 CH₃CN/H₂O + 0.1% TFA within 5 min, then 100% CH₃CN + 0.1% TFA): 5.08 min.

### e. 4-Methoxy-3-(4-methoxy-butyl)-benzoic acid

To a stirring suspension of 4-methoxy-3-(4-methoxy-butyl)-benzaldehyde (300 mg, 1.35 mmol) and sulfamic acid (175.7 mg, 1.81 mmol) in 80% CH₃CO₂H (2.3 mL), over 5 min a solution of 80% NaClO₂ (126.6 mg, 1.4 mmol in 170 µL of H₂O) in water (0.65 mL) is added while maintaining the temperature at 18-20°C by external cooling using an ice water bath. The yellow slurry is stirred at 20°C for an additional 2 h. NaClO₂ (126.6 mg,1.4 mmol in 170 µL of H₂O) in 0.65 mL of water and sulfamic acid in 80% CH₃COOH (130 mg, 1.34 mmol) are added to complete the reaction and the mixture is further stirred overnight. The reaction mixture is diluted with 2.3 mL of water and stirred over 30 min. The precipitate is filtered and dried to afford the title product. MS (LC-MS): 239.0 [M+H]⁺; t_{R} (HPLC, Interchrom OBD-25QS, 10-100% CH₃CN/H₂O/5 min, 100% CH₃CN/3 min, CH₃CN and H₂O containing 0.1% TFA, flow: 1.5 mL/min): 4.20 min.

### Example 64: N-((3R*,4R*)-4-Benzyl-pyrrolidin-3-ylmethyl)-N-isopropyl-4-methoxy-3-(3-methoxy-pronylamino)-benzamide

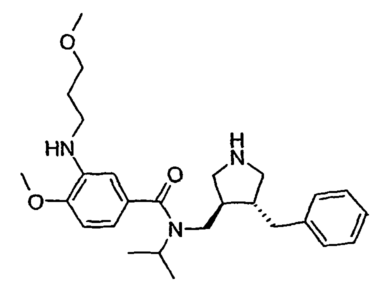

The title compound is prepared analogously as described for the title compound under C in Example 56 using Scheme11a. MS (LC-MS): 454.1. [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.18 min.

### 4-Methoxy-3-(3-methoxy-propylamino)-benzoic acid

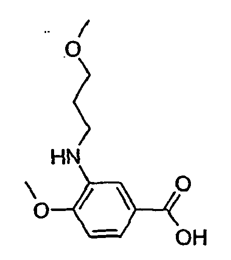

### a. 4-Methoxy-3-(3-methoxy-propylamino)-benzoic acid methyl ester

To a solution of 3-amino-4-methoxy-benzoic acid methyl ester (3 g, 16.5 mmol) in 70 mL of DMF is added Cs₂CO₃ (6.47 g, 19.8 mmol) and 1-bromo-3-methoxypropane (2.78 g, 18.2 mmol). The mixture is stirred at 80°C overnight. 1-Bromo-3-methoxypropane (1.26 g, 8.25 mmol), Cs₂CO₃ (2.7 g, 8.25 mmol), and Nal (8.25 mmol, 1.23 g) are added and the mixture further stirred at 80°C for 3 days. The mixture is then poured into water and extracted with twice with EtOAc. The combined organic extracts are dried (Na₂SO₄), filtered and concentrated. The crude product is purified by flash column chromatography on silica gel (c-hexane/EtOAc 4/1) to afford the title compound. MS (LC-MS): 254 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 3.96 min.

### b. 4-Methoxy-3-(3-methoxy-propylamino)-benzoic acid

To a stirring solution of 4-methoxy-3-(3-methoxy-propylamino)-benzoic acid methyl ester (2.21 g, 9.23 mmol) in MeOH (50 mL) cooled to 0°C, LiOH.H₂O (1.162 g, 27.7 mmol) is added. The reaction mixture is allowed to reach RT and stirred for 2 h. LiOHH₂O (3.48 g, 83.1 mmol) is added and the mixture further stirred for 24 h until completion. The reaction mixture is neutralized by the addition of HCl 1 N and extracted with CH₂Cl₂. The combined organics extracts are dried (Na₂SO₄), and the solvent is removed in *vacuo* to give the title product. MS (LC-MS): 454.1. TLC, Rf (CH₂Cl₂/MeOH 95/5) = 0.4.

### Example 65: 1-(3-Methoxy-propyl)-3-methyl-1H-indole-6-carboxylic acid ((3R,4R)-4-benzyl-pyrrolidin-3-ylmethyl)-isopropyl-amide

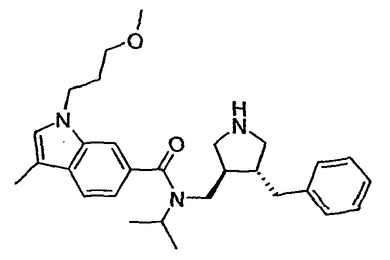

The title compound is prepared analogously as described for the title compound under C in Example 56 using Scheme11a. MS (LC-MS): 462.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 6.95 min.

### 1-(3-Methoxy-propyl)-3-methyl-1H-indole-6-carboxylic acid

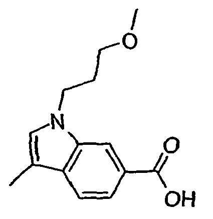

### 3-Methyl-1H-indole-6-carboxylic acid methyl ester

A mixture of 3-formyl-1H-indole-6-carboxylic acid methyl ester (5 g, 24.6 mmol), p-toluenesulfonic acid (704 mg, 3.7 mmol) and p-toluenesulfonylhydrazide (5.49 g, 29.5 mmol) in a mixture of dimethylformamide (50 mL) and sulfolane (25 mL) is heated at 100°C for 15 min. Then cooled to RT, before the addition of sodium cyanoborohydride (6.2 g, 98.4 mmol, 2 g portions after 10 min intervals). The resulting mixture is heated at 100°C for 2 h, cooled to RT and poured into a mixture of ice and water (250 mL) leading to a white precipitate. Water (500 mL) is added, and the mixture is stirred for 30 min before filtration. The off-white solid is washed with warm water. Toluene is added and removed by rotary evaporation to afford the title compound as a yellow solid. TLC, Rf (Hexane/EtOAc 4/1)= 0.3. MS (LC-MS): [M+H]+= 188.1. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.13 min.

### 1-(3-Methoxy-propyl)-3-methyl-1H-indole-6-carboxylic acid methyl ester

To a solution of 3-methyl-1H-indole-6-carboxylic acid methyl ester (2.5 g, 13.2 mmol) in DMF (25 mL), a solution of NaH (580 mg, 14.5 mmol, 60% dispension in grease) in DMF (25 mL) is slowly added under a N₂ atmosphere. The mixture is stirred at 80°C for 20 min, and cooled to RT before the addition of 1-bromo-3-methoxypropane (4.04 g, 26.4 mmol). The resulting mixture is stirred for 24 h. 1-bromo-3-methoxypropane (2.02 g, 13.2 mmol) and NaH (580 mg, 14.5 mmol) are added and the mixture further stirred for 24 h to complete the reaction. The solvent is concentrated under reduced pressure and the mixture diluted with AcOEt. An aqueous saturated solution of NaHCO₃ is added, the layers are separated, and the aqueous one is back-extracted with AcOEt. The combined organic extracts are dried over Na₂SO₄, filtered and concentrated. The crude residue is purified by flash chromatography on silica gel (eluent: c-hexane/EtOAc 80/20) to give the title compound. MS (LC-MS): 262.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mUmin): 5.80 min.

### 1-(3-Methoxy-propyl)-3-methyl-1H-indole-6-carboxylic acid

To a solution of 1-(3-methoxy-propyl)-3-methyl-1H-indofe-6-carboxylic acid methyl ester (1.56 g, 6.3 mmol) in MeOH (20 mL) and H₂O (1 mL) is added NaOH (756 mg, 18.9 mmol) and the mixture is stirred at 50°C overnight. Then neutralized by the addition of water and HCl 1.0 M (3 eq, 18.9 mmol). CH₂Cl₂ is added, the layers are separated, and the aqueous one is extracted twice with CH₂Cl₂. The combined organic layers are dried over Na₂SO₄, filtered and concentrated under reduced pressure. The crude material is obtained in a pure form and is used in the next step without purification. MS (LC-MS): 248.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.93 min.

### Example 66: 3-Benzyloxy-N-((3S*AS*)-4-benzyl-pyrrolidin-3-ylmethyl)-N-isopropyl-4-methoxy-benzamide

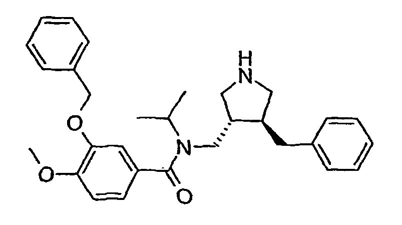

The title compound is prepared analogously as described for the title compound under C in Example 56 using Scheme11a. MS (LC-MS): 473.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.02 min.

### Example 67: 3-(3-Methoxy-propyl)-3H-benzoimidazole-5-carboxylic acid ((3S,4S)-4-benzyl-pyrrolidin-3-ylmethyl)-isopropyl-amide

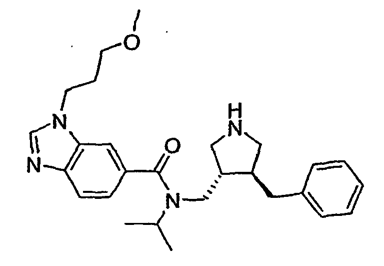

The title compound is prepared analogously as described for the title compound under C in Example 56 (Scheme11a) starting from (3R*,4S*)-3-benzyl-4-(isopropylamino-methyl)-pyrrolidine-1-carboxylic acid tert-butyl ester and 3-(3-methoxy-propyl)-3H-benzoimidazole-5-carboxylic acid. MS (LC-MS): 449.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.25 min.

### 3-(3-Methoxy-propyl)-3H-benzoimidazole-5-carboxylic acid

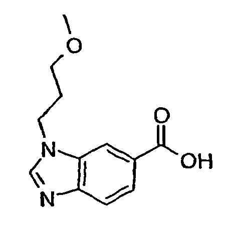

### a. 3-(3-Methoxy-propyl)-3H-benzoimidazole-5-carboxylic acid methyl ester and 1-(3-methoxy-propyl)-1H-benzoimidazole-5-carboxylic acid methyl ester

To a solution of 3H-benzoimidazole-5-carboxylic acid methyl ester (1.5 g, 8.5 mmol) in DMF (25 mL) is slowly added a solution of NaH (375 mg, 9.35 mmol, 60% dispension in grease) in DMF (25 mL) under a nitrogen atmosphere. The mixture is stirred at 80°C for 20 min, and cooled to RT before the addition of 1-bromo-3-methoxypropane (2.6 g, 17 mmol). The resulting mixture is stirred for 24 h. 1-bromo-3-methoxypropane (1.3 g, 8.5 mmol) and NaH (375 mg, 9.35 mmol) are added and the mixture was further stirred for 24 h to complete the reaction. The solvent is concentrated under reduced pressure and the mixture diluted with AcOEt. An aqueous saturated solution of NaHCO₃ is added, the layers are separated and the aqueous one back-extracted twice with AcOEt. The combined organic extracts are dried over Na₂SO₄, filtered and concentrated, to give a mixture of the two regioisomers together with a small amount of the two corresponding acids. The crude material is used without further purification in the next step.

### b. 3-(3-Methoxy-propyl)-3H-benzoimidazole-5-carboxylic acid and 1-(3-methoxypropyl)-1H-benzoimidazole-5-carboxylic acid

To a solution of the crude material described above (894 mg) in MeOH (50 mL) and H₂O (1 mL) is added NaOH (432 mg, 10.8 mmol) and the mixture is stirred at 50°C over-night. After completion of the reaction, acidification is done by the addition of water and HCl 1.0 M (21.6 mmol), CH₂Cl₂ is added, the organic layer is separated and the aqueous one extracted twice with CH₂Cl₂. The combined organic layers are dried over Na₂SO₄, filtered and concentrated under reduced pressure. The crude material is purified by preparative HPLC (C18 ODB 10 µm 28*250 mm; interchrom, eluent 5-->50% ACN in 45 min, 40 mL/min, loading : 100 mg of product in 4 mL of MeOH), to give the two regioisomeric acids : 3-(3-methoxy-propyl)-3H-benzoimidazole-5-carboxylic acid : MS (LC-MS): 235.0 [M+H]⁺; ¹H NMR (CD₃OD, 300 MHz): δ = 2.14 (q, 2H), 3.30 (s, 3H), 3.34 (t, 2H), 4.46 (t, 2H), 7.72 (d, 1H), 7.99 (d, 1H), 8.32 (s, 1H), 8.41 (s, 1H) and 1-(3-methoxy-propyl)-1H-benzoimidazole-5-carboxylic acid : MS (LC-MS): 235.0 [M+H]⁺; ¹H NMR (CD₃OD, 300 MHz): δ = 2.17 (q, 2H), 3.30 (s, 3H), 3.37 (t, 2H), 4.50 (t, 2H), 7.77 (d, 1H), 8.11 (d, 1H), 8.41 (s, 1H), 8.69 (s, 1H).

### Example 68: N-Isopropyl-4-methoxy-3-(3-methoxy-propoxy)-N-pyrrolidin-3-ylmethyl-benzamide

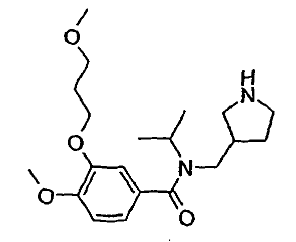

The title compound is prepared analogously as described for the title compound under C in Example 56 using Scheme11a starting from 1-boc-3-pyrrolidine carboxaldehyde. MS (LC-MS): 365.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 3.86 min.

### Example 69: Naphthalene-2-carboxylic acid ((3R*,4R*)-4-benzyl-pyrrolidin-3-ylmethyl)-isopropyl-amide

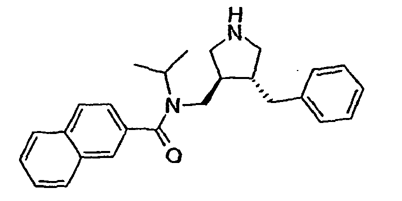

The title compound is prepared analogously as described for the title compound under C in Example 57 using Scheme11b. MS (LC-MS): 387.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.93 min.

### Example 70: N-((3S*4S*)-4-Benzyl-prolidin-3-ylmethyl)-N-[2-(3,5-dimethoxy-benzyloxy)-ethyl]-4-methoxy-3-(3-methoxy-propoxy)-benzamide

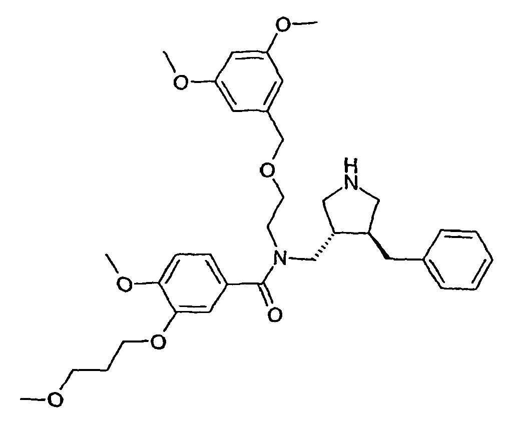

The title compound is prepared analogously as described for the title compound under C in Example 56 (Scheme11a) from (3R*,4R*)-3-benzyl-4-formyl-pyrrolidine-1-carboxylic acid tert-butyl ester and 2-(3,5-dimethoxybenzyloxy)-ethylamine. MS (LC-MS): 607.1 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mUmin): 4.87 min.

### Example 71: N-((3S*,4S*)-4-Benzyl-pyrrolidin-3-ylmethyl)-4-methoxy--3-(3-methoxy-propoxy)-N-(3-phenoxy-propyl)-benzamide

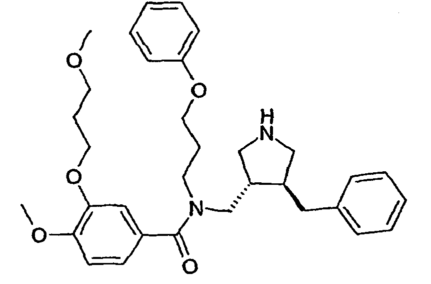

The title compound is prepared analogously as described for the title compound under C in Example 56 (Scheme11a) from (3R*,4R*)-3-benzyl4-formyl-pyrrolidine-1-carboxylic acid tert-butyl ester and 3-phenoxy-propylamine. MS (LC-MS): 547.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 4.86 min.

### Example 72: 4-Methoxy-3-(3-methoxy-propoxy)-N-[2-(3-phenoxy-phenyl)-ethyl]-N-pyrrolidin-3-ymethyl-benzamide

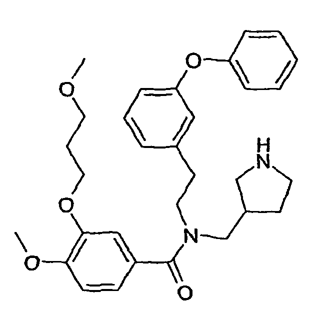

The title compound is prepared analogously as described for the title compound in Example 57 (Scheme11b) from 3-formyl-pyrrolidine-1-carboxylic acid tert-butyl ester and 2-(3-phenoxy-phenyl)-ethylamine. MS (LC-MS): 292.1 [M+2H]²⁺, 519.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.46 min.

### Example 73: 4-Methoxy-3-(3-methoxy-propoxy)-N-[2-(2-phenoxy-phenyl)-ethyl]-N-pyrrolidin-3-ylmethyl-benzamide

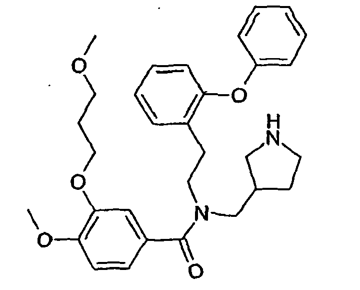

The title compound is prepared analogously as described for the title compound in Example 57 (Scheme11b) from 3-formyl-pyrrolidine-1-carboxylic acid tert-butyl ester and 2-(2-phenoxy-phenyl)-ethylamine. MS (LC-MS): 292.1 [M+2H]²⁺, 519.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.43 min.

### Example 74: N-((3R*,4R*)-4-Benzyl-pyrrolidin-3-ylmethyl)-N-cyclopentyl-4-methoxy-3-(3-methoxy-propoxy)-benzamide, hydrochloride

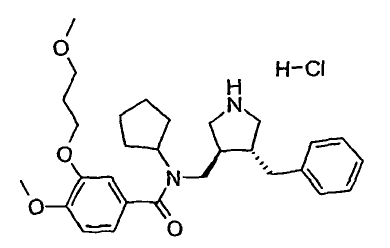

The title compound is prepared analogously as described for the title compound under C in Example 57 using Scheme11b from (3R*,4R*)-3-benzyl-4-formyl-pyrrolidine-1-carboxylic acid tert-butyl ester and cyclopentyl amine. MS: 481.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 5.39 min

### Example 75: N-((3R*,4R*)-4-Benzyl-pyrrolidin-3-ylmethyl)-N-cycloproyl-4-methooy-3-(3-methoxy-propoxy)-benzamide, hydrochloride

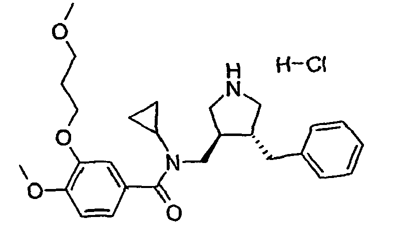

The title compound is prepared analogously as described for the title compound under C in Example 57 using Scheme11b from (3R*,4R*)-3-benzyl-4-formyl-pyrrolidine-1-carboxylic acid tert-butyl ester and cyclopropyl amine. MS: 453.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 4.86 min

### Example 76: N-((3R*,4R*)-4-Benzyl-pyrrolidin-3-ylmethyl)-4-methoxy-3-(3-methoxy-propoxy)-N-thiophen-2-ylmethyl-benzamide, hydrochloride

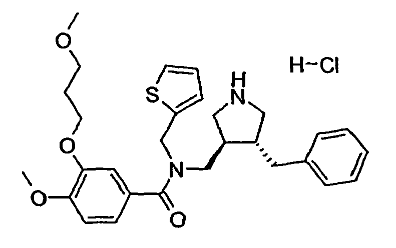

The title compound is prepared analogously as described for the title compound under C in Example 57 using Scheme11b from (3R*,4R*)-3-benzyl-4-formyl-pyrrolidine-1-carboxylic acid tert-butyl ester and thiophene-2-methylamine. MS: 509.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 5.23 min

### Example 77: N-((3R*,4R*)-4-Benzyl-pyrrolidin-3-ylmethyl)-4-methoxy-N-(2-methoxy-ethyl)-3-(3-methoxy-propoxy)-benzamide, hydrochloride

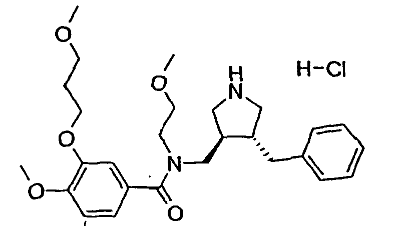

The title compound is prepared analogously as described for the title compound under C in Example 57 (Scheme11b) from (3R*,4R*)-3-benzyl-4-formyl-pyrrolidine-1-carboxylic acid tert-butyl ester and 2-methoxy-ethylamine. MS: 471.6 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 4.65 min

### Example 78: N-((3R*,4R*)-4-Benzyl-pyrrolidin-3-ylmethyl)-N-(4-chloro-benzyl)-4-methoxy-3-(3-methoxy-propoxy)-benzamide, hydrochloride

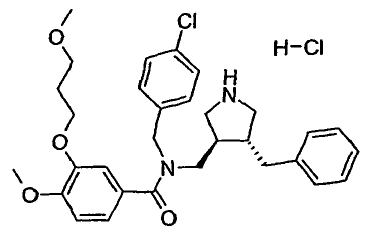

The title compound is prepared analogously as described for the title compound under C in Example 57 using Scheme11b from (3R*,4R*)-3-benzyl-4-formyl-pyrrolidine-1-carboxylic acid tert-butyl ester and 4-chloro benzylamine. MS: 537.5 [M+H]⁺; t_{R} (HPLC, C18 column, 5-100% CH₃CN+0.1%TFA/H₂O+0.1%TFA/8 min, 100% CH₃CN+0.1%TFA/₂ min, flow 1.5 mL/min): 5.71 min

### Example 79: N-((3R*,4R*)-4-Benzyl-pyrrolidin-3-ylmethyl)-4-methoxy-3-(3-methoxy-propoxy)-N-methyl-benzamide, hydrochloride

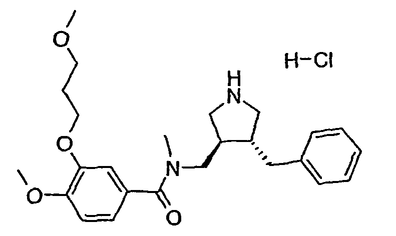

The title compound is prepared analogously as described for the title compound under C in Example 57 using Scheme11b from (3R*,4R*)-3-benzyl-4-formyl-pyrrolidine-1-carboxylic acid tert-butyl ester and methylamine. MS: 427.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 4.53 min

### Example 80: N-((3R* 4R*)-4-Benzyl-pyrrolidin-3-ylmethyl)-N-cyclobutyl-4-methoxy-3-(3-methoxy-propoxy)-benzamide, hydrochloride

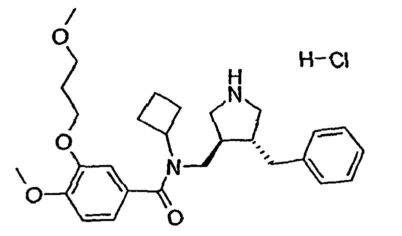

The title compound is prepared analogously as described for the title compound under C in Example 57 using Scheme11b from (3R*,4R*)-3-benzyl-4-formyl-pyrrolidine-1-carboxylic acid tert-butyl ester and cyclobutylamine. MS: 467.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 5.10 min

### Example 81: N-((3R*,4R*)-4-Benzyl-pyrrolidin-3-ylmethyl)-N-(1-ethyl-propyl)-4-methoxy-3-(3-methoxy-propoxy)-benzamide, hydrochloride

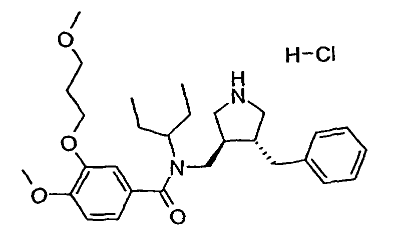

The title compound is prepared analogously as described for the title compound under C in Example 57 using Scheme11b from (3R*,4R*)-3-benzyl-4-formyl-pyrrolidine-1-carboxylic acid tert-butyl ester and 3-pentylamine. MS: 483.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 5.48 min

### Example 82: N-((3R*,4R*)-4-Benzyl-pyrrolidin-3-ylmethyl)-N-cyclopropylmethyl-4-methoxy-3-(3-methoxy-propoxy)-benzamide, hydrochloride

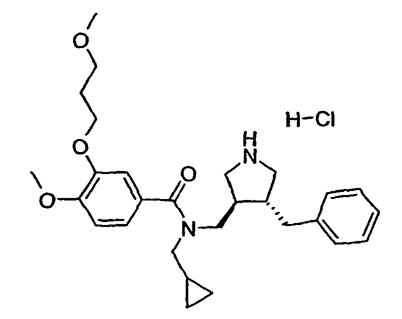

The title compound is prepared analogously as described for the title compound under C in Example 57 using Scheme11b from (3R*,4R*)-3-benzyl-4-formyl-pyrrolidine-1-carboxylic acid tert-butyl ester and aminomethylcyclopropane. MS: 467.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 5.18 min

### Example 83: N-((3R*,4R*)-4-Benzyl-pyrrolidin-3-ylmethyl)-4-methoxy-3-(3-methoxy-propoxy)-N-(2,2,2-trifluoro-ethyl)-benzamide, hydrochloride

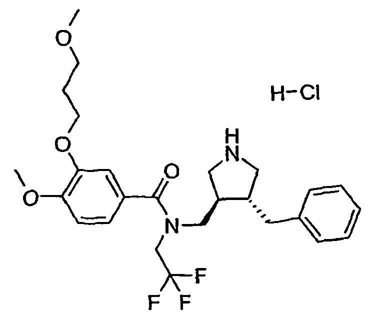

The title compound is prepared analogously as described for the title compound under C in Example 57 using Scheme11b from (3R*,4R*)-3-benzyl-4-formyl-pyrrolidine-1-carboxylic acid tert-butyl ester and 2,2,2-trifluoroethylamine. MS: 495.4 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mUmin): 5.13 min.

### Example 84: N-((3R*,4R*)-4-Benzyl-pyrrolidin-3-ylmethyl)-N-(4-cyano-benzyl)-4-methoxy-3-(3-methoxy-propoxy)-benzamide, hydrochloride

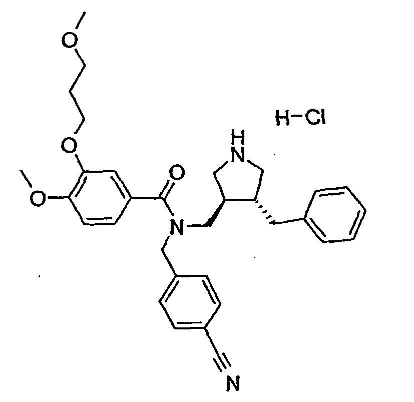

The title compound is prepared analogously as described for the title compound under C in Example 57, using Scheme11b from (3R*,4R*)-3-benzyl-4-formyl-pyrrolidine-1-carboxylic acid tert-butyl ester and 4-cyanobenzylamine. MS: 528.6 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 5.06 min.

### Example 85: N-((3R*,4R*)-4-Benzyl-pyrrolidin-3-ylmethyl)-N-(1,2-dimethyl-propyl)-4-methoxy-3-(3-methoxy-propoxy)-benzamide, hydrochloride

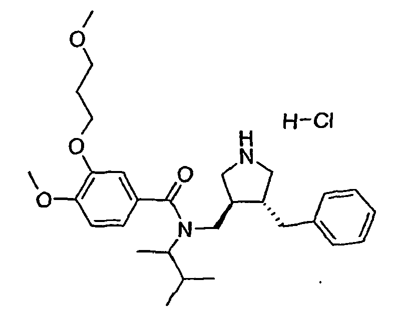

The title compound is prepared analogously as described for the title compound under C in Example 57 using Scheme11b from (3R*,4R*)-3-benzyl-4-formyl-pyrrolidine-1-carboxylic acid tert-butyl ester and (±)-2-amino-3-methylbutane. MS: 483.5 [M+H]⁺; t_{R} (HPLC, C18 column, 5-100% CH₃CN+0.1%TFA/H₂O+0.1%TFA/8 min, 100% CH₃CN+0.1%TFA/2 min, flow 1.5 mL/min): 5.35 and 5.45 min (diastereomers).

### Example 86: N-((3R*,4R*)-4-Benzyl-pyrrolidin-3-ylmethyl)-N-(4-chloro-benzyl)-2-phenoxy-benzamide

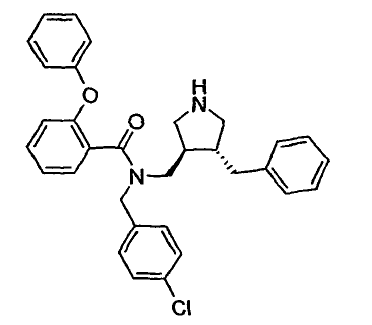

The title compound is prepared analogously as described for the title compound under C in Example 57 using Scheme11a from (3R*,4R*)-3-benzyl-4-formyl-pyrrolidine-1-carboxylic acid tert-butyl ester and 4-chloro benzylamine. MS (LC-MS): 511.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.37 min.

### Example 87: N-((3R*,4R*)-4-Benzyl-pyrrolidin-3-ylmethyl)-N-(4-chloro-benzyl)-3-phenoxy-benzamide

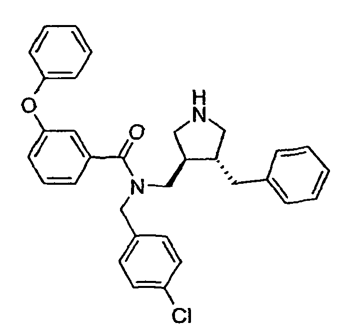

The title compound is prepared analogously as described for the title compound under C in Example 56, Scheme11a from (3R*,4R*)-3-benzyl-4-formyl-pyrrolidine-1-carboxylic acid tert-butyl ester and 4-chloro benzylamine. MS (LC-MS): 511.2 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.45 min.

### Example 88: N-((3R*,4R*)-4-Benzyl-pyrrolidin-3-ylmethyl)-N-(4-chloro-benzyl)-4-phenoxy-benzamide

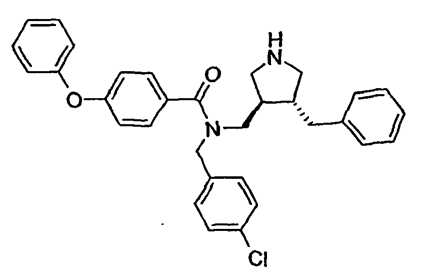

The title compound is prepared analogously as described for the title compound under C in Example 56 using Scheme11a from (3R*,4R*)-3-benzyl-4-formyl-pyrrolidine-1-carboxylic acid tert-butyl ester and 4-chloro benzylamine. MS (LC-MS): 510.9 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.51 min.

### Example 89: Benzofuran-5-carboxylic acid ((3R*,AR*)-4-benzyl-pyrrolidin-3-ylmethyl)-(4-chloro-benzyl)-amide

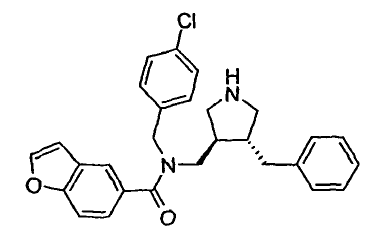

The title compound is prepared analogously as described for the title compound under C in Example 57 using Scheme11b from (3R*,4R*)-3-benzyl-4-formyl-pyrrolidine-1-carboxylic acid tert-butyl ester and 4-chloro benzylamine. MS (LC-MS): 458.9 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.03 min.

### Example 90: N-((3R*,4R*)-4-Benzyl-pyrrolidin-3-ylmethyl)-4-methoxy-3-(3-methoxy-propoxy)-N-(3-phenyl-propyl)-benzamide

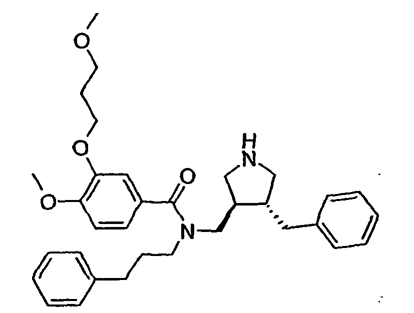

The title compound is prepared analogously as described for the title compound under C in Example 56 (Scheme11a) from (3R*,4R*)-3-benzyl-4-formyl-pyrrolidine-1-carboxylic acid tert-butyl ester and 3-phenyl-propylamine. MS (LC-MS): 531.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 4.93 min.

### Example 91: N-((3R*,4R*)-4-Benzyl-pyrrolidin-3-ylmethyl)-4-methoxy-3-(3-methoxy-propoxy)-N-phenethyl-benzamide

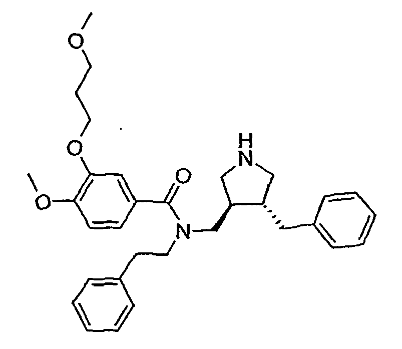

The title compound is prepared analogously as described for the title compound under C in Example 56 (Scheme11a) from (3R*,4R*)-3-benzyl-4-formyl-pyrrolidine-1-carboxylic acid tert-butyl ester and phenethylamine. MS (LC-MS): 517.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.79 min.

### Example 92: N-((3R*,4R*)-4-Benzyl-pyrrolidin-3-ylmethyl)-4-methoxy-3-(3-methoxy-propoxy)-N-(4-phenyl-butyl)-benzamide

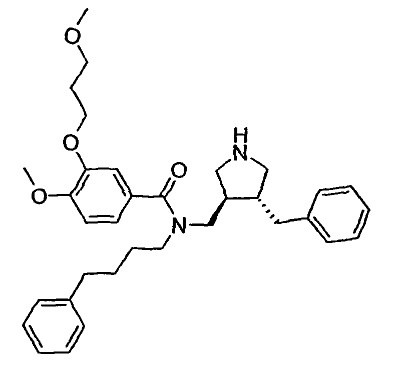

The title compound is prepared analogously as described for the title compound under C in Example 56 (Scheme11a) from (3R*,4R*)-3-benzyl-4-formyl-pyrrolidine-1-carboxylic acid tert-butyl ester and 4-phenyl-butylamine. MS (LC-MS): 545.1 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.13 min.

### Example 93: Naphthalene-2-carboxylic acid (4-benzyloxy-phenyl)-((3S*.4S*)-4-benzyl-pyrrolidin-3-ylmethyl)-amide (see also Scheme10 above)

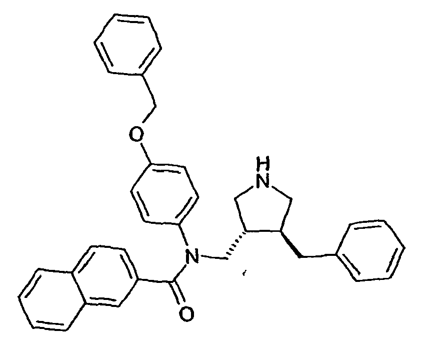

The title compound is prepared analogously as described for the title compound under C in Example 40 (Scheme10). MS (LC-MS): 527.0 [M+H]⁺, t_{R} (HPLC, C18 column, 10-100% CH₃CN/H₂O/5 min, 100% CH₃CN/3 min, 100-10% CH₃CN/H₂O/3 min, CH3CN and H2O containing 0.1% TFA, flow: 1.5 mL/min): 5.47 min.

### Example 94: (2-{5-[((3R*,4R*)-4-Benzyl-pyrrolidin-3-ylmethyl)-isopropyl-carbamoyl]-2-methoxy-phenyl}-ethyl)-carbamic acid methyl ester

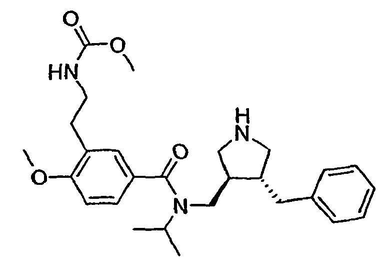

The title compound is prepared analogously as described for the title compound under C in Example 56 using Scheme11a. MS (LC-MS): 468.0 [M+H]⁺; t_{R} (HPLC, Nucleosil C18 column, 10-100% CH₃CN/H₂O/5 min, 100% CH₃CN/3 min, CH₃CN and H₂O containing 0.1 % TFA, flow: 1.5 mL/min): 4.49 min.

### 4-Methoxy-3-(2-methoxycarbonylamino-ethyl)-benzoic acid

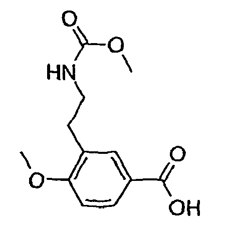

### a. 4-Methoxy-3-(2-methoxycarbonylamino-ethyl)-benzoic acid methyl ester

To a solution of 3-(2-amino-ethyl)-4-methoxy-benzoicacid methyl ester (1 g, 4.07 mmol) in CH₂Cl₂ (15 mL), methylchloroformate (0.34 mL, 4.48 mmol) and diisopropylethylamine (1.61 mL, 13.3 mmol) are added under N₂ atmosphere at 0°C. The mixture is stirred for 22 h at RT, diluted with CH₂Cl₂ and poured into an aqueous saturated solution of NaHCO₃. The organic layer is separated, and the aqueous one is extracted twice with CH₂Cl₂. The combined organic extracts are dried over Na₂SO₄, filtered and concentrated. The crude product is purified by flash chromatography on silica gel (eluent: hexane/AcOEt 2/1 to 1/1) to give the title product as a white solid. ¹H NMR (CDCl₃, 300 MHz): δ = 2.85 (t, 2H), 3.4 (q, 2H), 3.65 (s, 3H), 3.9 (s, 6H), 4.75 (m, 1H), 6.9 (d, 1H), 7.8 (bs, 1H), 7.95 (dd, 1H).

### b. 4-Methoxy-3-(2-methoxycarbonylamino-ethyl)-benzoic acid

A solution of 4-methoxy-3-(2-methoxycarbonylamino-ethyl)-benzoic acid methyl ester (0.9 mg, 3.67 mmol) and NaOH (1 N in water, 15 mL, 15 mmol) in EtOH (20 mL) is refluxed for 1 h. The reaction mixture is allowed to reach RT, and the solvent concentrated under reduced pressure. The residue is dissolved in water (200 mL) and washed with ether (50 mL x 3). The pH is adjusted to 3 by addition of 1 N HCl and the aqueous layer extracted with AcOEt (150 mL x 2). The combined organic extracts are dried over Na₂SO₄, filtered and concentrated *in vacuo.* The crude material is crystallized in ether /hexane (1/10, 15 mL) to afford the desired title product as a white powder. TLC, Rf (AcOEt) = 0.5. ¹H NMR (CDCl₃, 300 MHz): δ = 2.85 (t, 2H), 3.45 (q, 2H), 3.65 (s, 3H), 3.9 (s, 3H), 4.75 (m, 1H), 6.9 (d, 1H), 7.8 (bs, 1H), 7.95 (bd, 1H).

### Example 95: ((3S*,4S*)-4-Benzyl-pyrrolidin-3-ylmethyl)-(4-chloro-ohenyl)-[4-methooy-3-(3 methoxy-propoxy)-benzyl]-amine (see also Scheme9 above)

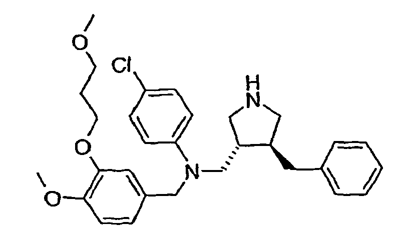

A solution of (3S*,4R*)-3-benzyl-4-[(4-chloro-phenylamino)-methyl]-pyrrolidine-1-carboxylic acid tert-butyl ester (300 mg, 0.75 mmol), 4-bromomethyl-1-methoxy-2-(3-methoxy-propoxy)-benzene (649 mg, 2.245 mmol) and triethylamine (156 µL, 1.12 mmol) in 3 mL DMF is heated at 80 °C for 6 h. The reaction mixture is concentrated, diluted with AcOEt and poured into an aqueous saturated solution of NaHCO₃. The layers are separated, and the aqueous one is extracted twice with AcOEt. The combined organic extracts are dried over Na₂SO₄, filtered and concentrated. According to H-NMR the Boc group is cleaved off. The crude mixture is purified by HPLC preparative (C18-ODB 10 µm , 28x250 mm, eluent: CH3CN /H2O + 0.1 % HCOOH, 5 => 100 % in 25 min, 40 mL/min). The HPLC fractions are collected, diluted with AcOEt and washed with a saturated aqueous solution of NaHCO₃. The organic layer is dried over Na₂SO₄, filtered and concentrated to give the title product. MS (LC-MS): 508.09 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.28 min.

### 4-Bromomethyl-1-methoxy-2-(3-methoxy-propoxy)-benzene

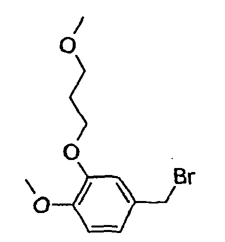

### a. [4-Methoxy-3-(3-methoxy-propoxy)-phenyl]-methanol

To a solution of 4-methoxy-3-(3-methoxy-propoxy)-benzoic acid (3 g, 12.5 mmol) in 30 mL dry THF, LiAlH₄ (1 N in THF, 25 mL, 25 mmol) is added. The solution is stirred for 30 min at RT, then refluxed for 3 h. The reaction mixture is cooled to RT, and 945 µL water are carefully added, followed by 945 µL of aqueous 15% NaOH and finally 2.85 mL of water. The mixture is stirred overnight, filtered and concentrated to give the title product which is used without purification in the next step. TLC, Rf (CH₂Cl₂/MeOH 95/5) = 0.2. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 3.82 min.

### b. 4-Bromomethyl-1-methoxy-2-(3-methoxy-propoxy)-benzene

To a solution of [4-methoxy-3-(3-methoxy-propoxy)-phenyl]-methanol (508 mg, 2.24 mmol) in 4 mL chloroform, trimethylbromosilane (430 µL, 3.37 mmol) is added with stirring at RT under nitrogen for 1h. The reaction mixture is concentrated to give the title product which is used without purification in the next alkylation step. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.72 min.

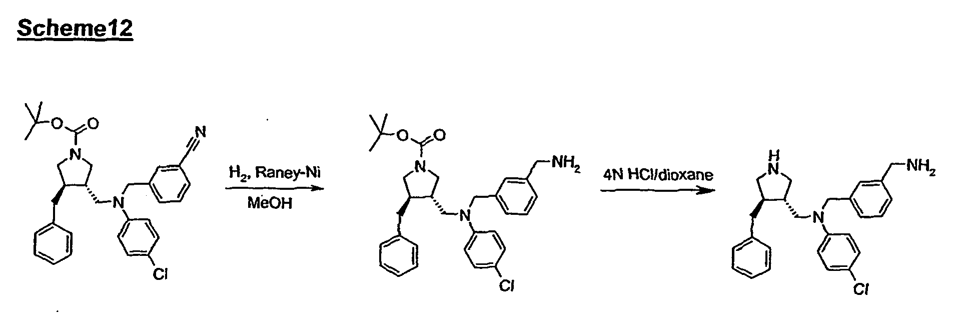

### Example 96: (3-Aminomethyl-benzyl)-((3R*,4R*)-4-benzyl-pyrrolidin-3-ylmethyl)-(4-chloro-phenyl)-amine

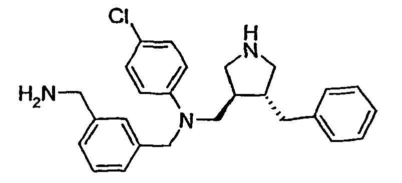

### A. (3S*4R*)-3-{[(3-Aminomethyl-benzyl)-(4-chloro-phenyl)-amino]-methyl}-4-benzyl-pyrrolidine-1-carboxylic acid tert-butyl ester

H₂ is bubbled through a suspension of Raney-Ni (50 mg) and (3S*,4R*)-3-benzyl-4-{[(4-chloro-phenyl)-(3-cyano-benzyl)-amino]-methyl}-pyrrolidine-1-carboxylic acid tert-butyl ester (420 mg, 0.81 mmol) in MeOH (20 mL) during 7 h at RT. Then, the catalyst is filtered off and washed with MeOH. Concentration affords the crude product which is purified by flash column chromatography (60g SiO₂, methyl tert-butyl ether/EtOH/NH₃ 9:1:0.05) to yield 3-{[(3-aminomethyl-benzyl)-(4-chloro-phenyl)-amino]-methyl}-4-benzyl-pyrrolidine-1-carboxylic acid tert-butyl ester as a yellow oil. MS (LC-MS): 520.9 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.83 min.

### B. (3-Aminomethyl-benzyl)-((3R*,4R*)-4-benzyl-pyrrolidin-3-ylmethyl)-(4-chloro-phenyl)-amine

A solution of 3-{[(3-aminomethyl-benzyl)-(4-chloro-phenyl)-amino]-methyl}-4-benzyl-pyrrolidine-1-carboxylic acid tert-butyl ester (230 mg, 0.44 mmol) in 4N HCl/dioxane (8 mL) is stirred at RT for 1 h. The reaction mixture is diluted with ethyl acetate and quenched by addition of a saturated solution of NaHCO₃. Extraction with ethyl acetate, drying (Na₂SO₄) and evaporation of the solvent give (3-aminomethyl-benzyl)-(4-benzyl-pyrrolidin-3-ylmethyl)-(4-chloro-phenyl)-amine which does not require further purification. Addition of 95 µl 4N HCl/dioxane (0.38 mmol) to a solution of the product in dioxane (10 mL) and lyophilization yields the corresponding bis-hydrochloride as a colorless solid. MS (LC-MS): 420.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.40 min.

### Example 97: (8-Aminomethyl-naphthalen-2-ylmethyl)-((3R*,4R*)-4-benzyl-pyrrolidin-3-ylmethyl)-(4-chloro-phenyl)-amine

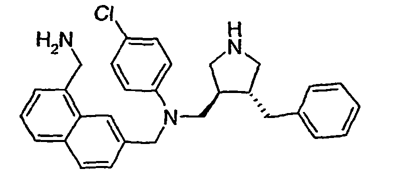

The title compound is prepared analogously as described for the title compound under B in Example 96. MS (LC-MS): 470.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.53 min

### Example 98: (4-Aminomethyl-phenyl)-benzyl-((3R*,4R*)-4-benzyl-pyrrolidin-3-ylmethyl)-amine

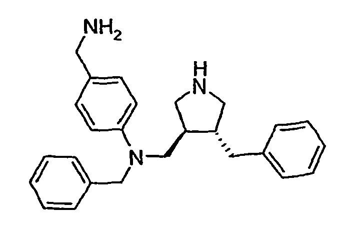

The title compound is prepared analogously as described for the title compound under B in Example 96. MS (LC-MS): 386.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.81 min

### Example 99: (7-Aminomethyl-naphthalen-2-ylmethyl)-((3R*,4R*)-4-benzyl-pyrrolidin-3-ylmethyl)-(4-chloro-phenyl)-amine

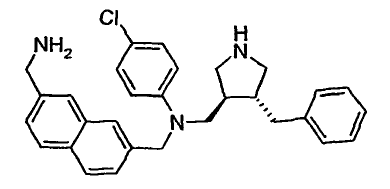

The title compound is prepared analogously as described for the title compound under B in Example 96. MS (LC-MS): 470.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.44 min

### Example 100: (4-Aminomethyl-naphthalen-1-ylmethyl)-((3R*,4R*)-4-benzyl-pyrrolidin-3-ylmethyl)-(4-chloro-phenyl)-amine

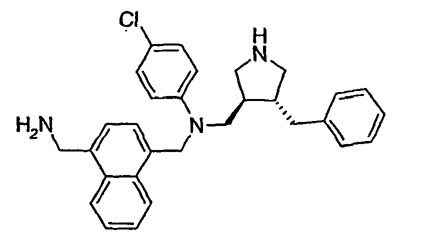

The title compound is prepared analogously as described for the title compound under B in Example 96. MS (LC-MS): 470.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.42 min. 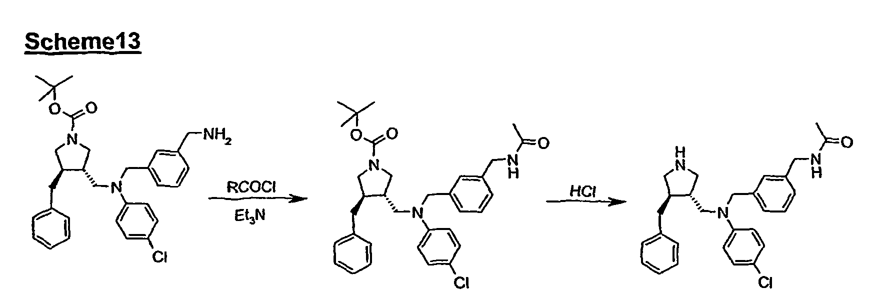 (alternatively to RCOCI, the corresponding anhydride RCO-O-OCR may be used)

### Example 101: N-(3-{[((3R*,4R*)-4-Benzyl-pyrrolidin-3-ylmethyl)-(4-chlororphenyl)-amino]-methyl}-benzyl)-acetamide

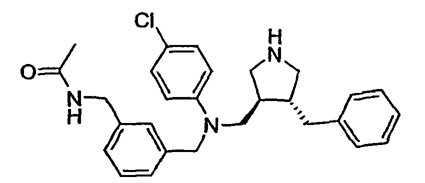

### A. (35*,4R*)-3-{[[3-(Acetylamino-methyl)-benzyl]-(4-chloro-phenyl)-amino]-methyl}-4-benzyl-pyrrolidine-1-carboxylic acid tert-butyl ester

A solution of NaHCO₃ (30 mg, 0.36 mmol) in H₂O (0.4 mL) is added to a solution of (3S,4R)-3-{[(3-aminomethyl-benzyl)-(4-chloro-phenyl)-amino]-methyl}-4-benzyl-pyrrolidine-1-carboxylic acid tert-butyl ester (120 mg, 0.23 mmol) in EtOH (1.5 mL) at RT. Then acetanhydride (22 µl, 0.23 mmol) is added and the suspension is stirred at RT for 14 h. For workup, H₂O is added and the mixture is extracted with CH₂Cl₂. Drying (Na₂SO₄) of the combined organic extracts followed by evaporation of the solvent affords the desired product as a colorless oil which is used directly without further purification. MS (LC-MS): 563.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 6.90 min.

### B. N-(3-{[((3R*,4R*)-4-Benzyl-pyrrolidin-3-ylmethyl)-(4-chloro-phenyl)-amino]-methyl}-benzyl)-acetamide

(35*,4R*)-3g[[3-(acetylamino-methyl)-benzyl]-(4-chloro-phenyl)-amino]-methyl}-4-benzyl-pyrrolidine-1-carboxylic acid tert-butyl ester (100 mg, 0.18 mmol) is treated with 4N HCl/dioxane (8 mL) at RT for 30 min. A saturated solution of NaHCO₃ is added, and the mixture is extracted with ethyl acetate. The combined organic extracts are dried (Na₂SO₄), and the solvent is evaporated to give the desired product as a yellowish oil. After lyophilization of a solution of the product in dioxane (2 mL) and 4N HCl/dioxane (45 µl), the corresponding mono-hydrochloride salt is obtained as a colorless solid. MS (LC-MS): 462.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.13 min.

### Example 102: N-(3-{[((3R*,4R*)-4-Benzyl-pyrrolidin-3-ylmethyl)-(4-chloro-phenyl)-amino]-methyl}-benzyl)-formamide

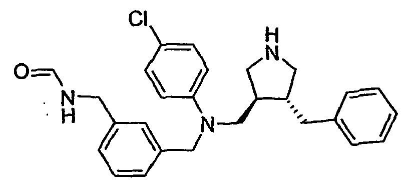

The title compound is prepared analogously as described for the title compound under B in Example 101 from (3S*,4R*)-3-{[(3-aminomethyl-benzyl)-(4-chloro-phenyl)-amino]-methyl}-4-benzyl-pyrrolidine-1-carboxylic acid tert-butyl ester and 4-nitrophenyl formate. MS (LC-MS): 448.9 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.79 min.

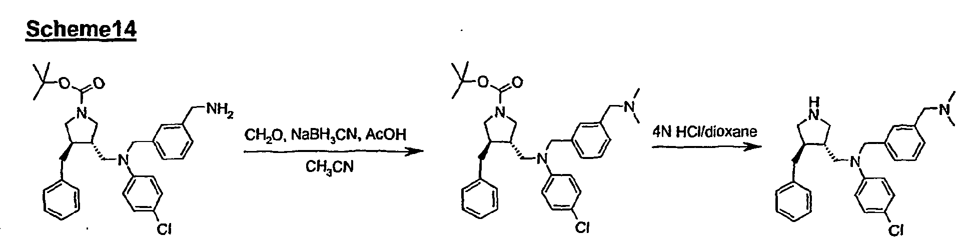

### Example 103: ((3R*,4R*)-4-Benzyl-pyrrolidin-3-ylmethyl)-(4-chloro-phenyl)-(3-dimethylaminomethyl-benzyl)-amine

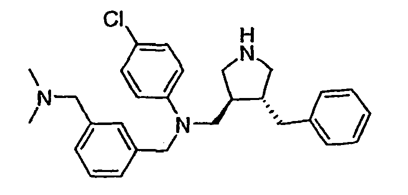

### A. (3R*,45*)-3-Benzyl-4-{[(4-chloro-phenyl)-(3-dimethylaminomethyl-benzyl)-amino]-methyl}-pyrrolidine-1-carboxylic acid tert-butyl ester

CH₂O (64 µl, 0.87 mmol, 37% in H₂O) is added to a solution of (3R*,4S*)-3-{[(3-aminomethyl-benzyl)-(4-chloro-phenyl)-amino]-methyl}-4-benzyl-pyrrolidine-1-carboxylic acid tert-butyl ester (90 mg, 0.17 mmol) in CH₃CN (3 mL). After stirring for 15 min, NaBH₃CN (22 mg, 0.35 mmol) is added, followed by AcOH (150 µl) after another 15 min. The reaction mixture is stirred for 30 min before a saturated solution of NaHCO₃ is added. Extraction with ethyl acetate, drying of the combined extracts (Na₂SO₄) and evaporation of the solvent affords the crude product. Purification by preparative HPLC affords the desired product as a colorless oil. MS (LC-MS): 548.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 6.08 min.

### B. ((3R*,4R*)-4-Benzyl-pyrrolidin-3-ylmethyl)-(4-chloro-phenyl)-(3-dimethylamino methyl-benzyl)-amine

This compound is prepared in analogy to the title compound under B in Example 101 by N-Boc-deprotection of (3S*,4R*)-3-benzyl-4-{[(4-chloro-phenyl)-(3-dimethylaminomethyl-benzyl)-amino]-methyl}-pyrrolidine-1-carboxylic acid tert-butyl ester (30 mg, 0.06 mmol) with 4N HCl/dioxane (2 mL). After lyophilization the desired product is obtained as bis-hydrochloride. MS (LC-MS): 449.1 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.52 min.

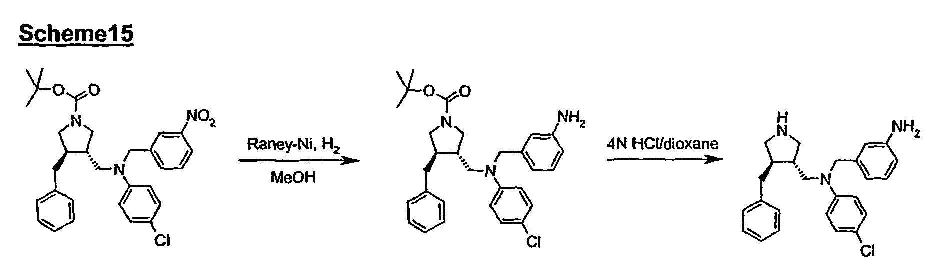

### Example 104: ((3R*,4R*)-4-Benzyl-pyrrolidin-3-ylmethyl)-(4-chloro-phenyl)-(3-amino-benzyl)-amine

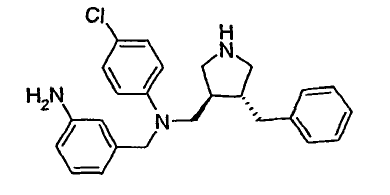

### A. (3R*,45*)-3-{[(3-Amino-benzyl)-(4-chloro-phenyl)-amino]-methyl}-4-benzyl -pyrrolidine-1-carboxylic acid tert-butyl ester

H₂ is bubbled through a suspension of (3S*,4R*)-3-benzyl-4-{[(4-chloro-phenyl)-(3-nitrobenzyl)-amino]-methyl}-pyrrofidine-1-carboxylic acid tert-butyl ester (0.45 g, 0.84 mmol) and Raney-Ni (0.15 g) during 3 h. The catalyst is filtered off and washed with MeOH. Concentration of the solution affords the desired title product as a foam which is directly used without further purification. MS (LC-MS): 506.0[M-H]⁺; t_{R} (HPLC, C8 column, 20-95% CH₃CN /H₂O/3.5 min, 95% CH₃CN/1 min, CH₃CN and H₂O containing 0.1 % TFA, flow: 0.8 mL/min): 4.01 min.

### B. ((3R*,4R*)-4-Benzyl-pyrrolidin-3-ylmethyl)-(4-chloro-phenyl)-(3-amino-benzyl)-amine

This compound is prepared in analogy to the title compound under B in Example 101 by N-Boc-deprotection of (3R*,4S*)-3-{[(3-amino-benzyl)-(4-chloro-phenyl)-amino]-methyl}-4-benzyl-pyrrolidine-1-carboxylic acid tert-butyl ester (0.36 g, 0.71 mmol) with 4N HCl/dioxane (20 mL). After lyophilization the desired product is obtained as bis-hydrochloride. MS (LC-MS): 406.0 [M+H]⁺; t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.21 min

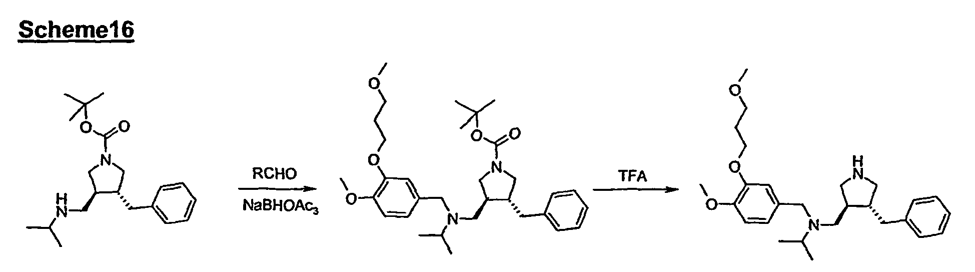

### Example 105: ((3R*4R*)-4-Benzyl-pyrrolidin-3-ylmethyl)-isoaropyl-[4-methoxy-3-(3-methoxy-propoxy)-benzyl]-amine

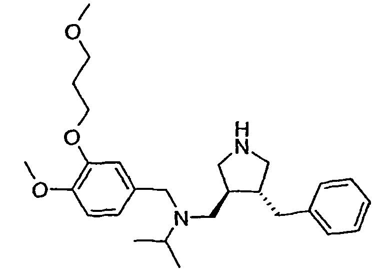

### A. (3R*,4S*)-3-Benzyl-4-({isopropyl-[4-methoxy-3-(3-methoxy-propoxy)-benzyl]-amino}-methyl)-pyrrolidine-1-carboxylic acid tert-butyl ester

(3R*,4S*)-3-Benzyl-4-(isopropylamino-methyl)-pyrrolidine-1-carboxylic acid tert-butyl ester (300 mg, 0.902 mmol) and 4-methoxy-3-(3-methoxy-propoxy)-benzaldehyde (202 mg, 0.90 mmol) are mixed in 1,2-dichloroethane (5 mL) and treated with sodium triacetoxyborohydride (765 mg, 3.61 mmol). The mixture is stirred at RT under nitrogen overnight and poured into an aqueous saturated solution of NaHCO₃. The layers are separated, and the aqueous one is extracted twice with CH₂Cl₂, dried over Na₂SO₄, filtered and concentrated. The crude material is purified by preparative HPLC (C18-ODS-AQ 5 µ, 20x50 mm, eluent: Actonitrile/H₂O + 0.1 % HCOOH).The pure HPLC fractions are collected, diluted with AcOEt and washed with a saturated aqueous solution of NaHCO₃. The organic layer is dried over Na₂SO₄, filtered and concentrated to give the title product. MS (LC-MS): 541 [M+H]⁺;

### B. ((3R*4R*)-4-Benzyl-pyrrolidin-3-ylmethyl)-isopropyl-[4-methoxy-3-(3-methoxy-propoxy)-benzyl]-amine

To a solution of (3R*,4S*)-3-benzyl-4-({isopropyl-[4-methoxy-3-(3-methoxy-propoxy)-benzyl]-amino}-methyl)-pyrrolidine-1-carboxylic acid tert-butyl ester (72 mg, 0.133 mmol) in 2 mL CH₂Cl₂, TFA (0.1 mL) is added, and the mixture is stirred at RT for 1 h. The mixture is concentrated, poured into saturated aqueous solution of NaHCO₃, extracted with AcOEt, dried over Na₂SO₄ and concentrated. The crude material is purified by flash chromatography over NH₂-Isolute (eluent: CH₂Cl₂, /MeOH 100/0 to 90/10+ 5 % of NH₄OH) to give the title product. To a solution of the compound in dioxane (2 mL), 0.11 mmol, 27 µL of 4N HCl in dioxane is added, and the resulting solution is lyophilized to afford the expected HCl salt as a white powder. MS (LC-MS): 441 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 3.88 min.

### 4-Methoxy-3-(3-methoxy-proeoxy)-benzaldehyde

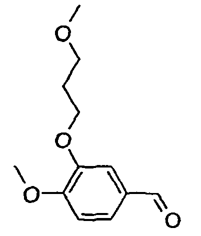

To a solution of 3-hydroxy-4-methoxy-benzaldehyde (3.04 g, 20 mmol) and 3-methoxypropanol (1.80 g, 20 mmol) in THF (150 mL), triphenylphosphine (5.24 g, 20 mmol) is added at RT under nitrogen atmosphere. To the stirring mixture, diethyl azodicarboxylate (3.11 mL, 20 mmol) is added over 10 min at RT, and the resulting solution is further stirred over 20 h. THF is removed under reduced pressure, and the remaining residue is purified by flash chromatography on silica gel (eluent: c-hexane/EtOAc 2/1) to afford the title compound. TLC, Rf (hexane/EtOAc 2/1) = 0.2.

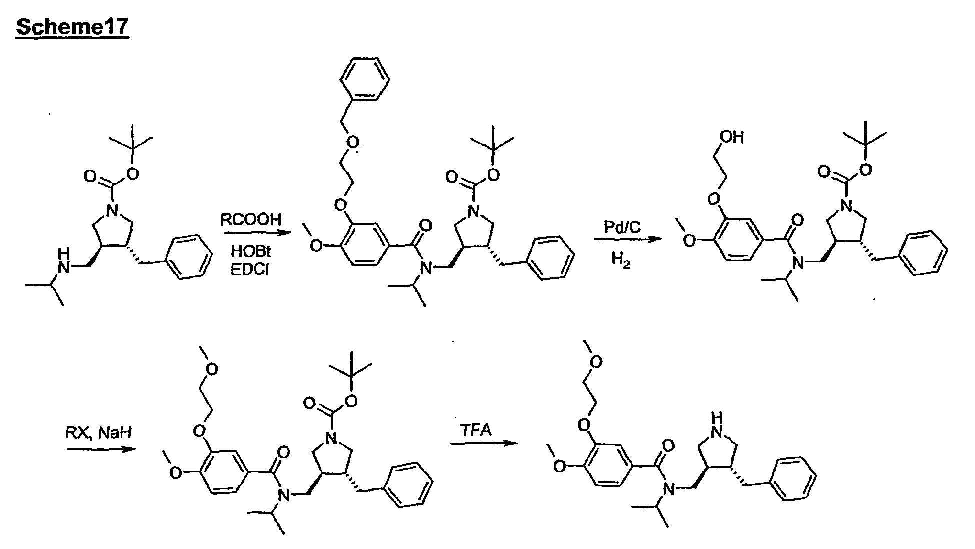

### Example 106: N-((3R*,4R*)-4-Benzvl-pyrrolidin-3-ylmethyl)-N-isopropyl-4-methoxy-3-(2-methoxy-ethoxy)-benzamide

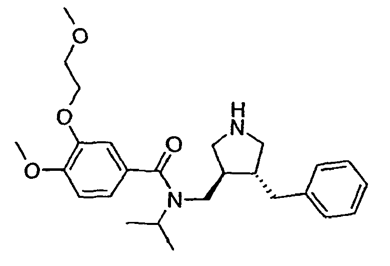

### A. (3R*,4S*)-3-Benzyl-4-({[3-(2-benzyloxy-ethoxy)-4-methoxy-benzoyl]-isopropylamino}-methyl)-pyrrolidine-1-carboxylic acid tert-butyl ester

(3R*,45*)-3-Benzyl-4-(isopropylamino-methyl)-pyrrolidine-1-carboxylic acid tert-butyl ester (1.82 g, 6.015 mmol), HOBT (0.813 g, 6.015 mmol) and EDCI (1.15 g, 6.015 mmol) are suspended in 10 mL dry CH₂Cl₂ under nitrogen. After stirring for 15 min, 3-(2-benzyloxy-ethoxy)-4-methoxy-benzoic acid (2.00 g, 6.015 mmol) in 10 mL CH₂Cl₂ is added, and the mixture is further stirred at RT for 48 h. EDCl (6.015 mmol, 1.15 g) and HOBT (6.015 mmol, 0.813 g) are added, and the mixture is stirred for 2 days at RT under nitrogen. After completion, the reaction mixture is concentrated, and the residue is dissolved in AcOEt and washed with HCl (0.05 N). The organic layer is neutralized with a saturated aqueous solution of NaHCO₃, extracted, dried over Na₂SO₄ and concentrated. The crude material is purified by flash chromatography on silica gel (eluent: c-hexane/AcOEt 90/10 then 2/1) to give the title compound. TLC, Rf (CH₂Cl₂/MeOH 95/5) = 0.5; MS (LC-MS): 517 [M+H-Boc]⁺; t_{R} (HPLC, Nucleosil C18 column, 10-100% CH₃CN/H₂O in 11 min, 100% CH₃CN for 3 min, CH₃CN and H₂O containing 0.1% TFA, flow: 1.5 mL/min): 6.52 min

### B. (3R*,4S*)-3-Benzyl-4-({[3-(2-hydroxy-ethoxy)-4-methoxy-benzoyl]-isopropyl-amino}-methyl)-pyrrolidine-1-carboxylic acid tert-butyl ester

A mixture of (3R*,4S*)-3-benzyl-4-({[3-(2-benzyloxy-ethoxy)-4-methoxy-benzoyl]-isopropylamino}-methyl)-pyrrolidine-1-carboxylic acid tert-butyl ester (442 mg, 0.717 mmol) and Pd(OH)₂/C (0.4 g, 10% wet) in MeOH (5 mL) is stirred under an hydrogen atmosphere for 20 h. The reaction mixture is filtered over a pad of Celite, dried over Na₂SO₄ and concentrated. The crude material is purified by preparative HPLC (Actonitrile/H₂O + 0.1 % TFA).The pure HPLC fractions are collected, diluted with AcOEt and washed with a saturated aqueous solution of NaHCO₃. The organic layer is separated, dried over Na₂SO₄, filtered and concentrated to give the title product. TLC, Rf (CH₂Cl₂/MeOH 95/5) = 0.45. MS (LC-MS): 427 [M+H]⁺.

### C. (3R*,4S*)-3-Benzyl-4-({isopropyl-[4-methoxy-3-(2-methoxy-ethoxy)-benzoyl]-amino}-methyl)-pyrrolidine-1-carboxylic acid tert-butyl ester

(3R*,45*)-3-Benzyl-4-({[3-(2-hydroxy-ethoxy)-4-methoxy-benzoyl]-isopropyl-amino}-methyl)-pyrrolidine-1-carboxylic acid tert-butyl ester (111.5 mg, 0.212 mmol) is suspended with NaH (60 % dispersion in mineral oil, 7 mg, 0.275 mmol) in 2 mL dry DMF under N₂. The mixture is stirred for 30 min before the addition of Mel (52.8 µL, 0.848 mmol). The mixture is further stirred overnight. NaH (60 % dispersion in mineral oil) and Mel (52.8 µL, 0.848 mmol) are added, and the reaction mixture is further stirred for 48 h. Then the mixture is concentrated under *vacuum* and the residue is diluted with CH₂Cl₂ and washed with a saturated aqueous solution of NaHCO₃. The organic layer is separated, and the aqueous one is extracted twice with CH₂Cl₂. The combined organic extracts are dried over Na₂SO₄, filtered and concentrated to give the title product. TLC, Rf (CH₂Cl₂/MeOH 95/5) = 0.4. MS (LC-MS): 441 [M+H-Boc]⁺

### D. N-((3R*,4R*)-4-Benzyl-pyrrolidin-3-ylmethyl)-N-isopropyl-4-methoxy-3-(2-methoxy-ethoxy)-benzamide

TFA (100 µL, 1.29 mmol) is added to a solution of (3R*,4S*)-3-benzyl-4-({isopropyl-[4-methoxy-3-(2-methoxy-ethoxy)-benzoyl]-amino}-methyl)-pyrrolidine-1-carboxylic acid tert-butyl ester (101 mg, 0.187 mmol) in CH₂Cl₂ (2 mL). The resulting mixture is stirred for 1 h at RT and concentrated, diluted with CH₂Cl₂ and quenched with a saturated aqueous solution of NaHCO₃. The layers are separated, and the aqueous one is extracted twice with CH₂Cl₂, dried over Na₂SO₄, filtered and concentrated. The crude material is purified by flash chromatography over NH₂-Isolute (eluent: CH₂Cl₂/MeOH 100/0 to 95/5) to afford the title product. To a solution of the compound in dioxane (2 mL), 0.059 mmol, 14.8 µL of 4N HCl in dioxane is added, and the resulting solution is lyophilized to afford the corresponding hydrochloride salt as a white powder. MS (LC-MS): 441 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.40 min.

### Example 107: N-((3R*,4R*)-4-Benzyl-pyrrolidin-3-ylmethyl)-3-(2-ethoxy-ethoxy)-N-isopropyl-4-methoxy-benzamide

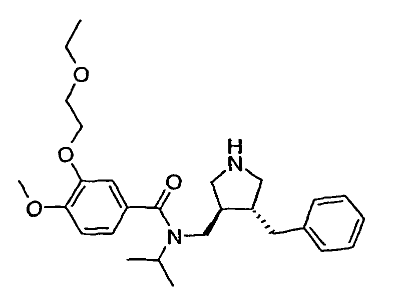

The title compound is prepared analogously as described for the title compound under D in Example 106 (Scheme17) using ethyl iodide instead of methyl iodide in the alkylation step C. MS (LC-MS): 455.0 [M+H]⁺. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.60 min.

### Example 108: N-((3S*,4S*)-4-Benzyl-pyrrolidin-3-ylmethyl)-N-isopropyl-4-methoxy-3-(2-methoxy-ethoxymethyl)-benzamide

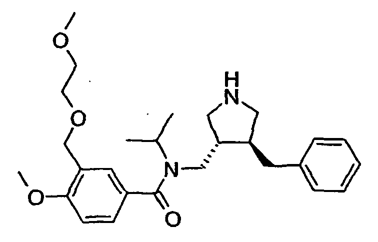

The title compound is prepared analogously as described for the title compound under D in Example 106 (Scheme17) starting from (3R*,4S*)-3-benzyl-4-(isopropylamino-methyl)-pyrrolidine-1-carboxylic acid tert-butyl ester and 3-hydroxymethyl-4-methoxy-benzoic acid in step A. The hydrogenation Step B is not performed. Step C and step D are performed as described for Example 106. MS (LC-MS): 455.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.6 min.

### Example 109: N-((3R*,4R*)-4-Benzyl-pyrrolidin-3-ylmethyl)-3-(3-hydroxy-Dropoxy)-N-isopropyl-4-methoxy-benzamide

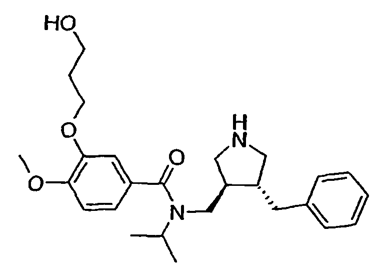

The title compound is prepared analogously as described for the title compound under D in Example 106 (Scheme17) starting from (3R*,4S*)-3-benzyl-4-(isopropylamino-methyl)-pyrrolidine-1-carboxylic acid tert-butyl ester and 3-benzyloxy-4-methoxy-benzoic acid in step A. Step B and step D are performed as described for Example 106. While the alkylation step C is performed as described for the title compound under C in Example 110 (Scheme18) using K₂CO₃ in DMF. MS (LC-MS): 441.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.21 min.

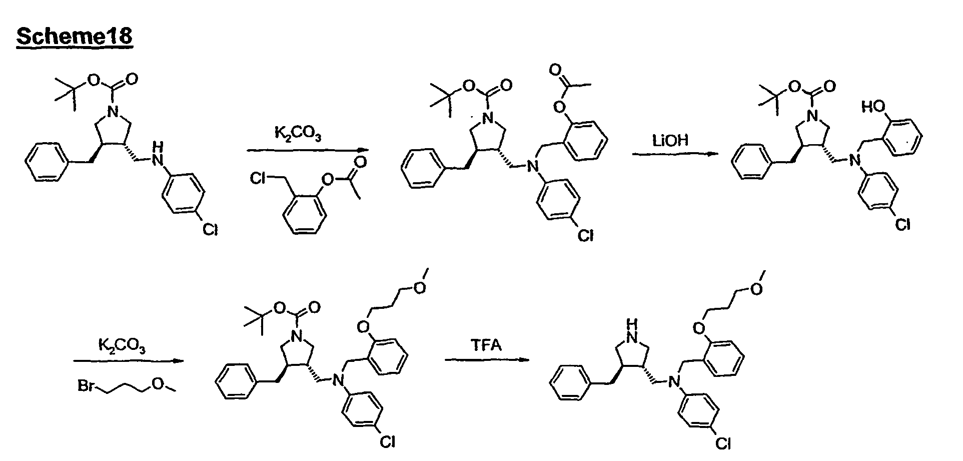

### Example 110: ((3R*,4R*)4-Benzyl-pyrrolidin-3-ylmethyl)-(4-chloro-phenyl)-[2-(3-methoxy-propoxy)-benzyl]-amine

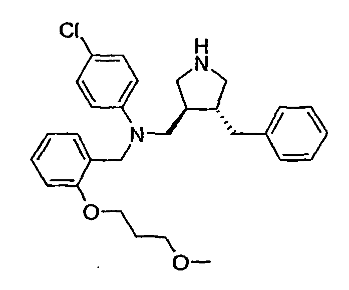

### A. (3S*,4R*)-3-{[(2-Acetoxy-benzyl)-(4-chloro-phenyl)-amino]-methyl}-4-benzyl-pyrrolidine-1-carboxylic acid tert-butyl ester

A mixture (3R*,4S*)-3-benzyl-4-[(4-chloro-phenylamino)-methyl]-pyrrolidine-1-carboxylic acid tert-butyl ester (575 mg, 1.43 mmol), acetic acid 2-chloromethyl-phenyl ester (344 mg, 1.86 mmol), K₂CO₃ (258 mg, 1.86 mmol) and Nal (25 mg, 0.17 mmol) in DMF (8 mL) is stirred under Argon at 80°C for 7 h. For workup an aqueous solution of NaHCO₃ is added, and the mixture is extracted with ethyl acetate. Drying (Na₂SO₄) of the combined extracts and evaporation of the solvent affords the crude product which is purified by flash column chromatography on silica gel (eluent: c-hexane/EtOAc 90/10) to give the title compound. TLC, Rf (Hexane/AcOEt 80/20) = 0.42. MS (LC-MS): 549 [M+H]⁺.

### B. (3R*,4S*)-3-Benzyl-4-{[(4-chloro-phenyl)-(2-hydroxy-benzyl)-amino]-methyl}-pyrrolidine-1-carboxylic acid tert-butyl ester

A solution of (3S*,4R*)-3-{[(2-acetoxy-benzyl)-(4-chloro-phenyl)-amino]-methyl}-4-benzyl-pyrrolidine-1-carboxylic acid tert-butyl ester (195.4 mg, 0.356 mmol) in MeOH (2 mL) is cooled to 0°C and LiOH.H₂O (89.6 mg, 2.14 mmol) is added. The reaction mixture is allowed to reach RT, further stirred for 2 days and neutralized with HCl 1N (2.14 mmol). CH₂Cl₂ and water are added, the organic layer is separated, and the aqueous one is extracted twice with CH₂Cl₂. The combined organic layers are dried over Na₂SO₄, filtered and concentrated *in vacuo.* The crude material is purified by flash chromatography on silica gel (c-hexane/AcOEt 90/10) to give the title compound. TLC, Rf (Hexane/AcOEt 2/1) = 0.58. MS (LC-MS): 507.0 [M+H]⁺.

### C. (3R*,4S*)-3-Benzyl-4-({(4-chforo-phenyl)-[2-(3-methoxy-propoxy)-benzyl]-amino}-methyl)-pyrrolidine-1-carboxylic acid tert-butyl ester

A mixture of (3R*,4S*)-3-benzyl-4-{[(4-chloro-phenyl)-(2-hydroxy-benzyl)-amino]-methyl}-pyrrolidine-1-carboxylic acid tert-butyl ester (100 mg, 0.197 mmol), 1-bromo-3-methoxypropane (37 mg, 0.240 mmol) and K₂CO₃ (218 mg, 1.576 mmol) in DMF (3 mL) is heated at 80°C overnight. AcOEt is added and the reaction mixture quenched by addition of an aqueous saturated solution of NaHCO₃. The organic layer is separated, and the aqueous one is extracted twice with AcOEt, dried over Na₂SO₄ and concentrated *in vacuo*. The crude material is purified by flash chromatography on silica gel (c-hexane/AcOEt 90/10) to give the title product. TLC, Rf (Hexane/AcOEt 80/20) = 0.44. MS (LC-MS): 579 [M+H]⁺.

### D. ((3R*,4R*)4-Benzyl-pyrrolidin-3-ylmethyl)-(4-chloro-phenyl)-[2-(3-methoxy-propoxy)-benzyl]-amine

To a solution of (3R*,4S*)-3-benryl-4-({(4-chtoro-phenyl)-[2-(3-methoxy-propoxy)-benzyl]-amino}-methyl)-pyrrolidine-1-carboxylic acid tert-butyl ester (96 mg, 0.166 mmol) in 2 mL CH₂Cl_{2.} TFA (120 µL) is added. The mixture is stirred at RT for 24 h, concentrated *in vacuo*, taken up in CH₂Cl₂ and poured into a saturated aqueous solution of NaHCO₃. The layers are separated, and the aqueous one is extracted twice with CH₂Cl₂. The combined organic layers are dried over Na₂SO₄ and concentrated under reduced pressure. The crude material is purified by flash chromatography over Isolute SPE Flash NH₂ column (c-hexane/ AcOEt 2/1 + 2% MeOH) to give the title product. To a solution of the compound in dioxane, 0.138 mmol, 34.4 µL of 4N HCl in dioxane is added, and the resulting solution is lyophilized to afford the corresponding hydrochloride salt as a white powder. MS (LC-MS): 479, 481 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.74 min.

### Example 111: ((3R*,4R*)-4-Benzyl-pyrrolidin-3-ylmethyl)-(4-chloro-phenyl)-[2-(2-methoxy-ethoxy)-benzyl]-amine

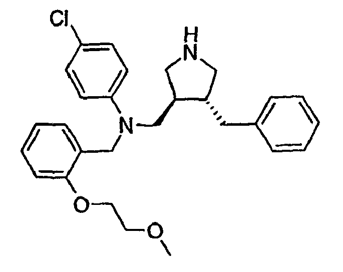

The title compound is prepared analogously as described for the title compound under D in Example 110 using 1-bromo-2-methoxyethane as the alkylating agent. MS (LC-MS): 465, 467 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.56 min

### Example 112: 2-{[((3R*,4R*)-4-Benzyl-pyrrolidin-3-ylmethyl)-(4-chloro-phenyl)-amino]-methyl}-phenol

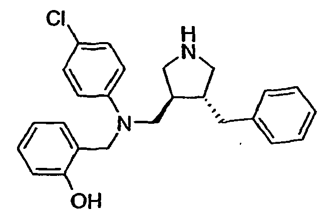

The title compound is prepared analogously as described for the title compound under D in Example 110 by N-Boc-deprotection of (3R*,4S*)-3-benzyl-4-{[(4-chloro-phenyl)-(2-hydroxybenzyl)-amino]-methyl}-pyrrolidine-1-carboxylic acid tert-butyl ester. MS (LC-MS): 407, 407.9 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.55 min.

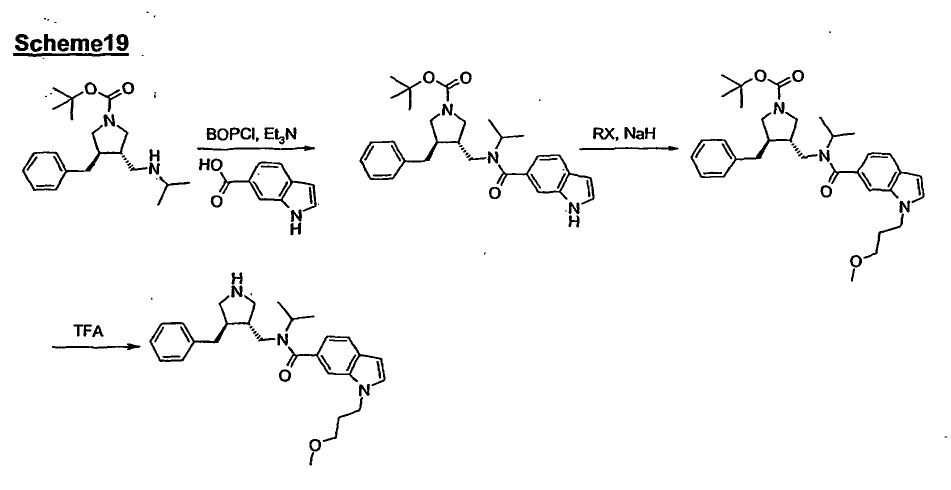

### Example 113: 1-(3-Methoxy-propyl)-1H-indole-6-carboxylic acid ((3R*,4R*)-4-benzyl-pyrrolidin-3-ylmethyl)-isopropyl-amide

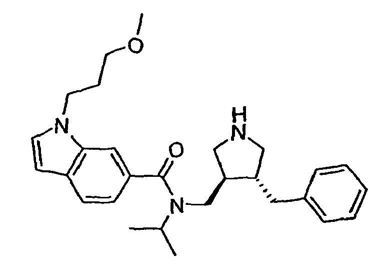

### A. (3R*,4S*)-3-Benzyl-4-{[(1H-indole-6-carbonyl)-isopropyl-amino]-methyl}-pyrrolidine-1-carboxylic acid tert-butyl ester

To a solution of (3R*,4S*)-3-benzyl-4-(isopropylamino-methyl)-pyrrolidine-1-carboxylic acid tert-butyl ester (1.25 g, 3.76 mmol) and indol-6-carboxylic acid (0,59 g, 3.68 mmol) in CH₂Cl₂ (30 mL), bis(2-oxo-3-oxazolidinyl)phosphinic chloride (1.1 g, 4.33 mmol) and triethylamine (2.1 mL, 15.04 mmol) are added. The mixture is heated at 60°C in a closed vial for 1 h and poured into a saturated solution of NaHCO₃. The layers are separated, and the aqueous one is extracted twice with CH₂Cl₂. The combined organic layers are dried over Na₂SO₄, filtered and concentrated. The crude material is purified by flash chromatography on silica gel (eluent: CH₂Cl₂/AcOEt 100/0 to 70/30) to give the title compound. MS (LC-MS): 474.1 [M-H]⁺; t_{R} (HPLC, Nucleosil C18 column, 10 to 100% CH₃CN in H₂O in 5 min, the 100 % CH₃CN for 3 min, CH₃CN and H₂O containing 0.1% TFA, flow: 1.5 mL/min): 6.23 min.

### B. (3R*,4S*)-3-Benzyl-4-({isopropyl-[1-(3-methoxy-propyl)-1H-indole-6-carbonyl]-amino}-methyl)-pyrrolidine-1-carboxylic acid tert-butyl ester

To a solution of (3R*,4S*)-3-benzyl-4-{[(1H-indole-6-carbonyl)-isopropyl-amino]-methyl}-pyrrolidine-1-carboxylic acid tert-butyl ester (300 mg, 0.63 mmol) in DMF (3 mL), slowly NaH (27.6mg, 0.69 mmol, 60% dispersion in grease) in DMF (3mL) is added under a N₂ atmosphere. The mixture is stirred at 80°C for 20 min, and cooled to RT before the addition of 3-chloro-methoxy propane (136.7 mg, 1.26 mmol). The reaction mixture is stirred at 60°C for 2 days, cooled to RT, diluted with AcOEt and extracted with an aqueous saturated solution of NaHCO₃. The aqueous layer is extracted twice with AcOEt, and the combined organic extracts are dried over Na₂SO₄, filtered and concentrated. The crude residue is purified by flash chromatography on silica gel (eluent: CH₂Cl₂/MeOH 100/0 to 90/10) to give the title compound. MS (LC-MS): 448.1[M+H-Boc]⁺; t_{R} (HPLC, Nucleosil C18 column, 10 to 100% CH₃CN in H₂O in 5 min, then 100% CH₃CN for 3 min, CH₃CN and H₂O containing 0.1% TFA, flow: 1.5 mL/min): 6.74 min.

### C. 1-(3-Methoxy-propyl)-1H-indole-6-carboxylic acid ((3R*,4R*)-4-benzyl-pyrrolidin-3-ylmethyl)-isopropyl-amide

To a solution of (3R*,4S*)-3-benzyl-4-({isopropyl-[1-(3-methoxy-propyl)-1H-indole-6-carbonyl]-amino}-methyl)-pyrrolidine-1-carboxylic acid tert-butyl ester (0.51 g, 0.93 mmol) in 5 mL CH₂Cl₂ is added TFA (764 µL). The mixture is stirred at RT for 2 h and poured into an aqueous saturated solution of NaHCO₃. The layers are separated, and the aqueous one is back-extracted twice with CH₂Cl₂. The combined organic extracts are dried over Na₂SO₄, filtered and concentrated. The crude material is purified by flash chromatography on silica gel (eluent: CH₂Cl₂/MeOH 90/10 to 90/10+1% NH₄OH) to give the title product. To a solution of the free base (80 mg, 0.18 mmol) in dioxane (2 mL), 1.0 equivalent (0.18 mmol, 46 µL) of 4N HCl in dioxane are added, and the resulting solution is lyophilized to afford the corresponding hydrochloride salt as a white powder. MS (LC-MS): 448.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.86 min.

### Example 114: 1-(2-Methoxy-ethyl)-1H-indole-6-carboxylic acid ((3R*,4R*)-4-benzyl-pyrrolidin-3-ylmethyl)-isopropyl-amide

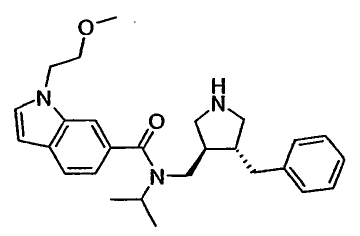

The title compound is prepared analogously as described for the title compound under C in Example 113 using 1-bromo-2-methoxyethane as the alkylating agent. MS (LC-MS): 434 [M+H]⁺; t_{R} (HPLC, Nucleosil C18 column, 10-100% CH₃CN/H₂O/5 min, 100% CH₃CN/3 min, CH₃CN and H₂O containing 0.1 % TFA, flow: 1.5 mL/min): 4.73 min.

### Example 115: 1-(3-Methoxy-propyl)-1H-indole-5-carboxylic acid ((3R*,4R*)-4-benzyl-pyrrolidin-3-ylmethyl)-isopropyl-amide (see also Scheme19 above)

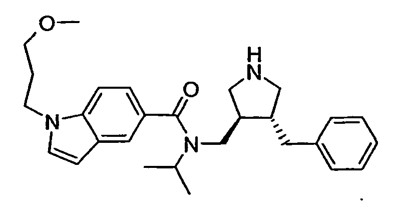

The title compound is prepared analogously as described for the title compound under C in Example 113 using indol-5-carboxylic acid in the coupling reaction step A and 1-bromo-2-methoxypropane as the alkylating agent in step B. MS (LC-MS): 448.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.78 min.

### Example 116: 1-(2-Methoxy-ethyl)-1H-indole-5-carboxylic acid ((3R*,4R*)-4-benzyl-pyrrolidin-3-ylmethyl)-isopropyl-amide (see also Scheme19 above)

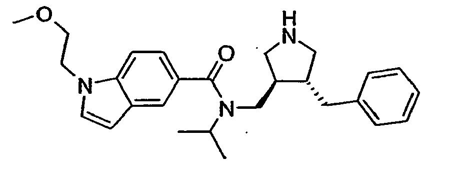

The title compound is prepared analogously as described for the title compound under C in Example 113 using indol-5-carboxylic acid in the coupling reaction step A and 1-bromo-2-methoxyethane as the alkylating agent in step B. MS (LC-MS): 434.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.70 min.

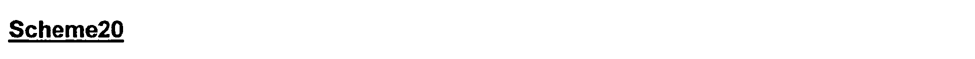

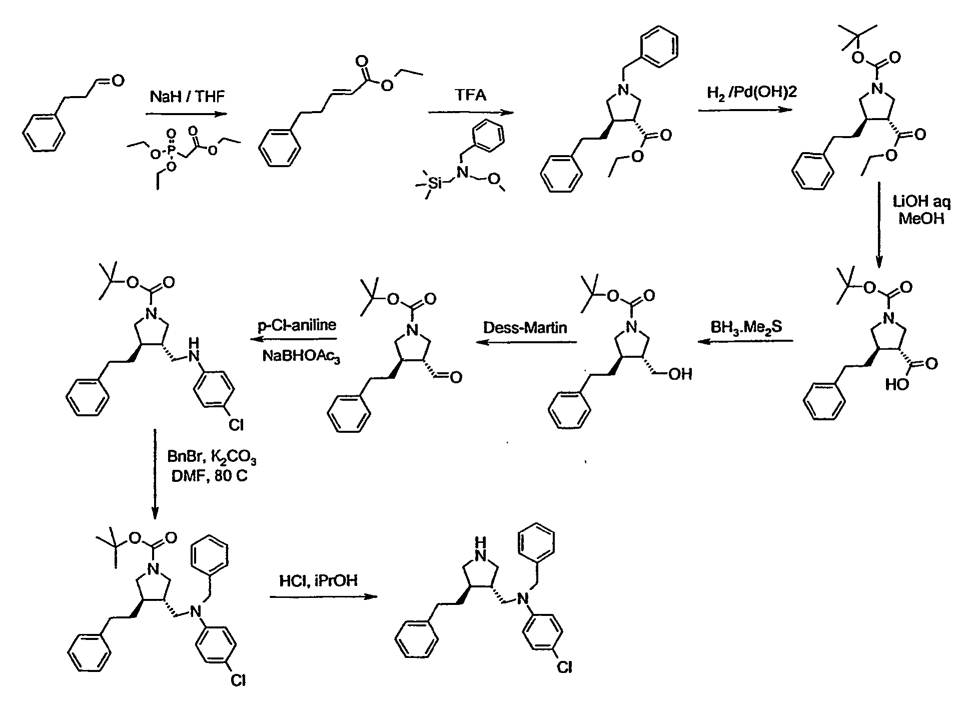

### Example 117: Benzyl-(4-chloro-phenyl)-((3R*,4R*)-4-phenethyl-pyrrolidin-3-ylmethyl)-amine

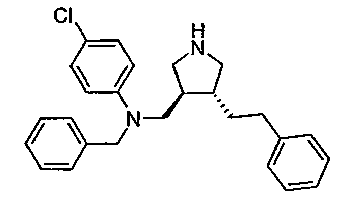

### A. (E)-5-Phenyl-pent-2-enoic acid ethyl ester

To a solution of triethylphosphono acetate (7.4 mL, 37.3 mmol) in THF (80 mL) under N₂ atmosphere, at RT sodium hydride (60% dispersion in oil, 1.49 g, 37.3 mmol) is added. The reaction mixture is stirred at 25°C for 45 min, and propionaldehyde (4.58 mL, 34.2 mmol) is added. After 30 min at RT, the reaction is partitioned between Et₂O and water. The aqueous layer is extracted 3 times with Et₂O, dried over Na₂SO₄, filtered and concentrated. The crude material is purified by flash chromatography on silica gel (eluent: hexane/ACOET 100/0 to 90/10) to give the title compound as a colorless oil. TLC, Rf (Hexane/AcOEt 90/10) = 0.5.
¹H NMR (CDCl₃, 400 MHz): δ = 1.35 (t, 3H), 2.55 (q, 2H), 2.8 (t, 3H), 4.22 (q, 2H), 5.85 (dt, 1H), 7.05 (dt, 1H), 7.22 (m, 3H), 7.35 (m, 2H).

### B. (3R*,4R*)-1-Benzyl-4-phenethyl-pyrrolidine-3-carboxylic acid ethyl ester

To a stirred solution of (E)-5-phenyl-pent-2-enoic acid ethyl ester (4.0 g, 19.58 mmol) in toluene (50 mL), N-benzyl-N-(methoxymethyl) trimethylsilyl amine (5.51 mL, 21.53 mmol) at 0°C is added under N₂. A solution of trifluoroacetic acid (1.9 mmol, 0.15 mL) is added at 0°C, and the mixture is stirred at 0°C for 30 min and then at RT for 2 days. The reaction is quenched with a saturated aqueous solution of NaHCO₃, extracted with AcOEt, dried over Na₂SO₄ and concentrated. The crude material is purified by flash chromatography on silica gel (eluent: hexane/AcOEt 90/10) to give the title compound as a colorless oil. TLC, Rf (Hexane/AcOEt 90/10) = 0.35. ¹H NMR (MeOD, 400 MHz): δ = 1.23 (t, 3H), 1.76 (m, 1H), 1.92 (m, 1H), 2.3 (dd, 1H), 2.44 (m, 1H), 2.62 (m, 2H), 2.66-277 (m, 2H), 2.88-2.93 (m, 2H), 3.56 (d, 1H), 3.68 (d, 1H), 4.09-4.18 (m, 2H), 7.18 (m, 2H), 7.27 (m, 3H), 7.35 (m, 5H).

### C. (3R*,4R*)-4-Phenethyl-pyrrolidine-1,3-dicarboxylic acid 1-tert-butyl ester 3-ethyl ester

A mixture of (3R*,4R*)-1-benzyl-4-phenethyl-pyrrolidine-3-carboxylic acid ethyl ester (0.98 g, 8.88 mmol), di-tert-butylcarbonate (1.94 g, 8.88 mmol) and Pd(OH)₂/C (1 g, 50% wet) in EtOH (100 mL) is stirred under an hydrogen atmosphere for 1 h. The crude material is filtered over a pad of Celite, dried over Na₂SO₄ and concentrated. Chromatography on silica gel (eluent: hexane/AcOEt 90/10) of the crude material gives the title compound. TLC, Rf (Hexane/AcOEt 80/20) = 0.4. ¹H NMR (CD₃OD, 400 MHz): δ = 1.28 (t, 3H), 1.49 (s, 9H), 1.72 (m, 1H), 1.95 (m, 1H), 2.65 (m, 1H), 2.69 (m, 2H), 2.86 (m, 1H), 3.04 (m, 1H), 3.64 (m, 1H), 3.67 (m, 2H), 4.20 (m, 2H), 7.21 (m, 3H), 7.30 (m, 2H).

### D. (3R*,4R*)-4-Phenethyl-pyrrolidine-1,3-dicarboxylic acid 1-tert-butyl ester

A solution of (3R*,4R*)-4-phenethyl-pyrrolidine-1,3-dicarboxylic acid 1-tert-butyl ester 3-ethyl ester (0.30 g, 0.86 mmol) in MeOH (5 mL) is cooled to 0°C, and (1.29 mL, 1.29 mmol) of a solution of LiOH 1N is added. The reaction mixture is allowed to reach RT and further stirred for 6 h. A solution of aqueous HCL (5%) is added, and the mixture is extracted with EtOAc (3 x 15 mL), dried over Na₂SO₄, filtered and concentrated to give the title compound which is used in the next step without purification. TLC, Rf (Hexane/AcOEt 80/20) = 0.05. ¹H NMR (CD₃OD, 400 MHz): δ = 1.5 (s, 9H), 1.7 (m, 1H), 2.05 (m, 1H), 2.21 (m, 1H), 2.7 (m, 2H), 2.82 (m, 1H), 3.02 (m, 1H), 3.52 (m, 1H), 3.63 (m, 2H), 7.07-7.15 (m, 5H).

### E. (3R*,4R*)-3-Hydroxymethyl-4-phenethyl-pyrrolidine-1-carboxylic acid tert-butyl ester

To a solution of (3R*,4R*)-4-phenethyl-pyrrolidine-1,3-dicarboxylic acid 1-tert-butyl ester (0.577 g, 1.81 mmol) in THF (10 mL), a solution of borane dimethylsulfide complex (2N in THF, 1.44 mL, 2.89 mmol) is slowly added at -10°C. The reaction mixture is stirred for 20 min at -10°C and allowed to reach RT and further stirred for 5 h. The mixture is carefully poured into MeOH and concentrated under reduced pressure. The residue is taken up in CH₂Cl₂ and extracted with an aqueous saturated solution of NaHCO₃. The organic layer is dried over Na₂SO₄, filtered and concentrated to give the title compound. The compound is used in the next step without purification. TLC, Rf (CH₂Cl₂/MeOH 90/10) = 0.45. ¹H NMR (CD₃OD, 400 MHz): δ = 1.49 (s, 9H), 1.58 (m, 1H), 1.90-2.10 (m, 3H), 2.66 (m, 2H), 3.04 (m, 1H), 3.08 (m, 1H), 3.51 (m, 1H), 3.58 (m, 2H), 3.67 (dd, 1H), 7.17-7.31 (m, 5H).

### F. (3R*,4R*)-3-Formyl-4-phenethyl-pyrrolidine-1-carboxylic acid tert-butyl ester

To a well stirred mixture of (3R*,4R*)-3-hydroxymethyl-4-phenethyl-pyrrolidine-1-carboxylic acid tert-butyl ester (0.54 g, 1.78 mmol) and Dess-martin Periodinane (0.75 g, 1.78 mmol) in CH₂Cl₂ (10 mL), slowly wet CH₂Cl₂ (1.95 mmol, 0.035 mL of water in 5 mL of CH₂Cl₂) is added. The clear solution becomes cloudy toward the end of wet CH₂Cl₂ addition. The mixture is diluted with Et₂O, then concentrated to a few mL of solvent by rotary evaporation. The residue is taken up in Et₂O (100 mL) and washed with 30 mL of 1/1 10% Na₂S₂O₃ saturated aqueous NaHCO₃, followed by 35 mL of H₂O and 35 mL of brine. The aqueous washings are back-extracted with 60 mL of Et₂O, and this organic layer is washed with H₂O and brine. The combined organic layers are dried with Na₂SO₄, filtered and concentrated. The crude material is purified by flash chromatography on silica gel (eluent; c-hexane/AcOEt 80/20) to give the title compound as a slightly yellow oil. TLC, Rf (c-hexane/AcOEt 80/20) = 0.25. ¹H NMR (CD₃OD, 400 MHz): δ = 1.49 (s, 9H), 1.58 (m, 1H), 2.13 (m, 3H), 2.67 (m, 2H), 3.04 (m, 1H), 3.25-3.32 (m, 1H), 3.48 (m, 1H), 3.63 (m, 1H), 4.46 (dd, 1H), 7.16-7.31 (m, 5H).

### G. (3S*,4R*)-3-[(4-Chloro-phenylamino)-methyl]-4-phenethyl-pyrrolidine-1-carboxylic acid tert-butyl ester

(3R*,4R*)-3-Formyl-4-phenethyl-pyrrolidine-1-carboxylic acid tert-butyl ester (0.54 g, 1.78 mmol) and 4-chloroaniline (0.22 g, 1.78 mmol) are mixed in 1,2-dichloroethane (5 mL) and treated with sodium triacetoxyborohydride (0.52 g, 2.49 mmol). The mixture is stirred at RT under nitrogen overnight, quenched by addition of 10 mL aqueous saturated solution of NaHCO₃, extracted with CH₂Cl₂, dried over Na₂SO₄, filtered and concentrated. The residual brown oil is purified by flash chromatography on silica gel (eluent: c-hexane/AcOEt 90/10) to give the title compound (0.54 g). TLC, Rf (c-hexane/AcOEt 90/10) = 0.25. MS (LC-MS): 358.94 [M+H-tBu]⁺.

### H. (3S*,4R*)-3-{[Benzyl-(4-chloro-phenyl)-amino]-methyl}-4-phenethyl-pyrrolidine-1-carboxylic acid tert-butyl ester

To a solution of (3S*,4R*)-3-[(4-chloro-phenylamino)-methyl]-4-phenethyl-pyrrolidine-1-carboxylic acid tert-butyl ester (0.15 g, 0.36 mmol) in DMF (5 mL), K₂CO₃ (0.059 g, 0.43 mmol) and benzylbromide (0.047 mL, 0.39 mmol) are added. The mixture is stirred at 80 °C overnight. The reaction mixture is then quenched with a saturated aqueous solution of NaHCO₃, extracted with AcOEt, dried over Na₂SO₄, filtered and concentrated. The crude material is purified by flash chromatography (eluent: c-Hexane /AcOEt 95/5 to 90/10) to give the title compound. TLC, Rf (c-hexane/AcOEt 80/20) = 0.55. ¹H NMR (CD₃OD, 400 MHz): δ = 1.49 (s, 9H), 1.63 (m, 1H), 1.95 (m, 2H), 2.39 (m, 1H), 2.59 (m, 2H), 3.05-3.21 (m, 2H), 3.48 (m, 1H), 3.52-3.57 (m, 3H), 4.53 (s, 2H), 6.72 (d, 2H), 7.12-7.33 (m, 7H).

### I. Benzyl-(4-chloro-phenyl)-((3R*,4R*)-4-phenethyl-pyrrolidin-3-ylmethyl)-amine

To a solution of (3S*,4R*)-3-{[benzyl-(4-chloro-phenyl)-amino]-methyl}-4-phenethyl-pyrrolidine-1-carboxylic acid tert-butyl ester (0.117 g, 0.23 mmol) in isopropanol (2 mL), slowly 6N HCL (2 mL) is added. The mixture is further stirred at RT overnight and concentrated to dryness, taken up in CH₂Cl₂ and neutralized with a saturated aqueous solution of NaHCO₃. The organic layer is separated, and the aqueous one is extracted twice with CH₂Cl₂, dried over Na₂SO₄, filtered and concentrated. The compound is taken up into dioxane (2 mL) and 1 equivalent of a 4 N HCl (0.027 mL) solution in dioxane is added, and the resulting solution is lyophilized to give the title compound as a white powder. MS (LC-MS): 405, 407 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.62 min.

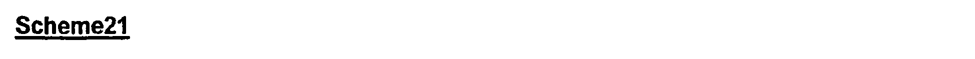

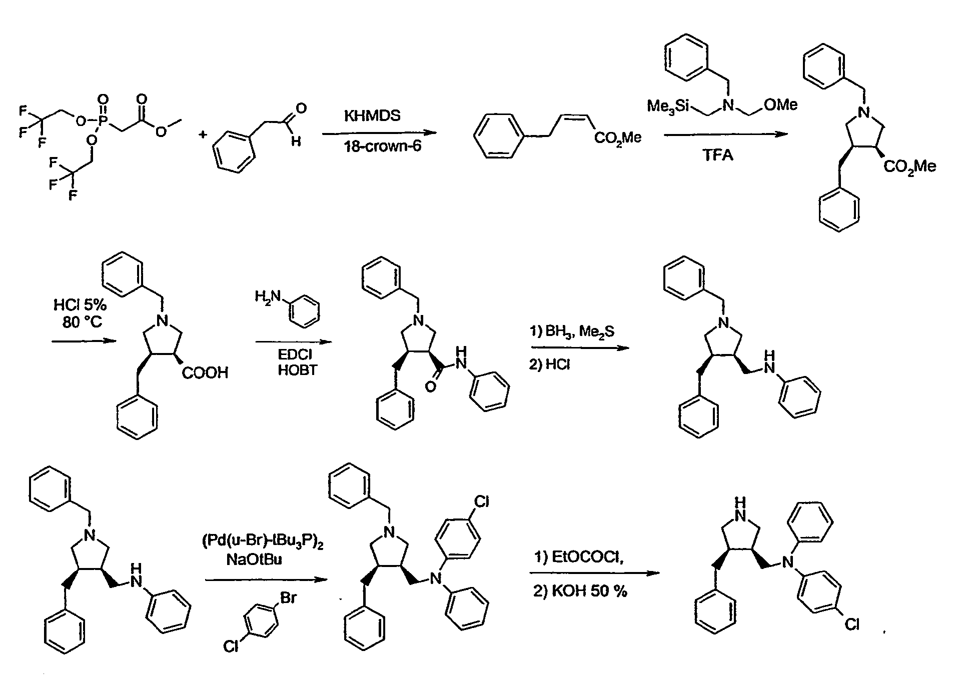

### Example 118: ((3S*,4R*)-4-Benzyl-pyrrolidin-3-ylmethyl)-(4-chloro-phenyl)-phenyl-amine

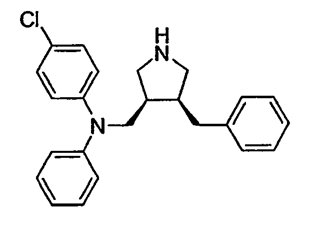

### A. ((Z)-But-2-enyl)-benzene

A solution of 18-crown-6 (13.22 g, 50.0 mmol) and bis(2,2,2-trifluoroethyl) (methoxycarbonylmethyl) phosphonate (3 mL, 14.1 mmol) in dry THF (80 mL) is cooled to -78°C, and treated with KHMDS (0.5 M in toluene, 28.20 mL, 14.1 mmol). The mixture is stirred 1 h, and phenylacetaldehyde (1.50 mL, 12.8 mmol) is added over a period of 15 min. After completion of the reaction, the mixture is poured into 100 mL of a saturated aqueous solution of NH₄Cl and extracted twice with ether (2x50 mL). The combined organic layers are dried over Na₂SO₄, filtered and concentrated. The crude residue is purified by flash chromatography on silica gel (eluent: c-hexane/EtOAc 98/2) to give the desired compound. TLC, Rf (c-hexane/AcOEt 95/5) = 0.45.

### B. (3S*,4R*)-1,4-Dibenzyl-pyrrolidine-3-carboxylic acid methyl ester

To a stirred solution of ((Z)-but-2-enyl)-benzene (317 mg, 1.80 mmol) and N-benzyl-N-(methoxymethyl) trimethylsilyl amine ( 0.55 mL, 2.16 mmol) in toluene (5 mL) under N₂, trifluoroacetic acid (14 µL, 0.18 mmol) is added at 0°C. The mixture is stirred at 0°C for 30 min and then at RT overnight. The reaction is quenched with a saturated aqueous solution of NaHCO₃ (30 mL), extracted with CH₂Cl₂ (2x30mL), dried over Na₂SO₄, filtered and concentrated. The crude material is purified by flash chromatography on silica gel (eluent : c-hexane/AcOEt 80/20) to give the title compound as a colorless oil. TLC, Rf (c-hexane/AcOEt 90/10) = 0.2. ¹H NMR (CD₃OD, 400 MHz): δ = 2.27 (m, 1H), 2.54 (m, 1H), 2.81-2.87 (m, 4H), 3.09 (bt, 1H), 3.24 (q, 1H), 3.62 (d, 1H), 3.69 (s, 3H), 3.71 (d, 1H), 7.17-7.34 (m, 10H).

### C. (3S*,4R*)-1,4-Dibenzyl-pyrrolidine-3-carboxylic acid

(3S*,4R*)-1,4-Dibenzyl-pyrrolidine-3-carboxylic acid methyl ester (1.20 g, 3.88 mmol) is treated with 5 % aqueous HCl solution (1.24 mL) and stirred overnight. The reaction mixture is neutralized by the addition of 1 equivalent of NaOH (1 N) and concentrated. The resulting residue is diluted with AcOEt, filtered and concentrated to give the title compound. TLC, Rf (c-hexane/AcOEt 90/10) = 0.15. ¹H NMR (CD₃OD, 400 MHz): δ = 2.54 (m, 2H), 2.78 (m, 2H), 3.03-3.17 (m, 4H), 3.83 (s, 2H), 7.14-7.39 (m, 10H).

### D. (3S*,4R*)-1,4-Dibenzyl-pyrrolidine-3-carboxylic acid phenylamide

(3S*,4R*)-1,4-Dibenzyl-pyrrolidine-3-carboxylic acid (600 mg, 2.03 mmol), HOBT (274 mg, 2.03 mmol) and EDCI (389 mg, 2.03 mmol) are suspended in 8 mL dry CH₂Cl₂ under N₂. After stirring 15 min, aniline (185 µL, 2.03 mmol) is added, and the reaction mixture is further stirred at RT for 23 h. The reaction mixture is then filtered, concentrated and the residue dissolved in AcOEt . An aqueous saturated solution of NaHCO₃ is added, the layers are separated, and the aqueous one is extracted twice with AcOEt. The combined organic layers are dried over Na₂SO₄ and concentrated. The crude material is purified by flash chromatography (CH₂Cl₂/MeOH 98/2) to give the title compound. TLC, Rf (CH₂Cl₂/MeOH 95/5) = 0.45. MS (LC-MS): 371 [M+H]⁺

### E. ((3R*,4R*)-1,4-Dibenzyl-pyrrolidin-3-ylmethyl)-phenyl-amine

To a solution of (3S*,4R*)-1,4-dibenzyl-pyrrolidine-3-carboxylic acid phenylamide (380 mg, 1.03 mmol) in 4 mL THF, BH₃.Me₂S (2N in THF, 3.08 mL, 6.15 mmol) is added. The mixture is refluxed for 5 h, MeOH is carefully added, and the solution is concentrated under reduced pressure. The residual oil is refluxed overnight in a mixture of cc. HCl (4 mL) and MeOH (4 mL). After cooling to RT, the reaction mixture is neutralized with NaOH 15 % and extracted twice with CH₂Cl₂, dried over Na₂SO₄ and concentrated. The crude material is purified by flash chromatography on silica gel (eluent: CH₂Cl₂/MeOH 100/0 to 97/3) to give the desired title compound. TLC, Rf (CH₂Cl₂/MeOH 90/10) = 0.65. MS (LC-MS): 357 [M+H]⁺

### F. (4-Chloro-phenyl)-((3S*,4R*)-1,4-dibenzyl-pyrrolidin-3-ylmethyl)-phenyl-amine

A dry three-necked flask, equipped with a magnetic stirring bar, septum, and condenser with argon inlet-outlet, is charged with [Pd(µ-Br)(t-Bu₃P)]₂ (17.1 mg, 0.022 mmol), sodium tert-butoxyde (64 mg, 0.665 mmol), 1-bromo-4-chlorobenzene (102 mg, 0.532 mmol) and ((3R*,4R*)-1,4-bibenzyl-pyrrolidin-3-ylmethyl)-phenyl-amine (158 mg, 0.443 mmol). Dry de-aerated toluene (1 mL) is added. The mixture is stirred at RT for 15 min, and the reaction mixture is heated at 110 °C and stirred with gentle reflux for 17 h. The reaction mixture is cooled to RT, quenched with a saturated aqueous solution of NaHCO₃, extracted with AcOEt, dried over Na₂SO₄, filtered and concentrated. The crude mixture is purified by flash chromatography (eluent: c-hexane/AcOEt 90/10) to give the title product. TLC, Rf (c-hexane/AcOEt 2/1) = 0.45. MS (LC-MS): 467 [M+H]⁺

### G. (3R*,4R*)-3-Benzyl-4-{[(4-chloro-phenyl)-phenyl-amino]-methyl}-pyrrolidine-1-carboxylic acid ethyl ester

A mixture of (4-chloro-phenyl)-((3S*,4R*)-1,4-dibenzyl-pyrrolidin-3-ylmethyl)-phenyl-amine (33.2 mg, 0.071 mmol) and ethyl chloroformate (13.6 µL, 0.142 mmol) in toluene (1 mL) is heated at 90-100 °C overnight. The solution is concentrated under reduced pressure, the residue is diluted with CH₂Cl₂; and an aqueous saturated solution of NaHCO₃ is added. The layers are separated, and the aqueous one is extracted twice with CH₂Cl₂. The combined organic extracts are dried over Na₂SO₄, filtered and concentrated. The crude product is purified by flash chromatography on silica gel (eluent: c-hexane/AcOEt 95/5) to give the title product. TLC, Rf (c-hexane/AcOEt 2/1) = 0.7.

### H. ((3S*,4R*)-4-Benzyl-pyrrolidin-3-ylmethyl)-(4-chloro-phenyl)-phenyl-amine

A suspension of (3R*,4R*)-3-benzyl-4-{[(4-chloro-phenyl)-phenyl-amino]-methyl}-pyrrolidine-1-carboxylic acid ethyl ester (38.9 mg, 0.087 mmol) and KOH 50 % (1 mL) in EtOH (1.00 mL) is heated at 110°C over for 2 days in a closed vial. The reaction mixture is allowed to cool to RT and concentrated. AcOEt and water are added, the layers are separated, and the aqueous one is extracted twice with AcOEt. The combined organic layers are dried over Na₂SO₄, filtered and concentrated. The crude material is purified by flash chromatography on silica gel (eluent: CH₂Cl₂/MeOH 90/10 to 90/10 + 3 % NH₄OH) to give the title product. MS (LC-MS): 377, 379 [M+H]⁺; t_{R} (HPLC, Nucleosil C18 column, 10 to 90% CH₃CN in H₂O in 11 min, CH₃CN and H₂O containing 0.1% TFA, flow: 1.5 mL/min): 5.89 min.

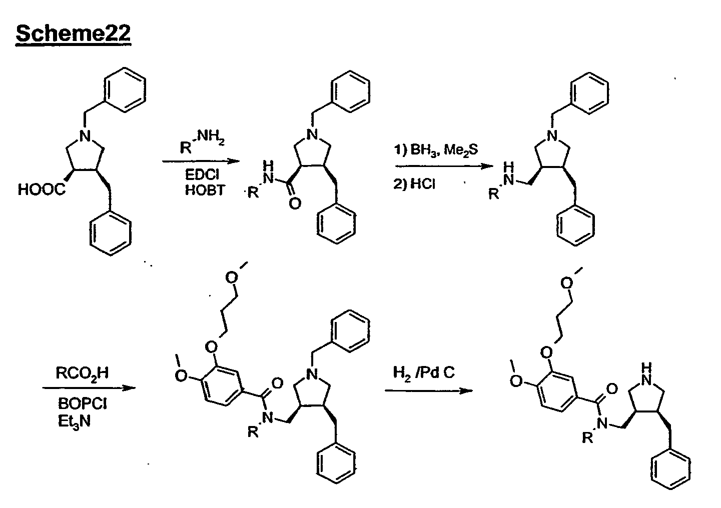

### Example 119: N-((3R*,4S*)-4-Benzyl-pyrrolidin-3-ylmethyl)-N-isopropyl-4-methoxy-3-(3-methoxy-propoxy)-benzamide

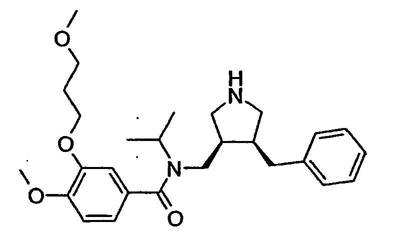

### A. (3R*,45*)-1,4-Dibenzyl-pyrrolidine-3-carboxylic acid isopropylamide

(3S*,4R*)-1,4-Dibenzyl-pyrrolidine-3-carboxylic acid (prepared according to Scheme21 product C) (3.92 mmol), HOBT (690 mg, 5.10 mmol), EDCI (978 mg, 5.10 mmol) are suspended in 60 mL dry CH₂Cl₂ under nitrogen and stirred 25 min, prior to the addition of isopropylamine (1.10 mL, 11.77 mmol). The resulting mixture is further stirred at RT overnight. EDCI (752 mg, 3.925 mmol), HOBT (530 mg, 3.925 mmol) and isopropylamine (674 µL, 7.85 mmol) are added to complete the reaction. The reaction mixture is filtered, concentrated and the residue is dissolved in AcOEt and acidified with an aqueous solution of HCl 0.5 N. the layers are separated and the organic phase neutralized with a saturated aqueous solution of NaHCO₃, dried over Na₂SO₄, filtered and concentrated. The crude material is purified by flash chromatography (CH₂Cl₂/MeOH 95/5) to give the title product. MS (LC-MS): 337.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mUmin): 4.47 min.

### B. ((3S*,4S*)-1,4-Dibenzyl-pyrrolidin-3-ylmethyl)-isopropyl-amine

To a solution of (3R*,4S*)-1,4-dibenzyl-pyrrolidine-3-carboxylic acid isopropylamide (732 mg, 2.17 mmol) in THF (8 mL), SH₃·Me₂S (2N in THF, 6.53 mL, 13.06 mmol) is added. The reaction mixture is refluxed overnight, and concentrated. The residual oil is taken up in MeOH (4 mL) and HCl cc (4 mL), refluxed overnight and poured into an aqueous solution of NaOH (15%). CH₂Cl₂ is added and the organic layer is separated. The resulting aqueous layer is extracted twice with CH₂Cl₂, and the combined organic layers are dried over anhydrous sodium sulfate, filtered and concentrated under reduced pressure. The resulting crude material is purified by flash chromatography on silica gel (eluent: CH₂Cl₂/MeOH 100/0 to 90/10) to give the title compound. MS (LC-MS): 323.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 3.77 min.

### C. N-((3R*,4S*)-1,4-Dibenzyl-pyrrolidin-3-ylmethyl)-N-isopropyl-4-methoxy-3-(3-methoxy-propoxy)-benzamide

A mixture of ((3S*,4S*)-1,4-dibenzyl-pyrrolidin-3-ylmethyl)-isopropyl-amine (0.25 g, 0.77 mmol), 3-(3-methoxy-propoxy)-4-methoxy-benzoic acid (0.18 g, 0.76 mmol), bis(2-oxo-3-oxazolidinyl)phosphinic chloride (0.22 g, 0.85 mmol) and triethylamine (0.43 mL, 3.1 mmol) in CH₂Cl₂ (110 mL) is refluxed for 1 h and then quenched by the addition of aqueous NaHCO₃ solution. The organic layer is separated, and the aqueous phase is extracted 3 times with CH₂Cl₂. The combined organic extracts are dried (Na₂SO₄), and the solvent is removed *in vacuo*. The crude product is purified by preparative HPLC (C18-ODB-AQ 5 µ, 20x50 mm, eluent: CH₃CN 5 to 100% /H₂O + 0.1 % HCOOH, 20 mL/min). The HPLC tubes are collected and diluted with AcOEt, washed with a saturated aqueous solution of NaHCO₃. The organic layer is dried over Na₂SO₄, filtered and concentrated to give the title product. TLC, Rf (CH₂Cl₂/MeOH 95/5 ) = 0.12. t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.90 min.

### D. N-((3R*,4S*)-4-Benzyl-pyrrolidin-3-ylmethyl)-N-isopropyl-4-methoxy-3-(3-methoxy-propoxy)-benzamide

A mixture of N-((3R*,4S*)-1,4-dibenzyl-pyrrolidin-3-ylmethyl)-N-isopropyl-4-methoxy-3-(3-methoxy-propoxy)-benzamide (0.092 g, 0.17 mmol)) and Pd(OH)₂ (20%/C) (0.02 g, 50% wet) in MeOH (10 mL) is stirred under a hydrogen atmosphere for 3 h. After completion of the reaction, the crude material is filtered over a pad of Celite, dried over Na₂SO₄ and concentrated. Flash chromatography on Isolute SPE Flash NH₂ column (eluent: CH₂Cl₂, /MeOH 99/1 to 90/10+ 1 % of NH₄OH) gives the title compound. Addition of 5.6 µl of 4N HCl/dioxane (0.022 mmol) to a solution of the product in dioxane (3 mL) and lyophilization yields the corresponding hydrochloride salt. MS (LC-MS): 455.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.67 min.

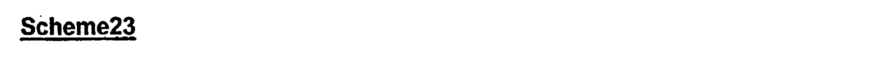

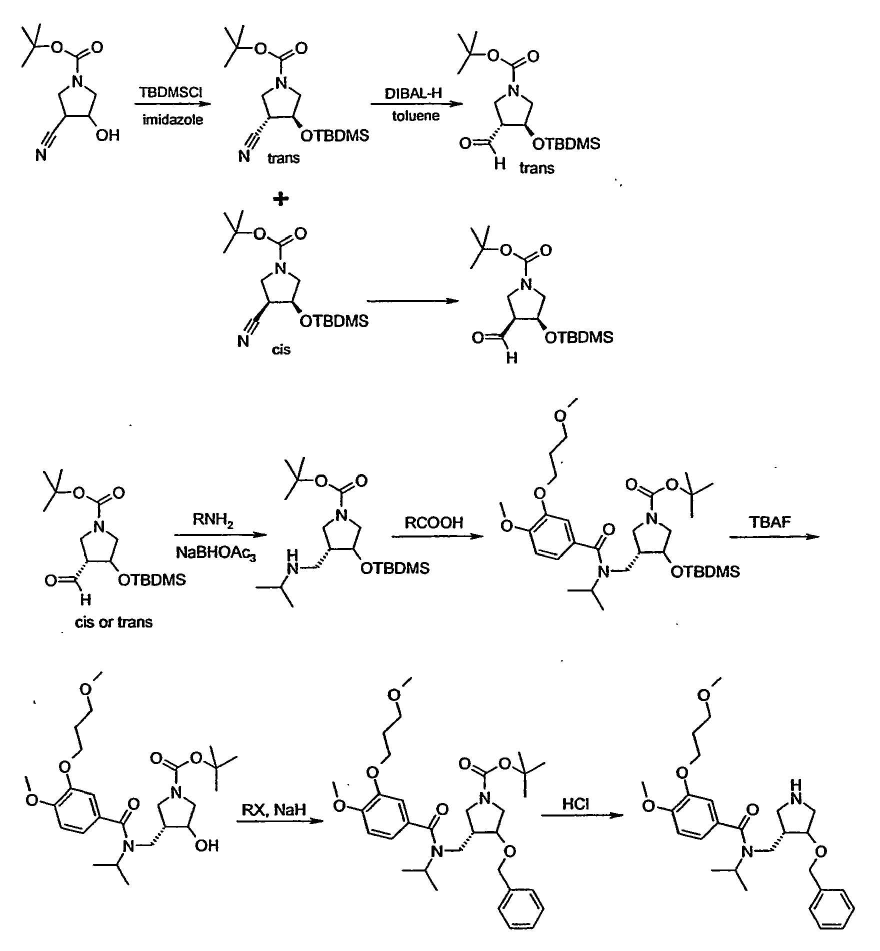

### Example 120: N-((3S*,4S*)-4-Benzyloxy-pyrrolidin-3-ylmethyl)-N-isopropyl-4-methoxy-3-(3-methoxy-propoxy)-benzamide

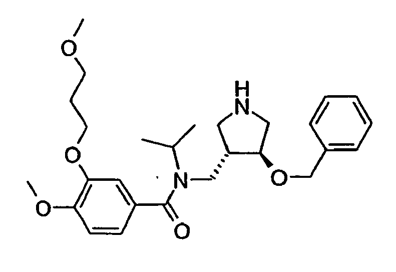

### A. (3S*,4S*)-3-(tert-Butyl-dimethyl-silanyloxy)-4-cyano-pyrrolidine-1-carboxylic acid tert-butyl ester and (3S*,4R*)-3-(tert-Butyl-dimethyl-silanyloxy)-4-cyano-pyrrolidine-1-carboxylic acid tert-butyl ester

A solution of 3-cyano-4-hydroxy-pyrrolidine-1-carboxylic acid tert-butyl ester (90.1 g, 0.43 mol) [see J. Med. Chem. 1997, 40, 3584], imidazole (37.5 g, 0.55 mol) and TBDMS-CI (70.3 g, 0.47 mol) in DMF (250 mL) is stirred at RT during 14 h. For workup, 10% citric acid is added, and the mixture is extracted with ethyl acetate. The combined organic extracts are washed with a saturated solution of NaHCO₃ and brine and dried (Na₂SO₄). Evaporation of the solvent affords the crude product. Flash column chromatography (hexane/ethyl acetate 9:1 → 1:1) thereof yields the desired trans-configured product as a colorless oil as well as the corresponding cis-configured product as a colorless solid.
Trans: (3S*,4S*)-3-(tert-Butyl-dimethyl-silanyloxy)-4-cyano-pyrrolidine-1-carboxylic acid tert-butyl ester: MS (LC-MS): 349.2 [M+Na]⁺; ¹H NMR (CDCl₃, 400 MHz): δ = 0.15 (s, 3H), 0.20 (s, 3H), 0.95 (s, 9H), s, 1.50 (s, 9H), 2.90-2.95 (m, 1H), 3.20-3.25 (m, 1H), 3.60-3.85 (m, 3H), 4.50-4.60 (m, 1H).
Cis: (3S*,4R*)-3-(tert-Butyl-dimethyl-silanyloxy)-4-cyano-pyrrolidine-1-carboxylic acid tert-butyl ester: MS (LC-MS): 271.0 [M+H-tBu]⁺; ¹H NMR (CDCl₃, 400 MHz): δ = 0.15 (s, 3H), 0.20 (s, 3H), 0.95 (s, 9H), s, 1.50 (s, 9H), 3.00-3.05 (m, 1H), 3.35-3.50 (m, 2H), 3.60-3.75 (m, 1H), 3.75-3.85 (m, 1H), 4.50-4.55 (m, 1H).

The two enantiomers of trans-configurated racemic (3S*,4S*)-3-(tert-butyl-dimethylsilanyloxy)-4-cyano-pyrrolidine-1-carboxylic acid tert-butyl ester were obtained by the following procedure.
(3S*,4S*)-3-(tert-Butyl-dimethyl-silanyloxy)-4-cyano-pyrrolidine-1-carboxylic acid tert-butyl ester is silyl-deprotected using TBAF as described under E in Example 120 (Scheme23), and the resulting racemic trans-configurated product (3S*,4S*)-3-cyano-4-hydroxy-pyrrolidine-1-carboxylic acid tert-butyl ester is subjected to separation of the enantiomers by preparative HPLC (Chiralpak AD (20 µm), 5×50cm, hexane/EtOH 85:15, flow: 80 mL/min):
(3R,4R)-3-Cyano-4-hydroxy-pyrrolidine-1-carboxylic acid tert-butyl ester: t_{R} (HPLC, Chiralcel AD column, hexane/EtOH 85:15, 95, flow: 1.0 mL/min): 6.89 min.
(3S,4S)-3-Cyano-4-hydroxy-pyrrolidine-1-carboxylic acid tert-butyl ester: t_{R} (HPLC, Chiralcel AD column, hexane/EtOH 85:15, 95, flow: 1.0 mL/min): 9.94 min.

The pure enantiomers then are silyl-reprotected as described under A in Example 120 (Scheme23).

### B. (3S*,4R*)-3-(tert-Butyl-dimethyl-silanyloxy)-4-formyl-pyrrolidine-1-carboxylic acid tert-butyl ester

At -78°C DIBAL-H (16.7 mL, 20 mmol, 1.2M solution in toluene) is slowly added to a solution of (3S*,4S*)-3-(tert-butyl-dimethyl-silanyloxy)-4-cyano-pyrrolidine-1-carboxylic acid tert-butyl ester (3.3 g, 10 mmol) in toluene (35 mL). After stirring at -78°C for 1 h, Rochelle salt is added and the mixture is allowed to warm to RT. Vigorous stirring of this mixture for 1 h followed by extraction with ethyl acetate, drying (Na₂SO₄) and evaporation of the solvent affords the desired product as a colorless oil which is directly used without further purification. MS (LC-MS): 230.1 [M+H-Boc]⁺; t_{R} (HPLC, C8 column, 20-95% CH₃CN/H₂O/3.5 min, 95% CH₃CN/1 min, CH₃CN and H₂O containing 0.1% TFA, flow: 0.8 mUmin): 2.97 min.

### (3S*,4S*)-3-(tert-Butyl-dimethyl-silanyloxy)-4-formyl-pyrrolidine-1-carboxylic acid tert-butyl ester

The title compound is prepared according to the same procedure as described above for the synthesis of the trans analog. TLC, Rf (toluene/AcOEt 4/1) = 0.28. MS (LC-MS): 330.2 [M+H]⁺

### C. (3S*,4R*)-3-(tert-Butyl-dimethyl-silanyloxy)-4-(isopropylamino-methyl)-pyrrolidine-1-carboxylic acid tert-butyl ester

The title compound is prepared according to the procedure described for the synthesis of the title compound under A in Example 56 (Scheme11).

### D. (3S*,4R*)-3-(tert-Butyl-dimethyl-silanyloxy)-4-({isopropyl-[4-methoxy-3-(3-methoxy-propoxy)-benzoyl]-amino}-methyl)-pyrrolidine-1-carboxylic acid tert-butyl ester

The title compound is prepared according to the procedure described for the synthesis of the title compound under B in Example 56 (Scheme11a)

### E. (3S*,4R*)-3-Hydroxy-4-({isopropyl-[4-methoxy-3-(3-methoxy-propoxy)-benzoyl]-amino}-methyl)-pyrrolidine-1-carboxylic acid tert-butyl ester

TBAF3 H₂O (2.64 g, 8.4 mmol) is added to a solution of (3S*,4R*)-3-(tert-butyl-dimethyl-silanyloxy)-4-({isopropyl-[4-methoxy-3-(3-methoxy-propoxy)-benzoyl]-amino}-methyl)-pyrrolidine-1-carboxylic acid tert-butyl ester (1.66 g, 2.8 mmol) in THF (30 mL) at RT. After stirring at RT for 45 min, H₂O is added, and the mixture is extracted with ethyl acetate. The combined organic extracts are dried (Na₂SO₄) and the solvent is evaporated. Purification of the crude product by flash column chromatography (ethyl acetate) affords the desired product as a colorless oil. MS (LC-MS): 481.3 [M+H]⁺; t_{R} (HPLC, C18 column, 35-100% CH₃CN/H₂O/15 min, CH₃CN and H₂O containing 0.1 % TFA, flow: 1.5 mL/min): 4.90 min

### F. (3S*,4R*)-3-Benzyloxy-4-({isopropyl-[4-methoxy-3-(3-methoxy-propoxy)-benzoyl]-amino}-methyl)-pyrrolidine-1-carboxylic acid tert-butyl ester

NaH (18 mg, 0.44 mmol, 60% in mineral oil) is added to a solution of (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester (130 mg, 0.27 mmol) in DMF (1 mL). Stirring of the resulting suspension at 80°C for 15 min, then cooling to RT and addition of benzyl bromide (59 µl, 0.54 mmol) follow, then stirring at RT for another 24h. H₂O is added, and the mixture is extracted with ethyl acetate. The organic extracts are dried (Na₂SO₄), and the solvent is evaporated to give the crude product. The desired product in pure form (105 mg) is obtained by flash column chromatography (ethyl acetate/hexane 1:1 → 7:3). MS (LC-MS): 571.2 [M+H]⁺; t_{R} (HPLC, C18 column, 65-100% CH₃CN/H₂O/15 min, CH₃CN and H₂O containing 0.1 % TFA, flow: 1.5 mL/min): 3.44 min.

### G. N-((3S*,4S*)-4-Benzyloxy-pyrrolidin-3-ylmethyl)-N-isopropyl-4-methoxy-3-(3-methoxy-propoxy)-benzamide

This compound is obtained as mono-hydrochloride (60 mg) in analogy to the title compound under B in Example 101 (Scheme13) by N-Boc-deprotection of (3S*,4R*)-3-benzyloxy-4-({isopropyl-[4-methoxy-3-(3-methoxy-propoxy)-benzoyl]-amino}-methyl)-pyrrolidine-1-carboxylic acid tert-butyl ester (95 mg) with 4N HCl/dioxane (3 mL). MS (LC-MS): 471.5 [M+H]⁺; t_{R} (HPLC, C18 column, 20-100% CH₃CN/H₂O/15 min, CH₃CN and H₂O containing 0.1% TFA, flow: 1.5 mL/min): 7.10 min.

### Example 121: N-((3S*,4R*)-4-Benzyloxy-pyrrolidin-3-ylmethyl)-N-isopropyl-4-methoxy-3-(3-methoxy-propoxy)-benzamide

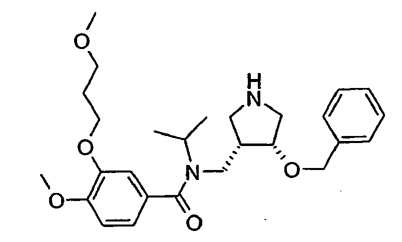

The title compound is prepared analogously as described for the title compound under G in Example 120 (Scheme23) from (3S*,4R*)-3-(tert-Butyl-dimethyl-silanyloxy)-4-cyanopyrrolidine-1-carboxylic acid tert-butyl ester. MS (LC-MS): 471.1 [M+H]⁺; t_{R} (HPLC, C18 column, 50-100% CH₃CN/H₂O/15 min, CH₃CN and H₂O containing 0.1% TFA, flow: 1.5 mL/min): 7.42 min.

### Example 122: N-((3S*,4S*)-4-Hydroxy-pyrrolidin-3-ylmethyl)-N-isopropyl-4-methoxy-3-(3-methoxy-propoxy)-benzamide

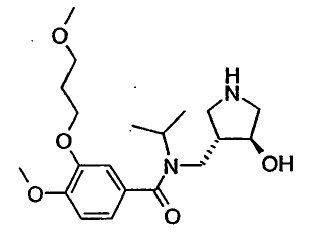

The title compound is prepared by N-deprotection of (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester according to the procedure described for the synthesis of the title compound under G in Example 120 (Scheme23). MS (LC-MS): 381.2 [M+H]⁺; t_{R} (HPLC, C18 column, 10-100% CH₃CN/H₂O/15 min, CH₃CN and H₂O containing 0.1% TFA, flow: 1.5 mL/min): 6.17 min.

### Example 123: N-((3S*,4R*)-4-Hydroxy-pyrrolidin-3-ylmethyl)-N-isopropyl-4-methoxy-3-(3-methoxy-propoxy)-benzamide

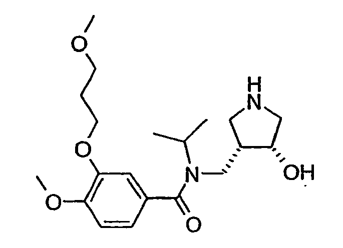

The title compound is prepared by N-deprotection of (3R*,4R*)-3-hydroxy-4-({isopropyl-[4-methoxy-3-(3-methoxy-propoxy)-benzoyl]-amino}-methyl)-pyrrolidine-1-carboxylic acid tert-butyl ester according to the procedure described for the synthesis of the title compound under G in Example 120 (Scheme23). MS (LC-MS): 381.2 [M+H]⁺; t_{R} (HPLC, C18 column, 10-100% CH₃CN/H₂O/15 min, CH₃CN and H₂O containing 0.1% TFA, flow: 1.5 mL/min): 6.48 min.

### Example 124: N-lsopropyl-4-methoxy-N-[(3S*,4S*)-4-(3-methoxy-benzyloxy)-pyrrolidin-3-ylmethyl]-3-(3-methoxy-propoxy)-benzamide

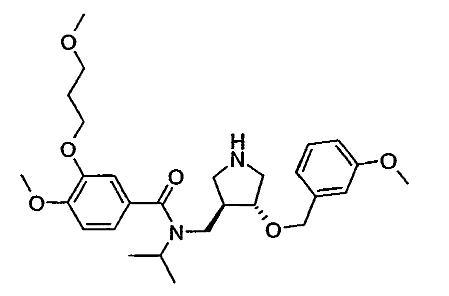

The title compound is prepared analogously as described for the title compound under G in Example 120 (Scheme23) (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-bromomethyl-3-methoxy-benzene. MS (LC-MS): 501.1 [M+H]⁺, t_{R} (HPLC, C18 column, 10-100% CH₃CN/H₂O/5 min, 100% CH₃CN/3 min, 100-10% CH₃CN/H₂O/3 min, CH3CN and H2O containing 0.1% TFA, CH3CN and H2O containing 0.1% TFA, flow: 1.5 mL/min): 4.49 min

### Example 125: N-Isopropyl-4-methoxy-3-(3-methoxy-propoxy)-N-[(3S*,4S*)-4-(naphthalen-2 -ylmethoxy)pyrrolidin-3-ylmethyl]-benzamide

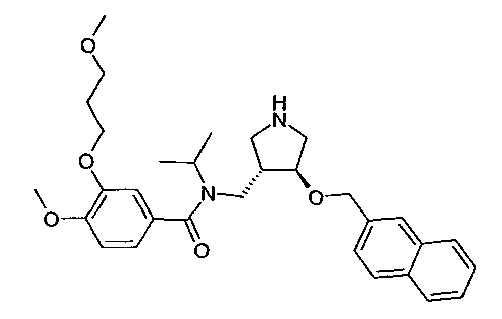

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-bromomethyl-naphthalene. MS (LC-MS): 521.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, CH₃CN and H₂O containing 0.1% TFA, flow: 1.5 mL/min): 4.79 min.

### Example 126: N-[(3S*,4S*)-4-(3,5-Dimethoxy-benzyloxy)-pyrrolidin-3-ylmethyl]-N-isopropyl-4-methoxy-3-(3-methoxy-propoxy)-benzamide

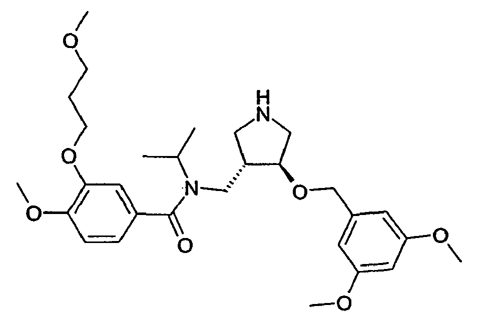

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-bromomethyl-3,5-dimethoxy-benzene. MS (LC-MS): 531.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.49 min.

### Example 127: N-[(3S*,4S*)-4-(3-Ethoxy-benzyloxy)-Pyrrolidin-3-ylmethyl]-N-isopropyl-4-methoxy-3-(3-methoxy-propoxy)-benzamide

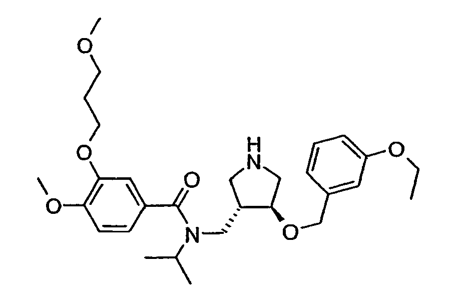

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-bromomethyl-3-ethoxy-benzene. 1-Bromomethyl-3-ethoxy-benzene was obtained from (3-ethoxy-phenyl)-methanol by reaction with CBr₄/PPh₃ according to a procedure described in Chem. Eur. J. **2000,** *6*, 3692. MS (LC-MS): 515.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.62 min.

### Example 128: N-[(3S*,4S*)-4-(3-Isoyroyoxy-benzyloxy)-pyrrolidin-3-ylmethyl]-N-isoyropyl-4-methoxy-3-(3-methoxy-propoxy)-benzamide

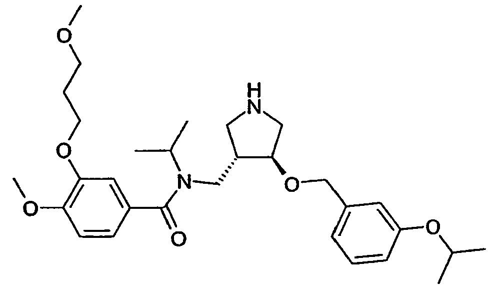

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-bromomethyl-3-isopropoxy-benzene. 1-Bromomethyl-3-isopropoxy-benzene was obtained from (3-isopropoxy-phenyl)-methanol by reaction with CBr₄/PPh₃ according to a procedure described in Chem. Eur. J. **2000**, *6*, 3692. (3-Isopropoxy-phenyl)-methanol was prepared by reaction of 3-hydroxymethyl phenol with 2-iodopropane in the presence of K₂CO₃ and 18-crown-6 according to a procedure described in Chem. Eur. J. 2000, 6, 3692. MS (LC-MS): 529.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.78 min.

### Example 129: N-[(3S*,4S*)-4-(3-Cyano-benzyloxy)-pyrrolidin-3-ylmethyl]-N-isopropyl-4-methoxy-3-(3-methoxy-propoxy)-benzamide

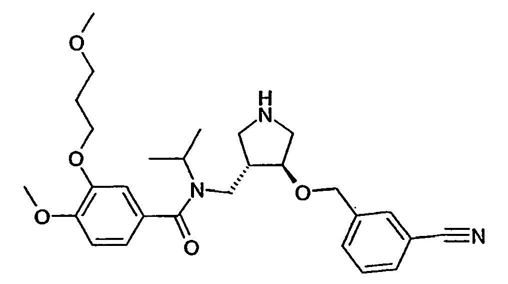

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3-bromomethyl-benzonitrile. MS (LC-MS): 496.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.41 min.

### Example 130: N-Isopropyl-4-methoxy-3-(3-methoxy-propoxy)-N-[(3S*,4S*)-4-(3-trifluoromethoxy-benzyloxy)-pyrrolidin-3-ylmethyl]-benzamide

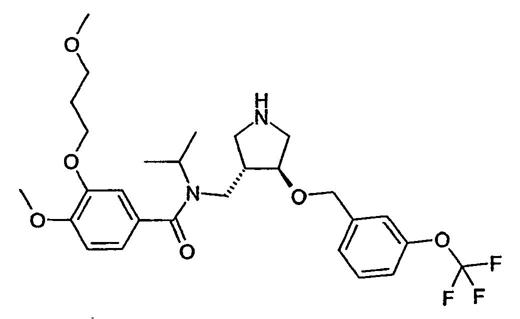

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3-trifluoromethoxy-benzylbromide. MS (LC-MS): 554.9 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.90 min.

### Example 131: N-Isopropyl-4-methoxy-3-(3-methoxy-propoxy)-N-[(3S*,4S*)-4-(3-propoxy-benzyloxy)-pyrrolidin-3-ylmethyl]-benzamide

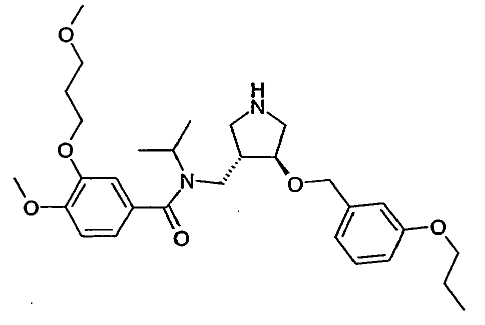

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-bromomethyl-3-propoxy-benzene. 1-Bromomethyl-3-propoxy-benzene was obtained from (3-propoxy-phenyl)-methanol by reaction with CBr₄/PPh₃ according to a procedure described in Chem. Eur. J. **2000**, *6*, 3692. 3-Isopropoxybenzyl alcohol was prepared by reaction of 3-hydroxymethyl phenol with propyl iodide in the presence of K₂CO₃ and 18-crown-6 according to a procedure described in Chem. Eur. J. 2000, 6, 3692. MS (LC-MS): 529.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 4.89 min.

### Example 132: N-[(3S*,4S*)-4-(Biphenyl-3-ylmethoxy)-pyrrolidin-3-ylmethyl]-N-isopropyl-4-methoxy-3-(3-methoxy-propoxy)-benzamide

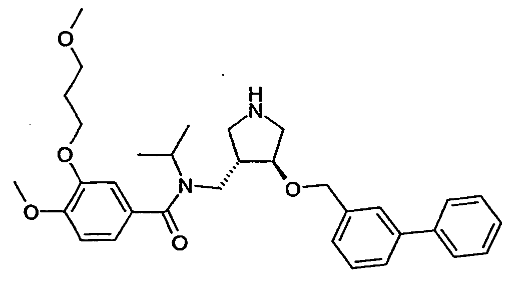

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3-bromomethyl-biphenyl. MS (LC-MS): 546.9 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.05 min.

### Example 133: N-Isopropyl-4-methoxy-3-(3-methoxy-propoxy)-N-[(3S*,4S*)-4-(3-phenoxy-benzyloxy)-pyrrolidin-3-ylmethyl]-benzamide

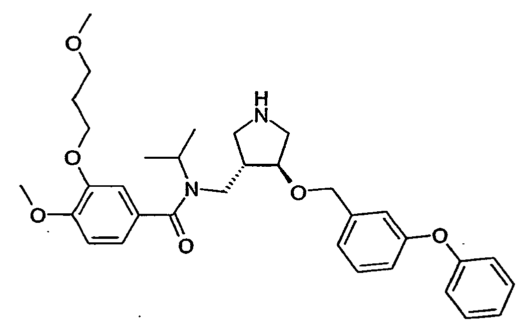

The title compound is prepared analogously as described for the title compound under G in Example 120 (Scheme23) from from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3-phenoxybenzyl chloride. MS (LC-MS): 563.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.06 min.

### Example 134: N-[(3S*,4S*)-4-(3,5-Diethoxy-benzyloxy)-pyrrolidin-3-ylmethyl]-N-isopropyl-4-methoxy-3-(3-methoxy-propoxy)-benzamide

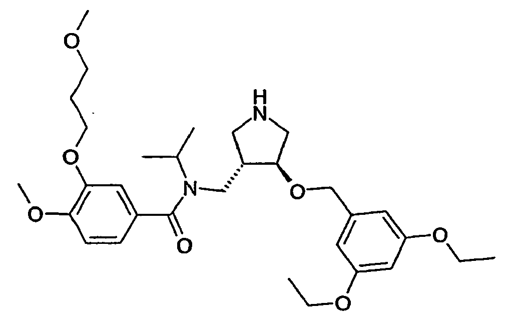

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-bromomethyl-3,5-diethoxy-benzene. 1-Bromomethyl-3,5-diethoxy-benzene was obtained from (3,5-diethoxy-phenyl)-methanol by reaction with CBr₄/PPh₃ according to a procedure described in Chem. Eur. J. 2000, 6, 3692. MS (LC-MS): 559.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 4.86 min.

### Example 135: N-Isopropyl-4-methoxy-3-(3-methoxy-propoxy)-N-[(3S*,4S*)-4-(5,5,8,8-tetr amethyl-5,6,7,8-tetrahydro-naphthalen-2-ylmethoxy)-pyrrolidin-3-ylmethyl]-benzamide

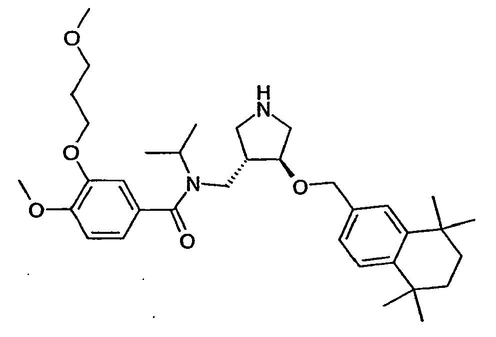

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 6-(bromomethyl)-1,1,4,4-tetramethyl-1,2,3,4-tetrahydronaphtalene. MS (LC-MS): 581.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.67 min.

### Example 136: N-[(3S*,4S*)-4-(3,4-Dihydro-2H-benzo[b][1,4]dioxepin-7-ylmethoxy)-pyrrolidin-3-ylmethyl]-N-isopropyl-4-methoxy-3-(3-methoxy-propoxy)-benzamide

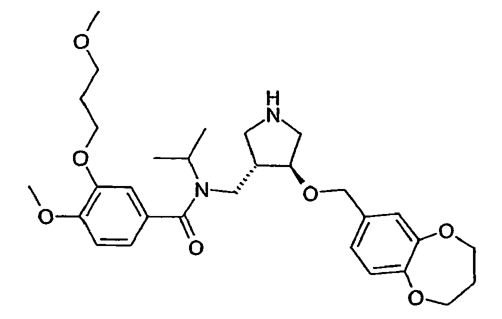

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 7-(chloromethyl)-3,4-dihydro-2H-1,5-benzodioxepine. MS (LC-MS): 543.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.51 min.

### Example 137: N-[(3S*,4S*)-4-(7-Cyano-naphthalen-2-ylmethoxy)-pyrrolidin-3-ylmethyl]-N -isopropyl-4-methoxy-3-(3-methoxy-propoxy)-benzamide

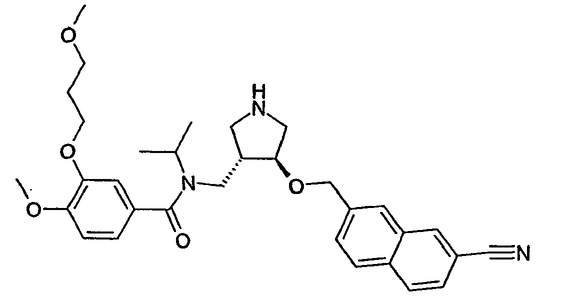

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 7-bromomethyl-naphthalene-2-carbonitrile. 7-Bromomethyl-naphthalene-2-carbonitrile was prepared according to J. Med. Chem. 1991, 34, 3105. MS (LC-MS): 546.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.68 min.

### Example 138: N-[(3S*,4S*)4-(2,3-Dimethoxy-benzyloxy)-pyrrolidin-3-ylmethyl]-N-isopropyl-4-methoxy-3-(3-methoxy-propoxy)-benzamide

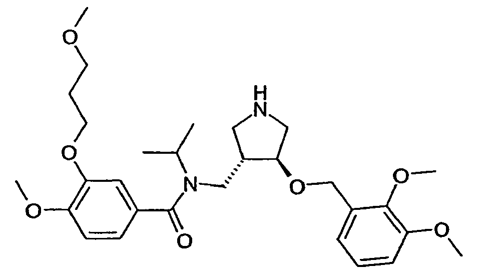

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-chloromethyl-2,3-dimethoxy-benzene. MS (LC-MS): 531.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.52 min.

### Example 139: N-[(3S*,4S*)-4-(3-Benzyloxy-benzyloxy)-pyrrolidin-3-ylmethyl]-N-isopropyl-4-methoxy-3-(3-methoxy-propoxy)-benzamide

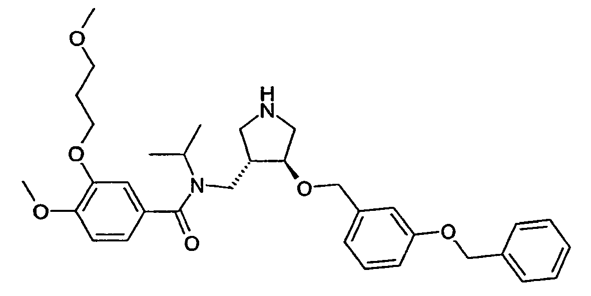

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-benzyloxy-3-bromomethyl-benzene. MS (LC-MS): 577.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.05 min.

### Example 140: N-Isopropyl-4-methoxy-3-(3-methoxy-propoxy)-N-[(3S*,4S*)-4-(3-pyrrol-1-yl-benzyloxy)-pyrrolidin-3-ylmethyl]-benzamide

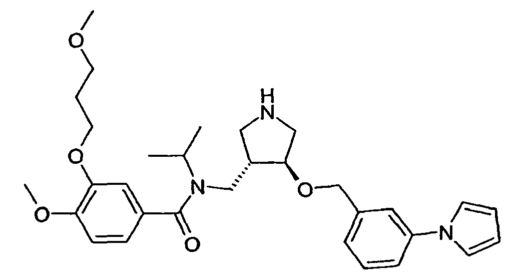

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-[3-(bromomethyl)phenyl]-1H-pyrrole. MS (LC-MS): 536.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 4.83 min.

### Example 141: N-[(3S*,4S*)-4-(Benzofuran-5-ylmethoxy)-pyrrolidin-3-ylmethyl]-N-isopropyl-4-methoxy-3-(3-methoxy-propoxy)-benzamide

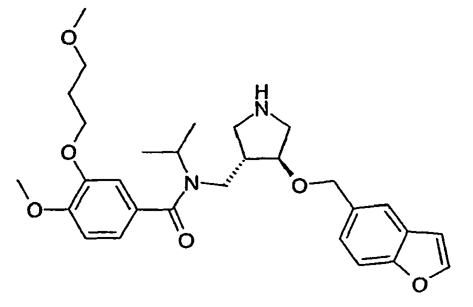

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 5-bromomethyl-benzofuran. 5-Bromomethyl-benzofuran was obtained from benzofuran-5-yl-methanol by reaction with CBr₄/PPh₃ according to a procedure described in Chem. Eur. J. 2000, 6, 3692. MS (LC-MS): 511.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.60 min.

### Example 142: N-[(3S*,4S*)-4-(2,3-Dihydro-benzo[1,4]dioxin-5-ylmethoxy)-pyrrolidin-3-ylmethyl]-N-isopropyl-4-methoxy-3-(3-methoxy-propoxy)-benzamide

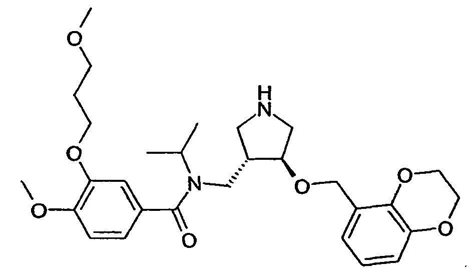

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 5-bromomethyl-2,3-dihydro-benzo[1,4]dioxine. 5-Bromomethyl-2,3-dihydro-benzo[1,4]dioxine was obtained from (2,3-dihydro-benzo[1,4]dioxin-5-yl)-methanol by reaction with CBr₄/PPh₃ according to a procedure described in Chem. Eur. J. 2000, 6, 3692. MS (LC-MS): 529.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.50 min.

### Example 143: N-[(3S*,4S*)-4-(3,4-Dimethyl-benzyloxy)-pyrrolidin-3-ylmethyl]-N-isopropyl-4-methoxy-3-(3-methoxy-propoxy)-benzamide

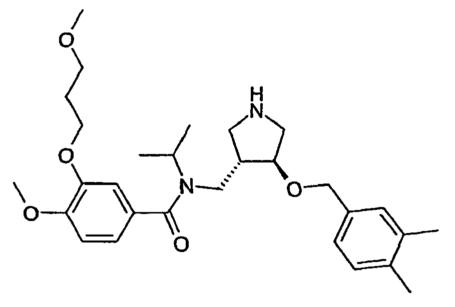

The title compound is prepared analogously as described for the title compound under G in Example 120 (Scheme23) from (3S*,4R*)-3-hydroxy-4-({isopropyl-[4-methoxy-3-(3-methoxy propoxy)-benzoyl]-amino}-methyl)-pyrrolidine-1-carboxylic acid tert-butyl ester and 4-chloromethyl-1,2-dimethyl-benzene. MS (LC-MS): 499.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.77 min.

### Example 144: N-[(3S*,4S*)-4-(3,4-Dihydro-2H-benzo[b][1,4]dioxepin-6-ylmethoxy)-pyrrolidin-3-ylmethyl]-N-isopropyl-4-methoxy-3-(3-methoxy-propoxy)-benzamide

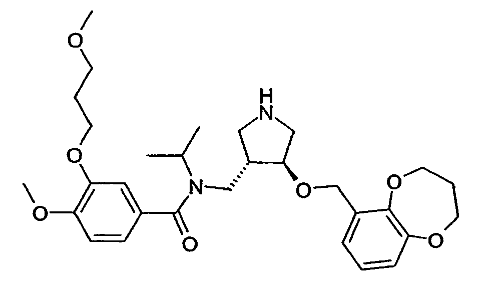

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 6-(bromomethyl)-3,4-dihydro-2H-1,5-benzodioxepine. MS (LC-MS): 543.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.55 min.

### Example 145: N-Isopropyl-4-methoxy-3-(3-methoxy-propoxy)-N-[(3S*,4S*)-4.-(naphthalen-1 -yimethoxy)-pyrrolidin-3-ylmethyl]-benzamide

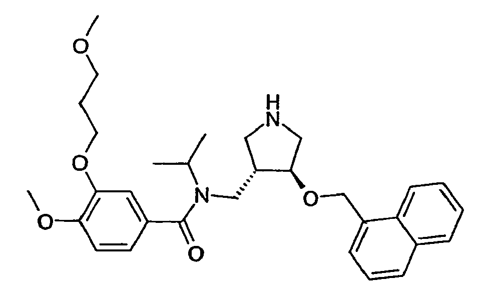

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-bromomethyl-naphthalene. 1-Bromomethyl-naphthalene was obtained from naphthalen-1-yl-methanol by reaction with CBr₄/PPh₃ according to a procedure described in Chem. Eur. J. 2000, 6, 3692. MS (LC-MS): 521.0 [M+H]+, tR (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.83 min.

### Example 146: N-Isopropyl-4-methoxy-N-{(3S*,4S*)-4-[3-(4-methoxy-phenoxy)-benzyloxy]-pyrrolidin-3-ylmethyl}-3-(3-methoxy-propoxy)-benzamide

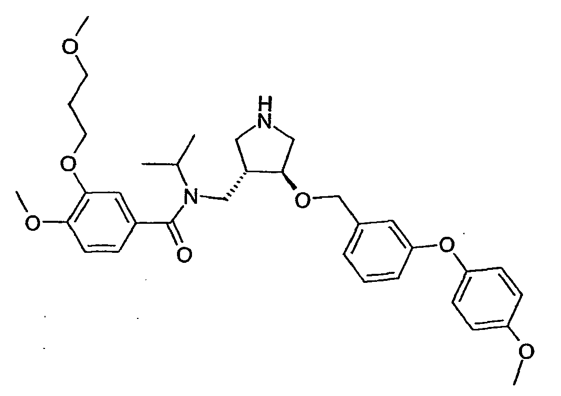

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and [3-(4-Methoxy-phenoxy)-phenyl]-chloromethane. MS (LC-MS): 593.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.05 min.

### Example 147: N-[(3S*,4S*)-4-(3-Benzo[1,3]dioxol-5-yl-benzyloxy)-pyrrolidin-3-ylmethyl]-N-isopropyl-4-methoxy-3-(3-methoxy-propoxy)-benzamide

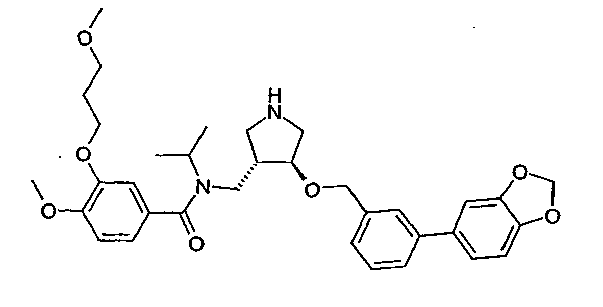

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrroidine-1-carboxylic acid tert-butyl ester and 5-(3-bromomethyl-phenyl)-benzo[1,3]dioxole. 5-(3-Bromomethyl-phenyl)-benzo[1,3] dioxole was obtained by Suzuki cross-coupling of (3-bromo-phenyl)-methanol with 3,4-methylenedioxyphenylboronic acid followed by reaction of the resulting product with CBr₄/PPh₃ according to procedures described in Chem. Eur. J. 2000, 6, 3692. MS (LC-MS): 591.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.98 min.

### Example 148: N-Isopropyl-4-methoxy-N-[(3S*,4S*)-4-(2'-methoxy-biphenyl-3-ylmethoxy)-pyrrolidin-3-ylmethyl]-3-(3-methoxy-propoxy)-benzamide

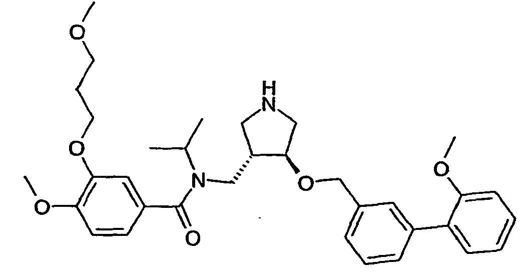

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3'-bromomethyl-2-methoxy-biphenyl. 3'-Bromomethyl-2-methoxy-biphenyl is obtained by Suzuki cross-coupling of (3-bromo-phenyl)-methanol with 2-methoxybenzeneboronic acid followed by reaction of the resulting product with CBr₄/PPh₃ according to procedures described in Chem. Eur. J. 2000, 6, 3692. MS (LC-MS): 577.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.06 min.

### Example 149: N-[(3S*,4S*)-4-(2',5'-Dimethoxy-biphenyl-3-ylmethoxy)-pyrrolidin-3-ylmethyl]-N-isopropyl-4-methoxy-3-(3-methoxy-propoxy)-benzamide

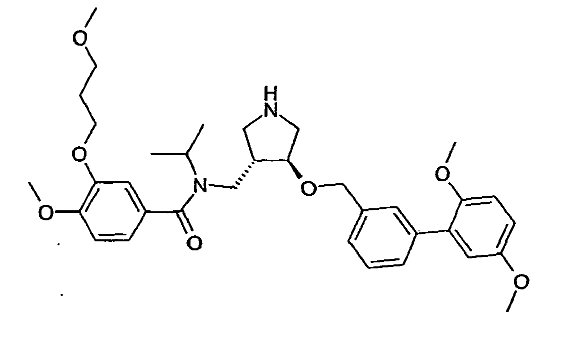

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3'-bromomethyl-2,5-dimethoxy-biphenyl. 3'-Bromomethyl-2,5-dimethoxy-biphenyl is obtained by Suzuki cross-coupling of (3-bromo-phenyl)-methanol with 2,5-dimethoxyphenylboronic acid followed by reaction of the resulting product with CBr₄/PPh₃ according to procedures described in Chem. Eur. J. 2000, 6, 3692. MS (LC-MS): 607.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.04 min.

### Example 150: N-Isopropyl-4-methoxy-3-(3-methoxy-aropoxy)-N-[(3S*.4S*)-4-(3'-methylbiphenyl-3-ylmethoxy)-pyrrolidin-3-ylmethyl]-benzamide

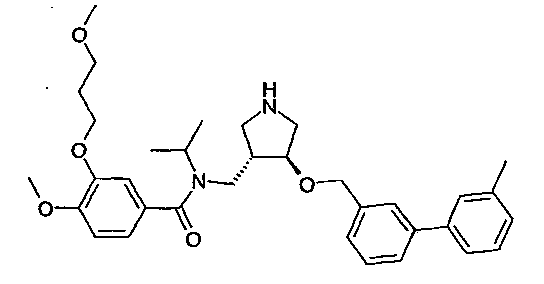

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3-bromomethyl-3'-methyl-biphenyl. 3-Bromomethyl-3'-methyl-biphenyl is obtained by Suzuki cross-coupling of (3-bromo-phenyl)-methanol with 3-methylphenylboronic acid followed by reaction of the resulting product with CBr₄/PPh₃ according to procedures described in Chem. Eur. J. 2000, 6, 3692. MS (LC-MS): 561.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.19 min.

### Example 151: N-[(3S*,4S*)-4-(3',4'-Dimethoxy-biphenyl-3-ylmethoxy)-pyrrolidin-3-ylmethyl]-N-isopropyl-4-methoxy-3-(3-methoxy-propoxy)-benzamide

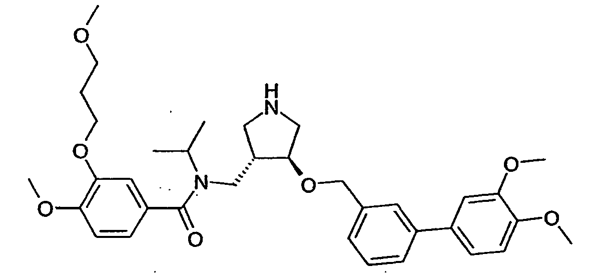

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3'-bromomethyl-3,4-dimethoxy-biphenyl. 3'-Bromomethyl-3,4-dimethoxy-biphenyl is obtained by Suzuki cross-coupling of (3-bromo-phenyl)-methanol with 3,4-dimethoxyphenylboronic acid followed by reaction of the resulting product with CBr₄/PPh₃ according to procedures described in Chem. Eur. J. 2000, 6, 3692. MS (LC-MS): 607.1 [M+H]⁺, t_{R} (HPLC, C18 column, 10-100% CH₃CNlH₂O/5 min, 100% CH₃CN/3 min, 100-10% CH₃CN/H₂O/3 min, CH₃CN and H₂O containing 0.1 % TFA, flow: 1.5 mL/min): 4.83 min.

### Example 152: N-[(3S*,4S*)-4-(2',5'-Dimethyl-biphenyl-3-ylmethoxy)-pyrrolidin-3-ylmethyl]-N-isopropyl-4-methoxy-3-(3-methoxy-propoxy)-benzamide

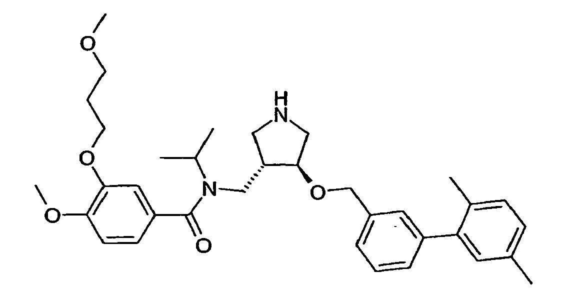

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrodidine-1-carboxylic acid tert-butyl ester and 3'-bromomethyl-2,5-dimethyl-biphenyl. 3'-Bromomethyl-2,5-dimethyl-biphenyl is obtained by Suzuki cross-coupling of (3-bromo-phenyl)-methanol with 2,5-dimethylbenzeneboronic acid followed by reaction of the resulting product with CBr₄/PPh₃ according to procedures described in Chem. Eur. J. 2000, 6, 3692. MS (LC-MS): 575.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.38 min.

### Example 153: N-[(3S*,4S*)-4-(2',3'-Dimethoxy-biphenyl-3-ylmethoxy)-pyrrolidin-3-ylmethyl]-N-isopropyl-4-methoxy-3-(3-methoxy-propoxy)-benzamide

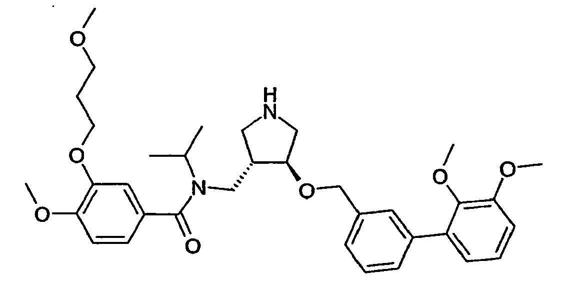

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3'-bromomethyl-2,3-dimethoxy-biphenyl. 3'-Bromomethyl-2,3-dimethoxy-biphenyl is obtained by Suzuki cross-coupling of (3-bromo-phenyl)-methanol with 2,3-dimethoxybenzeneboronic acid followed by reaction of the resulting product with CBr₄/PPh₃ according to procedures described in Chem. Eur. J. 2000, 6, 3692. MS (LC-MS): 607.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.01 min.

### Example 154: N-[(3S*,4S*)-4-(2'-Isopropoxy-biphenyl-3-ylmethoxy)-pyrrolidin-3-ylmethyl]-N-isopropyl-4-methoxy-3-(3-methoxy-propoxy)-benzamide

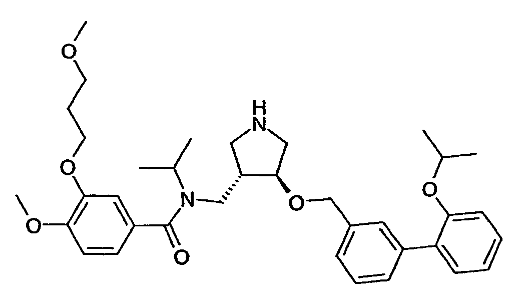

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3'-bromomethyl-2-isopropoxy-biphenyl. 3'-Bromomethyl-2-isopropoxy-biphenyl is obtained by Suzuki cross-coupling of (3-bromo-phenyl)-methanol with 2-isopropoxyphenylboronic acid followed by reaction of the resulting product with CBr₄/PPh₃ according to procedures described in Chem. Eur. J. 2000, 6, 3692. MS (LC-MS): 605.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.36 min.

### Example 155: N-Isopropyl-4-methoxy-N-[(3S*,4S*)-4-(2'-methoxy-5'-methyl-biphenyl-3-ylmethoxy)-pyrrolidin-3-ylmethyl]-3-(3-methoxy-propoxy)-benzamide

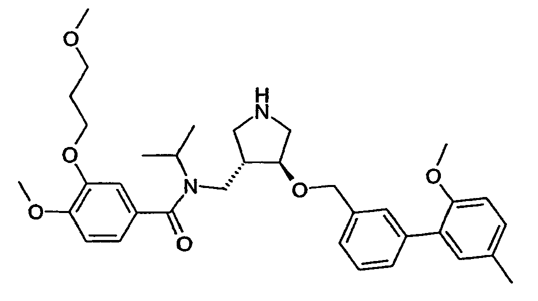

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3'-bromomethyl-2-methoxy-5-methyl-biphenyl 3'-Bromomethyl-2-methoxy-5-methyl-biphenyl is obtained by Suzuki cross-coupling of (3-bromo-phenyl)-methanol with 2-methoxy-5-methylphenylboronic acid followed by reaction of the resulting product with CBr₄/PPh₃ according to procedures described in Chem. Eur. J. 2000, 6, 3692. MS (LC-MS): 591.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.22 min.

### Example 156: N-Isopropyl-4-methoxy-3-(3-methoxy-propoxy)-N-[(3S*,4S*)-4-(3-phenyl-isoxazol-5-ylmethoxy)-pyrrolidin-3ylmethyl]-benzamide

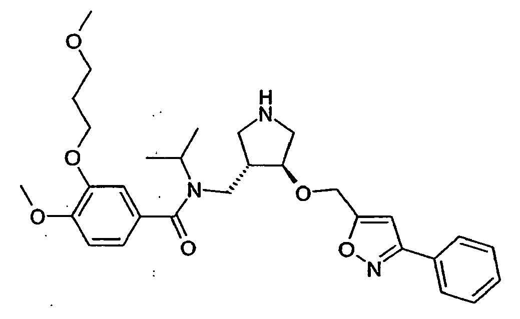

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 5-chloromethyl-3-phenyl-isoxazole. MS (LC-MS): 538.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.63 min.

### Example 157: N-{(3S*,4S*)-4-[3-(4-Chloro-phenyl)-isoxazol-5-ylmethoxy]-pyrrolidin-3-ylmethyl}-N-isopropyl-4-methoxy-3-(3-methoxy-propoxy)-benzamide

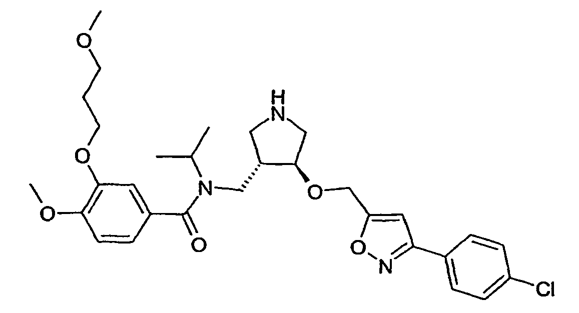

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-l-carboxylic acid tert-butyl ester and 5-chloromethyl-3-(4-chloro-phenyl)-isoxazole. MS (LC-MS): 571.9 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.90 min.

### Example 158: N-Isopropyl-4-methoxy-N-{(3S*,4S*)-4-[3-(4-methoxy-phenyl)-isoxazol-5-ylmethoxy]-pyrrolidin-3-ylmethyl}-3-(3-methoxy-propoxy)-benzamide

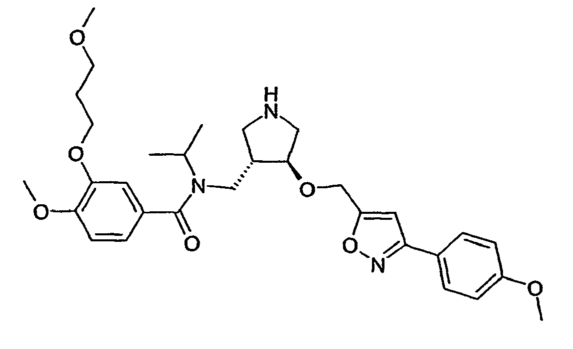

The title compound is prepared analogously as described for the title compound under G in Example 120 (Scheme23) from (3S*,4R*)-3-hydroxy-4-({Iisopropyl-[4-methoxy-3-(3-methoxy-propoxy)-benzoyl]-amino}-methyl)-pyrrolidine-1-carboxylic acid tert-butyl ester and 5-chloromethyl-3-(4-methoxy-phenyl)-isoxazole. MS (LC-MS): 568.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.70 min.

### Example 159: N-Isopropyl-4-methoxy-3-(3-methoxy-propoxy)-N-[(3S*,4S*)-4-(5-phenyl-[1,2,4]oxadiazol-3-ylmethoxy)-pyrrolidin-3-ylmethyl]-benzamide

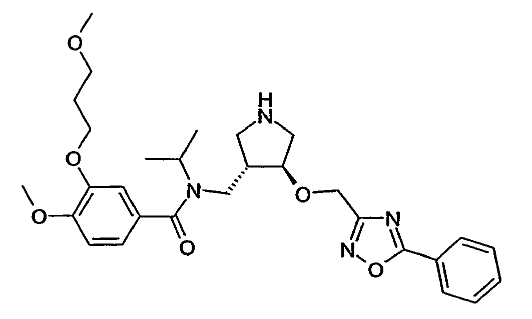

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3-chloromethyl-5-phenyl-[1,2,4]oxadiazole. MS (LC-MS): 539.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.65 min.

### Example 160: N-[(3S*,4S*)-4-(2'-Ethoxy-biphenyl-3-ylmethoxy)-pyrrolidin-3-ylmethyl]-N-isopropyl-4-methoxy-3-(3-methoxy-propoxy)-benzamide

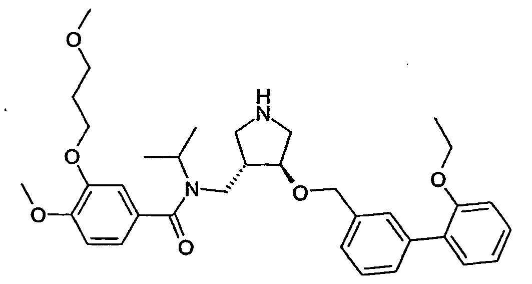

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3'-bromomethyl-2-ethoxy-biphenyl. 3'-Bromomethyl-2-ethoxy-biphenyl is obtained by Suzuki cross-coupling of (3-bromo-phenyl)-methanol with 2-ethoxyphenylboronic acid followed by reaction of the resulting product with CBr₄/PPh₃ according to procedures described in Chem. Eur. J. 2000, 6, 3692. MS (LC-MS): 591.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.30 min.

### Example 161: N-{(3S*,4S*)-4-[5-(4-Chloro-phenyl)-oxazol-2-ylmethoxy]-pyrrolidin-3-ylmethy}-N-isopropyl-4-methoxy-3-(3-methoxy-propoxy)-benzamide

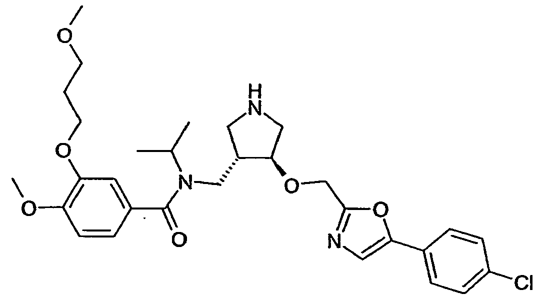

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 2-chloromethyl-5-(4-chloro-phenyl)-oxazole. MS (LC-MS): 571.9 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.87 min.

### Example 162: N-[(3S*,4S*)-4-(3'-Amino-biphenyl-3-ylmethoxy)-pyrrolidin-3-ylmethyl]-N-isopropyl-4-methoxy-3-(3-methoxy-propoxy)-benzamide

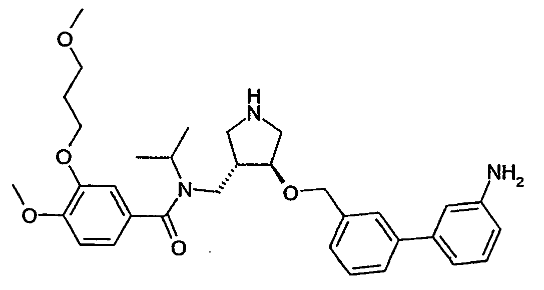

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3-bromomethyl-3'-nitro-biphenyl followed by hydrogenation as described for the title compound under A in Example 104 (Scheme15). 3-Bromomethyl-3'-nitro-biphenyl is obtained by Suzuki cross-coupling of (3-bromo-phenyl)-methanol with 3-nitroyphenylboronic acid followed by reaction of the resulting product with CBr₄/PPh₃ according to procedures described in Chem. Eur. J. 2000, 6, 3692. MS (LC-MS): 562.0 [M+H]⁺ t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.05 min.

### Example 163: N-[(3S*,4S*)-4-(3'-Acetylamino-biphenyl-3-ylmethoxy)-pyrrolidin-3-ylmethyl]-N-isopropyl-4-methoxy-3-(3-methoxy-propoxy)-benzamide

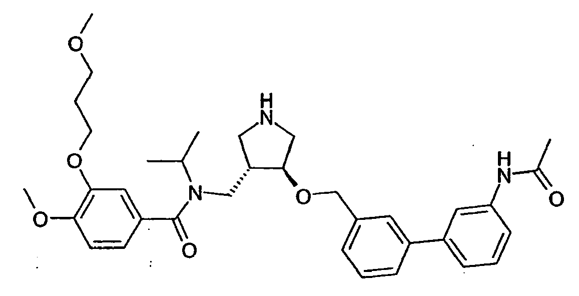

The title compound is prepared analogously as described for the title compound under G in Example 120 (Scheme23) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3-bromomethyl-3'-nitro-biphenyl followed by hydrogenation as described for the title compound under A in Example 104 (Scheme15) and acylation as described for the title compound under A in Example 101 (Scheme13). MS (LC-MS): 604.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 4.63 min.

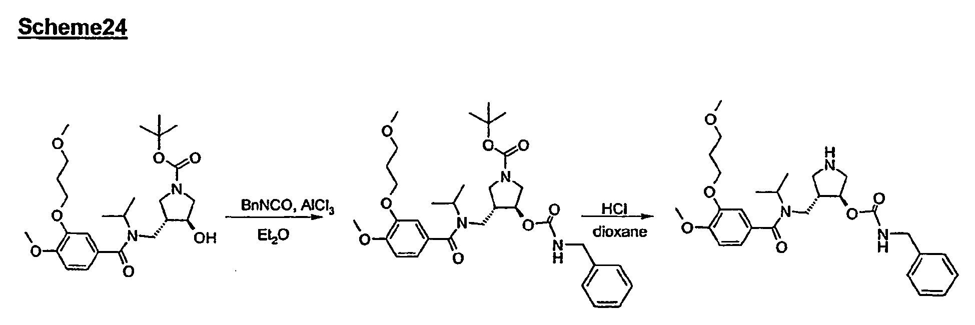

### Example 164: Benzyl-carbamic acid (3S*,4S*)-4-({isopropyl-[4-methoxy-3-(3-methoxy-propoxy)-benzoyl]-amino}-methyl)-pyrrolidin-3-yl ester

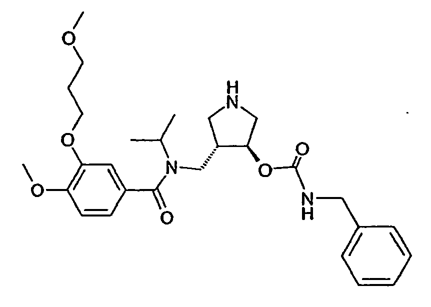

### A. (3S*,4R*)-3-Benzylcarbamoyloxy-4-({isopropyl-[4-methoxy-3-(3-methoxy-propoxy)-benzoyl]-amino}-methyl)-pyrrolidine-1-carboxylic acid tert-butyl ester

Benzyl isocyanate (53 µL, 0.43 mmol) followed by AlCl₃ (29 mg, 0.21 mmol) is added to a solution of (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester (103 mg, 0.21 mmol) in Et₂O (4 mL). The resulting mixture is stirred at RT for 12 h. For workup a sat. solution of NaHCO₃ is added and the mixture is extracted with ethyl acetate. The combined extracts are dried (Na₂SO₄) and the solvent is evaporated. The crude product is purified by preparative HPLC (C18 ODB-AQ column (YMC), 5% CH₃CN/H₂O/2.5 min, 5-100% CH₃CN/H₂O 10 min, 100% CH₃CN/2.5 min, CH₃CN and H₂O containing 0.1% TFA, flow: 20 mL/min) to give the desired product 71 mg) as a colorless oil. MS (LC-MS): 514.1 [M+H-Boc]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.84 min.

### B Benzyl-carbamic acid (3S*,4S*)-4-({isopropyl-[4-methoxy-3-(3-methoxy-propoxy)-benzoyl]-amino}-methyl)-pyrrolidin-3-yl ester

4N HCl/dioxane (2 mL) is added to a solution of (3S*,4R*)-3-benzylcarbamoyloxy-4-({isopropyl-[4-methoxy-3-(3-methoxy-propoxy)-benzoyl]-amino}-methyl)-pyrrolidine-1-carboxylic acid tert-butyl ester (71 mg, 0.12 mmol) in dioxane (3 mL). The mixture is stirred at RT for 4h before it is cooled to -78°C and lyophilized. The desired product (63 mg) is obtained as a colorless solid. MS (LC-MS): 514.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.44 min.

### Example 165: (3-Methoxy-phenyl)-carbamic acid (3S*,4S*)-4-({isopropyl-[4-methoxy-3-(3-methoxy-propoxy)-benzoyl]-amino}-methyl)-pyrrolidin-3-yl ester

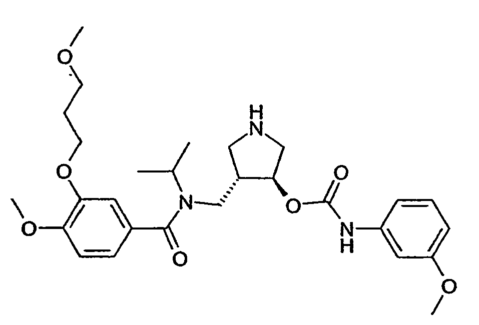

The title compound is prepared analogously as described for the title compound in Example 164 (Scheme24) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-isocyanato-3-methoxy-benzene. MS (LC-MS): 530.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.51 min.

### Example 166: Phenethyl-carbamic acid (3S*,4S*)-4-({isopropyl-[4-methoxy-3-(3-methoxy-propoxy)-benzoyl]-amino}-methyl)-pyrrolidin-3-yl ester

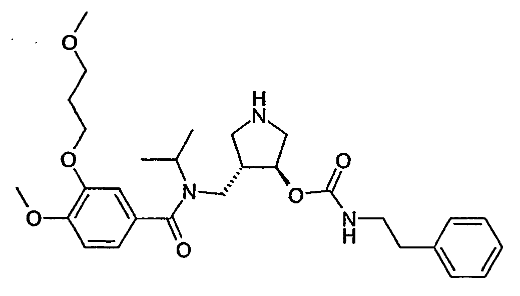

The title compound is prepared analogously as described for the title compound in Example 164 (Scheme24) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and (2-isocyanatoethyl)-benzene. MS (LC-MS): 529.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.57 min.

### Example 167: (3-Methoxy-benzyl)-carbamic acid (3S*,4S*)-4-({isopropyl-[4-methoxy-3-(3-methoxy-propoxy)-benzoyl]-amino}-methyl)-pyrrolidin-3-yl ester

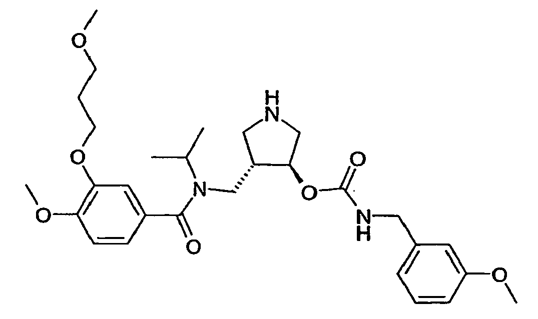

The title compound is prepared analogously as described for the title compound in Example 164 (Scheme24) from (3S*,4R*)3-hydroxy-4-({isopropyl-[4-methoxy-3-(3-methoxy-propoxy)-benzoyl]-amino}-methyl)-pyrrolidine-1-carboxylic acid tert-butyl ester and 1-isocyanatomethyl-3-methoxy-benzene. MS (LC-MS): 544.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.46 min.

### Example 168: (2-Ethoxy-benzyl)-carbamic acid (3S*,4S*)-4-({isopropyl-[4-methoxy-3-(3-methoxy-propoxy)-benzoyl]-amino}-methyl)-pyrrolidin-3-yl ester

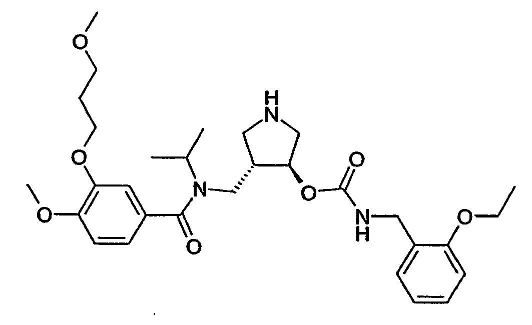

The title compound is prepared analogously as described for the title compound in Example 164 (Scheme24) from (3S*,4R*)-3-hydroxy-4-({isopropyl-[4-methoxy-3-(3-methoxy-propoxy)-benzoyl]-amino}-methyl)-pyrrofidine-1-carboxylic acid tert-butyl ester and 1-ethoxy-2-isocyanatomethyl-benzene. MS (LC-MS): 558.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.71 min.

### Example 169: Furan-2-ylmethyl-carbamic acid (3S*,4S*)-4-({isopropyl-[4-methoxy-3-(3-methoxy-propoxy)-benzoyl]-amino}-methyl)-pyrrolidin-3-yl ester

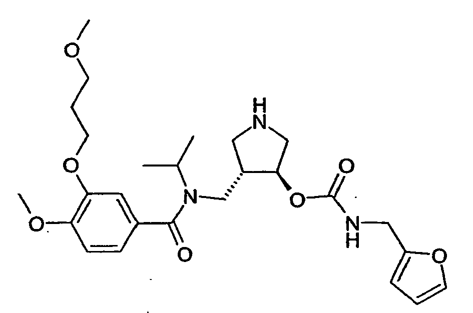

The title compound is prepared analogously as described for the title compound in Example 164 (Scheme24) from (3S*,4R*)3-hydroxy-4-({isopropyl-[4-methoxy-3-(3-methoxy-propoxy)-benzoyl]-amino}-methyl)-pyrrolidine-1-carboxylic acid tert-butyl ester and 2-isocyanatomethyl-furan. MS (LC-MS): 504.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.31 min.

### Example 170: (2-Methoxy-benzyl)-carbamic acid (3S*,4S*)-4-({isopropyl-[4-methoxy-3-(3-methoxy-propoxy)-benzoyl]-amino}-methyl)-pyrrolidin-3-yl ester

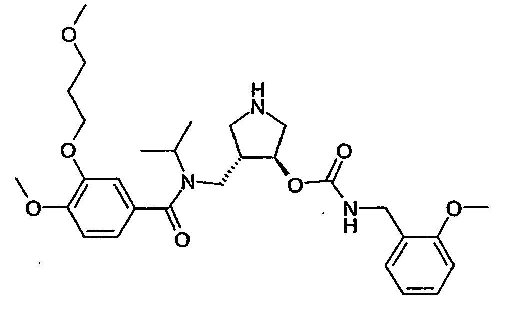

The title compound is prepared analogously as described for the title compound in Example 164 (Scheme24) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-isocyanatomethyl-2-methoxy-benzene. MS (LC-MS): 544.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.56 min.

### Example 171: ((R)-1-Naphthalen-1-yl-ethyl)-carbamic acid (3S*,4S*)-4-({isopropyl-[4-methoxy-3-(3-methoxy-propoxy)-benzoyl]-amino}-methyl)-pyrrolidin-3-yl ester

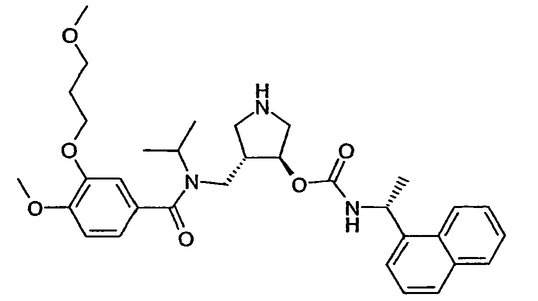

The title compound is prepared analogously as described for the title compound in Example 164 (Scheme24) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-((R)-1-isocyanato-ethyl)-naphthalene. MS (LC-MS): 578.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.86 min.

### Example 172: ((S)-1-Phenyl-ethyl)-carbamic acid (3S*,4S*)-4-({isopropyl-[4-methoxy-3-(3-methoxy-propoxy)-benzoyl]-amino}-methyl)-pyrrolidin-3-yl ester

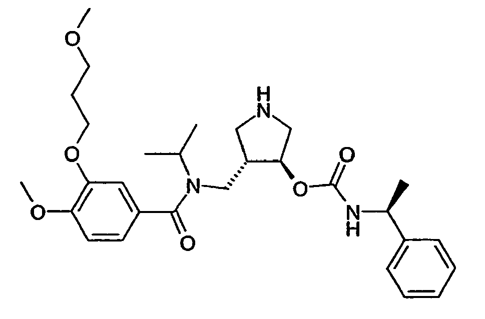

The title compound is prepared analogously as described for the title compound in Example 164 (Scheme24) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and ((S)-1-isocyanatoethyl)-benzene. MS (LC-MS): 528.3 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.59 min.

### Example 173: ((R)-1-Phenyl-ethyl)-carbamic acid (3S*,4S*)-4-({isopropyl-[4-methoxy-3-(3-methoxy-propoxy)-benzoyl]-amino}-methyl)-pyrrolidin-3-yl ester

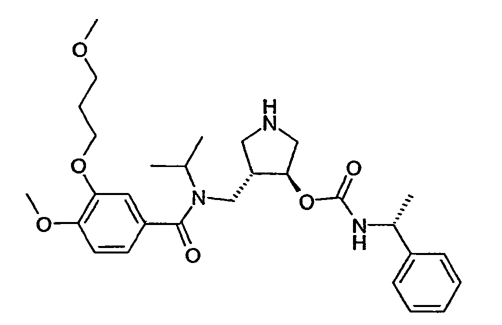

The title compound is prepared analogously as described for the title compound in Example 164 (Scheme24) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and ((S)-1-isocyanatoethyl)-benzene. MS (LC-MS): 528.30 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.56 min.

### Example 174: Benzyl-carbamic acid (3R,4R)-4-({isopropyl-[4-methoxy-3-(3-methoxy-propoxy)-benzoyl]-amino}-methyl)-pyrrolidin-3-yl ester

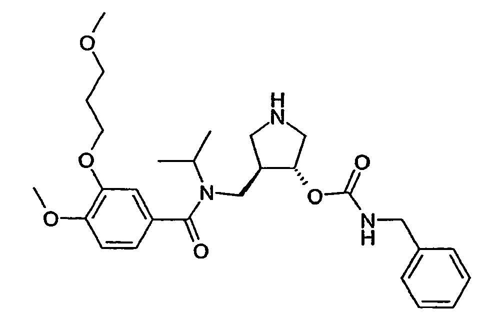

The title compound is prepared analogously as described for the title compound in Example 164 (Scheme24) from enantiomerically pure (3R,45)-3-hydroxy-4-({isopropyl-[4-methoxy-3-(3-methoxy-propoxy)-benzoyl]-amino}methyl)-pyrrolidine-1-carboxylic acid tert-butyl ester and isocyanatomethyl-benzene. MS (LC-MS): 514.0 [M+H]⁺, t_{R} (HPLC, C18 column, 10-100% CH3CN/H₂O/5 min, 100% CH₃CN/3 min, 100-10% CH₃CN/H₂O/3 min, CH₃CN and H₂O containing 0.1% TFA, flow: 1.5 mL/min): 4.47 min.

### Example 175: Benzyl-carbamic acid (3S,4S)-4-({isopropyl-[4-methoxy-3-(3-methoxy-propoxy)-benzoyl]-amino}-methyl)-pyrrolidin-3-yl ester

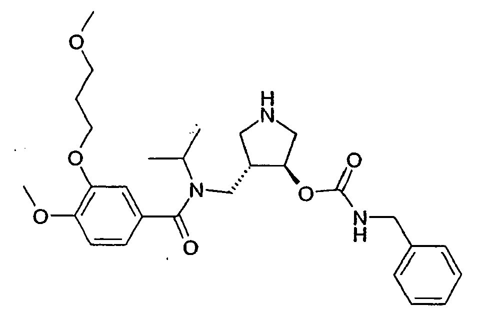

The title compound is prepared analogously as described for the title compound in Example 164 (Scheme24) from enantiomerically pure (3S,4R)-3-hydroxy-4-({isopropyl-[4-methoxy-3-(3-methoxy-propoxy)-benzoyl]-amino}-methyl)-pyrrolidine-1-carboxylic acid tert-butyl ester and isocyanatomethyl-benzene. MS (LC-MS): 514.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mUmin): 4.49 min.

### Example 176: Benzyl-carbamic acid (3R*,4S*)-4-({isopropyl-[4-methoxy-3-(3-methoxy-propoxy)-benzoyl]-amino}-methyl)-pyrrolidin-3-yl ester

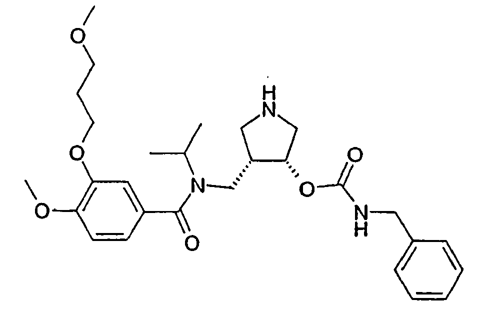

The title compound is prepared analogously as described for the title compound in Example 164 (Scheme24) from (3R*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and isocyanatomethyl-benzene. (3R*,4R*)-3-Hydroxy-4-({isopropyl-[4-methoxy-3-(3-methoxy-propoxy)-benzoyl]-amino}-methyl)-pyrrolidine-1-carboxylic acid tert-butyl ester is obtained from (3R*,4S*)-3-(tert-butyl-dimethyl-silanyloxy)4-cyano-pyrrolidine-1-carboxylic acid tert-butyl ester according to Example 120 (Scheme23). MS (LC-MS): 514.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.25 min.

### Example 177: (4-Methoxy-benzyl)-carbamic acid (3S*,4S*)4-({isopropyl-[4-methoxy-3-(3-methoxy-propoxy)-benzoyl]-amino}-methyl)-pyrrolidin-3-yl ester

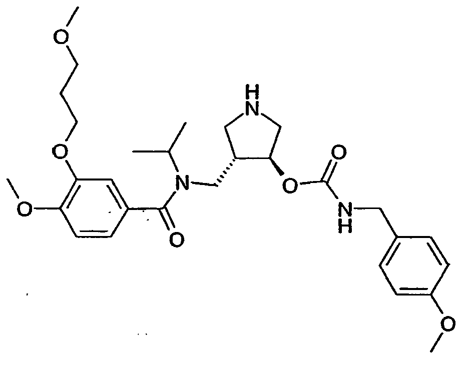

The title compound is prepared analogously as described for the title compound in Example 164 (Scheme24) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 1-isocyanatomethyl-4-methoxy-benzene. MS (LC-MS): 544.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 4.59 min.

### Example 178: Benzyl-carbamic acid (3S,4S)-4-({[4-ethyl-3-(3-methoxy-propoxy)-benzoyl]-isopropyl-amino}-methyl)-pyrrolidin-3-yl ester

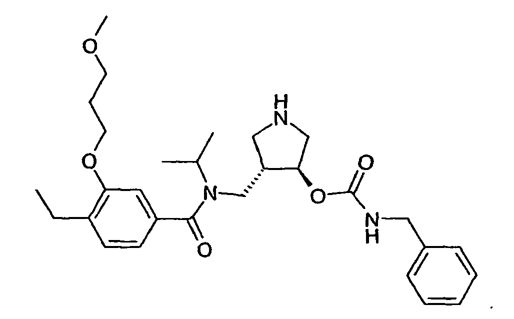

The title compound is prepared analogously as described for the title compound in Example 164 (Scheme24) from 4-ethyl-N-((3S*,4S*)-4-hydroxy-pyrrolidin-3-ylmethyl)-N-isopropyl-3-(3-methoxy-propoxy)-benzamide and isocyanatomethyl-benzene. 4-Ethyl-N-((3S*,4S*)-4-hydroxy-pyrrolidin-3-ylmethyl)-N-isopropyl-3-(3-methoxy-propoxy)-benzamide is obtained according to Example 120 (Scheme23) using 4-ethyl-3-(3-methoxy-propoxy)-benzoic acid (synthesis described in Example 60). MS (LC-MS): 512.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.33 min.

### Example 179: Benzyl-carbamic acid (3S*,4S*)-4-({isopropyl-[1-(3-methoxy-propyl)-3-methyl-1H-indole-6-carbonyl]-amino}-methyl)-pyrrolidin-3-yl ester

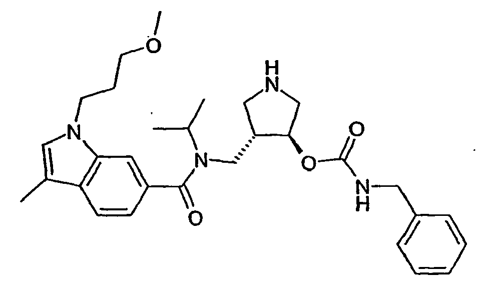

The title compound is prepared analogously as described for the title compound in Example 164 (Scheme24) from 1-(3-methoxy-propyl)-3-methyl-1H-indole-6-carboxylic acid ((3S*,4S*)-4-hydroxy-pyrrolidin-3-ylmethyl)-isopropyl-amide and isocyanatomethyl-benzene. 1-(3-Methoxy-propyl)-3-methyl-1H-indole-6-carboxylic acid ((3S*,4S*)-4-hydroxy-pyrrolidin-3-ylmethyl)-isopropyl-amide is obtained according to Example 120 (Scheme23) using 1-(3-methoxy-propyl)-3-methyl-1H-indole-6-carboxylic acid (synthesis described in Example 65). MS (LC-MS): 521.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.22 min.

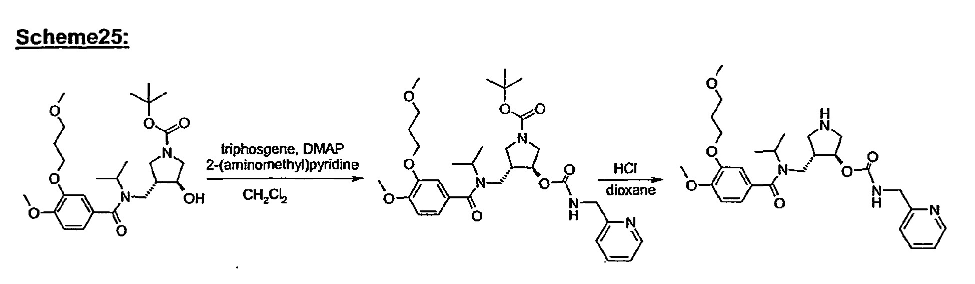

### Example 180: Pyridin-2-ylmethyl-carbamic acid (3S*,4S*)-4-({isopropyl-[4-methoxv-3-(3-methoxy-propoxy)-benzoyl]-amino}-methyl)-pyrrolidin-3-yl ester

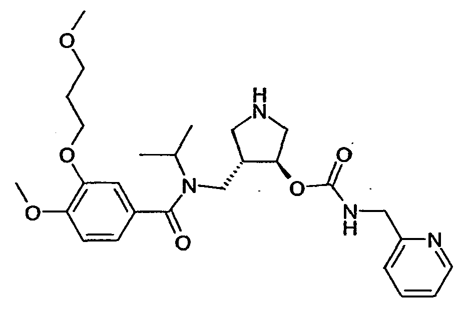

### A. (3R*,45*)-3-({Isopropyl-[4-methoxy-3-(3-methoxy-propoxy)-benzoyl]-amino}-methyl)-4-(pyridin-2-ylmethylcarbamoyloxy)-pyrrolidine-1-carboxylic acid tert-butyl ester

Triphosgene (36 mg, 0.127 mmol) and DMAP (128 mg, 1.05 mmol) are added to a solution of (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester (157 mg, 0.33 mmol) in CH₂Cl₂ (4 mL). After stirring for 15 min at RT 2-(aminomethyl)pyridine (51 µl, 0.49 mmol) is added and the resulting yellowish cloudy mixture is stirred at RT for another 2 h. The solvent is removed under reduced pressure and the residue is sublected to purification by preparative HPLC (C18 ODB-AQ column (YMC), 5% CH₃CN/H₂O/2.5 min, 5-100% CH₃CN/H₂O 10 min, 100% CH₃CN/2.5 min, CH₃CN and H₂O containing 0.1% TFA, flow: 20 mL/min) to give the desired product as a colorless oil. MS (LC-MS): 615.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.59 min.

### B Pyridin-2-ylmethyl-carbamic acid (3S*,4S*)-4-({isopropyl-[4-methoxy-3-(3-methoxy-propoxy)-benzoyl]-amino}-methyl)-pyrrolidin-3-yl ester

4N HCl/dioxane (2 mL) is added to a solution of (3R*,4S*)-3-({Isopropyl-[4-methoxy-3-(3-methoxy-propoxy)-benzoyl]-amino}-methyl)-4-(pyridin-2-ylmethylcarbamoyloxy)-pyrrolidine-1-carboxylic acid tert-butyl ester (130 mg, 0.21 mmol) in dioxane (5 mL). The mixture is stirred at RT for 12 h before it is cooled to -78°C and lyophilized. The desired product as its bis-hydrochloride is obtained as a colorless solid. MS (LC-MS): 515.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 3.64 min.

### Example 181: (2,3-Dimethoxy-benzyl)-carbamic acid (3S*,4S*)-4-({isopropyl-[4-methoxy-3-(3-methoxy-propoxy)-benzoyl]-amino}-methyl)-pyrrolidin-3-yl ester

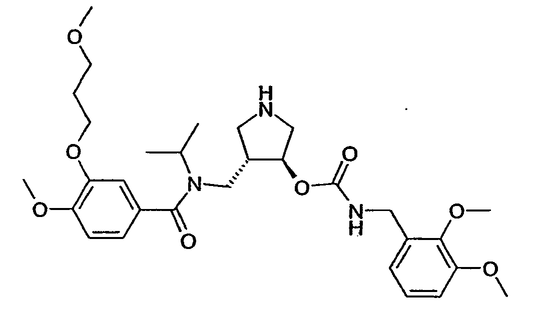

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 2,3-dimethoxybenzylamine. MS (LC-MS): 574.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.49 min.

### Example 182: Naphthalen-1-ylmethyl-carbamic acid (3S*,4S*)-4-({isopropyl-[4-methoxy-3-(3-methoxy-propoxy)-benzoyl]-amino}-methyl)-pyrrolidin-3-yl ester

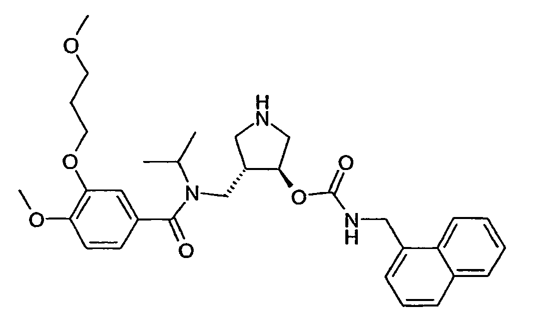

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-naphthalen-1-yl-methylamine. MS (LC-MS): 564.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.78 min.

### Example 183: (2-Isopropoxy-benzyl)-carbamic acid (3S*,4S*)-4-({isopropyl-[4-methoxy-3-(3-methoxy-propoxy)-benzoyl]-amino}-methyl)-pyrrolidin-3-yl ester

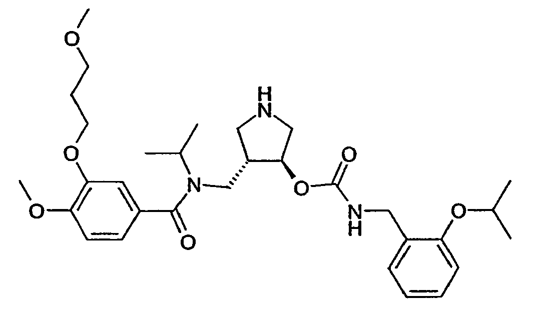

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 2-isopropoxy-benzylamine. MS (LC-MS): 572.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.87 min.

### Example 184: (2,3-Dihydro-benzo[1,4]dioxin-5-ylmethyl)-carbamic acid (3S*,4S*)-4-({isopropyl-[4-methoxy-3-(3-methoxy-propoxy)-benzoyl]-amino}-methyl)-pyrrolidin-3-yl ester

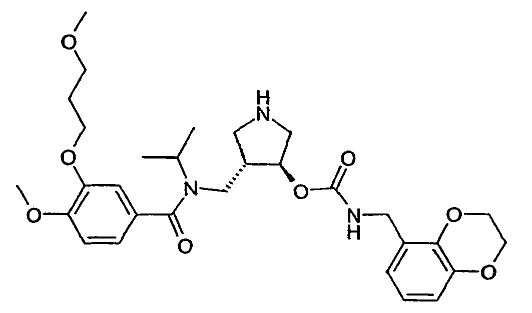

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-(2,3-dihydro-benzo[1,4]dioxin-5-yl)-methylamine. MS (LC-MS): 572.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.51 min.

### Example 185: (1-Methyl-1H-imidazol-4-ylmethyl)-carbamic acid (3S*,4S*)-4-({isopropyl-[4-methoxy-3-(3-methoxy-propoxy)-benzoyl]-amino}-methyl)-pyrrolidin-3-yl ester

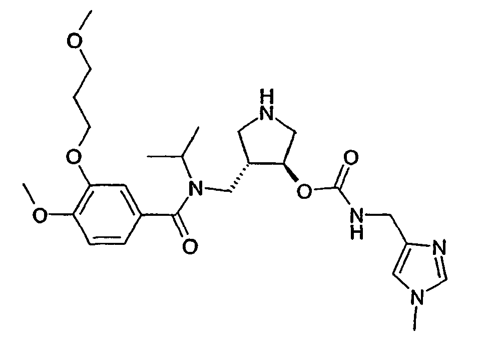

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-(1-methyl-1H-imidazol-4-yl)-methylamine. MS (LC-MS): 518.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 3.65 min.

### Example 186: Isoguinolin-1-ylmethyl-carbamic acid (3S*,4S*)-4-({isopropyl-[4-methoxy-3-(3-methoxy-propoxy)-benzoyl]-amino}-methyl)-pyrrolidin-3-yl ester

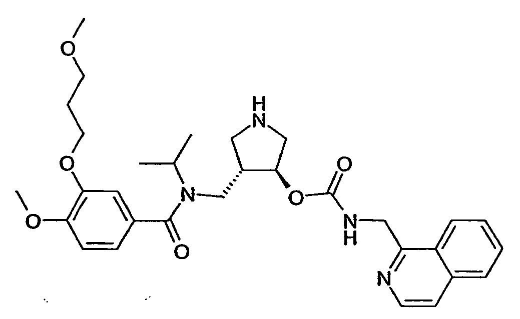

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-isoquinolin-1-yl-methylamine. MS (LC-MS): 565.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 3.91 min.

### Example 187: Pyridin-3-ylmethyl-carbamic acid (3S*,4S*)-4-({isopropyl-[4-methoxy-3-(3-methoxy-propoxy)-benzoyl]-amino}-methyl)-pyrrolidin-3-yl ester

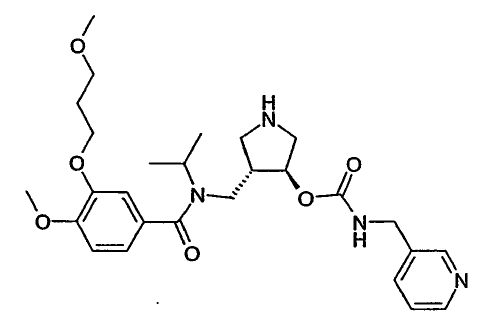

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-pyridin-3-yl-methylamine. MS (LC-MS): 258.1 [M+2H]²⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 3.72 min.

### Example 188: (1-Methyl-1H-benzoimidazol-2-ylmethyl)-carbamic acid (3S*,4S*)-4-({isopropyl-[4-methoxy-3-(3-methoxy-propoxy)-benzoyl]-amino}-methyl)-pyrrolidin-3-yl ester

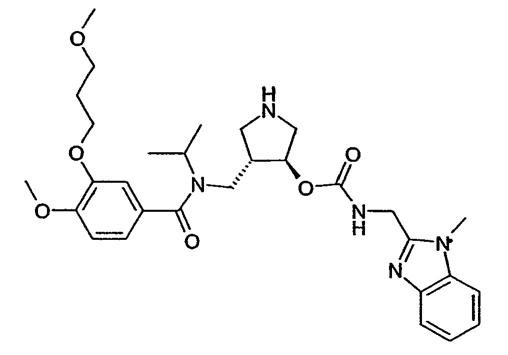

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-(1-methyl-1H-benzoimidazol-2-yl)-methylamine. MS (LC-MS): 568.0 [M+H]⁺, 284.6 [M+2H]²⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 3.93 min.

### Example 189: Thiazol-2-ylmethyl-carbamic acid (3S*,4S*)-4-({isopropyl-[4-methoxy-3-(3-methoxy-propoxy)-benzoyl]-amino}-methyl)-pyrrolidin-3-yl ester

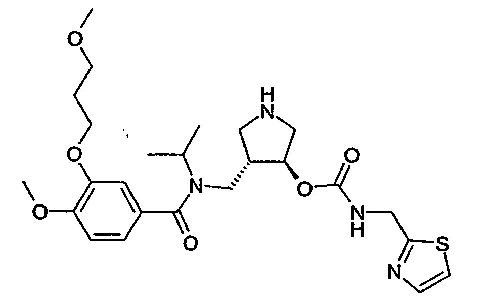

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-thiazol-2-yl-methylamine. MS (LC-MS): 521.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.08 min.

### Example 190: Benzo[1,3]dioxol-5-ylmethyl-carbamic acid (3S*,4S*)-4-({isopropyl-[4-methoxy-3-(3-methoxy-propoxy)-benzoyl]-amino}-methyl)-pyrrolidin-3-yl ester

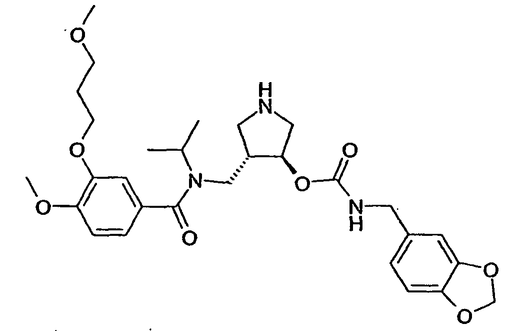

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-benzo[1,3]dioxol-5-yl-methylamine. MS (LC-MS): 558.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.49 min.

### Example 191: [(S)-1-(Tetrahydro-furan-2-yl)methyl]-carbamic acid (3S*,4S*)-4-({isopropyl-[4-methoxy-3-(3-methoxy-propoxy)-benzoyl]-amino}-methyl)-pyrrolidin-3-yl ester

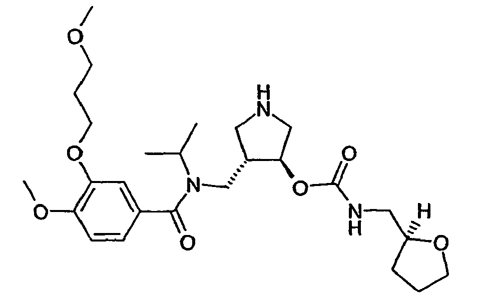

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-[(S)-1-(tetrahydro-furan-2-yl)]-methylamine. MS (LC-MS): 508.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.77 min.

### Example 192: Benzothiazol-2-ylmethyl-carbamic acid (3S*,4S*)-4-({isopropyl-[4-methoxy-3-(3-methoxy-propoxy)-benzoyl]-amino}-methyl)-pyrrolidin-3-yl ester

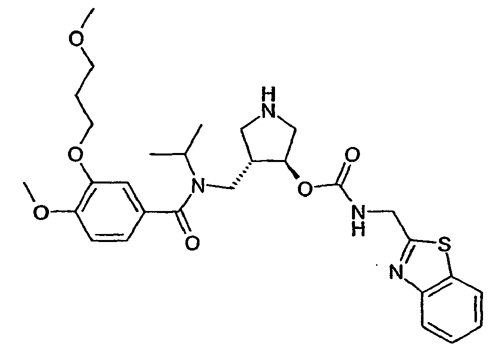

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-benzothiazol-2-yl-methylamine. MS (LC-MS): 571.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.49 min.

### Example 193: ((1S,2S)-2-Hydroxy-cyclopentyl)-carbamic acid (3S*,4S*)-4-({isopropyl-[4-methoxy-3-(3-methoxy-propoxy)-benzoyl]-amino}-methyl)-pyrrolidin-3-yl ester

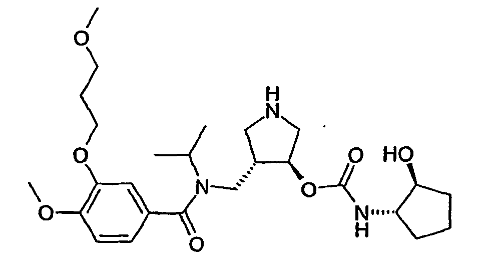

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and (1S,2S)-2-benzyloxy-cyclopentylamine. Benzyl-deprotection prior to Boc-deprotection is performed according to step C in Example 2 (Scheme2). MS (LC-MS): 508.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.05 and 4.09 min.

### Example 194: (3-Isopropoxy-benzyl)-carbamic acid (3S*,4S*)-4-({isopropyl-[4-methoxy-3-(3-methoxy-propoxy)-benzoyl]-amino}-methyl)-pyrrolidin-3-yl ester

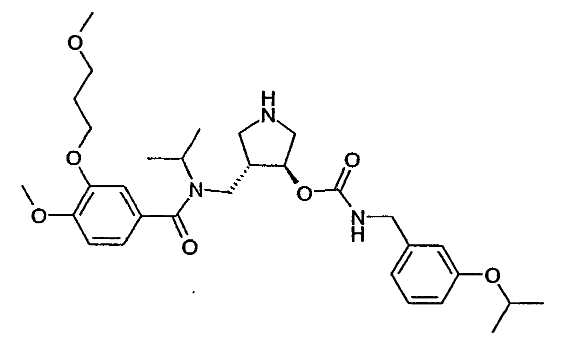

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3-isopropoxy-benzylamine. MS (LC-MS): 572.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.83 min.

### Example 195: Quinolin-4-ylmethyl-carbamic acid (3S*,4S*)-4-({isopropyl-[4-methoxy-3-(3-methoxy-propoxy)-benzoyl]-amino}-methyl)-pyrrolidin-3-yl ester

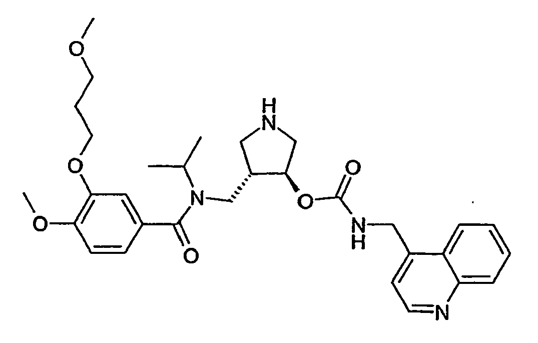

The title compound is prepared analogously as described for the title compound in Example 180A (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-quinolin-4-yl-methylamine. MS (LC-MS): 283.1 [M+2H]²⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 3.89 min.

### Example 196: ((1R,2R)-2-Hydroxy-cyclohexyl)-carbamic acid (3S*,4S*)-4-({isopropyl-[4-methoxy-3-(3-methoxy-propoxy)-benzoyl]-amino}-methyl)-pyrrolidin-3-yl ester

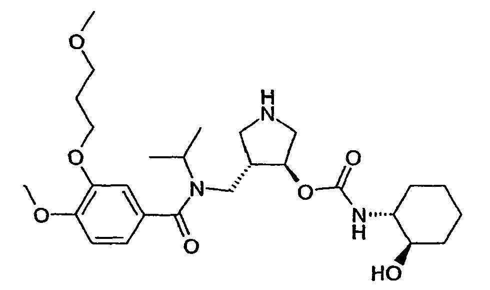

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and (1R,2R)-2-benzyloxy-cyclohexylamine. Benzyl-deprotection prior to Boc-deprotection is performed according to step C in Example 2 (Scheme2). MS (LC-MS): 522.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.22 and 4.27 min.

### Example 197: ((1R,2R)-2-Hydroxy-cyclopentyl)-carbamic acid (3S*,4S*)-4-({isopropyl-[4-methoxy-3-(3-methoxy-propoxy)-benzoyl]-amino}-methyl)-pyrrolidin-3-yl ester

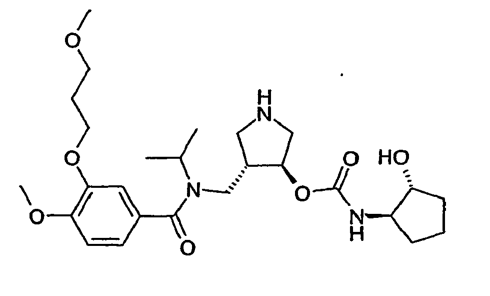

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and (1R,2R)-2-benzyloxy-cyclopentylamine. Benzyl-deprotection prior to Boc-deprotection is performed according to step C in Example 2 (Scheme2). MS (LC-MS): 508.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 4.07 and 4.11 min.

### Example 198: Benzyl-methyl-carbamic acid (3S*,4S*)-4-({isopropyl-[4-methoxy-3-(3-methoxy-propoxy)-benzoyl]-amino}-methyl)-pyrrolidin-3-yl ester

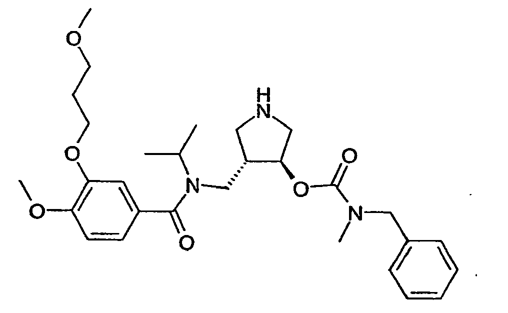

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and benzyl-methyl-amine. MS (LC-MS): 528.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.70 min.

### Example 199: (S)-1-Pyrrolidin-2-ylmethyl-carbamic acid (3S*,4S*)-4-({isopropyl-[4-methoxy-3-(3-methoxy-propoxy)-benzoyl]-amino}-methyl)-pyrrolidin-3-yl ester

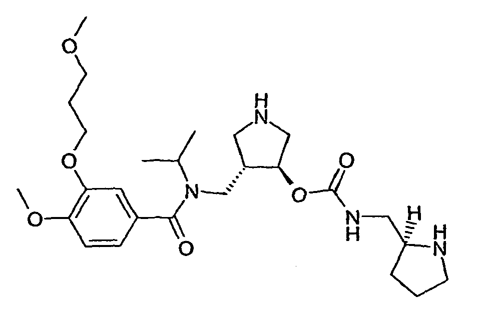

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and (S)-2-aminomethyl-pyrrolidine-1-carboxylic acid tert-butyl ester. MS (LC-MS): 254.2 [M+2H]²⁺, 507.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 3.79 min.

### Example 200: (2-Acetylamino-benzyl)-carbamic acid (3S,4S)-4-({isopropyl-[4-methoxy-3-(3-methoxy-propoxy)-benzoyl]-amino}-methyl)-pyrrolidin-3-yl ester

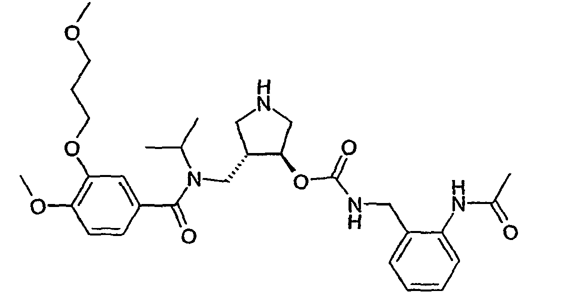

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 2-nitrobenzyl amine. Reduction of the nitro group is performed according to step A in Example 104 (Scheme15) followed by acetylation according to step A in Example 101 (Scheme13). Boc-deprotection is performed according to Example 180 (Scheme25). MS (LC-MS): 254.2 [M+2H]²⁺, 571.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.31 min.

### Example 201: (4-Acetylamino-benzyl)-carbamic acid (3S*,4S*)-4-({isopropyl-[4-methoxy-3-(3-methoxy-propoxy)-benzoyl]-amino}-methyl)-pyrrolidin-3-yl ester

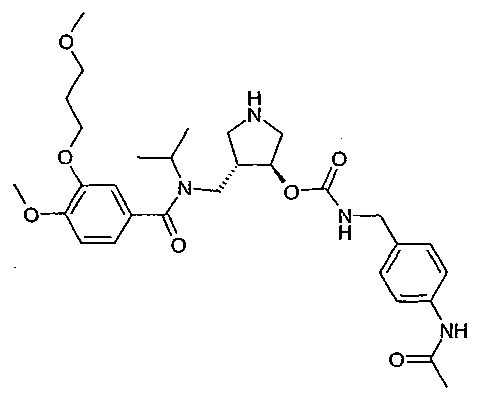

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 4-nitrobenzyl amine. Reduction of the nitro group is performed according to step A in Example 104 (Scheme15) followed by acetylation according to step A in Example 101 (Scheme13). Boc-deprotection is performed according to Example 180 (Scheme25). MS (LC-MS): 571.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.29 min.

### Example 202: (3-Pyrrol-1-yl-benzyl)-carbamic acid (3S*,4S*)-4-({isopropyl-[4-methoxy-3-(3-methoxy-propoxy)-benzoyl]-amino}-methyl)-pyrrolidin-3-yl ester

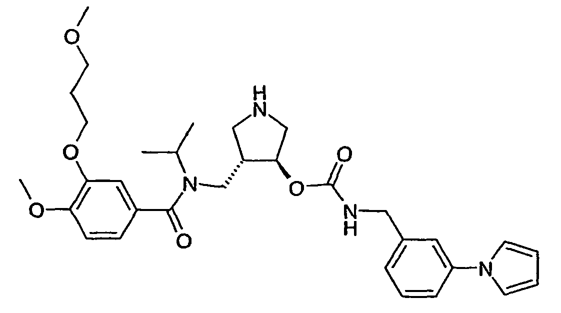

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3-pyrrol-1-yl-benzylamine. MS (LC-MS): 579.0 [M+H]⁺, t_{R} (HPLC, C18 column, 10-100% CH₃CN/H₂O/5 min, 100% CH₃CN/3 min, 100-10% CH₃CN/H₂O/₃ min, CH₃CN and H₂O containing 0.1% TFA, flow: 1.5 mL/min): 4.96 min.

### Example 203: Benzyl-ethyl-carbamic acid (3S*,4S*)-4-({isopropyl-[4-methoxy-3-(3-methoxy-propoxy)-benzoyl]-amino}-methyl)-pyrrolidin-3-yl ester

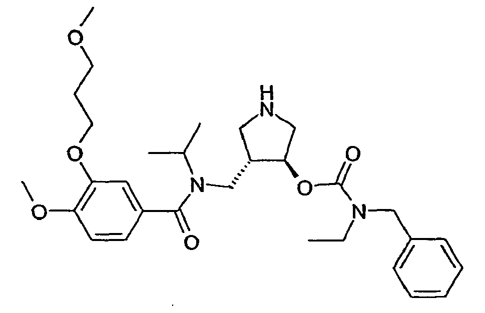

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and benzyl-ethyl-amine. MS (LC-MS): 542.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.93 min.

### Example 204: (2 3-Dihydro-benzofuran-5-ylmethyl)-carbamic acid (3S*,4S*)-4-({isopropyl-[4-methoxy-3-(3-methoxy-propoxy)-benzoyl]-amino}-methyl)-pyrrolidin-3-yl ester

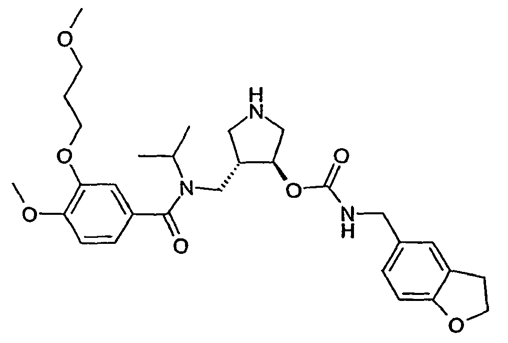

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-(2,3-dihydrobenzofuran-5-yl)-methylamine. MS (LC-MS): 556.2 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 4.66 min.

### Example 205: (2,3-Dihydro-benzo[1,4]dioxin-6-ylmethyl)-carbamic acid (3S*,4S*)-4-({isopropyl-[4-methoxy-3-(3-methoxy-propoxy)-benzoyl]-amino}-methyl)-pyrrolidin-3-yl ester

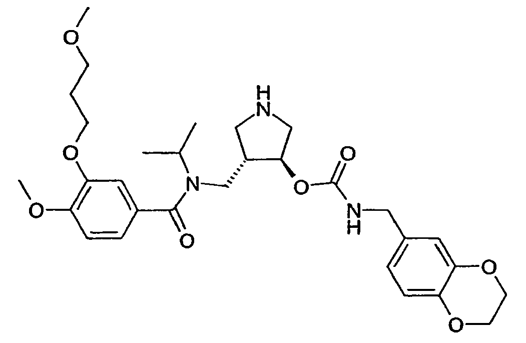

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-(2,3-dihydro-benzo[1,4]dioxin-6-yl)-methylamine. MS (LC-MS): 572.2 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.65 min.

### Example 206: (3,4-Dimethoxy-benzyl)-carbamic acid (3S*,4S*)-4-({isopropyl-[4-methoxy-3-(3-methoxy-propoxy)-benzoyl]-amino}-methyl)-pyrrolidin-3-yl ester

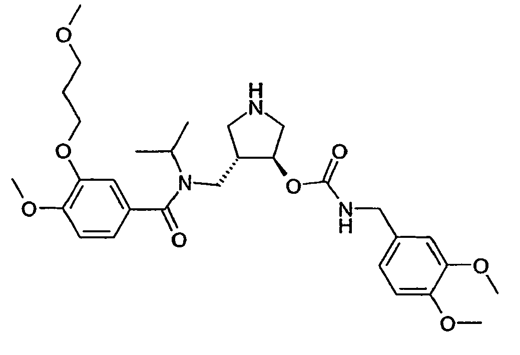

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 3,4-dimethoxybenzylamine. MS (LC-MS): 574.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.57 min.

### Example 207: (3-Acetylamino-benzyl)-carbamic acid (3S*,4S*)-4-({isopropyl-[4-methoxy-3-(3-methoxy-propoxy)-benzoyl]-amino}-methyl)-pyrrolidin-3-yl ester

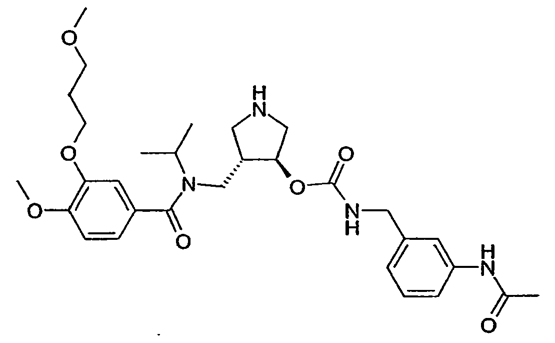

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and N-(3-aminomethylphenyl)-acetamide. MS (LC-MS): 571.0 [M+H]⁺, t_{R} (HPLC, C18 column, 5-100% CH₃CN/H₂O/5 min, CH₃CN and H₂O containing 0.1% formic acid, flow: 0.6 mL/min): 3.63 min.

### Example 208: Benzofuran-5-ylmethyl-carbamic acid (3S*,4S*)-4-({isopropyl-4-methoxy-3-(3-methoxy-propoxy)-benzoyl]-amino}-methyl)-pyrrolidin-3-yl ester

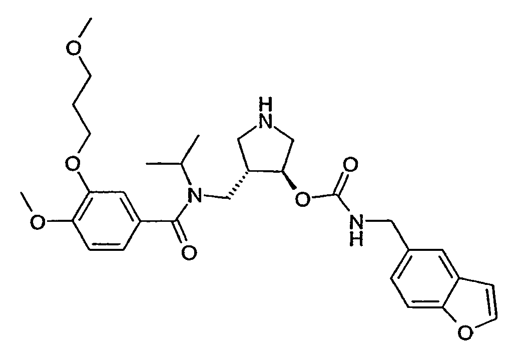

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and C-benzofuran-5-yl-methylamine. MS (LC-MS): 554.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.90 min.

### Example 209: Pyrrolidine-1-carboxylic acid (3S*,4S*)-4-({isopropyl-[4-methoxy-3-(3-methoxy-propoxy)-benzoyl]-amino}-methyl)-pyrrolidin-3-yl ester

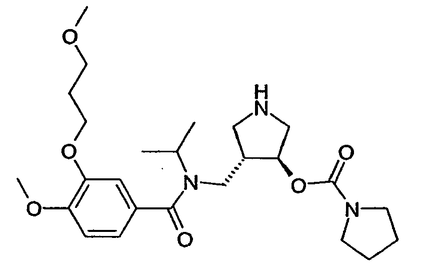

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and pyrrolidine. MS (LC-MS): 478.1 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.60 min.

### Example 210: (2-Pyrrol-1-yl-benzyl)-carbamic acid (3S*,4S*)-4-({isopropyl-[4-methoxy-3-(3-methoxy-propoxy)-benzoyl]-amino}-methyl)-pyrrolidin-3-yl ester

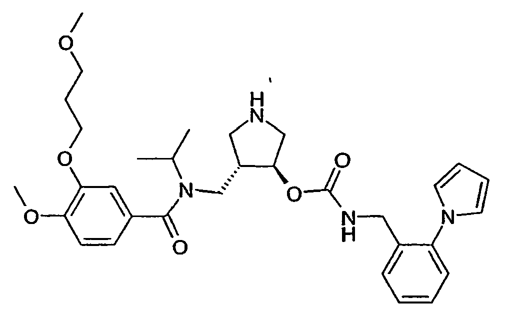

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 2-pyrrol-1-yl-benzylamine. MS (LC-MS): 579.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.10 min.

### Example 211: (S)-3-(4-Fluoro-phenyl)-pyrrolidine-1-carboxylic acid (3S*,4S*)-4-({isopropyl-[4-methoxy-3-(3-methoxy-propoxy)-benzoyl]-amino}-methyl)-pyrrolidin-3-yl ester

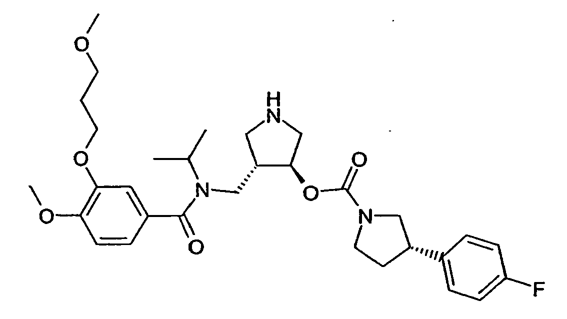

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and (S)-3-(4-fluorophenyl)-pyrrolidine. MS (LC-MS): 572.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 5.13 min.

### Example 212: (2-Morpholin-4-yl-benzyl)-carbamic acid (3S*,4S*)-4-({isopropyl-[4-methoxy-3-(3-methoxy-propoxy)-benzoyl]-amino}-methyl)-pyrrolidin-3-yl ester

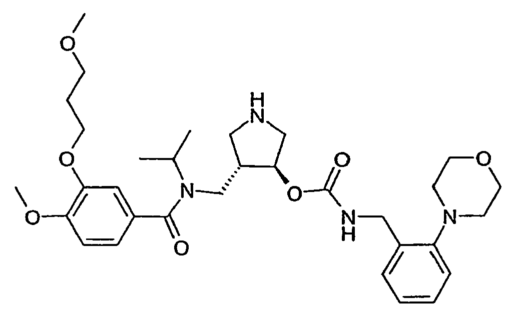

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 2-morpholin-4-yl-benzylamine. 2-Morpholin-4-yl-benzylamine is prepared according to literature procedures from 2-bromo benzonitrile and morpholine: Tetrahedron Lett. 2004, 45, 8319 and J. Med. Chem. 1998, 41, 5219. MS (LC-MS): 300.1 [M+2H]²⁺, 599.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1 % TFA, flow: 1.5 mL/min): 5.14 min.

### Example 213: (2-Piperidin-1-yl-benzyl)-carbamic acid (3S*,4S*)-4-({isopropyl-[4-methoxy-3-(3-methoxy-propoxy)-benzoyl]-amino}-methyl)-pyrrolidin-3-yl ester

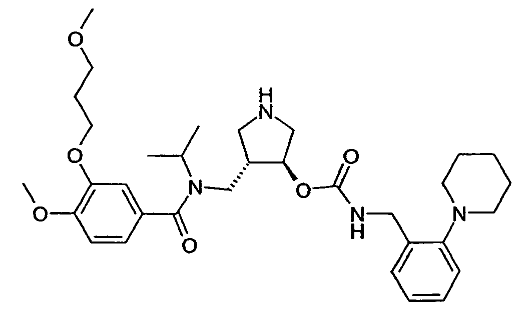

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 2-piperidin-1-yl-benzylamine. 2-Piperidin-1-yl-benzylamine is prepared according to literature procedures from 2-bromo benzonitrile and piperidine: Tetrahedron Lett. 2004, 45, 8319 and J. Med. Chem. 1998, 41, 5219. MS (LC-MS): 299.2 [M+2H]²⁺, 597.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.88 min.

### Example 214: (2-Pyrrolidin-1-yl-benzyl)-carbamic acid (3S*,4S*)-4-({isopropyl-[4-methoxy-3-(3-methoxy-propoxy)-benzoyl]-amino}-methyl)-pyrrolidin-3-yl ester

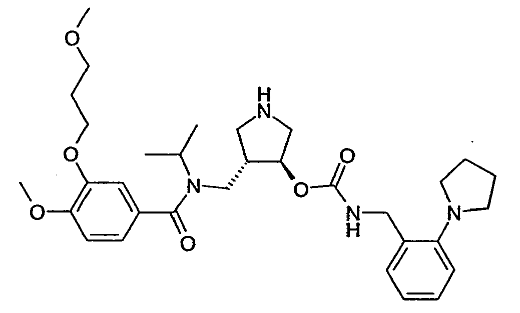

The title compound is prepared analogously as described for the title compound in Example 180 (Scheme25) from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 2-pyrrolidin-1-yl-benzylamine. 2-Pyrrolidin-1-yl-benzylamine is prepared according to literature procedures from 2-bromo benzonitrile and pyrrolidine: Tetrahedron Lett. 2004, 45, 8319 and J. Med. Chem. 1998, 41, 5219. MS (LC-MS): 292.1 [M+2H]²⁺, 583.0 [M+H]⁺, t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 4.90 min.

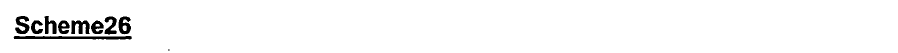

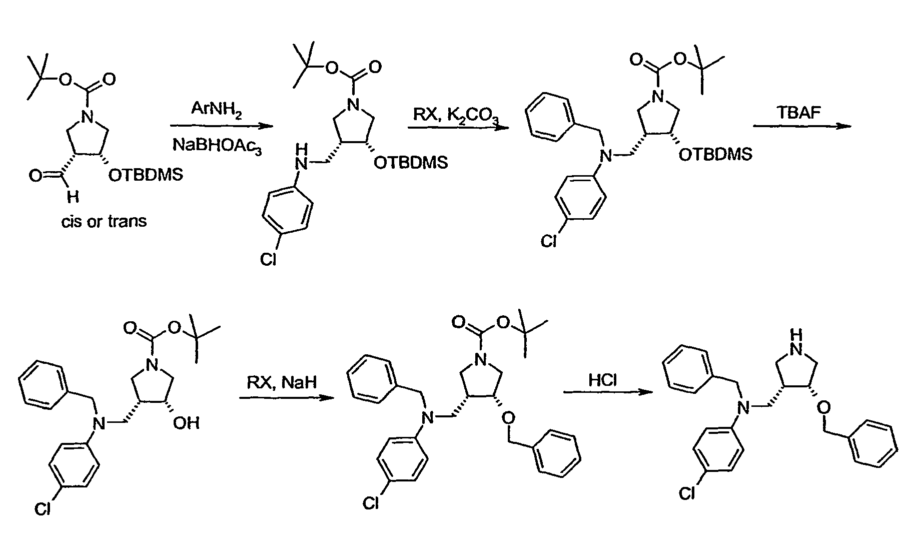

### Example 215: Benzyl-((3S*,4R*)-4-benzyloxy-pyrrolidin-3-ylmethyl)-(4-chloro-phenyl)-amine

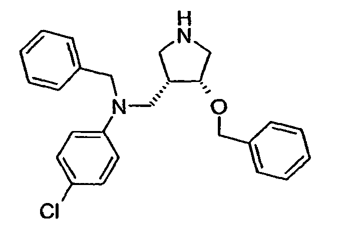

### A. (3R*,4R*)-3-(Benzylamino-methyl)-4-(tert-butyl-dimethyl-silanyloxy)-pyrrolidine-1-carboxylic acid tert-butyl ester

The title compound is prepared according to the procedure described for the synthesis of the title compound under A in Example 15 (Scheme9) starting from (3R*,4R*)-3-(tert-butyldimethyl-silanyloxy)-4-formyl-pyrrolidine-1-carboxylic acid tert-butyl ester and 4-chloroaniline.

### B. (3R*,4R*)-3-{[Benzyl-(4-chloro-phenyl)-amino]-methyl}-4-(tert-butyl-dimethyl-silanyloxy)-pyrrolidine-1-carboxylic acid tert-butyl ester

The title compound is prepared according to the procedure described for the synthesis of title compound under B in Example 15 (Scheme9) using benzylbromide as the alkylating agent.

### C. (3R*,4R*)-3-{[Benzyl-(4-chloro-phenyl)-amino]-methyl}-4-hydroxy-pyrrolidine-1-carboxylic acid tert-butyl ester

The title compound is prepared according to the procedure described for the synthesis of the title compound under E in Example 120 (Scheme23) starting from (3R*,4R*)-3-{[benzyl-(4-chloro-phenyl)-amino]-methyl}-4-(tert-butyl-dimethyl-silanyloxy)-pyrrolidine-1-carboxylic acid tert-butyl ester.

### D. (3R*,4R*)-3-{[Benzyl-(4-chloro-phenyl)-amino]-methyl}-4-benzyloxy-pyrrolidine-1-carboxylic acid tert-butyl ester

The title compound is prepared according to the procedure described for the synthesis of the title compound under F in Example 120 (Scheme23) by O-alkylation of (3R*,4R*)-3-{[benzyl-(4-chloro-phenyl)-amino]-methyl}-4-hydroxy-pyrrolidine-1-carboxylic acid tert-butyl ester.

### E. Benzyl-((3S*,4R*)-4-benzyloxy-pyrrolidin-3-ylmethyl)-(4-chloro-phenyl)-amine

The title compound is prepared according to the procedure described for the synthesis of the title compound under G in Example 120 (Scheme23) by N-Boc-deprotection of (3R*,4R*)-3-{[benzyl-(4-chloro-phenyl)-amino]-methyl}-4-benzyloxy-pyrrolidine-1-carboxylic acid tert-butyl ester.
MS (LC-MS): 407.2 [M+H]⁺; t_{R} (HPLC, C18 column, 20-100% CH₃CN/H₂O/15 min, CH₃CN and H₂O containing 0.1% TFA, flow: 1.5 mL/min): 10.32 min.

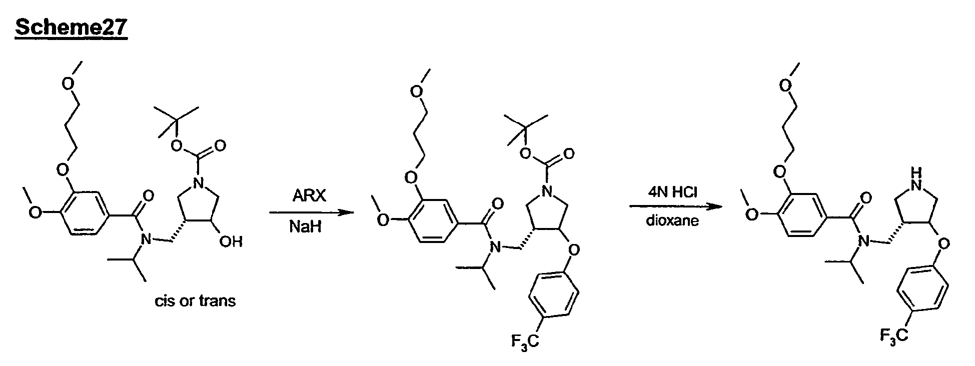

### Example 216: N-Isopropyl-4-methoxy-3-(3-methoxy-propoxy)-N-[(3S*,4S*)-4-(4-trifluoromethyl-phenoxy)-pyrrolidin-3-ylmethyl]-benzamide

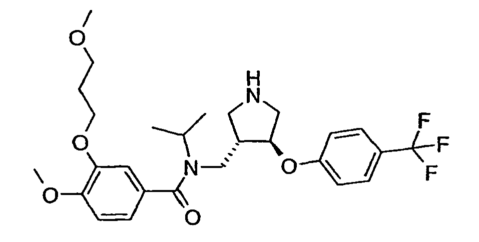

### A. (3R*,45*)-3-({Isopropyl-[4-methoxy-3-(3-methoxy-propoxy)-benzoyl]-amino}-methyl)-4-(4-trifluoromethyl-phenoxy)-pyrrolidine-1-carboxylic acid tert-butyl ester

NaH (18 mg, 0.44 mmol) is added to a solution of (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester (120 mg, 0.25 mmol) in DMF (1 mL), and the resulting suspension is heated at 80°C for 15 min. After cooling to RT, 4-fluorobenzotrifluoride (63 µl, 0.50 mmol) is added and the reaction mixture is stirred at RT for 2 h. For workup, a saturated solution of NaHCO₃ is added and the mixture is extracted with ethyl acetate. Drying (Na₂SO₄) of the combined extracts and evaporation of the solvent affords the crude product which is purified by flash column chromatography (ethyl acetate/hexane 1:1 → 7:3) to give the desired product. MS (LC-MS): 647.23 [M+Na]⁺; t_{R} (HPLC, C18 column, 65-100% CH₃CN/H₂O/15 min, CH₃CN and H₂O containing 0.1% TFA, flow: 1.5 mL/min): 5.02 min.

### B. N-Isopropyl-4-methoxy-3-(3-methoxy-propoxy)-N-[(3S*,4S*)-4-(4-trifluoromethyl-phenoxy)-pyrrolidin-3-ylmethyl]-benzamide

(3R*,4S*)-3-({Isopropyl-[4-methoxy-3-(3-methoxy-propoxy)-benzoyl]-amino}-methyl)-4-(4-trifluoromethyl-phenoxy)-pyrrolidine-1-carboxylic acid tert-butyl ester (72 mg, 0.14 mmol) is treated with 4N HCl/dioxane (3 mL) at RT for 1 h. The solvent is removed under reduced pressure, and the residue is re-dissolved in Et₂O. Evaporation of the solvent yields the mono-hydrochloride of N-isopropyl-4-methoxy-3-(3-methoxy-propoxy)-N-[(3S,4S)-4-(4-trifluoromethyl-phenoxy)-pyrrolidin-3-ylmethyl]-benzamide. MS (LC-MS): 525.3 [M+H]⁺; t_{R} (HPLC, C18 column, 35-100% CH₃CN/H₂O/15 min, CH₃CN and H₂O containing 0.1% TFA, flow: 1.5 mL/min): 5.24 min.

### Example 217: N-Isopropyl-4-methoxy-3-(3-methoxy-propoxy)-N-[(3S*,4R*)-4-(4-trifluoromethyl-phenoxy)-pyrrolidin-3-ylmethyl]-benzamide

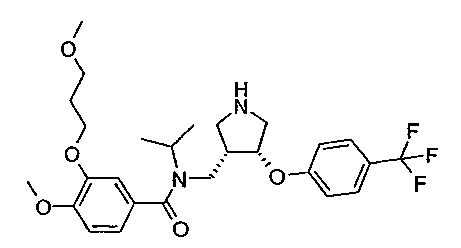

The title compound is prepared analogously as described for the title compound under B in Example 216 (Scheme27) from (3R*,4R*)-3-hydroxy-4-({isopropyl-[4-methoxy-3-(3-methoxy-propoxy)-benzoyl]-amino}-methyl)-pyrrolidine-1-carboxylic acid tert-butyl ester. MS (LC-MS): 525.3 [M+H]⁺; t_{R} (HPLC, C18 column, 35-100% CH₃CN/H₂O/15 min, CH₃CN and H₂O containing 0.1% TFA, flow: 1.5 mL/min): 5.24 min.

### Example 218: Benzyl-(4-chloro-phenyl)-[(3S*,4R*)-4-(4-trifluoromethyl-phenoxy)-pyrrolidin-3-ylmethyl]-amine

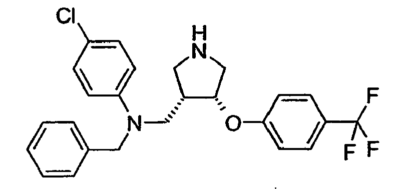

The title compound is prepared analogously as described for the title compound under B in Example 216 (Scheme27) from (3R*,4R*)-3-{[benzyl-(4-chloro-phenyl)-amino]-methyl}-4-hydroxy-pyrrolidine-1-carboxylic acid tert-butyl ester (prepared according to Scheme23). MS (LC-MS): 461 [M+H]⁺; t_{R} (HPLC, C18 column, 20-100% CH₃CN/H₂O/15 min, CH₃CN and H₂O containing 0.1% TFA, flow: 1.5 mL/min): 10.61 min.

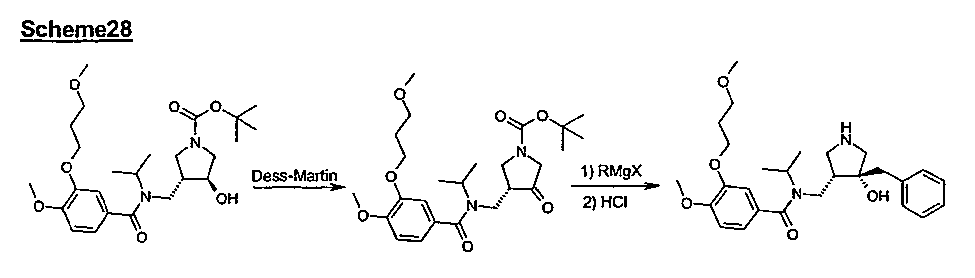

### Example 219: N-((3S*,4R*)-4-Benzyl-4-hydroxy-pyrrolidin-3-ylmethyl)-N-isopropyl-4-methoxy-3-(3-methoxy-propoxy)-benzamide

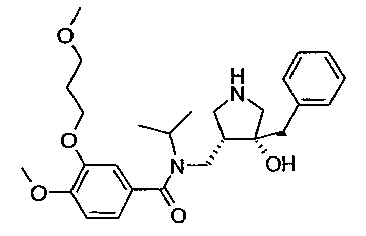

### A. 3-({Isopropyl-[4-methoxy-3-(3-methoxy-propoxy)-benzoyl]-amino}-methyl)-4-oxo-pyrrolidine-1-carboxylic acid tert-butyl ester

Dess-Martin-periodinane (1.9 g, 4.5 mmol) is added to a solution of (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester (1.1 g, 2.25 mmol) in CH₂Cl₂ (30 mL). The reaction mixture is stirred at RT for 4 h, then another portion of Dess-Martin-periodinane (1.0 g, 2.4 mmol) is added. After 2 h, a saturated solution of Na₂S₂O₃ is added, and extraction with ethyl acetate followed by drying of the combined organic extracts (Na₂SO₄) affords the crude product. Purification by flash column chromatography on silica gel (hexane/ethyl acetate 1:4 → ethyl acetate) yields the title product. MS (LC-MS): 479.3 [M+H]⁺; t_{R} (HPLC, C18 column, 35-100% CH₃CN/H₂O/15 min, CH₃CN and H₂O containing 0.1% TFA, flow: 1.5 mL/min): 6.29 min.

### B. N-((3S*,4R*)-4-Benzyl-4-hydroxy-pyrrolidin-3-ylmethyl)-N-isopropyl-4-methoxy-3-(3-methoxy-propoxy)-benzamide

A solution of CeCl₃ (309 mg, 1.25 mmol) in THF (2.5 mL) is stirred at RT for 1 h. Then a solution of benzylmagnesium chloride in Et₂O (1.25 mL, 1.25 mmol, 1.0 M) is added at -75°C, and the resulting mixture is kept stirring at -75°C for 90 min before a solution of 3-({isopropyl-[4-methoxy-3-(3-methoxy-propoxy)-benzoyl]-amino}-methyl)-4-oxo-pyrrolidine-1-carboxylic acid tert-butyl ester (120 mg, 0.25 mmol) in THF (1 mL) is added. After 4 h at -75°C, a saturated solution of NaHCO₃ is added and the mixture is extracted with ethyl acetate. Drying of the combined extracts (Na₂SO₄) and evaporation of the solvent affords the crude product which is purified by flash column chromatography (hexane/ethyl acetate 1:4 → ethyl acetate) to give a mixture of 3-benzyl-3-hydroxy-4-({isopropyl-[4-methoxy-3-(3-methoxy-propoxy)-benzoyl]-amino}-methyl)-pyrrolidine-1-carboxylic acid tert-butyl ester and starting material in a ratio of 1:2. This mixture is treated with 4N HCl/dioxane (3 mL) for 2 h. After evaporation of the solvent, purification by preparative HPLC (RP18 column, 20-100% CH₃CN/H₂O, flow: 3 mL/min) gives N-((3S*,4R*)-4-benzyl-4-hydroxy-pyrrolidin-3-ylmethyl)-N-isopropyl-4-methoxy-3-(3-methoxy-propoxy)-benzamide as a colorless foam. MS (LC-MS): 471.3 [M+H]⁺; t_{R} (HPLC, C18 column, 20-100% CH₃CN/H₂O/15 min, CH₃CN and H₂O containing 0.1% TFA, flow: 1.5 mL/min): 6.79 min.

### Example 220: N-[(3S*,4R*)-4-(2-Fluoro-benzyl)-4-hydroxy-pyrrolidin-3-ylmethyl]-N-isopropyl-4-methoxy-3-(3-methoxy-propoxy)-benzamide

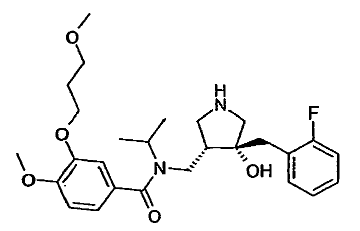

The title compound is prepared analogously as described for the title compound under B in Example 219. MS (LC-MS): 379 [M+H]⁺; t_{R} (HPLC, C18 column, 20-100% CH₃CN/H₂O/15 min, CH₃CN and H₂O containing 0.1% TFA, flow: 1.5 mL/min): 4.86 min.

### Example 221: N-[4-(2-Chloro-6-fluoro-benzyl)-4-hydroxy-pyrrolidin-3-ylmethyl]-N-isopropyl-4-methoxy-3-(3-methoxy-propoxy)-benzamide

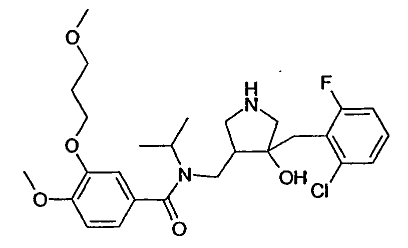

The title compound is prepared analogously as described for the title compound under B in Example 219 (Scheme28) and obtained as a mixture of diastereoisomers. MS (LC-MS): 523.3 [M+H]⁺; t_{R} (HPLC, C18 column, 20-100% CH₃CN/H₂O/15 min, CH₃CN and H₂O containing 0.1% TFA, flow: 1.5 mL/min): 7.52 min.

### Example 222: N-Isopropyl-4-methoxy-3-(3-methoxy-propoxy)-N-((S)-4-oxo-pyrrolidin-3-ylmethyl)-benzamide

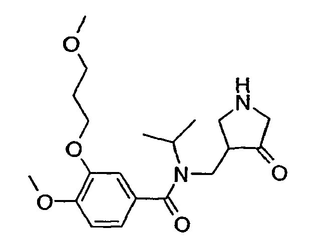

The title compound is prepared by N-Boc-deprotection of 3-({isopropyl-[4-methoxy-3-(3-methoxy-propoxy)-benzoyl]-amino}-methyl)-4-oxo-pyrrolidine-1-carboxylic acid tert-butyl ester analogously as described for the title compound under B in Example 219 (Scheme28). MS (LC-MS): 489.2 [M+H]⁺; t_{R} (HPLC, C18 column, 50-100% CH₃CN/H₂O/15 min, CH₃CN and H₂O containing 0.1% TFA, flow: 1.5 mL/min): 7.01 min.

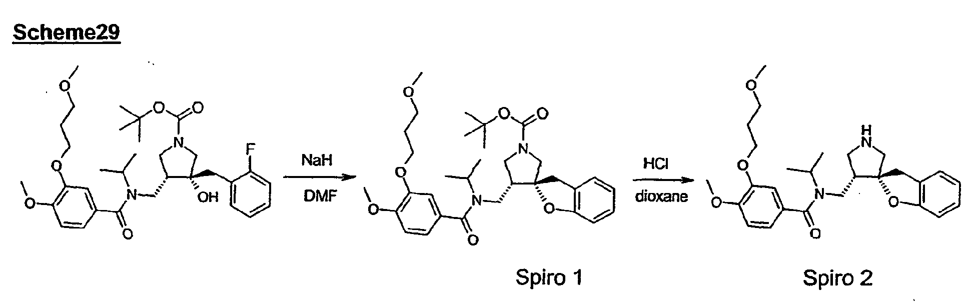

### Example 223:

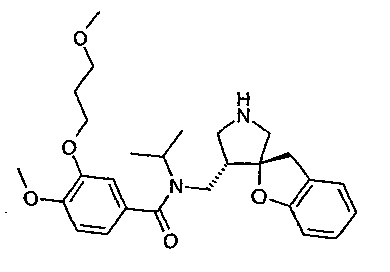

### A. Spiro 1

A suspension of (3R*,4R*)-3-(2-fluoro-benzyl)-3-hydroxy-4-({isopropyl-[4-methoxy-3-(3-methoxy-propoxy)-benzoyl]-amino}-methyl)-pyrrolidine-1-carboxylic acid tert-butyl ester (220 mg, 0.37 mmol) and NaH (36 mg, 0.82 mmol, 65% in mineral oil) in DMF (2 mL) is heated at 110°C. After 2h, 4h and 6h additional NaH (36 mg, 0.82 mmol) is added. For workup, a saturated solution of NaHCO₃ is added, and the resulting mixture is extracted with ethyl acetate. Drying (Na₂SO₄) of the combined extracts and evaporation affords the crude product which is subjected to purification by flash column chromatography (hexane/ethyl acetate 1:1 → 1:4) to give the title product. MS (LC-MS): 569.3 [M+H]⁺; t_{R} (HPLC, C18 column, 50-100% CH₃CN/H₂O/15 min, CH₃CN and H₂O containing 0.1% TFA, flow: 1.5 mL/min): 7.46 min.

### B. Spiro 2

Spiro 1 (75 mg, 0.13 mmol) is treated with 4N HCl/dioxane (2 mL) for 90 min at RT. The solvent is evaporated, and the crude product is purified by preparative HPLC (RP18 column, 25-70% CH₃CN/H₂O, flow: 3 mL/min) to afford the desired product. MS (LC-MS): 469.3 [M+H]⁺; t_{R} (HPLC, C18 column, 20-100% CH₃CN/H₂O/15 min, CH₃CN and H₂O containing 0.1% TFA, flow: 1.5 mL/min): 7.46 min.

### Example 224

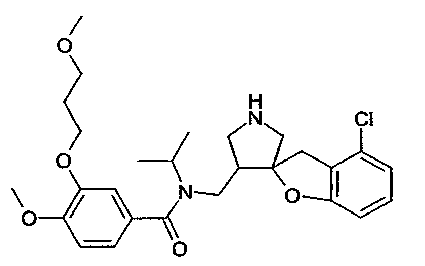

The title compound is prepared analogously as described for the title compound under B in Example 223 (Scheme29) and obtained as a mixture of diastereoisomers. MS (LC-MS): 503.2 [M+H]⁺; t_{R} (HPLC, C18 column, 20-100% CH₃CN/H₂O/15 min, CH₃CN and H₂O containing 0.1% TFA, flow: 1.5 mL/min): 8.06 min and 8.39 min.

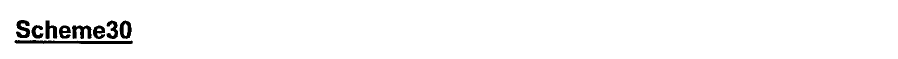

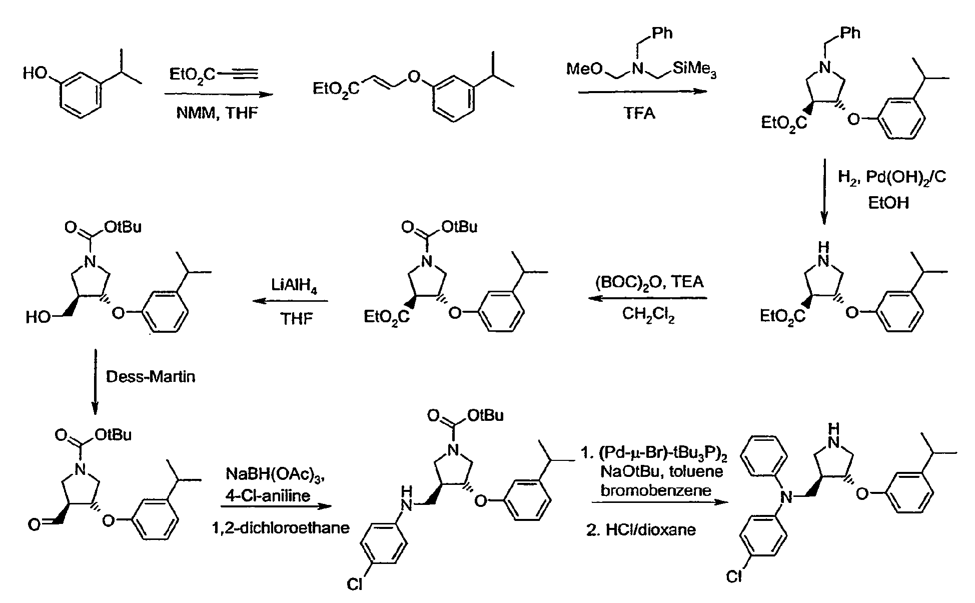

### Example 225: (3R*,4R*)-(4-Chloro-phenyl)-[4-(3-isopropyl-phenoxy)-pyrrolidin-3-ylmethyl]-Phenyl-amine

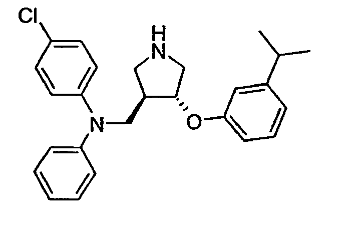

### A. (E)-3-(3-Isopropyl-phenoxy)-acrylic acid ethyl ester

To an ice-cold solution of ethyl propiolate (8.0 mL, 78 mmol) in THF (55 mL), 3-isopropylphenol (7.5 mL, 54 mmol) and NMM (2.4 mL, 22 mmol) are added. The reaction mixture is stirred for 2 h at ambient temperature, cooled to 0°C and quenched by addition of 2N HCl. After extraction with TBME, the organic layer is dried over Na₂SO₄, filtered and concentrated. The crude material is purified by flash chromatography on silica gel (eluent: hexane / EtOAc 19/1) to give the title compound. TLC, Rf (hexane/AcOEt 19/1) = 0.34.
MS(EI+): 235 (M+1)

### B. (3R*,4R*)-1-Benzyl-4-(3-isopropyl-phenoxy)-pyrrolidine-3-carboxylic acid ethyl ester

To an ice-cold solution of (E)-3-(3-isopropyl-phenoxy)-acrylic acid ethyl ester (10.7 g, 46 mmol) in toluene (150 mL), N-benzyl-N-(methoxymethyl) trimethylsilyl amine (20 mL, 78 mmol) is added, followed by trifluoroacetic acid (4.6 mmol, 0.35 mL), and the reaction mixture is stirred at ambient temperature overnight. The reaction is quenched with a saturated aqueous NaHCO₃ solution extracted with CH₂Cl₂, dried over Na₂SO₄ and concentrated. The crude material is purified by flash chromatography on silica gel (eluent : hexane/EtOAc 9/1) to give the title compound as a pale yellow oil. TLC, Rf (hexane/AcOEt 9/1) = 0.25. MS(EI+): 368 (M+1)

### C. (3R*,4R*)-4-(3-Isopropyl-phenoxy)-pyrrolidine-1,3-dicarboxylic acid 1-tert-butyl ester 3-ethyl ester

A mixture of (3R*,4R*)-1-benzyl-4-(3-isopropyl-phenoxy)-pyrrolidine-3-carboxylic acid ethyl ester (17.9 g, 49 mmol), di-tert-butylcarbonate (12.8 g, 58 mmol) and Pd(OH)₂/C (0.17 g, 50% wet) in EtOH (100 mL) is stirred overnight under an hydrogen atmosphere. The crude material is filtered over a pad of Celite, dried over Na₂SO₄ and concentrated. The residue is dissolved in CH₂Cl₂ (250 mL), and TEA (5 mL) followed by di-tert-butylcarbonate (7 g, 32 mmol) are added. After stirring overnight at ambient temperature, the solution is washed with brine, and the organic layer is dried over Na₂SO₄, filtered and concentrated. The crude material is purified by flash chromatography on silica gel (eluent : hexane/EtOAc 4/1) to give the title compound as a pale yellow oil. TLC, Rf (hexane/AcOEt 4/1) = 0.41. MS(EI+): 278 (M-100).

### D. (3R*,4R*)-3-Hydroxymethyl-4-(3-isopropyl-phenoxy)-pyrrolidine-1-carboxylic acid tert-butyl ester

To an ice-cold solution of (3R*,4R*)-4-(3-isopropyl-phenoxy)-pyrrolidine-1,3-dicarboxylic acid 1-tert-butyl ester 3-ethyl ester (3 g, 8 mmol) in THF (150 mL), a 1M-THF solution of LiAlH₄ (8.3 mL, 8.3 mmol) is added. After 10 min at 0°C, the reaction mixture is carefully quenched by addition of AcOEt (10mL) and solid Na₂SO₄-decahydrate. After no more gas evolution is observed, TBME is added and stirring is continued for 1 h. The suspension is filtered and washed thoroughly with TBME. The filtrate is concentrated in vacuo to give the title compound, which is used without further purification. TLC, Rf (CH₂Cl₂/MeOH 9/1) = 0.77
MS(EI+): 335 (M+1).

### E. (3R*,4R*)-3-Formyl-4-(3-isopropyl-phenoxy)-pyrrolidine-1-carboxylic acid tert-butyl ester

To a well stirred mixture of (3R*,4R*)-3-hydroxymethyl-4-(3-isopropyl-phenoxy)-pyrrolidine-1-carboxylic acid tert-butyl ester (1 g, 3 mmol) and Dess-Martin Periodinane (3.8 g, 9 mmol) in CH₂Cl₂ (30 mL), water (0.2 mL) is added. The suspension is stirred overnight, then saturated aqueous NaHCO₃ is added followed by Na₂S₂O₃. After stirring for 20 min, the aqueous layer is extracted with CH₂Cl₂. The organic extract is dried over Na₂SO₄, filtered and concentrated. The crude material is purified by flash chromatography on silica gel (eluent; CH₂Cl₂/MeOH 19/1) to give the title compound. TLC, Rf (CH₂Cl₂/MeOH 19/1) = 0.43. t_{R} (HPLC, Nucleosil C18 column, 20-100% CH₃CN/H₂O/6 min, 100% CH₃CN/1.5 min, 100-20% CH₃CN/H₂O/0.5 min, CH₃CN and H₂O containing 0.1% TFA, flow: 1.0 mL/min): 5.42 min.

### F. (3R*,4R*)-3-[(4-Chloro-phenylamino)-methyl]-4-(3-isopropyl-phenoxy)-pyrrolidine-1-carboxylic acid tert-butyl ester

To an ice-cold solution of (3R*,4R*)-3-formyl-4-(3-isopropyl-phenoxy)-pyrrolidine-1-carboxylic acid tert-butyl ester (0.26 g, 0.7 mmol) and 4-chloro aniline (0.12 g, 0.8 mmol) in 1,2-dichloroethane (85 mL), NaBH(OAc)₃ (0.21 g, 1 mmol) is added. After 30 min, the reaction mixture is warmed up to ambient temperature and stirred for 60 min. The solvent is removed *in vacuo,* and the residue is purified by flash chromatography on silica gel (eluent; CH₂Cl₂) to give the title compound. TLC, Rf (CH₂Cl₂/MeOH 98/2) = 0.75. MS(EI+): 389 (M-55)

### G. (3R*,4R*)-3-{[(4-Chloro-phenyl)-phenyl-amino]-methyl}-4-(3-isopropyl-phenoxy)-pyrrolidine-1-carboxylic acid tert-butyl ester

A two-necked flask, equipped with a magnetic stirring bar, septum and condenser with an argon inlet-outlet is charged with [Pd(µ-Br)(t-Bu₃P)]₂ (8 mg, 5 mol%), NaOtBu (32 mg, 0.34 mmol), (3R*,4R*)-3-[(4-chloro-phenylamino)-methyl]-4-(3-isopropyl-phenoxy)-pyrroli-dine-1-carboxylic acid tert-butyl ester (100 mg, 0.23 mmol) and bromobenzene (42 mg, 0.27 mmol). Abs. toluene (2 mL) is added, and the mixture is stirred for 10 min at RT and heated overnight under a gentle reflux. After cooling to RT, the reaction is quenched with a saturated aqueous NaHCO₃ solution, extracted with AcOEt, dried over MgSO₄ and concentrated. The residue is purified by flash chromatography on silica gel (eluent; CH₂Cl₂) to give the title compound. TLC, Rf (CH₂Cl₂) = 0.65. MS(EI+): 521 (M+).

### H. (3R*,4R*)-(4-Chloro-phenyl)-[4-(3-isopropyl-phenoxy)-pyrrolidin-3-ylmethyl]-phenylamine

To a solution of (3R*,4R*)-3-{[(4-chloro-phenyl)-phenyl-amino]-methyl}-4-(3-isopropyl-phenoxy)-pyrrolidine-1-carboxylic acid tert-butyl ester (0.12 g, 0.24 mmol) in 1,4-dioxane (5mL), 4N HCl (5 mL) in 1,4-dioxane is added. After stirring for 30 min, the solvent is removed *in vacuo,* and the residue is lyophilized overnight to give the title compound as a hydrochloride salt. t_{R} (HPLC, Nucleosil C18 column, 20-100% CH₃CN/H₂O/6 min, 100% CH₃CN/1.5 min, 100-20% CH₃CN/H₂O/0.5 min, CH₃CN and H₂O containing 0.1% TFA, flow: 1.0 mL/min): 5.38 min. MS(EI+): 421 (M+)

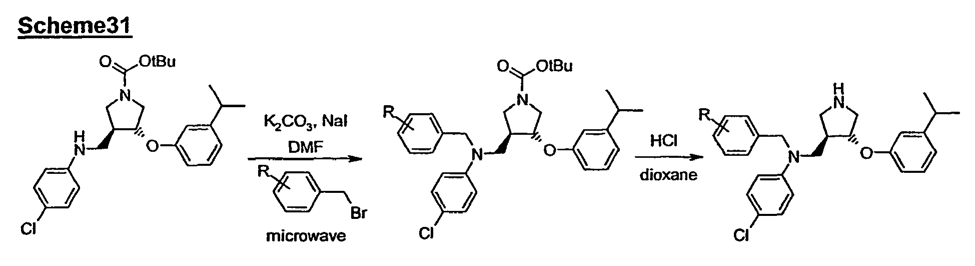

### Example 226: (3R*4R*)-Benzyl-(4-chloro-phenyl)-[4-(3-isopropyl-phenoxy)-pyrrolidin-3-ylmethyl]-amine

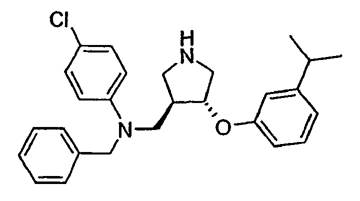

### A. (3R*,4R*)-3-{[Benzyl-(4-chloro-phenyl)-amino]-methyl}-4-(3-isopropyl-phenoxy)-pyrrolidine-1-carboxylic acid tert-butyl ester

A vial is charged with (3R*,4R*)-3-[(4-chloro-phenylamino)-methyl]-4-(3-isopropyl-phenoxy)-pyrrolidine-1-carboxylic acid tert-butyl ester (0.16 g, 0.35 mmol), benzylbromide (0.12 g, 0.71 mmol), K₂CO₃ (0.10 g, 0.71 mmol), sodium iodide (0.11 g, 0.71 mmol) in air and suspended in DMF (6 mL). The vial is sealed with an Al crimp top with septum and heated for 45 min at 120°C in a microwave apparatus (PersonalChemistry). The reaction mixture is diluted with water and AcOEt , and the aqueous layer is extracted with AcOEt. The combined organic extracts are washed with 10% aqueous LiCl, brine, dried over MgSO₄, filtered and concentrated. The residue is purified by flash chromatography on silica gel (eluent; CH₂Cl₂) to give the title compound. TLC, Rf (CH₂Cl₂) = 0.62. MS(EI+): 535 (M⁺).

### B. (3R*,4R*)-Benzyl-(4-chloro-phenyl)-[4-(3-isopropyl-phenoxy)-pyrrolidin-3-ylmethyl]-amine

To a solution of (3R*,4R*)-3-{[benzyl-(4-chloro-phenyl)-amino]-methyl}-4-(3-isopropyl-phenoxy)-pyrrolidine-1-carboxylic acid tert-butyl ester (0.13g, 0.24 mmol) in 1,4-dioxane, 4N HCl (5 mL) in 1,4-dioxane is added. After stirring for 2 h, the solvent is removed *in vacuo,* and the residue is lyophilized overnight to give the title compound as a hydrochloride salt. t_{R} (HPLC, Nucleosil C18 column, 40-100% CH₃CN/H₂O/6 min, 100% CH₃CN/1.5 min, 100-40% CH₃CN/H₂O/0.5 min, CH₃CN and H₂O containing 0.1% TFA, flow: 1.0 mL/min): 4.26 min.
MS(EI+): 435 (M+)

### Example 227: (3R*,4R*)-(4-Chloro-phenyl)-[4-(3-isopropyl-phenoxy)-pyrrolidin-3-ylmethyl]-(2-methoxy-benzyl)-amine

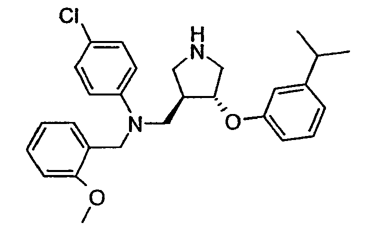

The title compound is prepared analogously as described for the title compound under B in Example 226 (Scheme31). t_{R} (HPLC, Nucleosil C18 column, 40-100% CH₃CN/H₂O/6 min, 100% CH₃CN/1.5 min, 100-40% CH₃CN/H₂O/0.5 min, CH₃CN and H₂O containing 0.1% TFA, flow: 1.0 mL/min): 4.34 min. MS(EI+): 465 (M+).

### Example 228: (3R*4R*)-(4-Chloro-phenyl)-[4-(3-isopropyl-phenoxy)-pyrrolidin-3-ylmethyl]-(3-methoxy-benzyl)-amine

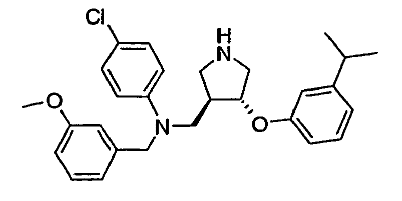

The title compound is prepared analogously as described for the title compound under B in Example 226 (Scheme31). t_{R} (HPLC, Nucleosil C18 column, 40-100% CH₃CN/H₂O/6 min, 100% CH₃CN/ 1.5 min, 100-40% CH₃CN/H₂O/0.5 min, CH₃CN and H₂O containing 0.1% TFA, flow: 1.0 mL/ min): 4.16 min. MS(EI+): 465 (M+).

### Example 229: (3R*,4R*)-Benzo[1,2,5]oxadiazol-5-ylmethyl-(4-chloro-phenyl)-[4-(3-isopropyl-phenoxy)-pyrrolidin-3-ylmethyl]-amine

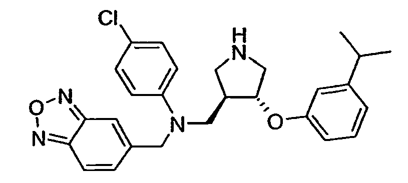

The title compound is prepared analogously as described for the title compound under B in Example 226 (Scheme31). t_{R} (HPLC, Nucleosil C18 column, 20-100% CH₃CN/H₂O/6 min, 100% CH₃CN/ 1.5 min, 100-20% CH₃CN/H₂O/0.5 min, CH₃CN and H₂O containing 0.1% TFA, flow: 1.0 mL/ min): 5.01 min. MS(EI+): 477 (M+).

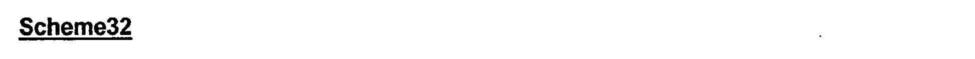

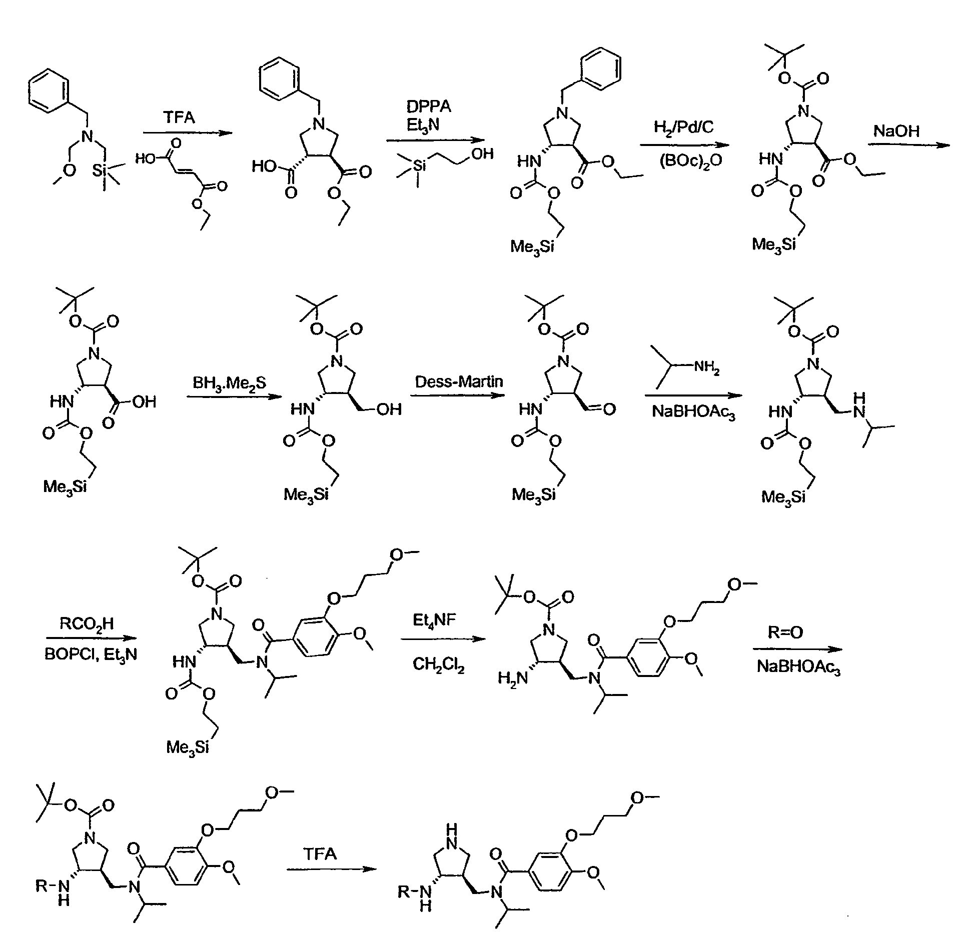

### Example 230: N-((3R*,4R*)-4-Cyclohexylamino-pyrrolidin-3-ylmethyl)-N-isopropyl-4-methoxy-3-(3-methoxy-propoxy)-benzamide

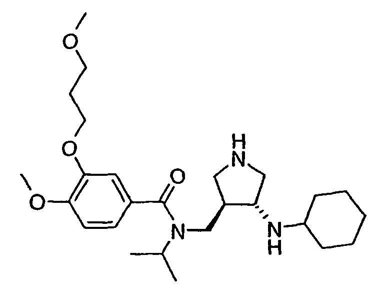

### A. (3S*,4S*)-1-Benzyl-pyrrolidine-3,4-dicarboxylic acid monoethyl ester

To a stirred solution of mono ethyl fumarate (2.85 g, 19.8 mmol) and trifluoroacetic acid (1.98 mmol, 0.15 mL) in methylene chloride (50 mL), N-benzyl-N-(methoxymethyl) trimethylsilyl amine ( 9.41 g, 39.6 mmol) is added at 0°C under N₂. The mixture is stirred at 0°C for 30 min and then at RT over 48 h. The crude material is concentrated and purified by flash chromatography on silica gel (eluent: CH₂Cl₂/MeOH 85/15 to 85/15+2% NH₄OH) to give the title compound. MS (LC-MS): 278.0 [M+H]⁺; ¹H NMR (CD₃OD, 400 MHz): δ = 1.29 (t, 3H), 3.28 (m, 5H), 3.60 (m, 1H), 4.07 (m, 2H), 4.20 (m, 2H), 7.47 (m, 5H).

### B. (3R*,4S*)-1-Benzyl-4-(2-trimethylsilanyl-ethoxycarbonylamino)-pyrrolidine-3-carboxylic acid ethyl ester

A mixture of (3S*,4S*)-1-benzyl-pyrrolidine-3,4-dicarboxylic acid monoethyl ester (3 g, 10.8 mmol) , DPPA (2.34 mL, 10.8 mmol), Et₃N (1.5 mL, 10.8 mmol) and trimethylsilyl ethanol (1.85 mL, 12.98 mmol) in dioxane (30 mL) is stirred at reflux for 48 h. The mixture is poured into an aqueous saturated solution of NaHCO₃ (30mL) and extracted 3 times with CH₂Cl₂ (50 mL). The combined organic layers are dried over Na₂SO₄, filtered and concentrated. Chromatography on silica gel (eluent: CH₂Cl₂/MeOH 98/2 to 96/4) gives the title compound. MS (LC-MS): 393.0 [M+H]⁺; ¹H NMR (CD₃OD, 400 MHz): δ = 0.5 (s, 9H), 1.0 (t, 2H), 1.27 (t, 3H), 2.53 (dd, 1H), 2.75 (m, 1H), 2.85-2.97 (m, 3H), 3.59-3.72 (m, 2H), 4.10-4.20 (m, 4H), 4.42 (m, 1H), 7.35 (m, 5H).

### C. (3R*,4S*)-4-(2-Trimethylsilanyl-ethoxycarbonylamino)-pyrrolidine-1,3-dicarboxylic acid 1-tert-butyl ester -ethyl ester

A mixture of (3R*,4S*)-1-benzyl-4-(2-trimethylsilanyl-ethoxycarbonylamino)-pyrrolidine-3-carboxylic acid ethyl ester (2.91 g, 10.5 mmol), di-tert-butylcarbonate (2.29 g, 10.5 mmol) and Pd(OH)₂/C (600 mg, 50% wet) in EtOH (60 mL) is stirred under an hydrogen atmosphere for 5 h. The crude material is filtered over a pad of Celite and concentrated. The crude material is purified by flash chromatography on silica gel (eluent: c-hexane/AcOEt 90/10 to 70/30) to give the title compound. TLC, Rf (c-hexane/AcOEt 50/50) = 0.55. t_{R} (HPLC, C18 column, 20-100% CH₃CN/H₂O/15 min, CH₃CN and H₂O containing 0.1% TFA, flow: 1.5 mL/min): 5.55 min.

### D. (3R*,4S*)4-(2-Trimethylsilanyl-ethoxycarbonylamino)-pyrrolidine-1,3-dicarboxylic acid 1-tert-butyl ester

To a solution of (3R*,4S*)-4-(2-trimethylsilanyl-ethoxycarbonylamino)-pyrrolidine-1,3-dicarboxylic acid 1-tert-butyl ester -ethyl ester (0.87 g, 2.16 mmol) in MeOH (18 mL), 3.24 mL of a solution of 1 N LiOH is added. The reaction mixture is stirred overnight. The mixture is concentrated to dryness and taken up in CH₂Cl₂, an aqueous HCL (5%) solution is added, and the mixture is extracted 3 times with CH₂Cl₂ (3 x 15 mL), dried over Na₂SO₄, filtered and concentrated. The crude material is used in the next step without purification. TLC, Rf (c-hexane/AcOEt 50/50) = 0.4. MS (LC-MS): 373.1 [M-H]⁻.

### E. (3R*,4S*)-3-Hydroxymethyl-4-(2-trimethylsilanyl-ethoxycarbonylamino)-pyrrolidine-1-carboxylic acid tert-butyl ester

To a solution of (3R*,4S*)4-(2-trimethylsilanyl-ethoxycarbonylamino)-pyrrolidine-1,3-dicarboxylic acid 1-tert-butyl ester (0.743 g, 1.98 mmol) in THF (10 mL), a solution of borane dimethylsulfide complex (2N in THF, 0.99 mL, 1.98 mmol) is slowly added at -10°C. The mixture is stirred for 20 min at -10°C, then allowed to reach RT and further stirred 4 h. MeOH is carefully added (exothermic!), and the mixture is concentrated under reduced pressure. Then MeOH is added, and the mixture is again concentrated. This operation is repeated 3 times, and the mixture is finally taken up into a saturated aqueous solution of NaHCO₃, then extracted 3 times with CH₂Cl₂. The combined organic extracts are dried over Na₂SO₄, filtered and concentrated. The crude material is purified by flash chromatography on silica gel (eluent: CH₂Cl₂/MeOH 98/2) to give the desired product. ¹H NMR (CD₃OD, 400 MHz): δ = 0.5 (s, 9H), 1.02 (t, 2H), 1.49 (s, 9H), 2.27 (m, 1H), 3.13 (m, 1H), 3.22 (m, 1H), 3.52-3.73 (m, 5H), 3.99 (m, 1H), 4.18 (q, 2H).

### F. (3R*,4S*)-3-Formyl-4-(2-trimethylsilanyl-ethoxycarbonylamino)-pyrrolidine-1-carboxylic acid tert-butyl ester

To a well stirred mixture of (3R*,4S*)-3-hydroxymethyl-4-(2-trimethylsilanyl-ethoxycarbonylamino)-pyrrolidine-1-carboxylic acid tert-butyl ester (0.416 g, 1.15 mmol) and Dess-martin periodinane (0.49 g, 1.15 mmol) in CH₂Cl₂ (10 mL), slowly wet CH₂Cl₂ (0.021 mL of water in 10 mL of CH₂Cl₂) is added. The clear solution becomes cloudy toward the end of wet CH₂Cl₂ addition. The mixture is diluted with Et₂O, then concentrated to a few mL of solvent by rotary evaporation. The residue is taken up in Et₂O (20 mL) and then washed with 50 mL of 1/1 10% Na₂S₂O₃ saturated aqueous NaHCO₃, followed by 50 mL of H₂O and 50 mL of brine. The aqueous washings are back-extracted with 200 mL of Et₂O, and this organic layer is washed with H₂O and brine. The combined organic layers are dried with Na₂SO₄, filtered and concentrated. The crude mixture is purified by flash chromatography on silica gel (eluent: CH₂Cl₂/MeOH 98/2 to 95/5) to give the title product. TLC, Rf (c-hexane/AcOEt 1/2) = 0.45. MS (LC-MS): 357.0 [M-H]⁻

### G. (3R*,4S*)-3-(Isopropylamino-methyl)-4-(2-trimethylsilanyl-ethoxycarbonylamino)-pyrrolidine-1-carboxylic acid tert-butyl ester

A solution of (3R*,4S*)-3-formyl-4-(2-trimethylsilanyl-ethoxycarbonylamino)-pyrrolidine-1-carboxylic acid tert-butyl ester (2.450 g, 6.83 mmol) and isopropylamine (645µL, 7.52 mmol) in 1,2-dichloroethane (70 mL) is stirred 20 min, before the addition of NaHB(OAc)₃ (2.028 g, 9.57 mmol) follows. The solution is stirred for 4 h, then diluted with CH₂Cl₂ and washed with an aqueous saturated solution of NaHCO₃. The combined organic extracts are dried over Na₂SO₄ and concentrated to give the title product. The compound is used in the next step without purification. MS (LC-MS): 402.1 [M+H]⁺; ¹H NMR (CD₃OD, 400 MHz): δ = 0.6 (s, 9H), 1.0 (t, 2H), 1.08 (d, 6H), 1.46 (s, 9H), 2.22 (m, 1H), 2.57 (m, 1H), 2.72-2.85 (m, 2H), 3.07 (m, 2H), 3.67 (m, 2H), 3.89 (m, 1H), 4.15 (t, 2H).

### H. (3R*,4S*)3-({Isopropyl-[4-methoxy-3-(3-methoxy-propoxy)-benzoyl]-amino}-methyl)-4-(2-trimethylsilanyl-ethoxycarbonylamino)-pyrrolidine-1-carboxylic acid tert-butyl ester

A mixture of (3R*,4S*)-3-(isopropylamino-methyl)-4-(2-trimethylsilanyl-ethoxycarbonyl amino)-pyrrolidine-1-carboxylic acid tert-butyl ester (2.51 g, 6.25 mmol), 3-(3-methoxy-propoxy)-4-methoxy-benzoic acid (1.65 g, 6.87 mmol), bis(2-oxo-3-oxazolidinyl)phosphinic chloride (1.99 g, 7.81 mmol) and triethylamine (3.48 mL, 25.0 mmol) in CH₂Cl₂ (110 mL) is refluxed for 2 h and then quenched by the addition of aqueous NaHCO₃ solution. The organic layer is separated, and the aqueous phase is extracted 3 times with CH₂Cl₂. The combined organic extracts are dried (Na₂SO₄), and the solvent is removed *in vacuo.* The crude product is purified by flash chromatography on silica gel (eluent : CH₂Cl₂/MeOH 100/0 to 95/5) to give the title product. t_{R} (HPLC, C18 column, 10-100% CH₃CN/H₂O/5 min, 100% CH₃CN/3 min, 100-10% CH₃CN/H₂O/3 min, CH₃CN and H₂O containing 0.1% TFA, flow: 1.5 mL/min): 6.35 min.

### I. (3S*,4R*)-3-Amino-4-({isopropyl-[4-methoxy-3-(3-methoxy-propoxy)-benzoyl]-amino}-methyl)-pyrrolidine-1-carboxylic acid tert-butyl ester

A mixture of (3R*,4S*)3-({isopropyl-[4-methoxy-3-(3-methoxy-propoxy)-benzoyl]-amino}-methyl)-4-(2-trimethylsilanyl-ethoxycarbonylamino)-pyrrolidine-1-carboxylic acid tert-butyl ester (3.85 g, 6.17 mmol) and tetraethylammonium fluoride hydrate (2.76 g, 18.5 mmol) is refluxed in CH₃CN (85 mL) for 4 h. The mixture is poured into a saturated aqueous solution of NaHCO₃ (10 mL), extracted with EtOAc (3 x 20 mL), dried over Na₂SO₄ and concentrated. Chromatography on silica gel (eluent: CH₂Cl₂/MeOH 100/0 to 90/10) of the crude material gives the title compound. TLC, Rf (CH₂Cl₂/MeOH/NH₄OH 90/10/1) = 0.35. MS (LC-MS): 480.0 [M+H]⁺

### J. (3S*,4R*)-3-Cyclohexytamino-4-({isopropyl-[4-methoxy-3-(3-methoxy-propoxy)-benzoyl]-amino}-methyl)-pyrrolidine-1-carboxylic acid tert-butyl ester

To a solution of (3S*,4R*)-3-amino-4-({isopropyl-[4-methoxy-3-(3-methoxy-propoxy)-benzoyl]-amino}-methyl)-pyrrolidine-1-carboxylic acid tert-butyl ester (200 mg, 0.417 mmol) in 1,2-dichloroethane (3 mL), cyclohexanone (45 mg, 0.459 mmol) is added. The mixture is stirred for 30 min, before the addition of NaBH(OAc)₃ (124 mg, 0.584 mmol) follows. The mixture is further stirred for 4 h, diluted with CH₂Cl₂, washed with an aqueous saturated solution of NaHCO₃, dried over Na₂SO₄, filtered and concentrated. The crude product is used in the next step without purification. TLC, Rf (CH₂Cl₂/MeOH 98/2) = 0.2. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.93 min.

### K. N-((3R*,4R*)-4-Cyclohexylamino-pyrrolidin-3-ylmethyl)-N-isopropyl-4-methoxy-3-(3-methoxy-propoxy)-benzamide

To a solution of (3S*,4R*)-3-cyclohexylamino-4-({isopropyl-[4-methoxy-3-(3-methoxy-propoxy)-benzoyl]-amino}-methyl)-pyrrolidine-1-carboxylic acid tert-butyl ester (225 mg, 0.40 mmol) in 3 mL CH₂Cl₂, TFA (463 µL, 6.01 mmol) is added. The mixture is stirred at RT for 1 h and poured into a saturated solution of NaHCO₃. The layers are separated, and the aqueous one is back-extracted twice with CH₂Cl₂. The combined organic extracts are dried over Na₂SO₄, filtered and concentrated. The crude material is purified by flash chromatography on silica gel (eluent: CH₂Cl₂/MeOH 100/0 to 90/10+1% NH₄OH) to give the title product. To a solution of the free base (135 mg, 0.585 mmol) in dioxane (2 mL), (146 µL, 0.585 mmol) of 4N HCl in dioxane is added, and the resulting solution is lyophilized to afford the corresponding hydrochloride salt as a white powder. MS (LC-MS): 462.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 3.57 min.

### Example 231: N-Isopropyl-N-((3R*,4R*)-4-isopropylamino-pyrrolidin-3-ylmethyl)-4-methoxy-3-(3-methoxy-propoxy)-benzamide

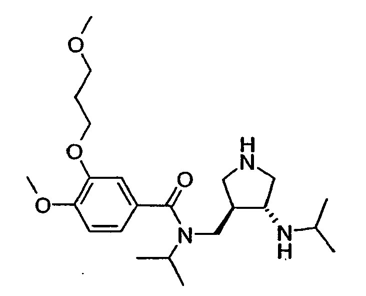

The title compound is prepared analogously as described for the title compound under K in Example 230 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and acetonee. MS (LC-MS): 422.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 3.58 min.

### Example 232: N-((3R*,4R*)-4-Benzylamino-pyrrolidin-3-ylmethyl)-N-isopropyl-4-methoxy-3-(3-methoxy-propoxy)-benzamide

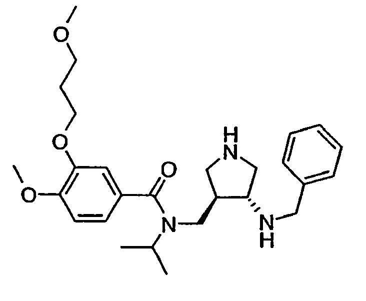

The title compound is prepared analogously as described for the title compound under K in Example 230 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and benzaldehyde.
MS (LC-MS): 470.1 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 3.91 min.

### Example 233: N-Isopropyl-4-methoxy-3-(3-methoxy-propoxy)-N-[(3R*,4R*)-4-(1-phenyl-ethylamino)-pyrrolidin-3-ylmethyl]-benzamide

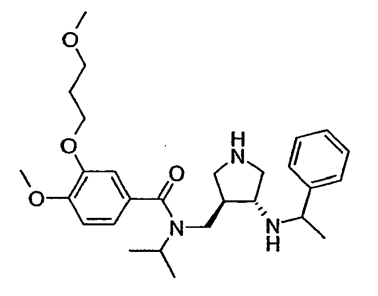

The title compound is prepared analogously as described for the title compound under K in Example 230 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and acetophenone. MS (LC-MS): 484.0 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 4.04 min.

### Example 234: N-((3R*,4R*)4-Isobutylamino-pyrrolidin-3-ylmethyl)-N-isopropyl-4-methoxy-3-(3-methoxy-propoxy)-benzamide

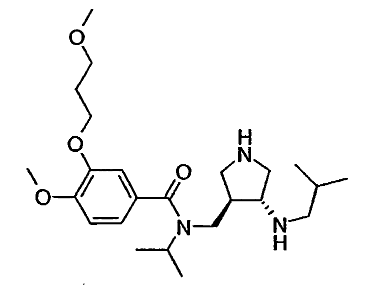

The title compound is prepared analogously as described for the title compound under K in Example 230 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and isobutyraldehyde. MS (LC-MS): 436.1 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 3.78 min.

### Example 235: N-((3R*,4R*)-4-Cyclobutylamino-pyrrolidin-3-ylmethyl)-N-isopropyl-4-methoxy-3-(3-methoxy-propoxy)-benzamide

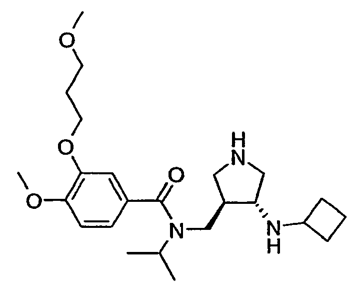

The title compound is prepared analogously as described for the title compound under K in Example 230 from (3S*,4R*)-3-amino-4-({isopropyl-[4-methoxy-3-(3-methoxy-propoxy)-benzoyl]-amino}methyl)-pyrrolidine-1-carboxylic acid tert-butyl ester and cyclobutanone. MS (LC-MS): 434.1 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 3.67 min.

### Example 236: N-((3R*,4R*)-4-Cyclopentylamino-pyrrolidin-3-ylmethyl)-N-isopropyl-4-methoxy-3-(3-methoxy-propoxy)-benzamide

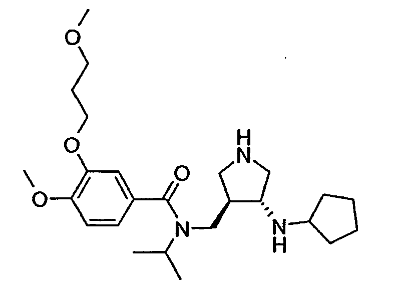

The title compound is prepared analogously as described for the title compound under K in Example 230 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and cyclopentanone. MS (LC-MS): 448.1 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 3.80 min.

### Example 237: N-[(3R*,4R*)-4-(Cyclopentylmethyl-amino)-pyrrolidin-3-ylmethyl]-N-isopropyl-4-methoxy-3-(3-methoxy-propoxy)-benzamide

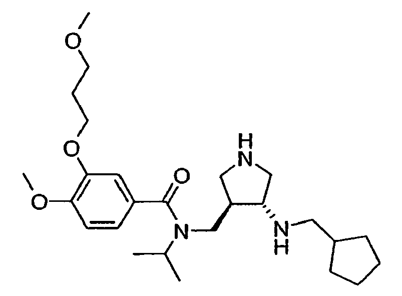

The title compound is prepared analogously as described for the title compound under K in Example 230 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and cyclopentane carboxaldehyde. MS (LC-MS): 462.1 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 4.0 min.

### Example 238: N-Isopropyl-4-methoxy-3-(3-methoxy-propoxy)-N-((3R*,4R*)-4-phenethylamino-pyrrolidin-3-ylmethyl)-benzamide

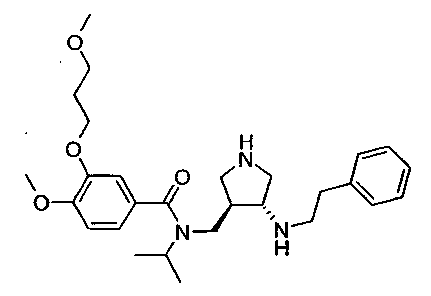

The title compound is prepared analogously as described for the title compound under K in Example 230 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and phenylacetaldehyde. MS (LC-MS): 484.1 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 4.04 min.

### Example 239: N-[(3S*,4S*)-4-(1-Benzyl-pyrrolidin-3-ylamino)-pyrrolidin-3-ylmethyl]-N-isopropyl-4-methoxy-3-(3-methoxy-propoxy)-benzamide

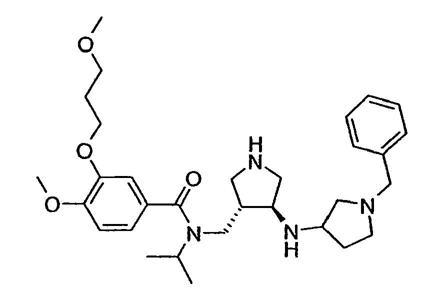

The title compound is prepared analogously as described for the title compound under K in Example 230 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and 1-benzyl-pyrrolidin-3-one. MS (LC-MS): 539.2 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 3.91 min.

### 1-benzyl-pyrrolidin-3-one

A solution of dimethyl sulfoxide (5 mL, 71.1 mmol) in dry CH₂Cl₂ (10.8 mL) is added dropwise to a stirred solution of oxalyl chloride (4.73 mg, 37.2 mmol) in dry CH₂Cl₂ (15 mL) at -78°C under N₂ atmosphere. After 15 min, a solution of 1-benzyl-3-pyrrolidinol (3 g, 16.7 mmol) in dry CH₂Cl₂ (23 mL) is added slowly, and stirring is continued for 30 min at -78°C. After addition of triethylamine (23.3 mL, 167 mmol), the mixture is gradually allowed to reach room temperature. The mixture is quenched with water and the aqueous layer is separated and back-extracted with CH₂Cl₂. The combined organic extracts are washed with brine, dried over Na₂SO₄, filtered and concentrated to give the title compound as a brown oil. MS (LC-MS): 176.1 [M+H]⁺.

### Example 240: N-{(35*,45*)-4-[(1H-Benzoimidazol-5-ylmethyl)-amino]-pyrrolidin-3-ylmethyl}-N-isopropyl-4-methoxy-3-(3-methoxy-propoxy)-benzamide

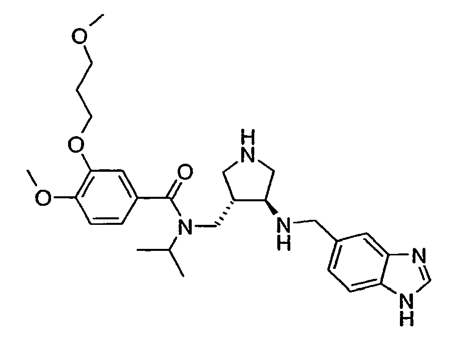

The title compound is prepared analogously as described for the title compound under K in Example 230 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and 1H-benzoimidazole-5-carbaldehyde (prepared according to J. Med. Chem., 1995, 38, 3638). MS (LC-MS): 510 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.78 min.

### Example 241: N-[(3R*,4R*)-4-(Bis-cyclopentylmethyl-amino)-pyrrolidin-3-ylmethyl]-N-isopropyl-4-methoxy-3-(3-methoxy-propoxy)-benzamide

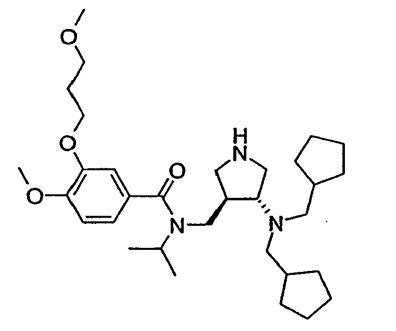

The title compound is prepared analogously as described for the title compound under K in Example 230 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and cyclopentane carboxaldehyde (2 equivalents with respect to the primary amine). MS (LC-MS): 544.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.54 min.

### Example 242: N-[(3R*,4R*)-4-(Benzyl-methyl-amino)-pyrrolidin-3-ylmethyl]-N-isopropyl-4-methoxy-3-(3-methoxy-propoxy)-benzamide

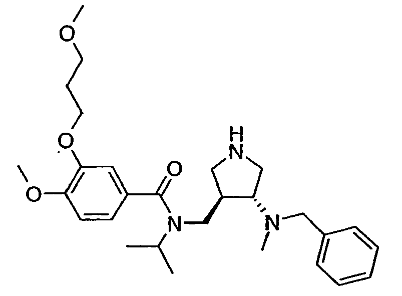

### A. (3R*,4S*)-3-Benzylamino-4-({isopropyl-[4-methoxy-3-(3-methoxy-propoxy)-benzoyl]-amino}-methyl)-pyrrolidine-1-carboxylic acid tert-butyl ester

To a solution of (3S*,4R*)-3-amino-4-({isopropyl-[4-methoxy-3-(3-methoxy-propoxy)-benzoyl]-amino}-methyl)-pyrrolidine-1-carboxylic acid tert-butyl ester (500 mg, 1.04 mmol) in 1,2-dichloroethane (13 mL), benzaldehyde (122 mg, 1.15 mmol) is added. The mixture is stirred for 30 min, before the addition of NaBH(OAc)₃ (309 mg, 1.46 mmol). The mixture is further stirred for 4 h, diluted with CH₂Cl₂, washed with an aqueous saturated solution of NaHCO₃, dried over Na₂SO₄, filtered and concentrated. The crude mixture is purified by flash chromatography on silica gel (eluent: c-hexane/AcOEt 50/50 to 0/100) to give the title product. TLC, Rf (AcOEt) = 0.15. MS (LC-MS): 570.1 [M+H]⁺. t_{R} (HPLC, Nucleosil C18 column, 10-100% CH3CN/H2O/5 min, 100% CH3CN/3 min, flow: 1.5 mL/min): 7.05 min.

### B. (3R*,4S*)-3-(Benzyl-methyl-amino)-4-({isopropyl-[4-methoxy-3-(3-methoxy-propoxy)-benzoyl]-amino}-methyl)-pyrrolidine-1-carboxylic acid tert-butyl ester

(3R*,4S*)-3-Benzylamino-4-({isopropyl-[4-methoxy-3-(3-methoxy-propoxy)-benzoyl]-amino}-methyl)-pyrrolidine-1-carboxylic acid tert-butyl ester (150 mg, 0.263 mmol) and formaldehyde 37% in water (22 µL, 0.289 mmol) are mixed in 1,2-dichloroethane (3 mL) and treated with sodium triacetoxyborohydride (78 mg, 0.368 mmol). The reaction mixture is stirred at RT under nitrogen overnight and poured into an aqueous saturated solution of NaHCO₃. The layers are separated and the aqueous one extracted twice with CH₂Cl₂. The combined organic extracts are dried over Na₂SO₄, filtered and concentrated. The crude material is purified by flash chromatography on silica gel (eluent: c-hexane/AcOEt 50/50 then 0/100) to give the title compound. MS (LC-MS): 584.1 [M+H]⁺. t_{R} (HPLC, Nucleosil C18 column, 10-100% CH₃CN/H₂O/5 min, 100% CH₃CN/3 min, flow: 1.5 mL/min): 5.05 min.

### C. N-[(3R*,4R*)-4-(Benzyl-methyl-amino)-pyrrolidin-3-ylmethyl]-N-isopropyl-4-methoxy-3-(3-methoxy-propoxy)-benzamide

To a solution of (3R*,4S*)-3-(benzyl-methyl-amino)-4-({isopropyl-[4-methoxy-3-(3-methoxy-propoxy)-benzoyl]-amino}-methyl)-pyrrolidine-1-carboxylic acid tert-butyl ester (165 mg, 0.283 mmol) in 3 mL CH₂Cl₂, TFA (261 µL, 3.4 mmol) is added. The mixture is stirred at RT for 1 h and poured into a saturated solution of NaHCO₃. The layers are separated, and the aqueous one is back-extracted twice with CH₂Cl₂. The combined organic extracts are dried over Na₂SO₄, filtered and concentrated. The crude material is purified by HPLC preparative (C18-ODS-AQ 5 µm, 20x50 mm, eluent: CH₃CN /H₂O + 0.1 % HCOOH). The HPLC fractions are collected, diluted with AcOEt and neutralized with a saturated aqueous solution of NaHCO₃. The combined organic layers are dried over Na₂SO₄, filtered and concentrated to give the title product. To a solution of the free base (100 mg, 0.207 mmol) in dioxane (3 mL), (129 µL, 0.517 mmol) of 4N HCl in dioxane is added, and the resulting solution is lyophilized to afford the corresponding dihydrochloride salt as a white powder. MS (LC-MS): 484.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 3.94 min.

### Example 243: N-Isopropyl-4-methoxy-3-(3-methoxy-propoxy)-N-{(3R* 4R*)-4-[(naphthalen-2-ylmethyl)-amino]-pyrrolidin-3-ylmethyl}-benzamide

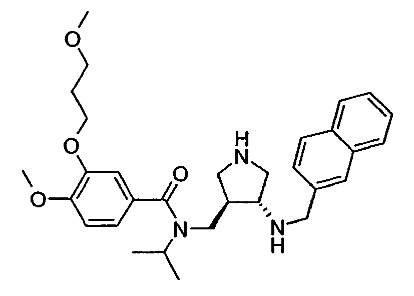

### A. (3S*,4R*)-3-({Isopropyl-[4-methoxy-3-(3-methoxy-propoxy)-benzoyl]-amino}-methyl)-4-[(naphthalen-2-ylmethyl)-amino]-pyrrolidine-1-carboxylic acid tert-butyl ester

A mixture (3S*,4R*)-3-amino-4-({isopropyl-[4-methoxy-3-(3-methoxy-propoxy)-benzoyl]-amino}-methyl)-pyrrolidine-1-carboxylic acid tert-butyl ester (90 mg, 0.188 mmol), (2-bromomethyl)naphthalene (50 mg, 0.225 mmol) and Na₂CO₃ (29 mg, 0.225 mmol) in DMF (2 mL) is stirred under Argone at RT overnight. H₂O is added, and the mixture is extracted with diethyl ether. The combined organic layers are dried (Na₂SO₄), filtered and concentrated to afford the crude product which is use in the next step without prior purification. MS (LC-MS): 620.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.33 min.

### B. N-Isopropyl-4-methoxy-3-(3-methoxy-propoxy)-N-{(3R*,4R*)-4-[(naphthalen-2-ylmethyl)-amino]-pyrrolidin-3-ylmethyl}-benzamide

To a solution of (3S*,4R*)-3-({isopropyl-[4-methoxy-3-(3-methoxy-propoxy)-benzoyl]-amino}-methyl)-4-[(naphthalen-2-ylmethyl)-amino]-pyrrolidine-1-carboxylic acid tert-butyl ester (165 mg, 0.25 mmol) in 3 mL CH₂Cl₂, TFA (205 µL, 2.5 mmol) is added. The mixture is stirred at RT for 2 h and poured into a saturated solution of NaHCO₃. The layers are separated, and the aqueous one is back-extracted twice with CH₂Cl₂. The combined organic extracts are dried over Na₂SO₄, filtered and concentrated. The crude material is purified by preparative HPLC (C18-ODS-AQ 5 µm, 20x50 mm, eluent: CH₃CN /H₂O + 0.1 % HCOOH). To a solution of the free base (60 mg, 0.115 mmol) in dioxane (3 mL), (72 µL, 0.289 mmol of 4N HCl in dioxane is added, and the resulting solution is lyophilized to afford the corresponding hydrochloride salt as a white powder. MS (LC-MS): 520.0 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 4.28 min.

### Example 244: N-Isopropyl-4-methoxy-3-(3-methoxy-propoxy)-N-{(3R,4R)-4-[(naphthalen-2-ylmethyl)-amino]-pyrrolidin-3-ylmethyl}-benzamide

### Example 245: N-Isopropyl-4-methoxy-3-(3-methoxy-propoxy)-N-{(3S,4S)-4-[(naphthalen-2-ylmethyl)-amino]-pyrrolidin-3-ylmethyl}-benzamide

The two enantiomers are separated via chiral preparative HPLC (Chiralpak AD 30 x 250 mm, flow rate 180 g /min, UV = 230 nM, injection = 50 mg in 2 mL ethanol) (eluent: methanol): N-Isopropyl-4-methoxy-3-(3-methoxy-propoxy)-N-{(3R,4R)-4-[(naphthalen-2-ylmethyl)-amino]-pyrrolidin-3-ylmethyl}-benzamide: t_{R} (Chiralpak AD 30 x 250 mm, flow rate 180 g /min, UV = 230 nM, injection = 50 mg in 2 mL ethanol) (eluent: methanol): 364 s. N-Isopropyl-4-methoxy-3-(3-methoxy-propoxy)-N-{(3S,4S)-4-[(naphthalen-2-ylmethyl)-amino]-pyrrolidin-3-ylmethyl}-benzamide: t_{R} (Chiralpak AD 30 x 250 mm, flow rate 180 g /min, UV = 230 nM, injection = 50 mg in 2 mL ethanol) (eluent: methanol): 580 s.

### Example 246: 4-Ethyl-N-isopropyl-3-(3-methoxy-propoxy)-N-{(3R*,4R*)-4-[(naphthalen-2-ymethyl)-amino]-pyrrolidin-3-ylmethyl}-benzamide

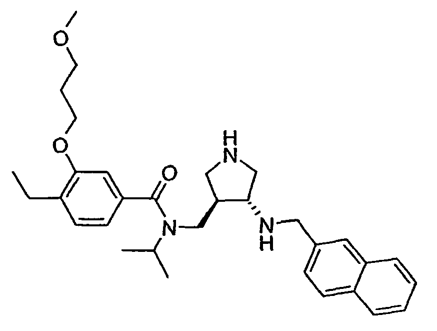

### A. (3S*,4R*)-3-Amino-4-({[4-ethyl-3-(3-methoxy-propoxy)-benzoyl]-isopropyl-amino}-methyl)-pyrrolidine-1-carboxylic acid tert-butyl ester

The title compound is prepared analogously as described for the title compound under I in Example 230 from (3R*,4S*)-3-(isopropylamino-methyl)-4-(2-trimethylsilanyl-ethoxycarbonyl amino)-pyrrolidine-1-carboxylic acid tert-butyl ester and 4-ethyl-3-(3-methoxy-propoxy)-benzoic acid (described in example 60): MS (LC-MS): 478.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.95 min.

### B. 4-Ethyl-N-isopropyl-3-(3-methoxy-propoxy)-N-{(3R*,4R*)-4-[(naphthalen-2-ylmethyl)-amino]-pyrrolidin-3-ylmethyl}-benzamide

The title compound is prepared analogously as described for the title compound under B in Example 243 from (3S*,4R*)-3-amino-4-({[4-ethyl-3-(3-methoxy-propoxy)-benzoyl]-isopropylamino}-methyl)-pyrrolidine-1-carboxylic acid tert-butyl ester and (2-bromomethyl)naphtalene. MS (LC-MS): 518 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.83 min.

### Example 247: N-isopropyl-4-methoxy-3-(3-methoxy-propoxy)-N-[(3R*,4R*)-4-(toluene-4-sulfonylamino)-pyrrolidin-3-ylmethyl]-benzamide

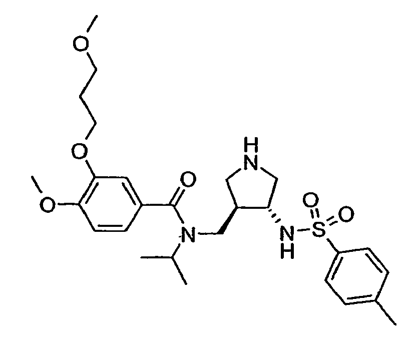

### A. (3S*,4R*)-3-({Isopropyl-[4-methoxy-3-(3-methoxy-propoxy)-benzoyl]-amino}-methyl)-4-(toluene-4-sulfonylamino)-pyrrolidine-1-carboxylic acid tert-butyl ester

To a solution of (3S*,4R*)-3-amino-4-({isopropyl-[4-methoxy-3-(3-methoxy-propoxy)-benzoyl]-amino}-methyl)-pyrrolidine-1-carboxylic acid tert-butyl ester (100 mg, 0.208 mmol) in CH₂Cl₂ (3 mL), tosylchloride (47.7 mg, 0.25 mmol) and triethylamine (35 µL, 0.25 mmol) are added under N₂ atmosphere. The mixture is stirred overnight at RT, diluted with CH₂Cl₂ and poured into an aqueous saturated solution of NaHCO₃. The organic layer is separated, and the aqueous one is extracted twice with CH₂Cl₂. The combined organic extracts are dried over Na₂SO₄, filtered and concentrated. The crude product is used in the next step without purification. TLC, Rf (AcOEt) = 0.5. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.74 min.

### B. N-Isopropyl-4-methoxy-3-(3-methoxy-propoxy)-N-[(3R*,4R*)-4-(toluene-4-sulfonylamino)-pyrrolidin-3-ylmethyl]-benzamide

To a solution of (3S*,4R*)-3-({isopropyl-[4-methoxy-3-(3-methoxy-propoxy)-benzoyl]-amino}-methyl)-4-(toluene-4-sulfonylamino)-pyrrolidine-l-carboxylic acid tert-butyl ester (126 mg, 0.199 mmol) in 3 mL CH₂Cl₂, TFA (184 µL, 2.38 mmol) is added. The mixture is stirred at RT for 1 h and poured into a saturated solution of NaHCO₃. The layers are separated, and the aqueous one is back-extracted twice with CH₂Cl₂. The combined organic extracts are dried over Na₂SO₄, filtered and concentrated. The crude material is purified by flash chromatography on silica gel (eluent: CH₂Cl₂/MeOH 100/0 to 90/10+1% NH₄OH) to give the title product. To a solution of the free base (46 mg, 0.086 mmol) in dioxane (2 mL), 32 µL, 0.129 mmol of 4N HCl in dioxane is added, and the resulting solution is lyophilized to afford the corresponding hydrochloride salt as a white powder. MS (LC-MS): 534 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.37 min.

### Example 248: N-Isopropyl-4-methoxy-N-[(3R*4R*)-4-(4-methoxy-benzenesulfonylamino)-pyrrolidin-3-ylmethyl]-3-(3-methoxy-propoxy)-benzamide

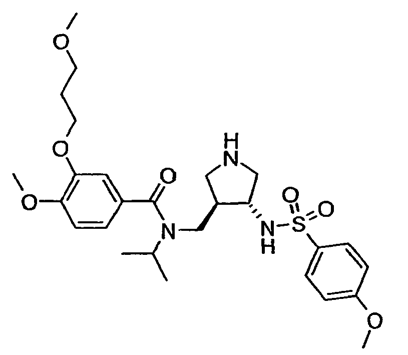

The title compound is prepared analogously as described for Example 247 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and 4-methoxy benzene sulfonyl chloride. MS (LC-MS): 449.1 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 4.33 min.

### Example 249: N-Isopropyl-4-methoxy-N-[(3R*,4R*)-4-(3-methoxy-benzenesulfonylamino)-pyrrolidin-3-ylmethyl]-3-(3-methoxy-propoxy)-benzamide

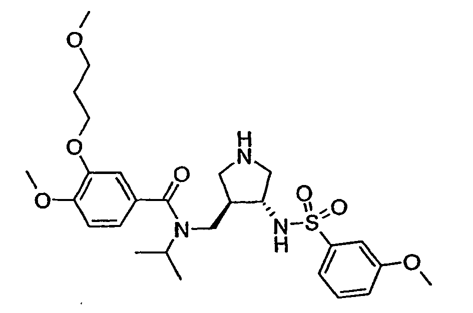

The title compound is prepared analogously as described for Example 247 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and 3-methoxy benzene sulfonyl chloride. MS (LC-MS): 549.9 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 4.38 min.

### Example 250: N-Isopropyl-4-methoxy-3-(3-methoxy-propoxy)-N-[(3R*,4R*)-4-(naphthalene-2-sulfonylamino)-pyrrolidin-3-ylmethyl]-benzamide

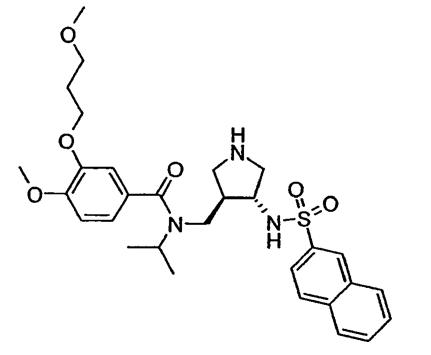

The title compound is prepared analogously as described for the title compound under B in Example 247 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and 2-naphtalenesulfonyl chloride. MS (LC-MS): 569.9 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 4.6 min.

### Example 251: N-Isopropyl-4-methoxy-3-(3-methoxy-propoxy)-N-((3S*,4S*)-4-phenylmethanesulfonylamino-pyrrolidin-3-ylmethyl)-benzamide

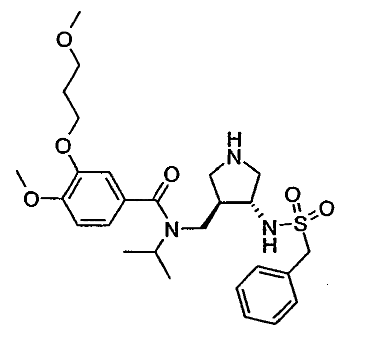

The title compound is prepared analogously as described for the title compound under B in Example 247 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and benzylsulfonyl chloride. MS (LC-MS): 334.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.35 min.

### Example 252: N-Isopropyl-4-methoxy-3-(3-methoxy-propoxy)-N-((3R,4R)-4-phenylmethane sulfonyl amino-pyrrolidin-3-ylmethyl)-benzamide and

### Example 253: N-Isopropyl-4-methoxy-3-(3-methoxy-propoxy)-N-((3S,4S)-4-phenylmethane sulfonylamino-pyrrolidin-3-ylmethyl)-benzamide

The two enantiomers of Example 251 are separated via chiral preparative HPLC (Chiralpak AD 30 x 250 mm, flow rate 120 g /min, UV = 230 nM, injection = 407 mg in 4 mL ethanol) (eluent: ethanol):
N-Isopropyl-4-methoxy-3-(3-methoxy-propoxy)-N-((3R,4R)-4-phenylmethanesulfonyl amino-pyrrolidin-3-ylmethyl)-benzamide: t_{R} (HPLC, Chiralpak AD-H, HPLC 250X4.6 mm, hexane/ethanol 50-50, flow: 1 mL/min): 8.78 min.
N-Isopropyl-4-methoxy-3-(3-methoxy-propoxy)-N-((3S,4S)-4-phenylmethanesulfonyl amino-pyrrolidin-3-ylmethyl)-benzamide: t_{R} (HPLC, Chiralpak AD-H, HPLC 250X4.6 mm, hexane/ethanol 50-50, flow: 1 mL/min): 7.99 min.

### Example 254: 4-Ethyl-N-isopropyl-3-(3-methoxy-propoxy)-N-((3R*,4R*)-4-phenylmethanesulfonylamino-pyrrolidin-3-ylmethyl)-benzamide

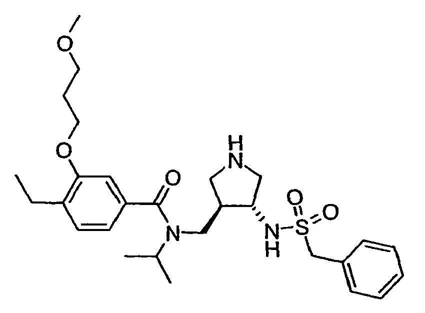

The title compound is prepared analogously as described for the title compound under B in Example 247 from (3S*,4R*)-3-amino-4-({[4-ethyl-3-(3-methoxy-propoxy)-benzoyl]-isopropylamino}-methyl)-pyrrolidine-1-carboxylic acid tert-butyl (described under A in Example 246) and benzylsulfonyl chloride. MS (LC-MS): 532 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mUmin): 4.96 min.

### Example 255: 4-Ethyl-N-isopropyl-3-(3-methoxy-propoxy)-N-[(3R*,4R*)-4-(toluene-4-sulfonylamino)-pyrrolidin-3-ylmethyl]-benzamide

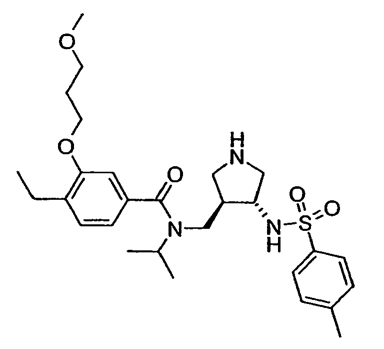

The title compound is prepared analogously as described for the title compound under B in Example 247 from (3S*,4R*)-3-amino-4-({[4-ethyl-3-(3-methoxy-propoxy)-benzoyl]-isopropylamino}-methyl)-pyrrolidine-1-carboxylic acid tert-butyl (described under A in Example 246) and tosyl chloride. MS (LC-MS): 532 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.02 min.

### Example 256: 1-(3-Methoxy-propyl)-3-methyl-1H-indole-6-carboxylic acid isopropyl-((3S*.4S*)-4-phenylmethanesulfonylamino-pyrrolidin-3-ylmethyl)-amide

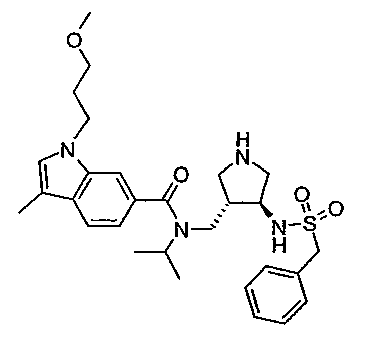

### A. (3S*,4R*)-3-Amino-4-({isopropyl-[1-(3-methoxy-propyl)-3-methyl-1H-indole-6-carbonyl]-amino}-methyl)-pyrrolidine-1-carboxylic acid tert-butyl ester

The title compound is prepared analogously as described for the title compound under I in Example 230 (Scheme32) from (3R*,4S*)-3-(isopropylamino-methyl)-4-(2-trimethylsilanyl-ethoxycarbonyl amino)-pyrrolidine-1-carboxylic acid tert-butyl ester and 1-(3-methoxypropyl)-3-methyl-1H-indole-6-carboxylic acid (described in example 65). TLC, Rf (CH2Cl2/MeOH 90/10) = 0.3. MS (LC-MS): 487.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.96 min.

### B. 1-(3-Methoxy-propyl)-3-methyl-1H-indole-6-carboxylic acid isopropyl-((3S*,4S*)-4-phenylmethanesulfonylamino-pyrrolidin-3-ylmethyl)-amide

The title compound is prepared analogously as described for the title compound under B in Example 247 from (3S*,4R*)-3-Amino-4-({isopropyl-[1-(3-methoxy-propyl)-3-methyl-1H-indole-6-carbonyl]-amino}-methyl)-pyrrolidine-1-carboxylic acid tert-butyl ester and benzylsulfonyl chloride. MS (LC-MS): 541 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 4.94 min.

### Example 257: 1-(3-Methoxy-propyl)-3-methyl-1H-indole-6-carboxylic acid [(3S*,4S*)-4-(4-fluoro-phenylmethanesulfonylamino)-pyrrolidin-3-ylmethyl]-isopropyl-amide

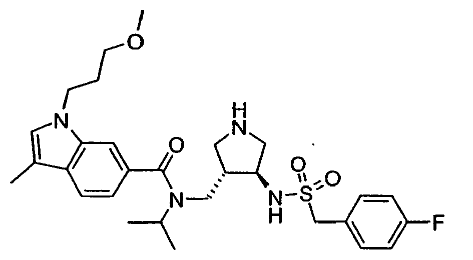

The title compound is prepared analogously as described for the title compound under B in Example 247 from (3S*,4R*)-3-amino-4-({isopropyl-[1-(3-methoxy-propyl)-3-methyl-1H-indole-6-carbonyl]-amino}-methyl)-pyrrolidine-1-carboxylic acid tert-butyl ester (described under A in Example 256) and (4-fluoro-phenyl)-methanesulfonyl chloride. MS (LC-MS): 559.2 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.56 min.

### Example 258: N-Isopropyl-4-methoxy-3-(3-methoxy-propoxy)-N-[(3S*.4S*)-4-(methyl-phenylmethanesulfonyl-amino)-pyrrolidin-3-ylmethyl]-benzamide

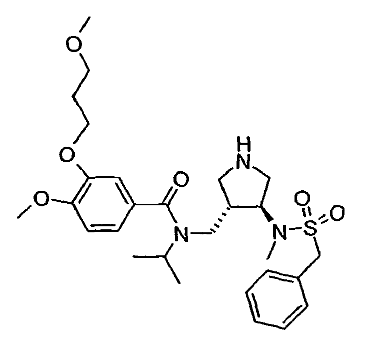

### A. (3R*,45*)-3-((Isopropyl-[4-methoxy-3-(3-methoxy-propoxy)-benzoyl]-amino)-methyl)-4-phenylmethanesulfonylamino-pyrrolidine-1-carboxylic acid tert-butyl ester

To a solution of (3S*,4R*)-3-amino-4-({isopropyl-[4-methoxy-3-(3-methoxy-propoxy)-benzoyl]-amino}-methyl)-pyrrolidine-1-carboxylic acid tert-butyl ester (800 mg, 1.67 mmol) in CH₂Cl₂ (18 mL), benzylsulfonyl chloride (382 mg, 2 mmol) and triethylamine (280 µL, 2 mmol) are added under N₂ atmosphere. The mixture is stirred overnight at RT, diluted with CH₂Cl₂ and poured into an aqueous saturated solution of NaHCO₃. The organic layer is separated, and the aqueous one is extracted twice with CH₂Cl₂. The combined organic extracts are dried over Na₂SO₄, filtered and concentrated. The crude material is purified by flash chromatography (eluent: CH2Cl2/MEOH 95/5) to afford the title product.
t_{R} (HPLC, nucleosil C18 column, 10-100% CH3CN/H2O/5 min, 100% CH3CN/3 min, flow: 1.5 mL/min): 6.07 min.

### B. (3R*,4S*)-3-({Isopropyl-[4-methoxy-3-(3-methoxy-propoxy)-benzoyl]-amino}-methyl)-4-(methyl-phenylmethanesulfonyl-amino)-pyrrolidine-1-carboxylic acid tert-butyl ester

To a solution of (3R*,4S*)-3-({Iisopropyl-[4-methoxy-3-(3-methoxy-propoxy)-benzoyl]-amino}-methyl)-4-phenylmethanesulfonylamino-pyrrolidine-1-carboxylic acid tert-butyl ester (123 mg, 0.194 mmol) in DMF (3 mL) are added K₂CO₃ (35 mg, 0.252 mmol) and iodomethane (16 µL, 0.252 mmol). The mixture is stirred at 80°C overnight. Iodomethane (6 µL, 0.097 mmol) and K₂CO₃ (13 mg, 0.097 mmol) are again added and the reaction mixture is further stirred for 2 days. The reaction mixture is poured into a saturated aqueous solution of NaHCO₃. The layers are separated and the aqueous one back-extracted twice with AcOEt, the combined organic extracts are dried over Na₂SO₄, filtered and concentrated. The crude mixture material is purified by HPLC preparative (C18-ODB-AQ, 5 µm, 20x50 mm, YMC, eluent: CH₃CN /H₂O + 0.1 % HCOOH flow: 20 mL/min). The HPLC fractions are collected and lyophilized to afford the title product. TLC, Rf (CH₂Cl₂/MeOH 90/10) = 0.5, MS (LC-MS): 592 [M+H-tBu]⁺; t_{R} (HPLC, nucleosil C18 column, 10-100% CH₃CN/H₂O/5 min, 100% CH₃CN/3 min, flow: 1.5 mL/min): 5.91 min.

### C. N-Isopropyl-4-methoxy-3-(3-methoxy-propoxy)-N-[(35*,45*)-4-(methyl-phenylmethanesulfonyl-amino)-pyrrolidin-3-ylmethyl]-benzamide

To a solution of (3R*,4S*)-3-({isopropyl-[4-methoxy-3-(3-methoxy-propoxy)-benzoyl]-amino}-methyl)-4-(methyl-phenylmethanesulfonyl-amino)-pyrrolidine-1-carboxylic acid tert-butyl ester (65 mg, 0.1 mmol) in 2 mL CH₂Cl₂, TFA (116 µL, 1.5 mmol) is added. The mixture is stirred at RT for 1 h and poured into a saturated solution of NaHCO₃. The layers are separated, and the aqueous one is back-extracted twice with CH₂Cl₂. The combined organic extracts are dried over Na₂SO₄, filtered and concentrated. The crude material is purified by flash chromatography on silica gel (eluent: CH₂Cl₂/MeOH 100/0 to 80/20) to give the title product. To a solution of the free base (46 mg, 0.086 mmol) in dioxane (2 mL) is added 4N HCl in dioxane (29 µL, 0.115 mmol), and the resulting solution is lyophilized to afford the corresponding hydrochloride salt as a white powder. MS (LC-MS): 548 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.59 min.

### Example 259: N-((3R*,4R*)-4-Benzylamino-pyrrolidin-3-ylmethyl)-N-isopropyl-4-methoxy-3-(3-methoxy-propoxy)-benzamide

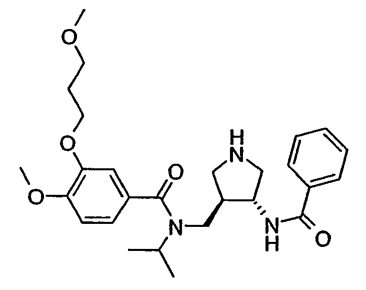

### A. (3R*,4S*)-3-Benzoylamino-4-({isopropyl-[4-methoxy-3-(3-methoxy-propoxy)-benzoyl]-amino}-methyl)-pyrrotidine-1-carboxylic acid tert-butyl ester

To a solution of (3S*,4R*)-3-amino-4-({isopropyl-[4-methoxy-3-(3-methoxy-propoxy)-benzoyl]-amino}-methyl)-pyrrolidine-1-carboxylic acid tert-butyl ester (200 mg, 0.417 mmol) in CH₂Cl₂ (3 mL), benzoylchloride (65 mg, 0.459 mmol) and triethylamine (64 µL, 0.459 mmol) are added under N₂ atmosphere. The mixture is stirred for 4 h, diluted with CH₂Cl₂ and poured into an aqueous saturated solution of NaHCO₃. The organic layer is separated, and the aqueous one is extracted twice with CH₂Cl₂. The combined organic extracts are dried over Na₂SO₄, filtered and concentrated. The crude product is used in the next step without purification. TLC, Rf (AcOEt) = 0.6. MS (LC-MS): 484 [M+H-boc]⁺.

### B. N-((3R*,4R*)-4-Benzylamino-pyrrolidin-3-ylmethyl)-N-isopropyl-4-methoxy-3-(3-methoxy-propoxy)-benzamide

To a solution of (3R*,4S*)-3-benzoylamino-4-({isopropyl-[4-methoxy-3-(3-methoxy-propoxy)-benzoyl]-amino}-methyl)-pyrrolidine-1-carboxylic acid tert-butyl ester (245 mg, 0.42 mmol) in 2 mL CH₂Cl₂, TFA (485 µL, 6.3 mmol) is added. The mixture is stirred at RT for 1 h and poured into a saturated solution of NaHCO₃. The layers are separated, and the aqueous one is back-extracted twice with CH₂Cl₂. The combined organic extracts are dried over Na₂SO₄, filtered and concentrated. The crude material is purified by preparative HPLC (C18-ODS-AQ 5 µm, 20x50 mm, eluent: CH₃CN /H2O + 0.1 % HCOOH). To a solution of the free base (105 mg, 0.22 mmol) in dioxane (3 mL), (81 µL, 0.326 mmol) of 4N HCl in dioxane is added, and the resulting solution is lyophilized to afford the corresponding hydrochloride salt as a white powder. MS (LC-MS): 484 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.19 min.

### Example 260: N-Isopropyl-4-methoxy-3-(3-methoxy-propoxy)-N-((35*,45*)-4-phenyl acetylamino-pyrrolidin-3-ylmethyl)-benzamide

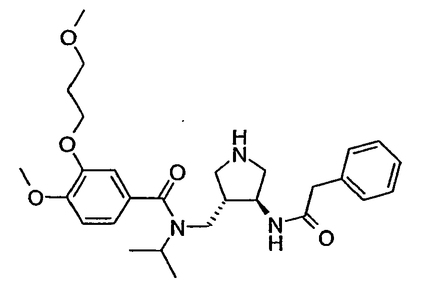

The title compound is prepared analogously as described for the title compound under B in Example 259 from (3S*,4R*)3-amino-4-({isopropyl-[4-methoxy-3-(3-methoxy-propoxy)-benzoyl]-amino}-methyl)-pyrrolidine-1-carboxylic acid tert-butyl ester and phenylacetyl chloride. MS (LC-MS): 498.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.27 min.

### Example 261: N-Isopropyl-4-methoxy-3-(3-methoxy-propoxy)-N-[(3S*,4S*)-4-(3-phenyl-propionylamino)-pyrrolidin-3-ylmethyl]-benzamide

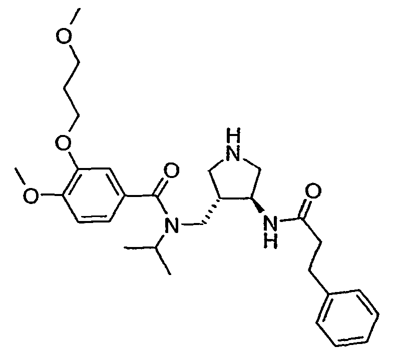

The title compound is prepared analogously as described for the title compound under B in Example 259 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and hydrocinnamoyl chloride. MS (LC-MS): 512 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.70 min.

### Example 262: [(3R*.4R*)-4-({Isopropyl-[4-methoxy-3-(3-methoxy-propoxy)-benzoyl]-amino}-methyl)-pyrrolidin-3-yl]-carbamicacid isopropyl ester

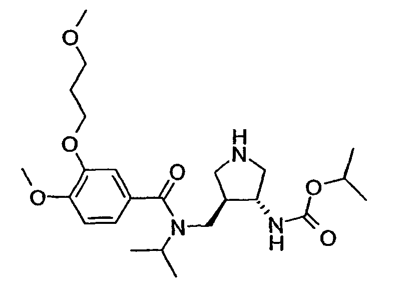

### A. (3R*,4S*)-3-Isopropoxycarbonylamino-4-({isopropyl-[4-methoxy-3-(3-methoxy-propoxy)-benzoyl]-amino}-methyl)-pyrrolidine-1-carboxylic acid tert-butyl ester

To a solution of (3S*,4R*)-3-amino-4-({isopropyl-[4-methoxy-3-(3-methoxy-propoxy)-benzoyl]-amino}-methyl)-pyrrolidine-1-carboxylic acid tert-butyl ester (200 mg, 0.417 mmol) in CH₂Cl₂ (4 mL), isopropyl chloroformate (1N in toluene, 460 µL, 0.459 mmol) and triethylamine (64 µL, 0.459 mmol) are added under N₂ atmosphere. The mixture is stirred for 4 h, diluted with CH₂Cl₂ and poured into an aqueous saturated solution of NaHCO₃. The organic layer is separated, and the aqueous one is extracted twice with CH₂Cl₂. The combined organic extracts are dried over Na₂SO₄, filtered and concentrated. The crude product is used in the next step without purification. TLC, Rf (AcOEt) = 0.4. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.62 min.

### B. [(3R*,4R*)-4-({Isopropyl-[4-methoxy-3-(3-methoxy-propoxy)-benzoyl]-amino)-methyl)-pyrrolidin-3-yl]-carbamicacid isopropyl ester

To a solution of (3R*,4S*)-3-isopropoxycarbonylamino-4-({isopropyl-[4-methoxy-3-(3-methoxy-propoxy)-benzoyl]-amino}-methyl)-pyrrolidine-1-carboxylic acid tert-butyl ester (250 mg, 0.442 mmol) in 5 mL CH₂Cl₂, TFA (510 µL, 6.6 mmol) is added. The mixture is stirred at RT for 1 h and poured into a saturated solution of NaHCO₃. The layers are separated, and the aqueous one is back-extracted twice with CH₂Cl₂. The combined organic extracts are dried over Na₂SO₄, filtered and concentrated. The crude material is purified by flash chromatography on silica gel (eluent : CH₂Cl₂/MeOH/NH₄OH 100/0 to 90/10/0 to 90/10/1). To a solution of the free base (48 mg, 0.103 mmol) in dioxane (23 mL), (31 µL, 0.124 mmol) of 4N HCl in dioxane is added, and the resulting solution is lyophilized to afford the corresponding hydrochloride salt as a white powder. MS (LC-MS): 466 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.20 min.

### Example 263: [(3S*,4S*)-4-({Isopropyl-[4-methoxy-3-(3-methoxy-propoxy)-benzoyl]-amino}-methyl)-pyrrolidin-3-yl]-carbamicacid benzyl ester

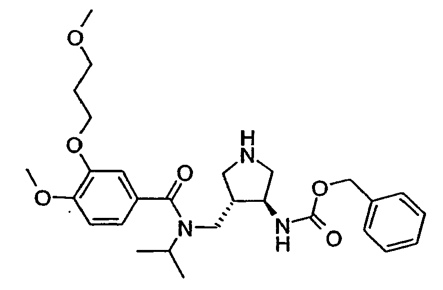

The title compound is prepared analogously as described for the title compound under B in Example 262 from (3S*,4R*)-3-amino-4-({isopropyl-[4-methoxy-3-(3-methoxy-propoxy)-benzoyl]-amino}-methyl)-pyrrolidine-1-carboxylic acid tert-butyl ester and benzyl chloroformate. MS (LC-MS): 514.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mUmin): 4.47 min.

### Example 264: [(3S*,4S*)-4-({Isopropyl-[1-(3-methoxy-propyl)-3-methyl-1H-indole-6-carbonyl]-amino}-methyl)-pyrrolidin-3-yl]-carbamic acid benzyl ester

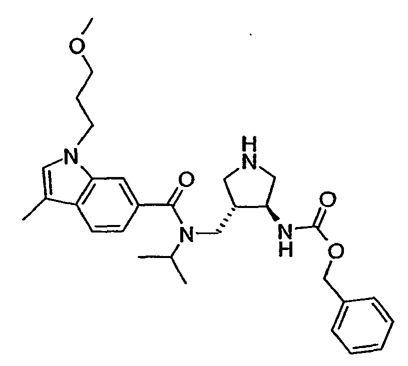

The title compound is prepared analogously as described for the title compound under B in Example 262 from (3S*,4R*)-3-amino-4-({isopropyl-[1-(3-methoxy-propyl)-3-methyl-1H-indole-6-carbonyl]-amino}-methyl)-pyrrolidine-1-carboxylic acid tert-butyl ester (described under A in Example 256) and benzyl chloroformate. MS (LC-MS): 514.1 [M+H]⁺; t_{R} (HPLC, Nucleosil C18 column, 10-100% CH₃CN/H₂O/5 min, 100% CH₃CN/3 min, CH₃CN and H₂O containing 0.1 % TFA, flow: 1.5 mL/min): 4.47 min.

### Example 265: [(3S*,4S*)-4-({[4-Ethyl-3-(3-methoxy-propoxy)-benzoyl]-isopropyl-amino}-methyl)-pyrrolidin-3-yll-carbamic acid benzyl ester

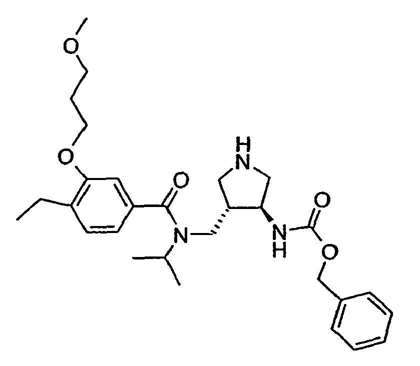

The title compound is prepared analogously as described for the title compound under B in Example 262 from (3S*,4R*)-3-amino-4-({[4-ethyl-3-(3-methooy-propoxy)-benzoyl]-isopropylamino}-methyl)-pyrrolidine-1-carboxylic acid tert-butyl (described under A in Example 246) and benzyl chloroformate. MS (LC-MS): 512 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.18 min.

### Example 266: N-[(3S*,4S*)-4-(3-Benzyl-ureido)-pyrrolidin-3-ylmethyl]-N-isopropyl-4-methoxy-3-(3-methoxy-propoxy)-benzamide

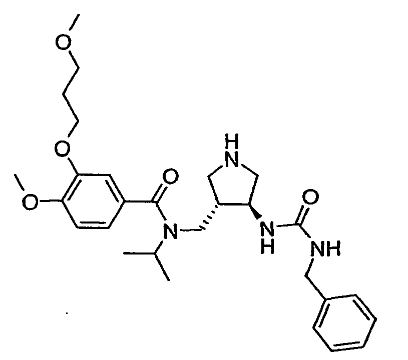

### A. (3S*,4R*)-3-(3-Benzyl-ureido)-4-({isopropyl-[4-methoxy-3-(3-methoxy-propoxy)-benzoyl]-amino}-methyl)-pyrrolidine-1-carboxylic acid tert-butyl ester

To a solution of (3S,4R)-3-amino-4-({isopropyl-[4-methoxy-3-(3-methoxy-propoxy)-benzoyl]-amino}-methyl)-pyrrolidine-1-carboxylic acid tert-butyl ester (100 mg, 0.208 mmol) in CH₂Cl₂ (2 mL) is added benzyl isocyanate (56 mg, 0.417 mmol). The mixture is stirred at RT under nitrogen overnight and quenched by addition of an aqueous saturated solution of NaHCO₃. CH₂Cl₂ is added and the layers are separated, the aqueous one being back-extracted twice with CH₂Cl₂. The combined organic extracts are dried over Na₂SO₄, filtered and concentrated to give the title compound which was used in the next step without further purification. MS (LC-MS): 613 [M+H]⁴; t_{R} (HPLC, nucleosil C18 column, 10-100% CH₃CN/H₂O/5 min, 100% CH₃CN/3 min, flow: 1.5 mL/min): 5.75 min.

### C. N-[(3S*,4S*)-4-(3-Benzyl-ureido)-pyrrolidin-3-ylmethyl]-N-isopropyl-4-methoxy-3-(3-methoxy-propoxy)-benzamide

To a solution of (3S*,4R*)-3-(3-benzyl-ureido)-4-({isopropyl-[4-methoxy-3-(3-methoxy-propoxy)-benzoyl]-amino}-methyl)-pyrrolidine-1-carboxylic acid tert-butyl ester (140 mg, 0.228 mmol) in 2 mL CH₂Cl₂, TFA (264 µL, 3.4 mmol) is added. The mixture is stirred at RT for 1 h and poured into a saturated solution of NaHCO₃. The layers are separated, and the aqueous one is back-extracted twice with CH₂Cl₂. The combined organic extracts are dried over Na₂SO₄, filtered and concentrated. The crude material is purified by HPLC preparative (C18-ODB-5 µm, 19x50 mm, eluent: CH₃CN /H₂O + 0.1 % HCOOH). The HPLC fractions are collected, AcOEt is added and the fractions are neutralized with a saturated aqueous solution of NaHCO₃. The layers are separated and the aqueous one back-extracted twice with AcOEt. The combined organic extracts are dried over Na₂SO₄, filtered and concentrated to give the desired title compound. To a solution of the free base (42 mg, 0.082 mmol) in dioxane (2 mL), is added 4N HCl in dioxane (22.5 µL, 0.09 mmol) and the resulting solution is lyophilized to afford the corresponding hydrochloride salt as a white powder. MS (LC-MS): 513 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.68 min.

### Example 267: N-[(3S*,4S*)-4-(Cyclopropylcarbamoylmethyl-amino)-pyrrolidin-3-ylmethyl]-N-isopropyl-4-methoxy-3-(3-methoxy-propoxy)-benzamide

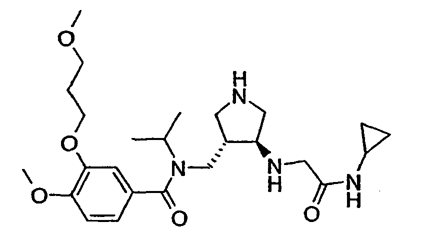

### A. (35*,4R*)-3-(Cyclopropylcarbamoylmethyl-amino)-4-({Isopropyl-[4-methoxy-3-(3-methoxy-propoxy)-benzoyl]-amino}-methyl)-pyrrolidine-1-carboxylicacid tert-butyl ester

To a solution of (3S*,4R*)-3-amino-4-({isopropyl-[4-methoxy-3-(3-methoxy-propoxy)-benzoyl]-amino}-methyl)-pyrrolidine-1-carboxylic acid tert-butyl ester (100 mg, 0.208 mmol) in DMF (2 mL), 2-bromo-N-cyclopropyl-acetamide (39 mg, 0.219 mmol) and cesium carbonate (71.3 mg, 0.219 mmol) are added under N₂ atmosphere. The mixture is stirred for 2 days at room temperature, diluted with AcOEt and poured into an aqueous saturated solution of NaHCO₃. The organic layer is separated, and the aqueous one is extracted twice with AcOEt. The combined organic extracts are dried over Na₂SO₄, filtered and concentrated. The crude product is purified by flash chromatography on silica gel (eluent: CH₂Cl₂/MeOH 100/0 to 95/5). TLC, Rf (CH₂Cl₂/MeOH, 90/10) = 0.4. MS (LC-MS): 577 [M+H]⁺.

### B. N-[(3S*,4S*)-4-(Cyclopropylcarbamoylmethyl-amino)-pyrrolidin-3-ylmethyl]-N-isopropyl-4-methoxy-3-(3-methoxy-propoxy)-benzamide

To a solution of (3S*,4R*)-3-(cyclopropylcarbamoylmethyl-amino)-4-({isopropyl-[4-methoxy-3-(3-methoxy-propoxy)-benzoyl]-amino}-methyl)-pyrrolidine-1-carboxylicacid tert-butyl ester (84 mg, 0.146 mmol) in 2 mL CH₂Cl₂, TFA (168 µL, 2.18 mmol) is added. The mixture is stirred at RT for 1 h and poured into a saturated solution of NaHCO₃. The layers are separated, and the aqueous one is back-extracted twice with CH₂Cl₂. The combined organic extracts are dried over Na₂SO₄, filtered and concentrated. The crude product is purified by preparative HPLC (C18 ODS-AQ 5 µm 20x50 mm, 20 mL/min. eluent CH₃CN/H₂O + 0.1% HCOOH). The combined pure fractions are neutralized by the addition of saturated aqueous Na₂CO₃ solution, and CH₃CN is removed *in vacuo.* The remaining aqueous phase is extracted twice with CH₂Cl₂. The combined organic layers are dried (Na₂SO₄) and evaporated *in vacuo* to afford the desired title compound. MS (LC-MS): 477 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 3.65 min.

### 2-bromo-N-cyclopropyl-acetamide

To a ice cooled solution of bromoacetic acid (200 mg, 1.44 mmol) in CH₂Cl₂ (5 mL), HOBt (195 mg, 1.44 mmol) and DCC (297 mg, 1.44 mmol) are added. The mixture is stirred for 30 min. at 0°C, before the addition of cyclopropylamine (82 mg, 1.44 mmol). The resulting mixture is further stirred for 30 min. at RT and the precipitated DCU is filtered off. The filtrate is extracted with an aqueous saturated solution of NaHCO₃. The organic layers are dried over Na₂SO₄, filtered and concentrated. The crude product is purified by flash chromatography on silica gel (eluent: AcOEt/c-Hex (1:1 to 2:1)) to afford the title product as a white solid. TLC, Rf (AcOEt) = 0.4. t_{R} (HPLC, Nucleosil C18 column, 10-100% CH₃CN/H₂O/ 5 min, 100% CH₃CN/3 min, CH₃CN and H₂O containing 0.1% TFA, flow: 1.5 mL/min): 3.91 min.

### Example 268: N-{(3S*,4S*)-4-[Bis-(benzylcarbamoyl-methyl)-amino]-pyrrolidin-3-ylmethyl}-N-isopropyl-4-methoxy-3-(3-methoxy-propoxy)-benzamide

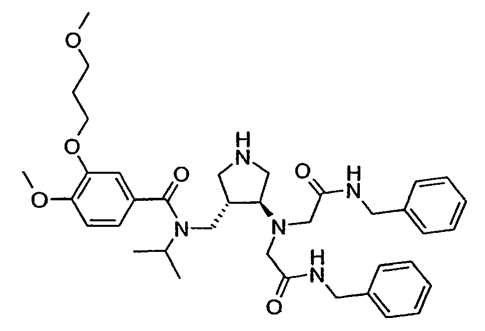

### A. (3S*,4R*)-3-[Bis-(benzylcarbamoyl-methyl)-amino]-4-({isopropyl-[4-methoxy-3-(3-methoxy-propoxy)-benzoyl]-amino}-methyl)-pyrrolidine-1-carboxylic acid tert-butyl ester

To a solution of (3S*,4R*)-3-amino-4-({isopropyl-[4-methoxy-3-(3-methoxy-propoxy)-benzoyl]-amino}-methyl)-pyrrolidine-1-carboxylic acid tert-butyl ester (195 mg, 0.406 mmol) and diisopropylethyl amine (139 µL, 0.813 mmol) in DMF (2 mL) is added a solution of N-benzyl-2-bromo-acetamide (93 mg, 0.406 mmol) in DMF (4 mL) at 0 °C. The reaction mixture is further stirred overnight at RT. Diisopropylethyl amine (139 µL, 0.813 mmol) and N-benzyl-2-bromo-acetamide (93 mg, 0.406 mmol) are added to complete the reaction and after 3 h the reaction is quenched by addition of a saturated aqueous solution of NaHCO₃. AcOEt is added, the layers are separated and the aqueous one extracted with AcOEt. The combined organic extracts are dried over Na₂SO₄, filtered and concentrated. The crude mixture is purified by preparative HPLC (C18-ODB-AQ, 5 µm, 20x50 mm, YMC, eluent: CH₃CN /H₂O + 0.1 % HCOOH flow: 20 mL/min). The HPLC fractions are collected, diluted with AcOEt and washed with a saturated aqueous solution of NaHCO₃. The organic layer is dried over Na₂SO₄, filtered and concentrated to give (3S*,4R*)-3-[bis-(benzylcarbamoyi-methyl)-amino]-4-({isopropyl-[4-methoxy-3-(3-methoxy-propoxy)-benzoyl]-amino}-methyl)-pyrrolidine-1-carboxylic acid tert-butyl ester as the first minor fraction: t_{R} (HPLC, nucleosil C18 column, 10-100% CH₃CN/H₂O/5 min, 100% CH₃CN/3 min, flow: 1.5 mL/min): 5.93 min and (3S*,4R*)-3-[(benzylcarbamoyl-methyl)-amino]-4-({isopropyl-[4-methoxy-3-(3-methoxy-propoxy)-benzoyl]-amino}-methyl)-pyrrolidine-1-carboxylic acid tert-butyl ester as the second major fraction: t_{R} (HPLC, nucleosil C18 column, 10-100% CH₃CN/H₂O/5 min, 100% CH₃CN/3 min, flow: 1.5 mL/min): 5.09 min.

### B. N{(3S*,4S*)-4-[Bis-(benzylcarbamoyl-methyl)-amino]-pyrrolidin-3-ylmethyl}-N-isopropyl-4-methoxy-3-(3-methoxy-propoxy)-benzamide

To a solution of (3S*,4R*)-3-[bis-(benzylcarbamoyl-methyl)-amino]-4-({psopropyl-[4-methoxy-3-(3-methoxy-propoxy)-benzoyl]-amino}-methyl)-pyrrolidine-1-carboxylic acid tert-butyl ester (34 mg, 0.044 mmol) in dioxane (1 mL), 4N HCl in dioxane (0.5 mL) is added, and the resulting solution is lyophilized to afford the corresponding bishydrochbride salt as a white powder. MS (LC-MS): 674 [M+H]⁺; t_{R} (HPLC, nucleosil C18 column, 10-100% CH₃CN/H₂O/5 min, 100% CH₃CN/3 min, flow: 1.5 mL/min): 4.94 min.

### N-benzyl-2-bromo-acetamide

To a solution of 2-bromoacetic acid (1 g, 7.19 mmol) in CH₂Cl₂ (20 mL) is added HOBt (0.97 g, 7.19 mmol) and EDCI (1.37 g, 7.19 mmol). The solution is stirred at RT for 15 min, before the addition of benzylamine (0.786 mL, 7.19 mmol). The solution is stirred at RT for 2 h, diluted with CH₂Cl₂ and extracted with an aqueous saturated solution of NaCl. The combined organic layers are extracted with an aqueous saturated solution of NaHCO₃, dried over Na₂SO₄, filtered and concentrated. The crude material is purified by flash chromatography on silica gel (eluent: c-hexane/AcOEt 80/20 to 70/30) to give the title compound as a white powder. TLC, Rf (c-hexane/AcOEt 70/30) = 0.2, MS (LC-MS): 228-230 [M+H]⁺; t_{R} (HPLC, nucleosil C18 column, 10-100% CH₃CN/H₂O/5 min, 100% CH₃CN/3 min, flow: 1.5 mL/min): 3.35 min.

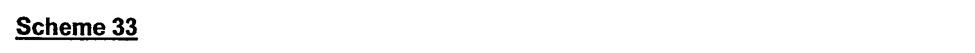

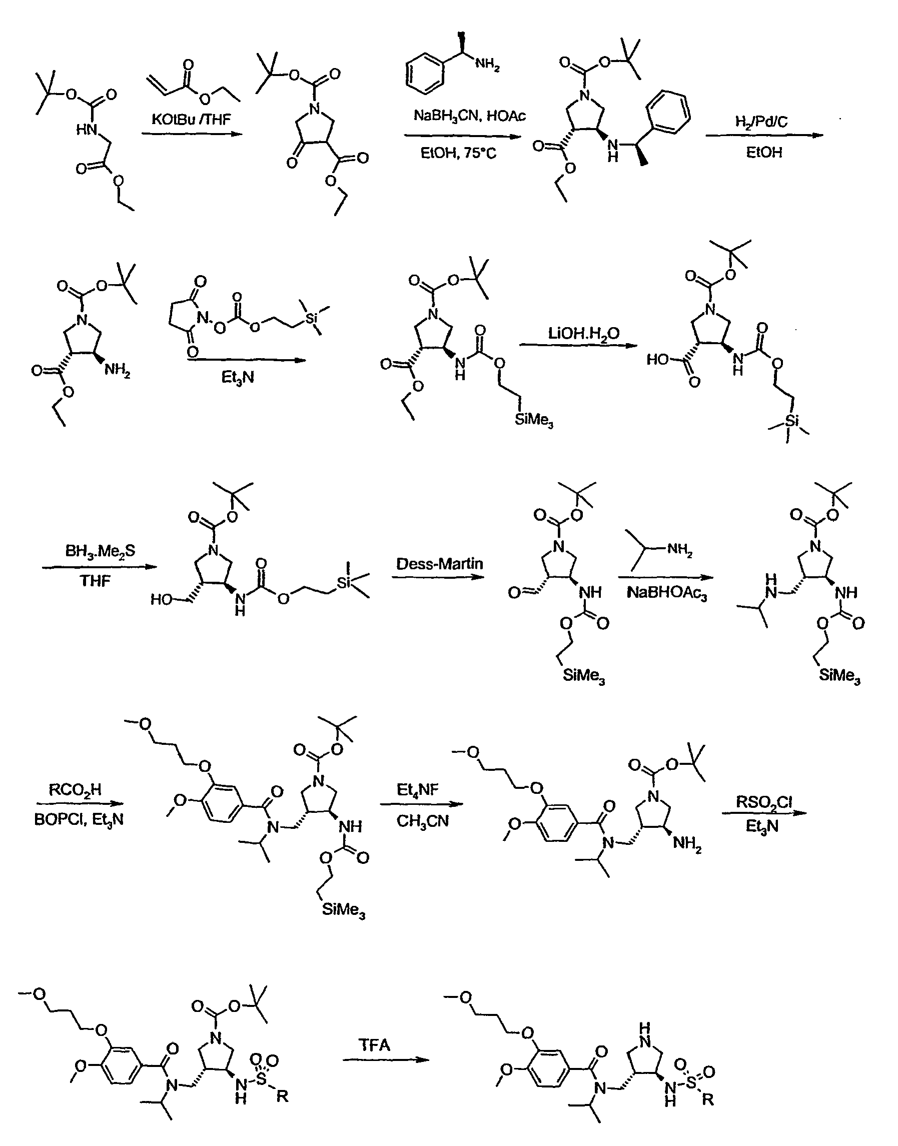

### Example 269: N-Isopropyl-4-methoxy-3-(3-methoxy-propoxy)-N-((3S,4S)-4-ehenylmethanesulfonylamino-pyrrolidin-3-ylmethyl)-benzamide

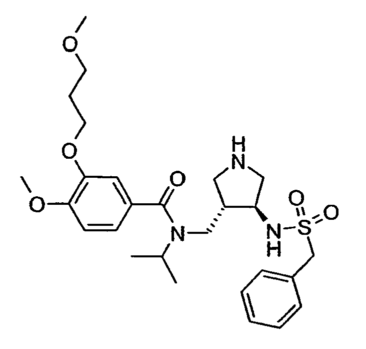

### A. 4-Oxo-pyrrolidine-1,3-dicarboxylic acid 1-tert-butyl ester 3-ethyl ester

To a solution of Boc-Gly-OEt (130 g, 599 mmol) and ethyl acrylate (65 mL, 599 mmol) in THF (1.0 L) at 0°C is added KOtBu (74 g, 659 mmol) portion-wise. The mixture is allowed to reach RT and stirred for 24h. The crude reaction mixture is concentrated, diluted with CH₂Cl₂ and neutralized with 0.1 N HCl solution. The layers are separated and the aqueous one extracted twice with CH₂Cl₂. The combined organic layers are washed with brine, dried over Na₂SO₄, filtered and concentrated. The crude material is purified by flash chromatography on silica gel (eluent: c-hexane/AcOEt 80/20 to 50/50) to give the title compound. TLC, Rf (c-hexane/AcOEt 50/50) = 0.5.

### B. (3R,4S)-4-((R)-1-Phenyl-ethylamino)-pyrrolidine-1,3-dicarboxylic acid 1-tert-butyl ester 3-ethyl ester

The title compound was prepared according to J. Org. Chem. 2001, 66, 3597.
To a stirring solution of 4-oxo-pyrrolidine-1,3-dicarboxylic acid 1-tert-butyl ester 3-ethyl ester (61 g, 237 mmol) in absolute ethanol (950 mL) under N₂ are added (R)-(+)-alpha-methylbenzylamine (54 mL, 474 mmol) and glacial acetic acid (28.5 g, 474 mmol). The reaction mixture is stirred at RT until the formation of the enamine is complete as indicated by TLC (R_{f}, c-hexane/AcOEt 1/2 = 0.4). Sodium cyanoborohydride (30 g, 474 mmol) is added to the reaction mixture, and the resulting solution heated at 75°C for 15 h. The crude mixture is concentrated, water is added and the mixture extracted three times with diethyl ether. The combined organic extracts are washed with brine, dried over Na₂SO₄, filtered and concentrated to an oil. The crude material was filtered through a plug of silica gel eluting with c-hexane/AcOEt ½. The resulting oil is diluted in AcOEt (1.6 L) and a solution of 4N HCl in dioxan (120 mL, 480 mmol) is added. The white precipitate is filtered off and washed twice with 250 mL portions of AcOEt. The resulting white solid is further purified by recrystalisation from CH₃CN (900 mL). TLC, Rf (CH₂Cl₂/MeOH 19/1) = 0.55. Mp = 194-195°C. t_{R} (HPLC, nucleosil C18 column, 10-100% CH₃CN/H₂O/5 min, 100% CH₃CN/3 min, flow: 1.5 mL/min): 4.52 min.

### C. (3R,4S)-4-Amino-pyrrolidine-1,3-dicarboxylic acid 1-tert-butyl ester 3-ethyl ester

To a solution of (3R,4S)-4-((R)-1-phenyl-ethylamino)-pyrrolidine-1,3-dicarboxylic acid 1-tert-butyl ester 3-ethyl ester (31.2 g, 78.2 mmol) in EtOH (936 mL) is added Pd/C 10% (3.4 g). The mixture is shaked overnight at RT under an hydrogen atmosphere and filtered to give the title compound. TLC, R_{f} (AcOEt) = 0.17. MS (LC-MS): 159.1 [M-Boc+H]⁺.

### D. (3R,4S)-4-(2-Trimethylsilanyl-ethoxycarbonylamino)-pyrrolidine-1,3-dicarboxylic acid 1-tert-butyl ester 3-ethyl ester

To a solution of (3R,4S)-4-amino-pyrrolidine-1,3-dicarboxylic acid 1-tert-butyl ester 3-ethyl ester (31.0 g, 105 mmol) in dioxan (500 mL) are added Teoc-OSuc (27.3 g, 105 mmol) and triethylamine (29.3 mL, 210 mmol) under a N₂ atmosphere. The mixture is stirred overnight at RT. After completion of the reaction, the crude material is diluted with CH₂Cl₂ and washed twice with an aqueous saturated solution of NaHCO₃. The organic layers is dried over Na₂SO₄, filtered and concentrated, to give the title product which is directly used in the next step without further purification. TLC, R_{f} (AcOEt/ c-hexane 1/1) = 0.54. MS (LC-MS): 401 [M+H]⁺.

### E. (3R,4S)-4-(2-Trimethylsilanyl-ethoxycarbonylamino)-pyrrolidine-1,3-dicarboxylic acid 1-tert-butyl ester

To a solution of (3R,4S)-4-(2-trimethylsilanyl-ethoxycarbonylamino)-pyrrolidine-1,3-dicarboxylic acid 1-tert-butyl ester 3-ethyl ester (53.6 g, 105 mmol) in MeOH (1.0 L) is added LiOH.H₂O (6.63 g, 158 mmol). The mixture is stirred overnight at RT and concentrated to dryness. The resulting orange oil was dissolved in CH₂Cl₂ and a solution of HCl 2N (80 mL) is added. The layers are separated and the aqueous one back extracted twice with CH₂Cl₂. The combined organic layers are dried over Na₂SO₄, filtered and concentrated to give the desired title product which was used without further purification in the next step. TLC, Rf (AcOEt) = 0.47. MS (LC-MS): 373 [M-H]⁺.

### F. (3R,4S)-3-Hydroxymethyl-4-(2-trimethylsilanyl-ethoxycarbonylamino)-pyrrolidine-1-carboxylic acid tert-butyl ester

To a solution of (3R,4S)-4-(2-trimethylsilanyl-ethoxycarbonylamino)-pyrrolidine-1,3-dicarboxylic acid 1-tert-butyl ester (39.1 g, 104 mmol) in THF (570 mL) is slowly added a solution of borane dimethylsulfide complex (2N in THF, 73 mL, 146 mmol) at -10°C under N₂ atmosphere. The mixture is stirred for 20 min at -10°C and allowed to reach RT and further stirred overnight until completion of the reaction. MeOH is carefully added, the resulting solution stirred for 1 h and concentrated by rotary evaporation. The crude oil is diluted with CH₂Cl₂, a saturated aqueous solution of NaHCO₃ is added and the layers separated. The aqueous layer is back-extracted twice with CH₂Cl₂ and the combined organic layers are dried over Na₂SO₄, filtered and concentrated. The crude material is purified by flash chromatography on silica gel (eluent c-hexane/AcOEt 50/50) to give the title compound. TLC, Rf (c-hexane/AcOEt 1/1) = 0.12. MS (LC-MS): 261 [M+H]⁺. t_{R} (HPLC, Chiralpak AD-H, HPLC 250X4.6 mm, hexane/isopropanol 95-5, flow: 1 mL/min): 11.67 min.

Alternatively, (3R,4S)-3-hydroxymethyl-4-(2-trimethylsilanyl-ethoxycarbonylamino)-pyrrolidine-1-carboxylic acid tert-butyl ester is prepared from (3R,4S)-4-(2-trimethylsilanyl-ethoxycarbonylamino)-pyrrolidine-1,3-dicarboxylic acid 1-tert-butyl ester 3-ethyl ester as described below.

To an ice-cooled solution of (3R,4S)-4-(2-trimethylsilanyl-ethoxycarbonylamino)-pyrrolidine-1,3-dicarboxylic acid 1-tert-butyl ester 3-ethyl ester (120 mg, 0.298 mmol) in THF (1.8 mL), is added a solution of LiBH₄ (2N in THF, 150 µL, 0.300 mmol). The reaction mixture is stirred overnight at room temperature and quenched with an aqueous saturated solution of NaHCO₃. The mixture is extracted twice with AcOEt and the combined organic layers are dried over Na₂SO₄, filtrated and concentrated to give (3R,4S)-3-hydroxymethyl-4-(2-trimethylsilanyl-ethoxycarbonylamino)-pyrrolidine-1-carboxylic acid tert-butyl ester.

### G. (3R,4S)-3-Formyl-4-(2-trimethylsilanyl-ethoxycarbonylamino)-pyrrolidine-1-carboxylic acid tert-butyl ester

The title compound is prepared analogously as described for the title compound under F in Example 230 (Scheme 32) from (3R,4S)-3-hydroxymethyl-4-(2-trimethylsilanylethoxy-carbonyl-amino)-pyrrolidine-1-carboxylic acid tert-butyl ester.

### H. (3R,4S)-3-(Isopropylamino-methyl)-4-(2-trimethylsilanyl-ethoxycarbonylamino)-pyrrolidine-1-carboxylic acid tert-butyl ester

The title compound is prepared analogously as described for the title compound under G in Example 230 (Scheme32) from (3R,4S)-3-formyl-4-(2-trimethylsilanyl-ethoxycarbonylamino)-pyrrolidine-1-carboxylic acid tert-butyl ester.

### 1. (3R,4S)-3-({Isopropyl-[4-methoxy-3-(3-methoxy-propoxy)-benzoyl]-amino}-methyl)-4-(2-trimethylsilanyl-ethoxycarbonylamino)-pyrrolidine-1-carboxylic acid tert-butyl ester

The title compound is prepared analogously as described for the title compound under H in Example 230 (Scheme 32) from (3R,4S)-3-(isopropylamino-methyl)-4-(2-trimethylsilanyl-ethoxycarbonylamino)-pyrrolidine-1-carboxylic acid tert-butyl ester.

### J. (3S,4R)-3-Amino-4-({isopropyl-[4-methoxy-3-(3-methoxy-propoxy)-benzoyl]-amino}-methyl)-pyrrolidine-1-carboxylic acid tert-butyl ester

The title compound is prepared analogously as described for the title compound under I in Example 230 (Scheme 32) from (3R,4S)-3-({isopropyl-[4-methoxy-3-(3-methoxy-propoxy)-benzoyl]-amino}-methyl)-4-(2-trimethylsilanyl-ethoxycarbonylamino)-pyrrolidine-1-carboxylic acid tert-butyl ester.

### K. (3R,4S)-3-({isopropyl-[4-methoxy-3-(3-methoxy-propoxy)-benzoyl]-amino}-methyl)-4-phenylmethanesulfonylamino-pyrrolidine-1-carboxylic acid tert-butyl ester

To a solution of (3S,4R)-3-amino-4-({isopropyl-[4-methoxy-3-(3-methoxy-propoxy)-benzoyl]-amino}-methyl)-pyrrolidine-1-carboxylic acid tert-butyl ester (750 mg, 1.56 mmol) in CH₂Cl₂ (15 mL), benzylsulfonyl chloride (418 mg, 2.19 mmol) and triethylamine (305 µL, 0.25 mmol) are added under N₂ atmosphere. The mixture is stirred overnight at RT, diluted with CH₂Cl₂ and poured into an aqueous saturated solution of NaHCO₃. The organic layer is separated, and the aqueous one is extracted twice with CH₂Cl₂. The combined organic extracts are dried over Na₂SO₄, filtered and concentrated. The crude material is purified by flash chromatography on silica gel (eluent: c-hexane/AcOEt 1/1 to 1/2) to give the title compound. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 6.07 min.

### L. N-isopropyl-4-methoxy-3-(3-methoxy-propoxy)-N-((3S,4S)-4-phenylmethane sulfonylamino-pyrrolidin-3-ylmethyl)-benzamide

To a solution of (3R,4S)-3-({Isopropyl-[4-methoxy-3-(3-methoxy-propoxy)-benzoyl]-amino}-methyl)-4-phenylmethanesulfonylamino-pyrrolidine-1-carboxylic acid tert-butyl ester (750 mg, 1.18 mmol) in 6 mL dioxane, a solution of HCl 4N in dioxane (2 mL) is added. The mixture is stirred at RT for 2 h and lyophilized to afford the corresponding hydrochloride salt as a white powder. MS (LC-MS): 534.0 [M+H]⁺; t_{R} (Nucleosil C18 column, 10-100% CH₃CN/H₂O/5 min, 100% CH₃CN/3 min, CH₃CN and H₂O containing 0.1% TFA, flow: 1.5 mL/min): 4.35 min.

### Example 270: N-((3S,4S)-4-Cyclohexylmethanesulfonylamino-pyrrolidin-3-ylmethyl)-N-isopropyl-4-methoxy-3-(3-methoxy-propoxy)-benzamide

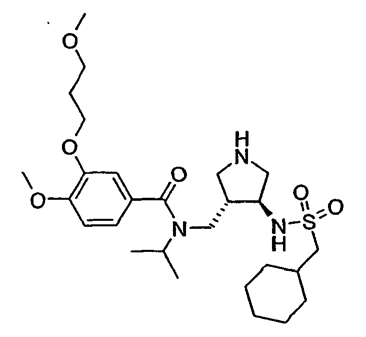

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and cyclohexyl-methanesulfonyl chloride. MS (LC-MS): 540 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.96 min.

### Example 271: N-[(3S,4S)-4-(4-Fluoro-phenylmethanesulfonylamino)-pyrrolidin-3-ylmethyl]-N-isopropyl-4-methoxy-3-(3-methoxy-propoxy)-benzamide

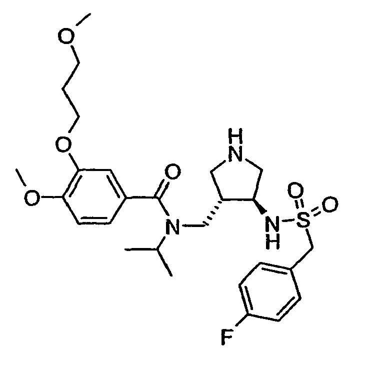

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and (4-fluoro-phenyl)-methanesulfonyl chloride. MS (LC-MS): 552 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.22 min.

### Example 272: N-Isopropyl-4-methoxy-N-[(3S,4S)-4-(3-methoxy-phenylmethanesulfonyl

### amino)-pyrrolidin-3-ylmethyl]-3-(3-methoxy-propoxy)-benzamide

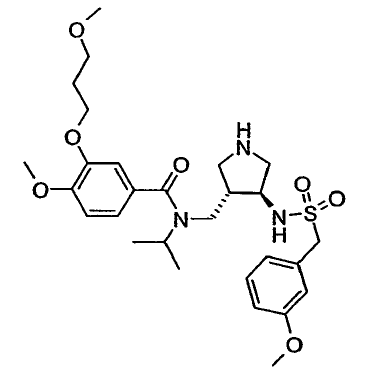

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and (3-methoxy-phenyl)-methanesulfonyl chloride. MS (LC-MS): 564 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.02 min.

### (3-Methoxy-phenyl)-methanesulfonyl chloride

### a. (3-Methoxy-phenyl)-methanesulfonic acid sodium salt

To a solution of 3-methoxybenzylchloride (2.0 g, 12.8 mmol) in acetonee (32 mL) is added an aqueous solution of sodium sulfite (1.7 g, 13.4 mmol) dissolved in water (22 mL) and the mixture is refluxed for 15h. After completion of the reaction, the mixture is cooled to RT and concentrated. The resulting white precipitate is washed with CH₂Cl₂ (30 mL) and dried under high vacuum to give the title compound. ¹H NMR (D₂O, 300 MHz): δ = 3.72 (s, 3H), 4.03 (s, 2H), 6.85-6.92 (m, 3H), 7.22 (t, 1H).

### b. (3-Methoxy-phenyl)-methanesulfonyl chloride

To a solution of (3-methoxy-phenyl)-methanesulfonic acid sodium salt (500 mg, 2.23 mmol) in CH₂Cl₂ (10mL) and DMF (0.27 mL) is slowly added a solution containing 20% COCl₂ in toluene (2.23 mL) and the mixture is stirred overnight at RT. The reaction mixture is diluted with CH₂Cl₂ and slowly poured into an ice-water mixture. The layers are separated and the aqueous one extracted twice with CH₂Cl₂. The combined organic layers are dried over Na₂SO₄, filtered and concentrated to give the title compound. ¹H NMR (CD₃OD, 300 MHz): δ = 3.82 (s, 3H), 5.19 (s, 2H), 7.03 (d, 1H), 7.10 (m, 2H), 7.34 (t, 1H).

### Example 273: N-[(3S,4S)-4-(3-Chloro-phenylmethanesulfonylamino)-pyrrolidin-3-ylmethyl]-N-isopropyl-4-methoxy-3-(3-methoxy-propoxy)-benzamide

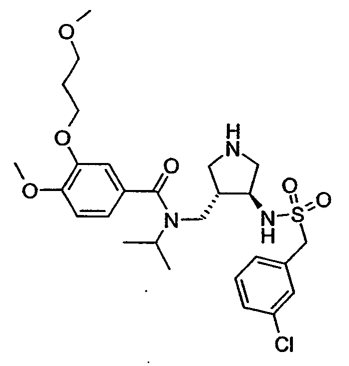

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and (3-chloro-phenyl)-methanesulfonyl chloride. MS (LC-MS): 568-570 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.19 min.

### Example 274: N-[(3S,4S)-4-(4-Chloro-phenylmethanesulfonylamino)-pyrrolidin-3-ylmethyl]-N-isopropyl-4-methoxy-3-(3-methoxy-propoxy)-benzamide

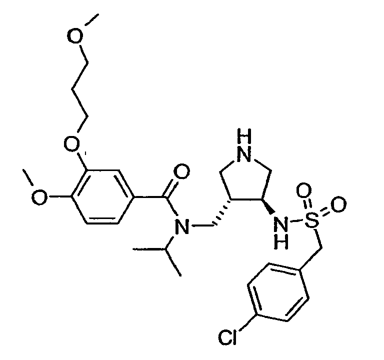

The title compound is prepared analogously as described for the title compound under L in Example 269 from (3S,4R)-3-amino-4.-({isopropyl-[4-methoxy-3-(3-methoxy-propoxy)-benzoyl]-amino}-methyl)-pyrrolidine-1-carboxylic acid tert-butyl ester and (4-chloro-phenyl)-methanesulfonyl chloride. MS (LC-MS): 568-570 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.16 min.

### Example 275: N-Isopropyl-4-methoxy-3-(3-methoxy-propoxy)-N-[(3S,4S)-4-(4-phenoxy-benzenesulfonylamino)-pyrrolidin-3-ylmethyl]-benzamide

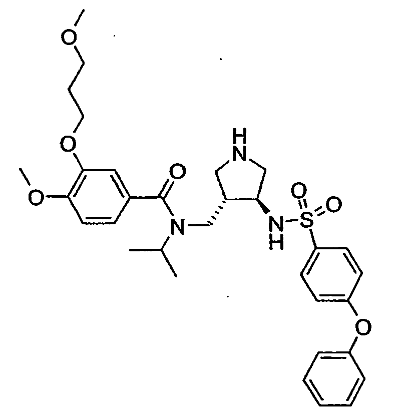

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 4-phenoxy-benzenesulfonyl chloride. MS (LC-MS): 612 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.43 min.

### Example 276: N-Isopropyl-4-methoxy-3-(3-methoxy-propoxy)-N-[(3S,4S)-4-(2-phenyl-ethanesulfonylamino)-pyrrolidin-3-ylmethyl]-benzamide

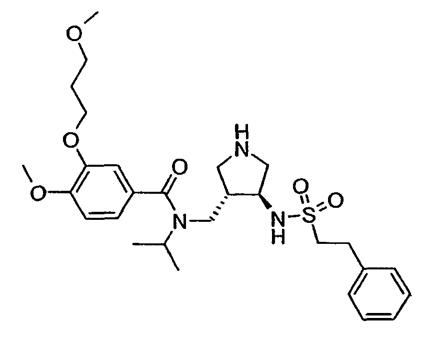

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 2-phenyl-ethanesulfonyl chloride. MS (LC-MS): 548 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.20 min.

### Example 277: N-Isopropyl-4-methoxy-3-(3-methoxy-propoxy)-N-((3S,4S)-4-m-tolyl methanesulfonylamino-pyrrolidin-3-ylmethyl)-benzamide

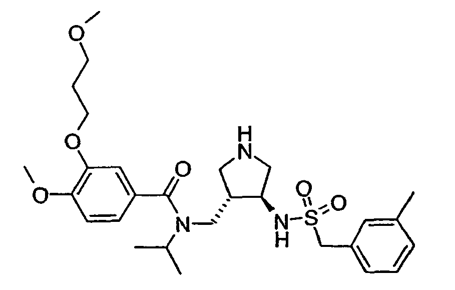

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 3-tolyl-methanesulfonyl chloride. MS (LC-MS): 548 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.28 min.

### Example 278: N-Isopropyl-4-methoxy-3-(3-methoxy-propoxy)-N-[(3S,4S)-4-(4-trifluoro methyl -phenylmethanesulfonylamino)-pyrrolidin-3-ylmethyl]-benzamide

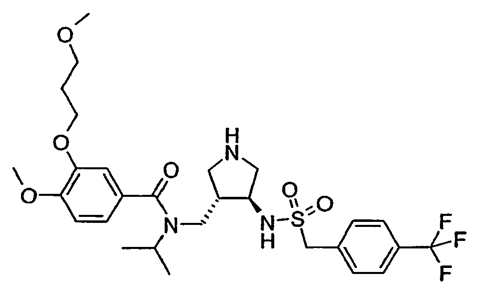

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 4-(trifluoromethylphenyl)-methanesulfonyl chloride. MS (LC-MS): 601.9 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.37 min.

### Example 279: N-((3S,4S)-4-Cyclopentylmethanesulfonylamino-pyrrolidin-3-ylmethyl)-N-isopropyl-4-methoxy-3-(3-methoxy-propoxy)-benzamide

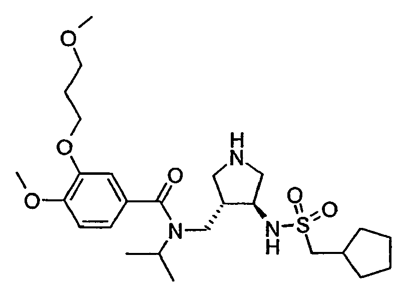

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and cyclopentyl-methanesulfonyl chloride. MS (LC-MS): 526 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.18 min.

### Example 280: N-Isopropyl-4-methoxy-3-(3-methoxy-propoxy)-N-((3S,4S)-4-p-tolyl methanesulfonylamino-pyrrolidin-3-ylmethyl)-benzamide

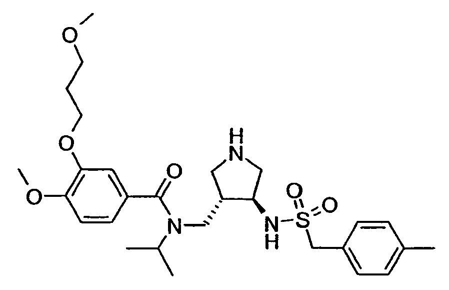

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 4-tolyl-methanesulfonyl chloride. MS (LC-MS): 548 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.22 min.

### Example 281: N-[(3S,4S)-4-(4-Isopropoxy-benzenesulfonylamino)-pyrrolidin-3-ylmethyl]-N-isopropyl-4-methoxy-3-(3-methoxy-propoxy)-benzamide

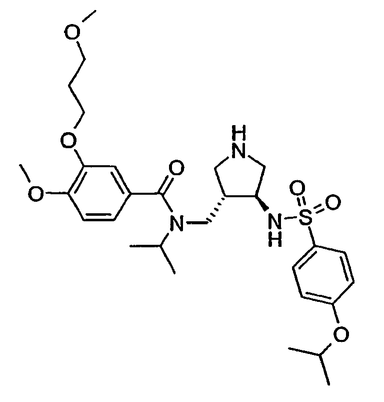

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 4-isopropoxy-benzenesulfonyl chloride. MS (LC-MS): 578 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.43 min.

### Example 282: N-Isopropyl-4-methoxy-3-(3-methoxy-propoxy)-N-[(3S,4S)-4-(4-trifluoro methoxy-benzenesulfonylamino)-pyrrolidin-3-ylmethyl]-benzamide

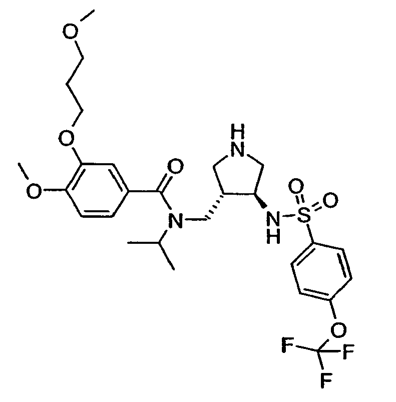

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 4-trifluoromethoxy-benzenesulfonyl chloride. MS (LC-MS): 603.9 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.45 min.

### Example 283: N-[(3S,4S)-4-(2-Chloro-yhenylmethanesulfonylamino)-pyrrolidin-3-ylmethyl]-N-isopropyl-4-methoxy-3-(3-methoxy-propoxy)-benzamide

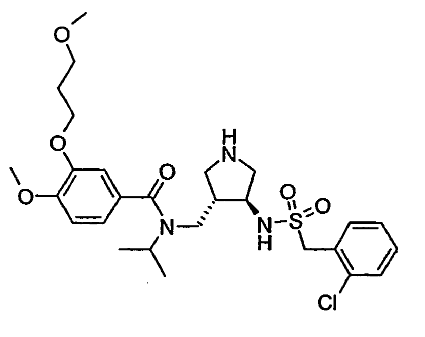

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and (2-chloro-phenyl)-methanesulfonyl chloride. MS (LC-MS): 568-570 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.45 min.

### Example 284: N-Isopropyl-4-methoxy-3-(3-methoxy-propoxy)-N-[(3S.4S)-4-(propane-1-sulfonylamino)-pyrrolidin-3-ylmethyl]-benzamide

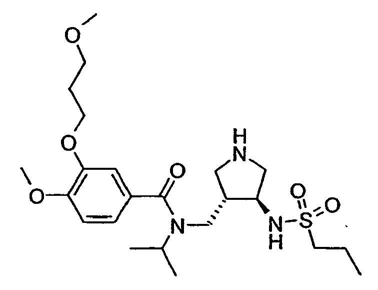

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 1-propylsulfonylchloride. MS (LC-MS): 486.2 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mUmin): 5.08 min.

### Example 285: N-Isopropyl-4-methoxy-3-(3-methoxy-propoxy)-N-[(3S,4S)-4-(tetrahydro-pyran-4-ylmethanesulfonylamino)-pyrrolidin-3-ylmethyl]-benzamide

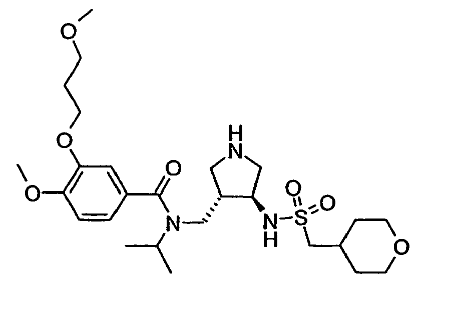

The title compound is prepared analogously as described for the title compound under L in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and (tetrahydro-pyran-4-yl)-methanesulfonyl chloride. MS (LC-MS): 542.3 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.02 min.

### (Tetrahydro-pyran-4-yl)-methanesulfonyl chloride

The title compound is prepared analogously as described for the title compound (3-methoxyphenyl)-methanesulfonyl chloride in Example 272 from 4-bromomethyl-tetrahydro-pyran. ¹H NMR (CDCl₃, 400 MHz): δ =1.51-1.62 (m, 2H), 1.92-1.96 (m, 2H), 2.50 (m, 1H), 3.47-3.53 (m, 2H), 3.70 (d, 2H), 3.98-4.06 (m, 2H).

### Example 286: N-[(3S,4S)-4-(3-Acetylamino-phenylmethanesulfonylamino)-pyrrolidin-3-ylmethyl]-N-isopropyl-4-methoxy-3-(3-methoxy-propoxy)-benzamide

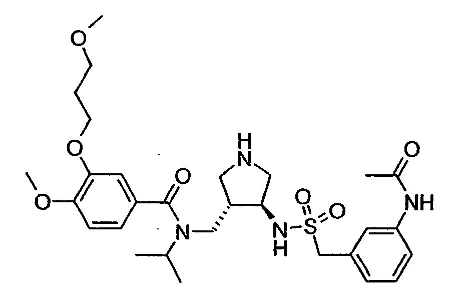

### A. (3R,4S)-3-({Isopropyl-[4-methoxy-3-(3-methoxy-propoxy)-benzoyl]-amino}-methyl)-4-(3-nitro-phenylmethanesulfonylamino)-pyrrolidine-1-carboxylic acid tert-butyl ester

The title compound is prepared analogously as described for the title compound under K in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 3-nitro-benzenesulfonylchloride. MS (LC-MS): 622.8 [M+H-tBu]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 6.08 min.

### B. (3S,4R)-3-(3-Amino-phenylmethanesulfonylamino)-4-({isopropyl-[4-methoxy-3-(3-methoxy-propoxy)-benzoyl]-amino}-methyl)-pyrrolidine-1-carboxylic acid tert-butyl ester

A mixture of (3R,4S)-3-({isopropyl-[4-methoxy-3-(3-methoxy-propoxy)-benzoyl]-amino}-methyl)-4-(3-nitro-phenylmethanesulfonylamino)-pyrrolidine-1-carboxylic acid tert-butyl ester (400 g, 0.59 mmol) and Pd/C (10%, 40 mg) in MeOH (8 mL) is stirred under an hydrogen atmosphere for 1 h. The crude material is filtered over a pad of Celite, dried over Na₂SO₄ and concentrated. Chromatography on silica gel (eluent: hexane/AcOEt 90/10) of the crude material gives the title compound. MS (LC-MS): 649.0 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.01 min.

### C. (3S,4R)-3-(3-Acetylamino-phenylmethanesulfonylamino)-4-({isopropyl-[4-methoxy-3-(3-methoxy-propoxy)-benzoyl]-amino}-methyl)-pyrrolidine-1-carboxylic acid tert-butyl ester

To an ice cooled solution of (3S,4R)-3-(3-amino-phenylmethanesulfonylamino)-4-({isopropyl-[4-methoxy-3-(3-methoxy-propoxy)-benzoyl]-amino}-methyl)-pyrrolidine-1-carboxylic acid tert-butyl ester (280 mg, 0.431 mmol) in CH₂Cl₂ (15 mL) are added acetyl chloride (33.5 µL, 0.475 mmol) and Et₃N (66 µL, 0.475 mmol). The resulting mixture is stirred 2 h at 0°C and allowed to reach RT for 2 h. Acetyl chloride (33.5 µL, 0.475 mmol) and Et₃N (66 µL, 0.475 mmol) in CH₂Cl₂ (1 mL) are added and the mixture stirred for 3 additional hours to complete the reaction. The mixture is diluted with CH₂Cl₂, an aqueous saturated solution of NaHCO₃ is added and the layers are separated, the aqueous on being extracted twice with CH₂Cl₂. The combined organic extracts are dried over Na₂SO₄, filtered and concentrated to give the title compound which is used in the next step without further purification. MS (LC-MS): 691 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.90 min.

### D. N-[(3S,4S)-4-(3-Acetylamino-phenylmethanesulfonylamino)-pyrrolidin-3-ylmethyl]-N-isopropyl-4-methoxy-3-(3-methoxy-propoxy)-benzamide

To a solution of (3S,4R)-3-(3-acetylamino-phenylmethanesulfonylamino)-4-({isopropyl-[4-methoxy-3-(3-methoxy-propoxy)-benzoyl]-amino}-methyl)-pyrrolidine-1-carboxylic acid tert-butyl ester (281 mg, 0.41 mmol) in 3 mL dioxane, a solution of HCl 4N in dioxane (1.5 mL) is added. The mixture is stirred at RT for 2 h and lyophilized to afford the corresponding hydrochloride salt as a white powder. MS (LC-MS): 591 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.16 min.

### Example 287: N-{(3S,4S)-4-[Ethyl-(4-fluoro-phenylmethanesulfonyl)-amino]-pyrrolidin-3-ylmethyl}-N-isopropyl-4-methoxy-3-(3-methoxy-propoxy)-benzamide

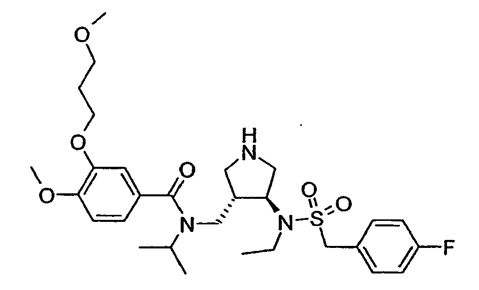

### A. (35,4R)-3-(4-Fluoro-phenylmethanesulfonylamino)-4-({isopropyl-[4-methoxy-3-(3-methoxy-propoxy)-benzoyl]-amino}-methyl)-pyrrolidine-1-carboxylic acid tert-butyl ester

The title compound is prepared analogously as described for the title compound under K in Example 269 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and (4-fluoro-phenyl)-methanesulfonyl chloride. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.88 min.

### B. (3S,4R)-3-[Ethyl-(4-fluoro-phenylmethanesulfonyl)-amino]-4-({isopropyl-[4-methoxy-3-(3-methoxy-propoxy)-benzoyl]-amino}-methyl)-pyrrolidine-1-carboxylic acid tert-butyl ester

To a solution of (3S,4R)-3-(4-fluoro-phenylmethanesulfonylamino)-4-({isopropyl-[4-methoxy-3-(3-methoxy-propoxy)-benzoyl]-amino}-methyl)-pyrrolidine-1-carboxylic acid tert-butyl ester (200 mg, 0.307 mmol) in DMF (3 mL) are added Cs₂CO₃ (130 mg, 0.399 mmol) and iodoethane (62 mg, 0.399 mmol). The mixture is stirred at 80°C in the microwave for 2 hours. The reaction mixture is poured into a saturated aqueous solution of NaHCO₃, AcOEt is added and the layers are separated, the aqueous one being back-extracted twice with AcOEt. The combined organic extracts are dried over Na₂SO₄, filtered and concentrated. The crude mixture material is purified flash chromatography (eluent: c-hexane/AcOEt 1/1 to 1/2) to afford the title product. MS (LC-MS): 624 [M+HtBu]⁺; t_{R} (HPLC, nucleosil C18 column, 10-100% CH₃CN/H₂O/5 min, 100% CH₃CN/3 min, flow: 1.5 mL/min): 5.87 min.

### C. N-{(3S,4S)-4-[Ethyl-(4-fluoro-phenylmethanesulfonyl)-amino]-pyrrolidin-3-ylmethyl}-N-isopropyl-4-methoxy-3-(3-methoxy-propoxy)-benzamide

To a solution of (3S,4R)-3-[ethyl-(4-fluoro-phenylmethanesulfonyl)-amino]-4-({isopropyl-[4-methoxy-3-(3-methoxy-propoxy)-benzoyl]-amino}-methyl)-pyrrolidine-1-carboxylic acid tert-butyl ester (160 mg, 0.235 mmol) in 2 mL dioxane, a solution of HCl 4N in dioxane (1.5 mL) is added. The mixture is stirred at RT for 2 h and lyophilized to afford the corresponding hydrochloride salt as a white powder. MS (LC-MS): 580 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.63 min.

### Example 288: 4-Ethyl-N-isopropyl-3-(3-methoxy-propoxy)-N-((3S,4S)-4-phenylmethane sulfonylamino-pyrrolidin-3-ylmethyl)-benzamide

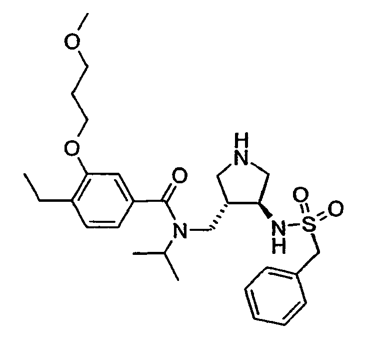

### A. (3S,4R)-3-Amino-4-({[4-ethyl-3-(3-methoxy-propoxy)-benzoyl]-isopropyl-amino}-methyl)-pyrrolidine-1-carboxylic acid tert-butyl ester

The title compound is prepared analogously as described for the title compound under J in Example 269 from (3R,4S)-3-(isopropylamino-methyl)-4-(2-trimethylsilanyl-ethoxycarbonyl amino)-pyrrolidine-1-carboxylic acid tert-butyl ester and 4-ethyl-3-(3-methoxy-propoxy)-benzoic acid (described in example 60). TLC, Rf (CH2CI2/MeOH 90/10) = 0.3. MS (LC-MS): 478.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.56 min.

### B. 4-Ethyl-N-isopropyl-3-(3-methoxy-propoxy)-N-((3S,4S)-4-phenylmethanesulfonyl amino-pyrrolidin-3-ylmethyl)-benzamide

The title compound is prepared analogously as described for the title compound under L in Example 269 from (3S,4R)-3-amino-4-({[4-ethyl-3-(3-methoxy-propoxy)-benzoyl]-isopropylamino}-methyl)-pyrrolidine-1-carboxylic acid tert-butyl ester and phenyl-methanesulfonyl chloride. MS (LC-MS): 532 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.58 min.

### Example 289: 4-Ethyl-N-[(3S,4S)-4-(4-fluoro-phenvimethanesulfonylamino)-pyrrolidin-3-ylmethyl]-N-isopropyl-3-(3-methoxy-propoxy)-benzamide

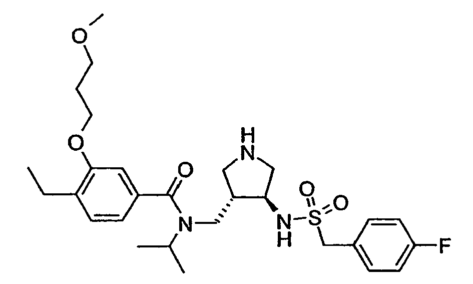

The title compound is prepared analogously as described for the title compound under B in example 288 from (3S,4R)-3-amino-4-({[4-ethyl-3-(3-methoxy-propoxy)-benzoyl]-isopropylamino}-methyl)-pyrrolidine-1-carboxylic acid tert-butyl ester and (4-fluoro-phenyl)-methanesulfonyl chloride. MS (LC-MS): 550.2 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.66 min.

### Example 290: N-Isopropyl-4-methoxy-N-((3S,4S)-4-phenylmethanesulfonylamino-pyrrolidin-3-ylmethyl)-3-[2-(tetrahydro-furan-2-yl)-ethoxy]-benzamide

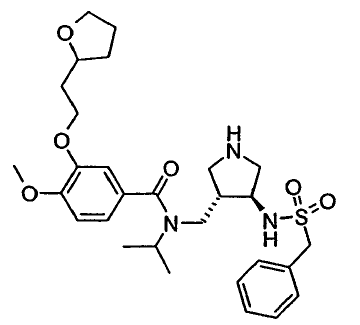

### A. (35,4R)-3-Amino-4-[(isopropyl-{4-methoxy-3-[2-(tetrahydro-furan-2-yl)-ethoxy]-benzoyl}-amino)-methyl]-pyrrolidine-1-carboxylic acid tert-butyl ester

The title compound is prepared analogously as described for the title compound under J in Example 269 from (3R,4S)-3-(isopropylamino-methyl)-4-(2-trimethylsilanyl-ethoxycarbonyl amino)-pyrrolidine-1-carboxylic acid tert-butyl ester and 4-methoxy-3-[2-(tetrahydro-furan-2-yl)-ethoxy]-benzoic acid. TLC, Rf (CH₂Cl₂/MeOH 90/10) = 0.3. MS (LC-MS): 506.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.18 min.

### B. N-lsopropyl-4-methoxy-N-((3S,4S)-4-phenylmethanesulfonylamino-pyrrolidin-3-ylmethyl)-3-[2-(tetrahydro-furan-2-yl)-ethoxy]-benzamide

The title compound is prepared analogously as described for the title compound under L in Example 269 from (3S,4R)-3-amino-4-[(isopropyl-{4-methoxy-3-[2-(tetrahydro-furan-2-yl)-ethoxy]-benzoyl}-amino)-methyl]-pyrrolidine-1-carboxylic acid tert-butyl ester and phenyl-methanesulfonyl chloride. MS (LC-MS): 560 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.27 min.

### 4-Methoxy-3-[2-(tetrahydro-furan-2-yl)-ethoxy]-benzoic acid

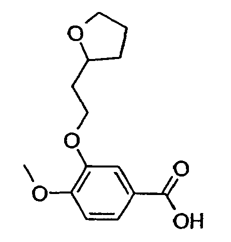

### a. 2-(Tetrahydro-furan-2-yl)-ethanol

To a solution of tetrahydrofuran-2-acetic acid ethyl ester (5.11 g, 32.3 mmol) in THF (70 mL) is added by portion LiBH₄ (4.22 g, 193.8 mmol) and the resulting solution is heated at 70°C overnight. The mixture is diluted with AcOEt and quenched with water. The layers are separated and the aqueous one extracted twice with AcOEt. The combined organic extracts are dried over Na₂SO₄, filtered and concentrated to give the title product. ¹H NMR (CD₃OD, 300 MHz): δ=1.48-1.57 (m, 1H), 1.66-1.80 (m, 2H), 1.87-1.95 (m, 2H), 1.99-2.09 (m, 1H), 3.64 (t, 2H), 3.72 (t, 1H), 3.80-3.87 (m, 1H), 3.94 (pent., 1H).

### b. Toluene-4-sulfonic acid 2-(tetrahydro-furan-2-yl)-ethyl ester

To a solution of 2-(tetrahydro-furan-2-yl)-ethanol (2.04 g, 17.59 mmol) in CH₂Cl₂ (20 mL) at 0°C is added (3.69 g, 19.35 mmol) of toluene-4-sulfonyl chloride, followed by triethylamine (2.7 mL , 19.35 mmol). The reaction mixture is allowed to reach RT and stirred overnight. Then poured into an aqueous saturated solution of NaHCO₃. The layers are separated and the aqueous one extracted twice with CH₂Cl₂. The combined organic extracts are dried over Na₂SO₄, filtered and concentrated. The crude material is purified by flash chromatography on silica gel (eluent: c-hexane-AcOEt 80/20) to give the title compound. TLC, Rf (c-Hexan/AcOEt 80/20) = 0.25. MS (LC-MS): 271.0 [M+H]⁺; t_{R} (HPLC, nucleosil C18 column, 10-100% CH₃CN/H₂O/5 min, 100% CH₃CN/3 min, flow: 1.5 mL/min): 5.27 min.

### c. 4-Methoxy-3-[2-(tetrahydro-furan-2-yl)-ethoxy]-benzoic acid methyl ester

A mixture of toluene-4-sulfonic acid 2-(tetrahydro-furan-2-yl)-ethyl ester (3.4 g, 12.58 mmol), methyl 3-hydroxy-4-methoxy-benzoate (1.91 g, 10.48 mmol) and K₂CO₃ (1.74 g, 12.58 mmol) in DMF (60 mL) is heated at 80°C overnight. AcOEt is added and the reaction mixture quenched by addition of an aqueous saturated solution of NaHCO₃. The layers are separated and the aqueous one extracted twice with AcOEt. The combined organic extracts are dried over Na₂SO₄, filtered and concentrated. The crude material is purified by flash chromatography on silica gel (eluent: c-hexane/AcOEt 70/30) to give the title compound. TLC, Rf (c-hexane/AcOEt) = 0.25. MS (LC-MS): 281.0 [M+H]⁺; t_{R} (HPLC, nucleosil C18 column, 10-100% CH₃CN/H₂O/5 min, 100% CH₃CN/3 min, flow: 1.5 mL/min): 5.17 min.

### d. 4-Methoxy-3-[2-(tetrahydro-furan-2-yl)-ethoxy]-benzoic acid

LiOH.H₂O (8.31 g, 198 mmol) is added to a solution of 4-methoxy-3-[2-(tetrahydro-furan-2-yl)-ethoxy]-benzoic acid methyl ester (3.09 g, 11 mmol) in MeOH (70 mL) cooled at 0°C. The reaction mixture is allowed to reach RT and stirred for 3 days. The mixture is acidified by addition of HCl 1 N and extracted three times with CH₂Cl₂. The combined organic layers are dried over Na₂SO₄, filtered and concentrated to give the title compound. TLC, Rf (c-hexane/AcOEt 2/1) = 0.1. MS (LC-MS): 267.1 [M+H]⁺; t_{R} (HPLC, nucleosil C18 column, 10-100% CH₃CN/H₂O/5 min, 100% CH₃CN/3 min, flow: 1.5 mL/min): 4.44 min.

### Example 291: 4-(3-Methoxy-propyl)-3,4-dihydro-2H-benzo[1,4]oxazine-6-carboxylic acid isopropyl-((3S,4S)-4-phenylmethanesulfonylamino-pyrrolidin-3-ylmethyl)-amide

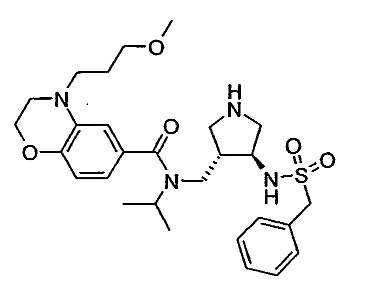

### A. (3S,4R)-3-Amino-4-({isopropyl-[4-(3-methoxy-propyl)-3,4-dihydro-2H-benzo[1,4]oxazine-6-carbonyl]-amino}-methyl)-pyrrolidine-1-carboxylic acid tert-butyl ester

The title compound is prepared analogously as described for the title compound under J in Example 269 from (3R,4S)-3-(isopropylamino-methyl)-4-(2-trimethylsilanyi-ethoxycarbonyl amino)-pyrrolidine-1-carboxylic acid tert-butyl ester and 4-(3-methoxy-propyl)-3,4-dihydro-2H-benzo[1,4]oxazine-6-carboxylic acid. TLC, Rf (CH₂Cl₂/MeOH 90/10) = 0.5. MS (LC-MS): 491.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.90 min.

### B. N-Isopropyl-4.-methoxy-N-((3S,4S)-4-phenylmethanesulfonylamino-pyrrolidin-3-ylmethyl)-3-[2-(tetrahydro-furan-2-yl)-ethoxy]-benzamide

The title compound is prepared analogously as described for the title compound under L in Example 269 from (3S,4R)-3-amino-4-({isopropyl-[4-(3-methoxy-propyl)-3,4-dihydro-2H-benzo[1,4]oxazine-6-carbonyl]-amino}-methyl)-pyrrolidine-1-carboxylic acid tert-butyl ester and phenyl-methanesulfonyl chloride. MS (LC-MS): 545 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.97 min.

### 4-(3-Methoxy-propyl)-3,4-dihydro-2H-benzo[1,4]oxazine-6-carboxylic acid

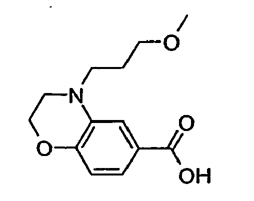

### a. 3-tert-Butoxycarbonylamino-4-hydroxy-benzoic acid methyl ester

A mixture of methyl 3-amino-4-hydroxybenzoate (3.89 g, 23.26 mmol) and di-tert-butyldicarbonate (10.15 g, 46.52 mmol) in THF is stirred at RT for 2 days. The mixture is concentrated, diluted with AcOEt and quenched with a saturated aqueous solution of NaHCO₃. The layers are separated and the aqueous one extracted twice with AcOEt, dried over Na₂SO₄, filtered and concentrated to give the title product, which is used in the next step without further purification. TLC, R_{f} (CH2Cl2/MeOH 95/5) = 0.45. MS (LC-MS): 266.1 [M-H]⁻; t_{R} (HPLC, nucleosil C18 column, 10-100% CH₃CN/H₂O/5 min, 100% CH₃CN/3 min, flow: 1.5 mL/min): 5.05 min.

### b. 2,3-Dihydro-benzo[1,4]oxazine-4,6-dicarboxylic acid 4-tert-butyl ester 6-methyl ester

A solution of 3-tert-butoxycarbonylamino-4-hydroxy-benzoic acid methyl ester (1.87 g, 6.985 mmol), dibromoethane (2.41 mL, 27.94 mmol) and K₂CO₃ (4.83 g, 34.925 mmol) in pentan-3-one (70 mL) is heated in the microwave for 2 h at 105°C. The reaction mixture is filtered and quenched by addition of an aqueous saturated solution of NaHCO₃. The layers are separated and the aqueous one extracted twice with AcOEt. The combined organic extracts are dried over Na₂SO₄, filtered and concentrated. The crude material is purified by flash chromatography on silica gel (eluent: c-hexane/AcOEt 90/10) to give the title product. TLC, R_{f} (c-hexane/AcOEt 90/10) = 0.3. MS (LC-MS): 238 [M+H-tBu]⁺; t_{R} (HPLC, nucleosil C18 column, 10-100% CH₃CN/H₂O/5 min, 100% CH₃CN/3 min, flow: 1.5 mL/min): 5.78 min.

### c. 3,4-Dihydro-2H-benzo[1,4]oxazine-6-carboxylic acid methyl ester

TFA (1.14 mL) is added to a solution of 2,3-dihydro-benzo[1,4]oxazine-4,6-dicarboxylic acid 4-tert-butyl ester 6-methyl ester (722 mg, 2.46 mmol) in CH₂Cl₂ (20 mL) and the resulting solution is stirred overnight at RT. Then diluted with CH₂Cl₂ and quenched with a saturated aqueous solution of NaHCO₃. The layers are separated and the aqueous one extracted twice with CH2Cl2, dried over Na₂SO₄, filtered and concentrated to give the title product, which is used without further purification in the next step.. TLC, Rf (c-hexane/AcOEt 80/20) = 0.2. MS (LC-MS): 194.1 [M+H]⁺; t_{R} (HPLC, nucleosil C18 column, 10-100% CH3CN/H2O/5 min, 100% CH3CN/3 min, flow: 1.5 mL/min): 4.26 min.

### d. 4-(3-Methoxy-propyl)-3,4-dihydro-2H-benzo[1,4]oxazine-6-carboxylic acid methyl ester

To a suspension of 3,4-dihydro-2H-benzo[1,4]oxazine-6-carboxylic acid methyl ester (3 g, 16.5 mmol) and Cs₂CO₃ (1.09 g, 3.34 mmol) in DMF (5 mL) is added 1-bromo-3-methoxypropane (510 mg, 3.34 mmol), and the mixture is stirred at 80°C for 2 days. Then poured into an aqueous saturated solution of NaHCO₃, AcOEt is added and the layers are separated, the aqueous one being extracted twice with AcOEt. The combined organic extracts are dried over Na₂SO₄, filtered and concentrated. The crude material was purified by flash chromatography on silica gel (eluent: c-hexane/AcOEt 80/20) to give the title compound. MS (LC-MS): 266.0 [M+H]⁺; t_{R} (HPLC, C18 column, 10-100% CH₃CN/H₂O/5 min, 100% CH₃CN/3 min, flow: 1.5 mL/min): 5.45 min.

### e. 4-(3-Methoxy-propyl)-3,4-dihydro-2H-benzo[1,4]oxazine-6-carboxylic acid

LiOH.H₂O (707 mg, 16.85 mmol) is added to a 0°C solution of 4-(3-methoxy-propyl)-3,4-dihydro-2H-benzo[1,4]oxazine-6-carboxylic acid methyl ester (298 mg, 1.123 mmol) in MeOH (10 mL). The reaction mixture is allowed to reach RT and stirred overnight. As the reaction is not complete THF and H₂O respectively (10 mL and 1 mL) are added and the mixture was heated at 50°C and stirred overnight. The mixture is neutralized with aqueous 4N HCl (16.85 mmol, 4.21 mL) and extracted with CH₂Cl₂ (3 times). The combined organic extracts are dried (Na₂SO₄), filtered and concentrated. The crude mixture is purified by flash chromatography on silica gel (CH₂Cl₂/MeOH 100/0 to 95/5) to give the title product. TLC, R_{f} (CH₂Cl₂/MeOH95/5) = 0.35. MS (LC-MS): 252.2 [M-H]⁺;
t_{R} (HPLC, nucleosil C18 column, 10-100% CH₃CN/H₂O/5 min, 100% CH₃CN/3 min, flow: 1.5 mL/min): 4.76 min.

### Example 292: 1-(3-Methoxy-propyl)-3-methyl-1H-indole-6-carboxylic acid isopropyl-((3S,4S)-4-phenylmethanesulfonylamino-pyrrolidin-3-ylmethyl)-amide

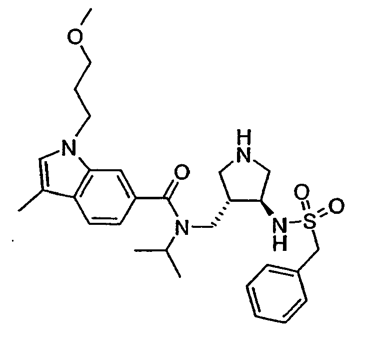

### A. (3S,4R)-3-Amino-4-({isopropyl-[1-(3-methoxy-propyl)-3-methyl-1H-indole-6-carbonyl]-amino}-methyl)-pyrrolidine-1-carboxylic acid tert-butyl ester

The title compound is prepared analogously as described for the title compound under J in Example 269 from (3R,4S)-3-(isopropylamino-methyl)-4-(2-trimethylsilanyl-ethoxycarbonyl amino)-pyrrolidine-1-carboxylic acid tert-butyl ester and 1-(3-methoxy-propyl)-3-methyl-1H-indole-6-carboxylic acid (described in example 65). TLC, R_{f} (CH2Cl2/MeOH 90/10) = 0.5. MS (LC-MS): 487.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.19 min.

### B. 1-(3-Methoxy-propyl)-3-methyl-1H-indole-6-carboxylic acid isopropyl-((3S,4S)-4-phenylmethanesulfonylamino-pyrrolidin-3-ylmethyl)-amide

The title compound is prepared analogously as described for the title compound under L in Example 269 from (3S,4R)-3-amino-4-({isopropyl-[1-(3-methoxy-propyl)-3-methyl-1H-indole-6-carbonyl]-amino}-methyl)-pyrrolidine-1-carboxylic acid tert-butyl ester and phenyl-methanesulfonyl chloride. MS (LC-MS): 541 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.31 min.

### Example 293: 3-(2-Acetylamino-ethoxy)-N-isopropyl-4-methoxy-N-((3S,4S)-4-phenyl methanesulfonylamino-pyrrolidin-3-ylmethyl)-benzamide

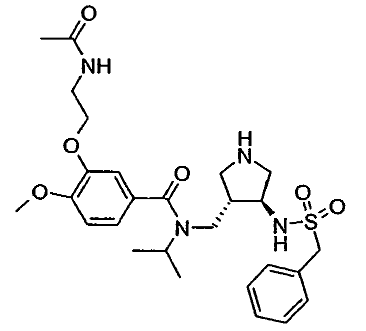

### A. (3R,4S)-3-({[3-(2-Acetylamino-ethoxy)-4-methoxy-benzoyl]-isopropyl-amino}-methyl)-4-amino-pyrrolidine-1-carboxylic acid tert-butyl ester

The title compound is prepared analogously as described for the title compound under J in Example 269 from (3R,4S)-3-(isopropylamino-methyl)-4-(2-trimethylsilanyl-ethoxycarbonyl amino)-pyrrolidine-1-carboxylic acid tert-butyl ester and 3-(2-acetylamino-ethoxy)-4-methoxybenzoic acid. TLC, R_{f} (CH2Cl2/MeOH 90/10) = 0.1. MS (LC-MS): 493 [M+H]⁺; t_{R} (HPLC, Nucleosil C18 column, 10-100% CH₃CN/H₂O/5 min, 100% CH₃CN/3 min, CH₃CN and H₂O containing 0.1 % TFA, flow: 1.5 mL/min): 4.50 min.

### B. 3-(2-Acetylamino-ethoxy)-N-isopropyl-4-methoxy-N-((3S,4S)-4-pheny[methane sulfonylamino-pyrrolidin-3-ylmethyl)-benzamide

The title compound is prepared analogously as described for the title compound under L in Example 269 from (3R,4S)-3-({[3-(2-acetylamino-ethoxy)-4-methoxy-benzoyl]-isopropylamino}-methyl)-4-amino-pyrrolidine-1-carboxylic acid tert-butyl ester and phenyl-methanesulfonyl chloride. MS (LC-MS): 546.9 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.64 min.

### 3-(2-Acetylamino-ethoxy)-4-methoxy-benzoic acid

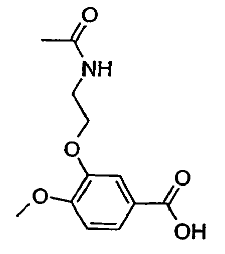

### a. 3-(2-Chloro-ethoxy)-4-methoxy-benzoic acid methyl ester

A mixture of methyl 3-hydroxy-4-methoxy-benzoate (4 g, 21.96 mmol), 1-bromo-2-chloroethane (3.8 g, 26.35 mmol) and K₂CO₃ (24.3 g, 175.7 mmol) in DMF (350 mL) is heated at 80°C for 2 days. Additional 1-bromo-2-chloroethane (9.45 g, 65.8 mmol) is added to complete the reaction and the reaction mixture is further stirred overnight at 80°C. AcOEt is added and the reaction mixture is quenched by addition of water. The layers are separated and the aqueous one extrated twice with AcOEt. The combined organic extracts are dried over Na₂SO₄, filtered and concentrated to give the title compound. TLC, R_{f} (c-hexane/AcOEt 80/20) = 0.30. MS (LC-MS): 245 [M+H]⁺; t_{R} (HPLC, nucleosil C18 column, 10-100% CH₃CN/H₂O/5 min, 100% CH₃CN/3 min, flow: 1.5 mL/min): 5.07 min.

### b. 3-(2-Azido-ethoxy)-4-methoxy-benzoic acid methyl ester

A mixture of 3-(2-chloro-ethoxy)-4-methoxy-benzoic acid methyl ester (3.22 g, 13.18 mmol) and NaN₃ (1.11 g, 17.13 mmol) in DMF (15 mL) is stirred at 80°C overnight under nitrogen. The mixture is allowed to cool to room temperature, AcOEt is added and the reaction mixture is quenched by addition of an aqueous saturated solution of NaHCO₃. The layers are separated and the aqueous one extrated twice with AcOEt, dried over Na₂SO₄, filtered and concentrated. The crude material is purified by flash chromatography on silica gel (eluent: c-hexane/AcOEt 90/10) to give the title compound. TLC, R_{f} (c-hexane/AcOEt 80/20) = 0.35. t_{R} (HPLC, nucleosil C18 column, 10-100% CH₃CN/H₂O/5 min, 100% CH₃CN/3 min, flow: 1.5 mL/min): 5.08 min.

### c. 3-(2-Amino-ethoxy)-4-methoxy-benzoic acid methyl ester

A solution of 3-(2-azido-ethoxy)-4-methoxy-benzoic acid methyl ester (1.2 g, 4.768 mmol) and Pd/C (400 mg, 10%) in MeOH (40 mL) is stirred under an hydrogen atmosphere for 16 h. The reaction mixture is filtered over a pad of celite, dried over Na₂SO₄ and concentrated. The crude material is purified by flash chromatography on silica gel (eluent: c-hexane/AcOEt 80/20, then CH₂Cl₂/MeOH 100/0 to 90/10+ 1 % NH₃) to give the title product. TLC, R_{f} (CH₂Cl₂/MeOH 95/5 + 1% NH₃) = 0.1. t_{R} (HPLC, nucleosil C18 column, 10-100% CH₃CN/H₂O/5 min, 100% CH₃CN/3 min, flow: 1.5 mL/min): 3.43 min.

### d. 3-(2-Acetylamino-ethoxy)-4-methoxy-benzoic acid methyl ester

To a solution of 3-(2-amino-ethoxy)-4-methoxy-benzoic acid methyl ester (1.15 g, 5.09 mmol) in CH₂Cl₂ (40 mL) are added acetyl chloride (434 µL, 6.11 mmol) and triethylamine (850 µL, 6.11 mmol) at 0°C under N₂ atmosphere. The reaction mixture is allowed to reach RT and stirred for 4 h, then diluted with CH₂Cl₂ and poured into an aqueous saturated solution of NaHCO₃. The layers are separated and the aqueous one extracted twice with CH₂Cl₂. The combined organic extracts are dried over Na₂SO₄, filtered and concentrated. The crude product is purified by flash chromatography on silica gel (eluent: c-hexane/MeOH 100/0 to 90/10) to give the title product. TLC, R_{f} (AcOEt) = 0.15. MS (LC-MS): 268.1 [M+H]⁺; t_{R} (HPLC, nucleosil C18 column, 10-100% CH₃CN/H₂O/5 min, 100% CH₃CN/3 min, flow: 1.5 mL/min): 4.30 min.

### e. 3-(2-Acetylamino-ethoxy)-4-methoxy-benzoic acid

LiOH,H₂O (2 g, 46 mmol) is added to a solution of 3-(2-acetylamino-ethoxy)-4-methoxybenzoic acid methyl ester (850 mg, 3.18 mmol) in a mixture of MeOH (20 mL), THF (10 mL) and water (2 mL). The reaction mixture is heated at 50°C and stirred overnight. The mixture is acidified by the addition of 4N aqueous HCl (47.7 mmol, 12 mL), CH₂Cl₂ is added and the layer are separated. The queous layer is extracted twice with CH₂Cl₂ and the combined organic extracts are dried (Na₂SO₄), filtered and concentrated to give the title product. TLC, R_{f} (CH₂Cl₂/MeOH 95/5) = 0.1. MS (LC-MS): 254.0 [M+H]⁺; t_{R} (HPLC, nucleosil C18 column, 10-100% CH₃CN/H₂O/5 min, 100% CH₃CN/3 min, flow: 1.5 mL/min): 3.80 min.

### Example 294: N-[(3S,4S)-4-(3-Benzyl-sulfonylureido)-pyrrolidin-3-ylmethyl]-N-isopropyl-4-methoxy-3-(3-methoxy-propoxy)-benzamide

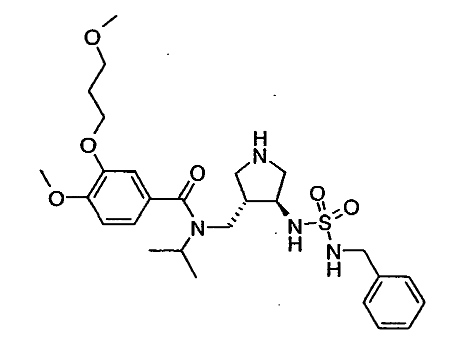

### A. (3S,4R)-3-(3-Benzyl-sulfonylureido)-4-({isopropyl.[4-methoxy-3-(3-methoxy-propoxy)-benzoyl]-amino}-methyl)-pyrrolidine-1-carboxylic acid tert-butyl ester

To a solution of benzylamine (500 µL, 4.17 mmol) in CH₂Cl₂ (30 mL) cooled at 0°C is added dropwise ClSO₃H (334 µL, 5 mmol). The resulting mixture is stirred at RT for 1 h. PCl₅ (1.04 g, 5.00 mmol) is then added and the solution heated at reflux for 4 h. The supernatant of the solution is added to a mixture of (3S,4R)-3-amino-4-({isopropyl-[4-methoxy-3-(3-methoxy-propoxy)-benzoyl]-amino}-methyl)-pyrrolidine-1-carboxylic acid tert-butyl ester (200 mg, 0.417 mmol) and Et₃N (88 µL, 0.625 mmol) in CH₂Cl₂ (10 mL) and the resulting solution is stirred under N₂ atmosphere at RT for 2 days. CH₂Cl₂ is added followed by an aqueous saturated solution of NaHCO₃. The layers are separated and the aqueous one extracted twice with CH₂Cl₂. The combined organic extracts are dried over Na₂SO₄, filtered and concentrated. The crude mixture is purified by flash chromatography on silica gel (eluent: c-hexane/AcOEt 50/50 to 0/100) to give the title product. t_{R} (HPLC, nucleosil C18 column, 10-100% CH₃CN/H₂O/5 min, 100% CH₃CN/3 min, flow: 1.5 mL/min): 5.45 min.

### B. N-[(3S,4S)-4-(3-Benzyl-sulfonylureido)-pyrrolidin-3-ylmethyl]-N-isopropyl-4-methoxy-3-(3-methoxy-propoxy)-benzamide

To a solution of (3S,4R)-3-(3-benzyl-sulfonylureido)-4-({isopropyl-[4-methoxy-3-(3-methoxy-propoxy)-benzoyl]-amino}-methyl)-pyrrolidine-1-carboxylic acid tert-butyl ester (60 mg, 0.093 mmol) in dioxane (1.5 mL) is added 4N HCl in dioxane (1.5 mL). The mixture is stirred for 2 h at RT and poured into an aqueous saturated solution of NaHCO₃. CH₂Cl₂ is added, the layer are separated and the aqueous one extracted twice with CH₂Cl₂. the combined organic extracts are dried over Na₂SO₄, filtered and concentrated. The crude material is purified by preparative HPLC (C18-ODB-5 µm, 19x50 mm, eluent: CH₃CN /H₂O + 0.1 % HCOOH) to give the title product. To a solution of the free base (31 mg, 0.056 mmol) in dioxane (2 mL), (21 µL, 0.084 mmol) of 4N HCl in dioxane is added, and the resulting solution is lyophilized to afford the corresponding hydrochloride salt as a white powder. MS (LC-MS): 549.3 [M+H]⁺; tR (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.38 min.

### Example 295: [(3S,4S)-4-({Isopropyl-[4-methoxy-3-(3-methoxy-propoxy)-benzoyl]-amino}-methyl)-pyrrolidin-3-yl]-carbamic acid benzyl ester

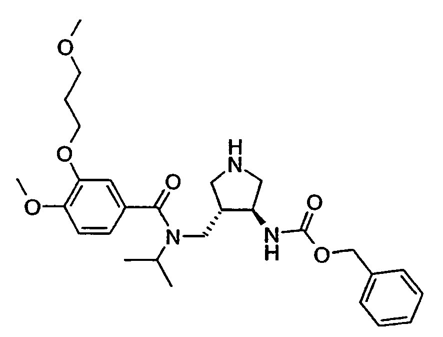

The title compound is prepared analogously as described for the title compound under B in Example 262 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and benzyl chloroformate. MS (LC-MS): 514.1 [M+H]⁺; t_{R} (HPLC, Nucleosil C18 column, 10-100% CH₃CN/H₂O/5 min, 100% CH₃CN/3 min, CH₃CN and H₂O containing 0.1 % TFA, flow: 1.5 mL/min): 5.13 min.

### Example 296: [(3S,4S)-4-({Isopropyl-[4-methoxy-3-(3-methoxy-propoxy)-benzoyl]-amino}-methyl)-pyrrolidin-3-yl]-carbamicacid cyclohexyl ester

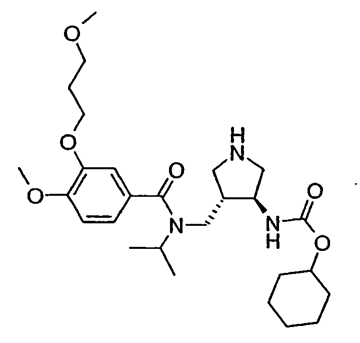

The title compound is prepared analogously as described for the title compound under B in Example 262 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and cyclohexyl chloroformate. MS (LC-MS): 506.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.34 min.

### Cyclohexyl chloroformate

To a solution of cyclohexanol (60 mg, 0.6 mmol) in toluene (5 mL) is added dropwise a commercially available solution of 20 wt% phosgene in toluene (640 µL, 1.2 mmol) at 0°C under an N₂ atmosphere. The mixture is stirred 1 h at 0°C and overnight at RT. The excess of phosgene is removed using a nitrogen stream and the solvent concentrated under vacuum to afford the title product which is directly used in the next step without further purification. ¹H NMR (CD₃OD, 300 MHz): δ=1.35-1.43 (m, 4H), 1.54-1.62 (m, 2H), 1.61-1.78 (m, 2H), 1.83-2.01 (m, 2H), 4.86 (m, 1H).

### Example 297: [(3S,4S)-4-({Isopropyl-[4-methoxy-3-(3-methoxy-propoxy)-benzoyl]-amino}-methyl)-pyrrolidin-3-yl]-carbamic acid phenyl ester

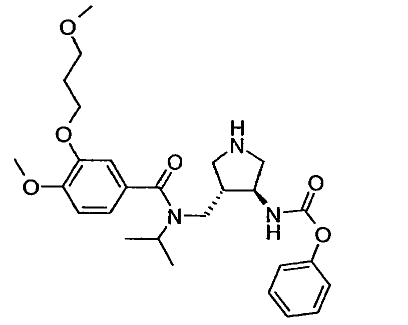

The title compound is prepared analogously as described for the title compound under B in Example 262 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and phenyl chloroformate. MS (LC-MS): 500 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.22 min.

### Example 298: [(3S,4S)-4-({Isopropyl-[4-methoxy-3-(3-methoxy-propoxy)-benzoyl]-amino}-methyl)-pyrrolidin-3-yl]-carbamicacid cyclohexylmethyl ester

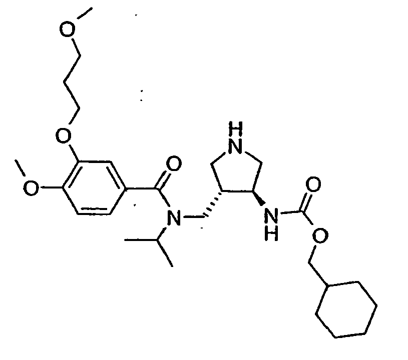

The title compound is prepared analogously as described for the title compound under B in Example 262 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and cyclohexylmethyl chloroformate (prepared analogously as deribed for cyclohexyl cloroformate in example 296). MS (LC-MS): 520.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.55 min.

### Example 299: [(3S,4S)-4-({Isopropyl-[4-methoxy-3-(3-methoxy-propoxy)-benzoyl]-amino}-methyl)-pyrrolidin-3-yl]-carbamic acid (R)-1-phenyl-propyl ester

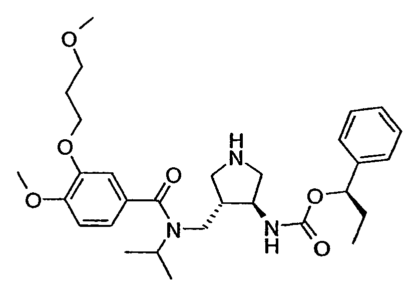

The title compound is prepared analogously as described for the title compound under B in Example 262 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and R(+)-1-phenyl-1-propyl chloroformate (prepared analogously as deribed for cyclohexyl cloroformate in example 296). MS (LC-MS): 542 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.27 min.

### Example 300: [(3S,4S)-4-({Isopropyl-[4-methoxy-3-(3-methoxy-propoxy)-benzoyl]-amino}-methyl)-pyrrolidin-3-yl]-carbamic acid (S)-1-phenyl-propyl ester

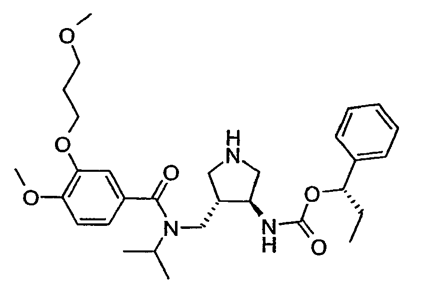

The title compound is prepared analogously as described for the title compound under B in Example 262 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and S(-)-1-phenyl-1-propyl chloroformate (prepared analogously as deribed for cyclohexyl cloroformate in example 296). MS (LC-MS): 542 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min); 4.28 min.

### Example 301: [(35,45)-4-({Isopropyl-[4-methoxy-3-(3-methoxy-propoxy)-benzoyl]-amino}-methyl)-pyrrolidin-3-yl]-methyl-carbamic acid benzyl ester

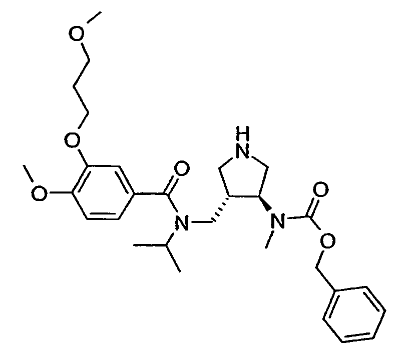

### A. (3S,4R)-3-Benzyloxycarbonylamino-4-({isopropyl-[4-methoxy-3-(3-methoxy-propoxy)-benzoyl]-amino}-methyl)-pyrrolidine-1-carboxylic acid tert-butyl ester

The title compound is prepared analogously as described for the title compound under A in Example 262 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and benzyl chloroformate. TLC, R_{f} (CH₂Cl₂/MeOH 95/5) = 0.3. MS (LC-MS): 514.1 [M+H-Boc]⁺; t_{R} (HPLC, Nucleosil C18 column, 10-100% CH₃CN/H₂O/5 min, 100% CH₃CN/3 min, CH₃CN and H₂O containing 0.1 % TFA, flow: 1.5 mL/min): 6.04 min.

### B. (3S,4R)-3-(Benzyloxycarbonyl-methyl-amino)-4-({isopropyl-[4-methoxy-3-(3-methoxy-propoxy)-benzoyl]-amino}-methyl)-pyrrolidine-1-carboxylic acid tert-butyl ester

To a solution of (3S,4R)-3-benzyloxycarbonylamino-4-({isopropyl-[4-methoxy-3-(3-methoxy-propoxy)-benzoyl]-amino}-methyl)-pyrrolidine-1-carboxylic acid tert-butyl ester (139.5 mg, 0.227 mmol) in THF (2 mL) is added under N₂ atmosphere NaH (60 % dispersion in mineral oil, 23 mg, 0.568 mmol). The resulting mixture is stirred for 15 min before the addition of methyliodide (43 µL, 0.681 mmol) and further stirred at RT for 3 h. CH₂Cl₂ is added and the mixture is quenched by addition of an aqueous saturated solution of NaHCO₃. The layers are separated and the aqueous one extracted twice with CH₂Cl₂. The combined organic extracts are dired over Na₂SO₄, filtered and concentrated. The crude material is purified by flash chromatography on silica gel (eluent: AcOEt) to give the title product. TLC, R_{f} (CH₂Cl₂/MeOH 95/5) = 0.5. MS (LC-MS): 528.3 [MH+H-Boc]⁺; t_{R} (HPLC, nucleosil C18 column, 10-100% CH₃CN/H₂O/5 min, 100% CH₃CN/3 min, flow: 1.5 mL/min): 6.53 min.

### C. [(3S,4S)-4-({Isopropyl-[4-methoxy-3-(3-methoxy-propoxy)-benzoyl]-amino}-methyl)-pyrrolidin-3-yl]-methyl-carbamic acid benzyl ester

To a solution of (3S,4R)-3-(benzyloxycarbonyl-methyl-amino)-4-({isopropyl-[4-methoxy-3-(3-methoxy-propoxy)-benzoyl]-amino}-methyl)-pyrrolidine-1-carboxylic acid tert-butyl ester (117 mg, 0.186 mmol) in dioxane (1 mL) is added 4N HCl in dioxane (1 mL). The mixture is stirred for 2h at RT and the resulting solution is lyophilized to afford the corresponding hydrochloride salt as a white powder. MS (LC-MS): 528.4 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.36 min.

### Example 302: N-[(3S,4S)-4-(3-Benzyl-3-ethyl-ureido)-pyrrolidin-3-ylmethyl]-N-isopropyl-4-methoxy-3-(3-methoxy-propoxy)-benzamide

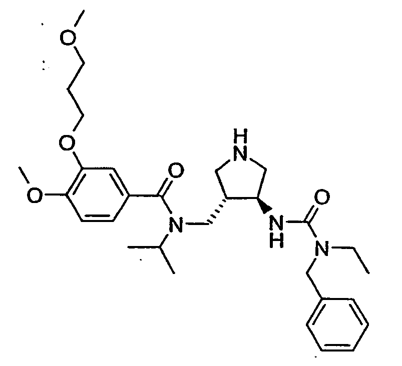

### A. (3S,4R)-3-(3-Benzyl-3-ethyl-ureido)-4-({isopropyl-[4-methoxy-3-(3-methoxy-propoxy)-benzoyl]-amino}-methyl)-pyrrolidine-1-carboxylic acid tert-butyl ester

To a solution of (3S,4R)-3-amino-4-({isopropyl-[4-methoxy-3-(3-methoxy-propoxy)-benzoyl]-amino}-methyl)-pyrrolidine-1-carboxylic acid tert-butyl ester (160 mg, 0.324 mmol) in CH₂Cl₂ (5 mL) are added benzyl-ethyl-carbamoyl chloride (88 mg, 0.421 mmol) in CH₂Cl₂ (5 mL) and triethylamine (59 µL, 0.421 mmol). And the mixture is stirred 2 days at RT under N₂ atmosphere. The reaction mixture is then diluted with CH₂Cl₂ and poured into an aqueous saturated solution of NaHCO₃. The layers are separated and the aqueous one extracted twice with CH₂Cl₂. The combined organic extracts are dried over Na₂SO₄, filtered and concentrated to the title product as an orange oil. TLC, R_{f} (CH₂Cl₂/ MeOH 95/5) = 0.27. MS (LC-MS): 667.0 [M+H]⁺; t_{R} (HPLC, nucleosil C18 column, 10-100% CH₃CN/H₂O/5 min, 100% CH₃CN/3 min, flow: 1.5 mL/min): 6.28 min.

### B. N-[(3S,4S)-4-(3-Benzyl-3-ethyl-ureido)-pyrrolidin-3-ylmethyl]-N-isopropyl-4-methoxy-3-(3-methoxy-propoxy)-benzamide

To a solution of (3S,4R)-3-(3-benzyl-3-ethyl-ureido)-4-({isopropyl-[4-methoxy-3-(3-methoxy-propoxy)-benzoyl]-amino}-methyl)-pyrrolidine-1-carboxylic acid tert-butyl ester (114 mg, 0.178 mmol) in dioxane (2 mL) is added 4N HCl in dioxane (1 mL). The mixture is stirred for 2 h at RT and lyophilized to afford the corresponding hydrochloride salt as a white powder. MS (LC-MS): 546.6 [M+H]+; t_{R} (HPLC, nucleosil C18-HD column, 20-100% CH₃CN/H₂O/6 min, 100% CH₃CN/1.5 min, flow: 1 mL/min): 5.41 min.

### Benzyl-ethyl-carbamoyl chloride

To a solution of triphosgene (79 mg, 0.264 mmol) in CH2Cl2 (2 mL) at - 78°C are added dropwise under N2 atmosphere pyridine (64 µL, 0.8 mmol) followed by a solution of N-ethylbenzylamine (119 µL, 0.8 mmol) in CH₂Cl₂ (2 mL). The mixture is slowly allowed to reach RT overnight. An aqueous saturated solution of NaCl is added , the layers are separated and the aqueous one extracted twice with CH₂Cl₂. The combined organic extracts are neutralized with an aqueous saturated solution of NaHCO₃, dried over Na₂SO₄, filtered and concentrated to afford the title compound. TLC, R_{f} (CH₂Cl₂/MeOH 95/5) = 0.9; t_{R}:(HPLC, nucleosil C18-HD column, 20-100% CH₃CN/H₂O/6 min, 100% CH₃CN/1.5 min, flow: 1 mL/min): 4.59 min

### Example 303: N-Isopropyl-4-methoxy-3-(3-methoxy-propoxy)-N-((3S,4S)-4-phenyl acetylamino-pyrrolidin-3-ylmethyl)-benzamide

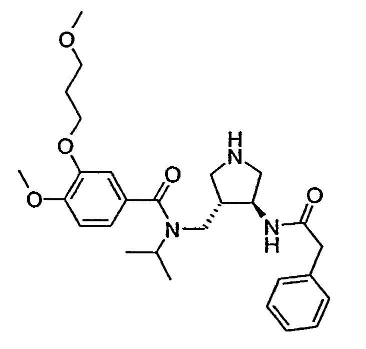

The title compound is prepared analogously as described for the title compound under B in Example 259 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and phenylacetyl chloride. MS (LC-MS): 498 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mUmin): 4.66 min.

### Example 304: N-[(3S,4S)-4-(2-Cyclohexyl-acetylamino)-pyrrolidin-3-ylmethyl]-N-isopropyl-4-methoxy-3-(3-methoxy-propoxy)-benzamide

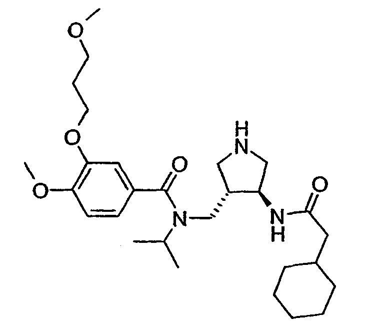

### A. (3S,4R)-3-(2-Cyclohexyl-acetylamino)-4-({isopropyl-[4-methoxy-3-(3-methoxy-propoxy)-benzoyl]-amino}-methyl)-pyrrolidine-1-carboxylic acid tert-butyl ester

Cyclohexylacetic acid (60 mg, 0.417 mmol), HOBT (68 mg, 0.5 mmol) and EDCl (97 mg, 0.5 mmol) are suspended in dry CH₂Cl₂ (5 mL) and stirred 15 min under nitrogen. (3S,4R)-3-amino-4-({isopropyl-[4-methoxy-3-(3-methoxy-propoxy)-benzoyl]-amino}-methyl)-pyrrolidine-1-carboxylic acid tert-butyl ester (200 mg, 0.417 mmol) is added and the mixture further stirred overnight at RT. HCl 1 N is added, the layers are separated and the aqueous one extracted twice with CH₂Cl₂. The combined organic extracts are neutralized with a 5% solution of NaHCO₃, washed with brine, dried over Na₂SO₄, filtered and concentrated. The crude material is purified by flash chromatography (eluent: CH₂Cl₂/MeOH 100/0 to 98/2) to give the title compound. TLC, R_{f} (CH₂Cl₂/MeOH 95/5) = 0.3. t_{R} (HPLC, nucleosil C18 column, 10-100% CH₃CN/H₂O/5 min, 100% CH₃CN/3 min, flow: 1.5 mL/min): 6.35 min.

### B. N-[(3S,4S)-4-(2-Cyclohexyl-acetylamino)-pyrrolidin-3-ylmethyl]-N-isopropyl-4-methoxy-3-(3-methoxy-propoxy)-benzamide

To a solution of (3S,4R)-3-(2-cyclohexyl-acetylamino)-4-({isopropyl-[4-methoxy-3-(3-methoxy-propoxy)-benzoyl]-amino}-methyl)-pyrrolidine-1-carboxylic acid tert-butyl ester (269 mg, 0.417 mmol) in dioxane (2 mL) is added 4N HCl in dioxane (2 mL). The mixture is stirred for 2h at RT and the resulting solution is lyophilized to afford the corresponding hydrochloride salt as a white powder. MS (LC-MS): 504.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.22 min.

### Example 305: N-[(3S,4S)-4-(Cyclohexanecarbonyl-amino)-pyrrolidin-3-ylmethyl]-N-isopropyl-4-methoxy-3-(3-methoxy-propoxy)-benzamide

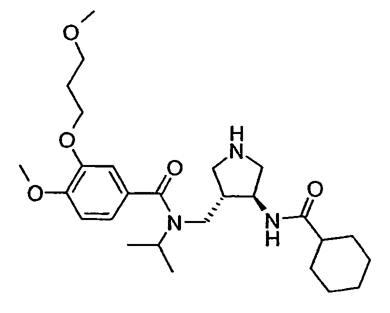

The title compound is prepared analogously as described for the title compound under B in Example 259 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and cyclohexancar- -bonyl chloride. MS (LC-MS): 490.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.14 min.

### Example 306: N-Isopropyl-4-methoxy-3-(3-methoxy-propoxy)-N-[(3S,4S)-4-(2-tetrahydro-pyran-4-yl-acetylamino)-pyrrolidin-3-ylmethyl]-benzamide

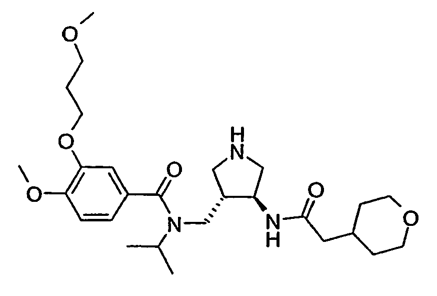

The title compound is prepared analogously as described for the title compound under B in Example 304 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and tetra-hydropyranyl-4-acetic acid. MS (LC-MS): 506 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.76 min.

### Example 307: N-[(3S,4S)-4-(2-Hydroxy-2-phenyl-acetylamino)-pyrrolidin-3-ylmethyl]-N-isopropyl-4-methoxy-3-(3-methoxy-propoxy)-benzamide

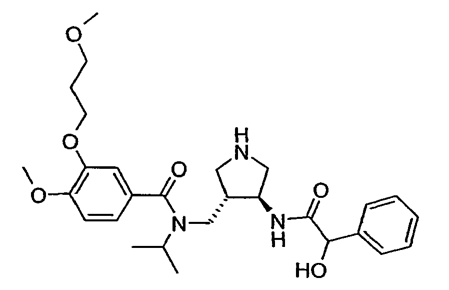

### A. (35,4R)-3-(2-Acetoxy-2-phenyl-acetylamino)-4-({isopropyl-[4-methoxy-3-(3-methoxy-propoxy)-benzoyl]-amino}-methyl)-pyrrolidine-1-carboxylic acid tert-butyl ester

The title compound is prepared analogously as described for the title compound under A in Example 259 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and O-acetylmandelic chloride. TLC, R_{f} (CH₂Cl₂/MeOH 95/5) = 0.3. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 6.10 min.

### B. (35,4R)-3-(2-Hydroxy-2-phenyl-acetylamino)-4-({isopropyl-[4-methoxy-3-(3-methoxy-propoxy)-benzoyl]-amino}-methyl)-pyrrolidine-1-carboxylic acid tert-butyl ester

A solution of (3S,4R)-3-(2-acetoxy-2-phenyl-acetylamino)-4-({isopropyl-[4-methoxy-3-(3-methoxy-propoxy)-benzoyl]-amino}-methyl)-pyrrolidine-1-carboxylic acid tert-butyl ester (309 mg, 0.417 mmol) in MeOH (3 mL) is cooled to 0°C and LiOH,H₂O (105 mg, 2.5 mmol) is added. The reaction mixture is allowed to reach RT and stirred overnight. After completion, the reaction mixture is acidified with HCl 1N, CH₂Cl₂ is added and the layers are separated, the aqueous one extracted twice with CH₂Cl₂. The combined organic extracts are dried over Na₂SO₄, filtered and concentrated. The crude material is purified by HPLC preparative (C18-ODB, 5 µm, 19x50 mm, Waters, eluent: CH₃CN /H₂O+ 0.1 % HCOOH flow: 20 mL/min). The HPLC fractions are collected, diluted with AcOEt and washed with a saturated aqueous solution of NaHCO₃. The organic layer is dried over Na₂SO₄, filtered and concentrated to give the title product. TLC, R_{f} (CH₂Cl₂/MeOH 95/5) = 0.3. MS (LC-MS): 514.1 [M+H-Boc]⁺; t_{R} (HPLC, nucleosil C18 column, 10-100% CH₃CN/H₂O/5 min, 100% CH₃CN/3 min, flow: 1.5 mL/min): 5.90 min.

### C. N-[(3S,4S)-4-(2-Hydroxy-2-phenyl-acetylamino)-pyrrolidin-3-yimethyl]-N-isopropyl-4-methoxy-3-(3-methoxy-propoxy)-benzamide

To a solution of (3S,4R)-3-(2-hydroxy-2-phenyl-acetylamino)-4-({isopropyl-[4-methoxy-3-(3-methoxy-propoxy)-benzoyl]-amino}-methyl)-pyrrolidine-1-carboxylic acid tert-butyl ester (255 mg, 0.417 mmol) in dioxane (2 mL) is added 4N HCl in dioxane (2 mL). The mixture is stirred for 2 h at RT and the resulting solution is lyophilized to afford the corresponding hydrochloride salt as a white powder. MS (LC-MS): 514.1 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.10 min.

### Example 308: Tetrahydro-pyran-4-carboxylic acid [(3S,4S)-4-({isopropyl-[4-methoxy-3-(3-methoxy-propoxy)-benzoyl]-amino}-methyl)-pyrrolidin-3-yl]-amide

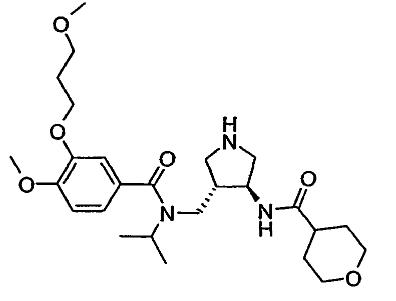

The title compound is prepared analogously as described for the title compound under B in Example 304 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and tetrahydro-pyran-4-carboxylic acid. MS (LC-MS): 492 [M+H]⁺; t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 4.73 min.

### Example 309: N-[(3S,4S)-4-(2-Benzylamino-acetylamino)-pyrrolidin-3-ylmethyl]-N-isopropyl-4-methoxy-3-(3-methoxy-propoxy)-benzamide

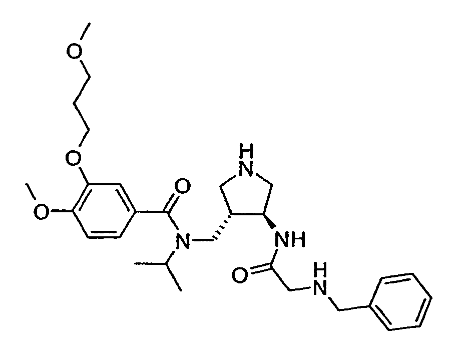

### A. (3S,4R)-3-(2-Chloro-acetylamino)-4-({isopropyl-[4-methoxy-3-(3-methoxy-propoxy)-benzoyl]-amino}-methyl)-pyrrolidine-1-carboxylic acid tert-butyl ester

To a solution of (3S,4R)-3-amino-4-({isopropyl-[4-methoxy-3-(3-methoxy-propoxy)-benzoyl]-amino}-methyl)-pyrrolidine-1-carboxylic acid tert-butyl ester (250 mg, 0.521 mmol) in CH₂Cl₂ (5 mL) are added triethylamine (73 µL, 0.521 mmol) and chloroacetyl chloride (41.5 µL, 0.521 mmol) at 0°C under a N₂ atmosphere and the mixture is stirred for 1 h at RT. After completion of the reaction CH₂Cl₂ is added followed by an aqueous saturated solution of NaHCO₃. The layers are separated and the aqueous one extracted twice with CH₂Cl₂. The combined organic extracts are dried over Na₂SO₄, filtered and concentrated to give the title product, which is used without further purification in the next step. TLC, R_{f} (CH₂Cl₂/MeOH 95/5) = 0.3.

### B. (3S,4R)-3-(2-Benzylamino-acetylamino)-4-({isopropyl-[4-methoxy-3-(3-methoxy-propoxy)-benzoyl]-amino}-methyl)-pyrrolidine-1-carboxylic acid tert-butyl ester

To a solution of (3S,4R)-3-(2-chloro-acetylamino)-4-({isopropyl-[4-methoxy-3-(3-methoxy-propoxy)-benzoyl]-amino}-methyl)-pyrrolidine-1-carboxylic acid tert-butyl ester (287 mg, 0.516 mmol), Nal (77 mg, 0.516 mmol) and DIEA (88.3 µL, 0.516 mmol) in DMF (5 mL) is added benzylamine (56.3 µL, 0.516 mmol) and the reaction mixture is stirred overnight at RT under a N₂ atmosphere. The reaction mixture is concentrated to dryness, AcOEt is added followed by a saturated aqueous solution of NaHCO₃. The layer are separated and the aqueous one extracted twice with AcOEt. The combined organic extracts are dried over Na₂SO₄, filtered and concentrated. The crude material is purified by preparative HPLC (C18-ODB, 5 µm, 19x50 mm, Waters, eluent: CH₃CN /H₂O + 0.1 % HCOOH flow: 20 mL/min). The HPLC fractions are collected, concentrated, diluted with AcOEt and washed with a saturated aqueous solution of NaHCO₃. The organic layer is dried over Na₂SO₄, filtered and concentrated to give the the title product. TLC, R_{f} (CH₂Cl₂/MeOH 95/5) = 0.2. MS (LC-MS): 627.2 [M-H]⁺; t_{R} (HPLC, nucleosil C18 column, 10-100% CH₃CN/H₂O/5 min, 100% CH₃CN/3 min, flow: 1.5 mL/min): 4.95 min.

### C. N-[(3S,4S)-4-(2-Benzylamino-acetylamino)-pyrrolidin-3-ylmethyl]-N-isopropyl-4-methoxy-3-(3-methoxy-propoxy)-benzamide

To a solution of (3S,4R)-3-(2-benzylamino-acetylamino)-4-({isopropyl-[4-methoxy-3-(3-methoxy-propoxy)-benzoyl]-amino}-methyl)-pyrrolidine-1-carboxylic acid tert-butyl ester (111 mg, 0.177 mmol) in dioxane (2 mL) is added 4N HCl in dioxane (1 mL). The mixture is stirred for 2 h at RT and the resulting solution is lyophilized to afford the corresponding bishydrochloride salt as a white powder. MS (LC-MS): 527 [M-H]⁺; t_{R} (HPLC, nucleosil C18 column, 10-100% CH₃CN/H₂O/5 min, 100% CH₃CN/3 min, flow: 1.5 mL/min): 4.10 min.

### Example 310: N-{(3S,4S)-4-[(Benzylcarbamoyl-methyl)-aminol-pyrrolidin-3-ylmethyl}-N-isopropyl-4-methoxy-3-(3-methoxy-propoxy)-benzamide

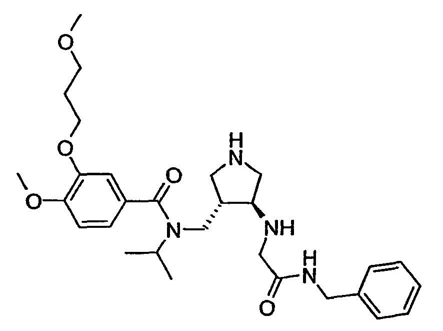

### A. (3S,4R)-3-[(Benzylcarbamoyl-methyl)-amino]-4-({isopropyl-[4-methoxy-3-(3-methoxy-propoxy)-benzoyl]-amino}-methyl)-pyrrolidine-1-carboxylic acid tert-butyl ester

To a solution of (3S,4R)-3-amino-4-({isopropyl-[4-methoxy-3-(3-methoxy-propoxy)-benzoyl]-amino}-methyl)-pyrrolidine-1-carboxylic acid tert-butyl ester (400 mg, 0.834 mmol) and DIEA (285 µL, 1.67 mmol) in DMF (4 mL) is added a solution of N-benzyl-2-bromo-acetamide (described in example 268) (190 mg, 0.834 mmol) in DMF (8 mL) at 0°C under N₂ atmosphere. The reaction mixture is stirred for 5 days at RT, and poured into a saturated aqueous solution of NaHCO₃. AcOEt is added, the layers are separated and the aqueous one extracted twice with AcOEt. The combined organic extracts are dried over Na₂SO₄, filtered and concentrated. The crude mixture is purified by flash chromatography on silica gel (eluent: CH₂Cl₂/MeOH 100/0 to 95/5) to give the title product. TLC, R_{f} (CH₂C₁₂/MeOH 80/20) = 0.75. MS (LC-MS): 627.0 [M-H]⁺; t_{R} (HPLC, nucleosil C18 column, 10-100% CH₃CN/H₂O/5 min, 100% CH₃CN/3 min, flow: 1.5 mL/min): 5.28 min.

### B. N-{(3S,4S)-4-[(Benrylcarbamoyl-methyl)-amino]-pyrrolidin-3-ylmethyl}-N-isopropyl-4-methoxy-3-(3-methoxy-propoxy)-benzamide

To a solution of (3S,4R)-3-[(benzylcarbamoyl-methyl)-amino]-4-({isopropyl-[4-methoxy-3-(3-methoxy-propoxy)-benzoyl]-amino}-methyl)-pyrrolidine-1-carboxylic acid tert-butyl ester (351 mg, 0.56 mmol) in dioxane (4 mL) is added 4N HCl in dioxane (3 mL). The mixture is stirred for 2 h at RT and the resulting solution is lyophilized to afford the corresponding bishydrochloride salt as a white powder. MS (LC-MS): 527.1 [M-H]⁺; t_{R} (HPLC, nucleosil C18 column, 10-100% CH₃CN/H₂O/5 min, 100% CH₃CN/3 min, flow: 1.5 mL/min): 4.50 min.

### Example 311: N-((3S,4S)-4-{[(Cyclohexylmethyl-carbamoyl)-methyl]-amino}-pyrrolidin-3-ylmethyl)-N-isopropyl-4-methoxy-3-(3-methoxy-propoxy)-benzamide

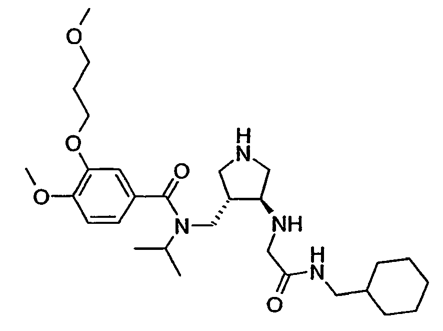

The title compound is prepared analogously as described for the title compound under B in Example 310 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 2-bromo-N-cyclohexylmethyl-acetamide. MS (LC-MS): 533.1 [M-H]+; t_{R} (HPLC, nucleosil C18 column, 10-100% CH₃CN/H₂O/5 min, 100% CH₃CN/3 min, flow: 1.5 mL/min): 4.25 min.

### 2-Bromo-N-cyclohexylmethyl-acetamide

The title compound is prepared analogously as described for the title compound N-benzyl-2-bromo-acetamide in Example 268 from 2-bromo acetic acid and cyclohexylamine. TLC, R_{f} (c-hexane/AcOEt 70/30) = 0.2. MS (LC-MS): 234-236 [M-H]⁺; t_{R} (HPLC, nucleosil C18 column, 10-100% CH₃CN/H₂O/5 min, 100% CH₃CN/3 min, flow: 1.5 mL/min): 4.99 min.

### Example 312: N-[(3S,4S)-4-(1-Benzylcarbamoyl-ethylamino)-pyrrolidin-3-ylmethyl]-N-isopropyl-4-methoxy-3-(3-methoxy-propoxy)-benzamide

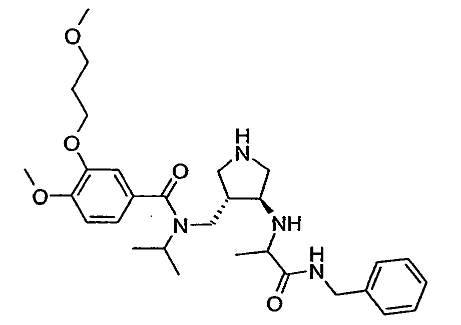

### A. (3S,4R)-3-(1-Benzylcarbamoyl-ethylamino)-4-({isopropyl-[4-methoxy-3-(3-methoxy-propoxy)-benzoyl]-amino}-methyl)-pyrrolidine-1-carboxylic acid tert-butyl ester

To a solution (3S,4R)-3-amino-4-({isopropyl-[4-methoxy-3-(3-methoxy-propoxy)-benzoyl]-amino}-methyl)-pyrrolidine-1-carboxylic acid tert-butyl ester (250 mg, 0.521 mmol) and DIEA (178 µL, 1.04 mmol) in CH₂Cl₂ (8 mL) is added N-benzyl-2-bromo-propionamide (126 mg, 0.521 mmol) and TBAI (192 mg, 0.521 mmol). The reaction mixture is stirred overnight at RT, and poured into a saturated aqueous solution of NaHCO₃. CH₂Cl₂ is added, the layers are separated and the aqueous one extracted twice with CH₂Cl₂. The combined organic extracts are dried over Na₂SO₄, filtered and concentrated. The crude mixture is purified by preparative HPLC (C18-ODB, 5 µm, 19x50 mm, Waters, eluent: CH₃CN /H₂O + 0.1 % HCOOH flow: 20 mL/min). The HPLC fractions are collected, concentrated, diluted with AcOEt and washed with a saturated aqueous solution of NaHCO₃. The organic layer is dried over Na₂SO₄, filtered and concentrated to give the title product. TLC, R_{f} (CH₂Cl₂/MeOH 95/5) = 0.2. MS (LC-MS): 641.1 [M-H]⁺; t_{R} (HPLC, nucleosil C18 column, 10-100% CH₃CN/H₂O/5 min, 100% CH₃CN/3 min, flow: 1.5 mL/min): 5.19 min.

### B. N-[(3S,4S)-4-(1-Benzylcarbamoyl-ethylamino)-pyrrolidin-3-ylmethyl]-N-isopropyl-4-methoxy-3-(3-methoxy-propoxy)-benzamide

To a solution of (3S,4R)-3-(1-benzylcarbamoyi-ethylamino)-4-({isopropyl-[4-methoxy-3-(3-methoxy-propoxy)-benzoyl]-amino}-methyl)-pyrrolidine-1-carboxylic acid tert-butyl ester (125 mg, 0.195 mmol) in dioxane (1 mL) is added 4N HCl in dioxane (1 mL). The mixture is stirred for 2 h at RT and the resulting solution is lyophilized to afford the corresponding bishydrochloride salt as a white powder. MS (LC-MS): 541.1 [M-H]⁺; t_{R} (HPLC, nucleosil C18 column, 10-100% CH₃CN/H₂O/5 min, 100% CH₃CN/3 min, flow: 1.5 mL/min): 4.02 min.

### N-Benzyl-2-bromo-propionamide

The title compound is prepared analogously as described for the title compound N-benzyl-2-bromo-acetamide in Example 268 from 2-bromo propionic acid and benzylamine. TLC, R_{f} (c-hexane/AcOEt 2/1 ) = 0.4. t_{R} (HPLC, nucleosil C18 column, 10-100% CH₃CN/H₂O/5 min, 100% CH₃CN/3 min, flow: 1.5 mL/min): 5.59 min.

### Example 313: N-[(3S,4S)-4-(1-Benzylcarbamoyl-propylamino)-pyrrolidin-3-ylmethyl]-N-isopropyl-4-methoxy-3-(3-methoxy-propoxy)-benzamide

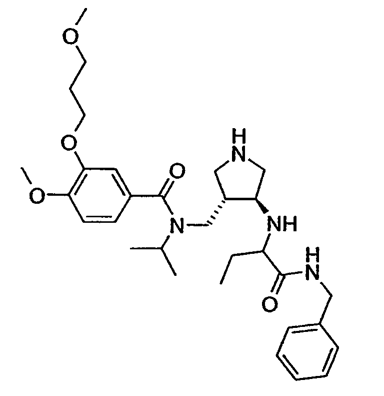

The title compound is prepared analogously as described for the title compound under B in Example 312 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and N-benzyl-2-bromo-butyramide. MS (LC-MS): 555 [M-H]⁺; t_{R} (HPLC, nucleosil C18 column, 10-100% CH₃CN/H₂O/5 min, 100% CH₃CN/3 min, flow: 1.5 mL/min): 4.31 min.

### N-Benzyl-2-bromo-butyramide

The title compound is prepared analogously as described for the title compound N-benzyl-2-bromo-acetamide in Example 268 from 2-bromobutyric acid and benzylamine. TLC, R_{f} (c-hexane/AcOEt 2/1) = 0.6. t_{R} (HPLC, nucleosil C18 column, 10-100% CH₃CN/H₂O/5 min, 100% CH₃CN/3 min, flow: 1.5 mL/min): 4.96 min.

### Example 314: N-((3S,4S)-4-{[(Benzyl-methyl-carbamoyl)-methyl]-amino}-pyrrolidin-3-ylmethyl)-N-isopropyl-4-methoxy-3-(3-methoxy-propoxy)benzamide

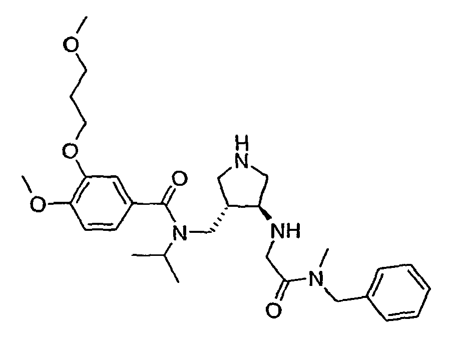

The title compound is prepared analogously as described for the title compound under B in Example 312 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and N-benzyl-2-chloro-N-methyl-acetamide. MS (LC-MS): 541 [M-H]⁺; t_{R} (HPLC, nucleosil C18 column, 10-100% CH₃CN/H₂O/5 min, 100% CH₃CN/3 min, flow: 1.5 mL/min): 4.07 min.

### N-Benzyl-2-chloro-N-methyl-acetamide

To a solution of N-benzylmethylamine (925 µL, 7.19 mmol) in CH₂Cl₂ (20 mL) is added chloroacethyl chloride (572 µL, 7.19 mmol) and triethylamine (1 mL, 7.19 mmol). The mixture is stirred overnight at RT under a N₂ atmosphere. Then diluted with CH₂Cl₂ and poured into an aqueous saturated solution of NaHCO₃. The layer are separated and the aqueous one extracted twice with CH₂Cl₂. The combined organic extracts are dried over Na₂SO₄, filtered and concentrated to give the title product which was used in the next step without further purification. TLC, R_{f} (c-hexane/AcOEt 2/1) = 0.3. MS (LC-MS): 198.1 [M-H]⁺; t_{R} (HPLC, nucleosil C18 column, 10-100% CH₃CN/H₂O/6 min, 100% CH₃CN/1.5 min, flow): 3.33 min.

### Example 315: N-((3S,4S)-4-{[(Benzyl-ethyl-carbamoyl)-methyl]amino}-pyrrolidin-3-ylmethyl)-N-isopropyl-4-methoxy-3-(3-methoxy-progoxy)-benzamide

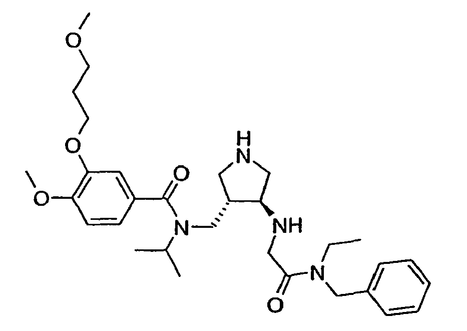

The title compound is prepared analogously as described for the title compound under B in Example 312 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and N-benzyl-2-chloro-N-ethyl-acetamide. MS (LC-MS): 555.2 [M-H]⁺; t_{R} (HPLC, nucleosil C18 column, 10-100% CH₃CN/H₂O/5 min, 100% CH₃CN/3 min, flow: 1.5 mL/min): 4.30 min.

### N-benzyl-2-chloro-N-ethyl-acetamide

The title compound is prepared analogously as described for the title compound N-benzyl-2-chloro-N-methyl-acetamide in Example 314 from chloroacetyl chloride and N-benzylethylamine. TLC, R_{f} (c-hexane/AcOEt 2/1) = 0.4. t_{R} (HPLC, nucleosil C18 column, 10-100% CH₃CN/H₂O/6 min, 100% CH₃CN/1.5 min): 3.77 min.

### Example 316: N-Isopropyl-4-methoxy-3-(3-methoxy-propoxy)-N-((3S,4S)-4-{[(tetrahydro-pyran-4-ylcarbamoyl)-methyl]-amino}-pyrrolidin-3-ylmethyl)-benzamide

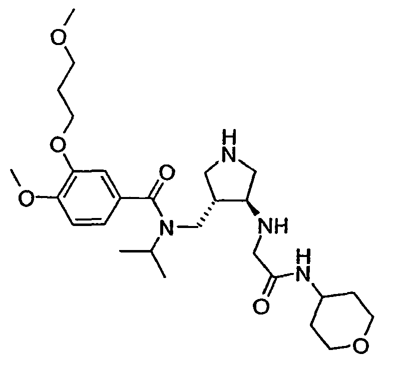

The title compound is prepared analogously as described for the title compound under B in Example 312 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 2-bromo-N-(tetrahydro-pyran-4-yl)-acetamide. MS (LC-MS): 521.3 [M-H]⁺; t_{R} (HPLC, nucleosil C18 column, 10-100% CH₃CN/H₂O/5 min, 100% CH₃CN/3 min, flow: 1.5 mL/min): 3.80 min.

### 2-Bromo-N-(tetrahydro-pyran-4-yl)-acetamide

The title compound is prepared analogously as described for the title compound N-benzyl-2-bromo-acetamide in Example 268 from 2-bromo acetic acid and 4-aminotetrahydropyran. TLC, R_{f} (CH₂Cl₂/MeOH 95/5 ) = 0.15. t_{R} (HPLC, nucleosil C18 column, 10-100% CH₃CN/H₂O/5 min, 100% CH₃CN/3 min, flow: 1.5 mL/min): 3.77 min.

### Example 317 : N-Isopropyl-4-methoxy-3-(3-methoxy-propoxy)-N-[(3S,4S)-4-({[(tetrahydro-pyran-4-ylmethyl)-carbamoyl]-methyl}-amino)-pyrrolidin-3-ylmethyl]-benzamide

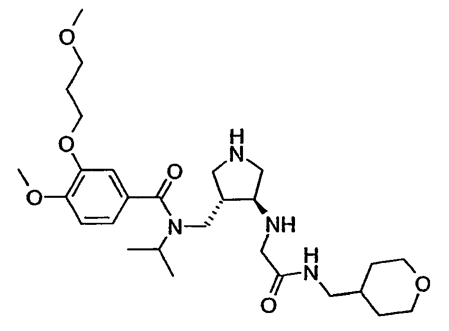

The title compound is prepared analogously as described for the title compound under B in Example 312 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 2-bromo-N-(tetrahydro-pyran-4-ylmethyl)-acetamide. MS (LC-MS): 535 [M-H]⁺; t_{R} (HPLC, nucleosil C18 column, 10-100% CH₃CN/H₂O/5 min, 100% CH₃CN/3 min, flow: 1.5 mL/min): 3.99 min.

### 2-Bromo-N-(tetrahydro-pyran-4-ylmethyl)-acetamide

The title compound is prepared analogously as described for the title compound N-benzyl-2-bromo-acetamide in Example 268 from 2-bromo acetic acid and 4-aminomethyltetrahydropyran. TLC, R_{f} (CH₂Cl₂/MeOH) = 0.15. t_{R} (HPLC, nucleosil C18 column, 10-100% CH₃CN/H₂O/5 min, 100% CH₃CN/3 min, flow: 1.5 mL/min): 3.90 min.

### Example 318: N-[(3S,4S)-4-(Cyclohexylcarbamoylmethyl-amino)-pyrrolidin-3-ylmethyl]-N-isopropyl-4-methoxy-3-(3-methoxy-propoxy)-benzamide

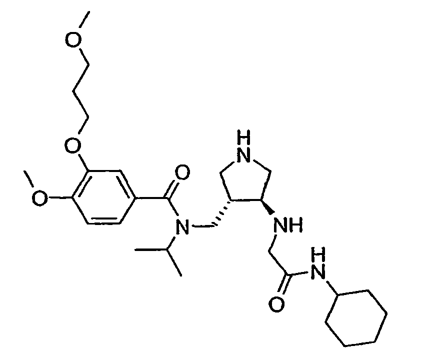

### A. (3S,4R)-3-(Cyclohexylcarbamoylmethyl-amino)-4-({isopropyl-[4-methoxy-3-(3-methoxy-propoxy)-benzoyl]-amino}-methyl)-pyrrolidine-1-carboxylic acid tert-butyl ester

To a solution of (3S,4R)-3-amino-4-({isopropyl-[4-methoxy-3-(3-methoxy-propoxy)-benzoyl]-amino}-methyl)-pyrrolidine-1-carboxylic acid tert-butyl ester (250 mg, 0.521 mmol) and DIEA (178 µL, 1.04 mmol) in DMF (3 mL) is added a solution of 2-bromo-N-cyclohexyl-acetamide (115 mg, 0.0521 mmol) and the mixture is stirred overnight at RT. 2-Bromo-N-cyclohexyl-acetamide (115 mg, 0.521 mmol) and Nal (78 mg, 0.521 mmol) are added and the mixture further stirred for 2 days to complete the reaction. A saturated aqueous solution of NaHCO₃ is added and the mixture extracted with AcOEt (3 times). The combined organic layers are dried over Na₂SO₄, filtered and concentrated and the crude mixture is purified by preparative HPLC (C18-ODB, 5 µm, 19x50 mm, Waters, eluent: CH₃CN /H₂O + 0.1 % HCOOH flow: 20 mL/min). The HPLC fractions are collected, concentrated, diluted with AcOEt and washed with a saturated aqueous solution of NaHCO₃. The organic layer is dried over Na₂SO₄, filtered and concentrated to give the title compound : MS (LC-MS): 619.2 [M-H]⁺; t_{R} (HPLC, nucleosil C18 column, 10-100% CH₃CN/H₂O/5 min, 100% CH₃CN/3 min, flow: 1.5 mL/min): 5.56 min. The bis alkylated product is also found in a second fraction. MS (LC-MS): 758.3 [M-H]⁺; t_{R} (HPLC, nucleosil C18 column, 10-100% CH₃CN/H₂O/5 min, 100% CH₃CN/3 min, flow: 1.5 mL/min): 6.70 min.

### B. N-[(3S,4S)-4-(Cyclohexylcarbamoylmethyl-amino)-pyrrolidin-3-ylmethyl]-N-isopropyl-4-methoxy-3-(3-methoxy-propoxy)-benzamide

To a solution of (3S,4R)-3-(cyclohexylcarbamoylmethyl-amino)-4-({isopropyl-[4-methoxy-3-(3-methoxy-propoxy)-benzoyl]-amino}-methyl)-pyrrolidine-1-carboxylic acid tert-butyl ester (30 mg, 0.05 mmol) in dioxane (1 mL) is added 4N HCl in dioxane (0.5 mL). The mixture is stirred for 2 h at RT and the resulting solution is lyophilized to afford the corresponding bishydrochloride salt as a white powder. MS (LC-MS): 519.4 [M-H]⁺; t_{R} (HPLC, nucleosil C18 column, 10-100% CH₃CN/H₂O/5 min, 100% CH₃CN/3 min, flow: 1.5 mL/min): 5.22 min.

### 2-Bromo-N-cyclohexyl-acetamide

The title compound is prepared analogously as described for the title compound N-benzyl-2-bromo-acetamide in Example 268 from 2-bromo acetic acid and cyclohexylamine. TLC, R_{f} (c-hexane/AcOEt 1/1 ) = 0.45. t_{R} (HPLC, nucleosil C18 column, 10-100% CH₃CN/H₂O/5 min, 100% CH₃CN/3 min, flow: 1.5 mL/min): 5.71 min.

### Example 319: N-Isopropyl-4-methoxy-3-(3-methoxy-propoxy)-N-[(3S,4S)-4-(2-oxo-2-piperidin-1-yl-ethylamino)-pyrrolidin-3-ylmethyl]-benzamide

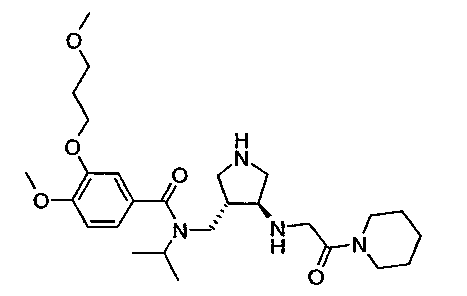

The title compound is prepared analogously as described for the title compound under B in Example 318 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 2-chloro-1-piperidin-1-yl-ethanone except that only one equivalent of the chloride derivative is used. MS (LC-MS): 505.4 [M-H]⁺; t_{R} (HPLC, nucleosil C18 column, 10-100% CH₃CN/H₂O/5 min, 100% CH₃CN/3 min, flow: 1.5 mL/min): 5.45 min.

### 2-Chloro-1-piperidin-1-yl-ethanone

The title compound is prepared analogously as described for the title compound N-benzyl-2-chloro-N-methyl-acetamide in Example 314 from chloroacetyl chloride and piperidine. TLC, R_{f} (CH₂Cl₂/MeOH 95/5) = 0.7. t_{R} (HPLC, nucleosil C18 column, 10-100% CH₃CN/H₂O/5 min, 100% CH₃CN/1.5 min): 3.85 min.

### Example 320: N-((3S,4S)-4-{[(Benzyl-cyclopropylmethyl-carbamoyl)-methyl]-amino}-pyrrolidin-3-ylmethyl)-N-isopropyl-4-methoxy-3-(3-methoxy-propoxy)-benzamide

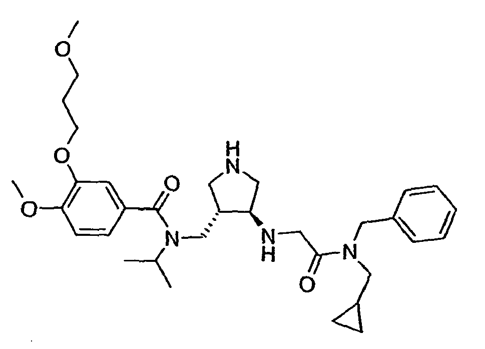

The title compound is prepared analogously as described for the title compound under B in Example 318 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and N-benzyl-2-chloro-N-cyclopropylmethyl-acetamide except that only one equivalent of the chloride derivative is used. MS (LC-MS): 581.5 [M-H]⁺; t_{R} (HPLC, nucleosil C18 column, 10-100% CH₃CN/H₂O/5 min, 100% CH₃CN/3 min, flow: 1.5 mL/min): 5.74 min.

### N-Benzyl-2-chloro-N-cyclopropylmethyl-acetamide

### a. Benzyl-cyclopropylmethyl-amine

A mixture of benzaldehyde (3 mL, 29.55 mmol), cyclopropanemethylamine (2.53 mL, 29.55 mmol), AcOH (1.7 mL, 29.55 mmol) in 1,2-dichloroethane (150 mL) is stirred at RT for 25 min. Sodium triacetoxyborohydride (8.8 g, 41.37 mmol) is added and the mixture further stirred overnight at RT. Sodium triacetoxyborohydride (3.13 g, 15 mmol) is added to complete the reaction and the mixture further stirred for 5 h, then quenched by the addition of NaOH 2N. The layers are separated and the aqueous one extracted twice with ether. The combined organic extracts are dried (Na₂SO₄), filtered and concentrated. The crude material is purified by flash chromatography on silica gel (eluent: CH₂Cl₂/MeOH 95/5 to 80/20 + 5% NH₃) to give the title product. t_{R} (HPLC, nucleosil C18 column, 10-100% CH₃CN/H₂O/5 min, 100% CH₃CN/3 min, flow: 1.5 mL/min): 4.91 min.

### b. N-Benzyl-2-chloro-N-cyclopropylmethyl-acetamide

The title compound is prepared analogously as described for the title compound N-benzyl-2-chloro-N-methyl-acetamide in Example 314 from chloroacetyl chloride and benzyl-cyclopropylmethyl-amine. TLC, R_{f} (CH₂Cl₂/MeOH 95/5) = 0.9. t_{R} (HPLC, nucleosil C18 column, 10-100% CH₃CN/H₂O/5 min, 100% CH₃CN/1.5 min): 6.18 min.

### Example 321: N-((3S,4S)-4-{[(Benzvl-cyclopropyl-carbamoyl)-methyl]-amino}-pyrrolidin-3-ylmethyl)-N-isopropyl-4-methoxy-3-(3-methoxy-propoxy)-benzamide

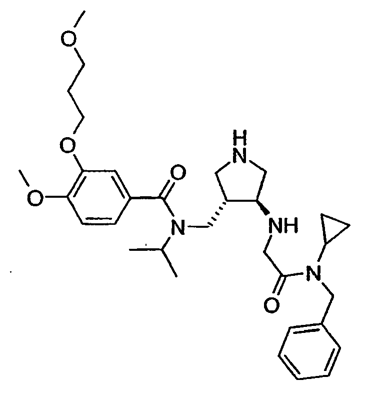

The title compound is prepared analogously as described for the title compound under B in Example 318 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and N-benzyl-2-chloro-N-cyclopropyl-acetamide except that only one equivalent of the chloride derivative is used. MS (LC-MS): 567 [M-H]⁺; t_{R} (HPLC, nucleosil C18 column, 10-100% CH₃CN/H₂O/5 min, 100% CH₃CN/3 min, flow: 1.5 mL/min): 5.68 min.

### N-Benzyl-2-chloro-N-cyclopropyl-acetamide

The title compound is prepared analogously as described for the title compound N-benzyl-2-chloro-N-cyclopropylmethyl-acetamide in Example 320 from chloroacetyl chloride and benzyl-cyclopropylmethyl-amine (prepared from benzaldehyde and cyclopropyl amine). TLC, R_{f} (CH₂Cl₂/MeOH 95/5) = 0.8. t_{R} (HPLC, nucleosil C18 column, 10-100% CH₃CN/H₂O/5 min, 100% CH₃CN/1.5 min): 5.07 min.

### Example 322: N-((3S,4S)-4-{[(Benzyl-cyclopropyl-carbamoyl)-methyl]-amino}-pyrrolidin-3-yimethyl)-4-ethyl-N-isopropyl-3-(3-methoxy-propoxy)-benzamide

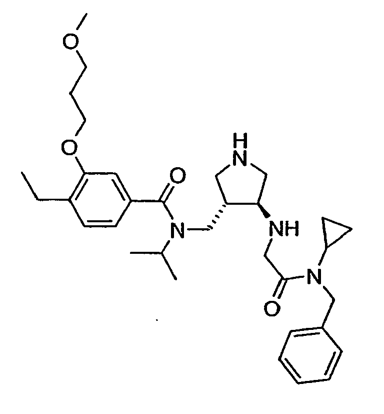

The title compound is prepared analogously as described for the title compound under B in Example 318 from (3S,4R)-3-amino-4-({[4-ethyl-3-(3-methoxy-propoxy)-benzoyl]-isopropylamino}-methyl)-pyrrolidine-1-carboxylic acid tert-butyl ester and N-benzyl-2-chloro-N-cyclopropyl-acetamide except that only one equivalent of the chloride derivative is used. MS (LC-MS): 565.3 [M-H]⁺; t_{R} (HPLC, nucleosil C18-HD column, 20-100% CH₃CN/H₂O/6 min, 100% CH₃CN/1.5 min, flow: 1 mL/min): 4.69 min.

### Example 323: N-((3S,4S)-4-{[(Cyclohexylmethyl-methyl-carbamoyl)-methyl]-amino}-pyrrolidin-3-ylmethyl)-N-isopropyl-4-methoxy-3-(3-methoxy-propoxy)-benzamide

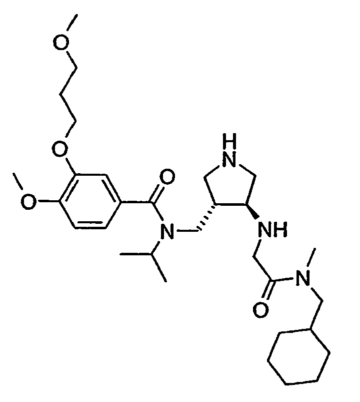

The title compound is prepared analogously as described for the title compound under B in Example 318 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 2-chloro-N-cyclohexylmethyl-N-methyl-acetamide except that only one equivalent of the chloride derivative is used. MS (LC-MS): 547 [M-H]⁺; t_{R} (HPLC, nucleosil C18 column, 10-100% CH₃CN/H₂O/5 min, 100% CH₃CN/3 min, flow: 1.5 mUmin): 5.75 min.

### 2-Chloro-N-cyclohexylmethyl-N-methyl-acetamide

### A. Cyclohexylmethyl-methyl-amine

To a solution of cyclohexancarbaldehyd (4 mL, 33.24 mmol) in 160 mL MeOH (2% AcOH) is added methylamine (50 mL, 99.8 mmol). The solution is stirred for 1 h, before the portion-wise addition of sodium borohydride (2.51 g, 66.48 mmol) at 0°C. The reaction mixture is further stirred at RT for 1 h. A solution of NaOH 1N is added and the solvent is concentrated. CH₂Cl₂ is added, the layers are separated and the aqueous one extracted twice with CH₂Cl₂. The combined organic extracts are dried over Na₂SO₄, filtered and concentrated. The crude material is purified by flash chromatography on silica gel (eluent: CH₂Cl₂/MeOH 95/5 to 80/20+ 5% NH₃) to give the title product. t_{R} (HPLC, nucleosil C18 column, 10-100% CH₃CN/H₂O/5 min, 100% CH₃CN/3 min, flow: 1.5 mL/min): 7.09 min.

### B. 2-Chloro-N-cyclohexylmethyl-N-methyl-acetamide

The title compound is prepared analogously as described for the title compound N-benzyl-2-chloro-N-methyl-acetamide in Example 314 from chloroacetyl chloride and cyclohexylmethyl-methyl-amine. TLC, R_{f} (CH₂Cl₂/MeOH 95/5) = 0.8; t_{R} (HPLC, nucleosil C18 column, 10-100% CH₃CN/H₂O/5 min, 100% CH₃CN/1.5 min): 5.27 min.

### Example 324: N-((3S,4S)-4-{[(Cyclohexylmethyl-methyl-carbamoyl)-methyl]-amino}-pyrrolidin-3-ylmethyl)-4-ethyl-N-isopropyl-3-(3-methoxy-propoxy)-benzamide

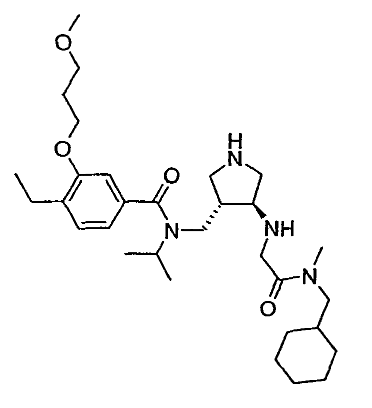

The title compound is prepared analogously as described for the title compound under B in Example 318 from (3S,4R)-3-amino-4-({[4-ethyl-3-(3-methoxy-propoxy)-benzoyl]-isopropylamino}-methyl)-pyrrolidine-1-carboxylic acid tert-butyl ester and 2-chloro-N-cyclohexylmethyl-N-methyl-acetamide except that only one equivalent of the chloride derivative is used. MS (LC-MS): 545.3 (M-H]⁺; t_{R} (HPLC, nucleosil C18-HD column, 20-100% CH₃CN/H₂O/6 min, 100% CH₃CN/1.5 min, flow: 1 mL/min): 5.26 min.

### Example 325: N-((35,45)-4-{[(Ethyl-phenyl-carbamoyl)-methyl]-amino}pyrrolidin-3-ylmethyl)-N-isopropyl-4-methoxy-3-(3-methoxy-propoxy)-benzamide

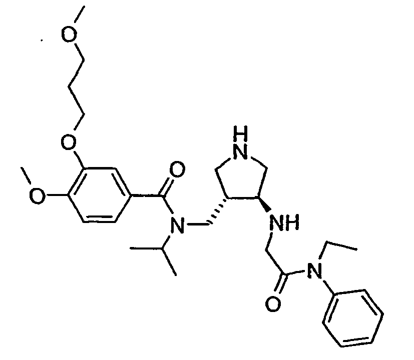

The title compound is prepared analogously as described for the title compound under B in Example 318 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 2-chloro-N-ethyl-N-phenyl-acetamide except that only one equivalent of the chloride derivative is used. MS (LC-MS): 541.5 [M-H]⁺; t_{R} (HPLC, nucleosil C18-HD column, 20-100% CH₃CN/H₂O/6 min, 100% CH₃CN/1.5 min, flow : 1 mL/min): 3.61 min.

### 2-Chloro-N-ethyl-N-phenyl-acetamide

The title compound is prepared analogously as described for the title compound N-benzyl-2-chloro-N-methyl-acetamide in Example 314 from chloroacetyl chloride and N-ethylaniline. TLC, R_{f} (CH₂Cl₂/MeOH 95/5) = 0.8; t_{R} (HPLC, nucleosil C18 column, 10-100% CH₃CN/H₂O/5 min, 100% CH₃CN/1.5 min): 4.95 min.

### Example 326: N-((3S,4S)-4-{[(Cyclohexylmethyl-cyclopropyl-carbamoyl)-methyl]-amino}-pyrrolidin-3-ylmethyl)-N-isopropyl-4-methoxy-3-(3-methoxy-propoxy)-benzamide

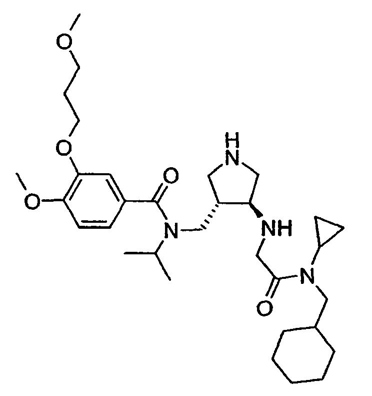

The title compound is prepared analogously as described for the title compound under B in Example 318 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 2-chloro-N-cyclohexylmethyl-N-cyclopropyl-acetamide except that only one equivalent of the chloride derivative is used. MS (LC-MS): 573.5 [M-H]⁺; t_{R} (HPLC, nucleosil C18-HD column, 20-100% CH₃CN/H₂O/6 min, 100% CH₃CN/1.5 min, flow : 1 mL/min): 4.65 min.

### 2-chloro-N-cyclohexylmethyl-N-cyclopropyl-acetamide

The title compound is prepared analogously as described for the title compound N-benzyl-2-chloro-N-methyl-acetamide in Example 314 from chloroacetyl chloride and cyclohexylmethyl-cyclopropyl-amine amine (prepared from cyclohexancarbaldehyde and cyclopropyl amine). TLC, R_{f} (CH₂Cl₂/MeOH 95/5) = 0.8; t_{R} (HPLC, nucleosil C18 column, 10-100% CH₃CN/H₂O/5 min, 100% CH₃CN/1.5 min): 5.99 min.

### Example 327: N-((3S,4S)-4-{[(Cyclohexyl-ethyl-carbamoyl)-methyl]-amino}-pyrrolidin-3-ylmethyl)-N-isopropyl-4-methoxy-3-(3-methoxy-propoxy)-benzamide

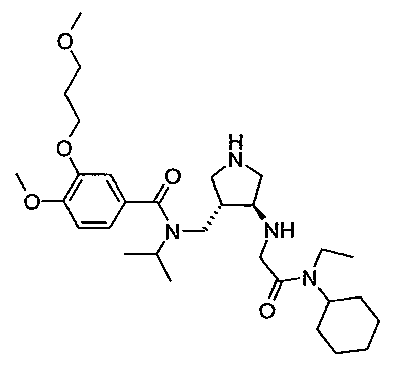

The title compound is prepared analogously as described for the title compound under B in Example 318 from (3S,4R)-3-amino-4-({isopropyl-[4-methoxy-3-(3-methoxy-propoxy)-benzoyl]-amino}-methyl)-pyrrolidine-1-carboxylic acid tert-butyl ester and 2-chloro-N-cyclohexyl-N-ethyl-acetamide except that only one equivalent of the chloride derivative is used. MS (LC-MS): 567.5 [M-H]⁺; t_{R} (HPLC, nucleosil C18-HD column, 20-100% CH₃CN/H₂O/6 min, 100% CH₃CN/1.5 min, flow : 1 mL/min): 4.04 min.

### 2-Chloro-N-cyclohexyl-N-ethyl-acetamide

The title compound is prepared analogously as described for the title compound N-benzyl-2-chloro-N-methyl-acetamide in Example 314 from chloroacetyl chloride and N-ethylcyclohexylamine. TLC, R_{f} (CH₂Cl₂/MeOH 95/5) = 0.8; t_{R} (HPLC, nucleosil C18 column, 10-100% CH₃CN/H₂O/5 min, 100% CH₃CN/1.5 min): 5.6 min.

### Example 328: N-[(3S,4S)-4-(2-Hydroxy-3-phenyl-propylamino)-pyrrolidin-3-ylmethyl]-N-isopropyl-4-methoxy-3-(3-methoxy-propoxy)-benzamide

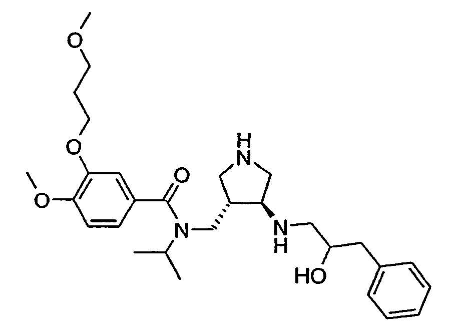

### A. (35,4R)-3-(2-Hydroxy-3-phenyl-propylamino)-4-({isopropyl-[4.methoxy-3-(3-methoxy-propoxy)-benzoyl]-amino}-methyl)-pyrrolidine-1-carboxylic acid tert-butyl ester

(35,4R)-3-Amino-4-({isopropyl-[4-methoxy-3-(3-methoxy-propoxy)-benzoyl]-amino}-methyl)-pyrrolidine-1-carboxylic acid tert-butyl ester (300 mg, 0.625 mmol) and (2,3-epoxypropyl)-benzene (83 µL, 0.625 mmol) in EtOH (5 mL) are heated at 50°C overnight. (2,3-epoxypropyl)-benzene (83 µL, 0.625 mmol) is again added and the mixture further stirred 1 day to complete the reaction. The solvent is concentrated and the crude material is purified by flash chromatography on silica gel (eluent: CH₂Cl₂/MeOH 100/0 to 95/5) to give the title product. TLC, R_{f} (CH₂Cl₂/MeOH 95/5) = 0.20. MS (LC-MS): 613.9 [M-H]⁺; t_{R} (HPLC, nucleosil C18 column, 10-100% CH₃CN/H₂O/5 min, 100% CH₃CN/3 min, flow: 1.5 mL/min): 4.54 min.

### B. N-[(3S,4S)-4-(2-Hydroxy-3-phenyl-propylamino)-pyrrolidin-3-ylmethyl]-N-isopropyl-4-methoxy-3-(3-methoxy-propoxy)-benzamide

To a solution of (3S,4R)-3-(2-hydroxy-3-phenyl-propylamino)-4-({isopropyl-[4-methoxy-3-(3-methoxy-propoxy)-benzoyl]-amino}-methyl)-pyrrolidine-1-carboxylic acid tert-butyl ester (225 mg, 0.367 mmol) in dioxane (3 mL) is added 4N HCl in dioxane (2 mL). The mixture is stirred for 2 h at RT and the resulting solution is lyophilized to afford the corresponding bishydrochloride salt as a white powder. MS (LC-MS): 514 [M-H]⁺; t_{R} (HPLC, nucleosil C18 column, 10-100% CH₃CN/H₂O/5 min, 100% CH₃CN/3 min, flow: 1.5 mL/min): 4.11 min.

### Example 329: N-{(3S,4S)-4-[(3,4-Dihydro-2H-benzo[1,4]oxazin-6-ylmethyl)-amino]-pyrrolidin-3-ylmethyl}-N-isopropyl-4-methoxy-3-(3-methoxy-propoxy)-benzamide

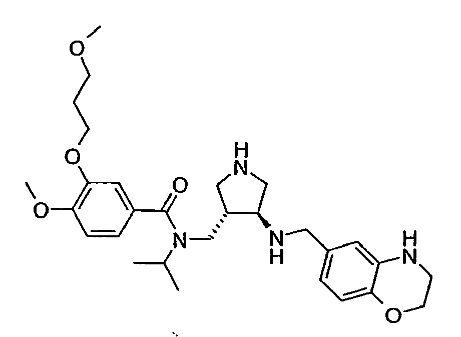

### A. 6-([(3S,4R)-l-tert-Butoxycarbonyl-4-({isopropyl-[4-methoxy-3-(3-methoxy-propoxy)-benzoyl]-amino}methyl)-pyrrolidin-3-ylamino]-methyl)-2,3-dihydro-benzo[1,4]oxazine-4-carboxylic acid tert-butyl ester

A mixture of (3S,4R)-3-Amino-4-({isopropyl-[4-methoxy-3-(3-methoxy-propoxy)-benzoyl]-amino}-methyl)-pyrrolidine-1-carboxylic acid tert-butyl ester (150 mg, 0.313 mmol) and 6-formyl-2,3-dihydro-benzo[1,4]oxazine-4-carboxylic acid tert-butyl ester (90 mg, 0.344 mmol) in 1,2-dichloroethane (4 mL) is stirred at RT for 30 min, before addition of sodium triacetoxyborohydride (199 mg, 0.939 mmol). The mixture is stirred for 2 days at RT under N₂ atmosphere and quenched by addition of a saturated aqueous NaHCO₃ solution. The layers are separated and the aqueous one twice with CH₂Cl₂. The combined organic extracts are dried (Na₂SO₄), filtered and concentrated. The crude material is purified by flash chromatography on silica gel (eluent: CH₂Cl₂/MeOH 95/5) to give the title product. TLC, R_{f} (CH₂Cl₂/MeOH 95/5) = 0.20. MS (LC-MS): 727.1 [M-H]⁺; t_{R} (HPLC, nucleosil C18 column, 10-100% CH₃CN/H₂O/5 min, 100% CH₃CN/3 min, flow: 1.5 mL/min): 5.99 min.

### B. N-{(3S,4S)-4-[(3,4-Dihydro-2H-benzo[1,4]oxazin-6-ylmethyl)-amino]-pyrrolidin-3-ylmethyl}-N-isopropyl-4-methoxy-3-(3-methoxy-propoxy)-benzamide

To a solution of 6-{[(3S,4R)-l-tert-butoxycarbonyl-4-({isopropyl-[4-methoxy-3-(3-methoxy-propoxy)-benzoyl]-amino}-methyl)-pyrrolidin-3-ylamino]-methyl}-2,3-dihydro-benzo[1,4]oxazine-4-carboxylic acid tert-butyl ester (104 mg, 0.143 mmol) in dioxane (2 mL) is added 4N HCl in dioxane (1 mL). The mixture is stirred for 2 h at RT and the resulting solution is lyophilized to afford the corresponding bishydrochloride salt as a white powder. MS (LC-MS): 527.1 [M-H]⁺; t_{R} (HPLC, nucleosil C18 column, 10-100% CH₃CN/H₂O/5 min, 100% CH₃CN/3 min, flow: 1.5 mL/min): 4.74 min.

### 6-Formyl-2,3-dihydro-benzo[1,4]oxazine-4-carboxylic acid tert-butyl ester

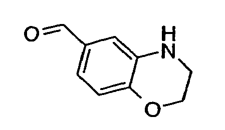

### a. 6-Hydroxymethyl-2,3-dihydro-benzo[1,4]oxazine-4-carboxylic acid tert-butyl ester

To a solution of 2,3-dihydro-benzo[1,4]oxazine-4,6-dicarboxylic acid 4-tert-butyl ester 6-methyl ester (described in Example 291) (250 mg, 0.852 mmol) in THF (3 mL), cooled in an iced bath, is added under N₂ a solution of LiBH₄ (2 N in THF, 426 µL, 0.852 mmol). The reaction mixture is allowed to reach RT and stirred overnight. LiBH₄ (852 µL, 1.704 mmol) is added and the reaction mixture is heated to 60°C to complete the reaction. The mixture is quenched by addition of an aqueous saturated solution of NaHCO₃, and extracted twice with AcOEt. The combined organic layers are dried over Na₂SO₄, filtered and concentrated to give the title product. TLC, R_{f} (c-hexane/AcOEt) = 0.1. t_{R} (HPLC, nucleosil C18 column, 10-100% CH₃CN/H₂O/5 min, 100% CH₃CN/3 min, flow: 1.5 mL/min): 5.01 min.

### b. 6-Formyl-2,3-dihydro-benzo[1,4]oxazine-4-carboxylic acid tert-butyl ester

To a well stirred mixture of 6-hydroxymethyl-2,3-dihydro-benzo[1,4]oxazine-4-carboxylic acid tert-butyl ester (218 mg, 0.822 mmol) and Dess-Martin Periodinane (349 mg, 0.822 mmol) in CH₂Cl_{z} (5 mL) is slowly added wet CH₂Cl₂ (16.3 µL of water in 2 mL of CH₂Cl₂). The cloudy mixture is stirred for 2 h at RT. Then diluted with Et₂O and carefully concentrated to a few mL of solvent by rotary evaporation. The residue is taken up in Et₂O and washed with a 1/1 solution of a 10% aqueous solution of Na₂S₂O₃ and a saturated aqueous solution of NaHCO₃. The layers are separated and the aqueous one is back-extracted twice with Et₂O. The combined organic extracts are dried with Na₂SO₄, filtered and concentrated to give the title product which is used witout further purification in the next step. TLC, R_{f} (c-hexane/AcOEt 2/1) = 0.6. MS (LC-MS): 264.0 [M-H]⁺; t_{R} (HPLC, nucleosil C18 column, 10-100% CH₃CN/H₂O/5 min, 100% CH₃CN/3 min, flow: 1.5 mL/min): 5.75 min.

### Example 330: N-{(3S*,4S*)-4-[(Benzyl-cyclopropyl-carbamoyl)-methoxy}-pyrrolidin-3-ylmethyl}-N-isopropyl-4-methoxy-3-(3-methoxy-propoxy)-benzamide

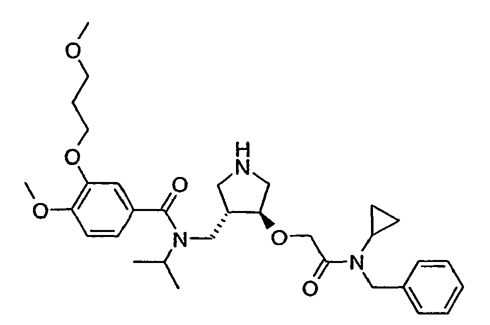

### A. (35*,4R*)-3-[(Benzyl-cyclopropyl-carbamoyl)-methoxy]-4-({isopropyl-[4-methoxy-3-(3-methoxy-propoxy)-benzoyl]-amino}-methyl)-pyrrolidine-1-carboxylic acid tert-butyl ester

To a stirred suspension of NaH (60% oily dispersion, 18 mg, 0.468 mmol) in THF (2 mL) at 0°C is added under N₂ atmosphere (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester (150 mg, 0.312 mmol) dropwise in THF (3 mL). The resulting mixture is stirred for 30 min. and a solution of N-benzyl-2-chloro-N-cyclopropyl-acetamide (described in example 321) (105 mg, 0.468 mmol) in THF (3 mL) is added dropwise. The stirring is further continued for 3 h at RT. The solvent is removed under vacuum, AcOEt is added and the reaction mixture poured into a saturated aqueous solution of NH₄Cl. The layers are separated and the aqueous one extracted twice with AcOEt. The combined organic extracts are washed with brine, dried over Na₂SO₄, filtered and concentrated. The crude material is purified by flash chromatography on silica gel (eluent CH₂Cl₂/MeOH 100/0 to 98/2) to give the title product. TLC, R_{f} (CH₂Cl₂/MeOH 95/5) = 0.2. MS (LC-MS): 668.3 [M+H]⁺; t_{R} (HPLC, nucleosil C18 column, 10-100% CH₃CN/H₂O/5 min, 100% CH₃CN/2.5 min, flow: 1.5 mL/min): 5.94 min.

### B. N-{(3S*,4S*)-4-[(Benzyl-cyclopropyl-carbamoyl)-methoxy]-pyrrolidin-3-yimethyl}-N-isopropyl-4-methoxy-3-(3-methoxy-propoxy)-benzamide

To a solution of (3S*,4R*)-3-[(benzyl-cyclopropyl-carbamoyl)-methoxy]-4-({isopropyl-[4-methoxy-3-(3-methoxy-propoxy)-benzoyl]-amino}-methyl)-pyrrolidine-1-carboxylic acid tert-butyl ester (124 mg, 0.186 mmol) in dioxane (2 mL) is added 4N HCl in dioxane (1 mL). The mixture is stirred for 2 h at RT and the resulting solution is lyophilized to afford the corresponding hydrochloride salt as a white powder. MS (LC-MS): 568.2 [M+H]⁺; t_{R} (HPLC, nucleosil C18-HD column, 20-100% CH₃CN/H₂O/6 min, 100% CH₃CN/1.5 min, flow: 1 mL/min): 4.01 min.

### Example 331: N-{(3S*,4S*)-4-[(Cyclohexylmethyl-methyl-carbamoyl)-methoxy]-pyrrolidin-3-ylmethyl}-N-isopropyl-4-methoxy-3-(3-methoxy-propoxy)-benzamide

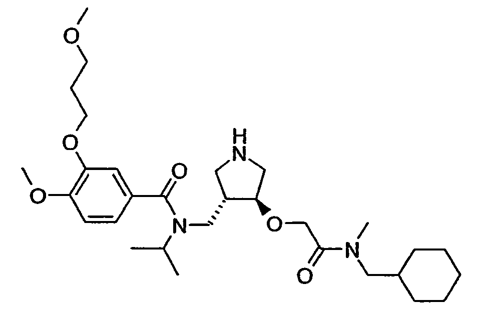

The title compound is prepared analogously as described for the title compound under B in Example 330 from (3S*,4R*)-3-hydroxy-4-({isopropyl-[4-methoxy-3-(3-methoxy-propoxy)-benzoyl]-amino}-methyl)-pyrrolidine-1-carboxylic acid tert-butyl ester and 2-chloro-N-cyclohexylmethyl-N-methyl-acetamide (described in example 323). MS (LC-MS): 546.6 [M-H]⁺; t_{R} (HPLC, nucleosil C18-HD column, 20-100% CH₃CN/H₂O/6 min, 100% CH₃CN/1.5 min, flow: 1 mL/min): 5.10 min. MS (LC-MS): 548.3 [M+H]⁺; t_{R} (HPLC, nucleosil C18-HD column, 20-100% CH₃CN/H₂O/6 min, 100% CH₃CN/1.5 min, flow: 1 mL/min): 4.16 min.

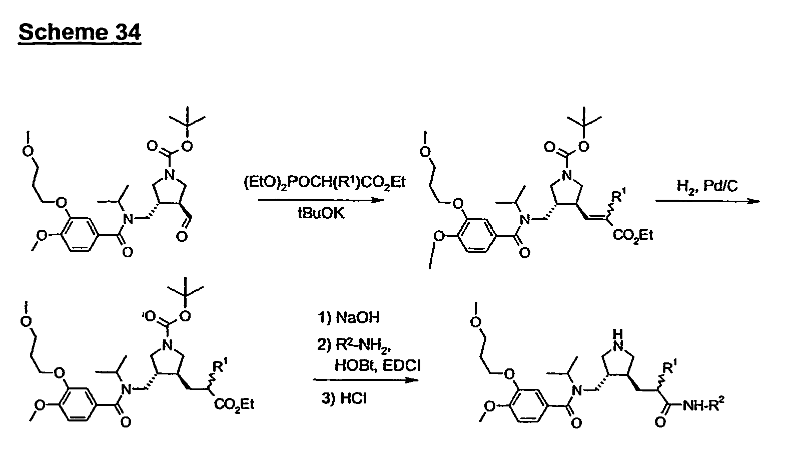

### Example 332: N-Isopropyl-4-methoxy-3-(3-methoxy-propoxy)-N-{(3S,4S)-4-[2-(tetrahydro-pyran-4-ylcarbamoyl)-ethyl]-pyrrolidin-3-ylmethyl}-benzamide

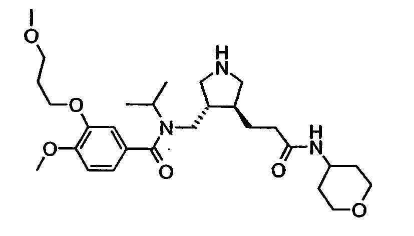

The title compound is prepared according to the route depicted in Scheme 34:
To a solution of (3R,4S)-3-({isopropyl-[4-methoxy-3-(3-methoxy-propoxy)-benzoyl]-amino}-methyl)-4-[2-(tetrahydro-pyran-4-ylcarbamoyl)-ethyl]-pyrrolidine-1-carboxylic acid tert-butyl ester (207 mg, 0.334 mmol) in dioxane (1.5 mL) is added a 4M solution of HCl in dioxane (0.835 mL) at room temperature. The mixture is stirred overnight, followed by freeze-drying in *high vacuo* to give the title compound as the hydrochloride salt. MS: 520.3 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 3.70 min.

The starting material is obtained as described below:

### A. (3R,4S)-3-({isopropyl-[4-methoxy-3-(3-methoxy-propoxy)-benzoyl]-amino}-methyl)-4-[2-(tetrahydro-pyran-4-ylcarbamoyl)-ethyl]-pyrrolidine-1-carboxylic acid tert-butyl ester

To a solution of (3S,4R)-3-(2-carboxy-ethyl)-4-({isopropyl-[4-methoxy-3-(3-methoxy-propoxy)-benzoyl]-amino}-methyl)-pyrrolidine-1-carboxylic acid tert-butyl ester (0.19 g, 0.354 mmol) in CH₂Cl₂ (4 mL) are subsequently added 4-amino-tetrahydropyrane (0.043 g, 0.425), HOBT (0.057 g, 0.425 mmol), EDCI (0.081 g, 0.425 mmol) and Et₃N (0.043 g, 0.425 mmol), and the reaction mixture is stirred at room temperature overnight. After dilution with CH₂Cl₂, the organic phase is washed with aqueous 1M HCl, saturated aqueous NaHCO₃ solution and brine (5 mL each), dried (MgSO₄) and concentrated. The residue is purified by flash chromatography on silica gel (CH₂Cl₂/MeOH 95:5) to give the title compound as colorless foam. MS: 620.4 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mUmin): 4.92 min.

### B. (3S,4R)-3-(2-Carboxy-ethyl)-4-({isopropyl-[4-methoxy-3-(3-methoxy-propoxy)-benzoyl]-amino}-methyl)-pyrrolidine-1-carboxylic acid tert-butylester

To the solution of (3S,4R)-3-(2-ethoxycarbonyl-ethyl)-4-({isopropyl-[4-methoxy-3-(3-methoxy-propoxy)-benzoyl]-amino}-methyl)-pyrrolidine-1-carboxylic acid tert-butyl ester (0.48 g, 0.765 mmol) in ethanol (5 mL) is added aqueous 2M NaOH (0.574 mL), and the mixture is stirred for 3 h at room temperature. Volatiles are removed *in vacuo*, and the diluted aqueous phase is acidified to pH 1 by addition of conc. HCl, followed by extraction with AcOEt. The combined organics are dried (Na₂SO₄) and concentrated to afford the title compound as colorless oil. MS: 537.2 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 4.91 min.

### C. (3S,4R)-3-(2-Ethoxycarbonyl-ethyl)-4-({isopropyl-[4-methoxy-3-(3-methoxy-propoxy)-benzoyl]-amino}-methyl)-pyrrolidine-1-carboxylic acid tert-butyl ester

The solution of (3S,4R)-3-((E/Z)-2-ethoxycarbonyl-vinyl)-4-({isopropyl-[4-methoxy-3-(3-methoxy-propoxy)-benzoyl]-amino}-methyl)-pyrrolidine-1-carboxylic acid tert-butyl ester (0.53 g, 0.94 mmol) in MeOH (10 mL) is hydrogenated in the presence of Pd/C 10% (0.1 g) at room temperature. The mixture is filtered through Hyflo®, and after washing the combined filtrates are concentrated *in vacuo* to give the title compound as colorless oil. MS: 565.2 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 5.65 min.

### D. (3S,4R)-3-((E/Z)-2-Ethoxycarbonyl-vinyl)-4-({isopropyl-[4-methoxy-3-(3-methoxy-propoxy)-benzoyl]-amino}-methyl)-pyrrolidine-1-carboxylic acid tert-butyl ester

To the solution of triethyl 2-phosphonoacetate (0.483 mL, 2.44 mmol) in THF (2 mL) is added dropwise a solution of potassium tert-butoxide (0.228 g, 2.03 mmol) in THF (1 mL) under a N₂ atmosphere. After stirring for 30 min, the solution of (3S,4R)-3-fonnyl-4-({isopropyl-[4-methoxy-3-(3-methoxy-propoxy)-benzoyl]-amino}methyl)-pyrrolidine-1-carboxylic acid tert-butyl ester (0.50 g, 1.02 mmol) in THF (1 mL) is added dropwise. To the resulting slurry is added THF (4 mL), followed by stirring for 2 h at room temperature. The mixture is poured into a mixture of saturated aqueous NH₄Cl (5 mL) and water (50 mL), followed by extraction with diethylether. The combined organics are washed with brine, dried (Na₂SO₄) and concentrated. Purification of the residue by flash chromatography over silica gel (hexane/AcOEt 1:3) gives the title compound as colorless oil. MS: 563.2 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 5.68 min.

### Example 333: N-{(3S,4S)-4-[2-(Benzyl-ethyl-carbamoyl)-ethyl]-pyrrolidin-3-ylmethyl}-N-isopropyl-4-methoxy-3-(3-methoxy-propoxy)-benzamide

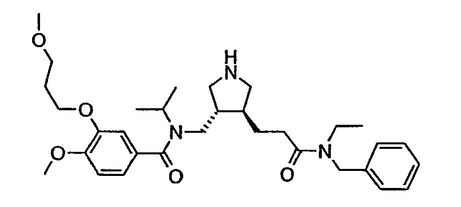

The title compound is prepared as its hydrochloride salt by the procedure described in Example 332, starting from (3S,4R)-3-[2-(benzyl-ethyl-carbamoyl)-ethyl]-4-({isopropyl-[4-methoxy-3-(3-methoxy-propoxy)-benzoyl]-amino}-methyl)-pyrrolidine-1-carboxylic acid tert-butyl ester (0.12 g, 0.184 mmol), to give a white foam. MS: 554.4 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 4.67 min.

The starting material is obtained as described follows:

### A. (35,4R)-3-[2-(Benzyl-ethyl-carbamoyl)-ethyl]-4-({isopropyl-[4-methoxy-3-(3-methoxy-propoxy)-benzoyl]-amino}-methyl)-pyrrolidine-1-carboxylic acid tert-butyl ester

From (3S,4R)-3-(2-carboxy-ethyl)-4-({isopropyl-[4-methoxy-3-(3-methoxy-propoxy)benzoyl]-amino}-methyl)-pyrrolidine-1-carboxylic acid tert-butyl ester (0.19 g, 0.354 mmol) and benzylethylamine (0.063 mL, 0.425 mmol) by the procedure described in Example 333/A, to give the title compound as colorless oil. MS: 654.4 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 5.84 min.

### Example 334: N-Isopropyl-4-methoxy-3-(3-methoxy-propoxy)-N-{(3S,4S)-4-[2(R,S)-(tetrahydro-pyran-4-ylcarbamoyl)-butyl]-pyrrolidin-3-ylmethyl}-benzamide

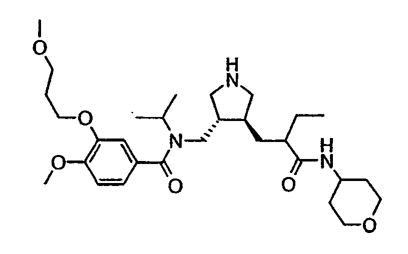

The title compound is prepared as its hydrochloride salt by the procedure described in Example 332, starting from (3R,4S)-3-({isopropyl-[4-methoxy-3-(3-methoxy-propoxy)-benzoyl]-amino}-methyl)-4-[2(R,S)-(tetrahydro-pyran-4-ylcarbamoyl)-butyl]-pyrrolidine-1-carboxylic acid tert-butyl ester (0.146 g, 0.225 mmol). MS: 548.4 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 3.95/4.00 min (ca. 1:1 ratio of diastereoisomers).

The starting material is obtained as described follows:

### A. (3R,4S)-3-({Isopropyl-[4-methoxy-3-(3-methoxy-propoxy)-benzoyl]-amino}-methyl)-4-[2(R,S)-(tetrahydro-pyran-4-ylcarbamoyl)-butyl]-pyrrolidine-1-carboxylic acid tert-butyl ester

From (3S,4R)-3-(2-carboxy-butyl)-4-({isopropyl-[4-methoxy-3-(3-methoxy-propoxy)-benzoyl]-amino}-methyl)-pyrrolidine-1-carboxylic acid tert-butyl ester (0.20 g, 0.354 mmol) according to the procedure described in Example 332/A and 4-amino-tetrahydropyrane (0.043 g, 0.425). The title compound is obrained after purification by flash chromatography on silica gel (hexane/ AcOEt 1:1 and AcOEt) as colorless oil. MS: 648.4 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 5.16 min.

### B. (3S,4R)-3-(2(R,S)-Carboxy-butyl)-4-({isopropyl-[4-methoxy-3-(3-methoxy-propoxy)-benzoyl]-amino}-methyl)-pyrrolidine-1-carboxylic acid tert-butyl ester

From (3S,4R)-3-(2(R,S)-ethoxycarbonyl-butyl)-4-({isopropyl-[4-methoxy-3-(3-methoxy-propoxy)-benzoyl]-amino}-methyl)-pyrrolidine-1-carboxylic acid tert-butyl ester (0.54 g, 0.91 mmol) according to the procedure described in Example 332/B as an off-white powder. MS: 565.3 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 5.26 min.

### C. (35,4R)-3-(2(R,S)-Ethoxycarbonyl-butyl)-4-({isopropyl-[4-methoxy-3-(3-methoxy-propoxy)-benzoyl]-amino}-methyl)-pyrrolidine-1-carboxylic acid tert-butyl ester

The solution of (3S,4R)-3-((E/Z)-2-ethoxycarbonyl-but-1-enyl)-4-({isopropyl-[4-methoxy-3-(3-methoxy-propoxy)-benzoyl]-amino}-methyl)-pyrrolidine-1-carboxylic acid tert-butyl ester (1.38 g, 2.34 mmol) in MeOH (25 mL) is hydrogenated in the presence of Pd/C 10% (0.25 g) at room temperature. The mixture is filtered through Hyflo®, and after washing the combined filtrates are concentrated. The residue is purified by flash chromatography on silica gel (hexane/ AcOEt 1:3) to give the title compound as colorless oil. MS: 593.4 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 6.04 min.

### D. (3S,4R)-3-((E/Z)-2-Ethoxycarbonyl-but-1-enyl)-4-({isopropyl-[4-methoxy-3-(3-methoxy-propoxy)-benzoyl]-amino}-methyl)-pyrrolidine-1-carboxylic acid tert-butyl ester

To the solution of triethyl 2-phosphonoacetate (1.33 mL, 5.60 mmol) in THF (4 mL) is added dropwise a solution of potassium tert-butoxide (0.524 g, 4.67 mmol) in THF (2 mL) under a N2 atmosphere. After stirring for 30 min, the solution of (3S,4R)-3-formyl-4-({isopropyl-[4-methoxy-3-(3-methoxy-propoxy)-benzoyl]-amino}-methyl)-pyrrolidine-1-carboxylic acid tert-butyl ester (1.15 g, 2.33 mmol) in THF (2 mL) is added dropwise. The resulting slurry is stirred for 2 h at room temperature. The mixture is poured into a mixture of saturated aqueous NH₄Cl (10 mL) and water (100 mL), followed by extraction with diethylether. The combined organics are washed with brine, dried (Na₂SO₄) and concentrated. Purification of the residue by flash chromatography over silica gel (hexane/ AcOEt 1:1 and 1:3) gives the title compound as colorless oil. MS: 591.2 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 6.07/6.17 min.

### Example 335: N-{(3S,4S)-4-[2(R,S)-(Benzyl-ethyl-carbamoyl)-butyl]-pyrrolidin-3-ylmethyl}-N-isopropyl-4-methoxy-3-(3-methoxy-propoxy)-benzamide

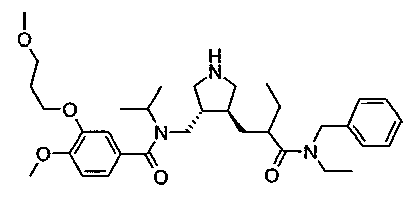

The title compound is prepared as its hydrochloride salt by the procedure described in Example 332, starting from (3S,4R)-3-[2(R,S)-(benzyl-ethyl-carbamoyl)-butyl]-4-({isopropyl-[4-methoxy-3-(3-methoxy-propoxy)-benzoyl]-amino}-methyl)-pyrrolidine-1-carboxylic acid tert-butyl ester (0.188 g, 0.276 mmol) to give a white solid. MS: 582.4 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 4.86 min.

The starting material is obtained as described follows:

### A. (3S,4R)-3-[2(R,S)-(Benzyl-ethyl-carbamoyl)-butyl]-4-({isopropyl-[4-methoxy-3-(3-methoxy-propoxy)-benzoyl]-amino}-methyl)-pyrrolidine-1-carboxylic acid tert-butyl ester

From (3S,4R)-3-(2-carboxy-butyl)-4-({isopropyf-[4-methoxy-3-(3-methoxy-propoxy)-benzoyl]-amino}-methyl)-pyrrolidine-1-carboxylic acid tert-butyl ester (0.20 g, 0.354 mmol) and benzylethylamine (0.063 mL, 0.425 mmol) by the procedure described in Example 334/A, to give the title compound as colorless oil. MS: 682.4 [M+H]⁺; t_{R} (HPLC, nucleosil C18-HD column (4x70 mm, 3 µm), 5-100% CH₃CN/H₂O/0.1% TFA/6 min, 100% CH₃CN/0.1% TFA, 1.5 min, flow: 1 mL/min): 6.08 min.

The starting material described in Example 332/D is prepared according to Scheme35 and as described below:

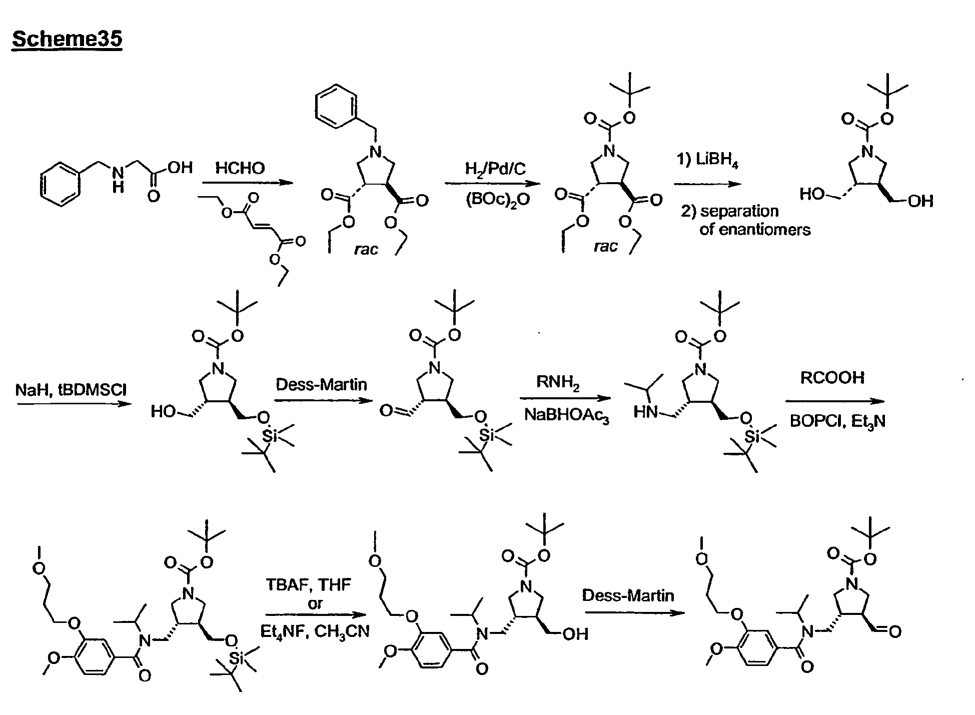

### Example 336: (3S,4R)-3-Formyl-4-({isopropyl-[4-methoxy-3-(3-methoxy-propoxy)-benzoyl]-amino}-methyl)-pyrrolidine-1-carboxylic acid tert-butyl ester

The title compound is prepared by the procedure described below under E from (3S,4R)-3-hydroxymethyl-4-({isopropyl-[4-methoxy-3-(3-methoxy-propoxy)-benzoyl]-amino}-methyl)-pyrrolidine-1-carboxylic acid tert-butyl ester. TLC, R_{f} (CH₂Cl₂/MeOH 95/5) = 0.3. t_{R} (HPLC, Nucleosil C18 column, 10-100% CH₃CN/H₂O/5 min, 100% CH₃CN/3 min, CH₃CN and H₂O containing 0.1% TFA, flow: 1.5 mL/min): 5.16 min.

The starting material is obtained as follows:

### A. (3S,4R)-3-Hydroxymethyl-4-({isopropyl-[4-methoxy-3-(3-methoxy-propoxy)-benzoyl]-amino}-methyl)-pyrrolidine-1-carboxylic acid tert-butyl ester

To a solution of (3S,4R)-3-(tert-butyl-dimethyl-silanyloxymethyl)-4-({isopropyl-[4-methoxy-3-(3-methoxy-propoxy)-benzoyl]-amino}-methyl)-pyrrolidine-1-carboxylic acid tert-butyl ester (8.75 g, 14.37 mmol) in THF (50 mL) is added tetrabutylammonium fluoride trihydrate (6.8 g, 24.55 mmol) under a nitrogen atmosphere. The reaction mixture is stirred overnight. Water and EtOAc are added, the layers are separated and the aqueous one extracted twice with EtOAc. The combined organic extracts are dried (Na₂SO₄), and the solvent is removed in *vacuo.* The crude product is purified by flash chromatography on silica gel (eluent: CH₂Cl₂/MeOH 97/3 to 95/5) to give the title product. TLC, R_{f} (CH₂Cl₂/MeOH 95/5) = 0.3. MS (LC-MS): 395.1 [M+H-Boc]+; t_{R} (HPLC, C18 column, 10-100% CH₃CN/H₂O/5 min, 100% CH₃CN/3 min, CH₃CN and H₂O containing 0.1% TFA, flow: 1.5 mL/min): 5.01 min.

### B. (3S,4R)-3-(tert-Butyl-dimethyl-silanyloxymethyl)-4-({isopropyl-[4-methoxy-3-(3-methoxy-propoxy)-benzoyl]-amino}-methyl)-pyrrolidine-1-carboxylic acid tert-butyl ester

A mixture of ((3S,4R)-3-(tert-butyl-dimethyl-silanyloxymethyl)-4-(isopropylamino-methyl)-pyrrolidine-1-carboxylic acid tert-butyl ester (8.9 g, 23.02 mmol), 3-(3-methoxy-propoxy)-4-methoxy-benzoic acid (6.08 g, 25.32 mmol), bis(2-oxo-3-oxazolidinyl)phosphinic chloride (6.45 g, 25.32 mmol) and triethylamine (7.62 mL, 92.07 mmol) in CH₂Cl₂ (230 mL) is refluxed for 3 h. The reaction is quenched by the addition of an aqueous saturated solution of NaHCO₃. The organic layer is separated, and the aqueous phase is extracted 3 times with AcOEt. The combined organic extracts are dried (Na₂SO₄), and the solvent is removed *in vacuo.* The crude product is purified by flash chromatography on silica gel (eluent: CH₂Cl₂/acetonee 95/5 to CH₂Cl₂/MeOH 95/5) to give the title product. TLC, R_{f} (CH₂Cl₂/MeOH 95/5) = 0.47. MS (LC-MS): 609.4 [M+H]⁺; t_{R} (HPLC, C18 column, 5-100% CH₃CN/H₂O/6 min, 100% CH₃CN/2 min, CH₃CN and H₂O containing 0.1% TFA, flow: 1 mL/min): 7.05 min.

### C. (3S,4R)-3-(tert-Butyl-dimethyl-silanyloxymethyl)-4-(isopropylamino-methyl)-pyrrolidine-1-carboxylic acid tert-butyl ester

A solution of (3S,4S)-3-(tert-butyl-dimethyl-silanyloxymethyl)-4-formyl-pyrrolidine-1-carboxylic acid tert-butyl ester (7.7 g, 22.41 mmol) and isopropylamine (5.78 mL, 67.24 mmol) in 1,2-dichloroethane (200 mL) is stirred 25 min before the addition of NaBH(OAc)₃ (11.88 g, 56.03 mmol). The solution is stirred for 5 h, then diluted with CH₂Cl₂ and washed with an aqueous saturated solution of NaHCO₃. The aqueous layer is back extracted twice with CH₂Cl₂ and the combined organic extracts are dried over Na₂SO₄, filtered and concentrated. The crude material is used in the next step without further purification. TLC, R_{f} (CH₂Cl₂/MeOH 90/10) = 0.39. MS (LC-MS): 387.2 [M+H]⁺.

### D. (3S,4S)-3-(tert-Butyl-dimethyl-silanyloxymethyl)-4-formyl-pyrrolidine-1-carboxylic acid tert-butyl ester

To a well stirred mixture of (3S,4S)-3-(tert-butyl-dimethyl-silanyloxymethyl)-4-hydroxymethyl-pyrrolidine-1-carboxylic acid tert-butyl ester (8.2 g, 23.73 mmol) and Dess-Martin periodinane (10.06 g, 23.73 mmol) in CH₂Cl₂ (60 mL), slowly wet CH₂Cl₂ (0.47 mL of water in 60 mL of CH₂Cl₂) is added. The clear solution becomes cloudy toward the end of wet CH₂Cl₂ addition and is further stirred over-night. Then concentrated to a few mL of solvent by rotary evaporation and taken up in Et₂O. A solution of 1/1 10% Na₂S₂O₃/saturated aqueous NaHCO₃ is added. The layers are separated and the organic extract is washed successively with H₂O and brine. The aqueous washings are back-extracted with Et₂O, and this organic layer is washed with H₂O and brine. The combined organic layers are dried with Na₂SO₄, filtered and concentrated. The crude mixture is used in the next step without further purification. TLC, R_{f} (CH₂Cl₂/MeOH 95/5) = 0.42. MS (LC-MS): 244.2 [M+H-Boc]⁺; t_{R} (HPLC, C18 column, 5-100% CH₃CN/H₂O/6 min, 100% CH₃CN/2 min, CH₃CN and H₂O containing 0.1% TFA, flow: 1 mL/min): 6.45 min.

### E. (3S,4S)-3-(tert-Butyl-dimethyl-silanyloxymethyl)-4-hydroxymethyl-pyrrolidine-1-carboxylic acid tert-butyl ester

To a suspension of sodium hydride (60 % in oil, 0.996 g, 24.9 mmol) (previously washed with pentane) in THF (40 mL) is added dropwise under a nitrogen atmosphere a solution of (3S,4S)-3,4-bis-hydroxymethyl-pyrrolidine-1-carboxylic acid tert-butyl ester (4.8 g, 20.75 mmol) in THF (40 mL) at 0°C. The mixture is stirred for 1.5 h at 0°C before the dropwise addition of a solution of tert-butyl(chloro)dimethylsilane (3.44 g, 22.83 mmol) in THF (40 mL). The resulting mixture is further stirred 1 h at 0°C and 1 h at RT, then poured into an aqueous solution of NaHCO₃ (5%) (150 mL) and extracted 3 times with Et₂O. The combined organic extracts are dried over Na₂SO₄, filtered and concentrated. The crude material is purified by flash chromatography on silica gel (eluent: CH₂Cl₂/MeOH 99/1 to 93/7). TLC, R_{f} (CH₂Cl₂/MeOH 95/5) = 0.33. MS (LC-MS): 346.2 [M+H]⁺.

### F. (3S*,4S*)-3,4-Bis-hydroxymethyl-pyrrolidine-1-carboxylic acid tert-butyl ester

To a ice-cooled solution of (3S*,4S*)-pyrrolidine-1,3,4-tricarboxylic acid 1-tert-butyl ester 3,4-diethyl ester (36 g, 114.15 mmol) in THF (1 L), is added dropwise a solution of LiBH₄ (228.3 mmol) in THF (250 mL). The reaction mixture is stirred overnight at room temperature and quenched with an aqueous solution of NaOH 2N (400 mL). Ether is added, the layers are separated and the aqueous one back extracted twice with ether. The combined organic extracts are dried over Na₂SO₄, filtered and concentrated to give the title compound which is used without further purification in the next step. TLC, R_{f} (CH₂Cl₂/MeOH 95/5) = 0.14. MS (LC-MS): 232.2 [M+H]⁺; t_{R} (HPLC, C18 column, 5-100% CH₃CN/H₂O/6 min, 100% CH₃CN/2 min, CH₃CN and H₂O containing 0.1% TFA, flow: 1 mL/min): 3.37 min.

### (3S,4S)-3,4-Bis-hydroxymethyl-pyrrolidine-1-carboxylic acid tert-butyl ester and (3R,4R)-3,4-Bis-hydroxymethyl-pyrrolidine-1-carboxylic acid tert-butyl ester

The two enantiomers are separated via chiral preparative HPLC using SMB "UOP SORBEX Prep." Technology with 16 columns "Princeton Chromatography Inc." (7.5 x 2.12 cm), stationary phase: Chiralpak AD Prep. 20 µm, (eluent: hexane/EtOH/MeOH 90/5/5) to give: (3S,4S)-3,4-bis-hydroxymethyl-pyrrolidine-1-carboxylic acid tert-butyl ester: t_{R} (Chiralpak AD-H, 250 x 4.6 mm, flow rate 1 mL /min) (eluent: hexane/EtOH: 90/10): 6.4 min. [α]_{D} = -11.1°(c = 1.795, CHCl₃); and (3R,4R)-3,4-bis-hydroxymethyl-pyrrolidine-1-carboxylic acid tert-butyl ester: t_{R} (Chiralpak AD-H, 250 x 4.6 mm, flow rate 1 mL /min) (eluent: hexane/EtOH: 90/10): 8.58 min. [α]_{D} = +10.2°(c = 1.795, CHCl₃).

### G. (3S*,4S*)-Pyrrolidine-1,3,4-tricarboxylic acid 1-tert-butyl ester 3,4-diethyl ester

A mixture of (3S*,4S*)-1-benzyl-pyrrolidine-3,4-dicarboxylic acid diethyl ester (82.8 g, 271.14 mmol), di-tert-butylcarbonate (88.76 g, 406.71 mmol) and Pd/C 10% (8 g) in EtOH (1.5 L) is stirred under hydrogen atmosphere. The crude material is filtered over a pad of Celite and concentrated. The crude material is purified by flash chromatography on silica gel (eluent: CH₂Cl₂/acetone 100/0 to 95/5). TLC, R_{f} (CH₂Cl₂/acetone 95/5) =0.51. MS (LC-MS): 216.2 [M+H-Boc]⁺.

### H. (3S*,4S*)-1-Benzyl-pyrrolidine-3,4-dicarboxylic acid diethyl ester

A mixture of N-benzylglycine (51.3 g, 310.55 mmol), diethylfumarate (51.85 mL, 316.76 mmol) and paraformaldehyde powder (10.25 g, 341.6 mmol) in toluene (500 mL) is heated at reflux for 2 h, while collecting water using a dean-stark apparatus. The solvent is concentrated and the mixture purified by distillation under vacuum (- 50 mbar), the desired title compound distilling at 80-85°C. TLC, R_{f} (CH₂Cl₂/Acetone 95/5) =0.56. MS (LC-MS)_{:} 306.2 [M+H]⁺; t_{R} (HPLC, RP8 column, 10-100% CH₃CN/H₂O/5 min, 100% CH₃CN/2.5min, CH₃CN and H₂O containing 0.1% TFA, flow: 1.5 mL/min): 4.35 min.

### Example of formulation 1: Soft Capsules

5000 soft gelatin capsules, each comprising as active ingredient 0.05 g of any one of the compounds of formula I mentioned in any one of the preceding Examples, are prepared as follows:

| | | |
|---|---|---|
| 1. | Composition | |
| | Active ingredient | 250 g |
| | Lauroglycol | 2 liters |

Preparation process: The pulverized active ingredient is suspended in Lauroglykol^{®} (propylene glycol laurate, Gattefossé S.A., Saint Priest, France) and ground in a wet pulverizer to produce a particle size of about 1 to 3 µm. 0.419 g portions of the mixture are then introduced into soft gelatin capsules using a capsule-filling machine.

Example of formulation 2: Tablets comprising compounds of the formula I

Tablets, comprising, as active ingredient, 100 mg of any one of the compounds of formula I in any one of the preceding Examples are prepared with the following composition, following standard procedures:

| | | |
|---|---|---|
| Composition | | |
| Active Ingredient | | 100 mg |
| crystalline lactose | 240 mg | |
| Avicel | 80 mg | |
| PVPPXL | | 20 mg |
| Aerosil | 2 mg | |
| magnesium stearate | 5 mg | |
| | | 447 mg |

Manufacture: The active ingredient is mixed with the carrier materials and compressed by means of a tabletting machine (Korsch EKO, stamp diameter 10 mm).

Avicel® is microcrystalline cellulose (FMC, Philadelphia, USA). PVPPXL is polyvinyl-polypyrrolidone, cross-linked (BASF, Germany). Aerosil® is silicon dioxide (Degussa, Germany).

## Claims

1. A compound of the formula I 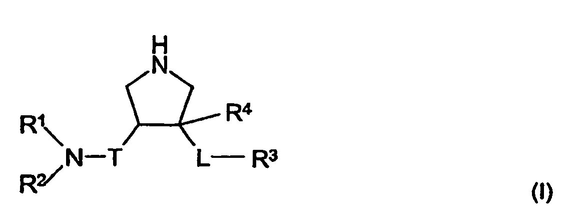 wherein
R¹ is unsubstituted or substituted aryl, unsubstituted or substituted mono- or bicyclic heterocyclyl, unsubstituted or substituted cycloalkyl, unsubstituted or substituted aryl-alkyl, unsubstituted or substituted mono- or bicyclic heterocyclyl-alkyl, unsubstituted or substituted cycloalkyl-alkyl, or acyl;
R² is unsubstituted or substituted alkyl, unsubstituted or substituted aryl, unsubstituted or substituted mono- or bicyclic heterocyclyl, unsubstituted or substituted cycloalkyl, unsubstituted or substituted aryl-alkyl, unsubstituted or substituted mono- or bicyclic heterocyclyl-alkyl or unsubstituted or substituted cycloalkyl-alkyl, with the proviso that if L is methylene (-CH₂-), oxy (-O-), thio (-S-) or unsubstituted (-NH-)or substituted imino, R² is selected from one of the mentioned groups and from hydrogen;
R³ is unsubstituted or substituted alkyl, substituted or unsubstituted aryl, unsubstituted or substituted heterocyclyl, unsubstituted or substituted cycloalkyl, unsubstituted or substituted aryl-alkyl, unsubstituted or substituted heterocyclyl-alkyl, unsubstituted or substituted cycloalkyl-alkyl, , or, if L is oxy, thio or unsubstituted or substituted imino, has one of the meanings just mentioned or is unsubstituted or substituted alkylcarbonyl, unsubstituted or substituted arylcarbonyl, unsubstituted or substituted heterocyclylcarbonyl, unsubstituted or substituted cycloalkylcarbonyl, etherified carboxy, carbamoyl, N-mono- or N,N-di-substituted amino-carbonyl, substituted or unsubstituted alkylsulfonyl, substituted or unsubstituted arylsulfonyl, substituted or unsubstituted heterocyclylsulfonyl or substituted or unsubstituted cycloalkylsulfonyl, sulfamoyl or N-mono-or N,N-di-substituted amino-sulfonyl;
R⁴ is hydrogen or hydroxy;
L is a bond, methylene (-CH₂-), oxy (-O-), thio (-S-) or unsubstituted (-NH-)or substituted imino, with the proviso that if L is a bond then R³ is one of the moieties mentioned for R³ other than substituted alkyl;
or R³ and R⁴ which then is -0- together with L which then is methylene and the carbon to which R³-L- and R⁴ are bound form a substituted or unsubstituted ring annealed to an unsubstituted or substituted aryl, unsubstituted or substituted heterocyclyl or unsubstituted or substituted cycloalkyl, thus forming a spiro compound of the formula I, or
R³ and R⁴ together with L form oxo (=O), thioxo (=S) or unsubstituted or substituted imino (=NH);
and
T is methylene or methylene monosubstituted by alkyl, carbonyl (-C(=O)-) or thiocarbonyl (-C(=S)-);
where in each case of occurrence above in this claim
unsubstituted or substituted aryl is mono- or polycyclic, with 6 to 22 carbon atoms, and is unsubstituted or substituted by one or more moieties, independently selected from the group consisting of
a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇-alkoxy, C₁-C₇-alkanoyloxy, mono- or di-(C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl;
from C₂-C₇-alkenyl, C₂-C₇-alkinyl, phenyl, naphtyl, cycloalkyl heterocyclyl, phenyl- or naphthyl- or heterocyclyl-C₁-C₇-alkyl wherein heterocyclyl is as defined below halo-C₁-C₇-alkyl, phenyloxy- or naphthyloxy-C₁-C₇-alkyl, cycloalkyl-C₁-C₇-alkyl, heterocyclyl-C₁-C₇-alkyl, phenyl-C₁-C₇-alkoxy- or naphthyl-C₁-C₇-alkoxy-C₁-C₇-alkyl cycloalkyl-C₁-C₇-alkoxy-C₁-C₇-alkyl, heterocyclyl-C₁-C₇-alkoxy-C₁-C₇-alkyl, di-(naphthyl- or phenyl)-amino-C₁-C₇-alkyl mono- or di-(heterocyclyl-, cycloalkyl-, naphthyl- or phenyl)-amino-C₁-C₇-alkyl, di-(naphthyl- or phenyl-C₁-C₇-alkyl)-amino-C₁-C₇-alkyl, mono- or di-(heterocyclyl-, cycloalkyl-, naphthyl- or phenyl-C₁-C₇-alkyl)-amino-C₁-C₇-alkyl, benzoyl- or naphthoylamino-C₁-C₇-alkyl, cycloalkyl-COamino-C₁-C₇-alkyl, heterocyclyl-COamino-C₁-C₇-alkyl, phenyl- or naphthylsulfonylamino-C₁-C₇-alkyl wherein phenyl or naphthyl is unsubstituted or substituted by one or more C₁-C₇-alkyl moieties, cycloalkylsulfonylamino-C₁-C₇-alkyl, heterocyclylsulfonylamino-C₁-C₇-alkyl, phenyl- or naphthyl-C₁-C₇-alkylsulfonylamino-C₁-C₇-alkyl, cycloalkyl-C₁-C₇-alkylsulfonylamino-C₁-C₇-alkyl, heterocyclyl-C₁-C₇-alkylsulfonylamino-C₁-C₇-alkyl, carboxy-C₁-C₇-alkyl, halo, hydroxy, phenyl-C₁-C₇-alkoxy wherein phenyl is unsubstituted or substituted by C₁-C₇-alkoxy and/or halo, halo-C₁-C₇-alkoxy, cycloalkyl-C₁-C₇-alkoxy, heterocyclyl-C₁-C₇-alkoxy, phenyl- or naphthyloxy, cycloalkyloxy, heterocyclyloxy, phenyl- or naphthyl-C₁-C₇-alkyloxy, cycloalkyl-C₁-C₇-alkyloxy, heterocyclyl-C₁-C₇-alkyloxy, benzoyl- or naphthoyloxy, halo-C₁-C₇-alkylthio, phenyl- or naphthylthio, cycloalkylthio, heterocyclylthio, phenyl- or naphthyl-C₁-C₇-alkylthio, cycloalkyl-C₁-C₇-alkylthio, heterocyclyl-C₁-C₇-alkylthio, benzoyl- or naphthoylthio, nitro, amino, mono- or di-(naphthyl- or phenyl-C₁-C₇-alkyl)-amino, mono- or di-(heterocyclyl-, cycloalkyl-, naphthyl- or phenyl-C₁-C₇-alkyl)-amino, benzoyl- or naphthoylamino, phenyl- or naphthylsulfonylamino wherein phenyl or naphthyl is unsubstituted or substituted by one or more, especially one to three, C₁-C₇-alkyl moieties, cycloalkylsulfonylamino, heterocyclylsulfonylamino, phenyl- or naphthyl-C₁-C₇-alkylsulfonylamino, cycloalkyl-C₁-C₇-alkylsulfonylamino, heterocyclyl-C₁-C₇-alkylsulfonylamino, carboxyl, C₁-C₇-alkyl-carbonyl, halo-C₁-C₇-alkylcarbonyl, hydroxy-C₁-C₇-alkylcarbonyl, C₁-C₇-alkoxy-C₁-C₇-alkylcarbonyl, amino-C₁-C₇-alkylcarbonyl, (N-) mono- or (N,N-) di-(C₁-C₇-alkyl)-amino-C₁-C₇-alkylcarbonyl, C₁-C₇-alkanoylamino-C₁-C₇-alkylcarbonyl, N-mono or (N,N-) di-(C₁-C₇-alkyl)-amino-C₁-C₇-alkoxycarbonyl, halo-C₁-C₇-alkoxycarbonyl, phenyl- or naphthyloxycarbonyl, phenyl- or naphthyl-C₁-C₇-alkoxycarbonyl, N-mono or (N,N-) di-(C₁-C₇-alkyl)-amino-C₁-C₇-alkoxycarbonyl, carbamoyl, N-mono or N,N-di-(heterocyclyl-, cycloalkyl-, naphthyl- or - phenyl-)-aminocarbonyl, N-mono- or N,N-di-(heterocyclyl-, cycloalkyl-, naphthyl- or phenyl-C₁-C₇-alkyl)-aminocarbonyl, cyano, C₁-C₇-alkylene which is unsubstituted or substituted by up to four C₁-C₇-alkyl substituents and bound to two adjacent ring atoms of the aryl moiety, C₂-C₇-alkenylene or -alkinylene which are bound to two adjacent ring atoms of the aryl moiety, sulfenyl, sulfinyl, C₁-C₇-alkylsulfinyl, phenyl- or naphthylsulfinyl wherein phenyl or naphthyl is unsubstituted or substituted by one or more, especially one to three, C₁-C₇-alkyl moieties, cycloalkylsulfinyl, heterocyclylsulfinyl, phenyl- or naphthyl-C₁-C₇-alkylsulfinyl, cycloalkyl-C₁-C₇-alkylsulfinyl, heterocyclyl-C₁-C₇-alkylsulfinyl, sulfonyl, C₁-C₇-alkylsulfonyl, halo-C₁-C₇-alkylsulfonyl, hydroxy-C₁-C₇-alkylsulfonyl, C₁-C₇-alkoxy-C₁-C₇-alkylsulfonyl, amino-C₁-C₇-alkylsulfonyl, N-mono or (N,N-) di-(C₁-C₇-alkyl)-amino-C₁-C₇-alkylsulfonyl, C₁-C₇-alkanoylamino-C₁-C₇-alkylsulfonyl, phenyl- or naphthylsulfonyl wherein phenyl or naphthyl is unsubstituted or substituted by one or more C₁-C₇-alkyl moieties, cycloalkylsulfonyl, heterocyclylsulfonyl, phenyl- or naphthyl-C₁-C₇-alkylsulfonyl, cycloalkyl-C₁-C₇-alkylsulfonyl, heterocyclyl-C₁-C₇-alkylsuloinyl, sulfamoyl and N-mono or N,N-di-(C₁-C₇-alkyl, phenyl-, naphthyl, heterocyclyl, cycloalkyl, phenyl-C₁-C₇-alkyl and/or naphthyl-C₁-C₇-alkyl, heterocyclyl-C₁-C₇-alkyl, cycloalkyl-C₁-C₇-alkyl)-aminosulfonyl;
unsubstituted or substituted heterocyclyl is a mono- or bicyclic or if not part of a substituent R¹ or R² or if not a substituent R¹ and R² further polycyclic, preferably a mono- or bicyclic or, if not part of a substituent R¹ or R² or if not a substituent R¹ and R², mono-, bi- or further tricyclic-, unsaturated, partially saturated or saturated ring system with 3 to 14 ring atoms and with one or more heteroatoms independently selected from nitrogen (=N-, -NH- or substituted -NH-), oxygen, sulfur (-S-, S(=O)- or S-(=O)₂-) which is unsubstituted or substituted by one or more substitutents independently selected from the subsitutents mentioned above for aryl and from oxo, optionally selected from: 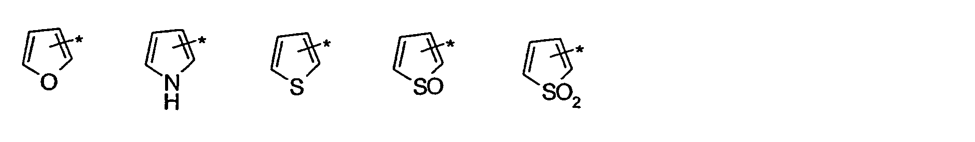 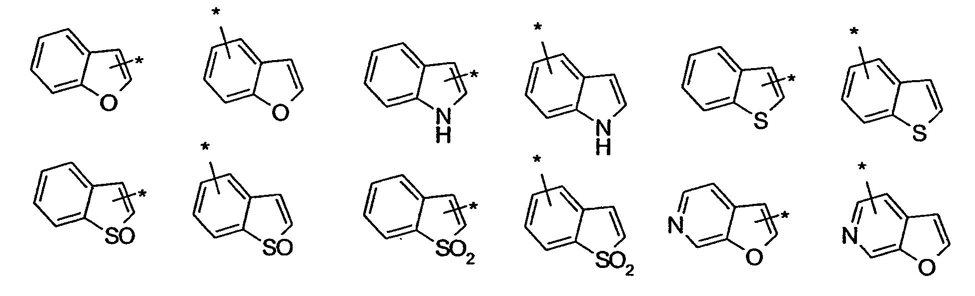 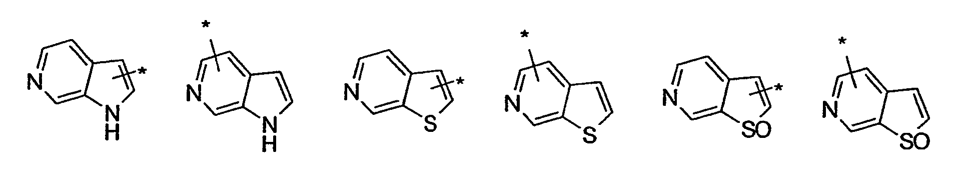 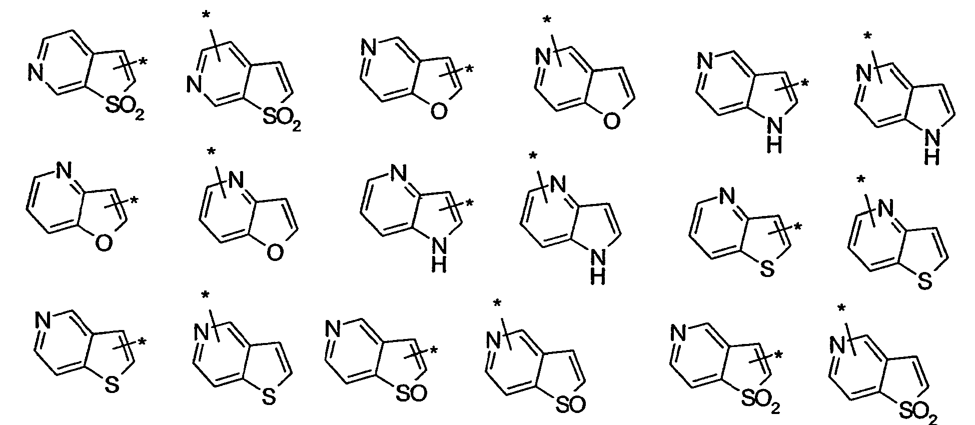 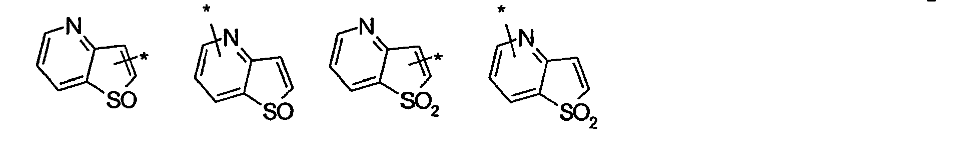 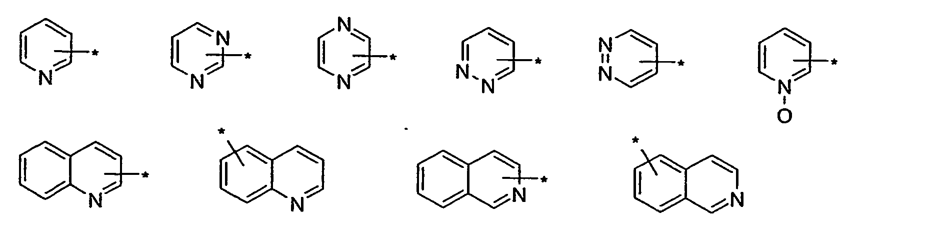 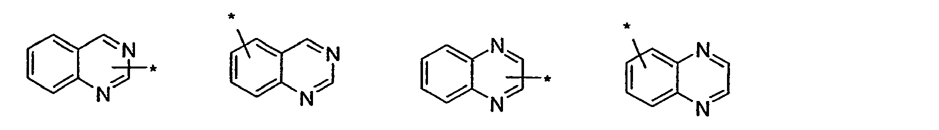 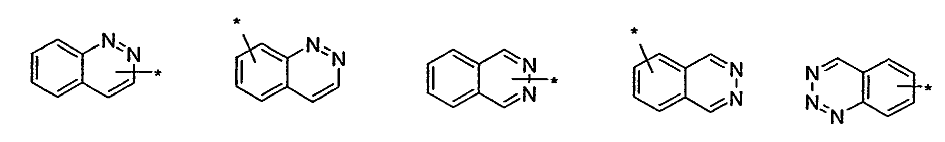 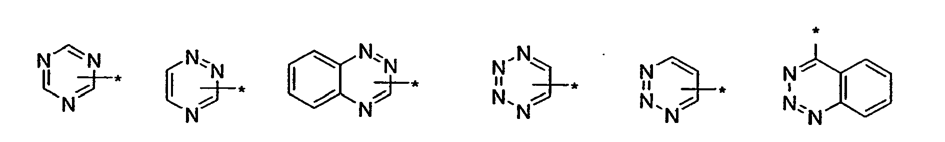 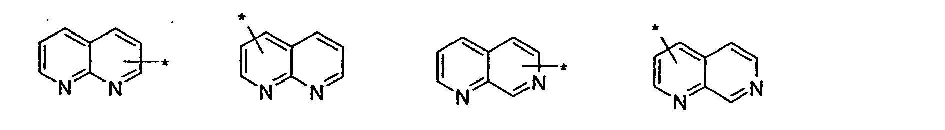 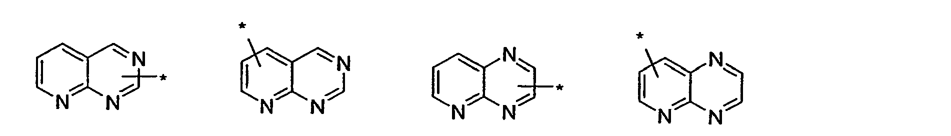 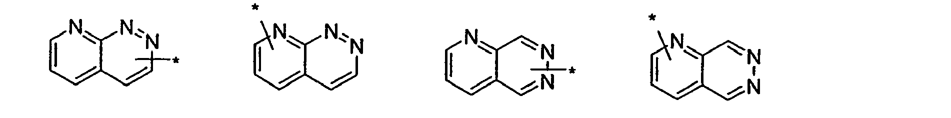 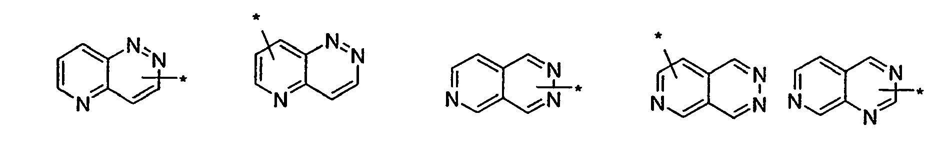 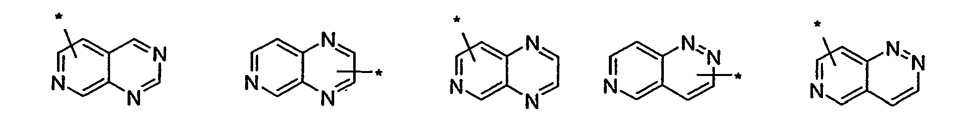 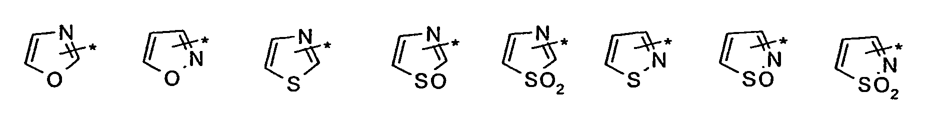 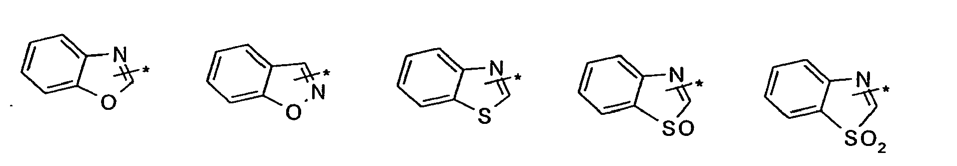 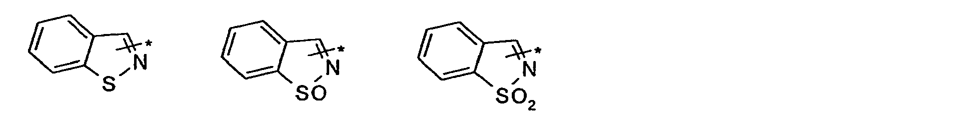 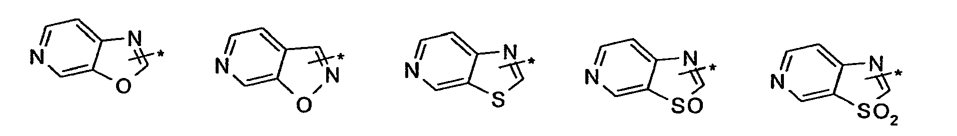 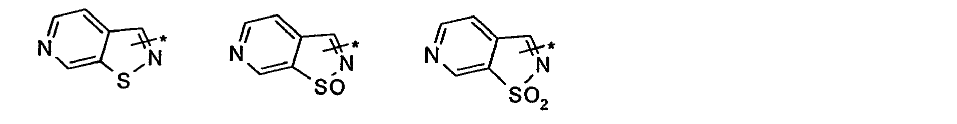 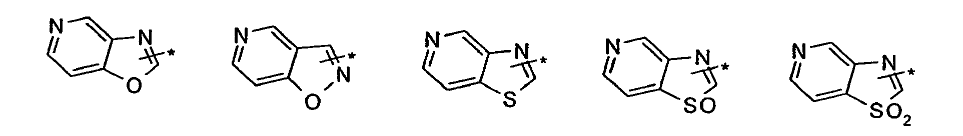 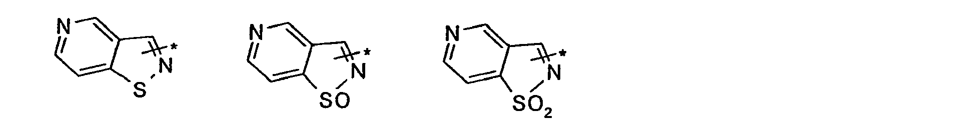 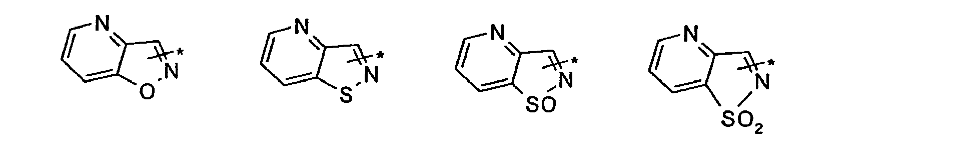 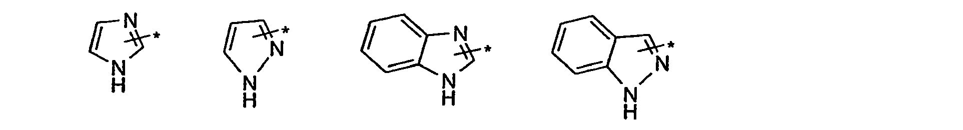 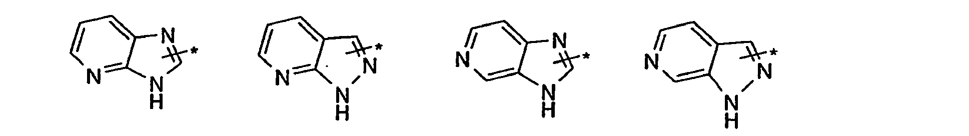 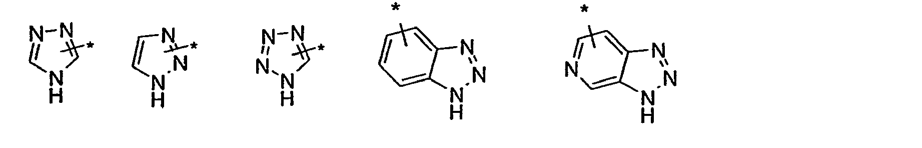 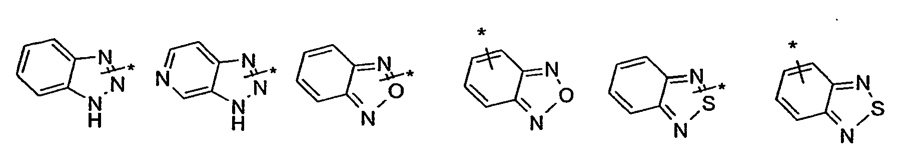 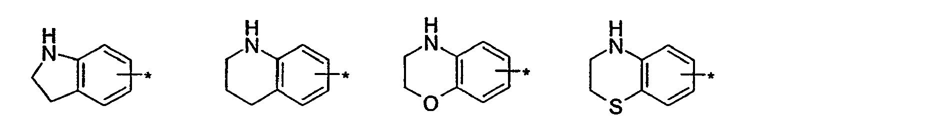 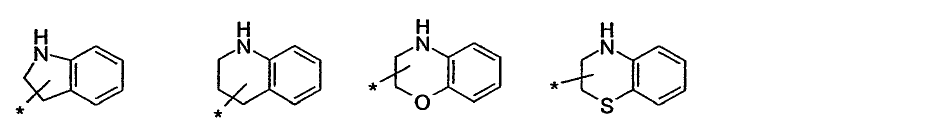 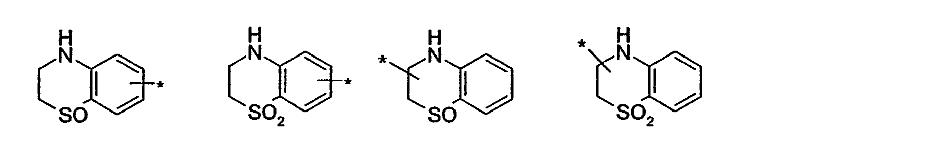 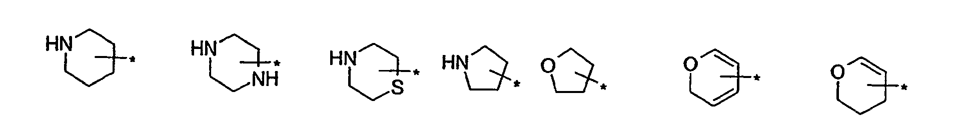 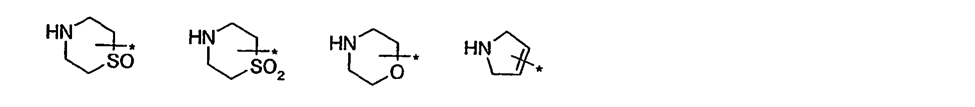 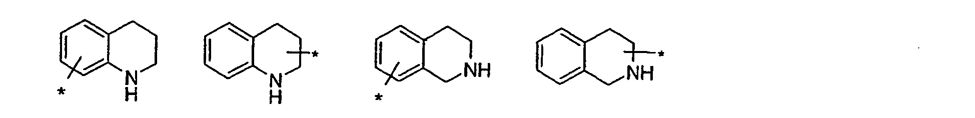 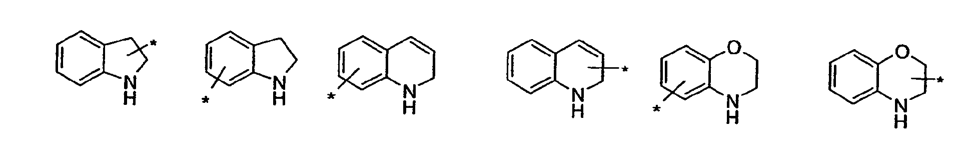 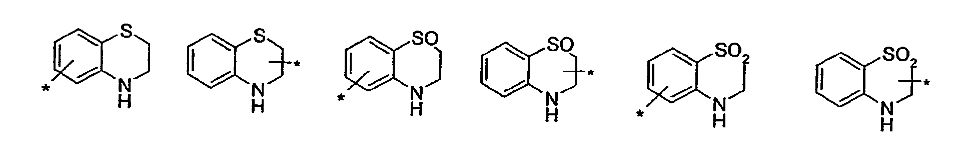 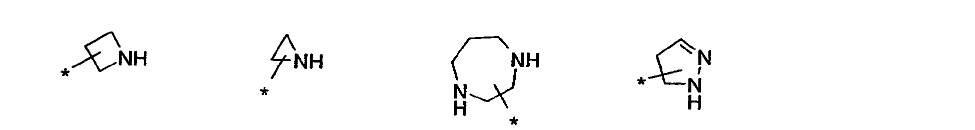 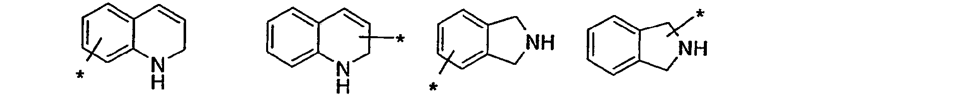 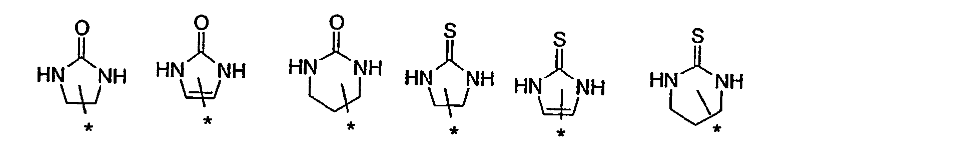 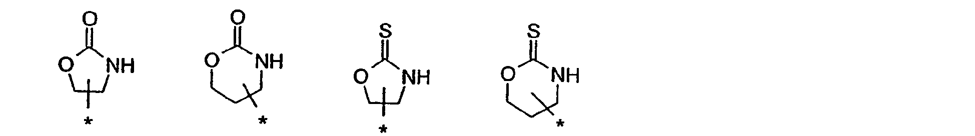 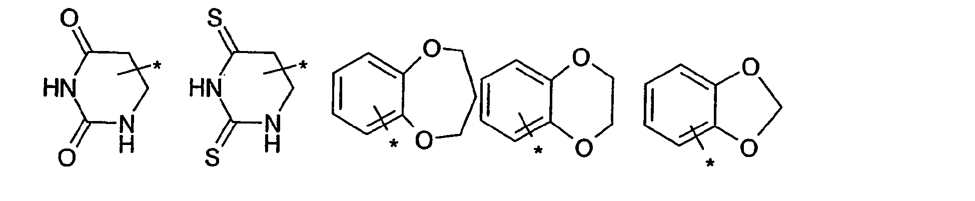 or in the case of where heterocyclyl is present in R₃ defined as unsubstituted or substituted heterocyclyl, unsubstituted or substituted heterocyclyl-alkyl or substituted or unsubstituted heterocyclylsulfonyl in addition selected from 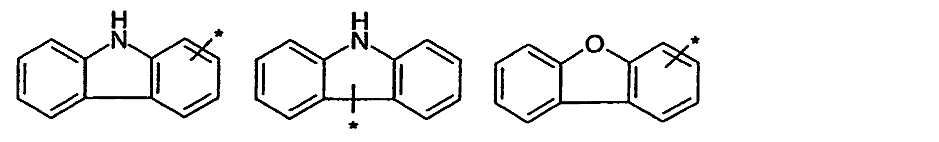 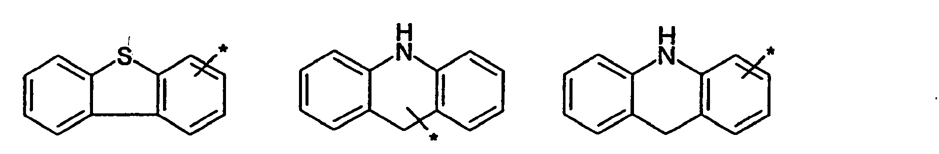 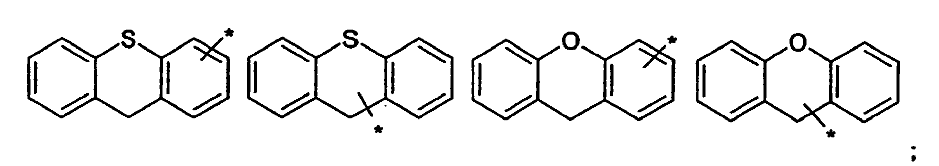 where in each case where an NH is present the bond with the asterisk connecting the respective heterocyclyl moiety to the rest of the molecule the H may be replaced with said bond and/or the H may be replaced by a substituent as defined above,
unsubstituted or substituted cycloalkyl is mono- or polycyclic C₃-C₁₀-cycloalkyl which may include one or more double (e.g. in cycloalkenyl) and/or triple bonds (e.g. in cycloalkinyl), and is unsubstituted or substituted by one or more substitutents independently selected from those mentioned above as substituents for aryl.
in unsubstituted or substituted aryl-alkyl, aryl, which is unsubstituted or substituted by one or more substituents independently selected from those mentioned above as substituents for aryl, is as described above for aryl and is bound to alkyl, either terminally or at any other carbon in the alkyl chain;
in unsubstituted or substituted heterocyclyl-alkyl, heterocyclyl is as described above and is unsubstituted or substituted by one or more substitutents independently selected from those mentioned above for substituted aryl, and heterocyclyl is bound to alkyl, either terminally or at any other carbon in the alkyl chain;
in unsubstituted or substituted cycloalkyl-alkyl, cycloalkyl is as described above and is unsubstituted or substituted by one or more substitutents independently selected from those mentioned above for substituted aryl, and cycloalkyl is bound to alkyl, either terminally or at any other carbon in the alkyl chain;
acyl is unsubstituted or substituted aryl-carbonyl or -sulfonyl, unsubstituted or substituted heterocyclylcarbonyl or -sulfonyl, unsubstituted or substituted cycloalkylcarbonyl or -sulfonyl, formyl or unsubstituted or substituted alkylcarbonyl or -sulfonyl, wherein unsubstituted or substituted aryl, unsubstituted or substituted heterocyclyl and unsubstituted or substituted cycloalkyl are as defined above and unsubstituted or substituted alkyl is as described below;
unsubstituted or substituted alkyl is C₁-C₇-alkyl, that is straight-chained or branched, which is unsubstituted or substituted by one or more moieties selected from unsubstituted or substituted aryl as described above; unsubstituted or substituted heterocycyclyl as described above; unsubstituted or substituted cycloalkyl as described above; C₂-C₇-alkenyl, C₂-C₇-alkinyl, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, benzoyl- or naphthoyloxy, C₁-C₇-alkylthio, halo-C₁-C₇-alkthio, such as trifluoromethylthio, hydroxy-C₁-C₇-alkylthio, C₁-C₇-alkoxy-C₁-C₇-alkylthio, phenyl- or naphthylthio, phenyl- or naphthyl-C₁-C₇-alkylthio, C₁-C₇-alkanoylthio, benzoyl- or naphthoylthio, nitro, amino, mono- or di-(C₁-C₇-alkyl, hydroxy-C₁-C₇-alkyl and/or C₁-C₇-alkoxy-C₁-C₇-alky)-amino, mono- or di-(naphthyl- or phenyl-C₁-C₇-alkyl)-amino, C₁-C₇-alkanoylamino, benzoyl- or naphthoylamino, C₁-C₇-alkylsulfonylamino, phenyl- or naphthylsulfonylamino wherein phenyl or naphthyl is unsubstituted or substituted by one or more C₁-C₇-alkyl moieties, phenyl- or naphthyl-C₁-C₇-alkylsulfonylamino, carboxyl, C₁-C₇-alkyl-arbonyl, C₁-C₇-alkoxy-carbonyl, phenyl- or naphthyloxycarbonyl, phenyl- or naphthyl-C₁-C₇-alkoxycarbonyl, carbamoyl, N- mono- or N,N-di-(C₁-C₇-alkyl)-aminocarbonyl, N-mono- or N,N-di-(naphthyl- or phenyl-C₁-C₇-alkyl)-aminocarbonyl, N-mono- or N,N-di-(alkyl, naphtyl, phenyl, heterocyclyl, cycloalkyl, naphthyl-, heterocyclyclyl-, cycloalkyl- or phenyl-C₁-C₇-alkyl)-aminocarbonyl, cyano, C₁-C₇-alkenylene or -alkinylene, C₁-C₇-alkylenedioxy, sulfenyl, sulfinyl, C₁-C₇-alkylsulfinyl, phenyl- or naphthylsulfinyl wherein phenyl or naphthyl is unsubstituted or substituted by one or more C₁-C₇-alkyl moieties, cycloalkylsulfinyl, heterocyclylsulfinyl, phenyl- or naphthyl-C₁-C₇-alkylsulfinyl, cycloalkyl -C₁-C₇-alkylsulfinyl, heterocyclyl -C₁-C₇-alkylsulfinyl, sulfonyl, C₁-C₇-alkylsulfonyl, phenyl- or naphthylsulfonyl wherein phenyl or naphthyl is unsubstituted or substituted by one or more C₁-C₇-alkyl moieties, cycloalkylsulfonyl, heterocyclylsulfonyl, phenyl- or naphthyl-C₁-C₇-alkylsulfonyl, cycloalkyl - C₁-C₇-alkylsulfonyl, heterocyclyl -C₁-C₇-alkylsulfonyl, sulfamoyl, N-mono- or N,N-di-(alkyl, naphtyl, phenyl, heterocyclyl, cycloalkyl, naphthyl-, heterocyclyclyl-, cycloalkyl- or phenyl-C₁-C₇-alkyl)-aminosulfonyl, N-mono-, N'-mono-, N,N-di- or N,N,N'-tri-(C₁-C₇-alkyl, hydroxy-C₁-C₇-alkyl and/or C₁-C₇-alkoxy-C₁-C₇-alkyl)-aminocarbonylamino and N-mono-, N'-mono-, N,N-di- or N,N,N'-tri-(C₁-C₇-alkyl, hydroxy-C₁-C₇-alkyl and/or C₁-C₇-alkoxy-C₁-C₇-alkyl) aminosulfonylamino;in substituted or unsubstituted alkylsulfonyl, substituted or unsubstituted alkyl is as defined above for unsubstituted or substituted alkyl;
in substituted or unsubstituted arylsulfonyl, substituted or unsubstituted aryl is as defined above for unsubstituted or substituted aryl;
in substituted or unsubstituted heterocyclylsulfonyl, substituted or unsubstituted heterocyclyl is as defined above for unsubstituted or substituted heterocyclyl;
in substituted or unsubstituted cycloalkylsulfonyl, unsubstituted or substituted cycloalkyl is as defined above for unsubstituted or substituted cycloalkyl;
when R³ and R⁴ which then is -0- together with L which then is methylene and the carbon to which R³-L- and R⁴ are bound form a substituted or unsubstituted ring (with one or more substituents independently selected from those mentioned above for aryl, preferably without substituent) annealed to an unsubstituted or substituted aryl, unsubstituted or substituted heterocyclyl or unsubstituted or substituted cycloalkyl, each of which is as defined above, thus forming a spiro compound of the formula I;
in unsubstituted or substituted imino as well as where substituted NH-groups are present in heterocycles, the substituents are preferably selected from the group consisting of a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-, -CO-NV-; -NV-SO₂-, -SO₂-NV; -NV-CO-NV-, -NV-CO-O-, -O-CO-NV-, -NV-SO₂-NV-,
C₂-C₇-alkenyl, C₂-C₇-alkinyl, cycloalkyl, phenyl, naphthyl, heterocyclyl, phenyl- or naphthyl-C₁-C₇-alkyl, cycloalkyl-C₁-C₇-alkyl, heterocyclyl-C₁-C₇-alkyl wherein heterocyclyl is as defined below halo-C₁-C₇-alkyl, phenyloxy- or naphthyloxy-C₁-C₇-alkyl, cycloalkyloxy-C₁-C₇-alkyl, heterocyclyloxy-C₁-C₇-alkyl, phenyl-C₁-C₇-alkoxy- or naphthyl-C₁-C₇-alkoxy-C₁-C₇-alkyl, cycloalkyl-C₁-C₇-alkoxy-C₁-C₇-alkyl, heterocyclyl-C₁-C₇-alkoxy-C₁-C₇-alkyl, di-(cycloalkyl, heterocyclyl, naphthyl or phenyl)-amino-C₁-C₁-alkyl, di-(cycloalkyl-, heterocyclyl-, naphthyl- or phenyl-C₁-C₇-alkyl)-amino-C₁-C₇-alkyl, benzoyl- or naphthoylamino-C₁-C₇-alkyl, , phenyl- or naphthylsulfonylamino-C₁-C₇-alkyl wherein phenyl or naphthyl is unsubstituted or substituted by one or more C₁-C₇-alkyl moieties, cycloalkylsulfonylamino-C₁-C₇-alkyl heterocyclylsulfonylamino-C₁-C₇-alkyl, phenyl- or naphthyl-C₁-C₇-alkylsulfonylamino-C₁-C₇-alkyl, cycloalkyl -C₁-C₇-alkylsulfonylamino-C₁-C₇-alkyl, heterocyclyl-C₁-C₇-alkylsulfonylamino-C₁-C₇-alkyl, C₁-C₇-alkyl-carbonyl, halo-C₁-C₇-alkylcarbonyl, hydroxy-C₁-C₇-alkylcarbonyl, C₁-C₇-alkoxy-C₁-C₇-alkylcarbonyl, amino-C₁-C₇-alkylcarbonyl, (N-) mono- or (N,N-) di-(C₁-C₇-alkyl)-amino-C₁-C₇-alkylcarbonyl, C₁-C₇-alkanoylamino-C₁-C₇-alkylcarbonyl, (N,N-) di-(C₁-C₇-alkyl)-amino-C₁-C₇-alkoxycarbonyl, halo-C₁-C₇-alkoxycarbonyl, phenyl- or naphthyloxycarbonyl, cycloalkyloxycarbonyl, heterocyclyloxycarbonyl, phenyl- or naphthyl-C₁-C₇-alkoxycarbonyl, cycloalkyl C₁-C₇-alkoxycarbonyl, heterocyclyl C₁-C₇-alkoxycarbonyl, C₁-C₇-alkylsulfinyl, phenyl- or naphthylsulfinyl wherein phenyl or naphthyl is unsubstituted or substituted by one or more C₁-C₇-alkyl moieties, cycloalkylsulfinyl, heterocyclylsulfinyl, phenyl- or naphthyl-C₁-C₇-alkylsulfinyl, cycloalkyl -C₁-C₇-alkylsulfinyl , heterocyclyl -C₁-C₇-alkylsulfinyl, sulfonyl, C₁-C₇-alkylsulfonyl, halo-C₁-C₇-alkylsulfonyl, hydroxy-C₁-C₇-alkylsulfonyl, C₁-C₇-alkoxy-C₁-C₇-alkylsulfonyl, amino-C₁-C₇-alkylsulfonyl, (N,N-) di-(C₁-C₇-alkyl)-amino-C₁-C₇-alkylsulfonyl, C₁-C₇-alkanoylamino-C₁-C₇-alkylsulfonyl, phenyl- or naphthylsulfonyl wherein phenyl or naphthyl is unsubstituted or substituted by one or more C₁-C₇-alkyl moieties, cycloalkylsulfonyl, heterocyclyl sulfonyl, phenyl- or naphthyl-C₁-C₇-alkylsulfonyl; cycloalkyl-C₁-C₇-alkylsulfonyl and
in unsubstituted or substituted alkylcarbonyl, unsubstituted or substituted alkyl is as defined above;
in unsubstituted or substituted arylcarbonyl, unsubstituted or substituted heterocyclylcarbonyl and unsubstituted or substituted cycloalkylcarbonyl, the unsubstituted or substituted aryl, heterocyclyl and cycloalkyl moieties, respectively, are preferably as described for the corresponding unsubstituted or substituted aryl, heterocyclyl and cycloalkyl moieties, respectively;
etherified carboxy is carbonyl, bound to L = oxy or especially imino, to which a moiety selected from unsubstituted or substituted alkyloxy, unsubstituted or substituted aryloxy, unsubstituted or substituted heterocyclyloxy or unsubstituted or substituted cycloalkyloxy, in each of which the unsubstituted or substituted alkyl, aryl, heterocyclyl or cycloalkyl moieties are defined as above, is bound as bound group; especially preferred is unsubstituted or substituted alkoxycarbonyl, especially C₁-C₇-alkoxycarbonyl, bound to L = imino;
N-mono- or N,N-di-substituted aminocarbonyl is aminocarbonyl, preferably bound to L = oxy or thio, that is mono- or di-substituted at the nitrogen by one or more moieties selected from unsubstituted or substituted alkyl, unsubstituted or substituted aryl, unsubstituted or substituted heterocyclyl or unsubstituted or substituted cycloalkyl, each of which is defined as above; a preferred example is aryl-C₁-C₇-alkylaminocarbonyl (= aryl-C₁-C₇-NH-C(=O)-), such as benzylaminocarbonyl, bound to L = oxy or further thio; and
N-mono- or N,N-di-substituted aminosulfonyl is sulfamoyl, preferably bound to L = imino or especially oxy, that is mono- or di-substituted at the nitrogen by one or more moieties selected from unsubstituted or substituted alkyl, unsubstituted or substituted aryl, unsubstituted or substituted heterocyclyl or unsubstituted or substituted cycloalkyl, each of which is preferably defined as above; a preferred example is aryl-C₁-C₇-alkylaminosulfonyl (= aryl-C₁-C₇-NH-S(=O)₂-), such as benzylaminosulfonyl, bound to L = oxy or further imino;
or a pharmaceutically acceptable salt thereof.

2. A compound of the formula I according to claim 1, wherein
R¹ is phenyl or naphthyl, each of which is unsubstituted or substituted by one or more substitutents selected from the group consisting of C₁-C₇-alkyl, phenyl, naphthyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C1₁-C₇-alkanoylamino-C₁-C₇-alkyl, C₁-C₇-alkyl-sulfonylamino-C₁-C₇-alkyl, halo, hydroxy, C₁-C₇-alkoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl and/or (phenyl- or naphthyl)-C₁-C₇-alkyl)-carbamoyl, C₁-C₇-alkylsulfonyl, unsubstituted or C₁-C₇-alkyl-substituted phenyl- or naphthylsulfonyl, N-mono- or N,N-di-(C₁-C₇-alkyl and/or (phenyl- or naphthyl)-C₁-C₇-alkyl)-sulfamoyl and cyano;
phenyl- or naphthyl-C₁-C₇-alkyl, wherein each of phenyl or naphthyl is unsubstituted or substituted by one or more substitutents selected from the group consisting of the substitutents just mentioned for substituted phenyl or naphthyl, pyrrolyl, furanyl, thienyl, pyrimidine-2,4-dione-1-, -2-, -3- or -5-yl and benzo[1,3]dioxalyl, each if which is unsubstituted or substituted by one or more substituents independently selected from those mentioned for substituted phenyl or naphthyl R¹ above; pyrrolyl-C₁-C₇-alkyl, furanyl-C₁-C₇-alkyl, thienyl-C₁-C₇-alkyl, pyrimidine-2,4-dione-1-, -2-, -3- or -5-yl-C₁-C₇-alkyl, indolyl-C₁-C₇-alkyl, benzofuranyl-C₁-C₇-alkyl, benzimidazolyl-C₁-C₇-alkyl, benzopyrazolyl-C₁-C₇-alkyl, quinolinyl-C₁-C₇-alkyl, isoquinolyl-C₁-C₇-alkyl or benzo[1,2,5]oxadiazolyl-C₁-C₇-alkyl, each if which is unsubstituted or substituted by one or more substituents independently selected from the substitutents mentioned above for substituted phenyl or naphthyl R¹;
C₃-C₁₀-cycloalkyl which is unsubstituted or substituted by one or more substituents independently selected from the substitutents mentioned above for substituted phenyl or naphthyl R¹;
C₃-C₁₀-cycloalkyl-C₁-C₇-alkyl wherein cycloalkyl is unsubstituted or substituted by one or more substituents independently selected from the substitutents mentioned above for substituted phenyl or naphthyl R¹;
phenyl- or naphthyl-carbonyl or phenyl- or naphthyl-C₁-C₇-alkylcarbonyl, wherein each phenyl or naphthyl is unsubstituted or substituted by one or more substitutents selected from the group consisting of
a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇alkanoylamino-C₁-C₇alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, phenyl- or naphthyl-C₁-C₇-alkoxycarbonyl, halo-C₁-C₇₋alkoxycarbonyl, C₁-C₇-alkylsulfonyl, carbamoyl and cyano;
heterocyclylcarbonyl such as each if which is unsubstituted or substituted by one or more substituents independently selected from a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -0-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, hydroxy-C₁-C₇-alkyl, C₁-C₇-alkoxy-C₁-C₇-alkyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl; C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, phenyl- or naphthyl-C₁-C₇-alkoxycarbonyl, halo-C₁-C₇-alkoxycarbonyl, C₁-C₇-alkylsulfonyl, carbamoyl and cyano;
or phenyl- or naphthyl-sulfonyl, wherein each phenyl or naphthyl is unsubstituted or substituted by one or more substitutents selected from those mentioned above for substituted phenyl or naphthyl R¹;
R² is
C₁-C₇-alkyl that is unsubstituted or substituted by one or more substitutents selected from the group consisting of halo, phenyl- or naphthyl, hydroxy, C₁-C₇-alkoxy, amino, mono- or di-(C₁-C₇-alkyl)-amino, C₁-C₇-alkanoylamino, C₁-C₇-alkyl-sulfonylamino, phenyl- or napthylsulfonylamino, phenyl- or naphthyl-C₁-C₇-alkylsulfonylamino, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl; and cyano;
phenyl or naphthyl, each of which is unsubstituted or substituted by one or more substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkylsulfonylamino-C₁-C₇-alkyl, halo, hydroxy, C₁-C₇-alkoxy, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, carboxyl, C₁-C₇-alkoxy-carbonyl, halo-C₁-C₇-alkoxycarbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl and cyano;
phenyl- or naphthyl-C₁-C₇-alkyl, wherein each of phenyl or naphthyl is unsubstituted or substituted by one or more substitutents selected from the group just mentioned for substituted phenyl or naphthyl R²;
C₃-C₁₀-cycloalkyl which is unsubstituted or substituted by one or more substitutents selected from the group just mentioned for substituted phenyl or naphthyl R²;
C₃-C₁₀-cycloalkyl-C₁-C₇-alkyl wherein cycloalkyl is unsubstituted or substituted by one or more substitutents selected from the group just mentioned for substituted phenyl or naphthyl R²;
or pyrrolyl, furanyl or thienyl,
or, if L is methylene, oxy, thio or imino, R² is selected from one of the groups of moieties R² just mentioned and from hydrogen;
R³ is
C₁-C₇-alkyl carbamoyl-C₁-C₇-alkyl, N-mono- or N,N-di-(C₃-C₈-cycloalkyl-, heterocyclyl-, phenyl-, naphtyl-, C₁-C₇-alkyl-, C₃-C₈-cycloalkyl-C₁-C₇-alkyl, heterocyclyl-C₁-C₇-alkyl, phenyl-C₁-C₇-alkyl- and/or naphthyl-C₁-C₇-alkyl-)aminocarbonyl-C₁-C₇-alkyl; phenyl, naphthyl, phenyl-C₁-C₇-alkyl or naphthyl-C₁-C₇alkyl, wherein each phenyl or naphthyl is unsubstituted or substituted by one or more substitutents selected from the group consisting of phenyl, naphtyl, C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylylamino-C₁-C₇-alkyl, phenyl, naphthyl, mono- or di-(C₁-C₇-alkoxy)-phenyl or -naphthyl, C₁-C₇-alkylendioxy-phenyl where the oxy atoms are bound to adjacent phenyl ring atoms, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, (unsubstituted or mono-, di- or tri-C₁-C₇-alkyl substituted)-phenyl- or -naphthyl-C₁-C₇-alkyloxy, C₁-C₇-alkanoyloxy, nitro, amino, mono- or di-(C₁-C₇-alkyl)-amino, C₁-C₇-alkanoylamino C₁-C₇-alkylsulfonylylamino, carboxyl, C₁-C₇-alkoxy-carbonyl, phenyl- or naphthyl-C₁-C₇-alkoxycarbonyl, carbamoyl and cyano, C₁-C₇-alkylsulfonyl, C₁-C₇-alkylene which is unsubstituted or substituted by up to four C₁-C₇-alkyl substituents and bound to two adjacent ring atoms of the phenyl or naphthyl moiety; pyrrolyl, furanyl and thienyl;
phenyl- or naphthyl-sulfonyl or phenyl-C₁-C₇-alkyl- or naphthyl-C₁-C₇-alkyl-sulfonyl, wherein each phenyl or naphthyl is unsubstituted or substituted by one or more substitutents selected from the group just described for substituted phenyl- or naphthyl R³,
C₃-C₁₀-cycloalkyl or C₃-C₁₀-cyclyoalkyl-C₁-C₇-alkyl, in both of which cycloalkyl is unsubstituted or substituted by one or more of the substitutents just mentioned for substituted phenyl or naphthyl R³;
or heterocyclyl or heterocyclyl-C₁-C₇-alkyl wherein heterocyclyl is selected from pyrrolyl, furanyl, thienyl, pyrimidine-2,4-dione-1-, -3- or -5-yl, indolyl, benzofuranyl, benzimidazolyl, benzopyrazolyl, quinolinyl, isoquinolinyl, methylene-dioxy-phenyl, ethylene-1,2-dioxy-phenyl or trimethylen-1,3-dioxyphenyl wherein the oxy groups are bound to adjacent ring atoms of the phenyl ring, where each of the heterocyclyl moieties is unsubstituted or substituted as mentioned above for substituted phenyl R³;
or, if L is imino, oxy or thio, can alternatively be phenyl- or naphthylcarbonyl, C₁-C₇-alkoxycarbonyl (meaning C₁-C₇-alkyl-O-C(=O)-), phenyloxycarbonyl, naphthyloxycarbonyl, cycloalkyloxycarbonyl, heterocyclyloxycarbonyl, phenyl-C₁-C₇-alkyloxycarbonyl, naphthyl-C₁-C₇-alkyloxycarbonyl, cycloalkyl C₁-C₇-alkoxycarbonyl, heterocyclyl C₁-C₇-alkoxycarbonyl or N-mono- or N,N-di-(alkyl, naphtyl, phenyl, heterocyclyl, cycloalkyl, naphthyl-, heterocyclyclyl-, cycloalkyl- or phenyl-C₁-C₇-alkyl) -aminocarbonyl, where in each case the phenyl or naphthyl rings are unsubstituted or substituted as mentioned above for substituted phenyl or naphthyl R³;
and
R⁴ is hydrogen or hydroxy; and
L is a bond, methylene (-CH₂-), oxy (-O-) or imino (-NH-);
or R³ and R⁴ which then is -O- together with L which then is methylene and the carbon to which R³-L₋ and R⁴ are bound form a substituted or unsubstituted 5-membered ring annealed to benzo where benzo is substituted by one or more substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, halo, hydroxy, C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl and cyano, or unsubstituted, thus forming a spiro compound of the formula I, or
R³ and R⁴ together with L form oxo (=O);
and T is methylene, carbonyl or thiocarbonyl;
or a pharmaceutically acceptable salt thereof.

3. A compound of the formula I according to any one of claims 1 or 2, wherein
R¹ is
phenyl- or naphthyl-carbonyl or phenyl- or naphthyl-C₁-C₇-alkylcarbonyl, wherein each phenyl or naphthyl is unsubstituted or substituted by one or more substitutents selected from the group consisting of
a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below,hydroxy-C_{1-C7-alkyl, C1-C7-}alkoxy-C₁-C₇-alkyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇-alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, halo-C₁-C₇-alkoxycarbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N-mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, halo-C₁-C₇-alkoxycarbonyl, phenyl- or naphthyl-C₁-C₇-alkoxycarbonyl, C₁-C₇-alkylsulfonyl, carbamoyl and cyano;
pyrrolylcarbonyl, furanylcarbonyl, thienylcarbonyl, pyrimidine-2,4-dione-1-, -2-, -3- or -5-yl-carbonyl, indolyl- carbonyl, benzimidazolyl-carbonyl, benzopyrazolyl-carbonyl benzofuranyl-carbonyl, quinolinyl-carbonyl or benzo[1,2,5]oxadiazolyl-carbonyl, each if which is unsubstituted or substituted by one or more substituents independently selected from a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y. if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, hydroxy-C₁-C₇-alkyl, C₁-C₇-alkoxy-C₁-C₇-alkyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or dl-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl;
from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, halo-C₁-C₇-alkoxycarbonyl, phenyl- or naphthyl-C₁-C₇-alkoxycarbonyl, C₁-C₇-alkylsulfonyl, carbamoyl and cyano;
R² is;
C₁-C₇-alkyl that is unsubstituted or substituted by one or more substitutents selected from the group consisting of halo, phenyl- or naphthyl, hydroxy, C₁-C₇-alkoxy, amino, mono- or di-(C₁-C₇-alkyl)-amino, C₁-C₇-alkanoylamino, C₁-C₇-alkyl-sulfonylamino, phenyl- or napthylsulfonylamino, phenyl- or naphthyl-C₁-C₇-alkylsulfonylamino, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, carboxyl, C₁-C₇-alkoxy-carbonyl, phenyl- or naphthyl-C₁-C₇-alkoxycarbonyl, carbamoyl, N-mono- or N.N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl; and cyano; C₃-C₁₀-cycloalkyl which is unsubstituted or substituted by one or more substituents independently selected from the substitutents mentioned above for substituted phenyl or naphthyl R¹, or C₃-C₁₀-cycloalkyl-C₁-C₇-alkyl wherein cycloalkyl is unsubstituted or substituted by one or more, e.g. up to three, substituents independently selected from the substitutents mentioned above for substituted phenyl or naphthyl R¹;
and R³ and R⁴ which then is -O- together with L which then is methylene and the carbon to which R³-L- and R⁴ are bound form a substituted or unsubstituted 5-membered ring annealed to benzo where benzo is substituted by one or more substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, halo, hydroxy, C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl and cyano, or unsubstituted, thus forming a spiro compound of the formula I, or
and T is carbonyl or thiocarbonyl or methylene;
or a pharmaceutically acceptable salt thereof.

4. A compound of the formula I according to any one of claims 1 or 2, wherein
R¹ is
phenyl- or naphthyl-carbonyl or phenyl- or naphthyl-C₁-C₇-alkylcarbonyl, wherein each phenyl or naphthyl is unsubstituted or substituted by one or more substitutents selected from the group consisting of
a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, hydroxy-C₁-C₇-alkyl, C₁-C₇-alkoxy-C₁-C₇-alkyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇-alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
from phenyl- or naphthyl-C₁-C₇-alkyl, halo-C₁-C₇-alkyl, halo, hydroxy, phenyl,- or naphthyloxy, phenyl- or naphthyl-C₁-C₇-alkyloxy, nitro, amino, amino-C₁-C₇-alkyl, carboxyl, halo-C₁-C₇-alkoxycarbonyl, phenyl- or naphthyl-C₁-C₇-alkoxycarbonyl, C₁-C₇-alkylsulfonyl, carbamoyl and cyano;
or pyrrolylcarbonyl, furanylcarbonyl, thienylcarbonyl, pyrimidine-2,4-dione-1-, -2-, -3- or -5-yl-carbonyl, indolyl- carbonyl, benzimidazolyl-carbonyl, benzopyrazolyl-carbonyl benzofuranyl-carbonyl, quinolinyl-carbonyl or benzo[1,2,5]oxadiazolyl-carbonyl, each if which is unsubstituted or substituted by one or more, e.g. up to three, substituents independently selected from a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-dt-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, phenyl- or naphthyl-C₁-C₇-alkoxycarbonyl, halo-C₁-C₇-alkoxycarbonyl, C₁-C₇-alkylsulfonyl, carbamoyl and cyano;
R² is
C₁-C₇-alkyl that is unsubstituted or substituted by one or more substitutents selected from the group consisting of halo, phenyl- or naphthyl, hydroxy, C₁-C₇-alkoxy, amino, mono- or di-(C₁-C₇-alkyl)-amino, C₁-C₇-alkanoylamino, C₁-C₇-alkyl-sulfonylamino, phenyl- or napthylsulfonylamino, phenyl or naphthyl-C₁-C₇-alkylsulfonylamino, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl; and cyano; C₃-C₁₀-cycloalkyl which is unsubstituted or substituted by one or more substituents independently selected from the substitutents mentioned above for substituted phenyl or naphthyl R¹, or C₃-C₁₀-cycloalkyl-C₁-C₇-alkyl wherein cycloalkyl is unsubstituted or substituted by one or more substituents independently selected from the substitutents mentioned above for substituted phenyl or naphthyl R¹;
R³ is phenyl, naphthyl, phenyl-C₁-C₇-alkyl or naphthyl-C₁-C₇alkyl, wherein each phenyl or naphthyl is unsubstituted or substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylylamino-C₁-C₇-alkyl, phenyl, naphthyl, mono- or di-(C₁-C₇-alkoxy)-phenyl or -naphthyl, C₁-C₇-alkylendioxy-phenyl where the oxy atoms are bound to adjacent phenyl ring atoms, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, (unsubstituted or mono-, di- or tri-C₁-C₇-alkyl substituted)-phenyl- or-naphthyl-C₁-C₇-alkyloxy, C₁-C₇-alkanoyloxy, nitro, amino, mono- or di-(C₁-C₇-alkyl)-amino, C₁-C₇-alkanoylamino C₁-C₇-alkylsulfonylylamino, carboxyl, C₁-C₇-alkoxy-carbonyl, phenyl- or naphthyl-C₁-C₇-alkoxycarbonyl, carbamoyl and cyano, C₁-C₇-alkylsulfonyl, C₁-C₇-alkylene which is unsubstituted or substituted by up to four C₁-C₇-alkyl substituents and bound to two adjacent ring atoms of the phenyl or naphthyl moiety; pyrrolyl, furanyl and thienyl;
R⁴ is hydroxy;
L is methylene;
and T is carbonyl, thiocarbonyl or preferably methylene;
or a pharmaceutically acceptable salt thereof.

5. A compound of the formula I according to any one of claims 1 or 2,
wherein
R¹ is phenyl or naphthyl, each of which is unsubstituted or substituted by one or more substitutents selected from the group consisting of C₁-C₇-alkyl, phenyl, naphthyl, phenyl- or naphthyl-C₁-C₇-alkyl, halo-C₁-C₇-alkyl, hydmxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylamino-C₁-C₇-alkyl, halo, hydroxy, C₁-C₇-alkoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoxyloxy, nitro, amino, mono- or di-(C₁-C₇-alkyl)-amino, C₁-C₇ alkanoylamino, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl and/or (phenyl- or naphthyl)-C₁-C₇-alkyl)-carbamoyl, C₁-C₇-alkylsulfonyl, unsubstituted or C₁-C₇-alkyl-substituted phenyl- or naphthylsulfonyl, N-mono- or N,N-di-(C₁-C₇-alkyl and/or (phenyl- or naphthyl)-C₁-C₇-alkyl)-sulfamoyl and cyano;
phenyl- or naphthyl-C₁-C₇-alkyl, wherein each of phenyl or naphthyl is unsubstituted or substituted by one or more substitutents selected from the group consisting of the substitutents just mentioned for substituted phenyl or naphthyl, pyrrolyl, furanyl, thienyl, pyrimidine-2.4-dione-1-, -2-, -3- or -5-yl and benzo[1,3]dioxalyl, each if which is unsubstituted or substituted by one or more substituents independently selected from those mentioned for substituted phenyl or naphthyl R¹ above, pyrrolyl-C₁-C₇-alkyl, furanyl-C₁-C₇-alkyl, thienyl-C₁-C₇-alkyl, pyrimidine-2,4-dione-1-, -2-, -3- or -5-yl-C₁-C₇-alkyl, indolyl-C₁-C₇-alkyl, benzofuranyl-C₁-C₇-alkyl, benzimidazolyl-C₁-C₇-alkyl, benzopyrazolyl-C₁-C₇-alkyl, quinolinyl-C₁-C₇-alkyl, isoquinolyl-C₁-C₇-alkyl or benzo[1,2,5]ox adiazolyl-C₁-C₇-alkyl, each if which is unsubstituted or substituted by one or more, e.g. up to three, substituents independently selected from the substitutents mentioned above for substituted phenyl or naphthyl R¹;
C₃-C₁₀-cycloalkyl which is unsubstituted or substituted by one or more, substituents independently selected from the substitutents mentioned above for substituted phenyl or naphthyl R¹;
C₃-C₁₀-cycloalkyl-C₁-C₇-alkyl wherein cycloalkyl is unsubstituted or substituted by one or more substituents independently selected from the substitutents mentioned above for substituted phenyl or naphthyl R¹;
phenyl- or naphthyl-carbonyl or phenyl- or naphthyl-C₁-C₇-alkylcarbonyl, wherein each phenyl or naphthyl is unsubstituted or substituted by one or more substitutents selected from the group consisting of
a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, hydroxy-C₁-C₇-alkyl, C₁-C₇-alkoxy-C₁-C₇-alkyl, C₁-C₇-alkoxy-C₁-C₇-alkoxy-C₁-C₇-alkyl, C₁-C₇-alkanoyloxy-C₁-C₇-alkyl, amino-C₁-C₇-alkyl, (N-) mono- or (N,N-) di-(C₁-C₇-alkyl)-amino-C₁-C₇-alkyl, C₁-C₇-alkoxy-C₁-C₇-alkylamino-C₁-C₇ alkyl, mono-(naphthyl- or phenylyamino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alky), carboxyl, phenyl- or naphthyl-C₁-C₇-alkoxycarbonyl, halo-C₁-C₇-alkoxycarbonyl, C₁-C₇-alkylsulfonyl, carbamoyl and cyano;
heterocyclylcarbonyl such as pyrrolylcarbonyl, furanylcarbonyl, thienylcarbonyl, pyrimidine-2,4-dione-1-, -2-, -3- or -5-yl-carbonyl, indolyl- carbonyl, 3- methylindolyl-carbonyl, benzimidazolyl-carbonyl, benzopyrazolyl-carbonyl benzofuranyl-carbonyl, quinolinyl-carbonyl or benzo[1,2,5]oxadiazolyl-mrbonyl, each if which is unsubstituted or substituted by one or more, e.g. up to three, substituents independently selected from a substitutent of the formula -(C₀-C₇-alkylene)-(X),-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇-alkoxy-C₁-C₇-alkyl, C₁-C₇-alkanoyloxy C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyt-O-CO-NH-C₁-C₇-alkyl, C₁-C₇-alkylsulfonylaminO-C₁-C₇-alkyl, C₁-C₇-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇-alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl, N-C₁-C₇-alkoxy-C₁-C₇-alkylcarbamoyl or N-mono- or N,N-di-(C₁-C₇-alkyl)-aminosulfonyl; or
from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amlno-C₁-C₇-alkyl, carboxyl, phenyl- or naphthyl-C₁-C₇-alkoxycarbonyl, halo-C₁-C₇-alkoxycarbonyl, C₁-C₇-alkylsulfonyl, carbamoyl and cyano;
or phenyl- or naphthyl-sulfonyl, wherein each phenyl or naphthyl is unsubstituted or substituted by one or more substitutents selected from those mentioned above for substituted phenyl or naphthyl R¹;
R² is
C₁-C₇-alkyl that is unsubstituted or substituted by one or more substitutents selected from the group consisting of halo, phenyl- or naphthyl, hydroxy, C₁-C₇-alkoxy, amino, mono- or di-(C₁-C₇-alkyl)-amino, C₁-C₇-alkanoylamino, C₁-C₇-alkyl-sulfonylamino, phenyl- or napthylsulfonylamino, phenyl- or naphthyl-C₁-C₇-alkylsulfonylamino, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyi; and cyano;
phenyl or naphthyl, each of which is unsubstituted or substituted by one or more substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkylsulfonylamino-C₁-C₇-alkyl, halo, hydroxy, C₁-C₇-alkoxy, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, carboxyl, C₁-C₇-alkoxy-carbonyl, halo-C₁-C₇-alkoxycarbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl and cyano;
phenyl- or naphthyl-C₁-C₇-alkyl, wherein each of phenyl or naphthyl is unsubstituted or substituted by one or more substitutents selected from the group just mentioned for substituted phenyl or naphthyl R²;
C₃-C₁₀-cycloalkyl which is unsubstituted or substituted by one or more substitutents selected from the group just mentioned for substituted phenyl or naphthyl R²;
C₃-C₁₀-cycloalkyl-C₁-C₇-alkyl wherein cycloalkyl is unsubstituted or substituted by one or more, e.g. up to three, substitutents selected from the group just mentioned for substituted phenyl or naphthyl R²;
or pyrrolyl, furanyl or thienyl,
or, if L is methylene, oxy, thio or imino, R² is selected from one of the groups of moieties R² just mentioned and from hydrogen;
R³ is
C₁-C₇-alkyl carbamoyl-C₁-C₇-alkyl, N-mono- or N,N-di-(C₃-C₈-cycloalkyl-, heterocyclyl-, phenyl-, naphtyl-, C₁-C₇-alkyl-, C₃-C₈-cycloalkyl-C₁-C₇-alkyl, heterocyclyl-C₁-C₇-alkyl, phenyl-C₁-C₇-alkyl- and/or naphthyl-C₁-C₇-alkyl-)aminocarbonyl-C₁-C₇-alkyl; phenyl, naphthyl, phenyl-C₁-C₇-alkyl or naphthyl-C₁-C₇-alkyl, wherein each phenyl or naphthyl is unsubstituted or substituted by one or more substitutents selected from the group consisting of phenyl, naphtyl, C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylylamino-C₁-C₇-alkyl, phenyl, naphthyl, mono- or di-(C₁-C₇-alkoxy)-phenyl or -naphthyl, C₁-C₇-alkylendioxy-phenyl where the oxy atoms are bound to adjacent phenyl ring atoms, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, (unsubstituted or mono-, di- or tri-C₁-C₇-alkyl substituted)-phenyl- or -naphthyl-C₁-C₇-alkyloxy, C₁-C₇-alkanoyloxy, nitro, amino, mono- or di-(C₁-C₇-alkyl)-amino, C₁-C₇-alkanoylamino C₁-C₇-alkylsulfonylylamino, carboxyl, C₁-C₇-alkoxy-carbonyl, phenyl- or naphthyl-C₁-C₇-alkoxycarbonyl, carbamoyl and cyano, C₁-C₇-alkylsulfonyl, C₁-C₇-alkylene which is unsubstituted or substituted by up to four C₁-C₇-alkyl substituents and bound to two adjacent ring atoms of the phenyl or naphthyl moiety; pyrrolyl, furanyl and thienyl;
phenyl- or naphthyl-sulfonyl or phenyl-C₁-C₇-alkyl- or naphthyl-C₁-C₇-alkyl-sulfonyl, wherein each phenyl or naphthyl is unsubstituted or substituted by one or more substitutents selected from the group just described for substituted phenyl- or naphthyl R³, C₃-C₁₀-cycloalkyl or C₃-C₁₀-cyclyoalkyl-C₁-C₇-alkyl, in both of which cycloalkyl is unsubstituted or substituted by one or more of the substitutents just mentioned for substituted phenyl or naphthyl R³;
or heterocyclyl or heterocyclyl-C₁-C₇-alkyl wherein heterocyclyl is selected from pyrrolyl, furanyl, thienyl, pyrimidine-2,4-dione-1-, -3- or -5-yl, indolyl, benzofuranyl, benzimidazolyl, benzopyrazolyl, quinolinyl, isoquinolinyl, methylene-dioxy-phenyl, ethylene-1,2-dioxy-phenyl or trimethylen-1,3-dioxyphenyl wherein the oxy groups are bound to adjacent ring atoms of the phenyl ring, where each of the heterocyclyl moieties is unsubstituted or substituted as mentioned above for substituted phenyl R³;
or, if L is imino, oxy or thio, can alternatively be phenyl- or naphthylcarbonyl, C₁-C₇-alkoxycarbonyl (meaning C₁-C₇-alkyl-O-C(=O)-), phenyloxycarbonyl, naphthyloxycarbonyl, cycloalkyloxycarbonyl, heterocyclyloxycarbonyl, phenyl-C₁-C₇-alkytoxycarbonyl, naphthyl-C₁-C₇-alkyloxycarbonyl, cycloalkyl C₁-C₇-alkoxycarbonyl, heterocyclyl C₁-C₇-alkoxycarbonyl or N-mono- or N,N-di-(alkyl, naphtyl, phenyl, heterocyclyl, cycloalkyl, naphthyl-, heterocyclyclyl-, cycloalkyl- or phenyl-C₁-C₇-alkyl) -aminocarbonyl, where in each case the phenyl or naphthyl rings are unsubstituted or substituted as mentioned above for substituted phenyl or naphthyl R³;
and
R⁴ is hydrogen;
L is methylene (-CH₂-), oxy (-O-) or imino (-NH-);
and T is carbonyl, thiocarbonyl or methylene;
or a pharmaceutically acceptable salt thereof.

6. A compound of the formula I according to any one of claims 1 or 2, wherein
R¹ is
phenyl, naphthyl, phenylcarbonyl, naphthylcarbonyl or phenyl- or naphthyl-C₁-C₇-alkyl, wherein each phenyl or naphthyl is unsubstituted or substituted by one or more, e.g. up to three, substitutents selected from the group consisting of
a substitutent of the formula -(C₀-C₇-alkylene)-(X)ᵣ-(C₁-C₇-alkylene)-(Y)ₛ-(C₀-C₇-alkylene)-H where C₀-alkylene means that a bond is present instead of bound alkylene, r and s, each independently of the other, are 0 or 1 and each of X and Y, if present and independently of the others, is -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- wherein V is hydrogen or unsubstituted or substituted alkyl as defined below, especially selected from C₁-C₇-alkyl, phenyl, naphthyl, phenyl- or naphthyl-C₁-C₇-alkyl and halo-C₁-C₇-alkyl; e.g. C₁-C₇-alkyl, such as methyl, ethyl, n-propyl, isopropyl, n-butyl, isobutyl, sec-butyl or tert-butyl, hydroxy-C₁-C₇-alkyl, C₁-C₇-alkoxy-C₁-C₇-alkyl, such as 3-methoxypropyl or 2-methoxyethyl, C₁-C₇-alkoxy-C₁-C₇₋alkoxy-C₁-C₇-alkyl, C₁-C₇-alkanoylooxy-C₁-C₇-alkyl, amino-C₁-C₇-alkyl, such as aminomethyl, (N-) mono- or (N,N-) di-(C₁-C₇-alkyl)-amino-C₁-C₇-alkyl, C₁-C₇-alkoxy-C₁-C₇-alkylamino-C₁-C₇-alkyl, mono-(naphthyl- or phenyl)-amino-C₁-C₇-alkyl, mono-(naphthyl- or phenyl-C₁-C₇-alkyl)-amino-C₁-C₇-alkyl, C₁-C₇-alkanoylamino-C₁-C₇-alkyl, C₁-C₇-alkyl-O-CO-NH-C₁-C₇-alkyl, C₁-C₇-alkylsulfonylamino-C₁-C₇-alkyl, C₁-C₇-alkyl-NH-CO-NH-C₁-C₇-alkyl, C₁-C₇-alkyl-NH-SO₂-NH-C₁-C₇-alkyl, C₁-C₇-alkoxy, hydroxy-C₁-C₇-alkoxy, C₁-C₇-alkoxy-C₁-C₇alkoxy, C₁-C₇-alkanoyloxy, mono- or di-(C₁-C₇-alkyl)-amino, mono- di-(naphthyl- or phenyl-C₁-C₇-alkyl)-amino, N-mono-C₁-C₇-alkoxy-C₁-C₇-alkylamino, C₁-C₇-alkanoylamino, C₁-C₇-alkylsulfonylamino, C₁-C₇-alkoxy-carbonyl, hydroxy-C₁-C₇-alkoxycarbonyl, C₁-C₇-alkoxy-C₁-C₇-alkoxycarbonyl, amino-C₁-C₇-alkoxycarbonyl, (N-) mono-(C₁-C₇-alkyl)-amino-C₁-C₇-alkoxycarbonyl, C₁-C₇-alkanoylamino-C₁-C₇-alkoxycarbonyl, N- mono- or N,N-di-(C₁-C₇-alkyl)-aminocarbonyl. N-C₁-C₇-alkoxy-C₁-C₇-alkylcarbamoyl or N-mono- or N,N-di-(C₁-C₇-alkyl)-aminosulfonyl; or
from phenyl- or naphthyl-C₁-C₇-alkyl, halo-C₁-C₇-alkyl, halo, hydroxy, phenyl- or naphthyloxy, phenyl- or naphthyl-C₁-C₇-alkyloxy, nitro, amino, amino-C₁-C₇-alkyl, carboxyl, halo-C₁-C₇-alkoxycarbonyl, phenyl- or naphthyl-C₁-C₇-alkoxycarbonyl, C₁-C₇-alkylsulfonyl, carbamoyl and cyano;
R² is;
C₁-C₇-alkyl that is unsubstituted or substituted by one or more, e.g. up to three, substitutents selected from the group consisting of halo, phenyl- or naphthyl, hydroxy, C₁-C₇-alkoxy, amino, mono- or di-(C₁-C₇-alkyl)-amino, C₁-C₇-alkanoylamino, C₁-C₇-alkyl-sulfonylamino, phenyl- or napthylsulfonylamino, phenyl- or naphthyl-C₁-C₇-alkylsulfonylamino, C₁-C₇-alkoxy-C₁-C₇-alkoxy, hydroxy-C₁-C₇-alkoxy, phenyl- or naphthyloxy, phenyl- or naphthyl-C₁-C₇-alkyloxy, C₁-C₇-alkanoyloxy, nitro, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl-, phenyl-, naphthyl-, phenyl-C₁-C₇-alkyl- or naphthyl-C₁-C₇-alkyl-)carbamoyl and N-mono- or N,N-di-(C₁-C₇-alkyl-, phenyl-, naphthyl-, phenyl-C₁-C₇-alkyl- or naphthyl-C₁-C₇-alkyl-)sulfamoyl; and cyano;
R³ is
phenyl or naphthyl, wherein each phenyl or naphthyl is unsubstituted or substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylylamino-C₁-C₇-alkyl, phenyl, naphthyl, mono- or di-(C₁-C₇-alkoxy)-phenyl or -naphthyl, C₁-C₇-alkylendioxy-phenyl where the oxy atoms are bound to adjacent phenyl ring atoms, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, (unsubstituted or mono-, di- or tri-C₁-C₇-alkyl substituted)-phenyl- or -naphthyl-C₁-C₇-alkyloxy, C₁-C₇-alkanoyloxy, nitro, amino, mono- or di-(C₁-C₇-alkyl)-amino, C₁-C₇-alkanoylamino C₁-C₇-alkylsulfonylylamino, carboxyl, C₁-C₇-alkoxy-carbonyl, phenyl- or naphthyl-C₁-C₇-alkoxycarbonyl, carbamoyl and cyano, C₁-C₇-alkylsulfonyl, C₁-C₇-alkylene which is unsubstituted or substituted by up to four C₁-C₇-alkyl substituents and bound to two adjacent ring atoms of the phenyl or naphthyl moiety; pyrrolyl, furanyl and thienyl;
R⁴ is hydrogen;
L is a bond;
and T is carbonyl, thiocarbonyl or preferably methylene;
or a pharmaceutically acceptable salt thereof.

7. A compound of the formula I according to any one of claims 1 or 2, wherein
R¹ is phenylmethyl or naphthylmethyl, where each phenyl or naphthyl is unsubstituted or substituted by one or more, e.g. up to three, substitutents selected from the group consisting of C₁-C₇-alkyl, phenyl, naphthyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-suffonylamino-C₁-C₇-alkyl, halo, hydroxy, C₁-C₇-alkoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl and/or (phenyl- or naphthyl)-C₁-C₇-alkyl)-carbamoyl, C₁-C₇-alkylsulfonyl, unsubstituted or C₁-C₇-alkyl-substituted phenyl- or naphthylsulfonyl, N-mono- or N,N-di-(C₁-C₇-alkyl and/or (phenyl- or naphthyl)-C₁-C₇-alkyl)-sulfamoyl and cyano;
R² is phenyl that is unsubstituted or substituted by one or more, e.g. up to three, substituents selected from the group consisting of C₁-C₇-alkyl, phenyl, naphthyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkylsulfonylamino-C₁-C₇-alkyl, halo, hydroxy, C₁-C₇-alkoxy, hydroxy-C₁-C₇-alkoxy, C₁-C₇-alkoxy-C₁-C₇-alkoxy, phenyl- or naphthyloxy, phenyl- or naphthyl-C₁-C₇-alkyloxy, C₁-C₇-alkanoyloxy, nitro, amino, mono- or di-(C₁-C₇-alkyl)-amino, C₁-C₇-alkanoylamino, amino-C₁-C₇-alkyl, mono- or di-(C₁-C₇-alkyl)-amino-C₁-C₇-alkyl, C₁-C₇-alkanoylamino-C₁-C₇-alkyl, carboxyl, C₁-C₇-alkoxy-carbonyl, phenyl- or naphthyl-C₁-C₇-alkoxycarbonyl, carbamoyl, N-mono- or N,N-di-(C₁-C₇-alkyl and/or (phenyl- or naphthyl)-C₁-C₇-alkyl)-carbamoyl, C₁-C₇-alkylsulfonyl, unsubstituted or C₁-C₇-alkyl-substituted phenyl- or naphthylsulfonyl, N-mono- or N,N-di-(C₁-C₇-alkyl and/or (phenyl- or naphthyl)-C₁-C₇-alkyl)-sulfamoyl and cyano;
R³ is phenyl, naphthyl, phenyl-C₁-C₇-alkyl or naphthyl-C₁-C₇alkyl, wherein each phenyl or naphthyl is unsubstituted or substituted by one or more, e.g. up to three, substitutents selected from the group consisting of C₁-C₇-alkyl, phenyl- or naphthyl-C₁-C₇-alkyl, halo-C₁-C₇-alkyl, hydroxy-C₁-C₇-alkyl, C₁-C₇-alkoxy-C₁-C₇-alkyl, amino-C₁-C₇-alkyl, mono- or di-(C₁-C₇-alkyl)-amino-C₁-C₇-alkyl, C₁-C₇-alkanoylamino-C₁-C₇-alkyl, C₁-C₇-alkyl-sulfonylylamino-C₁-C₇-alkyl, phenyl, naphthyl, mono- or di-(C₁-C₇-alkoxy)-phenyl or -naphthyl, C₁-C₇-alkylendioxy-phenyl where the oxy atoms are bound to adjacent phenyl ring atoms, halo, hydroxy, C₁-C₇-alkoxy, halo-C₁-C₇-alkoxy, hydroxy-C₁-C₇-alkoxy, C₁-C₇-alkoxy-C₁-C₇-alkoxy, phenyl- or naphthyloxy, (unsubstituted or mono-, di- or tri-C₁-C₇-alkyl substituted)-phenyl- or - naphthyl-C₁-C₇-alkyloxy, C₁-C₇-alkanoyloxy, nitro, amino, mono- or di-(C₁-C₇-alkyl)-amino, C₁-C₇-alkanoylamino C₁-C₇-alkylsulfonylylamino, carboxyl, C₁-C₇-alkoxy-carbonyl, phenyl- or naphthyl-C₁-C₇-alkoxycarbonyl, carbamoyl and cyano, C₁-C₇-alkylsulfonyl, C₁-C₇-alkylene which is unsubstituted or substituted by up to four C₁-C₇-alkyl substituents and bound to two adjacent ring atoms of the phenyl or naphthyl moiety, pyrrolyl, furanyl and thienyl;
phenyl- or naphthyl-sulfonyl or phenyl-C₁-C₇-alkyl- or naphthyl-C₁-C₇-alkyl-sulfonyl, wherein each phenyl or naphthyl is unsubstituted or substituted by one or more, e.g. up to three, substitutents selected from the group just described for substituted phenyl- or naphthyl R³, C₃-C₁₀-cycloalkyl or C₃-C₁₀-cyclyoalkyl-C₁-C₇-alkyl, in both of which cycloalkyl is unsubstituted or substituted by one or more of the substitutents just mentioned for substituted phenyl or naphthyl R³, especially by C₁-C₇-alkyl, phenyl, naphthyl, phenyl-C₁-C₇-alkyl or naphthyl-C₁-C₇-alkyl;
or heterocyclyl or heterocyclyl-C₁-C₇-alkyl wherein heterocyclyl is selected from pyrrolyl, furanyl, thienyl, pyrimidine-2,4-dione-1-, -3- or-5-yl, indolyl, benzofuranyl, benzimidazolyl, benzopyrazolyl, quinolinyl, isoquinolinyl, methylene-dioxy-phenyl, ethylene-1,2-dioxy-phenyl or trimethylen-1,3-dioxyphenyt wherein the oxy groups are bound to adjacent ring atoms of the phenyl ring, where each of the heterocyclyl moieties is unsubstituted or substituted as mentioned above for substituted phenyl R³;
R⁴ is hydrogen;
L is methylene;
and T is methylene;
or a pharmaceutically acceptable salt thereof.

8. A compound of the formula I according to any one of claims 1 to 7 having the formula IA, 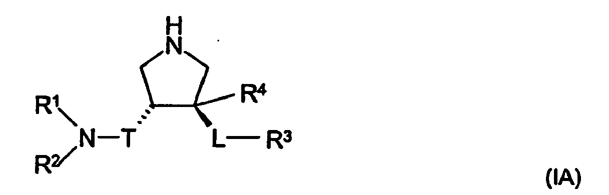 wherein R¹, R², R³, R⁴, T and L are as defined in any one of the preceding claims, or a pharmaceutically acceptable salt thereof.

9. A compound of the formula I according to any one of claims 1 to 7, having the formula IB, 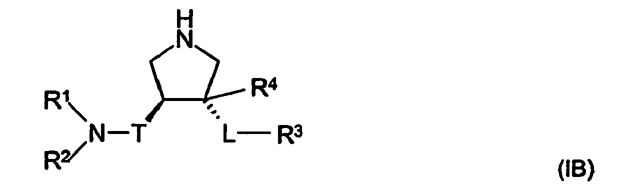 the formula IC, 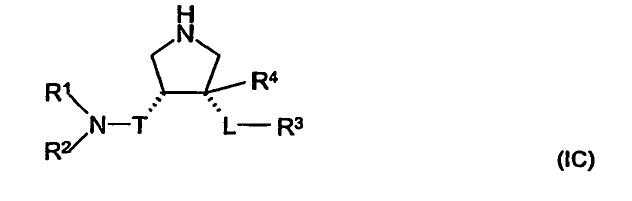 or the formula ID, 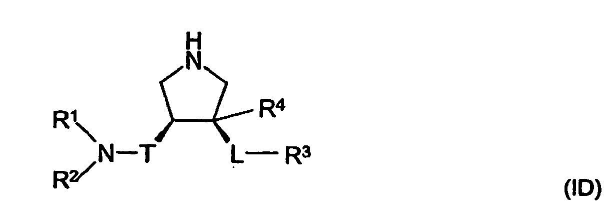 wherein R¹, R², R³, R⁴, T and L are as defined in any one of the preceding claims, or a pharmaceutically acceptable salt thereof.

10. A compound of the formula I according to any of the preceding claims
wherein
R¹ is unsubstituted or substituted aryl, unsubstituted or substituted aryl-alkyl, or acyl;
R² is unsubstituted or substituted alkyl, unsubstituted or substituted aryl, unsubstituted or substituted cycloalkyl, unsubstituted or substituted aryl-alkyl, unsubstituted or substituted mono- or bicyclic heterocyclyl-alkyl, unsubstituted or substituted cycloalkyl-alkyl, with the proviso that if L is methylene (-CH₂-), oxy (-O-), thio (-S-) or unsubstituted (-NH-)or substituted imino, R² is selected from one of the mentioned groups and from hydrogen;
R³ is unsubstituted or substituted alkyl, substituted or unsubstituted aryl, unsubstituted or substituted heterocyclyl, unsubstituted or substituted cycloalkyl, unsubstituted or substituted aryl-alkyl, unsubstituted or substituted heterocyclyl-alkyl, unsubstituted or substituted cycloalkyl-alkyl or, if L is oxy or unsubstituted or substituted imino, has one of the meanings just mentioned or is unsubstituted or substituted alkylcarbonyl, unsubstituted or substituted arylcarbonyl, unsubstituted or substituted heterocyclylcarbonyl, unsubstituted or substituted cycloalkylcarbonyl, unsubstituted or substituted aryl-alkylcarbonyl, unsubstituted or substituted heterocyclyl-alkyl carbonyl, unsubstituted or substituted cycloalkyl-alkyl carbonyl, etherified carboxy, carbamoyl or N-mono- or N,N-di-substituted amino-carbonyl; substituted or unsubstituted alkylsulfonyl, substituted or unsubstituted arylsulfonyl: substituted or unsubstituted aryl-alkyl sulfonyl N-mono- or N,N-di-substituted amino-sulfonyl;
R⁴ is hydrogen or hydroxy;
L is a bond, methylene (-CH₂-), oxy (-O-), or unsubstituted (-NH-)or substituted imino, with the proviso that if L is a bond then R³ is one of the moieties mentioned for R³ other than substituted alkyl;
or R³ and R⁴ which then is -O- together with L which then is methylene and the carbon to which R³-L- and R⁴ are bound form a substituted or unsubstituted ring annealed to an unsubstituted or substituted aryl, thus forming a spiro compound of the formula I, or
R³ and R⁴ together with L form oxo (=O);
and
T is methylene;
or a salt thereof.

11. A compound of the formula I according to any of the preceding claims
wherein
L is methylene; and
R³ has one of the following definitions (a) or (b):
(a) R³ is unsubstituted or substituted aryl as defined above,
(b) R³ is alkyl, which is optionally substituted by one or two substituent selected from the group consisting of O-C₁-C₄-alkyl, halo, hydroxy, unsubstituted or substituted phenyl, unsubstituted or substituted naphthyl, unsubstituted or substituted phenyl- or naphthyloxy, unsubstituted or substituted phenyl- or naphthyl-C₁-C₇-alkyloxy, unsubstituted or substituted cycloalkyl, nitro, amino, amino-C₁-C₇-alkyl, N-mono- or N,N-di-substituted aminocarbonyl, carboxyl, and cyano.

12. A compound of the formula I according to any of the preceding claims
wherein
L is O or ; and
R³ is one of the following (a) to (f):
(a) unsubstituted or substituted aryl wherein suitable substituents are selected from the group consisiting of C₁-C₇-alkyl, -O-C₁-C₇-alkyl, halo-C₁-C₇-alkyl, halo, hydroxy, unsubstituted or substituted phenyl- or naphthyloxy, unsubstituted or substituted phenyl- or naphthyl-C₁-C₇-alkyloxy, nitro, amino, amino-C₁-C₇-alkyl, carboxyl, and cyano;
(b) unsubstituted or substituted aryl alkyl wherein suitable substituents are selected from the group consisting of C₁-C₇-alkyl, -O-C₁-C₇-alkyl, halo-C₁-C₇-alkyl, halo-C₁-C₇-alkyl-O-, halo, hydroxy, unsubstituted or substituted phenyl, unsubstituted or substituted naphthyl, unsubstituted or substituted phenyl- or naphthyloxy, unsubstituted or substituted phenyl- or naphthyl-C₁-C₇-alkyloxy, unsubstituted or substituted heterocyclyl, nitro, amino, amino-C₁-C₇-alkyl, carboxyl, and cyano, whereby if phenyl, naphthyl, phenyl- or naphthyloxy, phenyl- or naphthyl-C₁-C₇-alkyloxy, heterocyclyl are substituted, they are preferably mono-or di-substituted by C₁-C₇-alkyl, -O-C₁-C₇-alkyl, halo-C₁-C₇-alkyl, halo, hydroxy, amino, amino-C₁-C₇-alkyl, acylamino, heterocyclyl, or cyano;
(c) unsubstituted or substituted heterocyclyl-alkyl, wherein suitable substituents are selected from the group consisiting of halo, hydroxy, unsubstituted or substituted phenyl, unsubstituted or substituted phenyl- or naphthyloxy, unsubstituted or substituted phenyl- or naphthyl-C₁-C₇-alkyloxy, nitro, amino, amino-C₁-C₇-alkyl, carboxyl, and cyano;
(d) unsubstituted or substituted alkyl wherein suitable substituents are selected from the group consisiting of O-C₁-C₄-alkyl, halo, hydroxy, unsubstituted or substituted phenyl, unsubstituted or substituted naphthyl, unsubstituted or substituted phenyl- or naphthyloxy, unsubstituted or substituted phenyl- or naphthyl-C₁-C₇-alkyloxy, unsubstituted or substituted cycloalkyl, nitro, amino, amino-C₁-C₇-alkyl, N-mono- or N,N-di-substituted aminocarbonyl, carboxyl, and cyano;
(e) unsubstituted or substituted aminocarbonyl that is mono- or di-substituted at the nitrogen by one or more moieties selected from unsubstituted or substituted alkyl, unsubstituted or substituted aryl, or unsubstituted or substituted cycloalkyl, whereby alkyl substitutents are selected from
(i) aryl, which may be unsubstituted or further substituted by O-C₁-C₄-alkyl, halo, hydroxy, unsubstituted or substituted heterocyclyl, nitro, amino, acylamino, amino-C₁-C₇-alkyl, carboxyl, and cyano;
(ii) heterocyclyl, such as 5- or 6 membered rings optionally containing a nitrogen, or oxygen atom, which may be unsubstituted or further substituted by C₁-C₇-alkyl, halo, hydroxy, nitro, unsubstituted or substituted amino, unsubstituted or substituted amino-C₁-C₇-alkyl, carboxyl, and cyano;
(iii) halo, hydroxy, nitro, amino, amino-C₁-C₇-alkyl, carboxyl, and cyano;
(f) unsubstituted or substituted heterocyclyl carbonyl, wherein suitable substituents are selected from the group consisiting of C₁-C₇-alkyl, O-C₁-C₇-alkyl, halo-C₁-C₇-alkyl, halo, hydroxy, unsubstituted or substituted phenyl or naphthyl, unsubstituted or substituted phenyl- or naphthyloxy, unsubstituted or substituted phenyl- or naphthyl-C₁-C₇-alkyloxy, nitro, amino, amino-C₁-C₇-alkyl, carboxyl, and cyano.

13. A compound of the formula I according to any of the preceding claims
wherein
L is NH or substituted NH, whereby substituted NH means substituted with cycloalkyl alkyl, alkyl or with N-mono- or N.N-di-substituted aminocarbonyl substituted alkyl; and
R³ is one of the following (a) to (m):
(a) unsubstituted or substituted aryl-alkyl, wherein suitable substituents are selected from the group consisiting of -(C₀-C₇-alkylene)-(X)ᵣ-(C₁-C₇-alkylene)-(Y)ₛ-(C₀-C₇-alkylene)-H, wherein r and s are 0 or 1 and Y and X are independently O, NH or -NH-CO-O-, -CO-NH-, NHCO, N(C₁-C₇-alkyl), halo-C₁-C₇-alkyl, halo-C₁-C₇-alkyl-O-, halo, hydroxy, unsubstituted or substituted phenyl- or naphthyloxy, unsubstituted or substituted phenyl- or naphthyl-C₁-C₇-alkyloxy, nitro, amino, N(mono or di- CO-C₁-C₇-alkyl or formyl) unsubstituted or substituted amino, unsubstituted or substituted amino-C₁-C₇-alkyl, whereby the amino moiety can be substituted by -C₁-C₇-alkyl, cycloalkyl, phenyl or heterocyclyl; carboxyl, and cyano;
(b) unsubstituted or substituted heterocyclyl-alkyl or unsubstituted or substituted heterocyclyl wherein the heterocyclyl moiety is a 5-membered ring containing a nitrogen atom; or a bicyclic ring system containing a nitrogen or oxygen atom, wherein suitable substituents are selected from the group consisiting of C₁-C₇-alkyl, halo, hydroxy, unsubstituted or substituted phenyl, unsubstituted or substituted phenyl- or naphthyloxy, unsubstituted or substituted phenyl- or naphthyl-C₁-C₇-alkyl, nitro, amino, amino-C₁-C₇-alkyl, carboxyl, and cyano;
(c) unsubstituted or substituted cycloalkyl wherein substituents are selected from the group consisiting of O-C₁-C₄-alkyl, halo, hydroxy, unsubstituted or substituted phenyl, naphthyl, unsubstituted or substituted phenyl- or naphthyloxy, unsubstituted or substituted phenyl- or naphthyl-C₁-C₇-alkyloxy, nitro, amino, amino-C₁-C₇-alkyl, carboxyl, and cyano;
(d) unsubstituted or substituted cycloalkyl-alkyl wherein substituents are selected from the group consisiting of O-C₁-C₄-alkyl, halo, hydroxy, unsubstituted or substituted phenyl, naphthyl, unsubstituted or substituted phenyl- or naphthyloxy, unsubstituted or substituted phenyl- or naphthyl-C₁-C₇-alkyloxy, nitro, unsubstituted or substituted amino, unsubstituted or substituted amino-C₁-C₇-alkyl, whereby the amino moiety can be substituted by -C₁-C₇-alkyl, cycloalkyl, phenyl or heterocyclyl; carboxyl, and cyano;
(e) unsubstituted or substituted alkyl wherein substituents are selected from the group consisiting of O-C₁-C₄-alkyl, halo, hydroxy, unsubstituted or substituted phenyl, unsubstituted or substituted naphthyl, unsubstituted or substituted phenyl- or naphthyloxy, unsubstituted or substituted phenyl- or naphthyl-C₁-C₇-alkyloxy, unsubstituted or substituted cycloalkyl, nitro, amino, amino-C₁-C₇-alkyl, N-mono- or N,N-di-substituted aminocarbonyl, carboxyl, and cyano;
(f) unsubstituted or substituted arylcarbonyl, wherein substituents are selected from the group consisiting of -(C₀-C₇-alkylene)-(X)ᵣ-(C₁-C₇-alkylene)-(Y)ₛ-(C₀-C₇-alkylene)-H, wherein r and s are 0 or 1 and Y and X are independently O, NH or -NH-CO-O-, -CO-NH-, NHCO, N(C₁-C₇-alkyl), halo-C₁-C₇-alkyl, halo, hydroxy, unsubstituted or substituted phenyl- or naphthyloxy, unsubstituted or substituted phenyl- or naphthyl-C₁-C₇-alkyloxy, nitro, unsubstituted or substituted amino, N(mono or di- CO-C₁-C₇-alkyl or formyl) amino, amino-C₁-C₇-alkyl, carboxyl, and cyano;
(g) unsubstituted or substituted alkylcarbonyl wherein substituents are selected from the group consisiting of O-C₁-C₄-akyl, halo, hydroxy, unsubstituted or substituted phenyl or naphthyl, unsubstituted or substituted C₃-C₇-cycloalkyl, or substituted heterocyclyl, nitro, amino, amino-C₁-C₇-alkyl, carboxyl, and cyano;
(h) unsubstituted or substituted cycloalkyl-carbonyl wherein suitable substituents are selected from the group consisiting of C₁-C₇-alkyl, O-C₁-C₄-alkyl, halo, hydroxy, unsubstituted or substituted phenyl, naphthyl, unsubstituted or substituted phenyl- or naphthyloxy, unsubstituted or substituted phenyl- or naphthyl-C₁-C₇-alkyloxy, nitro, amino, amino-C₁-C₇-alkyl, carboxyl, and cyano;
(i) unsubstituted or substituted heterocyclyl-carbonyl wherein the heterocyclyl moiety includes 6-membered rings optionally containing an oxygen atom; and bicyclic ring systems wherein suitable substituents are selected from the group consisiting of halo, hydroxy, unsubstituted or substituted phenyl, unsubstituted or substituted phenyl- or naphthyloxy, unsubstituted or substituted phenyl- or naphthyl-C₁-C₇-alkyl, nitro, amino, amino-C₁-C₇-alkyl, carboxyl, and cyano,;
(j) unsubstituted or substituted etherified carboxy to which one of the following groups are bound:
(i) O-alkyl, which is optionally substituted wherein suitable substituents are selected from the group consisiting of O-C₁-C₄-alkyl, halo, hydroxy, unsubstituted or substituted phenyl, unsubstituted or substituted naphthyl, unsubstituted or substituted phenyl- or naphthyloxy, unsubstituted or substituted phenyl- or naphthyl-C₁-C₇-alkyloxy, unsubstituted or substituted cycloalkyl, nitro, amino, amino-C₁-C₇-alkyl, N-mono- or N,N-di-substituted aminocarbonyl,
(ii) O-aryl, which may be substituted wherein suitable substituents are selected from the group consisiting of C₁-C₇-alkyl, O-C₁-C₄-alkyl, halo-C₁-C₇-alkyl, halo, hydroxy, substituted or unsubstituted amino, acylamino, substituted or unsubstituted amino-C₁-C₇-alkyl, carboxyl, and cyano;
(iii) O-cycloalkyl, which may be substituted wherein suitable substituents are selected from the group consisiting of C₁-C₇-alkyl, O-C₁-C₄-alkyl, halo, hydroxy, nitro, substituted or unsubstituted amino, substituted or unsubstituted amino-C₁-C₇-alkyl, carboxyl, and cyano;
(k) unsubstituted or substituted aminocarbonyl. that is mono- or di-substituted at the nitrogen by one or more moieties selected from unsubstituted or substituted alkyl, unsubstituted or substituted aryl, or unsubstituted or substituted cycloalkyl, wherein suitable substituents for the alkyl moiety is aryl, and wherein aryl may be unsubstituted or further substituted by C₁-C₇-alkyl, O-C₁-C₄-alkyl, halo-C₁-C₇-alkyl, halo, hydroxy, nitro, amino, acylamino, amino-C₁-C₇-alkyl, carboxyl, and cyano;
(l) unsubstituted or substituted arylsufonyl wherein substituents are selected from the group consisiting of C₁-C₇-alkyl, O-C₁-C₄-alkyl, halo-C₁-C₇-alkyl, halo, hydroxy, unsubstituted or substituted phenyl, unsubstituted or substituted phenyl- or naphthyloxy, unsubstituted or substituted phenyl- or naphthyl-C₁-C₇-alkyl, amino, acylamino, amino-C₁-C₇-alkyl, carboxyl, and cyano;
(m) unsubstituted or substituted alkylsufonyl, wherein substituents are selected from the group consisiting of O-C₁-C₄-alkyl, halo, hydroxy, unsubstituted or substituted phenyl or naphthyl, unsubstituted or substituted phenyl- or naphthyloxy, unsubstituted or substituted phenyl- or naphthyl-C₁-C₇-alkyl, unsubstituted or substituted C₃-C₇-cycloalkyl; or substituted or unsubstituted heterocyclyl, nitro, amino, amino-C₁-C₇-alkyl, carboxyl, and cyano.

14. A compound of the formula I according to claim 1, selected from the group consisting of ((3S*,4S*)-4-benzyl-pyrrolidin-3-ylmethyl)-(4-chloro-phenyl)-phenyl-amine; ((3R*,4R*)-4-benzyl-pyrrolidin-3-ylmethyl)-diphenyl-amine; 3-[((3R*,4R*)-4-benzyl-pyrrolidin-3-ylmethyl)-phenyl-amino]-phenol; ((3S*,4S*)-4-benzyl-pyrrolidin-3-ylmethyl)-phenethyl-phenyl-amine; (3R*,4R*)-benzyl-(4-chloro-phenyl)-[4-(3-isopropyl-benzyl)-pyrrolidin-3-ylmethyl]-amine; (3R*,4R*)-(4-chloro-phenyl)-[4-(3-isopropyl-benzyl)-pyrrolidin-3-ylmethyl]-phenyl-amine; N-((3S*,4R*)-4-benzylamino-pyrrolidin-3-ylmethyl)-N-isopropyl-4-methoxy-3-(3-methoxy-propoxy)-benzamide ((3R*,4R*)-4-benzyl-pyrrolidin-3-ylmethyl)-(4-chloro-phenyl)-cyclohexylamine; ((3R*,4R*)-4-benzyl-pyrrolidin-3-ylmethyl)-(4-chloro-phenyl)-((R)-1-phenyl-ethyl)-amine; ((3R*,4R*)-4-benzyl-pyrrolidin-3-ylmethyl)-(4-chloro-3-methoxy-phenyl)-phenyl-amine; 3-{[((3R*,4R*)-4-benzyl-pyrrolidin-3-ylmethyl)-(4-chloro-phenyl)-amino]-methyl}-benzonitrile; benzyl-((3R*,4R*)-4-benzyl-pyrrolidin-3-ylmethyl)-(4-chloro-phenyl)-amine; benzyl-((3R,4R)-4-benzyl-pyrrolidin-3-ylmethyl)-(4-chloro-phenyl)-amine; benzyl-((3S,4S)-4-benzyl-pyrrolidin-3-ylmethyl)-(4-chloro-phenyl)-amine; ((3R*,4R*)-4-benzyl-pyrrolidin-3-ylmethyl)-(4-chloro-phenyl)-(2-methoxy-benzyl)-amine; ((3R*,4R*)-4-benzyl-pyrrolidin-3-ylmethyl)-(4-chloro-phenyl)-(3-methoxy-benzyl)-amine; benzyl-((3R*,4R*)-4-benzyl-pyrrolidin-3-ylmethyl)-(4-methoxy-phenyl)-amine; benzyl-((3R*,4R*)-4-benzyl-pyrrolidin-3-ylmethyl)-p-tolyl-amine; benzyl-((3R*,4R*)-4-benzyl-pyrrolidin-3-ylmethyl)-(4-fluoro-phenyl)-amine; ((3R*,4R*)-4-benzyl-pyrrolidin-3-ylmethyl)-(4-chloro-phenyl)-naphthalen-1-ylmethyl-amine; 3-{[((3R*,4R*)-4-benzyl-pyrrolidin-3-ylmethyl)-(4-chloro-phenyl)-amino]-methyl}-benzamide; ((3R*,4R*)-4-benzyl-pyrrolidin-3-ylmethyl)-(4-chloro-phenyl)-naphthalen-2-ylmethyl-amine; ((3R*,4R*)-4-benzyl-pyrrolidin-3-ylmethyl)-(4-chloro-phenyl)-(4-propoxy-2-trifluoromethyl-quinolin-6-ylmethyl)-amine; 7-{[((3R*,4R*)-4-benzyl-pyrrolidin-3-ylmethyl)-(4-chloro-phenyl)-amino]-methyl}-naphthalene-2-carbonitrile; 7-{[((3R*,4R*)-4-benzyl-pyrrolidin-3-ylmethyl)-(4-chloro-phenyl)-amino]-methyl}-naphthalene-1-carbonitrile; 6-{[((3R*,4R*)-4-benzyl-pyrrolidin-3-ylmethyl)-(4-chloro-phenyl)-amino]-methyl}-naphthalene-2-carbonitrile; 6-{[((3R*,4R*)-4-benzyl-pyrrolidin-3-ylmethyl)-(4-chloro-phenyl)-amino]-methyl}-1H-pyrimidine-2,4-dione; 5-{[((3R*,4R*)-4-benzyl-pyrrolidin-3-ylmethyl)-(4-chloro-phenyl)-amino]-methyl}-naphthalene-2-carbonitrile; 5-{[((3R*,4R*)-4-benzyl-pyrrolidin-3-ylmethyl)-(4-chloro-phenyl)-amino]-methyl}-naphthalene-1-carbonitrile; 4-{[((3R*,4R*)-4-benzyl-pyrrolidin-3-ylmethyl)-(4-chloro-phenyl)-amino]-methyl}-naphthalene-1-carbonitrile; 4-{[((3R*,4R*)-4-benzyl-pyrrolidin-3-ylmethyl)-(4-chloro-phenyl)-amino]-methyl}-benzonitrile; 5-{[((3R*,4R*)-4-benzyl-pyrrolidin-3-ylmethyl)-(4-chloro-phenyl)-amino]-methyl}-2-fluoro-benzonitrile; 4-[((3R*,4R*)-4-benzyl-pyrrolidin-3-ylmethyl)-naphthalen-2-ylmethyl-amino]-benzonitrile; 4-{[((3R*,4R*)-4-benzyl-pyrrolidin-3-ylmethyl)-(4-chloro-benzyl)-amino]-methyl}-benzonitrile; N-((3R*,4R*)-4-benzyl-pyrrolidin-3-ylmethyl)-N-(3-cyano-phenyl)-4-methoxy-3-(3-methoxy-propoxy)-benzamide; N-((3R*,4R*)-4-benzyl-pyrrolidin-3-ylmethyl)-3,N-diphenyl-propionamide, hydrochloride; (1R*,2R*)-2-phenyl-cyclopropanecarboxylic acid ((3R*,4R*)-4-benzyl-pyrrolidin-3-ylmethyl)-phenyl-amide; 1R*,2R*)-2-phenyl-cyclopropanecarboxylic acid ((3R*,4R*)-4-benzyl-pyrrolidin-3-ylmethyl)-(4-chloro-phenyl)-amide; naphthalene-2-carboxylic acid ((3R*,4R*)-4-benzyl-pyrrolidin-3-ylmethyl)-(4-chloro-phenyl)-amide; naphthalene-1-carboxylic acid ((3R*,4R*)-4-benzyl-pyrrolidin-3-ylmethyl)-(4-chloro-phenyl)-amide; N-((3R*,4R*)-4-benzyl-pyrrolidin-3-ylmethyl)-N-(4-chloro-phenyl)-4-methoxy-3-(3-methoxy-propoxy)-benzamide; N-((3R*,4R*)-4-benzyl-pyrrolidin-3-ylmethyl)-4-methoxy-3-(3-methoxy-propoxy)-N-phenyl-benzamide; N-((3R*,4R*)-4-benzyl-pyrrolidin-3-ylmethyl)-N-(4-cyano-phenyl)-4-methoxy-3-(3-methoxy-propoxy)-benzamide; naphthalene-2-carboxylic acid ((3R*,4R*)-4-benzyl-pyrrolidin-3-ylmethyl)-(4-cyano-phenyl)-amide; benzofuran-5-carboxylic acid ((3R*,4R*)-4-benzyl-pyrrolidin-3-ylmethyl)-(4-cyano-phenyl)-amide; naphthalene-2-carboxylic acid ((3R*,4R*)-4-benzyl-pyrrolidin-3-ylmethyl)-(4-phenoxy-phenyl)-amide; benzofuran-5-carboxylic acid ((3R*,4R*)-4-benzyl-pyrrolidin-3-ylmethyl)-(4-chloro-phenyl)-amide; N-((3R*,4R*)-4-benzyl-pyrrolidin-3-ylmethyl)-N-(4-chloro-phenyl)-2-phenoxy-benzamide; N-((3R*,4R*)-4-benzyl-pyrrolidin-3-ylmethyl)-N-(4-chloro-phenyl)-benzenesulfonamide; ((3R*,4R*)-4-benzyl-pyrrolidin-3-ylmethyl)-(4-chloro-phenyl)-phenethyl-amine; N-((3S*,4S*)-4-benzyl-pyrrolidin-3-ylmethyl)-N-isopropyl-3-(3-methoxy-propoxy)-4-methyl-benzamide; N-((3S*,4S*)-4-benzyl-pyrrolidin-3-ylmethyl)-N-isopropyl-4-methoxy-3-(3-methoxy-propoxy)-benzamide; N-((3S,4S)-4-benzyl-pyrrolidin-3-ylmethyl)-N-isopropyl-4-methoxy-3-(3-methoxy-propoxy)-benzamide; N-((3R,4R)-4-benzyl-pyrrolidin-3-ylmethyl)-N-isopropyl-4-methoxy-3-(3-methoxy-propoxy)-benzamide; naphthalene-2-carboxylic acid ((3R*,4R*)-4-benzyl-pyrrolidin-3-ylmethyl)-isopropyl-amide; N-((3R*,4R*)-4-benzyl-pyrrolidin-3-ylmethyl)-4-ethyl-N-isopropyl-3-(3-methoxy-propoxy)-benzamide; N-((3R*,4R*)-4-benzyl-pyrrolidin-3-ylmethyl)-N-isopropyl-3-(3-methoxy-propoxy)-benzamide; 1-(3-methoxy-propyl)-1H-indole-2-carboxylic acid ((3R*,4R*)-4-benzyl-pyrrolidin-3-ylmethyl)-isopropyl-amide; N-((3R*,4R*)-4-benzyl-pyrrolidin-3-ylmethyl)-N-cyclopentyl-4-methoxy-3-(3-methoxy-propoxy)-benzamide; N-((3R*,4R*)-4-benzyl-pyrrolidin-3-ylmethyl)-N-cycloproyl-4-methoxy-3-(3-methoxy-propoxy)-benzamide; N-((3R*,4R*)-4-benzyl-pyrrolidin-3-ylmethyl)-4-methoxy-3-(3-methoxy-propoxy)-N-thiophen-2-ylmethyl-benzamide; N-((3R*,4R*)-4-benzyl-pyrrolidin-3-ylmethyl)-4-methoxy-N-(2-methoxy-ethyl)-3-(3-methoxy-propoxy)-benzamide; N-((3R*,4R*)-4.-benzyl-pyrrolidin-3-ylmethyl)-N-(4-chloro-benzyl)-4-methoxy-3-(3-methoxy-propoxy)-benzamide; N-((3R*,4R*)-4-benzyl-pyrrolidin-3-ylmethyl)-4-methoxy-3-(3-methoxy-propoxy)-N-methyl-benzamide; N-((3R*,4R*)-4-benzyl-pyrrolidin-3-ylmethyl)-N-cyclobutyl-4-methoxy-3-(3-methoxy-propoxy)-benzamide; N-((3R*,4R*)-4-benzyl-pyrrolidin-3-ylmethyl)-N-(1-ethyl-propyl)-4-methoxy-3-(3-methoxy-propoxy)-benzamide; N-((3R*,4R*)-4-benzyl-pyrrolidin-3-ylmethyl)-N-cyclopropylmethyl-4-methoxy-3-(3-methoxy-propoxy)-benzamide; N-((3R*,4R*)-4-benzyl-pyrrolidin-3-ylmethyl)-4-methoxy-3-(3-methoxy-propoxy)-N-(2,2,2-trifluoro-ethyl)-benzamide; N-((3R*,4R*)-4-benzyl-pyrrolidin-3-ylmethyl)-N-(4-cyano-benzyl)-4-methoxy-3-(3-methoxy-propoxy)-benzamide; N-((3R*,4R*)-4-benzyl-pyrrolidin-3-ylmethyl)-N-(1,2-dimethyl-propyl)-4-methoxy-3-(3-methoxy-propoxy)-benzamide; N-((3R*,4R*)-4-benzyl-pyrrolidin-3-ylmethyl)-N-(4-chloro-benzyl)-2-phenoxy-benzamide; N-((3R*,4R*)-4-benzyl-pyrrolidin-3-ylmethyl)-N-(4-chloro-benzyl)-3-phenoxy-benzamide; N-((3R*,4R*)-4-benzyl-pyrrolidin-3-ylmethyl)-N-(4-chloro-benzyl)-4-phenoxy-benzamide; ((3S*,4S*)-4-benzyl-pyrrolidin-3-ylmethyl)-(4-chloro-phenyl)-[4-methoxy-3-(3-methoxy-propoxy)-benzyl]-amine; benzofuran-5-carboxylic acid ((3R*,4R*)-4-benzyl-pyrrolidin-3-ylmethyl)-(4-chloro-benzyl)-amide; N-((3R*,4R*)-4-benzyl-pyrrolidin-3-ylmethyl)-4-methoxy-3-(3-methoxy-propoxy)-N-(3-phenyl-propyl)-benzamide; N-((3R*,4R*)-4-benzyl-pyrrolidin-3-ylmethyl)-4-methoxy-3-(3-methoxy-propoxy)-N-phenethyl-benzamide; N-((3R*,4R*)-4-benzyl-pyrrolidin-3-ylmethyl)-4-methoxy-3-(3-methoxy-propoxy)-N-(4-phenyl-butyl)-benzamide; naphthalene-2-carboxylic acid (4-benzyloxy-phenyl)-((3S*,4S*)-4-benzyl-pyrrolidin-3-ylmethyl)-amide; (3-aminomethyl-benzyl)-((3R*,4R*)-4-benzyl-pyrrolidin-3-ylmethyl)-(4-chloro-phenyl)-amine; (8-aminomethyl-naphthalen-2-ylmethyl)-((3R*,4R*)-4-benzyl-pyrrolidin-3-ylmethyl)-(4-chloro-phenyl)-amine; (4-aminomethyl-phenyl)-benzyl-((3R*,4R*)-4-benzyl-pyrrolidin-3-ylmethyl)-amine; (7-aminomethyl-naphthalen-2-ylmethyl)-((3R*,4R*)-4-benzyl-pyrrolidin-3-ylmethyl)-(4-chloro-phenyl)-amine; (4-aminomethyl-naphthalen-1-ylmethyl)-((3R*,4R*)-4-benzyl-pyrrolidin-3-ylmethyl)-(4-chloro-phenyl)-amine; N-(3-{[((3R*,4R*)-4-benzyl-pyrrolidin-3-ylmethyl)-(4-chloro-phenyl)-amino]-methyl}-benzyl)-acetamide; N-(3-{[((3R*,4R*)-4-benzyl-pyrrolidin-3-ylmethyl)-(4-chloro-phenyl)-amino]-methyl}-benzyl)-formamide; ((3R*,4R*)-4-benzyl-pyrrolidin-3-ylmethyl)-(4-chloro-phenyl)-(3-dimethylaminomethyl-benzyl)-amine; ((3R*,4R*)-4-benzyl-pyrrolidin-3-ylmethyl)-(4-chloro-phenyl)-(3-amino-benzyl)-amine; ((3R*4R*)-4-benzyl-pyrrolidin-3-ylmethyl)-isopropyl-[4-methoxy-3-(3-methoxy-propoxy)-benzyl]-amine; N-((3R*,4R*)-4-benzyl-pyrrolidin-3-ylmethyl)-N-isopropyl-4-methoxy-3-(2-methoxy-ethoxy)-benzamide; ((3R*,4R*)4-benzyl-pyrrolidin-3-ylmethyl)-(4-chloro-phenyl)-[2-(3-methoxy-propoxy)-benzyl]-amine; ((3R*,4R*)-4-benzyl-pyrrolidin-3-ylmethyl)-(4-chloro-phenyl)-[2-(2-methoxy-ethoxy)-benzyl]-amine; 2-{[((3R*,4R*)-4-benzyl-pyrrolidin-3-ylmethyl)-(4-chloro-phenyl)-amino]-methyl}-phenol; 1-(3-methoxy-propyl)-1H-indole-6-carboxylic acid ((3R*,4R*)-4-benzyl-pyrrolidin-3-ylmethyl)-isopropyl-amide; 1-(2-methoxyethyl)-1H-indole-6-carboxylic acid ((3R*,4R*)-4-benzyl-pyrrolidin-3-ylmethyl)-isopropyl-amide; benzyl-(4-chloro-phenyl)-((3R*,4R*)-4-phenethyl-pyrrolidin-3-ylmethyl)-amine; ((3S*,4R*)-4-benzyl-pyrrolidin-3-ylmethyl)-(4-chloro-phenyl)-phenyl-amine; N-((3S*,4S*)-4-benzyloxy-pyrrolidin-3-ylmethyl)-N-isopropyl-4-methoxy-3-(3-methoxy-propoxy)-benzamide; N-((3S*,4R*)-4-benzyloxy-pyrrolidin-3-ylmethyl)-N-isopropyl-4-methoxy-3-(3-methoxy-propoxy)-benzamide; N-((3S*,4S*)-4-hydroxy-pyrrolidin-3-ylmethyl)-N-isopropyl-4-methoxy-3-(3-methoxy-propoxy)-benzamide; N-((3S*,4R*)-4-hydroxy-pyrrolidin-3-ylmethyl)-N-isopropyl-4-methoxy-3-(3-methoxy-propoxy)-benzamide; benzyl-((3S*,4R*)-4-benzyloxy-pyrrolidin-3-ylmethyl)-(4-chloro-phenyl)-amine; N-isopropyl-4-methoxy-3-(3-methoxy-propoxy)-N-[(3S*,45*)-4-(4-trifluoromethyl-phenoxy)-pyrrolidin-3-ylmethyl]-benzamide; N-isopropyl-4-methoxy-3-(3-methoxy-propoxy)-N-[(3S*,4R*)-4-(4-trifluoromethyl-phenoxy)-pyrrolidin-3-ylmethyl]-benzamide; benzyl-(4-chloro-phenyl)-[(35*,4R*)-4-(4-trifluoromethyl-phenoxy)-pyrrolidin-3-ylmethyl]-amine; (3R*,4R*)-(4-chloro-phenyl)-[4-(3-isopropyl-phenoxy)-pyrrolidin-3-ylmethyl]-phenyl-amine; (3R*4R*)-benzyl-(4-chloro-phenyl)-[4-(3-isopropyl-phenoxy)-pyrrolidin-3-ylmethyl]-amine; (3R*,4R*)-(4-chloro-phenyl)-[4-(3-isopropyl-phenoxy)-pyrrolidin-3-ylmethyl]-(2-methoxy-benzyl)-amine; (3R*4R*)-(4-chloro-phenyl)-[4-(3-isopropyl-phenoxy)-pyrrolidin-3-ylmethyl]-(3-methoxy-benzyl)-amine; (3R*,4R*)-benzo[1,2,5]oxadiazol-5-ylmethyl-(4-chloro-phenyl)-[4-(3-isopropyl-phenoxy)-pyrrolidin-3-ylmethyl]-amine; N-((3S*,4R*)-4-benzyl-4-hydroxy-pyrrolidin-3-ylmethyl)-N-isopropyl-4-methoxy-3-(3-methoxy-propoxy)-benzamide; N-[(3S*,4R*)-4-(2-fluoro-benzyl)-4-hydroxy-pyrrolidin-3-ylmethyl]-N-isopropyl-4-methoxy-3-(3-methoxy-propoxy)-benzamide; N-isopropyl-4-methoxy-3-(3-methoxy-propoxy)-N-((S)-4-oxo-pyrrolidin-3-ylmethyl)-benzamide; N-((3R*,4R*)-4-cydohexylamino-pyrrolidin-3-ylmethyl)-N-isopropyl-4-methoxy-3-(3-methoxy-propoxy)-benzamide; N-isopropyl-N-((3R*,4R*)-4-isopropylamino-pyrrolidin-3-ylmethyl)-4-methoxy-3-(3-methoxy-propoxy)-benzamide; N-isopropyl-4-methoxy-3-(3-methoxy-propoxy)-N-[(3R*,4R*)-4-(toluene-4-sulfonylamino)-pyrrolidin-3-ylmethyl]-benzamide; N-isopropyl-4-methoxy-N-[(3R*,4R*)-4-(4-methoxy-benzenesulfonylamino)-pyrrolidin-3-ylmethyl]-3-(3-methoxy-propoxy)-benzamide; N-isopropyl-4-methoxy-N-[(3R*,4R*)-4-(3-methoxy-benzenesulfonylamino)-pyrolidin-3-ylmethyl]-3-(3-methoxy-propoxy)-benzamide; N-isopropyl-4-methoxy-3-(3-methoxy-propoxy)-N-[(3R*,4R*)-4-(naphthalene-2-sulfonylamino)-pyrrolidin-3-ylmethyl]-benzamide; N-isopropyl-4-methoxy-3-(3-methoxy-propoxy)-N-((3R*,4R*)-4-phenylmethanesulfonylamino-pyrrolidin-3-ylmethyl)-benzamide; N-((3R*,4R*)-4-benzylamino-pyrrolidin-3-ylmethyl)-N-isopropyl-4-methoxy-3-(3-methoxy-propoxy)-benzamide; [(3R*,4R*)-4-((lsopropyl-[4-methoxy-3-(3-methoxy-propoxy)-benzoyl]-amino)-methyl)-pyrrolidin-3-yl]-carbamicacid isopropyl ester, N-[(3S*,4S*)-4-(cyclopropylcarbamoyl-methyl-amino)-pyrrolidin-3-ylmethyl]-N-isopropyl-4-methoxy-3-(3-methoxy-propoxy)-benzamide; N-((3R*,4R*)-4-benzyl-pyrrolidin-3-ylmethyl)-N-isopropyl-4-methoxy-3-(4-methoxy-butyl)-benzamide; N-((3R*,4R*)-4-benzyl-pyrrolidin-3-ylmethyl)-N-isopropyl-4-methoxy-3-(3-methoxy-propylamino)-benzamide; 1-(3-methoxy-propyl)-3-methyl-1H-indole-6-carboxylic acid ((3R,4R)-4-benzyl-pyrrolidin-3-ylmethyl)-isopropyl-amide; 3-benzyloxy-N-((3S*,4S*)-4-benzyl-pyrrolidin-3-ylmethyl)-N-isopropyl-4-methoxy-benzamide; N-isopropyl-4-methoxy-3-(3-methoxy-propoxy)-N-pyrrolidin-3-ylmethyl-benzamide; N-(2-{[((3R*,4R*)-4-benzyl-pyrrolidin-3-ylmethyl)-(4-chloro-phenyl)-amino]-methyl}-phenyl)-acetamide; N-((3R*,4R*)-4-benzyl-pyrrolidin-3-ylmethyl)-3-(2-ethoxy-ethoxy)-N-isopropyl-4-methoxybenzamide; N-((3S*,4S*)-4-benzyl-pyrrolidin-3-ylmethyl)-N-isopropyl-4-methoxy-3-(2-methoxy-ethoxymethyl)-benzamide; N-((3R*,4R*)-4-benzyl-pyrrolidin-3-ylmethyl)-3-(3-hydroxy-propoxy)-N-isopropyl-4-methoxy-benzamide; 1-(3-methoxy-propyl)-1H-indole-5-carboxylic acid ((3R*,4R*)-4-benzyl-pyrrolidin-3-ylmethyl)-isopropyl-amide; 1-(2-methoxyethyl)-1H-indole-5-carboxylic acid ((3R*,4R*)-4-benzyl-pyrrolidin-3-ylmethyl)-isopropyl-amide; (3R*,4R*)-(4-chloro-phenyl)-[4-(3-isopropyl-phenyl)-pyrrolidin-3-ylmethyl]-phenyl-amine; 138: (3R*,4R*)-benzyl-(4-chloro-3-methoxy-phenyl)-[4-(3-isopropyl-phenyl)-pyrrolidin-3-ylmethyl]-amine; (3R*,4R*)-(4-chloro-3-methoxy-phenyl)-[4-(3-isopropyl-phenyl)-pyrrolidin-3-ylmethyl]-phenyl-amine; N-((3R*,4S*)-4-benzyl-pyrrolidin-3-ylmethyl)-N-isopropyl-4-methoxy-3-(3-methoxy-propoxy)-benzamide; N-isopropyl-4-methoxy-N-[(3S*,4S*)-4-(3-methoxy-benzyl-oxy)-pyrrolidin-3-ylmethyl]-3-(3-methoxy-propoxy)-benzamide; N-isopropyl-4-methoxy-3-(3-methoxy-propoxy)-N-[(3S*,4S*)-4-(naphthalen-2-ylmethoxy)-pyrrolidin-3-ylmethyl]-benzamide; N-[(3S*,4S*)-4-(3,5-dimethoxy-benzyloxy)-pyrrolidin-3-ylmethyl]-N-isopropyl-4-methoxy-3-(3-methoxy-propoxy)-benzamide; N-[(3S*,4S*)-4-(3-ethoxy-benzyloxy)-pyrrolidin-3-ylmethyl]-N-isopropyl-4-methoxy-3-(3-methoxy-propoxy)-benzamide; N-[(3S*,4S*)-4-(3-isopropoxy-benzyloxy)-pyrrolidin-3-ylmethyl]-N-isopropyl-4-methoxy-3-(3-methoxy-propoxy)-benzamide; N-[(3S*,4S*)-4-(3-cyano-benzyloxy)-pyrrolidin-3-ylmethyl]-N-isopropyl-4-methoxy-3-(3-methoxy-propoxy)-benzamide; N-isopropyl-4-methoxy-3-(3-methoxy-propoxy)-N-[(3S,4S)-4-(3-trifluoromethoxy-benzyloxy)-pyrrolidin-3-ylmethyl]-benzamide; N-isopropyl-4-methoxy-3-(3-methoxy-propoxy)-N-[(3S,4S)-4-(3-propoxy-benzyloxy)-pyrrolidin-3-ylmethyl]-benzamide; N-[(3S,4S)-4-(biphenyl-3-ylmethoxy)-pyrrolidin-3-ylmethyl]-N-isopropyl-4-methoxy-3-(3-methoxy-propoxy)-benzamide; N-isopropyl-4-methoxy-3-(3-methoxy-propoxy)-N-[(3S,4S)-4-(3-phenoxy-benzyloxy)-pyrrolidin-3-ylmethyl]-benzamide; N-[(3S*,4S*)-4-(3,5-diethoxy-benzyloxy)-pyrrolidin-3-ylmethyl]-N-isopropyl-4-methoxy-3-(3-methoxy-propoxy)-benzamide; N-isopropyl-4-methoxy-3-(3-methoxy-propoxy)-N-[(3S*,4S*)-4-(5,5,8,8-tetramethyl-5,6,7,8-tetrahydro-naphthalen-2-ylmethoxy)-pyrrolidin-3-ylmethyl]-benzamide; N-[(3S*,4S*)-4-(3,4-dihydro-2H-benzo[b][1,4]dioxepin-7-ylmethoxy)-pyrrolidin-3-ylmethyl]-N-isopropyl-4-methoxy-3-(3-methoxy-propoxy)-benzamide; N-[(3S*,4S*)-4-(7-Cyano-naphthalen-2-ylmethoxy)-pyrrolidin-3-ylmethyl]-N-isopropyl-4-methoxy-3-(3-methoxy-propoxy)-benzamide; N-[(3S*,4S*)-4-(2,3-dimethoxy-benzyloxy)-pyrrolidin-3-ylmethyl]-N-isopropyl-4-methoxy-3-(3-methoxy-propoxy)-benzamide; N-[(3S*,4S*)-4-(3-benzyloxy-benzyloxy)-pyrrolidin-3-ylmethyl]-N-isopropyl-4-methoxy-3-(3-methoxy-propoxy)-benzamide; N-isopropyl-4-methoxy-3-(3-methoxy-propoxy)-N-[(3S*,4S*)4-(3-pyrrol-1-yl-benzyloxy)-pyrrolidin-3-ylmethyl]-benzamide; N-[(3S*,4S*)-4-(benzofuran-5-ylmethoxy)-pyrrolidin-3-ylmethyl]-N-isopropyl-4-methoxy-3-(3-methoxy-propoxy)-benzamide; N-[(3S*,4S*)-4-(2,3-dihydro-benzo[1,4]dioxin-5-ylmethoxy)-pyrrolidin-3-ylmethyl]-N-isopropyl-4-methoxy-3-(3-methoxy-propoxy)-benzamide; N-[(3S*,4S*)-4-(3,4-dimethyl-benzyloxy)-pyrrolidin-3-ylmethyl]-N-isopropyl-4-methoxy-3-(3-methoxy-propoxy)-benzamide; N-[(3S*,4S*)-4.-(3,4-dihydro-2H-benzo[b][1,4]dioxepin-6-ylmethoxy)-pyrrolidin-3-ylmethyl]-N-isopropyl-4-methoxy-3-(3-methoxy-propoxy)-benzamide; N-isopropyl-4-methoxy-3-(3-methoxy-propoxy)-N-[(3S*,4S*)-4-(naphthalen-1-ylmethoxy)-pyrrolidin-3-ylmethyl]-benzamide; N-isopropyl-4-methoxy-N-{(3S,4S)-4-[3-(4-methoxy-phenoxy)-benzyloxy]-pyrrolidin-3-ylmethyl}-3-(3-methoxy-propoxy)-benzamide; N-[(3S*,4S*)-4-(3-benzo[1,3]dioxol-5-yl-benzyloxy)-pyrrolidin-3-ylmethyl]-N-isopropyl-4-methoxy-3-(3-methoxy-propoxy)-benzamide; N-isopropyl-4-methoxy-N-[(3S,4S)-4-(2'-methoxy-biphenyl-3-ylmethoxy)-pyrrolidin-3-ylmethyl]-3-(3-methoxy-propoxy)-benzamide; N-[4-(2-chloro-6-fluoro-benzyl)-4-hydroxy-pyrrolidin-3-ylmethyl]-N-isopropyl-4-methoxy-3-(3-methoxy-propoxy)-benzamide; N-((3R*,4R*)-4-benzylamino-pyrrolidin-3-ylmethyl)-N-isopropyl-4-methoxy-3-(3-methoxy-propoxy)-benzamide; N-isopropyl-4-methoxy-3-(3-methoxy-propoxy)-N-[(3R*,4R*)-4-(1-phenyl-ethylamino)-pyrrolidin-3-ylmethyl]-benzamide; N-((3R*,4R*)-4-isobutylamino-pyrrolidin-3-ylmethyl)-N-isopropyl-4-methoxy-3-(3-methoxy-propoxy)-benzamide; N-((3R*,4R*)-4-cyclobutylamino-pyrrolidin-3-ylmethyl)-N-isopropyl-4-methoxy-3-(3-methoxy-propoxy)-benzamide; N-((3R*,4R*)-4-cyclopentylamino-pyrrolidin-3-ylmethyl)-N-isopropyl-4-methoxy-3-(3-methoxy-propoxy)-benzamide; N-[(3R*,4R*)-4-(cyclopentylmethyl-amino)-pyrrolidin-3-ylmethyl]-N-isopropyl-4-methoxy-3-(3-methoxy-propoxy)-benzamide; N-isopropyl-4-methoxy-3-(3-methoxy-propoxy)-N-((3R*,4R*)-4-phenethylamino-pyrrolidin-3-ylmethyl)-benzamide; and N-isopropyl-4-methoxy-3-(3-methoxy-propoxy)-N-{(3R*,4R*)-4-[(naphthalen-2-ylmethyl)-amino]-pyrrolidin-3-ylmethyl}-benzamide;
or from the compounds of the formulae 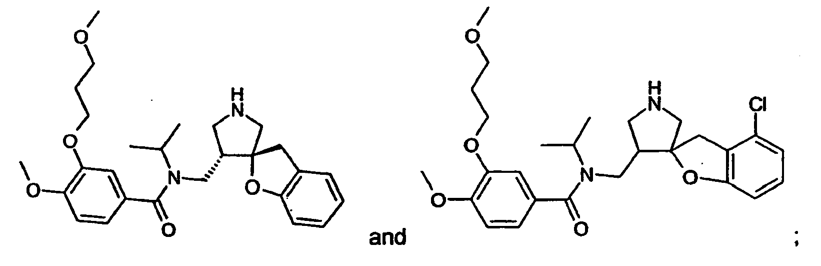 or a pharmaceutically acceptable salt thereof.

15. A compound of the formula I according to claim 1, selected from the group of compounds consisting of
benzyl-carbamic acid (3S*,4S*)-4-({isopropyl-[4-methoxy-3-(3-methoxy-propoxy)-benzoyl]-amino}-methyl)-pyrrolidin-3-yl ester; and
N-isopropyl-4-methoxy-3-(3-methoxy-propoxy)-N-[(3R*,4R*)-4-(naphthalene-2-sulfonylamino)-pyrrolidin-3-ylmethyl]-benzamide;
or a pharmaceutically acceptable salt thereof.

16. A compound of the formula I, or a pharmaceutically acceptable salt thereof, according to any one of claims 1 to 15 for use in the diagnostic or therapeutic treatment of a warm-blooded animal.

17. A compound of the formula I, or a pharmaceutically acceptable salt thereof, according to any one of claims 1 to 15 for use according to claim 14 in the treatment of a disease (= disorder) that depends on activity of renin.

18. The use of a compound of the formula I, or a pharmaceutically acceptable salt thereof, according to any one of claims 1 to 15 for the manufacture of a pharmaceutical composition for the treatment of a disease that depends on activity of renin.

19. A pharmaceutical formulation, comprising a compound of the formula I, or a pharmaceutically acceptable salt thereof, according to any one of claims 1 to 15 and at least one pharmaceutically acceptable carrier material.

20. A process for the manufacture of a compound of the formula I, or a pharmaceutically acceptable salt thereof, according to any one of claims 1 to 15, comprising
A) for the synthesis of a compound of the formula I wherein T is methylene, carbonyl or thiocarbonyl and R¹, R², R³, R⁴ and T have the meanings given above or below for a compound of the formula I, reacting an acid of the formula II, 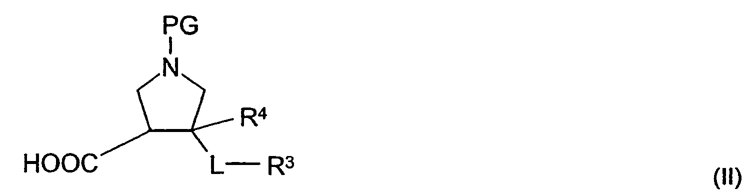 or a reactive derivative thereof, wherein R³, R⁴ and L are as defined for a compound of the formula I an PG is a protecting group, with
(i) an amino compound of the formula III,
R¹R²NH (III)
wherein R¹ and R² are as defined for a compound of the formula I, under condensation conditions and
(a) to obtain a compound of the formula I wherein T is carbonyl and wherein R¹, R², R³, R⁴, L and PG are as defined for compounds of the formula I, removing protecting groups or
(b) , if desired, reducing the carbonyl group in the obtainable compound of the formula IV (a special compound of the formula I), 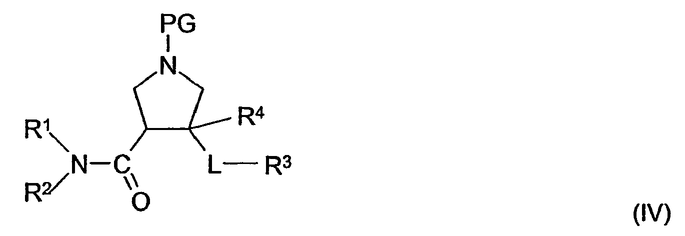 wherein R¹, R², R³, R⁴, L and PG are as defined for compounds of the formula II and III, to a methylene group, and, to obtain a compound of the formula I wherein R¹, R², R³, R⁴, L and PG are as defined for compounds of the formula I and T is methylene, removing protecting groups;
or
(ii) with an amino compound of the formula V,
R²-NH₂ (V)
wherein R¹ is as defined for a compound of the formula I, to give a compound of the formula VI, 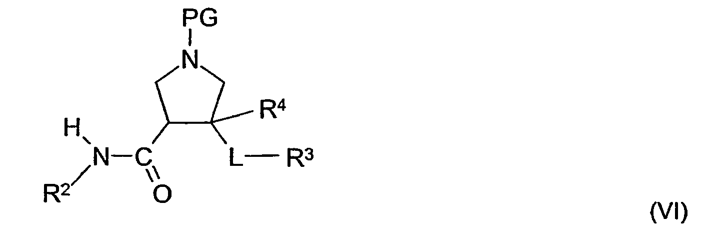 wherein R², R³, R⁴ and L are as defined for a compound for the formula I and PG is a protecting group, and either
(a) reducing the carbonyl group whereby a compound of the formula VII 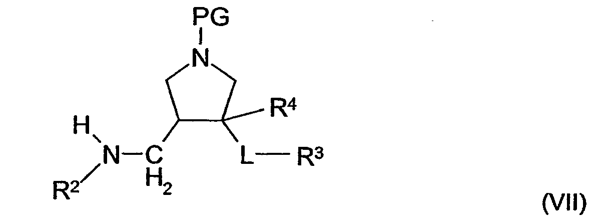 is obtained wherein R², R³, R⁴, L and PG are as defined for a compound of the formula VI, and reacting the compound of the formula VII with a compound of the formula VIII,
R¹-Z (VIII)
wherein R¹ is as defined for a compound of the formula I and Z is a leaving group,
and, to obtain a compound of the formula I wherein T is methylene and R¹, R², R³, R⁴ and L are as defined for a corresponding compound of the formula I, removing protecting groups;
or
(b) reacting the compound of the formula VI with a compound of the formula VIII as defined above and, to obtain a compound of the formula I wherein T is carbonyl and R¹, R², R³, R⁴ and L are as defined for a compound of the formula I, removing protecting groups;
B) for the synthesis of a compound of the formula I wherein T is methylene and R¹, R², R³, R⁴ and T have the meanings given above or below for a compound of the formula I, reacting an aldehyde of the formula IX, 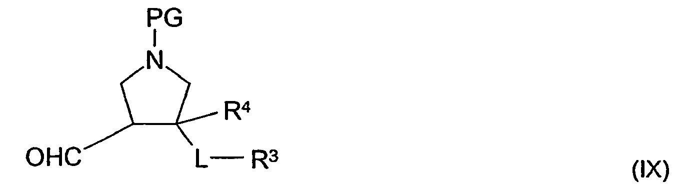 wherein R³, R⁴ and L are as defined for a compound of the formula I and PG is a protecting group, either
(i) with an amino compound of the formula III as defined above under the conditions for reductive amination and, to obtain a compound of the formula I wherein R¹, R², R³, R⁴ and L are as defined for a compound of the formula I and T is methylene, removing protecting groups;
or
(ii) with an amino compound of the formula V as defined above whereby a compound of the formula X 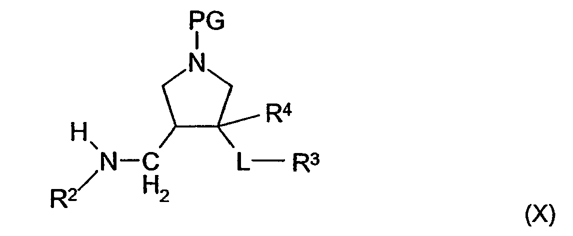 is obtained wherein R², R³, R⁴ and L are as defined for a compound of the formula I and PG is a protecting group, under conditions for reductive amination and then reacting the compound of the formula X
(I) with a compound of the formula VIII as defined above or
(II) for introduction of a moiety R¹ bound vial a methylene group that is part of said R¹, with an aldehyde of the formula VIII*
R¹*-CHO (VIII*)
wherein R¹* is a moiety complementing the moiety R¹*-CH₂- thus obtainable to a corresponding moiety R¹ in the resulting compound, under conditions of reductive amination,
and, to obtain a compound of the formula I wherein T is methylene and R¹, R², R³, R⁴ and L are as defined for a compound of the formula I, removing protecting groups;
C) for the synthesis of a compound of the formula I wherein R¹, R² and T are as defined for a compound of the formula I, R³ is unsubstituted or substituted alkyl, substituted or unsubstituted aryl, unsubstituted or substituted heterocyclyl, unsubstituted or substituted unsubstituted or substituted cycloalkyl, unsubstituted or substituted aryl-alkyl, unsubstituted or substituted heterocyclyl-alkyl, unsubstituted or substituted cycloalkyl-alkyl, substituted or unsubstituted alkylsulfonyl, substituted or unsubstituted arylsulfonyl, substituted or unsubstituted heterocyclylsulfonyl, substituted or unsubstituted cycloalkylsulfonyl, unsubstituted or substituted alkylcarbonyl, unsubstituted or substituted arylcarbonyl, unsubstituted or substituted heterocyclylcarbonyletherified carboxy or N-mono- or N,N-disubstituted amino-sulfonyl, R⁴ is hydrogen and L is oxy, thio or unsubstituted or substituted imino, a compound of the formula XI, 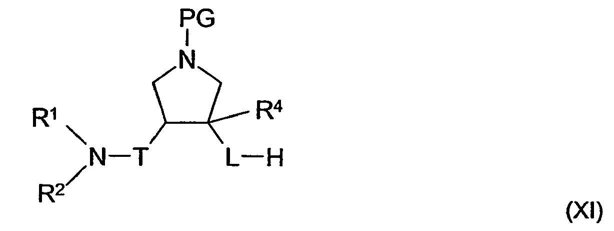 wherein R¹, R², R⁴ and T are as just defined, PG is a protecting group and L is oxy, thio or unsubstituted or substituted imino, is reacted
(i) with a compound of the formula XII,
R³-Z (XII)
wherein Z is a leaving group and R³ is as just defined,
or
(ii) in the case where L is imino or monosubstituted imino, under the conditions of reductive amination with an aldehyde of the formula XIIA
R³*-CHO (XIIA)
wherein R³* is a moiety completing a moiety R³*-CH₂ thus obtainable to a corresponding moiety R³ in the resulting compound,
and, to obtain a corresponding compound of the formula I, removing protecting groups;
D) for the preparation of a compound of the formula I wherein R¹, R² and T are as defined under formula I and R³ and R⁴ together with L form oxo, thioxo or unsubstituted or substituted imino, oxidising a compound of the formula XI as defined above but wherein L is oxy to a corresponding oxo compound of the formula XIII, 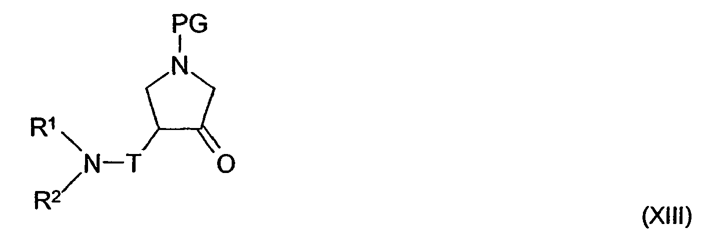 wherein R¹, R² and T are as defined under formula I and, if desired, converting the oxo group to a thioxo or unsubstituted or substituted imino group, and, to obtain a corresponding compound of the formula I, removing the protecting group(s);
E) for the synthesis of a compound of the formula I, wherein R¹, R², L and T are as defined for a compound of the formula I, R³ is unsubstituted or substituted alkyl, substituted or unsubstituted aryl, unsubstituted or substituted heterocyclyl, unsubstituted or substituted cycloalkyl, unsubstituted or substituted aryl-alkyl, unsubstituted or substituted heterocyclyl-alkyl or unsubstituted or substituted cycloalkyl-alkyl, and R⁴ is hydroxy, reacting a compound of the formula XIII as defined above with a metallo reagent of the formula XIV,
R³-L-Mg-Hal (XIV)
wherein R³ is as just defined and Hal is halo, and, to obtain a corresponding compound of the formula I, removing protecting groups;
F) for the synthesis of a spiro compound of the formula I wherein R¹, R² and T are as defined for a compound of the formula I and R³ and R⁴ which then is -O- together with L which then is methylene and the carbon to which R³-L- and R⁴ are bound form a substituted or unsubstituted ring annealed to an unsubstituted or substituted aryl, unsubstituted or substituted heterocyclyl or unsubstituted or substituted cycloalkyl, reacting a compound of the formula XV, 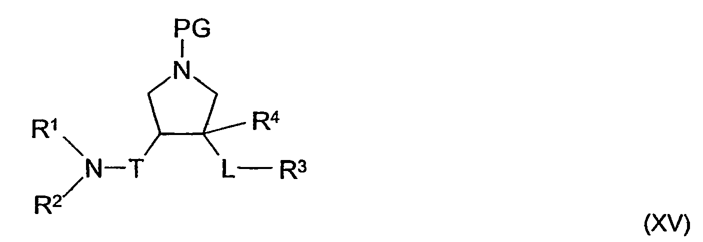 wherein R¹, R² and T are as defined for a compound of the formula I, R³ is substituted or unsubstituted aryl, unsubstituted or substituted heterocyclyl, or unsubstituted or substituted cycloalkyl, each of which carries a leaving group, L is methylene and R⁴ is hydroxy, in the presence of a strong base to obtain a corresponding spiro compound of the formula I, removing protecting groups;
G) for the synthesis of a compound of the formula I wherein R¹, R² and L are as defined for a compound of the formula I, R⁴ is hydrogen, L is oxy, thio or imino and R³ is N-mono-substituted amino-carbonyl, reacting a compound of the formula XI as shown above under C) wherein L is oxy, thio or imino and the other moieties are as described above, with an ioscyanate compound of the formula XIB,
R³**-NCO (XIIB)
wherein R³** is a substitutent completing the corresponding N-mono-substituted amino-carbonyl, and removing protecting groups to obtain the corresponding compound of the formula I; or
H) for the synthesis of a compound of the formula I wherein R¹, R² and T are as defined for a compound of the formula I, L is oxy, thio or unsubstituted or substituted imino and R³ is as defined above, reacting a reactive derivative of a compound of the formula XI as defined above under C), wherein instead of -L-H a leaving group is present, R⁴ is hydrogen and the other moieties are as defined under C), with a compound of the formula XIIC,
R³-L-H (XIIC)
wherein R³ is as defined for a compound of the formula I and L is oxy, thio or unsubstituted or substituted imino, and removing protecting groups to obtain the corresponding compound of the formula I;
and, if desired, subsequent to any one or more of the processes mentioned under (A) to (H) converting an obtainable compound of the formula I or a protected form thereof into a different compound of the formula I, converting a salt of an obtainable compound of formula I into the free compound or a different salt, converting an obtainable free compound of formula I into a salt thereof, and/or separating an obtainable mixture of isomers of a compound of formula I into individual isomers;
where in any of the starting materials, in addition to specific protecting groups mentioned, further protecting groups may be present, and any protecting groups are removed at an appropriate stage in order to obtain the corresponding compound of the formula I, or a salt thereof.

## Patentansprüche

1. Verbindungen der Formel I 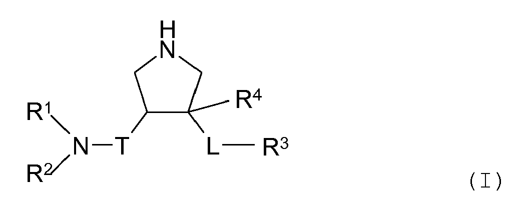 in welcher
R¹ für unsubstituiertes oder substituiertes Aryl, unsubstituiertes oder substituiertes mono- oder bicyclisches Heterocyclyl, unsubstituiertes oder substituiertes Cycloalkyl, unsubstituiertes oder substituiertes Aryl-alkyl, unsubstituiertes oder substituiertes mono- oder bicyclisches Heterocyclyl-alkyl, unsubstituiertes oder substituiertes Cycloalkyl-alkyl, oder Acyl steht;
R² für unsubstituiertes oder substituiertes Alkyl, unsubstituiertes oder substituiertes Aryl, unsubstituiertes oder substituiertes mono- oder bicyclisches Heterocyclyl, unsubstituiertes oder substituiertes Cycloalkyl, unsubstituiertes oder substituiertes Aryl-alkyl, unsubstituiertes oder substituiertes mono- oder bicycliches Heterocyclyl-alkyl oder unsubstituiertes oder substituiertes Cycloalkyl-alkyl steht, mit der Maßgabe, dass, wenn L für Methylen (-CH₂-), Oxy (-O-), Thio (-S-) oder unsubstituiertes (-NH-) oder substituiertes Imino steht, R² aus einer der erwähnten Gruppen und Wasserstoff ausgewählt ist;
R³ für unsubstituiertes oder substituiertes Alkyl, substituiertes oder unsubstituiertes Aryl, unsubstituiertes oder substituiertes Heterocyclyl, unsubstituiertes oder substituiertes Cycloalkyl, unsubstituiertes oder substituiertes Aryl-alkyl, unsubstituiertes oder substituiertes Heterocyclyl-alkyl, unsubstituiertes oder substituiertes Cycloalkyl-alkyl, steht oder, wenn L für Oxy, Thio oder unsubstituiertes oder substituiertes Imino steht, eine der gerade erwähnten Bedeutungen hat oder für unsubstituiertes oder substituiertes Alkylcarbonyl, unsubstituiertes oder substituiertes Arylcarbonyl, unsubstituiertes oder substituiertes Heterocyclylcarbonyl, unsubstituiertes oder substituiertes Cycloalkylcarbonyl, verethertes Carboxyl, Carbamoyl, N-mono- oder N,N-disubstituiertes Amino-carbonyl, substituiertes oder unsubstituiertes Alkylsulfonyl, substituiertes oder unsubstituiertes Arylsulfonyl, substituiertes oder unsubstituiertes Heterocyclylsulfonyl oder substituiertes oder unsubstituiertes Cycloalkylsulfonyl, Sulfamoyl oder N-mono- oder N,N-disubstituiertes Amino-sulfonyl steht;
R⁴ für Wasserstoff oder Hydroxy steht;
L für eine Bindung, Methylen (-CH₂-), Oxy (-O-), Thio (-S-) oder unsubstituiertes (-NH-) oder substituiertes Imino steht, mit der Maßgabe, dass, wenn L für eine Bindung steht, R³ dann für eine der für R³ erwähnten Einheiten mit Ausnahme von substituiertem Alkyl steht; oder R³ und R⁴, die dann für -O- stehen, zusammen mit L, das dann für Methylen steht, und dem Kohlenstoff, an den R³-L- und R⁴ gebunden sind, einen substituierten oder unsubstituierten Ring bilden, der an ein unsubstituiertes oder substituiertes Aryl, unsubstituiertes oder substituiertes Heterocyclyl oder unsubstituiertes oder substituiertes Cycloalkyl kondensiert ist, und so eine spiro-Verbindung der Formel I bilden, oder
R³ und R⁴ zusammen mit L Oxo (=O), Thioxo (=S) oder unsubstituiertes oder substituiertes Imino (=NH) bilden;
und
T für Methylen oder durch Alkyl monosubstituiertes Methylen, Carbonyl (-C(=0)-) oder Thiocarbonyl (-C(=S)-) steht;
wobei bei jedem Vorkommen oben in diesem Anspruch unsubstituiertes oder substituiertes Aryl mono- oder polycyclisch mit 6 bis 22 Kohlenstoffatomen ist und unsubstituiert oder durch eine oder mehrere Einheiten substituiert ist, die unabhängig voneinander ausgewählt sind aus der Gruppe bestehend aus
einem Substituenten der Formel -(C₀-C₇-Alkylen)-(X)ᵣ-(C₁-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- stehen, wobei V für Wasserstoff oder wie unten definiertes unsubstituiertes oder substituiertes Alkyl, Hydroxy-C₁-C₇-alkyl, C₁-C₇-Alkoxy-C₁-C₇-alkyl, wie 3-Methoxypropyl oder 2-Methoxyethyl, C₁-C₇-Alkoxy-C₁-C₇-alkoxy-C₁-C₇-alkyl, C₁-C₇-Alkanoyloxy-C₁-C₇-alkyl, Amino-C₁-C₇-alkyl, wie Aminomethyl, (N)-Mono- oder (N,N)-Di-(C₁-C₇-alkyl) -amino-C₁-C₇-alkyl, C₁-C₇-Alkoxy-C₁-C₇-alkylamino-C₁-C₇-alkyl, Mono-(naphthyl- oder -phenyl)-amino-C₁-C₇-alkyl, Mono- (naphthyl- oder -phenyl-C₁-C₇-alkyl)-amino-C₁-C₇-alkyl, C₁-C₇-Alkanoylamino-C₁-C₇-alkyl, C₁-C₇-Alkyl-O-CO-NH-C₁-C₇-alkyl, C₁-C₇-Alkylsulfonylamino-C₁-C₇-alkyl, C₁-C₇-Alkyl-NH-CO-NH-C₁-C₇-alkyl, C₁-C₇-Alkyl-NH-SO₂-NH-C₁-C₇-alkyl, C₁-C₇-Alkoxy, Hydroxy-C₁-C₇-alkoxy, C₁-C₇-Alkoxy-C₁-C₇-alkoxy, C₁-C₇-Alkanoyloxy, Mono- oder Di-(C₁-C₇-alkyl)-amino, N-Mono-C₁-C₇-alkoxy-C₁-C₇-alkylamino, C₁-C₇-Alkanoylamino, C₁-C₇-Alkylsulfonylamino, C₁-C₇-Alkoxy-carbonyl, Hydroxy-C₁-C₇-alkoxycarbonyl, C₁-C₇-Alkoxy-C₁-C₇-alkoxycarbonyl, Amino-C₁-C₇-alkoxycarbonyl, (N) -Mono- (C₁-C₇-alkyl) -amino-C₁-C₇-alkoxycarbonyl, C₁-C₇-Alkanoylamino-C₁-C₇-alkoxycarbonyl, N-Mono- oder N,N-Di-(C₁-C₇-alkyl)-aminocarbonyl, N-C₁-C₇-Alkoxy-C₁-C₇-alkylcarbamoyl oder N-Mono- oder N,N-Di-(C₁-C₇-alkyl)-aminosulfonyl steht;
aus C₂-C₇-Alkenyl, C₂-C₇-Alkinyl, Phenyl, Naphthyl, Cycloalkyl, Heterocyclyl, Phenyl- oder Naphthyl- oder Heterocyclyl-C₁-C₇-alkyl, wobei Heterocyclyl wie unten definiert ist, Halogen-C₁-C₇-alkyl, Phenyloxy- oder Naphthyloxy-C₁-C₇-alkyl, Cycloalkyl-C₁-C₇-alkyl, Heterocyclyl-C₁-C₇-alkyl, Phenyl-C₁-C₇-alkoxy- oder Naphthyl-C₁-C₇-alkoxy-C₁-C₇-alkyl, Cycloalkyl-C₁-C₇-alkoxy-C₁-C₇-alkyl, Heterocyclyl-C₁-C₇-alkoxy-C₁-C₇-alkyl, Di-(naphthyl- oder -phenyl)-amino-C₁-C₇-alkyl, Mono- oder Di-(heterocyclyl-, -cycloalkyl-, -naphthyl- oder -phenyl)-amino-C₁-C₇-alkyl, Di-(naphthyl- oder -phenyl-C₁-C₇-alkyl)-amino-C₁-C₇-alkyl, Mono- oder Di-(heterocyclyl-, -cycloalkyl-, -naphthyl- oder -phenyl-C₁-C₇-alkyl)-amino-C₁-C₇-alkyl, Benzoyl- oder Naphthoylamino-C₁-C₇-alkyl, Cycloalkyl-CO-amino-C₁-C₇-alkyl, Heterocyclyl-CO-amino-C₁-C₇-alkyl, Phenyl- oder Naphthylsulfonylamino-C₁-C₇-alkyl, wobei Phenyl und Naphthyl unsubstituiert oder durch eine oder mehrere C₁-C₇-Alkyl-Einheiten substituiert sind, Cycloalkylsulfonylamino-C₁-C₇-alkyl, Heterocyclylsulfonylamino-C₁-C₇-alkyl, Phenyl- oder Naphthyl-C₁-C₇-alkylsulfonylamino-C₁-C₇-alkyl, Cycloalkyl-C₁-C₇-alkylsulfonylamino-C₁-C₇-alkyl, Heterocyclyl-C₁-C₇-alkylsulfonylamino-C₁-C₇-alkyl, Carboxy-C₁-C₇-alkyl, Halogen, Hydroxy, Phenyl-C₁-C₇-alkoxy, wobei Phenyl unsubstituiert oder durch C₁-C₇-Alkoxy und/oder Halogen substituiert ist, Halogen-C₁-C₇-alkoxy, Cycloalkyl-C₁-C₇-alkoxy, Heterocyclyl-C₁-C₇-alkoxy, Phenyl- oder Naphthyloxy, Cycloalkyloxy, Heterocyclyloxy, Phenyl- oder Naphthyl-C₁-C₇-alkyloxy, Cycloalkyl-C₁-C₇-alkyloxy, Heterocyclyl-C₁-C₇-alkyloxy, Benzoyl- oder Naphthoyloxy, Halogen-C₁-C₇-alkylthio, Phenyl- oder Naphthylthio, Cycloalkylthio, Heterocyclylthio, Phenyl- oder Naphthyl-C₁-C₇-alkylthio, Cycloalkyl-C₁-C₇-alkylthio, Heterocyclyl-C₁-C₇-alkylthio, Benzoyl- oder Naphthoylthio, Nitro, Amino, Mono- oder Di-(naphthyl- oder -phenyl-C₁-C₇-alkyl)-amino, Mono- oder Di-(heterocyclyl-, -cycloalkyl-, -naphthyl- oder-phenyl-C₁-C₇-alkyl)-amino, Benzoyl- oder Naphthoylamino, Phenyl- oder Naphthylsulfonylamino, wobei Phenyl und Naphthyl unsubstituiert oder durch eine oder mehrere, insbesondere eine bis drei, C₁-C₇-Alkyl-Einheiten substituiert sind, Cycloalkylsulfonylamino, Heterocyclylsulfonylamino, Phenyl- oder Naphthyl-C₁-C₇-alkylsulfonylamino, Cycloalkyl-C₁-C₇-alkylsulfonylamino, Heterocyclyl-C₁-C₇-alkylsulfonylamino, Carboxyl, C₁-C₇-Alkyl-carbonyl, Halogen-C₁-C₇-alkylcarbonyl, Hydroxy-C₁-C₇-alkylcarbonyl, C₁-C₇-Alkoxy-C₁-C₇-alkylcarbonyl, Amino-C₁-C₇-alkylcarbonyl, (N)-Mono- oder (N,N)-Di-(C₁-C₇-alkyl)-amino-C₁-C₇-alkylcarbonyl, C₁-C₇-Alkanoylamino-C₁-C₇-alkylcarbonyl, N-Mono- oder (N,N)-Di-(C₁-C₇-alkyl)-amino-C₁-C₇-alkoxycarbonyl, Halogen-C₁-C₇-alkoxycarbonyl, Phenyl- oder Naphthyloxycarbonyl, Phenyl- oder Naphthyl-C₁-C₇-alkoxycarbonyl, N-Mono- oder (N,N)-Di-(C₁-C₇-alkyl)-amino-C₁-C₇-alkoxycarbonyl, Carbamoyl, N-Mono- oder N,N-Di-(heterocyclyl-, -cycloalkyl-, -naphthyl- oder -phenyl)-aminocarbonyl, N-Mono- oder N,N-Di-(heterocyclyl-, -cycloalkyl-, -naphthyl- oder -phenyl-C₁-C₇-alkyl)-aminocarbonyl, Cyano, C₁-C₇-Alkylen, welches unsubstituiert oder durch bis zu vier C₁-C₇-Alkyl-Substituenten substituiert und an zwei benachbarte Ringatome der Aryl-Einheit gebunden ist, C₂-C₇-Alkenylen oder -alkinylen, welche an zwei benachbarte Ringatome der Aryl-Einheit gebunden sind, Sulfenyl, Sulfinyl, C₁-C₇-Alkylsulfinyl, Phenyl- oder Naphthylsulfinyl, wobei Phenyl und Naphthyl unsubstituiert oder durch eine oder mehrere, insbesondere eine bis drei, C₁-C₇-Alkyl-Einheiten substituiert sind, Cycloalkylsulfinyl, Heterocyclylsulfinyl, Phenyl- oder Naphthyl-C₁-C₇-alkylsulfinyl, Cycloalkyl-C₁-C₇-alkylsulfinyl, Heterocyclyl-C₁-C₇-alkylsulfinyl, Sulfonyl, C₁-C₇-Alkylsulfonyl, Halogen-C₁-C₇-alkylsulfonyl, Hydroxy-C₁-C₇-alkylsulfonyl, C₁-C₇-Alkoxy-C₁-C₇-alkylsulfonyl, Amino-C₁-C₇-alkylsulfonyl, N-Mono- oder (N,N)-Di-(C₁-C₇-alkyl)-amino-C₁-C₇-alkylsulfonyl, C₁-C₇-Alkanoylamino-C₁-C₇-alkylsulfonyl, Phenyl- oder Naphthylsulfonyl, wobei Phenyl und Naphthyl unsubstituiert oder durch eine oder mehrere C₁-C₇-Alkyl-Einheiten substituiert sind, Cycloalkylsulfonyl, Heterocyclylsulfonyl, Phenyl- oder Naphthyl-C₁-C₇-alkylsulfonyl, Cycloalkyl-C₁-C₇-alkylsulfonyl, Heterocyclyl-C₁-C₇-alkylsulfonyl, Sulfamoyl und N-Mono- oder N,N-Di-(C₁-C₇-alkyl-, -phenyl-, -naphthyl, -heterocyclyl, -cycloalkyl, -phenyl-C₁-C₇-alkyl und/oder -naphthyl-C₁-C₇-alkyl, -heterocyclyl-C₁-C₇-alkyl, -cycloalkyl-C₁-C₇-alkyl)-aminosulfonyl;
unsubstituiertes oder substituiertes Heterocyclyl ein mono- oder bicyclisches oder, wenn es nicht Teil eines Substituenten R¹ oder R² ist oder wenn es nicht ein Substituent R¹ und R² ist, weiterhin polycyclisches, vorzugsweise ein mono- oder bicyclisches oder, wenn es nicht Teil eines Substituenten R¹ oder R² ist oder wenn es nicht ein Substituent R¹ und R² ist, ein mono-, bi- oder weiterhin tricyclisches ungesättigtes, teilweise gesättigtes oder gesättigtes Ringsystem ist mit 3 bis 14 Ringatomen und mit einem oder mehreren Heteroatomen, unabhängig voneinander ausgewählt aus Stickstoff (=N-, -NH- oder substituiertem -NH-), Sauerstoff, Schwefel (-S-, S(=O)- oder S-(=O)₂-), welches unsubstituiert oder substituiert ist durch einen oder mehrere Substituenten unabhängig voneinander ausgewählt aus den oben für Aryl erwähnten Substitutenten und aus Oxo, gegebenenfalls ausgewählt aus: 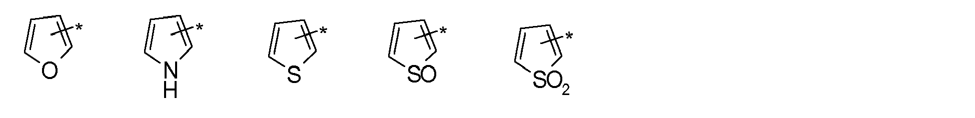 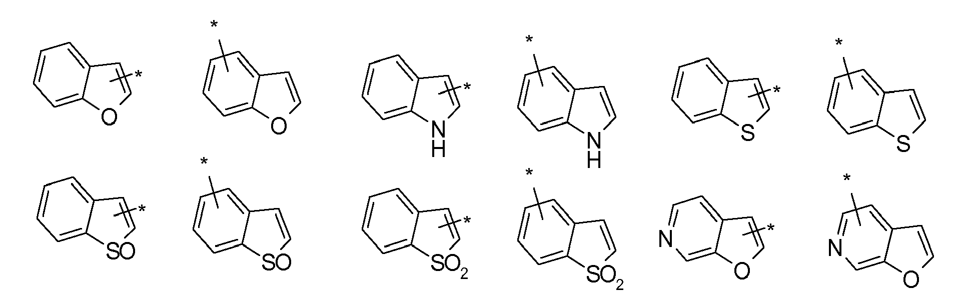 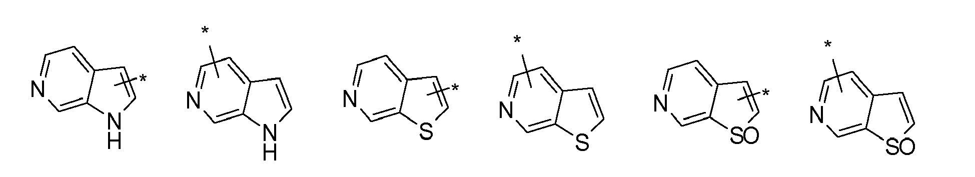 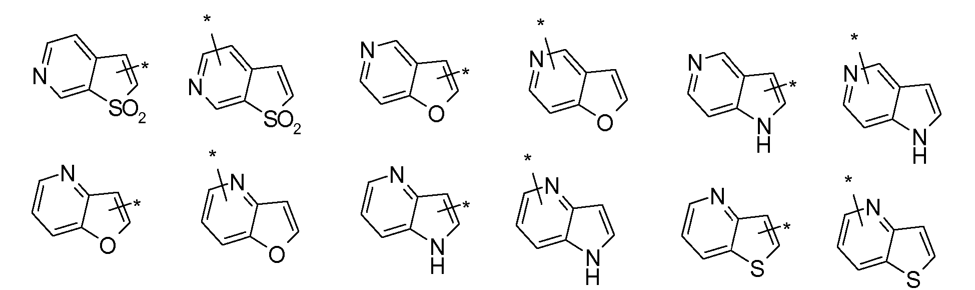 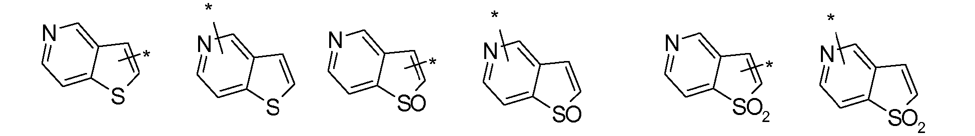 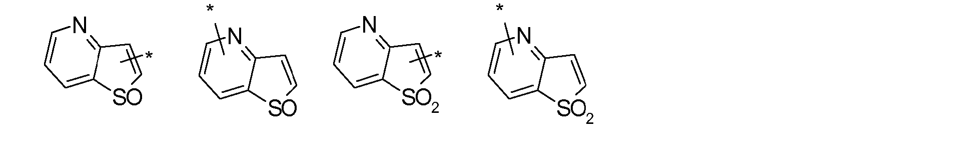 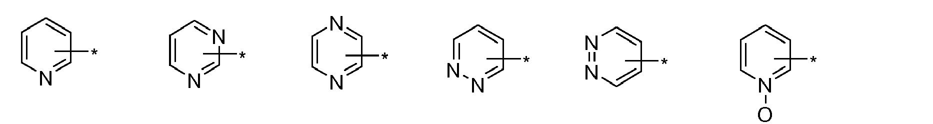 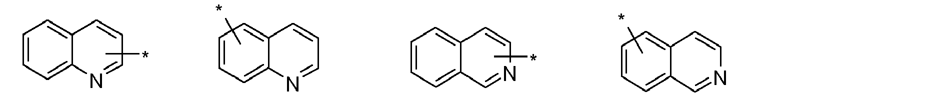 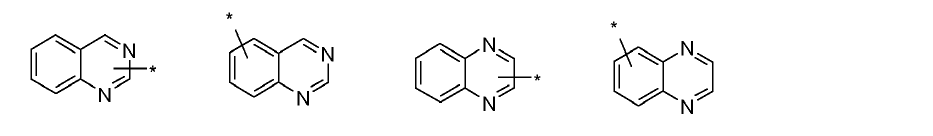 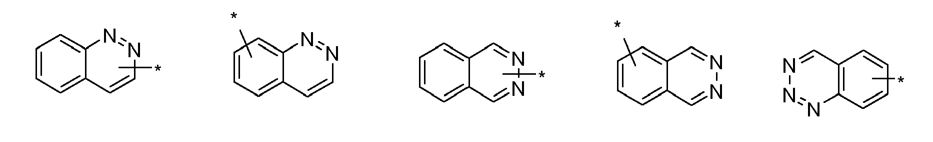 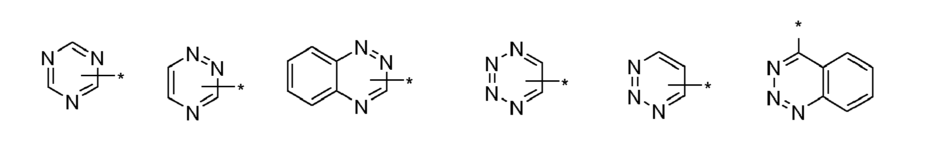 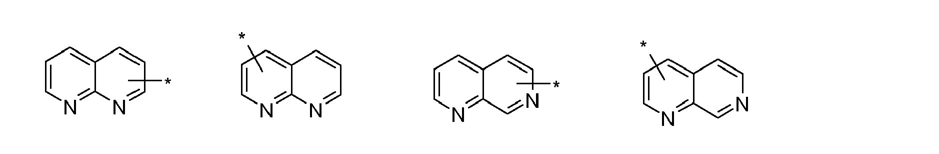 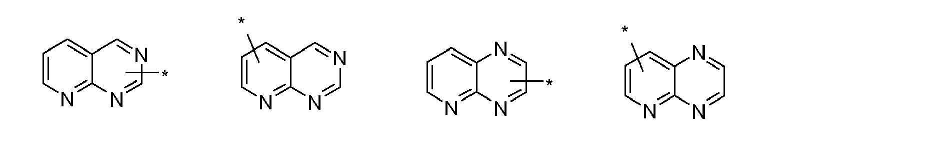 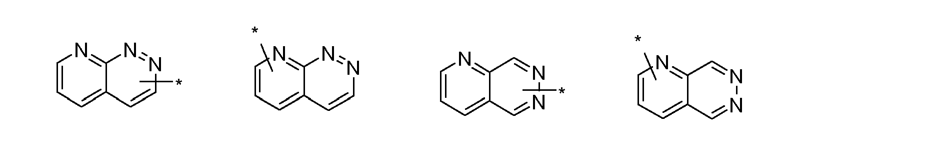 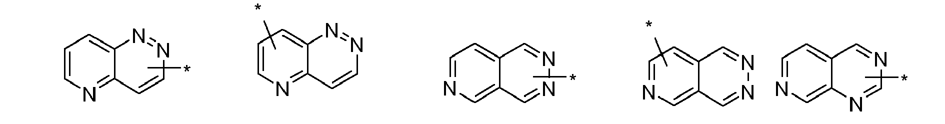 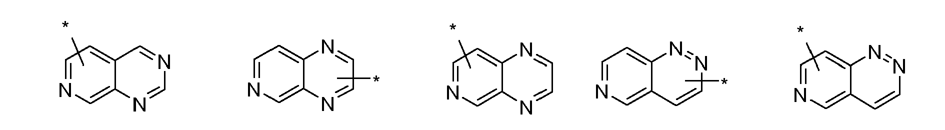 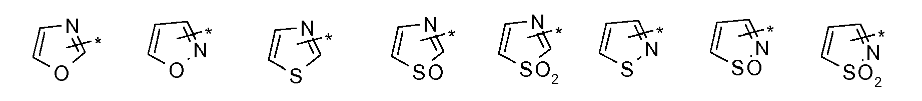 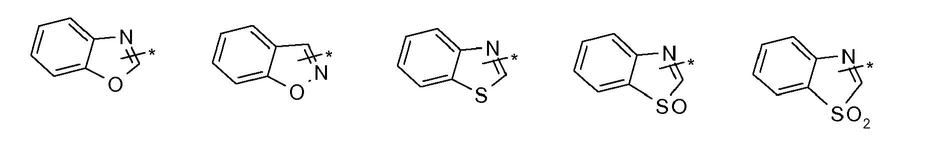 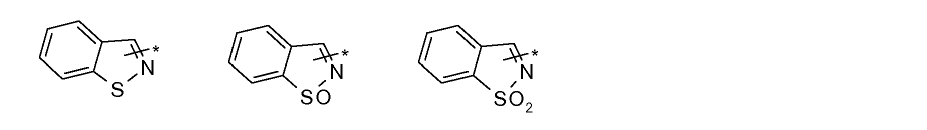 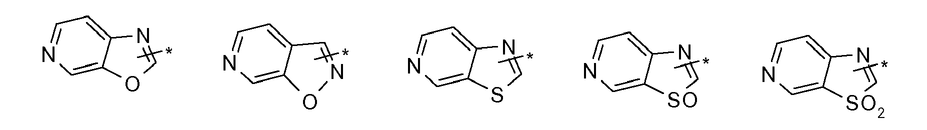 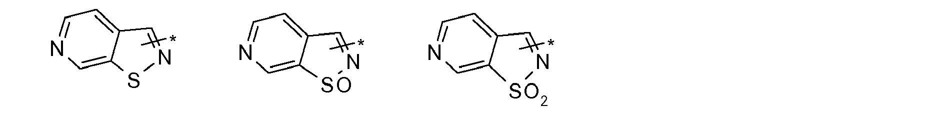 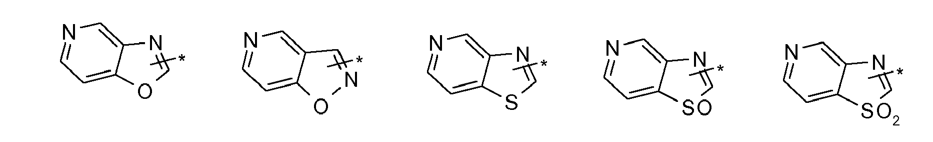 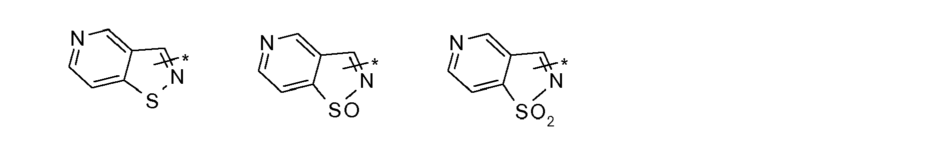 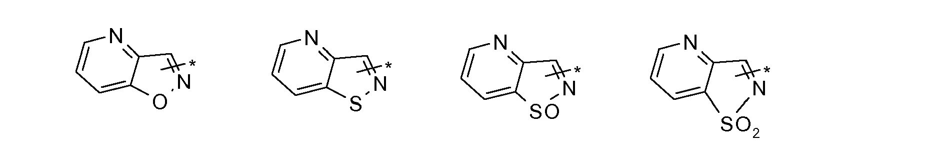 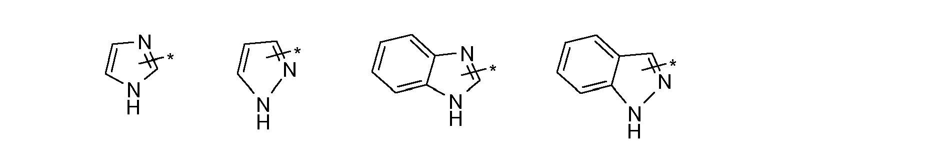 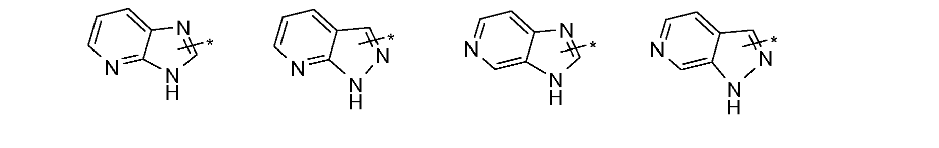 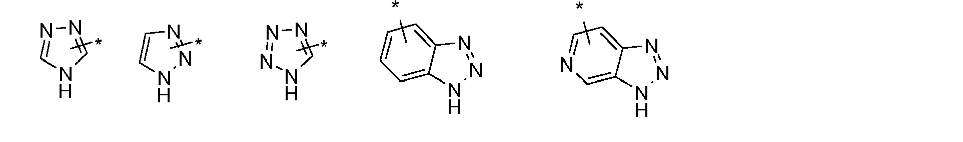 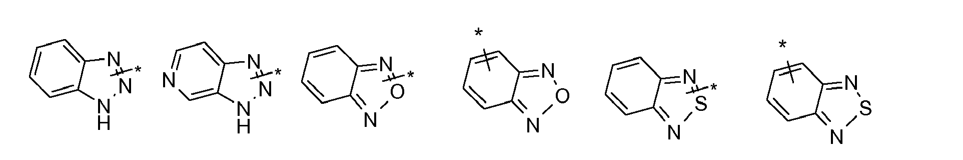 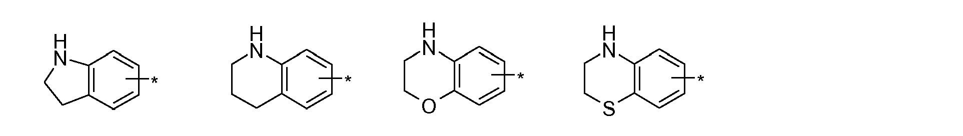 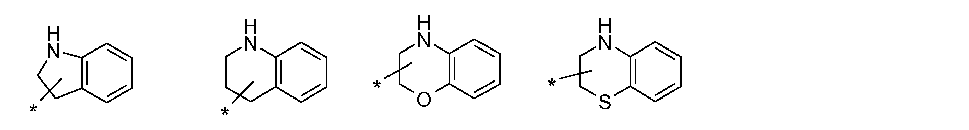 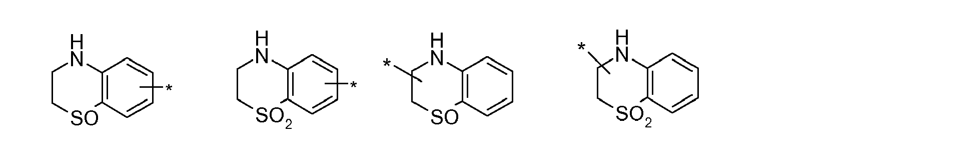 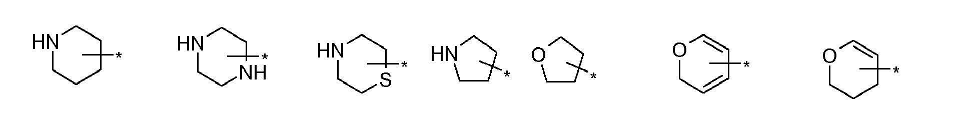 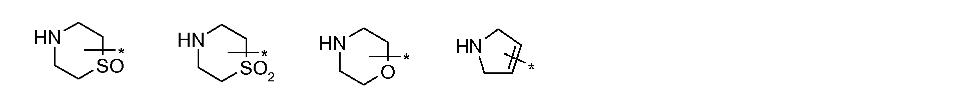 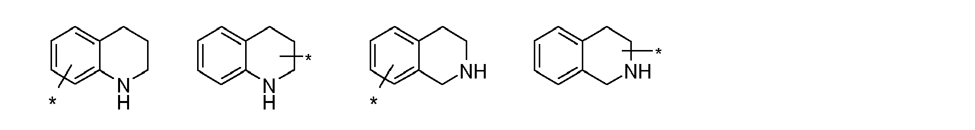 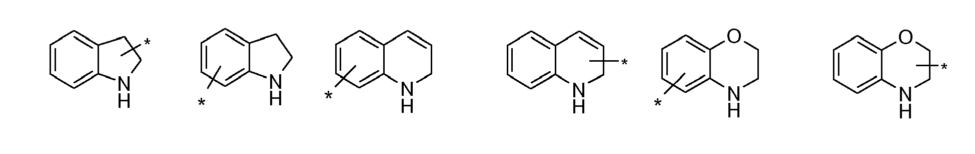 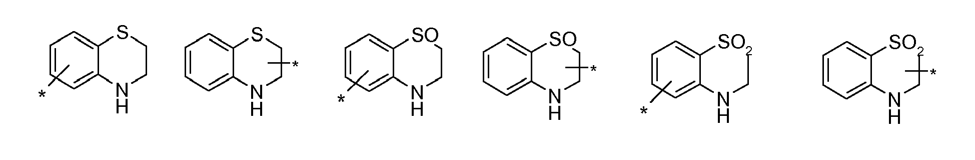 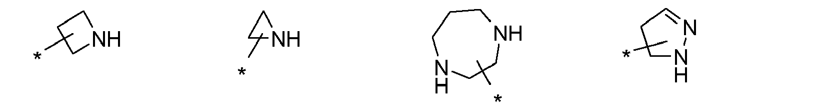 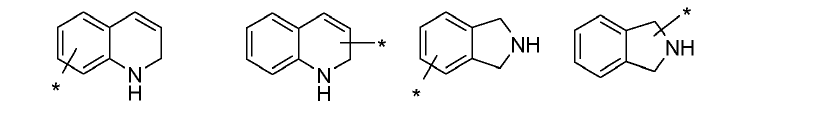 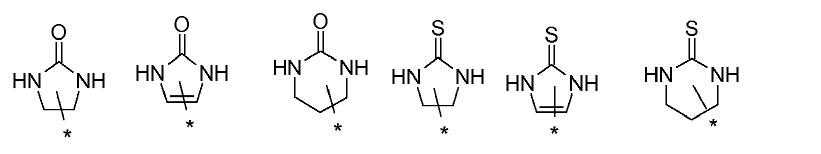 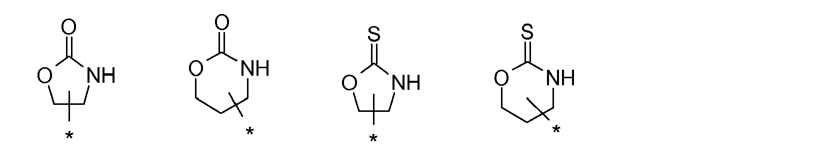 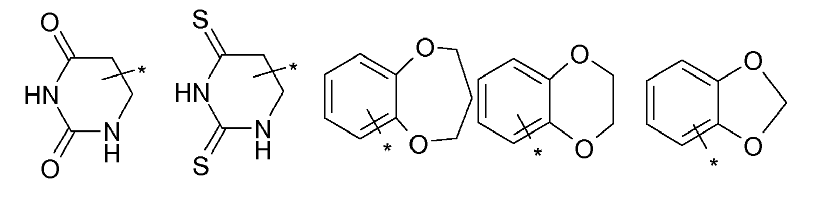 oder, wenn das Heterocyclyl in R³ vorhanden und als unsubstituiertes oder substituiertes Heterocyclyl, unsubstituiertes oder substituiertes Heterocyclyl-alkyl oder substituiertes oder unsubstituiertes Heterocyclylsulfonyl definiert ist, zusätzlich ausgewählt aus 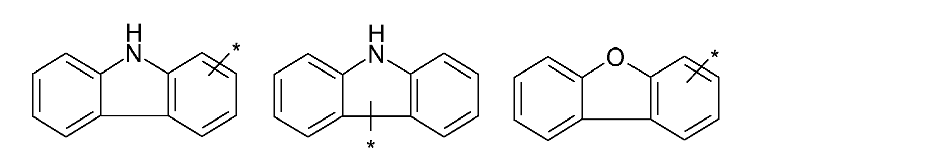 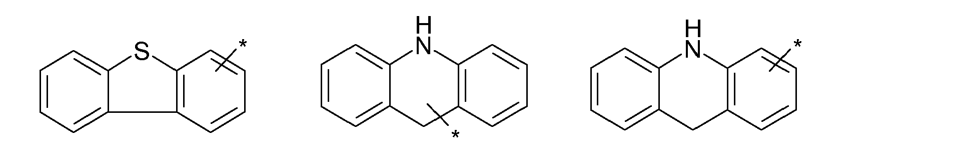 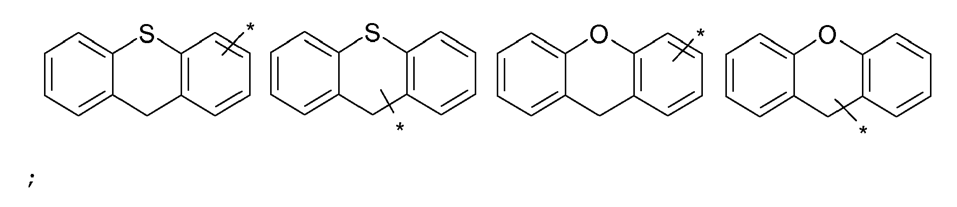 wobei, wenn ein NH vorhanden ist, die Bindung mit dem Sternchen, die die betreffende Heterocyclyl-Einheit an den Rest des Moleküls bindet, das H jeweils durch diese Bindung ersetzt sein kann und/oder das H durch einen wie oben definierten Substituenten ersetzt sein kann,
unsubstituiertes oder substituiertes Cycloalkyl für mono- oder polycyclisches C₃-C₁₀-Cycloalkyl steht, welches eine oder mehrere Doppelbindungen (z.B. in Cycloalkenyl) und/oder Dreifachbindungen (z.B. in Cycloalkinyl) umfassen kann und unsubstituiert oder durch einen oder mehrere Substituenten unabhängig voneinander ausgewählt aus den oben als Substituenten für Aryl erwähnten substituiert ist,
bei unsubstituiertem oder substituiertem Aryl-alkyl Aryl, welches unsubstituiert oder durch einen oder mehrere Substituenten unabhängig voneinander ausgewählt aus den oben als Substituenten für Aryl erwähnten substituiert ist, wie oben für Aryl beschrieben ist und an Alkyl gebunden ist, entweder terminal oder an einem anderen Kohlenstoff in der Alkylkette;
bei unsubstituiertem oder substituiertem Heterocyclyl-alkyl Heterocyclyl wie oben beschrieben ist und unsubstituiert oder durch einen oder mehrere Substituenten unabhängig voneinander ausgewählt aus den oben für substituiertes Aryl erwähnten substituiert ist, und Heterocyclyl an Alkyl gebunden ist, entweder terminal oder an einem anderen Kohlenstoff in der Alkylkette;
bei unsubstituiertem oder substituiertem Cycloalkyl-alkyl Cycloalkyl wie oben beschrieben ist und unsubstituiert oder durch einen oder mehrere Substituenten unabhängig voneinander ausgewählt aus den oben für substituiertes Aryl erwähnten substituiert ist, und Cycloalkyl an Alkyl gebunden ist, entweder terminal oder an einem anderen Kohlenstoff in der Alkylkette;
Acyl für unsubstituiertes oder substituiertes Arylcarbonyl oder -sulfonyl, unsubstituiertes oder substituiertes Heterocyclylcarbonyl oder -sulfonyl, unsubstituiertes oder substituiertes Cycloalkylcarbonyl oder -sulfonyl, Formyl oder unsubstituiertes oder substituiertes Alkylcarbonyl oder -sulfonyl steht, wobei unsubstituiertes oder substituiertes Aryl, unsubstituiertes oder substituiertes Heterocyclyl und unsubstituiertes oder substituiertes Cycloalkyl wie oben definiert sind und unsubstituiertes oder substituiertes Alkyl wie unten beschrieben ist;
unsubstitutiertes oder substituiertes Alkyl für C₁-C₇-Alkyl steht, welches geradkettig oder verzweigt ist und unsubstituiert oder durch eine oder mehrere Einheiten ausgewählt aus wie oben beschriebenem unsubstituiertem oder substituiertem Aryl, wie oben beschriebenem unsubstituiertem oder substituiertem Heterocyclyl; wie oben beschriebenem unsubstituiertem oder substituiertem Cycloalkyl; C₂-C₇-Alkenyl, C₂-C₇-Alkinyl, Halogen, Hydroxy, C₁-C₇-Alkoxy, Halogen-C₁-C₇-alkoxy, Hydroxy-C₁-C₇-alkoxy, C₁-C₇-Alkoxy-C₁-C₇-alkoxy, Phenyl- oder Naphthyloxy, Phenyl- oder Naphthyl-C₁-C₇-alkyloxy, C₁-C₇-Alkanoyloxy, Benzoyl- oder Naphthoyloxy, C₁-C₇-Alkylthio, Halogen-C₁-C₇-alkylthio wie Trifluormethylthio, Hydroxy-C₁-C₇-alkylthio, C₁-C₇-Alkoxy-C₁-C₇-alkylthio, Phenyl- oder Naphthylthio, Phenyl- oder Naphthyl-C₁-C₇-alkylthio, C₁-C₇-Alkanoylthio, Benzoyl- oder Naphthoylthio, Nitro, Amino, Mono- oder Di-(C₁-C₇-alkyl, Hydroxy-C₁-C₇-alkyl und/oder C₁-C₇-Alkoxy-C₁-C₇-alkyl)-amino, Mono- oder Di-(naphthyl- oder -phenyl-C₁-C₇-alkyl) -amino, C₁-C₇-Alkanoylamino, Benzoyl- oder Naphthoylamino, C₁-C₇-Alkylsulfonylamino, Phenyl- oder Naphthylsulfonylamino, wobei Phenyl und Naphthyl unsubstituiert oder durch eine oder mehrere C₁-C₇-Alkyl-Einheiten substituiert sind, Phenyl- oder Naphthyl-C₁-C₇-alkylsulfonylamino, Carboxyl, C₁-C₇-Alkyl-carbonyl, C₁-C₇-Alkoxy-carbonyl, Phenyl- oder Naphthyloxycarbonyl, Phenyl- oder Naphthyl-C₁-C₇-alkoxycarbonyl, Carbamoyl, N-Mono- oder N,N-Di-(C₁-C₇-alkyl)-aminocarbonyl, N-Mono- oder N,N-Di-(naphthyl- oder -phenyl-C₁-C₇-alkyl)-aminocarbonyl, N-Mono- oder N,N-Di-(alkyl-, -naphthyl-, -phenyl-, - heterocyclyl-, -cycloalkyl-, -naphthyl-, -heterocyclyclyl-, -cycloalkyl- oder -phenyl-C₁-C₇-alkyl)-aminocarbonyl, Cyano, C₁-C₇-Alkenylen oder -alkinylen, C₁-C₇-Alkylendioxy, Sulfenyl, Sulfinyl, C₁-C₇-Alkylsulfinyl, Phenyl- oder Naphthylsulfinyl, wobei Phenyl und Naphthyl unsubstituiert oder durch eine oder mehrere C₁-C₇-Alkyl-Einheiten substituiert sind, Cycloalkylsulfinyl, Heterocyclylsulfinyl, Phenyl- oder Naphthyl-C₁-C₇-alkylsulfinyl, Cycloalkyl-C₁-C₇-alkylsulfinyl, Heterocyclyl-C₁-C₇-alkylsulfinyl, Sulfonyl, C₁-C₇-Alkylsulfonyl, Phenyl- oder Naphthylsulfonyl, wobei Phenyl und Naphthyl unsubstituiert oder durch eine oder mehrere C₁-C₇-Alkyl-Einheiten substituiert sind, Cycloalkylsulfonyl, Heterocyclylsulfonyl, Phenyl- oder Naphthyl-C₁-C₇-alkylsulfonyl, Cycloalkyl-C₁-C₇-alkylsulfonyl, Heterocyclyl-C₁-C₇-alkylsulfonyl, Sulfamoyl, N-Mono- oder N,N-Di-(alkyl-, -naphthyl-, - phenyl-, -heterocyclyl-, -cycloalkyl-, -naphthyl-, -heterocyclyclyl-, -cycloalkyl- oder -phenyl-C₁-C₇-alkyl)-aminosulfonyl, N-Mono-, N'-Mono-, N,N-Di- oder N,N,N'-Tri-(C₁-C₇-alkyl, -hydroxy-C₁-C₇-alkyl- und/oder - C₁-C₇-alkoxy-C₁-C₇-alkyl)-aminocarbonylamino und N-Mono-, N'-Mono-, N,N-Di- oder N,N,N'-Tri-(C₁-C₇-alkyl-, - hydroxy-C₁-C₇-alkyl- und/oder -C₁-C₇-alkoxy-C₁-C₇-alkyl)-aminosulfonylamino substituiert ist; bei substituiertem oder unsubstituiertem Alkylsulfonyl substituiertes bzw. unsubstituiertes Alkyl wie oben für unsubstituiertes bzw. substituiertes Alkyl definiert ist;
bei substituiertem oder unsubstituiertem Arylsulfonyl substituiertes bzw. unsubstituiertes Aryl wie oben für unsubstituiertes bzw. substituiertes Aryl definiert ist;
bei substituiertem oder unsubstituiertem Heterocyclylsulfonyl substituiertes bzw. unsubstituiertes Heterocyclyl wie oben für unsubstituiertes bzw. substituiertes Heterocyclyl definiert ist;
bei substituiertem oder unsubstituiertem Cycloalkylsulfonyl unsubstituiertes bzw. substituiertes Cycloalkyl wie oben für unsubstituiertes bzw. substituiertes Cycloalkyl definiert ist;
wenn R³ und R⁴, die dann für -O- stehen, zusammen mit L, das dann für Methylen steht, und dem Kohlenstoff, an den R³-L- und R⁴ gebunden sind, einen substituierten oder unsubstituierten Ring (mit einem oder mehreren Substituenten unabhängig voneinander ausgewählt aus den oben für Aryl erwähnten, vorzugsweise ohne Substituent) bilden, der an ein unsubstituiertes oder substituiertes Aryl, unsubstituiertes oder substituiertes Heterocyclyl oder unsubstituiertes oder substituiertes Cycloalkyl, die jeweils wie oben definiert sind, kondensiert ist, und so eine spiro-Verbindung der Formel I bilden;
bei unsubstituiertem oder substituiertem Imino sowie wo substituierte NH-Gruppen in Heterocyclen vorhanden sind, die Substituenten vorzugsweise ausgewählt sind aus der Gruppe bestehend aus
einem Substituenten der Formel -(C₀-C₇-Alkylen)-(X)ᵣ-(C₁-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-, -CO-NV-; -NV-SO₂-, -SO₂-NV; -NV-CO-NV-, -NV-CO-O-, -O-CO-NV-, -NV-SO₂-NV- stehen,
C₂-C₇-Alkenyl, C₂-C₇-Alkinyl, Cycloalkyl, Phenyl, Naphthyl, Heterocyclyl, Phenyl- oder Naphthyl-C₁-C₇-alkyl, Cycloalkyl-C₁-C₇-alkyl, Heterocyclyl-C₁-C₇-alkyl, wobei Heterocyclyl wie unten definiert ist, Halogen-C₁-C₇-alkyl, Phenyloxy- oder Naphthyloxy-C₁-C₇-alkyl, Cycloalkyloxy-C₁-C₇-alkyl, Heterocyclyloxy-C₁-C₇-alkyl, Phenyl-C₁-C₇-alkoxy- oder Naphthyl-C₁-C₇-alkoxy-C₁-C₇-alkyl, Cycloalkyl-C₁-C₇-alkoxy-C₁-C₇-alkyl, Heterocyclyl-C₁-C₇-alkoxy-C₁-C₇-alkyl, Di-(cycloalkyl-, -heterocyclyl-, -naphthyl- oder -phenyl)-amino-C₁-C₁-alkyl, Di-(cycloalkyl-, -heterocyclyl-, -naphthyl- oder -phenyl-C₁-C₇-alkyl)-amino-C₁-C₇-alkyl, Benzoyl- oder Naphthoylamino-C₁-C₇-alkyl, Phenyl- oder Naphthylsulfonylamino-C₁-C₇-alkyl, wobei Phenyl und Naphthyl unsubstituiert oder durch eine oder mehrere C₁-C₇-Alkyl-Einheiten substituiert sind, Cycloalkylsulfonylamino-C₁-C₇-alkyl, Heterocyclylsulfonylamino-C₁-C₇-alkyl, Phenyl- oder Naphthyl-C₁-C₇-alkylsulfonylamino-C₁-C₇-alkyl, Cycloalkyl-C₁-C₇-alkylsulfonylamino-C₁-C₇-alkyl, Heterocyclyl-C₁-C₇-alkylsulfonylamino-C₁-C₇-alkyl, C₁-C₇-Alkylcarbonyl, Halogen-C₁-C₇-alkylcarbonyl, Hydroxy-C₁-C₇-alkylcarbonyl, C₁-C₇-Alkoxy-C₁-C₇-alkylcarbonyl, Amino-C₁-C₇-alkylcarbonyl, (N)-Mono- oder (N, N) -Di- (C₁-C₇-alkyl)-amino-C₁-C₇-alkylcarbonyl, C₁-C₇-Alkanoylamino-C₁-C₇-alkylcarbonyl, (N,N)-Di-(C₁-C₇-alkyl)-amino-C₁-C₇-alkoxycarbonyl, Halogen-C₁-C₇-alkoxycarbonyl, Phenyl- oder Naphthyloxycarbonyl, Cycloalkyloxycarbonyl, Heterocyclyloxycarbonyl, Phenyl- oder Naphthyl-C₁-C₇-alkoxycarbonyl, Cycloalkyl-C₁-C₇-alkoxycarbonyl, Heterocyclyl-C₁-C₇-alkoxycarbonyl, C₁-C₇-Alkylsulfinyl, Phenyl- oder Naphthylsulfinyl, wobei Phenyl und Naphthyl unsubstituiert oder durch eine oder mehrere C₁-C₇-Alkyl-Einheiten substituiert sind, Cycloalkylsulfinyl, Heterocyclylsulfinyl, Phenyl- oder Naphthyl-C₁-C₇-alkylsulfinyl, Cycloalkyl-C₁-C₇-alkylsulfinyl, Heterocyclyl-C₁-C₇-alkylsulfinyl, Sulfonyl, C₁-C₇-Alkylsulfonyl, Halogen-C₁-C₇-alkylsulfonyl, Hydroxy-C₁-C₇-alkylsulfonyl, C₁-C₇-Alkoxy-C₁-C₇-alkylsulfonyl, Amino-C₁-C₇-alkylsulfonyl, (N,N)-Di-(C₁-C₇-alkyl)-amino-C₁-C₇-alkylsulfonyl, C₁-C₇-Alkanoylamino-C₁-C₇-alkylsulfonyl, Phenyl- oder Naphthylsulfonyl, wobei Phenyl und Naphthyl unsubstituiert oder durch eine oder mehrere C₁-C₇-Alkyl-Einheiten substituiert sind, Cycloalkylsulfonyl, Heterocyclylsulfonyl, Phenyl- oder Naphthyl-C₁-C₇-alkylsulfonyl; Cycloalkyl-C₁-C₇-alkylsulfonyl und
bei unsubstituiertem oder substituiertem Alkylcarbonyl unsubstituiertes bzw. substituiertes Alkyl wie oben definiert ist;
bei unsubstituiertem oder substituiertem Arylcarbonyl, unsubstituiertem oder substituiertem Heterocyclylcarbonyl und unsubstituiertem oder substituiertem Cycloalkylcarbonyl die unsubstituierten bzw. substituierten Aryl-, Heterocyclyl- beziehungsweise Cycloalkyl-Einheiten vorzugsweise wie für die entsprechenden unsubstituierten oder substituierten Aryl-, Heterocyclyl- beziehungsweise Cycloalkyl-Einheiten beschrieben sind;
verethertes Carboxyl für Carbonyl steht, das an L = Oxy oder insbesondere Imino gebunden ist, an das eine Einheit ausgewählt aus unsubstituiertem oder substituiertem Alkyloxy, unsubstituiertem oder substituiertem Aryloxy, unsubstituiertem oder substituiertem Heterocyclyloxy oder unsubstituiertem oder substituiertem Cycloalkyloxy, in welchen die unsubstituierten bzw. substituierten Alkyl-, Aryl-, Heterocyclyl- bzw. Cycloalkyl-Einheiten jeweils wie oben definiert sind, als Bindungsgruppe gebunden ist; besonders bevorzugt ist unsubstituiertes oder substituiertes Alkoxycarbonyl, insbesondere C₁-C₇-Alkoxycarbonyl, das an L = Imino gebunden ist;
N-mono- oder N,N-disubstituiertes Aminocarbonyl für Aminocarbonyl steht, vorzugsweise gebunden an L = Oxy oder Thio, das am Stickstoff mono- oder disubstituiert ist durch eine oder mehrere Einheiten ausgewählt aus unsubstituiertem oder substituiertem Alkyl, unsubstituiertem oder substituiertem Aryl, unsubstituiertem oder substituiertem Heterocyclyl oder unsubstituiertem oder substituiertem Cycloalkyl, die jeweils wie oben definiert sind; ein bevorzugtes Beispiel ist Aryl-C₁-C₇-alkylaminocarbonyl (= Aryl-C₁-C₇-NH-C (=O) -) wie Benzylaminocarbonyl, gebunden an L = Oxy oder weiterhin Thio; und
N-mono- oder N,N-disubstituiertes Aminosulfonyl für Sulfamoyl steht, vorzugsweise gebunden an L = Imino oder insbesondere Oxy, das am Stickstoff mono- oder disubstituiert ist durch eine oder mehrere Einheiten ausgewählt aus unsubstituiertem oder substituiertem Alkyl, unsubstituiertem oder substituiertem Aryl, unsubstituiertem oder substituiertem Heterocyclyl oder unsubstituiertem oder substituiertem Cycloalkyl, die jeweils vorzugsweise wie oben definiert sind; ein bevorzugtes Beispiel ist Aryl-C₁-C₇-alkylaminosulfonyl (= Aryl-C₁-C₇-NH-S(=O)₂-) wie Benzylaminosulfonyl, gebunden an L = Oxy oder weiterhin Imino;
und deren pharmazeutisch unbedenkliche Salze.

2. Verbindungen der Formel I nach Anspruch 1, wobei
R¹ für Phenyl oder Naphthyl, jeweils unsubstituiert oder substituiert durch einen oder mehrere Substituenten ausgewählt aus der Gruppe bestehend aus C₁-C₇-Alkyl, Phenyl, Naphthyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkyl-sulfonylamino-C₁-C₇-alkyl, Halogen, Hydroxy, C₁-C₇-Alkoxy, Hydroxy-C₁-C₇-alkoxy, C₁-C₇-Alkoxy-C₁-C₇-alkoxy, Phenyl- oder Naphthyloxy, Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, Carboxyl, C₁-C₇-Alkoxy-carbonyl, Phenyl- oder Naphthyl-C₁-C₇-alkoxycarbonyl, Carbamoyl, N-Mono- oder N,N-Di-(C₁-C₇-alkyl- und/oder -(phenyl- oder naphthyl)-C₁-C₇-alkyl)-carbamoyl, C₁-C₇-Alkylsulfonyl, unsubstituiertes oder C₁-C₇-alkyl-substitutiertes Phenyl- oder Naphthylsulfonyl, N-Mono- oder N,N-Di-(C₁-C₇-alkyl und/oder -(phenyl- oder -naphthyl)-C₁-C₇-alkyl)-sulfamoyl und Cyano;
Phenyl- oder Naphthyl-C₁-C₇-alkyl, wobei Phenyl und Naphthyl jeweils unsubstituiert sind oder substituiert durch einen oder mehrere Substituenten ausgewählt aus der Gruppe bestehend aus den gerade für substituiertes Phenyl oder Naphthyl erwähnten Substituenten, Pyrrolyl, Furanyl, Thienyl, Pyrimidin-2,4-dion-1-, -2-, -3- oder -5-yl und Benzo[1,3]dioxalyl, die jeweils unsubstituiert sind oder substituiert durch einen oder mehrere Substituenten unabhängig voneinander ausgewählt aus den oben für substituiertes Phenyl- oder Naphthyl-R¹ erwähnten;
Pyrrolyl-C₁-C₇-alkyl, Furanyl-C₁-C₇-alkyl, Thienyl-C₁-C₇-alkyl, Pyrimidin-2,4-dion-1-, -2-, -3- oder -5-yl-C₁-C₇-alkyl, Indolyl-C₁-C₇-alkyl, Benzofuranyl-C₁-C₇-alkyl, Benzimidazolyl-C₁-C₇-alkyl, Benzopyrazolyl-C₁-C₇-alkyl, Chinolinyl-C₁-C₇-alkyl, Isochinolyl-C₁-C₇-alkyl oder Benzo[1,2,5]oxadiazolyl-C₁-C₇-alkyl, die jeweils unsubstituiert sind oder substituiert durch einen oder mehrere Substituenten unabhängig voneinander ausgewählt aus den oben für substituiertes Phenyl- oder Naphthyl-R¹ erwähnten;
C₃-C₁₀-Cycloalkyl, welches unsubstituiert ist oder substituiert durch einen oder mehrere Substituenten unabhängig voneinander ausgewählt aus den oben für substituiertes Phenyl- oder Naphthyl-carbonyl oder Phenyl- oder Naphthyl-C₁-C₇-alkylcarbonyl erwähnten Substituenten, wobei Phenyl und Naphthyl jeweils unsubstituiert sind oder substituiert durch einen oder mehrere Substituenten ausgewählt aus der Gruppe bestehend aus
einem Substituenten der Formel -(C₀-C₇-Alkylen)-(X)ᵣ-(C₁-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- stehen, wobei V für Wasserstoff oder wie unten definiertes unsubstituiertes oder substituiertes Alkyl, Hydroxy-C₁-C₇-alkyl, C₁-C₇-Alkoxy-C₁-C₇-alkyl wie 3-Methoxypropyl oder 2-Methoxyethyl, C₁-C₇-Alkoxy-C₁-C₇-alkoxy-C₁-C₇-alkyl, C₁-C₇-Alkanoyloxy-C₁-C₇-alkyl, Amino-C₁-C₇-alkyl wie Aminomethyl, (N)-Mono- oder (N,N)-Di-(C₁-C₇-alkyl) -amino-C₁-C₇-alkyl, C₁-C₇-Alkoxy-C₁-C₇-alkylamino-C₁-C₇-alkyl, Mono-(naphthyl- oder -phenyl)-amino-C₁-C₇-alkyl, Mono- (naphthyl- oder -phenyl-C₁-C₇-alkyl)-amino-C₁-C₇-alkyl, C₁-C₇-Alkanoylamino-C₁-C₇-alkyl, C₁-C₇-Alkyl-O-CO-NH-C₁-C₇-alkyl, C₁-C₇-Alkylsulfonylamino-C₁-C₇-alkyl, C₁-C₇-Alkyl-NH-CO-NH-C₁-C₇-alkyl, C₁-C₇-Alkyl-NH-SO₂-NH-C₁-C₇-alkyl, C₁-C₇-Alkoxy, Hydroxy-C₁-C₇-alkoxy, C₁-C₇-Alkoxy-C₁-C₇-alkoxy, C₁-C₇-Alkanoyloxy, Mono- oder Di-(C₁-C₇-alkyl)-amino, Mono- oder Di-(naphthyl- oder -phenyl-C₁-C₇-alkyl)-amino, N-Mono-C₁-C₇-alkoxy-C₁-C₇-alkylamino, C₁-C₇-Alkanoylamino, C₁-C₇-Alkylsulfonylamino, C₁-C₇-Alkoxy-carbonyl, Hydroxy-C₁-C₇-alkoxycarbonyl, C₁-C₇-Alkoxy-C₁-C₇-alkoxycarbonyl, Amino-C₁-C₇-alkoxycarbonyl, (N) -Mono- (C₁-C₇-alkyl) -amino-C₁-C₇-alkoxycarbonyl, C₁-C₇-Alkanoylamino-C₁-C₇-alkoxycarbonyl, N-Mono- oder N,N-Di-(C₁-C₇-alkyl)-aminocarbonyl, N-C₁-C₇-Alkoxy-C₁-C₇-alkylcarbamoyl oder N-Mono- oder N,N-Di-(C₁-C₇-alkyl)-aminosulfonyl steht;
oder
aus Phenyl- oder Naphthyl-C₁-C₇-alkyl, Halogen-C₁-C₇-alkyl, Halogen, Hydroxy, Phenyl- oder Naphthyloxy, Phenyl- oder Naphthyl-C₁-C₇-alkyloxy, Nitro, Amino, Amino-C₁-C₇-alkyl, Carboxyl, Phenyl- oder Naphthyl-C₁-C₇-alkoxycarbonyl, Halogen-C₁-C₇-alkoxycarbonyl, C₁-C₇-Alkylsulfonyl, Carbamoyl und Cyano; Heterocyclylcarbonyl, jeweils unsubstituiert oder substituiert durch einen oder mehrere Substituenten unabhängig voneinander ausgewählt aus einem Substituenten der Formel -(C₀-C₇-Alkylen)-(X)ᵣ-(C₁-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- stehen, wobei V für Wasserstoff oder wie unten definiertes unsubstituiertes oder substituiertes Alkyl, Hydroxy-C₁-C₇-alkyl, C₁-C₇-Alkoxy-C₁-C₇-alkyl, C₁-C₇-Alkoxy-C₁-C₇-alkoxy-C₁-C₇-alkyl, C₁-C₇-Alkanoyloxy-C₁-C₇-alkyl, Amino-C₁-C₇-alkyl, (N)-Mono- oder (N,N)-Di-(C₁-C₇-alkyl)-amino-C₁-C₇-alkyl, C₁-C₇-Alkoxy-C₁-C₇-alkylamino-C₁-C₇-alkyl, Mono-(naphthyl- oder -phenyl)-amino-C₁-C₇-alkyl, Mono-(naphthyl- oder - phenyl-C₁-C₇-alkyl)-amino-C₁-C₇-alkyl, C₁-C₇-Alkanoylamino-C₁-C₇-alkyl, C₁-C₇-Alkyl-O-CO-NH-C₁-C₇-alkyl, C₁-C₇-Alkylsulfonylamino-C₁-C₇-alkyl, C₁-C₇-Alkyl-NH-CO-NH-C₁-C₇-alkyl, C₁-C₇-Alkyl-NH-SO₂-NH-C₁-C₇-alkyl, C₁-C₇-Alkoxy, Hydroxy-C₁-C₇-alkoxy, C₁-C₇-Alkoxy-C₁-C₇-alkoxy, C₁-C₇-Alkanoyloxy, Mono- oder Di-(C₁-C₇-alkyl)-amino, Mono- oder Di-(naphthyl- oder -phenyl-C₁-C₇-alkyl)-amino, N-Mono-C₁-C₇-alkoxy-C₁-C₇-alkylamino, C₁-C₇-Alkanoylamino, C₁-C₇-Alkylsulfonylamino, C₁-C₇-Alkoxy-carbonyl, Hydroxy-C₁-C₇-alkoxycarbonyl, C₁-C₇-Alkoxy-C₁-C₇-alkoxycarbonyl, Amino-C₁-C₇-alkoxycarbonyl, (N) -Mono- (C₁-C₇-alkyl) -amino-C₁-C₇-alkoxycarbonyl, C₁-C₇-Alkanoylamino-C₁-C₇-alkoxycarbonyl, N-Mono- oder N,N-Di-(C₁-C₇-alkyl)-aminocarbonyl, N-C₁-C₇-Alkoxy-C₁-C₇-alkylcarbamoyl oder N-Mono- oder N,N-Di-(C₁-C₇-alkyl)-aminosulfonyl steht;
oder
aus Phenyl- oder Naphthyl-C₁-C₇-alkyl, Halogen-C₁-C₇-alkyl, Halogen, Hydroxy, Phenyl- oder Naphthyloxy, Phenyl- oder Naphthyl-C₁-C₇-alkyloxy, Nitro, Amino, Amino-C₁-C₇-alkyl, Carboxyl, Phenyl- oder Naphthyl-C₁-C₇-alkoxycarbonyl, Halogen-C₁-C₇-alkoxycarbonyl, C₁-C₇-Alkylsulfonyl, Carbamoyl und Cyano;
oder Phenyl- oder Naphthyl-sulfonyl, wobei Phenyl und Naphthyl jeweils unsubstituiert sind oder substituiert durch einen oder mehrere Substituenten ausgewählt aus den oben für substituiertes Phenyl- oder Naphthyl-R¹ erwähnten, steht;
R² für
C₁-C₇-Alkyl, das unsubstituiert ist oder substituiert durch einen oder mehrere Substituenten ausgewählt aus der Gruppe bestehend aus Halogen, Phenyl- oder Naphthyl, Hydroxy, C₁-C₇-Alkoxy, Amino, Mono- oder Di-(C₁-C₇-alkyl)-amino, C₁-C₇-Alkanoylamino, C₁-C₇-Alkylsulfonylamino, Phenyl- oder Naphthylsulfonylamino, Phenyl- oder Naphthyl-C₁-C₇-alkylsulfonylamino, C₁-C₇-Alkoxy-C₁-C₇-alkoxy, Hydroxy-C₁-C₇-alkoxy, Phenyl- oder Naphthyloxy, Phenyl- oder Naphthyl-C₁-C₇-alkyloxy, C₁-C₇-Alkanoyloxy, Nitro, Carboxyl, C₁-C₇-Alkoxy-carbonyl, Phenyl- oder Naphthyl-C₁-C₇-alkoxycarbonyl, Carbamoyl, N-Mono- oder N,N-Di-(C₁-C₇-alkyl-, -phenyl-, -naphthyl-, -phenyl-C₁-C₇-alkyl- oder -naphthyl-C₁-C₇-alkyl)-carbamoyl und N-Mono- oder N,N-Di-(C₁-C₇-alkyl-, -phenyl-, -naphthyl-, -phenyl-C₁-C₇-alkyl- oder -naphthyl-C₁-C₇-alkyl)-sulfamoyl; und Cyano;
Phenyl oder Naphthyl, jeweils unsubstituiert oder substituiert durch einen oder mehrere Substituenten ausgewählt aus der Gruppe bestehend aus C₁-C₇-Alkyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkylsulfonylamino-C₁-C₇-alkyl, Halogen, Hydroxy, C₁-C₇-Alkoxy, C₁-C₇-Alkoxy-C₁-C₇-alkoxy, Hydroxy-C₁-C₇-alkoxy, Phenyl- oder Naphthyloxy, Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-alkanoylamino, Carboxyl, C₁-C₇-alkoxycarbonyl, Halogen-C₁-C₇-alkoxycarbonyl, Phenyl- oder Naphthyl-C₁-C₇-alkoxycarbonyl, Carbamoyl, N-Mono- oder N,N-Di-(C₁-C₇-alkyl-, -phenyl-, -naphthyl-, -phenyl-C₁-C₇-alkyl- oder -naphthyl-C₁-C₇-alkyl)-carbamoyl und N-Mono- oder N,N-Di-(C₁-C₇-alkyl-, -phenyl-, -naphthyl-, -phenyl-C₁-C₇-alkyl- oder -naphthyl-C₁-C₇-alkyl)-sulfamoyl und Cyano;
Phenyl- oder Naphthyl-C₁-C₇-alkyl, wobei Phenyl und Naphthyl jeweils unsubstituiert sind oder substituiert durch einen oder mehrere Substituenten ausgewählt aus der gerade für substituiertes Phenyl- oder Naphthyl-R² erwähnten Gruppe;
C₃-C₁₀-Cycloalkyl, welches unsubstituiert ist oder substituiert durch einen oder mehrere Substituenten ausgewählt aus der gerade für substituiertes Phenyl- oder Naphthyl-R² erwähnten Gruppe; C₃-C₁₀-Cycloalkyl-C₁-C₇-alkyl, wobei Cycloalkyl unsubstituiert ist oder substituiert durch einen oder mehrere Substituenten ausgewählt aus der gerade für substituiertes Phenyl- oder Naphthyl-R² erwähnten Gruppe;
oder Pyrrolyl, Furanyl oder Thienyl steht, oder, wenn L für Methylen, Oxy, Thio oder Imino steht, R² ausgewählt ist aus einer der gerade erwähnten Gruppen von R²-Einheiten,und aus Wasserstoff;
R³ für
C₁-C₇-Alkyl, Carbamoyl-C₁-C₇-alkyl, N-Mono- oder N,N-Di-(C₃-C₈-cycloalkyl-, -heterocyclyl-, -phenyl-, -naphthyl-, -C₁-C₇-alkyl-, -C₃-C₈-cycloalkyl-C₁-C₇-alkyl-, -heterocyclyl-C₁-C₇-alkyl-, -phenyl-C₁-C₇-alkyl- und/oder -naphthyl-C₁-C₇-alkyl)-aminocarbonyl-C₁-C₇- alkyl; Phenyl, Naphthyl, Phenyl-C₁-C₇-alkyl oder Naphthyl-C₁-C₇-alkyl, wobei Phenyl und Naphthyl jeweils unsubstituiert sind oder substituiert durch einen oder mehrere Substituenten ausgewählt aus der Gruppe bestehend aus Phenyl, Naphthyl, C₁-C₇-Alkyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkyl-sulfonylamino-C₁-C₇-alkyl, Phenyl, Naphthyl, Mono- oder Di-(C₁-C₇-alkoxy)-phenyl oder -naphthyl, C₁-C₇-Alkylendioxy-phenyl, wobei die Oxy-Atome an benachbarte Phenyl-Ringatome gebunden sind, Halogen, Hydroxy, C₁-C₇-Alkoxy, Halogen-C₁-C₇-alkoxy, Hydroxy-C₁-C₇-alkoxy, C₁-C₇-Alkoxy-C₁-C₇-alkoxy, Phenyl- oder Naphthyloxy, (unsubstituiertes oder mono-, di- oder tri-C₁-C₇-alkyl-substituiertes) Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, C₁-C₇-Alkylsulfonylamino, Carboxyl, C₁-C₇-Alkoxy-carbonyl, Phenyl- oder Naphthyl-C₁-C₇-alkoxycarbonyl, Carbamoyl und Cyano, C₁-C₇-Alkylsulfonyl, C₁-C₇-Alkylen, welches unsubstituiert oder durch bis zu vier C₁-C₇-Alkyl-Substituenten substituiert und an zwei benachbarte Ringatome der Phenyl- bzw. Naphthyl-Einheit gebunden ist; Pyrrolyl, Furanyl und Thienyl;
Phenyl- oder Naphthyl-sulfonyl oder Phenyl-C₁-C₇-alkyl- oder Naphthyl-C₁-C₇-alkyl-sulfonyl, wobei Phenyl und Naphthyl jeweils unsubstituiert oder durch einen oder mehrere Substituenten ausgewählt aus der gerade für substituiertes Phenyl- oder Naphthyl-R³ beschriebenen Gruppe substituiert sind,
C₃-C₁₀-Cycloalkyl oder C₃-C₁₀-Cyclyoalkyl-C₁-C₇-alkyl, wobei in beiden Cycloalkyl unsubstituiert ist oder substituiert durch einen oder mehrere der gerade für substituiertes Phenyl- oder Naphthyl-R³ erwähnten Substituenten;
oder Heterocyclyl oder Heterocyclyl-C₁-C₇-alkyl, wobei Heterocyclyl ausgewählt ist aus Pyrrolyl, Furanyl, Thienyl, Pyrimidin-2,4-dion-1-, -3- oder -5-yl, Indolyl, Benzofuranyl, Benzimidazolyl, Benzopyrazolyl, Chinolinyl, Isochinolinyl, Methylen-dioxy-phenyl, Ethylen-1,2-dioxy-phenyl oder Trimethylen-1,3-dioxy-phenyl, wobei die Oxy-Gruppen an benachbarte Ringatome des Phenylrings gebunden sind, wobei die Heterocyclyl-Einheiten jeweils unsubstituiert oder wie oben für substituiertes Phenyl-R³ beschrieben substituiert sind, steht;
oder, wenn L für Imino, Oxy oder Thio steht, alternativ für Phenyl- oder Naphthylcarbonyl, C₁-C₇-Alkoxycarbonyl (was C₁-C₇-Alkyl-O-C(=O)- bedeutet), Phenyloxycarbonyl, Naphthyloxycarbonyl, Cycloalkyloxycarbonyl, Heterocyclyloxycarbonyl, Phenyl-C₁-C₇-alkyloxycarbonyl, Naphthyl-C₁-C₇-alkyloxycarbonyl, Cycloalkyl-C₁-C₇-alkoxycarbonyl, Heterocyclyl-C₁-C₇-alkoxycarbonyl oder N-Mono- oder N,N-Di-(alkyl-, -naphthyl-, -phenyl-, -heterocyclyl-, -cycloalkyl-, -naphthyl-, -heterocyclyclyl-, - cycloalkyl- oder -phenyl-C₁-C₇-alkyl)-aminocarbonyl stehen kann, wobei die Phenyl- und Naphthylringe jeweils unsubstituiert oder wie oben für substituiertes Phenyl- bzw. Naphthyl-R³ erwähnt substituiert sind;
und
R⁴ für Wasserstoff oder Hydroxy steht; und
L für eine Bindung, Methylen (-CH₂-), Oxy (-O-) oder Imino (-NH-) steht;
R³ und R⁴, die dann für -O- stehen, zusammen mit L, das dann für Methylen steht, und dem Kohlenstoff, an den R³-L- und R⁴ gebunden sind, einen substituierten oder unsubstituierten 5-gliedrigen Ring bilden, der an Benzo kondensiert ist, wobei Benzo durch einen oder mehrere Substituenten ausgewählt aus der Gruppe bestehend aus C₁-C₇-Alkyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, Halogen, Hydroxy, C₁-C₇-Alkoxy, C₁-C₇-Alkoxy-C₁-C₇-alkoxy, Phenyl- oder Naphthyloxy, Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, Amino-C₁-C₇-alkyl, Mono- oder Di-(C₁-C₇-alkyl)-amino-C₁-C₇-alkyl, C₁-C₇-Alkanoylamino-C₁-C₇-alkyl, Carboxyl, C₁-C₇-Alkoxy-carbonyl, Phenyl- oder Naphthyl-C₁-C₇-alkoxycarbonyl, Carbamoyl und Cyano substituiert oder unsubstituiert ist, und so eine spiro-Verbindung der Formel I bilden, oder
R³ und R⁴ zusammen mit L Oxo (=O) bilden;
und T für Methylen, Carbonyl oder Thiocarbonyl steht;
und deren pharmazeutisch unbedenkliche Salze.

3. Verbindungen der Formel I nach Anspruch 1 oder 2, wobei
R¹ für
Phenyl- oder Naphthyl-carbonyl oder Phenyl- oder Naphthyl-C₁-C₇-alkylcarbonyl steht, wobei Phenyl und Naphthyl jeweils unsubstituiert sind oder substituiert durch einen oder mehrere Substituenten ausgewählt aus der Gruppe bestehend aus
einem Substituenten der Formel -(C₀-C₇-alkylen)-(X)ᵣ-(C₁-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- stehen, wobei V für Wasserstoff oder wie unten definiertes unsubstituiertes oder substituiertes Alkyl, Hydroxy-C₁-C₇-alkyl, C₁-C₇-Alkoxy-C₁-C₇-alkyl, C₁-C₇-Alkoxy-C₁-C₇-alkoxy-C₁-C₇-alkyl, C₁-C₇-Alkanoyloxy-C₁-C₇-alkyl, Amino-C₁-C₇-alkyl, (N)-Mono- oder (N,N)-Di-(C₁-C₇-alkyl)-amino-C₁-C₇-alkyl, C₁-C₇-Alkoxy-C₁-C₇-alkylamino-C₁-C₇-alkyl, Mono- (naphthyl- oder -phenyl)-amino-C₁-C₇-alkyl, Mono-(naphthyl- oder -phenyl-C₁-C₇-alkyl)-amino-C₁-C₇-alkyl, C₁-C₇-Alkanoylamino-C₁-C₇-alkyl, C₁-C₇-Alkyl-O-CO-NH-C₁-C₇-alkyl, C₁-C₇-Alkylsulfonylamino-C₁-C₇-alkyl, C₁-C₇-Alkyl-NH-CO-NH-C₁-C₇-alkyl, C₁-C₇-Alkyl-NH-SO₂-NH-C₁-C₇-alkyl, C₁-C₇-Alkoxy, Hydroxy-C₁-C₇-alkoxy, C₁-C₇-Alkoxy-C₁-C₇-alkoxy, C₁-C₇-Alkanoyloxy, Mono- oder Di-(C₁-C₇-alkyl)-amino, Mono- oder Di-(naphthyl- oder - phenyl-C₁-C₇-alkyl)-amino, N-Mono-C₁-C₇-alkoxy-C₁-C₇-alkylamino, C₁-C₇-Alkanoylamino, C₁-C₇-Alkylsulfonylamino, C₁-C₇-Alkoxy-carbonyl, Halogen-C₁-C₇-alkoxycarbonyl, Hydroxy-C₁-C₇-alkoxycarbonyl, C₁-C₇-Alkoxy-C₁-C₇-alkoxycarbonyl, Amino-C₁-C₇-alkoxycarbonyl, (N)-Mono-(C₁-C₇-alkyl)-amino-C₁-C₇-alkoxycarbonyl, C₁-C₇-Alkanoylamino-C₁-C₇-alkoxycarbonyl, N-Mono- oder N,N-Di-(C₁-C₇-alkyl)-aminocarbonyl, N-C₁-C₇-Alkoxy-C₁-C₇-alkylcarbamoyl oder N-Mono- oder N,N-Di- (C₁-C₇-alkyl) -aminosulfonyl steht; oder
aus Phenyl- oder Naphthyl-C₁-C₇-alkyl, Halogen-C₁-C₇-alkyl, Halogen, Hydroxy, Phenyl- oder Naphthyloxy, Phenyl- oder Naphthyl-C₁-C₇-alkyloxy, Nitro, Amino, Amino-C₁-C₇-alkyl, Carboxyl, Halogen-C₁-C₇-alkoxycarbonyl, Phenyl- oder Naphthyl-C₁-C₇-alkoxycarbonyl, C₁-C₇-Alkylsulfonyl, Carbamoyl und Cyano;
Pyrrolylcarbonyl, Furanylcarbonyl, Thienylcarbonyl, Pyrimidin-2,4-dion-1-, -2-, -3- oder -5-yl-carbonyl, Indolyl-carbonyl, Benzimidazolyl-carbonyl, Benzopyrazolyl-carbonyl, Benzofuranyl-carbonyl, Chinolinyl-carbonyl oder Benzo[1,2,5]oxadiazolylcarbonyl, die jeweils unsubstituiert sind oder substituiert durch einen oder mehrere Substituenten unabhängig voneinander ausgewählt aus einem Substituenten der Formel -(C₀-C₇-Alkylen)-(X)ᵣ-(C₁-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- stehen, wobei V für Wasserstoff oder wie unten definiertes unsubstituiertes oder substituiertes Alkyl, Hydroxy-C₁-C₇-alkyl, C₁-C₇-Alkoxy-C₁-C₇-alkyl, C₁-C₇-Alkoxy-C₁-C₇-alkoxy-C₁-C₇-alkyl, C₁-C₇-Alkanoyloxy-C₁-C₇-alkyl, Amino-C₁-C₇-alkyl, (N)-Mono- oder (N,N)-Di-(C₁-C₇-alkyl)-amino-C₁-C₇-alkyl, C₁-C₇-Alkoxy-C₁-C₇-alkylamino-C₁-C₇-alkyl, Mono- (naphthyl- oder -phenyl)-amino-C₁-C₇-alkyl, Mono-(naphthyl- oder -phenyl-C₁-C₇-alkyl)-amino-C₁-C₇-alkyl, C₁-C₇-Alkanoylamino-C₁-C₇-alkyl, C₁-C₇-Alkyl-O-CO-NH-C₁-C₇-alkyl, C₁-C₇-Alkylsulfonylamino-C₁-C₇-alkyl, C₁-C₇-Alkyl-NH-CO-NH-C₁-C₇-alkyl, C₁-C₇-Alkyl-NH-SO₂-NH-C₁-C₇-alkyl, C₁-C₇-Alkoxy, Hydroxy-C₁-C₇-alkoxy, C₁-C₇-Alkoxy-C₁-C₇-alkoxy, C₁-C₇-Alkanoyloxy, Mono- oder Di-(C₁-C₇-alkyl)-amino, Mono- oder Di- (naphthyl- oder -phenyl-C₁-C₇-alkyl)-amino, N-Mono-C₁-C₇-alkoxy-C₁-C₇-alkylamino, C₁-C₇-Alkanoylamino, C₁-C₇-Alkylsulfonylamino, C₁-C₇-Alkoxy-carbonyl, Hydroxy-C₁-C₇-alkoxycarbonyl, C₁-C₇-Alkoxy-C₁-C₇-alkoxycarbonyl, Amino-C₁-C₇-alkoxycarbonyl, (N) -Mono- (C₁-C₇-alkyl) -amino-C₁-C₇-alkoxycarbonyl, C₁-C₇-Alkanoylamino-C₁-C₇-alkoxycarbonyl, N-Mono- oder N,N-Di-(C₁-C₇-alkyl)-aminocarbonyl, N-C₁-C₇-Alkoxy-C₁-C₇-alkylcarbamoyl oder N-Mono- oder N,N-Di-(C₁-C₇-alkyl)-aminosulfonyl steht;
aus Phenyl- oder Naphthyl-C₁-C₇-alkyl, Halogen-C₁-C₇-alkyl, Halogen, Hydroxy, Phenyl- oder Naphthyloxy, Phenyl- oder Naphthyl-C₁-C₇-alkyloxy, Nitro, Amino, Amino-C₁-C₇-alkyl, Carboxyl, Halogen-C₁-C₇-alkoxycarbonyl, Phenyl- oder Naphthyl-C₁-C₇-alkoxycarbonyl, C₁-C₇-Alkylsulfonyl, Carbamoyl und Cyano;
R² für
C₁-C₇-Alkyl, das unsubstituiert ist oder substituiert durch einen oder mehrere Substituenten ausgewählt aus der Gruppe bestehend aus Halogen, Phenyl- oder Naphthyl, Hydroxy, C₁-C₇-Alkoxy, Amino, Mono- oder Di-(C₁-C₇-alkyl)-amino, C₁-C₇-Alkanoylamino, C₁-C₇-Alkylsulfonylamino, Phenyl- oder Naphthylsulfonylamino, Phenyl- oder Naphthyl-C₁-C₇-alkylsulfonylamino, C₁-C₇-Alkoxy-C₁-C₇-alkoxy, Hydroxy-C₁-C₇-alkoxy, Phenyl- oder Naphthyloxy, Phenyl- oder Naphthyl-C₁-C₇-alkyloxy, C₁-C₇-Alkanoyloxy, Nitro, Carboxyl, C₁-C₇-Alkoxy-carbonyl, Phenyl- oder Naphthyl-C₁-C₇-alkoxycarbonyl, Carbamoyl, N-Mono- oder N,N-Di-(C₁-C₇-alkyl-, -phenyl-, -naphthyl-, -phenyl-C₁-C₇-alkyl- oder -naphthyl-C₁-C₇-alkyl)-carbamoyl und N-Mono- oder N,N-Di-(C₁-C₇-alkyl-, -phenyl-, -naphthyl-, -phenyl-C₁-C₇-alkyl- oder -naphthyl-C₁-C₇-alkyl)-sulfamoyl; und Cyano; C₃-C₁₀-Cycloalkyl, welches unsubstituiert ist oder substituiert durch einen oder mehrere Substituenten unabhängig voneinander ausgewählt aus den oben für substituiertes Phenyl- oder Naphthyl-R¹ erwähnten Substituenten, oder C₃-C₁₀-Cycloalkyl-C₁-C₇-alkyl, wobei Cycloalkyl unsubstituiert ist oder substituiert durch einen oder mehrere, z.B. bis zu drei, Substituenten unabhängig voneinander ausgewählt aus den oben für substituiertes Phenyl- oder Naphthyl-R¹ erwähnten Substituenten, steht;
und R³ und R⁴, die dann für -O- stehen, zusammen mit L, das dann für Methylen steht, und dem Kohlenstoff, an den R³-L- und R⁴ gebunden sind, einen substituierten oder unsubstituierten 5-gliedrigen Ring bilden, der an Benzo kondensiert ist, wobei Benzo durch einen oder mehrere Substituenten ausgewählt aus der Gruppe bestehend aus C₁-C₇-Alkyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, Halogen, Hydroxy, C₁-C₇-alkoxy, C₁-C₇-Alkoxy-C₁-C₇-alkoxy, Phenyl- oder Naphthyloxy, Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, Amino-C₁-C₇-alkyl, Mono- oder Di- (C₁-C₇-alkyl) -amino-C₁-C₇-alkyl, C₁-C₇-Alkanoylamino-C₁-C₇-alkyl, Carboxyl, C₁-C₇-Alkoxy-carbonyl, Phenyl- oder Naphthyl-C₁-C₇-alkoxycarbonyl, Carbamoyl und Cyano, substituiert oder unsubstituiert ist, und so eine spiro-Verbindung der Formel I bilden, oder
und T für Carbonyl oder Thiocarbonyl oder Methylen steht;
und deren pharmazeutisch unbedenkliche Salze.

4. Verbindungen der Formel I nach Anspruch 1 oder 2, wobei
R¹ für
Phenyl- oder Naphthyl-carbonyl oder Phenyl- oder Naphthyl-C₁-C₇-alkylcarbonyl steht, wobei Phenyl und Naphthyl jeweils unsubstituiert sind oder substituiert durch einen oder mehrere Substituenten ausgewählt aus der Gruppe bestehend aus
einem Substituenten der Formel -(C₀-C₇-alkylen)-(X)ᵣ-(C₁-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- stehen, wobei V für Wasserstoff oder wie unten definiertes unsubstituiertes oder substituiertes Alkyl, Hydroxy-C₁-C₇-alkyl, C₁-C₇-Alkoxy-C₁-C₇-alkyl, C₁-C₇-Alkoxy-C₁-C₇-alkoxy-C₁-C₇-alkyl, C₁-C₇-Alkanoyloxy-C₁-C₇-alkyl, Amino-C₁-C₇-alkyl, (N)-Mono- oder (N,N)-Di-(C₁-C₇-alkyl)-amino-C₁-C₇-alkyl, C₁-C₇-Alkoxy-C₁-C₇-alkylamino-C₁-C₇-alkyl, Mono- (naphthyl- oder -phenyl)-amino-C₁-C₇-alkyl, Mono-(naphthyl- oder -phenyl-C₁-C₇-alkyl)-amino-C₁-C₇-alkyl, C₁-C₇-Alkanoylamino-C₁-C₇-alkyl, C₁-C₇-Alkyl-O-CO-NH-C₁-C₇-alkyl, C₁-C₇-Alkylsulfonylamino-C₁-C₇-alkyl, C₁-C₇-Alkyl-NH-CO-NH-C₁-C₇-alkyl, C₁-C₇-Alkyl-NH-SO₂-NH-C₁-C₇-alkyl, C₁-C₇-Alkoxy, Hydroxy-C₁-C₇-alkoxy, C1-C₇-Alkoxy-C₁-C₇-alkoxy, C₁-C₇-Alkanoyloxy, Mono- oder Di-(C₁-C₇-alkyl)-amino, Mono- oder Di-(naphthyl- oder - phenyl-C₁-C₇-alkyl)-amino, N-Mono-C₁-C₇-alkoxy-C₁-C₇-alkylamino, C₁-C₇-Alkanoylamino, C₁-C₇-Alkylsulfonylamino, C₁-C₇-Alkoxy-carbonyl, Hydroxy-C₁-C₇-alkoxycarbonyl, C₁-C₇-Alkoxy-C₁-C₇-alkoxycarbonyl, Amino-C₁-C₇-alkoxycarbonyl, (N)-Mono-(C₁-C₇-alkyl)-amino-C₁-C₇-alkoxycarbonyl, C₁-C₇-Alkanoylamino-C₁-C₇-alkoxycarbonyl, N-Mono- oder N,N-Di-(C₁-C₇-alkyl)-aminocarbonyl, N-C₁-C₇-Alkoxy-C₁-C₇-alkylcarbamoyl oder N-Mono- oder N,N-Di-(C₁-C₇-alkyl)-aminosulfonyl steht; oder
aus Phenyl- oder Naphthyl-C₁-C₇-alkyl, Halogen-C₁-C₇-alkyl, Halogen, Hydroxy, Phenyl- oder Naphthyloxy, Phenyl- oder Naphthyl-C₁-C₇-alkyloxy, Nitro, Amino, Amino-C₁-C₇-alkyl, Carboxyl, Halogen-C₁-C₇-alkoxycarbonyl, Phenyl- oder Naphthyl-C₁-C₇-alkoxycarbonyl, C₁-C₇-Alkylsulfonyl, Carbamoyl und Cyano;
oder Pyrrolylcarbonyl, Furanylcarbonyl, Thienylcarbonyl, Pyrimidin-2,4-dion-1-, -2-, -3- oder -5-yl-carbonyl, Indolyl-carbonyl, Benzimidazolyl-carbonyl, Benzopyrazolyl-carbonyl, Benzofuranyl-carbonyl, Chinolinyl-carbonyl oder Benzo[1,2,5]oxadiazolyl-carbonyl, die jeweils unsubstituiert sind oder substituiert durch einen oder mehrere, z.B. bis zu drei, Substituenten unabhängig voneinander ausgewählt aus einem Substituenten der Formel -(C₀-C₇-Alkylen)-(X)ᵣ-(C₁-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-; - CO-NV-; -NV-SO₂-, -SO₂-NV; -NV-CO-NV-, -NV-CO-O-, -O-CO-NV-, -NV-SO₂-NV- stehen, wobei V für Wasserstoff oder wie unten definiertes unsubstituiertes oder substituiertes Alkyl, Hydroxy-C₁-C₇-alkyl, C₁-C₇-Alkoxy-C₁-C₇-alkyl wie 3-Methoxypropyl oder 2-Methoxyethyl, C₁-C₇-Alkoxy-C₁-C₇-alkoxy-C₁-C₇-alkyl, C₁-C₇-Alkanoyloxy-C₁-C₇-alkyl, Amino-C₁-C₇-alkyl, (N)-Mono- oder (N,N)-Di-(C₁-C₇-alkyl)-amino-C₁-C₇-alkyl, C₁-C₇-Alkoxy-C₁-C₇-alkylamino-C₁-C₇-alkyl, Mono-(naphthyl- oder -phenyl)-amino-C₁-C₇-alkyl, Mono- (naphthyl- oder -phenyl-C₁-C₇-alkyl)-amino-C₁-C₇-alkyl, C₁-C₇-Alkanoylamino-C₁-C₇-alkyl, C₁-C₇-Alkyl-O-CO-NH-C₁-C₇-alkyl, C₁-C₇-Alkylsulfonylamino-C₁-C₇-alkyl, C₁-C₇-Alkyl-NH-CO-NH-C₁-C₇-alkyl, C₁-C₇-Alkyl-NH-SO₂-NH-C₁-C₇-alkyl, C₁-C₇-Alkoxy, Hydroxy-C₁-C₇-alkoxy, C₁-C₇-Alkoxy-C₁-C₇-alkoxy, C₁-C₇-Alkanoyloxy, Mono- oder Di-(C₁-C₇-alkyl)-amino, Mono- oder Di-(naphthyl- oder -phenyl-C₁-C₇-alkyl)-amino, N-Mono-C₁-C₇-alkoxy-C₁-C₇-alkylamino, C₁-C₇-Alkanoylamino, C₁-C₇-Alkylsulfonylamino, C₁-C₇-Alkoxy-carbonyl, Hydroxy-C₁-C₇-alkoxycarbonyl, C₁-C₇-Alkoxy-C₁-C₇-alkoxycarbonyl, Amino-C₁-C₇-alkoxycarbonyl, (N) -Mono- (C₁-C₇-alkyl) -amino-C₁-C₇-alkoxycarbonyl, C₁-C₇-Alkanoylamino-C₁-C₇-alkoxycarbonyl, N-Mono- oder N,N-Di-(C₁-C₇-alkyl)-aminocarbonyl, N-C₁-C₇-Alkoxy-C₁-C₇-alkylcarbamoyl oder N-Mono- oder N,N-Di-(C₁-C₇-alkyl)-aminosulfonyl steht; oder
aus Phenyl- oder Naphthyl-C₁-C₇-alkyl, Halogen-C₁-C₇-alkyl, Halogen, Hydroxy, Phenyl- oder Naphthyloxy, Phenyl- oder Naphthyl-C₁-C₇-alkyloxy, Nitro, Amino, Amino-C₁-C₇-alkyl, Carboxyl, Phenyl- oder Naphthyl-C₁-C₇-alkoxycarbonyl, Halogen-C₁-C₇-alkoxycarbonyl, C₁-C₇-Alkylsulfonyl, Carbamoyl und Cyano;
R² für
C₁-C₇-Alkyl, das unsubstituiert ist oder substituiert durch einen oder mehrere Substituenten ausgewählt aus der Gruppe bestehend aus Halogen, Phenyl- oder Naphthyl, Hydroxy, C₁-C₇-Alkoxy, Amino, Mono- oder Di-(C₁-C₇-alkyl)-amino, C₁-C₇-Alkanoylamino, C₁-C₇-Alkylsulfonylamino, Phenyl- oder Naphthylsulfonylamino, Phenyl- oder Naphthyl-C₁-C₇-alkylsulfonylamino, C₁-C₇-Alkoxy-C₁-C₇-alkoxy, Hydroxy-C₁-C₇-alkoxy, Phenyl- oder Naphthyloxy, Phenyl- oder Naphthyl-C₁-C₇-alkyloxy, C₁-C₇-Alkanoyloxy, Nitro, Carboxyl, C₁-C₇-Alkoxy-carbonyl, Phenyl- oder Naphthyl-C₁-C₇-alkoxycarbonyl, Carbamoyl, N-Mono- oder N,N-Di-(C₁-C₇-alkyl-, -phenyl-, -naphthyl-, -phenyl-C₁-C₇-alkyl- oder -naphthyl-C₁-C₇-alkyl)-carbamoyl und N-Mono- oder N,N-Di-(C₁-C₇-alkyl-, -phenyl-, -naphthyl-, -phenyl-C₁-C₇-alkyl- oder -naphthyl-C₁-C₇-alkyl)-sulfamoyl; und Cyano; C₃-C₁₀-Cycloalkyl, welches unsubstituiert ist oder substituiert durch einen oder mehrere Substituenten unabhängig voneinander ausgewählt aus den oben für substituiertes Phenyl- oder Naphthyl-R¹ erwähnten Substituenten, oder C₃-C₁₀-Cycloalkyl-C₁-C₇-alkyl, wobei Cycloalkyl unsubstituiert ist oder substituiert durch einen oder mehrere Substituenten unabhängig voneinander ausgewählt aus den oben für substituiertes Phenyl- oder Naphthyl-R¹ erwähnten Substituenten, steht;
R³ für Phenyl, Naphthyl, Phenyl-C₁-C₇-alkyl oder Naphthyl-C₁-C₇-alkyl steht, wobei Phenyl und Naphthyl jeweils unsubstituiert sind oder substituiert durch einen oder mehrere, z.B. bis zu drei, Substituenten ausgewählt aus der Gruppe bestehend aus C₁-C₇-Alkyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkylsulfonylamino-C₁-C₇-alkyl, Phenyl, Naphthyl, Mono- oder Di- (C₁-C₇-alkoxy) -phenyl oder -naphthyl, C₁-C₇-Alkylendioxy-phenyl, wobei die Oxy-Atome an benachbarte Phenyl-Ringatome gebunden sind, Halogen, Hydroxy, C₁-C₇-Alkoxy, Halogen-C₁-C₇-alkoxy, Hydroxy-C₁-C₇-alkoxy, C₁-C₇-alkoxy-C₁-C₇-alkoxy, Phenyl- oder Naphthyloxy, (unsubstituiertes oder mono-, di- oder tri-C₁-C₇-alkylsubstituiertes) Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, C₁-C₇-Alkylsulfonylamino, Carboxyl, C₁-C₇-Alkoxy-carbonyl, Phenyl- oder Naphthyl-C₁-C₇-alkoxycarbonyl, Carbamoyl und Cyano, C₁-C₇-Alkylsulfonyl, C₁-C₇-Alkylen, welches unsubstituiert oder durch bis zu vier C₁-C₇-AlkylSubstituenten substituiert und an zwei benachbarte Ringatome der Phenyl- oder Naphthyl-Einheit gebunden ist; Pyrrolyl, Furanyl und Thienyl;
R⁴ für Hydroxy steht;
L für Methylen steht;
und T für Carbonyl, Thiocarbonyl oder vorzugsweise Methylen steht;
und deren pharmazeutisch unbedenkliche Salze.

5. Verbindungen der Formel I nach Anspruch 1 oder 2, wobei
R¹ für Phenyl oder Naphthyl, jeweils unsubstituiert oder substituiert durch einen oder mehrere Substituenten ausgewählt aus der Gruppe bestehend aus C₁-C₇-Alkyl, Phenyl, Naphthyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkyl-sulfonylamino-C₁-C₇-alkyl, Halogen, Hydroxy, C₁-C₇-Alkoxy, Hydroxy-C₁-C₇-alkoxy, C₁-C₇-Alkoxy-C₁-C₇-alkoxy, Phenyl- oder Naphthyloxy, Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, Carboxyl, C₁-C₇-Alkoxy-carbonyl, Phenyl- oder Naphthyl-C₁-C₇-alkoxycarbonyl, Carbamoyl, N-Mono- oder N,N-Di-(C₁-C₇-alkyl- und/oder - (phenyl- oder -naphthyl)-C₁-C₇-alkyl)-carbamoyl, C₁-C₇-Alkylsulfonyl, unsubstituiertes oder C₁-C₇-alkyl-substituiertes Phenyl- oder Naphthylsulfonyl, N-Mono- oder N,N-Di-(C₁-C₇-alkyl- und/oder -(phenyl- oder -naphthyl)-C₁-C₇-alkyl)-sulfamoyl und Cyano;
Phenyl- oder Naphthyl-C₁-C₇-alkyl, wobei Phenyl und Naphthyl jeweils unsubstituiert sind oder substituiert durch einen oder mehrere Substituenten ausgewählt aus der Gruppe bestehend aus den gerade für substituiertes Phenyl oder Naphthyl erwähnten Substituenten, Pyrrolyl, Furanyl, Thienyl, Pyrimidin-2,4-dion-1-, -2-, -3- oder -5-yl und Benzo[1,3]dioxalyl, die jeweils unsubstituiert sind oder substituiert durch einen oder mehrere Substituenten unabhängig voneinander ausgewählt aus den oben für substituiertes Phenyl- oder Naphthyl-R¹ erwähnten;
Pyrrolyl-C₁-C₇-alkyl, Furanyl-C₁-C₇-alkyl, Thienyl-C₁-C₇-alkyl, Pyrimidin-2,4-dion-1-, -2-, -3- oder -5-yl-C₁-C₇-alkyl, Indolyl-C₁-C₇-alkyl, Benzofuranyl-C₁-C₇-alkyl, Benzimidazolyl-C₁-C₇-alkyl, Benzopyrazolyl-C₁-C₇-alkyl, Chinolinyl-C₁-C₇-alkyl, Isochinolyl-C₁-C₇-alkyl oder Benzo[1,2,5]oxadiazolyl-C₁-C₇-alkyl, die jeweils unsubstituiert sind oder substituiert durch einen oder mehrere, z.B. bis zu drei, Substituenten unabhängig voneinander ausgewählt aus den oben für substituiertes Phenyl- oder Naphthyl-R¹ erwähnten Substituenten;
C₃-C₁₀-Cycloalkyl, welches unsubstituiert ist oder substituiert durch einen oder mehrere Substituenten unabhängig voneinander ausgewählt aus den oben für substituiertes Phenyl- oder Naphthyl-R¹ erwähnten Substituenten;
C₃-C₁₀-Cycloalkyl-C₁-C₇-alkyl, wobei Cycloalkyl unsubstituiert ist oder substituiert durch einen oder mehrere Substituenten unabhängig voneinander ausgewählt aus den oben für substituiertes Phenyl- oder Naphthyl-R¹ erwähnten Substituenten;
Phenyl- oder Naphthyl-carbonyl oder Phenyl- oder Naphthyl-C₁-C₇-alkylcarbonyl, wobei Phenyl und Naphthyl jeweils unsubstituiert sind oder substituiert durch einen oder mehrere Substituenten ausgewählt aus der Gruppe bestehend aus
einem Substituenten der Formel -(C₀-C₇-Alkylen)-(X)ᵣ-(C1-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- stehen, wobei V für Wasserstoff oder wie unten definiertes unsubstituiertes oder substituiertes Alkyl, Hydroxy-C₁-C₇-alkyl, C₁-C₇-Alkoxy-C₁-C₇-alkyl, C₁-C₇-Alkoxy-C₁-C₇-alkoxy-C₁-C₇-alkyl, C₁-C₇-Alkanoyloxy-C₁-C₇-alkyl, Amino-C₁-C₇-alkyl, (N)-Mono- oder (N,N)-Di-(C₁-C₇-alkyl)-amino-C₁-C₇-alkyl, C₁-C₇-Alkoxy-C₁-C₇-alkylamino-C₁-C₇-alkyl, Mono- (naphthyl- oder -phenyl)-amino-C₁-C₇-alkyl, Mono-(naphthyl- oder -phenyl-C₁-C₇-alkyl)-amino-C₁-C₇-alkyl, C₁-C₇-Alkanoylamino-C₁-C₇-alkyl, C₁-C₇-Alkyl-O-CO-NH-C₁-C₇-alkyl, C₁-C₇-Alkylsulfonylamino-C₁-C₇-alkyl, C₁-C₇-Alkyl-NH-CO-NH-C₁-C₇-alkyl, C₁-C₇-Alkyl-NH-SO₂-NH-C₁-C₇-alkyl, C₁-C₇-Alkoxy, Hydroxy-C₁-C₇-alkoxy, C₁-C₇-Alkoxy-C₁-C₇-alkoxy, C₁-C₇-Alkanoyloxy, Mono- oder Di-(C₁-C₇-alkyl)-amino, Mono- oder Di- (naphthyl- oder -phenyl-C₁-C₇-alkyl)-amino, N-Mono-C₁-C₇-alkoxy-C₁-C₇-alkylamino, C₁-C₇-Alkanoylamino, C₁-C₇-Alkylsulfonylamino, C₁-C₇-Alkoxy-carbonyl, Hydroxy-C₁-C₇-alkoxycarbonyl, C₁-C₇-Alkoxy-C₁-C₇-alkoxycarbonyl, Amino-C₁-C₇-alkoxycarbonyl, (N) -Mono- (C₁-C₇-alkyl) -amino-C₁-C₇-alkoxycarbonyl, C₁-C₇-Alkanoylamino-C₁-C₇-alkoxycarbonyl, N-Mono- oder N,N-Di-(C₁-C₇-alkyl)-aminocarbonyl, N-C₁-C₇-Alkoxy-C₁-C₇-alkylcarbamoyl oder N-Mono- oder N,N-Di-(C₁-C₇-alkyl)-aminosulfonyl steht; oder
aus Phenyl- oder Naphthyl-C₁-C₇-alkyl, Halogen-C₁-C₇-alkyl, Halogen, Hydroxy, Phenyl- oder Naphthyloxy, Phenyl- oder Naphthyl-C₁-C₇-alkyloxy, Nitro, Amino, Amino-C₁-C₇-alkyl, Carboxyl, Phenyl- oder Naphthyl-C₁-C₇-alkoxycarbonyl, Halogen-C₁-C₇-alkoxycarbonyl, C₁-C₇-Alkylsulfonyl, Carbamoyl und Cyano;
Heterocyclylcarbonyl wie Pyrrolylcarbonyl, Furanylcarbonyl, Thienylcarbonyl, Pyrimidin-2,4-dion-1-, -2-, -3- oder -5-yl-carbonyl, Indolyl-carbonyl, 3-Methylindolyl-carbonyl, Benzimidazolyl-carbonyl, Benzopyrazolyl-carbonyl, Benzofuranyl-carbonyl, Chinolinyl-carbonyl oder Benzo[1,2,5]oxadiazolyl-carbonyl, die jeweils unsubstituiert sind oder substituiert durch einen oder mehrere, z.B. bis zu drei, Substituenten unabhängig voneinander ausgewählt aus einem Substituenten der Formel -(C₀-C₇-Alkylen)-(X)ᵣ-(C₁-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-; - CO-NV-; -NV-SO₂-, -SO₂-NV; -NV-CO-NV-, -NV-CO-O-, -O-CO-NV-, -NV-SO₂-NV- stehen, wobei V für Wasserstoff oder wie unten definiertes unsubstituiertes oder substituiertes Alkyl, Hydroxy-C₁-C₇-alkyl, C₁-C₇-Alkoxy-C₁-C₇-alkyl wie 3-Methoxypropyl oder 2-Methoxyethyl, C₁-C₇-Alkoxy-C₁-C₇-alkoxy-C₁-C₇-alkyl, C₁-C₇-Alkanoyloxy-C₁-C₇-alkyl, Amino-C₁-C₇-alkyl wie Aminomethyl, (N)-Mono- oder (N,N)-Di-(C₁-C₇-alkyl)-amino-C₁-C₇-alkyl, C₁-C₇-Alkoxy-C₁-C₇-alkylamino-C₁-C₇-alkyl, Mono-(naphthyl- oder -phenyl)-amino-C₁-C₇-alkyl, Mono-(naphthyl- oder -phenyl-C₁-C₇-alkyl)-amino-C₁-C₇-alkyl, C₁-C₇-Alkanoylamino-C₁-C₇-alkyl, C₁-C₇-Alkyl-O-CO-NH-C₁-C₇-alkyl, C₁-C₇-Alkylsulfonylamino-C₁-C₇-alkyl, C₁-C₇-Alkyl-NH-CO-NH-C₁-C₇-alkyl, C₁-C₇-Alkyl-NH-SO₂-NH-C₁-C₇-alkyl, C₁-C₇-Alkoxy, Hydroxy-C₁-C₇-alkoxy, C₁-C₇-Alkoxy-C₁-C₇-alkoxy, C₁-C₇-Alkanoyloxy, Mono- oder Di- (C₁-C₇-alkyl) - amino, Mono- oder Di-(naphthyl- oder -phenyl-C₁-C₇-alkyl)-amino, N-Mono-C₁-C₇-alkoxy-C₁-C₇-alkylamino, C₁-C₇-Alkanoylamino, C₁-C₇-Alkylsulfonylamino, C₁-C₇-Alkoxycarbonyl, Hydroxy-C₁-C₇-alkoxycarbonyl, C₁-C₇-Alkoxy-C₁-C₇-alkoxycarbonyl, Amino-C₁-C₇-alkoxycarbonyl, (N)-Mono-(C₁-C₇-alkyl)-amino-C₁-C₇-alkoxycarbonyl, C₁-C₇-Alkanoylamino-C₁-C₇-alkoxycarbonyl, N-Mono- oder N,N-Di-(C₁-C₇-alkyl) -aminocarbonyl, N-C₁-C₇-Alkoxy-C₁-C₇-alkylcarbamoyl oder N-Mono- oder N,N-Di-(C₁-C₇-alkyl)-aminosulfonyl steht; oder
aus Phenyl- oder Naphthyl-C₁-C₇-alkyl, Halogen-C₁-C₇-alkyl, Halogen, Hydroxy, Phenyl- oder Naphthyloxy, Phenyl- oder Naphthyl-C₁-C₇-alkyloxy, Nitro, Amino, Amino-C₁-C₇-alkyl, Carboxyl, Phenyl- oder Naphthyl-C₁-C₇-alkoxycarbonyl, Halogen-C₁-C₇-alkoxycarbonyl, C₁-C₇-Alkylsulfonyl, Carbamoyl und Cyano;
oder Phenyl- oder Naphthyl-sulfonyl, wobei Phenyl und Naphthyl jeweils unsubstituiert sind oder substituiert durch einen oder mehrere Substituenten ausgewählt aus den oben für substituiertes Phenyl- oder Naphthyl-R¹ erwähnten, steht;
R² für
C₁-C₇-Alkyl, das unsubstituiert ist oder substituiert durch einen oder mehrere Substituenten ausgewählt aus der Gruppe bestehend aus Halogen, Phenyl- oder Naphthyl, Hydroxy, C₁-C₇-Alkoxy, Amino, Mono- oder Di-(C₁-C₇-alkyl)-amino, C₁-C₇-Alkanoylamino, C₁-C₇-Alkylsulfonylamino, Phenyl- oder Naphthylsulfonylamino, Phenyl- oder Naphthyl-C₁-C₇-alkylsulfonylamino, C₁-C₇-Alkoxy-C₁-C₇-alkoxy, Hydroxy-C₁-C₇-alkoxy, Phenyl- oder Naphthyloxy, Phenyl- oder Naphthyl-C₁-C₇-alkyloxy, C₁-C₇-Alkanoyloxy, Nitro, Carboxyl, C₁-C₇-Alkoxy-carbonyl, Phenyl- oder Naphthyl-C₁-C₇-alkoxycarbonyl, Carbamoyl, N-Mono- oder N,N-Di-(C₁-C₇-alkyl-, -phenyl-, -naphthyl-, -phenyl-C₁-C₇-alkyl- oder -naphthyl-C₁-C₇-alkyl)-carbamoyl und N-Mono- oder N,N-Di-(C₁-C₇-alkyl-, -phenyl-, -naphthyl-, -phenyl-C₁-C₇-alkyl- oder -naphthyl-C₁-C₇-alkyl)-sulfamoyl; und Cyano;
Phenyl oder Naphthyl, jeweils unsubstituiert oder substituiert durch einen oder mehrere Substituenten ausgewählt aus der Gruppe bestehend aus C₁-C₇-Alkyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkylsulfonylamino-C₁-C₇-alkyl, Halogen, Hydroxy, C₁-C₇-Alkoxy, C₁-C₇-Alkoxy-C₁-C₇-alkoxy, Hydroxy-C₁-C₇-alkoxy, Phenyl- oder Naphthyloxy, Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, Carboxyl, C₁-C₇-Alkoxycarbonyl, Halogen-C₁-C₇-alkoxycarbonyl, Phenyl- oder Naphthyl-C₁-C₇-alkoxycarbonyl, Carbamoyl, N-Mono- oder N,N-Di-(C₁-C₇-alkyl-, -phenyl-, -naphthyl-, -phenyl-C₁-C₇-alkyl- oder -naphthyl-C₁-C₇-alkyl)-carbamoyl und N-Mono- oder N,N-Di-(C₁-C₇-alkyl-, -phenyl-, -naphthyl-, -phenyl-C₁-C₇-alkyl- oder -naphthyl-C₁-C₇-alkyl)-sulfamoyl und Cyano;
Phenyl- oder Naphthyl-C₁-C₇-alkyl, wobei Phenyl und Naphthyl jeweils unsubstituiert sind oder substituiert durch einen oder mehrere Substituenten ausgewählt aus der gerade für substituiertes Phenyl- oder Naphthyl-R² erwähnten Gruppe;
C₃-C₁₀-Cycloalkyl, welches unsubstituiert ist oder substituiert durch einen oder mehrere Substituenten ausgewählt aus der gerade für substituiertes Phenyl- oder Naphthyl-R² erwähnten Gruppe;
C₃-C₁₀-Cycloalkyl-C₁-C₇-alkyl, wobei Cycloalkyl unsubstituiert ist oder substituiert durch einen oder mehrere, z.B. bis zu drei, Substituenten ausgewählt aus der gerade für substituiertes Phenyl- oder Naphthyl-R² erwähnten Gruppe;
oder Pyrrolyl, Furanyl oder Thienyl steht, oder, wenn L für Methylen, Oxy, Thio oder Imino steht, R² ausgewählt ist aus einer der gerade erwähnten Gruppen von R²-Einheiten, und aus Wasserstoff;
R³ für
C₁-C₇-Alkyl, Carbamoyl-C₁-C₇-alkyl, N-Mono- oder N,N-Di-(C₃-C₈-cycloalkyl-, -heterocyclyl-, -phenyl-, -naphthyl-, -C₁-C₇-alkyl-, -C₃-C₈-cycloalkyl-C₁-C₇-alkyl-, -heterocyclyl-C₁-C₇-alkyl-, -phenyl-C₁-C₇-alkyl- und/oder -naphthyl-C₁-C₇-alkyl)-aminocarbonyl-C₁-C₇-alkyl; Phenyl, Naphthyl, Phenyl-C₁-C₇-alkyl oder Naphthyl-C₁-C₇-alkyl, wobei Phenyl und Naphthyl jeweils unsubstituiert sind oder substituiert durch einen oder mehrere Substituenten ausgewählt aus der Gruppe bestehend aus Phenyl, Naphthyl, C₁-C₇-Alkyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkyl-sulfonylamino-C₁-C₇-alkyl, Phenyl, Naphthyl, Mono- oder Di-(C₁-C₇-alkoxy)-phenyl oder -naphthyl, C₁-C₇-Alkylendioxyphenyl, wobei die Oxy-Atome an benachbarte Phenyl-Ringatome gebunden sind, Halogen, Hydroxy, C₁-C₇-Alkoxy, Halogen-C₁-C₇-alkoxy, Hydroxy-C₁-C₇-alkoxy, C₁-C₇-Alkoxy-C₁-C₇-alkoxy, Phenyl- oder Naphthyloxy, (unsubstituiertes oder mono-, di- oder tri-C₁-C₇-alkyl-substituiertes) Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, C₁-C₇-Alkylsulfonylamino, Carboxyl, C₁-C₇-alkoxy-carbonyl, Phenyl- oder Naphthyl-C₁-C₇-alkoxycarbonyl, Carbamoyl und Cyano, C₁-C₇-Alkylsulfonyl, C₁-C₇-Alkylen, welches unsubstituiert oder durch bis zu vier C₁-C₇-Alkyl-Substituenten substituiert und an zwei benachbarte Ringatome der Phenyl- bzw. Naphthyl-Einheit gebunden ist; Pyrrolyl, Furanyl und Thienyl;
Phenyl- oder Naphthyl-sulfonyl oder Phenyl-C₁-C₇-alkyl- oder Naphthyl-C₁-C₇-alkyl-sulfonyl, wobei Phenyl und Naphthyl jeweils unsubstituiert oder durch einen oder mehrere Substituenten ausgewählt aus der gerade für substituiertes Phenyl- oder Naphthyl-R³ beschriebenen Gruppe substituiert sind,
C₃-C₁₀-Cycloalkyl oder C₃-C₁₀-Cyclyoalkyl-C₁-C₇-alkyl, wobei in beiden Cycloalkyl unsubstituiert ist oder substituiert durch einen oder mehrere der gerade für substituiertes Phenyl- oder Naphthyl-R³ erwähnten Substituenten;
oder Heterocyclyl oder Heterocyclyl-C₁-C₇-alkyl, wobei Heterocyclyl ausgewählt ist aus Pyrrolyl, Furanyl, Thienyl, Pyrimidin-2,4-dion-1-, -3- oder -5-yl, Indolyl, Benzofuranyl, Benzimidazolyl, Benzopyrazolyl, Chinolinyl, Isochinolinyl, Methylen-dioxy-phenyl, Ethylen-1,2-dioxy-phenyl oder Trimethylen-1,3-dioxy-phenyl, wobei die Oxy-Gruppen an benachbarte Ringatome des Phenylrings gebunden sind, wobei die Heterocyclyl-Einheit jeweils unsubstituiert oder wie oben für substituiertes Phenyl-R³ beschrieben substituiert ist, steht;
oder, wenn L für Imino, Oxy oder Thio steht, alternativ für Phenyl- oder Naphthylcarbonyl, C₁-C₇-Alkoxycarbonyl (was C₁-C₇-Alkyl-O-C(=O)- bedeutet), Phenyloxycarbonyl, Naphthyloxycarbonyl, Cycloalkyloxycarbonyl, Heterocyclyloxycarbonyl, Phenyl-C₁-C₇-alkyloxycarbonyl, Naphthyl-C₁-C₇-alkyloxycarbonyl, Cycloalkyl-C₁-C₇-alkoxycarbonyl, Heterocyclyl-C₁-C₇-alkoxycarbonyl oder N-Mono- oder N,N-Di-(alkyl-, -naphthyl-, -phenyl-, -heterocyclyl-, -cycloalkyl-, -naphthyl-, -heterocyclyclyl-, - cycloalkyl- oder -phenyl-C₁-C₇-alkyl)-aminocarbonyl stehen kann, wobei die Phenyl- und Naphthylringe jeweils unsubstituiert oder wie oben für substituiertes Phenyl- bzw. Naphthyl-R³ erwähnt substituiert sind;
und
R⁴ für Wasserstoff steht;
L für Methylen (-CH₂-), Oxy (-O-) oder Imino (-NH-) steht;
und T für Carbonyl, Thiocarbonyl oder Methylen steht;
und deren pharmazeutisch unbedenkliche Salze.

6. Verbindungen der Formel I nach Anspruch 1 oder 2, wobei
R¹ für
Phenyl, Naphthyl, Phenylcarbonyl, Naphthylcarbonyl oder Phenyl- oder Naphthyl-C₁-C₇-alkyl, wobei Phenyl und Naphthyl jeweils unsubstituiert sind oder substituiert durch einen oder mehrere, z.B. bis zu drei, Substituenten ausgewählt aus der Gruppe bestehend aus einem Substituenten der Formel -(C₀-C₇-Alkylen)-(X)ᵣ-(C₁-C₇-alkylen)-(Y)ₛ-(C₀-C₇-alkylen)-H, wobei C₀-Alkylen bedeutet, dass anstelle von gebundenem Alkylen eine Bindung vorhanden ist, r und s jeweils unabhängig voneinander für 0 oder 1 stehen und X und Y jeweils, falls vorhanden und unabhängig von den anderen, für -O-, -NV-, -S-, -O-CO-, -CO-O-, -NV-CO-; -CO-NV-; -NV-SO₂-, -SO₂-NV; -NV-CO-NV-, -NV-CO-O-, -O-CO-NV-, -NV-SO₂-NV- stehen, wobei V für Wasserstoff oder wie unten definiertes unsubstituiertes oder substituiertes Alkyl steht, insbesondere ausgewählt aus C₁-C₇-Alkyl, Phenyl, Naphthyl, Phenyl- oder Naphthyl-C₁-C₇-alkyl und Halogen-C₁-C₇-alkyl; z.B. C₁-C₇-Alkyl wie Methyl, Ethyl, n-Propyl, Isopropyl, n-Butyl, Isobutyl, sek.-Butyl oder tert.-Butyl, Hydroxy-C₁-C₇-alkyl, C₁-C₇-Alkoxy-C₁-C₇-alkyl wie 3-Methoxypropyl oder 2-Methoxyethyl, C₁-C₇-Alkoxy-C₁-C₇-alkoxy-C₁-C₇-alkyl, C₁-C₇-Alkanoyloxy-C₁-C₇-alkyl, Amino-C₁-C₇-alkyl wie Aminomethyl, (N)-Mono- oder (N,N)-Di-(C₁-C₇-alkyl)-amino-C₁-C₇-alkyl, C₁-C₇-Alkoxy-C₁-C₇-alkylamino-C₁-C₇-alkyl, Mono-(naphthyl- oder - phenyl)-amino-C₁-C₇-alkyl, Mono-(naphthyl- oder -phenyl-C₁-C₇-alkyl)-amino-C₁-C₇-alkyl, C₁-C₇-Alkanoylamino-C₁-C₇-alkyl, C₁-C₇-Alkyl-O-CO-NH-C₁-C₇-alkyl, C₁-C₇-Alkylsulfonylamino-C₁-C₇-alkyl, C₁-C₇-Alkyl-NH-CO-NH-C₁-C₇-alkyl, C₁-C₇-Alkyl-NH-SO₂-NH-C₁-C₇-alkyl, C₁-C₇-Alkoxy, Hydroxy-C₁-C₇-alkoxy, C₁-C₇-Alkoxy-C₁-C₇-alkoxy, C₁-C₇-Alkanoyloxy, Mono- oder Di-(C₁-C₇-alkyl)-amino, Mono- oder Di-(naphthyl- oder -phenyl-C₁-C₇-alkyl)-amino, N-Mono-C₁-C₇-alkoxy-C₁-C₇-alkylamino, C₁-C₇-Alkanoylamino, C₁-C₇-Alkylsulfonylamino, C₁-C₇-Alkoxy-carbonyl, Hydroxy-C₁-C₇-alkoxycarbonyl, C₁-C₇-Alkoxy-C₁-C₇-alkoxycarbonyl, Amino-C₁-C₇-alkoxycarbonyl, (N) -Mono-(C₁-C₇-alkyl)-amino-C₁-C₇-alkoxycarbonyl, C₁-C₇-Alkanoylamino-C₁-C₇-alkoxycarbonyl, N-Mono- oder N,N-Di-(C₁-C₇-alkyl)-aminocarbonyl, N-C₁-C₇-Alkoxy-C₁-C₇-alkylcarbamoyl oder N-Mono- oder N,N-Di-(C₁-C₇-alkyl)-aminosulfonyl; oder aus Phenyl- oder Naphthyl-C₁-C₇-alkyl, Halogen-C₁-C₇-alkyl, Halogen, Hydroxy, Phenyl- oder Naphthyloxy, Phenyl- oder Naphthyl-C₁-C₇-alkyloxy, Nitro, Amino, Amino-C₁-C₇-alkyl, Carboxyl, Halogen-C₁-C₇-alkoxycarbonyl, Phenyl- oder Naphthyl-C₁-C₇-alkoxycarbonyl, C₁-C₇-Alkylsulfonyl, Carbamoyl und Cyano, steht;
R² für
C₁-C₇-Alkyl, das unsubstituiert ist oder substituiert durch einen oder mehrere, z.B. bis zu drei, Substituenten ausgewählt aus der Gruppe bestehend aus Halogen, Phenyl- oder Naphthyl, Hydroxy, C₁-C₇-alkoxy, Amino, Mono- oder Di- (C₁-C₇-alkyl) -amino, C₁-C₇-Alkanoylamino, C₁-C₇-Alkyl-sulfonylamino, Phenyl- oder Naphthylsulfonylamino, Phenyl- oder Naphthyl-C₁-C₇-alkylsulfonylamino, C₁-C₇-Alkoxy-C₁-C₇-alkoxy, Hydroxy-C₁-C₇-alkoxy, Phenyl- oder Naphthyloxy, Phenyl- oder Naphthyl-C₁-C₇-alkyloxy, C₁-C₇-Alkanoyloxy, Nitro, Carboxyl, C₁-C₇-alkoxy-carbonyl, Phenyl- oder Naphthyl-C₁-C₇-alkoxycarbonyl, Carbamoyl, N-Mono- oder N,N-Di-(C₁-C₇-alkyl-, -phenyl-, -naphthyl-, -phenyl-C₁-C₇-alkyl- oder -naphthyl-C₁-C₇-alkyl)-carbamoyl und N-Mono- oder N,N-Di-(C₁-C₇-alkyl-, -phenyl-, -naphthyl-, -phenyl-C₁-C₇-alkyl- oder -naphthyl-C₁-C₇-alkyl)-sulfamoyl; und Cyano steht;
R³ für
Phenyl oder Naphthyl, wobei Phenyl und Naphthyl jeweils unsubstituiert sind oder substituiert durch einen oder mehrere, z.B. bis zu drei, Substituenten ausgewählt aus der Gruppe bestehend aus C₁-C₇-Alkyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkyl-sulfonylamino-C₁-C₇-alkyl, Phenyl, Naphthyl, Mono- oder Di-(C₁-C₇-alkoxy)-phenyl oder -naphthyl, C₁-C₇-Alkylendioxy-phenyl, wobei die Oxy-Atome an benachbarte Phenyl-Ringatome gebunden sind, Halogen, Hydroxy, C₁-C₇-Alkoxy, Halogen-C₁-C₇-alkoxy, Hydroxy-C₁-C₇-alkoxy, C₁-C₇-Alkoxy-C₁-C₇-alkoxy, Phenyl- oder Naphthyloxy, (unsubstituiertes oder mono-, di- oder tri-C₁-C₇-alkyl-substituiertes) Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, C₁-C₇-Alkylsulfonylamino, Carboxyl, C₁-C₇-Alkoxycarbonyl, Phenyl- oder Naphthyl-C₁-C₇-alkoxycarbonyl, Carbamoyl und Cyano, C₁-C₇-Alkylsulfonyl, C₁-C₇-Alkylen, welches unsubstituiert oder durch bis zu vier C₁-C₇-Alkyl-Substituenten substituiert ist und an zwei benachbarte Ringatome der Phenyl- bzw. Naphthyl-Einheit gebunden ist; Pyrrolyl, Furanyl und Thienyl steht;
R⁴ für Wasserstoff steht;
L für eine Bindung steht;
und T für Carbonyl, Thiocarbonyl oder vorzugsweise Methylen steht;
und deren pharmazeutisch unbedenkliche Salze.

7. Verbindungen der Formel I nach Anspruch 1 oder 2, wobei
R¹ für Phenylmethyl oder Naphthylmethyl steht, wobei Phenyl und Naphthyl jeweils unsubstituiert sind oder substituiert durch einen oder mehrere, z.B. bis zu drei, Substituenten ausgewählt aus der Gruppe bestehend aus C₁-C₇-Alkyl, Phenyl, Naphthyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkyl-sulfonylamino-C₁-C₇-alkyl, Halogen, Hydroxy, C₁-C₇-alkoxy, Hydroxy-C₁-C₇-alkoxy, C₁-C₇-Alkoxy-C₁-C₇-alkoxy, Phenyl- oder Naphthyloxy, Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, Amino-C₁-C₇-alkyl, Mono- oder Di-(C₁-C₇-alkyl)-amino-C₁-C₇-alkyl, C₁-C₇-Alkanoylamino-C₁-C₇-alkyl, Carboxyl, C₁-C₇-Alkoxy-carbonyl, Phenyl- oder Naphthyl-C₁-C₇-alkoxycarbonyl, Carbamoyl, N-Mono- oder N,N-Di-(C₁-C₇-alkyl- und/oder -(phenyl- oder -naphthyl) -C₁-C₇-alkyl) -carbamoyl, C₁-C₇-Alkylsulfonyl, unsubstituiertem oder C₁-C₇-alkyl-substituiertem Phenyl- oder Naphthylsulfonyl, N-Mono- oder N,N-Di-(C₁-C₇-alkyl- und/oder - (phenyl- oder naphthyl)-C₁-C₇-alkyl)-sulfamoyl und Cyano;
R² für Phenyl steht, das unsubstituiert ist oder substituiert durch einen oder mehrere, z.B. bis zu drei, Substituenten ausgewählt aus der Gruppe bestehend aus C₁-C₇-Alkyl, Phenyl, Naphthyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkyl-sulfonylamino-C₁-C₇-alkyl, Halogen, Hydroxy, C₁-C₇-Alkoxy, Hydroxy-C₁-C₇-alkoxy, C₁-C₇-Alkoxy-C₁-C₇-alkoxy, Phenyl- oder Naphthyloxy, Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, Amino-C₁-C₇-alkyl, Mono- oder Di-(C₁-C₇-alkyl)-amino-C₁-C₇-alkyl, C₁-C₇-Alkanoylamino-C₁-C₇-alkyl, Carboxyl, C₁-C₇-Alkoxy-carbonyl, Phenyl- oder Naphthyl-C₁-C₇-alkoxycarbonyl, Carbamoyl, N-Mono- oder N,N-Di-(C₁-C₇-alkyl- und/oder -(phenyl- oder -naphthyl) -C₁-C₇-alkyl) -carbamoyl, C₁-C₇-Alkylsulfonyl, unsubstituiertem oder C₁-C₇-alkyl-substituiertem Phenyl- oder Naphthylsulfonyl, N-Mono- oder N,N-Di-(C₁-C₇-alkyl und/oder - (phenyl- oder -naphthyl)-C₁-C₇-alkyl)-sulfamoyl und Cyano;
R³ für Phenyl, Naphthyl, Phenyl-C₁-C₇-alkyl oder Naphthyl-C₁-C₇-alkyl, wobei Phenyl und Naphthyl jeweils unsubstituiert sind oder substituiert durch einen oder mehrere, z.B. bis zu drei, Substituenten ausgewählt aus der Gruppe bestehend aus C₁-C₇-Alkyl, Phenyl- oder Naphthyl-C₁-C₇-alkyl, Halogen-C₁-C₇-alkyl, Hydroxy-C₁-C₇-alkyl, C₁-C₇-Alkoxy-C₁-C₇-alkyl, Amino-C₁-C₇-alkyl, Mono- oder Di-(C₁-C₇-alkyl)-amino-C₁-C₇-alkyl, C₁-C₇-Alkanoylamino-C₁-C₇-alkyl, C₁-C₇-Alkyl-sulfonylamino-C₁-C₇-alkyl, Phenyl, Naphthyl, Mono- oder Di-(C₁-C₇-alkoxy)-phenyl oder -naphthyl, C₁-C₇-Alkylendioxyphenyl, wobei die Oxy-Atome an benachbarte Phenyl-Ringatome gebunden sind, Halogen, Hydroxy, C₁-C₇-Alkoxy, Halogen-C₁-C₇-alkoxy, Hydroxy-C₁-C₇-alkoxy, C₁-C₇-Alkoxy-C₁-C₇-alkoxy, Phenyl- oder Naphthyloxy, (unsubstituiertem oder mono-, di- oder tri-C₁-C₇-alkyl substituiertem) Phenyl- oder Naphthyl-C₁-C₇-alkyloxy, C₁-C₇-Alkanoyloxy, Nitro, Amino, Mono- oder Di-(C₁-C₇-alkyl)-amino, C₁-C₇-Alkanoylamino, C₁-C₇-Alkylsulfonylamino, Carboxyl, C₁-C₇-Alkoxy-carbonyl, Phenyl- oder Naphthyl-C₁-C₇-alkoxycarbonyl, Carbamoyl und Cyano, C₁-C₇-Alkylsulfonyl, C₁-C₇-Alkylen, welches unsubstituiert oder durch bis zu vier C₁-C₇-Alkyl-Substituenten substituiert und an zwei benachbarte Ringatome der Phenyl- oder Naphthyl-Einheit gebunden ist; Pyrrolyl, Furanyl und Thienyl;
Phenyl- oder Naphthyl-sulfonyl oder Phenyl-C₁-C₇-alkyl- oder Naphthyl-C₁-C₇-alkyl-sulfonyl, wobei Phenyl und Naphthyl jeweils unsubstituiert sind oder substituiert durch einen oder mehrere, z.B. bis zu drei, Substituenten ausgewählt aus der gerade für substituiertes Phenyl- oder Naphthyl-R³ beschriebenen Gruppe,
C₃-C₁₀-Cycloalkyl oder C₃-C₁₀-Cyclyoalkyl-C₁-C₇-alkyl, wobei in beiden Cycloalkyl unsubstituiert ist oder substituiert durch einen oder mehrere der gerade für substituiertes Phenyl- oder Naphthyl-R³ erwähnten Substituenten, insbesondere durch C₁-C₇-Alkyl, Phenyl, Naphthyl, Phenyl-C₁-C₇-alkyl oder Naphthyl-C₁-C₇-alkyl; oder Heterocyclyl oder Heterocyclyl-C₁-C₇-alkyl, wobei Heterocyclyl ausgewählt ist aus Pyrrolyl, Furanyl, Thienyl, Pyrimidin-2,4-dion-1-, -3- oder -5-yl, Indolyl, Benzofuranyl, Benzimidazolyl, Benzopyrazolyl, Chinolinyl, Isochinolinyl, Methylen-dioxy-phenyl, Ethylen-1,2-dioxy-phenyl oder Trimethylen-1,3-dioxyphenyl, wobei die Oxy-Gruppen an benachbarte Ringatome des Phenylrings gebunden sind, wobei die Heterocyclyl-Einheiten jeweils unsubstituiert oder wie oben für substituiertes Phenyl-R³ erwähnt substituiert sind, steht;
R⁴ für Wasserstoff steht;
L für Methylen steht;
und T für Methylen steht;
und deren pharmazeutisch unbedenkliche Salze.

8. Verbindungen der Formel I nach einem der Ansprüche 1 bis 7 mit der Formel IA, 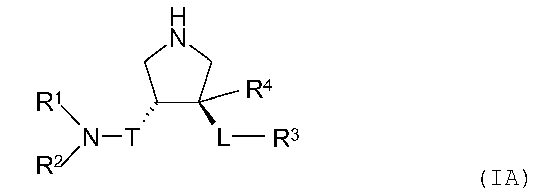 wobei R¹, R², R³, R⁴, T und L wie in einem der vorhergehenden Ansprüche definiert sind, und deren pharmazeutisch unbedenkliche Salze.

9. Verbindungen der Formel I nach einem der Ansprüche 1 bis 7 mit der Formel IB, 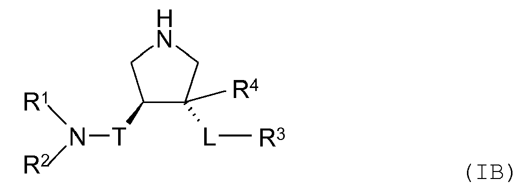 der Formel IC, 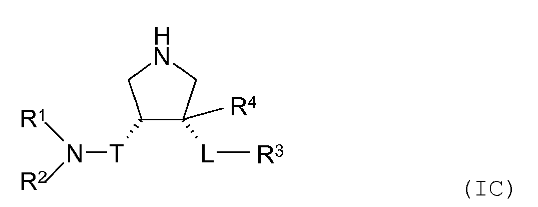 oder der Formel ID, 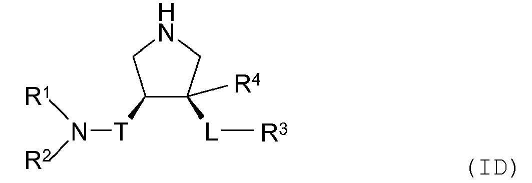 wobei R¹, R², R³, R⁴, T und L wie in einem der vorhergehenden Ansprüche definiert sind, und deren pharmazeutisch unbedenkliche Salze.

10. Verbindungen der Formel I nach einem der vorhergehenden Ansprüche,
wobei
R¹ für unsubstituiertes oder substituiertes Aryl, unsubstituiertes oder substituiertes Aryl-alkyl oder Acyl steht;
R² für unsubstituiertes oder substituiertes Alkyl, unsubstituiertes oder substituiertes Aryl, unsubstituiertes oder substituiertes Cycloalkyl, unsubstituiertes oder substituiertes Aryl-alkyl, unsubstituiertes oder substituiertes mono- oder bicyclisches Heterocyclyl-alkyl, unsubstituiertes oder substituiertes Cycloalkyl-alkyl steht, mit der Maßgabe, dass, wenn L für Methylen (-CH₂-), Oxy (-O-), Thio (-S-) oder unsubstituiertes (-NH-) oder substituiertes Imino steht, R² ausgewählt ist aus einer der erwähnten Gruppen und aus Wasserstoff;
R³ für unsubstituiertes oder substituiertes Alkyl, substituiertes oder unsubstituiertes Aryl, unsubstituiertes oder substituiertes Heterocyclyl, unsubstituiertes oder substituiertes Cycloalkyl, unsubstituiertes oder substituiertes Aryl-alkyl, unsubstituiertes oder substituiertes Heterocyclyl-alkyl, unsubstituiertes oder substituiertes Cycloalkyl-alkyl steht oder, wenn L für Oxy oder unsubstituiertes oder substituiertes Imino steht, eine der gerade erwähnten Bedeutungen hat oder für unsubstituiertes oder substituiertes Alkylcarbonyl, unsubstituiertes oder substituiertes Arylcarbonyl, unsubstituiertes oder substituiertes Heterocyclylcarbonyl, unsubstituiertes oder substituiertes Cycloalkylcarbonyl, unsubstituiertes oder substituiertes Aryl-alkylcarbonyl, unsubstituiertes oder substituiertes Heterocyclyl-alkylcarbonyl, unsubstituiertes oder substituiertes Cycloalkyl-alkylcarbonyl, verethertes Carboxyl, Carbamoyl oder N-mono- oder N,N-disubstituiertes Amino-carbonyl; substituiertes oder unsubstituiertes Alkylsulfonyl, substituiertes oder unsubstituiertes Arylsulfonyl; substituiertes oder unsubstituiertes Aryl-alkylsulfonyl, N-mono- oder N,N-disubstituiertes Amino-sulfonyl steht;
R⁴ für Wasserstoff oder Hydroxy steht;
L für eine Bindung, Methylen (-CH₂-), Oxy (-O-), oder unsubstituiertes (-NH-) oder substituiertes Imino steht, mit der Maßgabe, dass, wenn L für eine Bindung steht, R³ dann für eine der für R³ erwähnten Einheiten mit Ausnahme von substituiertem Alkyl steht;
oder R³ und R⁴, die dann für -O- stehen, zusammen mit L, das dann für Methylen steht, und dem Kohlenstoff, an den R³-L- und R⁴ gebunden sind, einen substituierten oder unsubstituierten Ring bilden, der an unsubstituiertes oder substituiertes Aryl kondensiert ist, und so eine spiro-Verbindung der Formel I bilden, oder
R³ und R⁴ zusammen mit L Oxo (=O) bilden;
und
T für Methylen steht;
und deren Salze.

11. Verbindungen der Formel I nach einem der vorhergehenden Ansprüche,
wobei
L für Methylen steht; und
R³ eine der folgenden Definitionen (a) oder (b) hat:
(a) R³ steht für wie oben definiertes unsubstituiertes oder substituiertes Aryl,
(b) R³ steht für Alkyl, welches gegebenenfalls substituiert ist durch einen oder zwei Substituenten ausgewählt aus der Gruppe bestehend aus O-C₁-C₄-Alkyl, Halogen, Hydroxy, unsubstituiertem oder substituiertem Phenyl, unsubstituiertem oder substituiertem Naphthyl, unsubstituiertem oder substituiertem Phenyl- oder Naphthyloxy, unsubstituiertem oder substituiertem Phenyl- oder Naphthyl-C₁-C₇-alkyloxy, unsubstituiertem oder substituiertem Cycloalkyl, Nitro, Amino, Amino-C₁-C₇-alkyl, N-mono- oder N,N-disubstituiertem Aminocarbonyl, Carboxyl und Cyano.

12. Verbindungen der Formel I nach einem der vorhergehenden Ansprüche,
wobei
L für O oder steht; und
R³ eine der folgenden Definitionen (a) bis (f) hat:
(a) unsubstituiertes oder substituiertes Aryl, wobei geeignete Substituenten ausgewählt sind aus der Gruppe bestehend aus C₁-C₇-Alkyl, -O-C₁-C₇-Alkyl, Halogen-C₁-C₇-alkyl, Halogen, Hydroxy, unsubstituiertem oder substituiertem Phenyl- oder Naphthyloxy, unsubstituiertem oder substituiertem Phenyl- oder Naphthyl-C₁-C₇-alkyloxy, Nitro, Amino, Amino-C₁-C₇-alkyl, Carboxyl und Cyano;
(b) unsubstituiertes oder substituiertes Aryl-alkyl, wobei geeignete Substituenten ausgewählt sind aus der Gruppe bestehend aus C₁-C₇-Alkyl, -O-C₁-C₇-Alkyl, Halogen-C₁-C₇-alkyl, Halogen-C₁-C₇-alkyl-O-, Halogen, Hydroxy, unsubstituiertem oder substituiertem Phenyl, unsubstituiertem oder substituiertem Naphthyl, unsubstituiertem oder substituiertem Phenyl- oder Naphthyloxy, unsubstituiertem oder substituiertem Phenyl- oder Naphthyl-C₁-C₇-alkyloxy, unsubstituiertem oder substituiertem Heterocyclyl, Nitro, Amino, Amino-C₁-C₇-alkyl, Carboxyl und Cyano, wobei, wenn Phenyl, Naphthyl, Phenyl- oder Naphthyloxy, Phenyl- oder Naphthyl-C₁-C₇-alkyloxy, Heterocyclyl substituiert sind, sie vorzugsweise mono- oder disubstituiert sind durch C₁-C₇-Alkyl, -O-C₁-C₇-Alkyl, Halogen-C₁-C₇-alkyl, Halogen, Hydroxy, Amino, Amino-C₁-C₇-alkyl, Acylamino, Heterocyclyl oder Cyano;
(c) unsubstituiertes oder substituiertes Heterocyclyl-alkyl, wobei geeignete Substituenten ausgewählt sind aus der Gruppe bestehend aus Halogen, Hydroxy, unsubstituiertem oder substituiertem Phenyl, unsubstituiertem oder substituiertem Phenyl- oder Naphthyloxy, unsubstituiertem oder substituiertem Phenyl- oder Naphthyl-C₁-C₇-alkyloxy, Nitro, Amino, Amino-C₁-C₇-alkyl, Carboxyl und Cyano;
(d) unsubstituiertes oder substituiertes Alkyl, wobei geeignete Substituenten ausgewählt sind aus der Gruppe bestehend aus O-C₁-C₄-Alkyl, Halogen, Hydroxy, unsubstituiertem oder substituiertem Phenyl, unsubstituiertem oder substituiertem Naphthyl, unsubstituiertem oder substituiertem Phenyl- oder Naphthyloxy, unsubstituiertem oder substituiertem Phenyl- oder Naphthyl-C₁-C₇-alkyloxy, unsubstituiertem oder substituiertem Cycloalkyl, Nitro, Amino, Amino-C₁-C₇-alkyl, N-mono- oder N,N-disubstituiertem Aminocarbonyl, Carboxyl und Cyano;
(e) unsubstituiertes oder substituiertes Aminocarbonyl, das am Stickstoff mono- oder disubstituiert ist durch eine oder mehrere Einheiten ausgewählt aus unsubstituiertem oder substituiertem Alkyl, unsubstituiertem oder substituiertem Aryl oder unsubstituiertem oder substituiertem Cycloalkyl, wobei Alkylsubstituenten ausgewählt sind aus
(i) Aryl, welches unsubstituiert oder weiter durch O-C₁-C₄-Alkyl, Halogen, Hydroxy, unsubstituiertes oder substituiertes Heterocyclyl, Nitro, Amino, Acylamino, Amino-C₁-C₇-alkyl, Carboxyl und Cyano substituiert sein kann;
(ii) Heterocyclyl wie z.B. gegebenenfalls ein Stickstoff- oder Sauerstoffatom enthaltende 5- oder 6-gliedrige Ringe, die unsubstituiert oder weiter durch C₁-C₇-Alkyl, Halogen, Hydroxy, Nitro, unsubstituiertes oder substituiertes Amino, unsubstituiertes oder substituiertes Amino-C₁-C₇-alkyl, Carboxyl und Cyano substituiert sein können;
(iii) Halogen, Hydroxy, Nitro, Amino, Amino-C₁-C₇-alkyl, Carboxyl und Cyano;
(f) unsubstituiertes oder substituiertes Heterocyclylcarbonyl, wobei geeignete Substituenten ausgewählt sind aus der Gruppe bestehend aus C₁-C₇-Alkyl, O-C₁-C₇-Alkyl, Halogen-C₁-C₇-alkyl, Halogen, Hydroxy, unsubstituiertem oder substituiertem Phenyl oder Naphthyl, unsubstituiertem oder substituiertem Phenyl- oder Naphthyloxy, unsubstituiertem oder substituiertem Phenyl- oder Naphthyl-C₁-C₇-alkyloxy, Nitro, Amino, Amino-C₁-C₇-alkyl, Carboxyl und Cyano.

13. Verbindungen der Formel I nach einem der vorhergehenden Ansprüche
wobei
L für NH oder substituiertes NH steht, wobei substituiertes NH substituiert durch Cycloalkyl-alkyl, Alkyl oder durch N-mono- oder N,N-disubstituiertes aminocarbonylsubstituiertes Alkyl bedeutet;
und
R³ eine der folgenden Definitionen (a) bis (m) hat:
(a) unsubstituiertes oder substituiertes Aryl-alkyl, wobei geeignete Substituenten ausgewählt sind aus der Gruppe bestehend aus - (C₀-C₇-Alkylen) - (X)ᵣ- (C₁-C₇-alkylen)-(Y)ₛ-(C₀-C₇-alkylen)-H, wobei r und s für 0 oder 1 stehen und Y und X unabhängig voneinander für O, NH oder -NH-CO-O- stehen, -CO-NH-, NHCO, N(C₁-C₇-Alkyl), Halogen-C₁-C₇-alkyl, Halogen-C₁-C₇-alkyl-O-, Halogen, Hydroxy, unsubstituiertem oder substituiertem Phenyl- oder Naphthyloxy, unsubstituiertem oder substituiertem Phenyl- oder Naphthyl-C₁-C₇-alkyloxy, Nitro, Amino, N(Mono- oder -Di-CO-C₁-C₇-alkyl oder -formyl) unsubstituiertem oder substituiertem Amino, unsubstituiertem oder substituiertem Amino-C₁-C₇-alkyl, wobei die Amino-Einheit durch -C₁-C₇-Alkyl, Cycloalkyl, Phenyl oder Heterocyclyl substituiert sein kann; Carboxyl und Cyano;
(b) unsubstituiertes oder substituiertes Heterocyclyl-alkyl oder unsubstituiertes oder substituiertes Heterocyclyl, wobei es sich bei der Heterocyclyl-Einheit um einen 5-gliedrigen Ring mit einem Stickstoffatom oder ein bicyclisches Ringsystem mit einem Stickstoff- oder Sauerstoffatom handelt, wobei geeignete Substituenten ausgewählt sind aus der Gruppe bestehend aus C₁-C₇-Alkyl, Halogen, Hydroxy, unsubstituiertem oder substituiertem Phenyl, unsubstituiertem oder substituiertem Phenyl- oder Naphthyloxy, unsubstituiertem oder substituiertem Phenyl- oder Naphthyl-C₁-C₇-alkyl, Nitro, Amino, Amino-C₁-C₇-alkyl, Carboxyl und Cyano;
(c) unsubstituiertes oder substituiertes Cycloalkyl, wobei die Substituenten ausgewählt sind aus der Gruppe bestehend aus O-C₁-C₄-Alkyl, Halogen, Hydroxy, unsubstituiertem oder substituiertem Phenyl, Naphthyl, unsubstituiertem oder substituiertem Phenyl- oder Naphthyloxy, unsubstituiertem oder substituiertem Phenyl- oder Naphthyl-C₁-C₇-alkyloxy, Nitro, Amino, Amino-C₁-C₇-alkyl, Carboxyl und Cyano;
(d) unsubstituiertes oder substituiertes Cycloalkyl-alkyl, wobei die Substituenten ausgewählt sind aus der Gruppe bestehend aus O-C₁-C₄-Alkyl, Halogen, Hydroxy, unsubstituiertem oder substituiertem Phenyl, Naphthyl, unsubstituiertem oder substituiertem Phenyl- oder Naphthyloxy, unsubstituiertem oder substituiertem Phenyl- oder Naphthyl-C₁-C₇-alkyloxy, Nitro, unsubstituiertem oder substituiertem Amino, unsubstituiertem oder substituiertem Amino-C₁-C₇-alkyl, wobei die Amino-Einheit durch -C₁-C₇-Alkyl, Cycloalkyl, Phenyl oder Heterocyclyl substituiert sein kann; Carboxyl und Cyano;
(e) unsubstituiertes oder substituiertes Alkyl, wobei die Substituenten ausgewählt sind aus der Gruppe bestehend aus O-C₁-C₄-Alkyl, Halogen, Hydroxy, unsubstituiertem oder substituiertem Phenyl, unsubstituiertem oder substituiertem Naphthyl, unsubstituiertem oder substituiertem Phenyl- oder Naphthyloxy, unsubstituiertem oder substituiertem Phenyl- oder Naphthyl-C₁-C₇-alkyloxy, unsubstituiertem oder substituiertem Cycloalkyl, Nitro, Amino, Amino-C₁-C₇-alkyl, N-mono- oder N,N-disubstituiertem Aminocarbonyl, Carboxyl und Cyano;
(f) unsubstituiertes oder substituiertes Arylcarbonyl, wobei die Substituenten ausgewählt sind aus der Gruppe bestehend aus -(C₀-C₇-Alkylen)-(X)ᵣ-(C₁-C₇-alkylen)-(Y)ₛ-(C₀-C₇-alkylen)-H, wobei r und s für 0 oder 1 stehen und Y und X unabhängig voneinander für O, NH oder -NH-CO-O-stehen, -CO-NH-, NHCO, N(C₁-C₇-Alkyl), Halogen-C₁-C₇-alkyl, Halogen, Hydroxy, unsubstituiertem oder substituiertem Phenyl- oder Naphthyloxy, unsubstituiertem oder substituiertem Phenyl- oder Naphthyl-C₁-C₇-alkyloxy, Nitro, unsubstituiertem oder substituiertem Amino, N(Mono- oder -Di-CO-C₁-C₇-alkyl oder -formyl) -amino, Amino-C₁-C₇-alkyl, Carboxyl und Cyano;
(g) unsubstituiertes oder substituiertes Alkylcarbonyl, wobei die Substituenten ausgewählt sind aus der Gruppe bestehend aus O-C₁-C₄-Alkyl, Halogen, Hydroxy, unsubstituiertem oder substituiertem Phenyl oder Naphthyl, unsubstituiertem oder substituiertem C₃-C₇-Cycloalkyl, oder substituiertem Heterocyclyl, Nitro, Amino, Amino-C₁-C₇-alkyl, Carboxyl und Cyano;
(h) unsubstituiertes oder substituiertes Cycloalkylcarbonyl, wobei geeignete Substituenten ausgewählt sind aus der Gruppe bestehend aus C₁-C₇-Alkyl, O-C₁-C₄-Alkyl, Halogen, Hydroxy, unsubstituiertem oder substituiertem Phenyl, Naphthyl, unsubstituiertem oder substituiertem Phenyl- oder Naphthyloxy, unsubstituiertem oder substituiertem Phenyl- oder Naphthyl-C₁-C₇-alkyloxy, Nitro, Amino, Amino-C₁-C₇-alkyl, Carboxyl und Cyano;
(i) unsubstituiertes oder substituiertes Heterocyclylcarbonyl, wobei die Heterocyclyl-Einheit 6-gliedrige Ringe, die gegebenenfalls ein Sauerstoffatom enthalten, und bicyclische Ringsysteme umfasst, wobei geeignete Substituenten ausgewählt sind aus der Gruppe bestehend aus Halogen, Hydroxy, unsubstituiertem oder substituiertem Phenyl, unsubstituiertem oder substituiertem Phenyl- oder Naphthyloxy, unsubstituiertem oder substituiertem Phenyl- oder Naphthyl-C₁-C₇-alkyl, Nitro, Amino, Amino-C₁-C₇-alkyl, Carboxyl, und Cyano;
(j) unsubstituiertes oder substituiertes verethertes Carboxyl, an welches eine der folgenden Gruppen gebunden ist:
(i) O-Alkyl, welches gegebenenfalls substituiert ist, wobei geeignete Substituenten ausgewählt sind aus der Gruppe bestehend aus O-C₁-C₄-Alkyl, Halogen, Hydroxy, unsubstituiertem oder substituiertem Phenyl, unsubstituiertem oder substituiertem Naphthyl, unsubstituiertem oder substituiertem Phenyl- oder Naphthyloxy, unsubstituiertem oder substituiertem Phenyl- oder Naphthyl-C₁-C₇-alkyloxy, unsubstituiertem oder substituiertem Cycloalkyl, Nitro, Amino, Amino-C₁-C₇-alkyl, N-mono- oder N,N-disubstituiertem Aminocarbonyl;
(ii) O-Aryl, welches substituiert sein kann, wobei geeignete Substituenten ausgewählt sind aus der Gruppe bestehend aus C₁-C₇-Alkyl, O-C₁-C₄-Alkyl, Halogen-C₁-C₇-alkyl, Halogen, Hydroxy, substituiertem oder unsubstituiertem Amino, Acylamino, substituiertem oder unsubstituiertem Amino-C₁-C₇-alkyl, Carboxyl und Cyano;
(iii) O-Cycloalkyl, welches substituiert sein kann, wobei geeignete Substituenten ausgewählt sind aus der Gruppe bestehend aus C₁-C₇-Alkyl, O-C₁-C₄-Alkyl, Halogen, Hydroxy, Nitro, substituiertem oder unsubstituiertem Amino, substituiertem oder unsubstituiertem Amino-C₁-C₇-alkyl, Carboxyl und Cyano;
(k) unsubstituiertes oder substituiertes Aminocarbonyl, das am Stickstoff mono- oder disubstituiert ist durch eine oder mehrere Einheiten ausgewählt aus unsubstituiertem oder substituiertem Alkyl, unsubstituiertem oder substituiertem Aryl, oder unsubstituiertem oder substituiertem Cycloalkyl, wobei ein geeigneter Substituent für die Alkyl-Einheit Aryl ist, und wobei Aryl unsubstituiert oder weiter durch C₁-C₇-Alkyl, O-C₁-C₄-Alkyl, Halogen-C₁-C₇-alkyl, Halogen, Hydroxy, Nitro, Amino, Acylamino, Amino-C₁-C₇-alkyl, Carboxyl und Cyano substituiert sein kann;
(l) unsubstituiertes oder substituiertes Arylsulfonyl, wobei die Substituenten ausgewählt sind aus der Gruppe bestehend aus C₁-C₇-Alkyl, O-C₁-C₄-Alkyl, Halogen-C₁-C₇-alkyl, Halogen, Hydroxy, unsubstituiertem oder substituiertem Phenyl, unsubstituiertem oder substituiertem Phenyl- oder Naphthyloxy, unsubstituiertem oder substituiertem Phenyl- oder Naphthyl-C₁-C₇-alkyl, Amino, Acylamino, Amino-C₁-C₇-alkyl, Carboxyl und Cyano;
(m) unsubstituiertes oder substituiertes Alkylsulfonyl, wobei die Substituenten ausgewählt sind aus der Gruppe bestehend aus O-C₁-C₄-Alkyl, Halogen, Hydroxy, unsubstituiertem oder substituiertem Phenyl oder Naphthyl, unsubstituiertem oder substituiertem Phenyl- oder Naphthyloxy, unsubstituiertem oder substituiertem Phenyl- oder Naphthyl-C₁-C₇-alkyl, unsubstituiertem oder substituiertem C₃-C₇-Cycloalkyl; oder substituiertem oder unsubstituiertem Heterocyclyl, Nitro, Amino, Amino-C₁-C₇-alkyl, Carboxyl und Cyano.

14. Verbindungen der Formel I nach Anspruch 1, ausgewählt aus der Gruppe bestehend aus ((3S*,4S*)-4-Benzyl-pyrrolidin-3-ylmethyl)-(4-chlor-phenyl)-phenylamin; ((3R*,4R*)-4-Benzyl-pyrrolidin-3-ylmethyl)-diphenyl-amin; 3-[((3R*,4R*)-4-Benzyl-pyrrolidin-3-ylmethyl)-phenyl-amino]-phenol; ((3S*,4S*)-4-Benzyl-pyrrolidin-3-ylmethyl)-phenethyl-phenyl-amin; (3R*,4R*)-Benzyl-(4-chlor-phenyl)-[4-(3-isopropylbenzyl)-pyrrolidin-3-ylmethyl]-amin; (3R*,4R*)-(4-Chlor-phenyl)-[4-(3-isopropyl-benzyl)-pyrrolidin-3-ylmethyl]-phenyl-amin; N-((3S*,4R*)-4-Benzylamino-pyrrolidin-3-ylmethyl)-N-isopropyl-4-methoxy-3-(3-methoxy-propoxy)-benzamid; ((3R*,4R*)-4-Benzyl-pyrrolidin-3-ylmethyl)-(4-chlor-phenyl)-cyclohexylamin; ((3R*,4R*)-4-Benzyl-pyrrolidin-3-ylmethyl)-(4-chlor-phenyl)-((R)-1-phenyl-ethyl)-amin; ((3R*,4R*)-4-Benzyl-pyrrolidin-3-ylmethyl)-(4-chlor-3-methoxyphenyl)-phenyl-amin; 3-{[((3R*,4R*)-4-Benzyl-pyrrolidin-3-ylmethyl)-(4-chlor-phenyl)-amino]-methyl}-benzonitril; Benzyl-((3R*,4R*)-4-benzyl-pyrrolidin-3-ylmethyl)-(4-chlor-phenyl)-amin; Benzyl-((3R,4R)-4-benzyl-pyrrolidin-3-ylmethyl)-(4-chlor-phenyl)-amin; Benzyl-((3S,4S)-4-benzyl-pyrrolidin-3-ylmethyl)-(4-chlor-phenyl)-amin; ((3R*,4R*)-4-Benzyl-pyrrolidin-3-ylmethyl)-(4-chlor-phenyl)-(2-methoxy-benzyl)-amin; ((3R*,4R*)-4-Benzyl-pyrrolidin-3-ylmethyl)-(4-chlorphenyl)-(3-methoxy-benzyl)-amin; Benzyl-((3R*,4R*)-4-benzyl-pyrrolidin-3-ylmethyl)-(4-methoxy-phenyl)-amin; Benzyl-((3R*,4R*)-4-benzyl-pyrrolidin-3-ylmethyl)-p-tolyl-amin; Benzyl-((3R*,4R*)-4-benzyl-pyrrolidin-3-ylmethyl)-(4-fluor-phenyl)-amin; ((3R*,4R*)-4-Benzyl-pyrrolidin-3-ylmethyl)-(4-chlor-phenyl)-naphthalin-1-ylmethyl-amin; 3-{[((3R*,4R*)-4-Benzyl-pyrrolidin-3-ylmethyl)-(4-chlor-phenyl)-amino]-methyl}-benzamid; ((3R*,4R*)-4-Benzyl-pyrrolidin-3-ylmethyl)-(4-chlorphenyl)-naphthalin-2-ylmethyl-amin; ((3R*,4R*)-4-Benzyl-pyrrolidin-3-ylmethyl)-(4-chlor-phenyl)-(4-propoxy-2-trifluormethyl-chinolin-6-ylmethyl)-amin; 7-{[((3R*,4R*)-4-Benzyl-pyrrolidin-3-ylmethyl)-(4-chlorphenyl)-amino]-methyl}-naphthalin-2-carbonsäurenitril; 7-{[((3R*,4R*)-4-Benzyl-pyrrolidin-3-ylmethyl)-(4-chlor-phenyl)-amino]-methyl}-naphthalin-1-carbonsäurenitril; 6-{[((3R*,4R*)-4-Benzyl-pyrrolidin-3-ylmethyl)-(4-chlor-phenyl)-amino]-methyl}-naphthalin-2-carbonsäurenitril; 6-{[((3R*,4R*)-4-Benzyl-pyrrolidin-3-ylmethyl)-(4-chlor-phenyl)-amino]-methyl}-1H-pyrimidin-2,4-dion; 5-{[((3R*,4R*)-4-Benzyl-pyrrolidin-3-ylmethyl)-(4-chlor-phenyl)-amino]-methyl}-naphthalin-2-carbonsäurenitril; 5-{[((3R*,4R*)-4-Benzyl-pyrrolidin-3-ylmethyl)-(4-chlor-phenyl)-amino]-methyl}-naphthalin-1-carbonsäurenitril; 4-{[((3R*,4R*)-4-Benzyl-pyrrolidin-3-ylmethyl)-(4-chlor-phenyl)-amino]-methyl}-naphthalin-1-carbonsäurenitril; 4-{[((3R*,4R*)-4-Benzyl-pyrrolidin-3-ylmethyl)-(4-chlorphenyl)-amino]-methyl}-benzonitril; 5-{[((3R*,4R*)-4-Benzyl-pyrrolidin-3-ylmethyl)-(4-chlor-phenyl)-amino]-methyl}-2-fluor-benzonitril; 4-[((3R*,4R*)-4-Benzyl-pyrrolidin-3-ylmethyl)-naphthalin-2-ylmethyl-amino]-benzonitril; 4-{[((3R*,4R*)-4-Benzyl-pyrrolidin-3-ylmethyl)-(4-chlor-benzyl)-amino]-methyl}-benzonitril; N-((3R*,4R*)-4-Benzyl-pyrrolidin-3-ylmethyl)-N-(3-cyano-phenyl)-4-methoxy-3-(3-methoxy-propoxy)-benzamid; N-((3R*,4R*)-4-Benzyl-pyrrolidin-3-ylmethyl)-3,N-diphenyl-propionamidhydrochlorid; (1R*,2R*)-2-Phenyl-CyClopropancarbonsäure-((3R*,4R*)-4-benzyl-pyrrolidin-3-ylmethyl)-phenyl-amid; (1R*,2R*)-2-Phenyl-CyClopropancarbonsäure-((3R*,4R*)-4-benzyl-pyrrolidin-3-ylmethyl)-(4-chlor-phenyl)-amid; Naphthalin-2-carbonsäure-((3R*,4R*)-4-benzyl-pyrrolidin-3-ylmethyl)-(4-chlor-phenyl)-amid; Naphthalin-1-carbonsäure-((3R*,4R*)-4-benzyl-pyrrolidin-3-ylmethyl)-(4-chlorphenyl)-amid; N-((3R*,4R*)-4-Benzyl-pyrrolidin-3-ylmethyl)-N-(4-chlor-phenyl)-4-methoxy-3-(3-methoxy-propoxy)-benzamid; N-((3R*,4R*)-4-Benzyl-pyrrolidin-3-ylmethyl)-4-methoxy-3-(3-methoxy-propoxy)-N-phenyl-benzamid; N-((3R*,4R*)-4-Benzyl-pyrrolidin-3-ylmethyl)-N-(4-cyano-phenyl)-4-methoxy-3-(3-methoxy-propoxy)-benzamid; Naphthalin-2-carbonsäure-((3R*,4R*)-4-benzyl-pyrrolidin-3-ylmethyl)-(4-cyano-phenyl)-amid; Benzofuran-5-carbonsäure-((3R*,4R*)-4-benzyl-pyrrolidin-3-ylmethyl)-(4-cyano-phenyl)-amid; Naphthalin-2-carbonsäure-((3R*,4R*)-4-benzyl-pyrrolidin-3-ylmethyl)-(4-phenoxy-phenyl)-amid; Benzofuran-5-carbonsäure-((3R*,4R*)-4-benzyl-pyrrolidin-3-ylmethyl)-(4-chlor-phenyl)-amid; N-((3R*,4R*)-4-Benzyl-pyrrolidin-3-ylmethyl)-N-(4-chlorphenyl)-2-phenoxy-benzamid; N-((3R*,4R*)-4-Benzyl-pyrrolidin-3-ylmethyl)-N-(4-chlor-phenyl)-benzolsulfonamid; ((3R*,4R*)-4-Benzyl-pyrrolidin-3-ylmethyl)-(4-chlor-phenyl)-phenethyl-amin; N-((3S*,4S*)-4-Benzyl-pyrrolidin-3-ylmethyl)-N-isopropyl-3-(3-methoxy-propoxy)-4-methyl-benzamid; N-((3S*,4S*)-4-Benzyl-pyrrolidin-3-ylmethyl)-N-isopropyl-4-methoxy-3-(3-methoxy-propoxy)-benzamid; N-((3S,4S)-4-Benzyl-pyrrolidin-3-ylmethyl)-N-isopropyl-4-methoxy-3-(3-methoxy-propoxy)-benzamid; N-((3R,4R)-4-Benzyl-pyrrolidin-3-ylmethyl)-N-isopropyl-4-methoxy-3-(3-methoxy-propoxy)-benzamid; Naphthalin-2-carbonsäure-((3R*,4R*)-4-benzyl-pyrrolidin-3-ylmethyl)-isopropyl-amid; N-((3R*,4R*)-4-Benzyl-pyrrolidin-3-ylmethyl)-4-ethyl-N-isopropyl-3-(3-methoxy-propoxy)-benzamid; N-((3R*,4R*)-4-Benzyl-pyrrolidin-3-ylmethyl)-N-isopropyl-3-(3-methoxy-propoxy)-benzamid; 1-(3-Methoxy-propyl)-1H-indol-2-carbonsäure-((3R*,4R*)-4-benzyl-pyrrolidin-3-ylmethyl)-isopropyl-amid; N-((3R*,4R*)-4-Benzyl-pyrrolidin-3-ylmethyl)-N-cyclopentyl-4-methoxy-3-(3-methoxy-propoxy)-benzamid; N-((3R*,4R*)-4-Benzyl-pyrrolidin-3-ylmethyl)-N-cycloproyl-4-methoxy-3-(3-methoxy-propoxy)-benzamid; N-((3R*,4R*)-4-Benzyl-pyrrolidin-3-ylmethyl)-4-methoxy-3-(3-methoxy-propoxy)-N-thiophen-2-ylmethyl-benzamid; N-((3R*,4R*)-4-Benzyl-pyrrolidin-3-ylmethyl)-4-methoxy-N-(2-methoxy-ethyl)-3-(3-methoxy-propoxy)-benzamid; N-((3R*,4R*)-4-Benzyl-pyrrolidin-3-ylmethyl)-N-(4-chlor-benzyl)-4-methoxy-3-(3-methoxy-propoxy)-benzamid; N-((3R*,4R*)-4-Benzyl-pyrrolidin-3-ylmethyl)-4-methoxy-3-(3-methoxy-propoxy)-N-methyl-benzamid; N-((3R*,4R*)-4-Benzyl-pyrrolidin-3-ylmethyl)-N-cyclobutyl-4-methoxy-3-(3-methoxy-propoxy)-benzamid; N-((3R*,4R*)-4-Benzyl-pyrrolidin-3-ylmethyl)-N-(1-ethyl-propyl)-4-methoxy-3-(3-methoxy-propoxy)-benzamid; N-((3R*,4R*)-4-Benzyl-pyrrolidin-3-ylmethyl)-N-cyclopropylmethyl-4-methoxy-3-(3-methoxy-propoxy)-benzamid; N-((3R*,4R*)-4-Benzyl-pyrrolidin-3-ylmethyl)-4-methoxy-3-(3-methoxy-propoxy)-N-(2,2,2-trifluorethyl)-benzamid; N-((3R*,4R*)-4-Benzyl-pyrrolidin-3-ylmethyl)-N-(4-cyano-benzyl)-4-methoxy-3-(3-methoxy-propoxy)-benzamid; N-((3R*,4R*)-4-Benzyl-pyrrolidin-3-ylmethyl)-N-(1,2-dimethyl-propyl)-4-methoxy-3-(3-methoxy-propoxy)-benzamid; N-((3R*,4R*)-4-Benzyl-pyrrolidin-3-ylmethyl)-N-(4-chlor-benzyl)-2-phenoxy-benzamid; N-((3R*,4R*)-4-Benzyl-pyrrolidin-3-ylmethyl)-N-(4-chlor-benzyl)-3-phenoxy-benzamid; N-((3R*,4R*)-4-Benzyl-pyrrolidin-3-ylmethyl)-N-(4-chlor-benzyl)-4-phenoxy-benzamid; ((3S*,4S*)-4-Benzyl-pyrrolidin-3-ylmethyl)-(4-chlor-phenyl)-[4-methoxy-3-(3-methoxy-propoxy)-benzyl]-amin; Benzofuran-5-carbonsäure-((3R*,4R*)-4-benzyl-pyrrolidin-3-ylmethyl)-(4-chlorbenzyl)-amid; N-((3R*,4R*)-4-Benzyl-pyrrolidin-3-ylmethyl)-4-methoxy-3-(3-methoxy-propoxy)-N-(3-phenylpropyl)-benzamid; N-((3R*,4R*)-4-Benzyl-pyrrolidin-3-ylmethyl)-4-methoxy-3-(3-methoxy-propoxy)-N-phenethyl-benzamid; N-((3R*,4R*)-4-Benzyl-pyrrolidin-3-ylmethyl)-4-methoxy-3-(3-methoxy-propoxy)-N-(4-phenyl-butyl)-benzamid; Naphthalin-2-carbonsäure-(4-benzyloxy-phenyl)-((3S*,4S*)-4-benzyl-pyrrolidin-3-ylmethyl)-amid; (3-Aminomethyl-benzyl)-((3R*,4R*)-4-benzyl-pyrrolidin-3-ylmethyl)-(4-chlor-phenyl)-amin; (8-Aminomethyl-naphthalin-2-ylmethyl)-((3R*,4R*)-4-benzyl-pyrrolidin-3-ylmethyl)-(4-chlor-phenyl)-amin; (4-Aminomethyl-phenyl)-benzyl-((3R*,4R*)-4-benzyl-pyrrolidin-3-ylmethyl)-amin; (7-Aminomethyl-naphthalin-2-ylmethyl)-((3R*,4R*)-4-benzyl-pyrrolidin-3-ylmethyl)-(4-chlor-phenyl)-amin; (4-Aminomethyl-naphthalin-l-ylmethyl)-((3R*,4R*)-4-benzyl-pyrrolidin-3-ylmethyl)-(4-chlor-phenyl)-amin; N-(3-{[((3R*,4R*)-4-Benzyl-pyrrolidin-3-ylmethyl)-(4-chlor-phenyl)-amino]-methyl}-benzyl)-acetamid; N-(3-{[((3R*,4R*)-4-Benzyl-pyrrolidin-3-ylmethyl)-(4-chlor-phenyl)-amino]-methyl}-benzyl)-formamid; ((3R*,4R*)-4-Benzyl-pyrrolidin-3-ylmethyl)-(4-chlor-phenyl)-(3-dimethylaminomethyl-benzyl)-amin; ((3R*,4R*)-4-Benzyl-pyrrolidin-3-ylmethyl)-(4-chlor-phenyl)-(3-amino-benzyl)-amin; ((3R*4R*)-4-Benzyl-pyrrolidin-3-ylmethyl)-isopropyl-[4-methoxy-3-(3-methoxy-propoxy)-benzyl]-amin; N-((3R*,4R*)-4-Benzyl-pyrrolidin-3-ylmethyl)-N-isopropyl-4-methoxy-3-(2-methoxy-ethoxy)-benzamid; ((3R*,4R*)-4-Benzyl-pyrrolidin-3-ylmethyl)-(4-chlor-phenyl)-[2-(3-methoxy-propoxy)-benzyl]-amin; ((3R*,4R*)-4-Benzyl-pyrrolidin-3-ylmethyl)-(4-chlor-phenyl)-[2-(2-methoxy-ethoxy)-benzyl]-amin; 2-{[((3R*,4R*)-4-Benzyl-pyrrolidin-3-ylmethyl)-(4-chlor-phenyl)-amino]-methyl}-phenol; 1-(3-Methoxy-propyl)-1H-indol-6-carbonsäure-((3R*,4R*)-4-benzyl-pyrrolidin-3-ylmethyl)-isopropyl-amid; 1-(2-Methoxy-ethyl)-1H-indol-6-carbonsäure-((3R*,4R*)-4-benzyl-pyrrolidin-3-ylmethyl)-isopropyl-amid; Benzyl-(4-chlor-phenyl)-((3R*,4R*)-4-phenethyl-pyrrolidin-3-ylmethyl)-amin; ((3S*,4R*)-4-Benzyl-pyrrolidin-3-ylmethyl)-(4-chlor-phenyl)-phenyl-amin; N-((3S*,4S*)-4-Benzyloxy-pyrrolidin-3-ylmethyl)-N-isopropyl-4-methoxy-3-(3-methoxy-propoxy)-benzamid; N-((3S*,4R*)-4-Benzyloxy-pyrrolidin-3-ylmethyl)-N-isopropyl-4-methoxy-3-(3-methoxy-propoxy)-benzamid; N-((3S*,4S*)-4-Hydroxy-pyrrolidin-3-ylmethyl)-N-isopropyl-4-methoxy-3-(3-methoxy-propoxy)-benzamid; N-((3S*,4R*)-4-Hydroxy-pyrrolidin-3-ylmethyl)-N-isopropyl-4-methoxy-3-(3-methoxy-propoxy)-benzamid; Benzyl-((3S*,4R*)-4-benzyloxy-pyrrolidin-3-ylmethyl)-(4-chlor-phenyl)-amin; N-Isopropyl-4-methoxy-3-(3-methoxy-propoxy)-N-[(3S*,4S*)-4-(4-trifluormethyl-phenoxy)-pyrrolidin-3-ylmethyl]-benzamid; N-Isopropyl-4-methoxy-3-(3-methoxy-propoxy)-N-[(3S*,4R*)-4-(4-trifluormethyl-phenoxy)-pyrrolidin-3-ylmethyl]-benzamid; Benzyl-(4-chlor-phenyl)-[(3S*,4R*)-4-(4-trifluormethyl-phenoxy)-pyrrolidin-3-ylmethyl]-amin; (3R*,4R*)-(4-Chlor-phenyl)-[4-(3-isopropyl-phenoxy)-pyrrolidin-3-ylmethyl]-phenyl-amin; (3R*4R*)-Benzyl-(4-chlor-phenyl)-[4-(3-isopropyl-phenoxy)-pyrrolidin-3-ylmethyl]-amin; (3R*,4R*)-(4-Chlor-phenyl)-[4-(3-isopropyl-phenoxy)-pyrrolidin-3-ylmethyl]-(2-methoxybenzyl)-amin; (3R*4R*)-(4-Chlor-phenyl)-[4-(3-isopropyl-phenoxy)-pyrrolidin-3-ylmethyl]-(3-methoxybenzyl)-amin; (3R*,4R*)-Benzo[1,2,5]oxadiazol-5-ylmethyl-(4-chlor-phenyl)-[4-(3-isopropyl-phenoxy)-pyrrolidin-3-ylmethyl]-amin; N-((3S*,4R*)-4-Benzyl-4-hydroxy-pyrrolidin-3-ylmethyl)-N-isopropyl-4-methoxy-3-(3-methoxy-propoxy)-benzamid; N-[(3S*,4R*)-4-(2-Fluorbenzyl)-4-hydroxy-pyrrolidin-3-ylmethyl]-N-isopropyl-4-methoxy-3-(3-methoxy-propoxy)-benzamid; N-Isopropyl-4-methoxy-3-(3-methoxy-propoxy)-N-((S)-4-oxo-pyrrolidin-3-ylmethyl)-benzamid; N-((3R*,4R*)-4-Cyclohexylamino-pyrrolidin-3-ylmethyl)-N-isopropyl-4-methoxy-3-(3-methoxy-propoxy)-benzamid; N-Isopropyl-N-((3R*,4R*)-4-isopropylamino-pyrrolidin-3-ylmethyl)-4-methoxy-3-(3-methoxy-propoxy)-benzamid; N-Isopropyl-4-methoxy-3-(3-methoxy-propoxy)-N-[(3R*,4R*)-4-(toluol-4-sulfonylamino)-pyrrolidin-3-ylmethyl]-benzamid; N-Isopropyl-4-methoxy-N-[(3R*,4R*)-4-(4-methoxy-benzolsulfonylamino)-pyrrolidin-3-ylmethyl]-3-(3-methoxy-propoxy)-benzamid; N-Isopropyl-4-methoxy-N-[(3R*,4R*)-4-(3-methoxy-benzolsulfonylamino)-pyrrolidin-3-ylmethyl]-3-(3-methoxy-propoxy)-benzamid; N-Isopropyl-4-methoxy-3-(3-methoxy-propoxy)-N-[(3R*,4R*)-4-(naphthalin-2-sulfonylamino)-pyrrolidin-3-ylmethyl]-benzamid; N-Isopropyl-4-methoxy-3-(3-methoxy-propoxy)-N-((3R*,4R*)-4-phenylmethansulfonylamino-pyrrolidin-3-ylmethyl)-benzamid; N-((3R*,4R*)-4-Benzylamino-pyrrolidin-3-ylmethyl)-N-isopropyl-4-methoxy-3-(3-methoxy-propoxy)-benzamid; [(3R*,4R*)-4-({Isopropyl-[4-methoxy-3-(3-methoxy-propoxy)-benzoyl]-amino}-methyl)-pyrrolidin-3-yl]-carbaminsäureisopropyl-ester; N-[(3S*,4S*)-4-(Cyclopropylcarbamoyl-methyl-amino)-pyrrolidin-3-ylmethyl]-N-isopropyl-4-methoxy-3-(3-methoxy-propoxy)-benzamid; N-((3R*,4R*)-4-Benzyl-pyrrolidin-3-ylmethyl)-N-isopropyl-4-methoxy-3-(4-methoxy-butyl)-benzamid; N-((3R*,4R*)-4-Benzyl-pyrrolidin-3-ylmethyl)-N-isopropyl-4-methoxy-3-(3-methoxy-propylamino)-benzamid; 1-(3-Methoxy-propyl)-3-methyl-1H-indol-6-carbonsäure-((3R,4R)-4-benzyl-pyrrolidin-3-ylmethyl)-isopropyl-amid; 3-Benzyloxy-N-((3S*,4S*)-4-benzyl-pyrrolidin-3-ylmethyl)-N-isopropyl-4-methoxy-benzamid; N-Isopropyl-4-methoxy-3-(3-methoxy-propoxy)-N-pyrrolidin-3-ylmethyl-benzamid; N-(2-{[((3R*,4R*)-4-Benzyl-pyrrolidin-3-ylmethyl)-(4-chlorphenyl)-amino]-methyl}-phenyl)-acetamid; N-((3R*,4R*)-4-Benzyl-pyrrolidin-3-ylmethyl)-3-(2-ethoxy-ethoxy)-N-isopropyl-4-methoxy-benzamid; N-((3S*,4S*)-4-Benzyl-pyrrolidin-3-ylmethyl)-N-isopropyl-4-methoxy-3-(2-methoxy-ethoxymethyl)-benzamid; N-((3R*,4R*)-4-Benzyl-pyrrolidin-3-ylmethyl)-3-(3-hydroxy-propoxy)-N-isopropyl-4-methoxy-benzamid; 1-(3-Methoxy-propyl)-1H-indol-5-carbonsäure-((3R*,4R*)-4-benzyl-pyrrolidin-3-ylmethyl)-isopropyl-amid; 1-(2-Methoxy-ethyl)-1H-indol-5-carbonsäure-((3R*,4R*)-4-benzyl-pyrrolidin-3-ylmethyl)-isopropyl-amid; (3R*,4R*)-(4-Chlor-phenyl)-[4-(3-isopropyl-phenyl)-pyrrolidin-3-ylmethyl]-phenyl-amin; (3R*,4R*)-Benzyl-(4-chlor-3-methoxy-phenyl)-[4-(3-isopropyl-phenyl)-pyrrolidin-3-ylmethyl]-amin; (3R*,4R*)-(4-Chlor-3-methoxy-phenyl)-[4-(3-isopropylphenyl)-pyrrolidin-3-ylmethyl]-phenyl-amin; N-((3R*,4S*)-4-Benzyl-pyrrolidin-3-ylmethyl)-N-isopropyl-4-methoxy-3-(3-methoxy-propoxy)-benzamid; N-Isopropyl-4-methoxy-N-[(3S*,4S*)-4-(3-methoxy-benzyloxy)-pyrrolidin-3-ylmethyl]-3-(3-methoxy-propoxy)-benzamid; N-Isopropyl-4-methoxy-3-(3-methoxy-propoxy)-N-[(3S*,4S*)-4-(naphthalin-2-ylmethoxy)-pyrrolidin-3-ylmethyl]-benzamid; N-[(3S*,4S*)-4-(3,5-Dimethoxy-benzyloxy)-pyrrolidin-3-ylmethyl]-N-isopropyl-4-methoxy-3-(3-methoxy-propoxy)-benzamid; N-[(3S*,4S*)-4-(3-Ethoxy-benzyloxy)-pyrrolidin-3-ylmethyl]-N-isopropyl-4-methoxy-3-(3-methoxy-propoxy)-benzamid; N-[(3S*,4S*)-4-(3-Isopropoxy-benzyloxy)-pyrrolidin-3-ylmethyl]-N-isopropyl-4-methoxy-3-(3-methoxy-propoxy)-benzamid; N-[(3S*,4S*)-4-(3-Cyano-benzyloxy)-pyrrolidin-3-ylmethyl]-N-isopropyl-4-methoxy-3-(3-methoxy-propoxy)-benzamid; N-Isopropyl-4-methoxy-3-(3-methoxy-propoxy)-N-[(3S,4S)-4-(3-trifluormethoxy-benzyloxy)-pyrrolidin-3-ylmethyl]-benzamid; N-Isopropyl-4-methoxy-3-(3-methoxy-propoxy)-N-[(3S,4S)-4-(3-propoxy-benzyloxy)-pyrrolidin-3-ylmethyl]-benzamid; N-[(3S,4S)-4-(Biphenyl-3-ylmethoxy)-pyrrolidin-3-ylmethyl]-N-isopropyl-4-methoxy-3-(3-methoxy-propoxy)-benzamid; N-Isopropyl-4-methoxy-3-(3-methoxy-propoxy)-N-[(3S,4S)-4-(3-phenoxy-benzyloxy)-pyrrolidin-3-ylmethyl]-benzamid; N-[(3S*,4S*)-4-(3,5-Diethoxy-benzyloxy)-pyrrolidin-3-ylmethyl]-N-isopropyl-4-methoxy-3-(3-methoxy-propoxy)-benzamid; N-Isopropyl-4-methoxy-3-(3-methoxy-propoxy)-N-[(3S*,4S*)-4-(5,5,8,8-tetramethyl-5,6,7,8-tetrahydro-naphthalin-2-ylmethoxy)-pyrrolidin-3-ylmethyl]-benzamid; N-[(3S*,4S*)-4-(3,4-Dihydro-2H-benzo[b][1,4]dioxepin-7-ylmethoxy)-pyrrolidin-3-ylmethyl]-N-isopropyl-4-methoxy-3-(3-methoxy-propoxy)-benzamid; N-[(3S*,4S*)-4-(7-Cyano-naphthalin-2-ylmethoxy)-pyrrolidin-3-ylmethyl]-N-isopropyl-4-methoxy-3-(3-methoxy-propoxy)-benzamid; N-[(3S*,4S*)-4-(2,3-Dimethoxy-benzyloxy)-pyrrolidin-3-ylmethyl]-N-isopropyl-4-methoxy-3-(3-methoxy-propoxy)-benzamid; N-[(3S*,4S*)-4-(3-Benzyloxy-benzyloxy)-pyrrolidin-3-ylmethyl]-N-isopropyl-4-methoxy-3-(3-methoxy-propoxy)-benzamid; N-Isopropyl-4-methoxy-3-(3-methoxy-propoxy)-N-[(3S*,4S*)-4-(3-pyrrol-1-yl-benzyloxy)-pyrrolidin-3-ylmethyl]-benzamid; N-[(3S*,4S*)-4-(Benzofuran-5-ylmethoxy)-pyrrolidin-3-ylmethyl]-N-isopropyl-4-methoxy-3-(3-methoxy-propoxy)-benzamid; N-[(3S*,4S*)-4-(2,3-Dihydro-benzo[1,4]dioxin-5-ylmethoxy)-pyrrolidin-3-ylmethyl]-N-isopropyl-4-methoxy-3-(3-methoxy-propoxy)-benzamid; N-[(3S*,4S*)-4-(3,4-Dimethyl-benzyloxy)-pyrrolidin-3-ylmethyl]-N-isopropyl-4-methoxy-3-(3-methoxy-propoxy)-benzamid; N-[(3S*,4S*)-4-(3,4-Dihydro-2H-benzo[b][1,4]dioxepin-6-ylmethoxy)-pyrrolidin-3-ylmethyl]-N-isopropyl-4-methoxy-3-(3-methoxy-propoxy)-benzamid; N-Isopropyl-4-methoxy-3-(3-methoxy-propoxy)-N-[(3S*,4S*)-4-(naphthalin-1-ylmethoxy)-pyrrolidin-3-ylmethyl]-benzamid; N-Isopropyl-4-methoxy-N-{(3S,4S)-4-[3-(4-methoxy-phenoxy)-benzyloxy]-pyrrolidin-3-ylmethyl}-3-(3-methoxy-propoxy)-benzamid; N-[(3S*,4S*)-4-(3-Benzo[1,3]dioxol-5-yl-benzyloxy)-pyrrolidin-3-ylmethyl]-N-isopropyl-4-methoxy-3-(3-methoxy-propoxy)-benzamid; N-Isopropyl-4-methoxy-N-[(3S,4S)-4-(2'-methoxy-biphenyl-3-ylmethoxy)-pyrrolidin-3-ylmethyl]-3-(3-methoxy-propoxy)-benzamid; N-[4-(2-Chlor-6-fluorbenzyl)-4-hydroxy-pyrrolidin-3-ylmethyl]-N-isopropyl-4-methoxy-3-(3-methoxy-propoxy)-benzamid; N-((3R*,4R*)-4-Benzylamino-pyrrolidin-3-ylmethyl)-N-isopropyl-4-methoxy-3-(3-methoxy-propoxy)-benzamid; N-Isopropyl-4-methoxy-3-(3-methoxy-propoxy)-N-[(3R*,4R*)-4-(1-phenyl-ethylamino)-pyrrolidin-3-ylmethyl]-benzamid; N-((3R*,4R*)-4-Isobutylamino-pyrrolidin-3-ylmethyl)-N-isopropyl-4-methoxy-3-(3-methoxy-propoxy)-benzamid; N-((3R*,4R*)-4-Cyclobutylamino-pyrrolidin-3-ylmethyl)-N-isopropyl-4-methoxy-3-(3-methoxy-propoxy)-benzamid; N-((3R*,4R*)-4-Cyclopentylamino-pyrrolidin-3-ylmethyl)-N-isopropyl-4-methoxy-3-(3-methoxy-propoxy)-benzamid; N-[(3R*,4R*)-4-(Cyclopentylmethyl-amino)-pyrrolidin-3-ylmethyl]-N-isopropyl-4-methoxy-3-(3-methoxy-propoxy)-benzamid; N-Isopropyl-4-methoxy-3-(3-methoxy-propoxy)-N-((3R*,4R*)-4-phenethylamino-pyrrolidin-3-ylmethyl)-benzamid; und N-Isopropyl-4-methoxy-3-(3-methoxy-propoxy)-N-{(3R*,4R*)-4-[(naphthalin-2-ylmethyl)-amino]-pyrrolidin-3-ylmethyl}-benzamid;
und aus den Verbindungen der Formeln 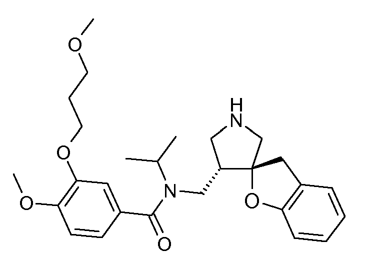 und 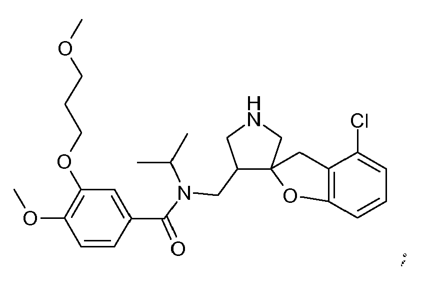 und deren pharmazeutisch unbedenklichen Salzen.

15. Verbindungen der Formel I nach Anspruch 1, ausgewählt aus der Gruppe von Verbindungen bestehend aus
Benzyl-carbaminsäure-(3S*,4S*)-4-({isopropyl-[4-methoxy-3-(3-methoxy-propoxy)-benzoyl]-amino}-methyl)-pyrrolidin-3-yl-ester; und
N-Isopropyl-4-methoxy-3-(3-methoxy-propoxy)-N-[(3R*,4R*)-4-(naphthalin-2-sulfonylamino)-pyrrolidin-3-ylmethyl]-benzamid;
und deren pharmazeutisch unbedenkliche Salze.

16. Verbindungen der Formel I und deren pharmazeutisch unbedenkliche Salze nach einem der Ansprüche 1 bis 15 zur Verwendung bei der diagnostischen oder therapeutischen Behandlung eines Warmblüters.

17. Verbindungen der Formel I und deren pharmazeutisch unbedenkliche Salze nach einem der Ansprüche 1 bis 15 zur Verwendung nach Anspruch 14 bei der Behandlung einer Krankheit, die von der Reninaktivität abhängt.

18. Verwendung einer Verbindung der Formel I oder eines pharmazeutisch unbedenklichen Salzes davon nach einem der Ansprüche 1 bis 15 zur Herstellung einer pharmazeutischen Zusammensetzung zur Behandlung einer Krankheit, die von der Reninaktivität abhängt.

19. Pharmazeutische Formulierung, enthaltend eine Verbindung der Formel I oder ein pharmazeutisch unbedenkliches Salz davon nach einem der Ansprüche 1 bis 15 und wenigstens ein pharmazeutisch unbedenkliches Trägermaterial.

20. Verfahren zur Herstellung einer Verbindung der Formel I oder eines pharmazeutisch unbedenklichen Salzes davon nach einem der Ansprüche 1 bis 15, bei dem man
A) zur Synthese einer Verbindung der Formel I, in welcher T für Methylen, Carbonyl oder Thiocarbonyl steht und R¹, R², R³, R⁴ und T die oben oder unten für eine Verbindung der Formel I angegebenen Bedeutungen haben, eine Säure der Formel II, 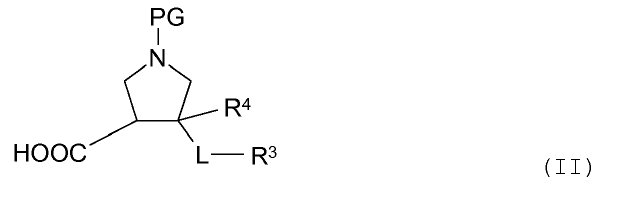 oder ein reaktives Derivat davon, in welchem R³, R⁴ und L wie für eine Verbindung der Formel I definiert sind und PG für eine Schutzgruppe steht, mit
(i) einer Aminoverbindung der Formel III,
R¹R²NH (III)
in welcher R¹ und R² wie für eine Verbindung der Formel I definiert sind, unter Kondensationsbedingungen umsetzt und
(a) zur Darstellung einer Verbindung der Formel I, in welcher T für Carbonyl steht und in welcher R¹, R², R³, R⁴, L und PG wie für Verbindungen der Formel I definiert sind, Schutzgruppen entfernt oder
(b) , falls gewünscht, die Carbonylgruppe in der erhältlichen Verbindung der Formel IV (einer speziellen Verbindung der Formel I), 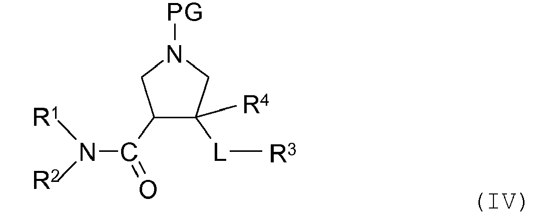 in welcher R¹, R², R³, R⁴, L und PG wie für Verbindungen der Formel II und III definiert sind, zu einer Methylengruppe reduziert, und zur Darstellung einer Verbindung der Formel I, in welcher R¹, R², R³, R⁴, L und PG wie für Verbindungen der Formel I definiert sind und T für Methylen steht, Schutzgruppen entfernt;
oder
(ii) mit einer Aminoverbindung der Formel V,
R²-NH₂ (V)
in welcher R² wie für eine Verbindung der Formel I definiert ist, zu einer Verbindung der Formel VI umsetzt, 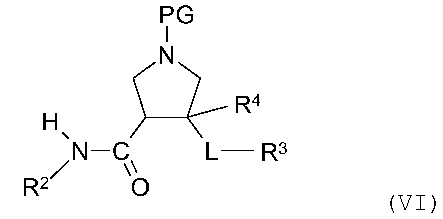 in welcher R², R³, R⁴ und L wie für eine Verbindung der Formel I definiert sind und PG für eine Schutzgruppe steht, und entweder
(a) die Carbonylgruppe reduziert, wodurch man eine Verbindung der Formel VII 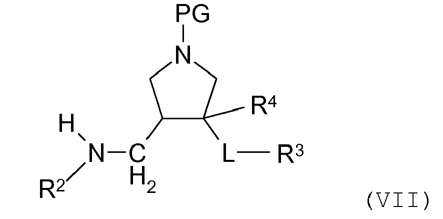 erhält, in welcher R², R³, R⁴, L und PG wie für eine Verbindung der Formel VI definiert sind, und die Verbindung der Formel VII mit einer Verbindung der Formel VIII,
R¹-Z (VIII)
in welcher R¹ wie für eine Verbindung der Formel I definiert ist und Z für eine Abgangsgruppe steht, umsetzt, und, zur Darstellung einer Verbindung der Formel I, in welcher T für Methylen steht und R¹, R², R³, R⁴ und L wie für eine entsprechende Verbindung der Formel I definiert sind, Schutzgruppen entfernt;
oder
(b) die Verbindung der Formel VI mit einer wie oben definierten Verbindung der Formel VIII umsetzt und, zur Darstellung einer Verbindung der Formel I, in welcher T für Carbonyl steht und R¹, R², R³, R⁴ und L wie für eine Verbindung der Formel I definiert sind, Schutzgruppen entfernt;
B) zur Synthese einer Verbindung der Formel I, in welcher T für Methylen steht und R¹, R², R³, R⁴ und T die oben oder unten für eine Verbindung der Formel I angegebenen Bedeutungen haben, ein Aldehyd der Formel IX, 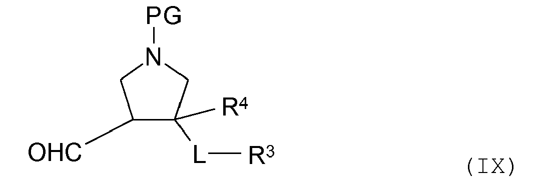 in welcher R³, R⁴ und L wie für eine Verbindung der Formel I definiert sind und PG für eine Schutzgruppe steht, entweder
(i) unter den Bedingungen einer reduktiven Aminierung mit einer wie oben definierten Aminoverbindung der Formel III umsetzt und zur Darstellung einer Verbindung der Formel I, in welcher R¹, R², R³, R⁴ und L wie für eine Verbindung der Formel I definiert sind und T für Methylen steht, Schutzgruppen entfernt; oder
(ii) unter den Bedingungen einer reduktiven Aminierung mit einer wie oben definierten Aminoverbindung der Formel V umsetzt, wodurch man eine Verbindung der Formel X 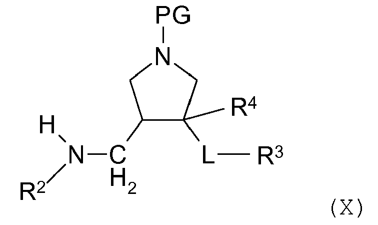 erhält, in welcher R², R³, R⁴ und L wie für eine Verbindung der Formel I definiert sind und PG für eine Schutzgruppe steht, und dann die Verbindung der Formel X
(I) mit einer wie oben definierten Verbindung der Formel VIII umsetzt oder
(II) zur Einführung einer R¹-Einheit, die über eine Methylengruppe gebunden ist, die Teil dieser R¹-Einheit ist, unter den Bedingungen einer reduktiven Aminierung mit einem Aldehyd der Formel VIII*
R¹*-CHO (VIII*)
in welcher R¹* für eine die so erhältliche R¹*-CH₂-Einheit zu einer entsprechenden R¹-Einheit in der erhaltenen Verbindung komplementierende Einheit steht, umsetzt,
und zur Darstellung einer Verbindung der Formel I, in welcher T für Methylen steht und R¹, R², R³, R⁴ und L wie für eine Verbindung der Formel I definiert sind, Schutzgruppen entfernt;
C) zur Synthese einer Verbindung der Formel I, in welcher R¹, R² und T wie für eine Verbindung der Formel I definiert sind, R³ für unsubstituiertes oder substituiertes Alkyl, substituiertes oder unsubstituiertes Aryl, unsubstituiertes oder substituiertes Heterocyclyl, unsubstituiertes oder substituiertes unsubstituiertes oder substituiertes Cycloalkyl, unsubstituiertes oder substituiertes Aryl-alkyl, unsubstituiertes oder substituiertes Heterocyclyl-alkyl, unsubstituiertes oder substituiertes Cycloalkyl-alkyl, substituiertes oder unsubstituiertes Alkylsulfonyl, substituiertes oder unsubstituiertes Arylsulfonyl, substituiertes oder unsubstituiertes Heterocyclylsulfonyl, substituiertes oder unsubstituiertes Cycloalkylsulfonyl, unsubstituiertes oder substituiertes Alkylcarbonyl, unsubstituiertes oder substituiertes Arylcarbonyl, unsubstituiertes oder substituiertes heterocyclylcarbonylverethertes Carboxyl oder N-mono- oder N,N-disubstituiertes Amino-sulfonyl steht, R⁴ für Wasserstoff steht und L für Oxy, Thio oder unsubstituiertes oder substituiertes Imino steht, eine Verbindung der Formel XI, 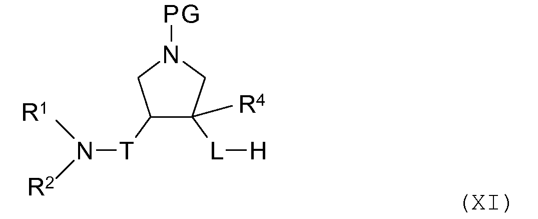 in welcher R¹, R², R⁴ und T wie gerade definiert sind, PG für eine Schutzgruppe steht und L für Oxy, Thio oder unsubstituiertes oder substituiertes Imino steht,
(i) mit einer Verbindung der Formel XII,
R³-Z (XII)
in welcher Z für eine Abgangsgruppe steht und R³ wie gerade definiert ist, umsetzt
oder
(ii) wenn L für Imino oder monosubstituiertes Imino steht, unter den Bedingungen einer reduktiven Aminierung mit einem Aldehyd der Formel XIIA
R³*-CHO (XIIA)
in welcher R³* für eine die so erhältliche R³*-CH₂-Einheit zu einer entsprechenden R³-Einheit in der erhaltenen Verbindung vervollständigende Einheit steht, umsetzt,
und zur Darstellung einer entsprechenden Verbindung der Formel I Schutzgruppen entfernt;
D) zur Darstellung einer Verbindung der Formel I, in welcher R¹, R² und T wie unter Formel I definiert sind und R³ und R⁴ zusammen mit L Oxo, Thioxo oder unsubstituiertes oder substituiertes Imino bilden, eine wie oben definierte Verbindung der Formel XI, in welcher jedoch L für Oxy steht, zu einer entsprechenden Oxoverbindung der Formel XIII, 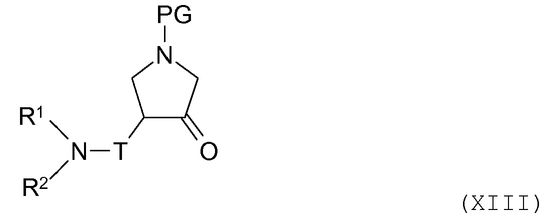 in welcher R¹, R² und T wie unter Formel I definiert sind, oxidiert und, falls gewünscht, die Oxogruppe in eine Thioxo- oder unsubstituierte oder substituierte Iminogruppe umwandelt, und zur Darstellung einer entsprechenden Verbindung der Formel I die Schutzgruppe(n) entfernt;
E) zur Synthese einer Verbindung der Formel I, in welcher R¹, R², L und T wie für eine Verbindung der Formel I definiert sind, R³ für unsubstituiertes oder substituiertes Alkyl, substituiertes oder unsubstituiertes Aryl, unsubstituiertes oder substituiertes Heterocyclyl, unsubstituiertes oder substituiertes Cycloalkyl, unsubstituiertes oder substituiertes Aryl-alkyl, unsubstituiertes oder substituiertes Heterocyclyl-alkyl oder unsubstituiertes oder substituiertes Cycloalkyl-alkyl steht und R⁴ für Hydroxy steht, eine wie oben definierte Verbindung der Formel XIII mit einem Metalloreagens der Formel XIV,
R³-L-Mg-Hal (XIV)
in welcher R³ wie gerade definiert ist und Hal für Halogen steht, umsetzt, und zur Darstellung einer entsprechenden Verbindung der Formel I die Schutzgruppen entfernt;
F) zur Synthese einer spiro-Verbindung der Formel I, in welcher R¹, R² und T wie für eine Verbindung der Formel I definiert sind und R³ und R⁴, die dann für -O- stehen, zusammen mit L, das dann für Methylen steht, und dem Kohlenstoff, an den R³-L- und R⁴ gebunden sind, einen substituierten oder unsubstituierten Ring bilden, der an unsubstituiertes oder substituiertes Aryl, unsubstituiertes oder substituiertes Heterocyclyl oder unsubstituiertes oder substituiertes Cycloalkyl kondensiert ist, eine Verbindung der Formel XV, 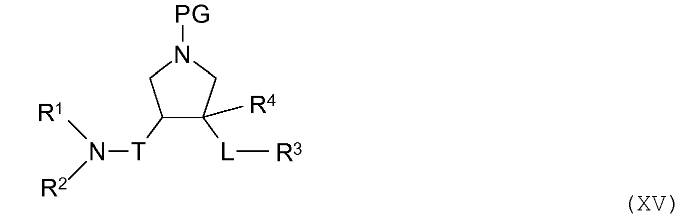 in welcher R¹, R² und T wie für eine Verbindung der Formel I definiert sind, R³ für substituiertes oder unsubstituiertes Aryl, unsubstituiertes oder substituiertes Heterocyclyl, oder unsubstituiertes oder substituiertes Cycloalkyl, die jeweils eine Abgangsgruppe tragen, steht, L für Methylen steht und R⁴ für Hydroxy steht, in Gegenwart einer starken Base umsetzt, zur Darstellung einer entsprechenden spiro-Verbindung der Formel I, wobei die Schutzgruppen entfernt werden;
G) zur Synthese einer Verbindung der Formel I, in welcher R¹, R² und L wie für eine Verbindung der Formel I definiert sind, R⁴ für Wasserstoff steht, L für Oxy, Thio oder Imino steht und R³ für N-monosubstituiertes Amino-carbonyl steht, eine wie oben unter C) gezeigte Verbindung der Formel XI, in welcher L für Oxy, Thio oder Imino steht und die anderen Einheiten wie oben beschrieben sind, mit einer Isocyanatverbindung der Formel XIB,
R³**-NCO (XIIB)
in welcher R³** für einen Substituenten steht, der das entsprechende N-monosubstituierte Amino-carbonyl vervollständigt, umsetzt und die Schutzgruppen entfernt, wodurch man die entsprechende Verbindung der Formel I erhält; oder
H) zur Synthese einer Verbindung der Formel I, in welcher R¹, R² und T wie für eine Verbindung der Formel I definiert sind, L für Oxy, Thio oder unsubstituiertes oder substituiertes Imino steht und R³ wie oben definiert ist, ein reaktives Derivat einer wie oben unter C) definierten Verbindung der Formel XI, in welcher anstelle von -L-H eine Abgangsgruppe vorhanden ist, R⁴ für Wasserstoff steht und die anderen Einheiten wie unter C) definiert sind, mit einer Verbindung der Formel XIIC,
R³-L-H (XIIC)
in welcher R³ wie für eine Verbindung der Formel I definiert ist und L für Oxy, Thio oder unsubstituiertes oder substituiertes Imino steht, umsetzt und Schutzgruppen entfernt, wodurch man die entsprechende Verbindung der Formel I erhält;
und, falls erwünscht, im Anschluss an eines oder mehrere der unter (A) bis (H) erwähnten Verfahren eine erhältliche Verbindung der Formel I oder eine geschützte Form davon in eine andere Verbindung der Formel I umwandelt, ein Salz einer erhältlichen Verbindung der Formel I in die freie Verbindung oder ein anderes Salz umwandelt, eine erhältliche freie Verbindung der Formel I in ein Salz davon umwandelt und/oder eine erhältliche Mischung von Isomeren einer Verbindung der Formel I in die einzelnen Isomere trennt;
wobei in den Ausgangsmaterialien zusätzlich zu den erwähnten speziellen Schutzgruppen weitere Schutzgruppen vorhanden sein können und alle Schutzgruppen auf einer geeigneten Stufe entfernt werden, so dass man die entsprechende Verbindung der Formel I oder ein Salz davon erhält.

## Revendications

1. Composé de formule I 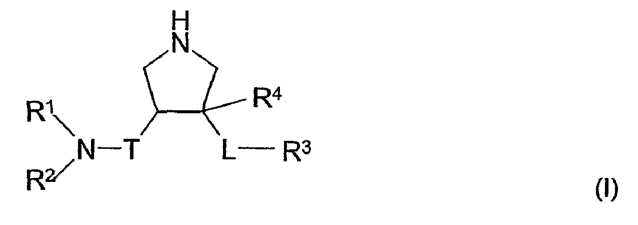 dans laquelle
R¹ est aryle non substitué ou substitué, hétérocyclyle mono- ou bicyclique non substitué ou substitué, cycloalkyle non substitué ou substitué, aryl-alkyle non substitué ou substitué, hétérocyclyl-alkyle mono- ou bicyclique non substitué ou substitué, cycloalkyl-alkyle non substitué ou substitué, ou acyle ;
R² est alkyle non substitué ou substitué, aryle non substitué ou substitué, hétérocyclyle mono- ou bicyclique non substitué ou substitué, cycloalkyle non substitué ou substitué, aryl-alkyle non substitué ou substitué, hétérocyclyl-alkyle mono- ou bicyclique non substitué ou substitué ou cycloalkyl-alkyle non substitué ou substitué, à condition que si L est méthylène (-CH₂-), oxy (-O-), thio (-S-) ou imino (-NH-) non substitué ou substitué, R² est choisi parmi l'un des groupements mentionnés et parmi hydrogène ;
R³ est alkyle non substitué ou substitué, aryle non substitué ou substitué, hétérocyclyle non substitué ou substitué, cycloalkyle non substitué ou substitué, aryl-alkyle non substitué ou substitué, hétérocyclyl-alkyle non substitué ou substitué, cycloalkyl-alkyle non substitué ou substitué, ou, si L est oxy, thio ou imino non substitué ou substitué, possède l'une des significations venant d'être mentionnée ou est alkylcarbonyle non substitué ou substitué, arylcarbonyle non substitué ou substitué, hétérocyclylcarbonyle non substitué ou substitué, cycloalkylcarbonyle non substitué ou substitué, carboxy éthérifié, carbamoyle, amino-carbonyle N-mono- ou N,N-di-substitué, alkylsulfonyle non substitué ou substitué, arylsulfonyle non substitué ou substitué, hétérocyclylsulfonyle non substitué ou substitué ou cycloalkylsulfonyle non substitué ou substitué, sulfamoyle ou amino-sulfonyle N-mono- ou N,N-di-substitué ;
R⁴ est hydrogène ou hydroxy ;
L est une liaison, méthylène (-CH₂-), oxy (-O-), thio (-S-) ou imino (-NH-) non substitué ou substitué, à condition que si L est une liaison alors R³ est l'un des motifs mentionnés pour R³ autre qu'alkyle substitué ;
ou R³ et R⁴ qui est alors -O- conjointement avec L qui est alors méthylène et le carbone auquel R³-L- et R⁴ sont liés forment un cycle non substitué ou substitué annelé à un aryle non substitué ou substitué, hétérocyclyle non substitué ou substitué ou cycloalkyle non substitué ou substitué, formant ainsi un composé spiro de formule I, ou R³ et R⁴ conjointement avec L forment oxo (=O), thioxo (=S) ou imino (=NH) non substitué ou substitué ; et
T est méthylène ou méthylène monosubstitué par alkyle, carbonyle (-C(=O)-) ou thiocarbonyle (-C(=S)-) ;
où dans chaque cas d'occurrence ci-dessus dans la présente revendication
aryle non substitué ou substitué est mono- ou polycyclique, ayant de 6 à 22 atomes de carbone, et est non substitué ou substitué par un ou plusieurs motifs, choisis indépendamment parmi le groupe constitué par un substituant de formule -(C₀-C₇-alkylène)-(X)ᵣ-(C₁-C₇-alkylène)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NV-CO-, -CO-NV-, -NV-SO₂-, -SO₂-NV, -NV-CO-NV-, -NV-CO-O-, -O-CO-NV-, -NV-SO₂-NV- où V est hydrogène ou alkyle non substitué ou substitué tel que défini ci-après, hydroxy-C₁-C₇-alkyle, C₁-C₇-alcoxy-C₁-C₇-alkyle, tel que 3-méthoxypropyle ou 2-méthoxyéthyle, C₁-C₇-alcoxy-C₁-C₇-alcoxy-C₁-C₇-alkyle, C₁-C₇-alcanoyloxy-C₁-C₇-alkyle, amino-C₁-C₇-alkyle, tel que aminométhyle, (N-) mono- ou (N,N-) di-(C₁-C₇-alkyl)-amino-C₁-C₇-alkyle, C₁-C₇-alcoxy-C₁-C₇-alkylamino-C₁-C₇-alkyle, mono-(naphtyl- ou phényl)-amino-C₁-C₇-alkyle, mono-(naphtyl- ou phényl-C₁-C₇-alkyl)-amino-C₁-C₇-alkyle, C₁-C₇-alcanoylamino-C₁-C₇-alkyle, C₁-C₇-alkyl-O-CO-NH-C₁-C₇-alkyle, C₁-C₇-alkylsulfonylamino-C₁-C₇-alkyle, C₁-C₇-alkyl-NH-CO-NH-C₁-C₇-alkyle, C₁-C₇-alkyl-NH-SO₂-NH-C₁-C₇-alkyle, C₁-C₇-alcoxy, hydroxy-C₁-C₇-alcoxy, C₁-C₇-alcoxy-C₁-C₇-alcoxy, C₁-C₇-alcanoyloxy, mono- ou di-(C₁-C₇-alkyl)-amino, N-mono-C₁-C₇-alcoxy-C₁-C₇-alkylamino, C₁-C₇-alcanoylamino, C₁-C₇-alkylsulfonylamino, C₁-C₇-alcoxycarbonyle, hydroxy-C₁-C₇-alcoxycarbonyle, C₁-C₇-alcoxy-C₁-C₇-alcoxycarbonyle, amino-C₁-C₇-alcoxycarbonyle, (N-) mono (C₁-C₇-alkyl) -amino-C₁-C₇-alcoxycarbonyle, C₁-C₇-alcanoylamino-C₁-C₇-alcoxycarbonyle, N-mono- ou N,N-di-(C₁-C₇-alkyl) - aminocarbonyle, N-C₁-C₇-alcoxy-C₁-C₇-alkylcarbamoyle ou N-mono- ou N,N-di-(C₁-C₇-alkyl)-aminosulfonyle ;
parmi C₂-C₇-alcényle, C₂-C₇-alcynyle, phényle, naphtyle, cycloalkyle, hétérocyclyle, phényl- ou naphtyl- ou hétérocyclyl-C₁-C₇-alkyle où hétérocyclyle est tel que défini ci-après, halogéno-C₁-C₇-alkyle, phényloxy- ou naphtyloxy-C₁-C₇-alkyle, cycloalkyl-C₁-C₇-alkyle, hétérocyclyl-C₁-C₇-alkyle, phényl-C₁-C₇-alcoxy- ou naphtyl-C₁-C₇-alcoxy-C₁-C₇-alkyle, cycloalkyl-C₁-C₇-alcoxy-C₁-C₇-alkyle, hétérocyclyl-C₁-C₇-alcoxy-C₁-C₇-alkyle, di-(naphtyl- ou phényl)-amino-C₁-C₇-alkyle mono- ou di-(hétérocyclyl-, cycloalkyl-, naphtyl- ou phényl)-amino-C₁-C₇-alkyle, di(naphtyl- ou phényl-C₁-C₇-alkyl)-amino-C₁-C₇-alkyle, mono- ou di-(hétérocyclyl-, cycloalkyl-, naphtyl- ou phényl-C₁-C₇-alkyl)-amino-C₁-C₇-alkyle, benzoyl- ou naphtoylamino-C₁-C₇-alkyle, cycloalkyl-CO-amino-C₁-C₇-alkyle, hétérocyclyl-CO-amino-C₁-C₇-alkyle, phényl- ou naphtylsulfonylamino-C₁-C₇-alkyle où phényle ou naphtyle est non substitué ou substitué par un ou plusieurs motifs C₁-C₇-alkyle, cycloalkylsulfonylamino-C₁-C₇-alkyle, hétérocyclylsulfonylamino-C₁-C₇-alkyle, phényl- ou naphtyl-C₁-C₇-alkylsulfonylamino-C₁-C₇-alkyle, cycloalkyl-C₁-C₇-alkylsulfonylamino-C₁-C₇-alkyle, hétérocyclyl-C₁-C₇-alkylsulfonylamino-C₁-C₇-alkyle, carboxy-C₁-C₇-alkyle, halogéno, hydroxy, phényl-C₁-C₇-alcoxy où phényle est non substitué ou substitué par C₁-C₇-alcoxy et/ou halogéno, halogéno-C₁-C₇-alcoxy, cycloalkyl-C₁-C₇-alcoxy, hétérocyclyl-C₁-C₇-alcoxy, phényl- ou naphtyloxy, cycloalkyloxy, hétérocyclyloxy, phényl- ou naphtyl-C₁-C₇-alkyloxy, cycloalkyl-C₁-C₇-alkyloxy, hétérocyclyl-C₁-C₇-alkyloxy, benzoyl- ou naphtoyloxy, halogéno-C₁-C₇-alkylthio, phényl- ou naphtylthio, cycloalkylthio, hétérocyclylthio, phényl- ou naphtyl-C₁-C₇-alkylthio, cycloalkyl-C₁-C₇-alkylthio, hétérocyclyl-C₁-C₇-alkylthio, benzoyl- ou naphtoylthio, nitro, amino, mono- ou di(naphtyl- ou phényl-C₁-C₇-alkyl)-amino, mono- ou di-(hétérocyclyl-, cycloalkyl-, naphtyl- ou phényl-C₁-C₇-alkyl)-amino, benzoyl- ou naphtoylamino, phényl- ou naphtylsulfonylamino où phényle ou naphtyle est non substitué ou substitué par un ou plusieurs, notamment de un à trois, motifs C₁-C₇-alkyle, cycloalkylsulfonylamino, hétérocyclylsulfonylamino, phényl- ou naphtyl-C₁-C₇-alkylsulfonylamino, cycloalkyl-C₁-C₇-alkylsulfonylamino, hétérocyclyl-C₁-C₇-alkylsulfonylamino, carboxyle, C₁-C₇-alkyl-carbonyle, halogéno-C₁-C₇-alkylcarbonyle, hydroxy-C₁-C₇-alkylcarbonyle, C₁-C₇-alcoxy-C₁-C₇-alkylcarbonyle, amino-C₁-C₇-alkylcarbonyle, (N-) mono- ou (N, N) di-(C₁-C₇-alkyl) -amino-C₁-C₇-alkylcarbonyle, C₁-C₇-alcanoylamino-C₁-C₇-alkylcarbonyle, N-mono ou (N,N-) di-(C₁-C₇-alkyl)-amino-C₁-C₇-alcoxycarbonyle, halogéno-C₁-C₇-alcoxycarbonyle, phényl- ou naphtyloxycarbonyle, phényl- ou naphtyl-C₁-C₇-alcoxycarbonyle, N-mono ou (N,N-) di-(C₁-C₇-alkyl)-amino-C₁-C₇-alcoxycarbonyle, carbamoyle, N-mono ou N,N-di-(hétérocyclyl-, cycloalkyl-, naphtyl- ou -phényl-)-aminocarbonyle, N-mono- ou N,N-di-(hétérocyclyl-, cycloalkyl-, naphtyl- ou phényl-C₁-C₇-alkyl)-aminocarbonyle, cyano, C₁-C₇-alkylène qui est non substitué ou substitué par jusqu'à quatre substituants C₁-C₇-alkyle et lié à deux atomes de cycle adjacents du motif aryle, C₂-C₇-alcénylène ou -alcynylène qui sont liés à deux atomes de cycle adjacents du motif aryle, sulfényle, sulfinyle, C₁-C₇-alkylsulfinyle, phényl- ou naphtylsulfinyle où phényle ou naphtyle est non substitué ou substitué par un ou plusieurs, notamment de un à trois, motifs C₁-C₇-alkyle, cycloalkylsulfinyle, hétérocyclylsulfinyle, phényl- ou naphtyl-C₁-C₇-alkylsulfinyle, cycloalkyl-C₁-C₇-alkylsulfinyle, hétérocyclyl-C₁-C₇-alkylsulfinyle, sulfonyle, C₁-C₇-alkylsulfonyle, halogéno-C₁-C₇-alkylsulfonyle, hydroxy-C₁-C₇-alkylsulfonyle, C₁-C₇-alcoxy-C₁-C₇-alkylsulfonyle, amino-C₁-C₇-alkylsulfonyle, N-mono ou (N,N-) di- (C₁-C₇-alkyl) -amino-C₁-C₇-alkylsulfonyle, C₁-C₇-alcanoylamino-C₁-C₇-alkylsulfonyle, phényl- ou naphtylsulfonyle où phényle ou naphtyle est non substitué ou substitué par un ou plusieurs motifs C₁-C₇-alkyle, cycloalkylsulfonyle, hétérocyclylsulfonyle, phényl- ou naphtyl-C₁-C₇-alkylsulfonyle, cycloalkyl-C₁-C₇-alkylsulfonyle, hétérocyclyl-C₁-C₇-alkylsulfonyle, sulfamoyle et N-mono ou N,N-di-(C₁-C₇-alkyle, phényl-, naphtyle, hétérocyclyle, cycloalkyle, phényl-C₁-C₇-alkyle et/ou naphtyl-C₁-C₇-alkyle, hétérocyclyl-C₁-C₇-alkyle, cycloalkyl-C₁-C₇-alkyl)-aminosulfonyle ;
hétérocyclyle non substitué ou substitué est un noyau insaturé, partiellement saturé ou saturé mono- ou bicyclique ou, s'il ne fait pas partie d'un substituant R¹ ou R² ou s'il n'est pas un substituant R¹ et R² en outre polycyclique, préférablement mono- ou bicyclique ou, s'il ne fait pas partie d'un substituant R¹ ou R² ou s'il n'est pas un substituant R¹ et R², mono-, bi- ou en outre tricyclique, ayant de 3 à 14 atomes de cycle et avec un ou plusieurs hétéroatomes choisis indépendamment parmi azote (=N-, -NH- ou -NH- substitué), oxygène, soufre (-S-, S(=O)- ou S-(=O)₂-) qui est non substitué ou substitué par un ou plusieurs substituants choisis indépendamment parmi les substituants susmentionnés pour aryle et parmi oxo, éventuellement choisis parmi : 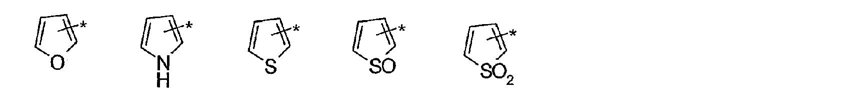 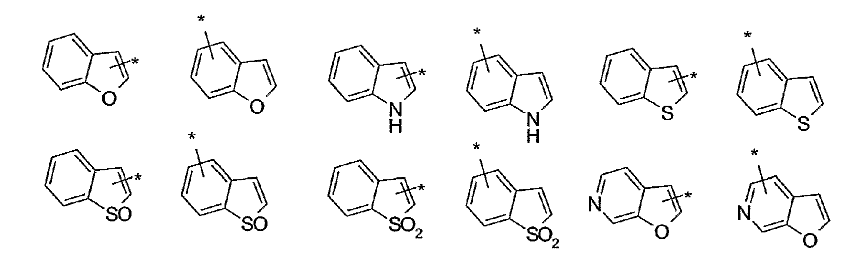 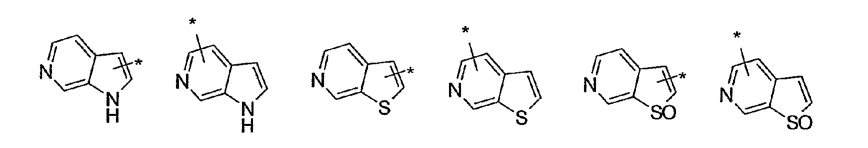 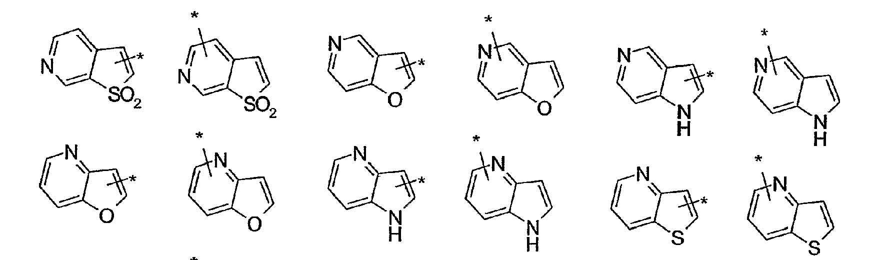 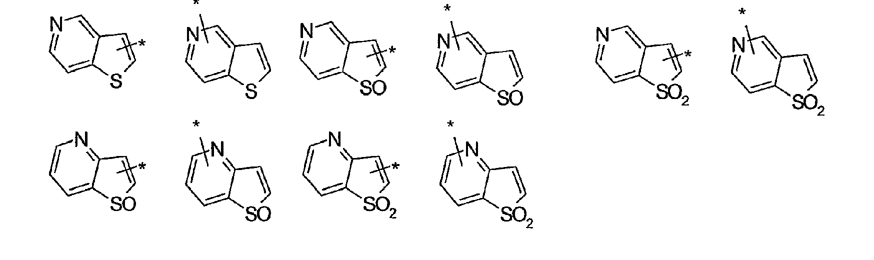 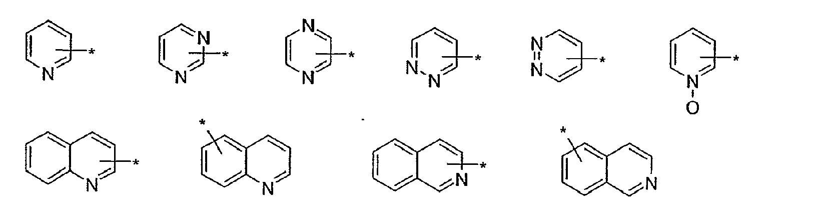 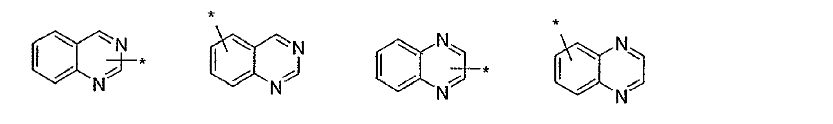 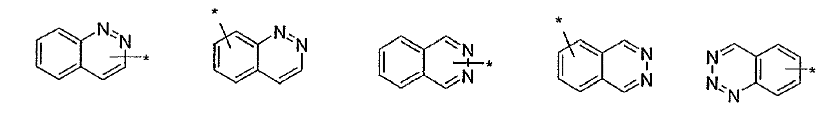 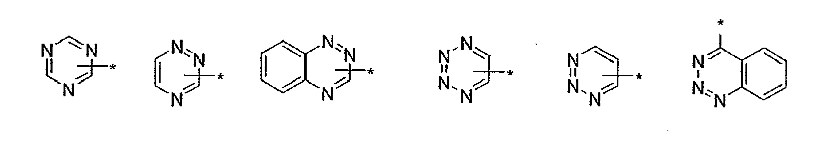 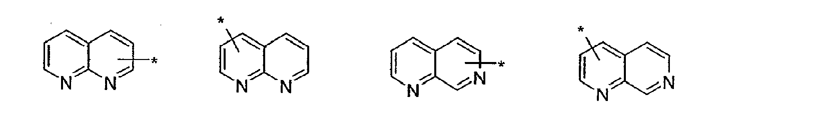 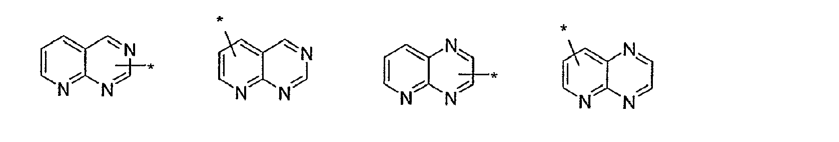 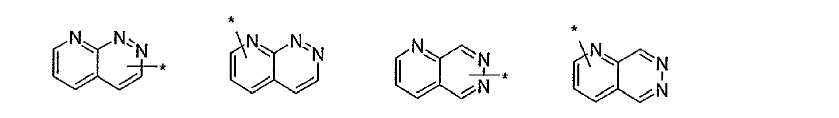 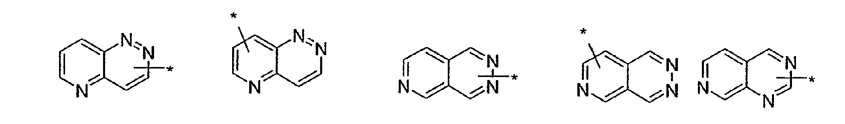 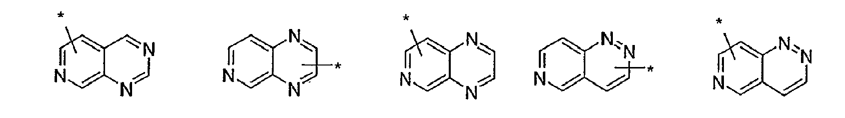 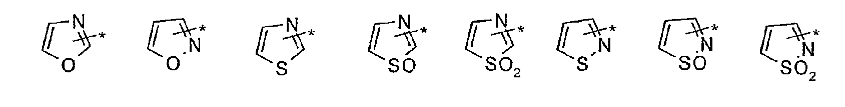 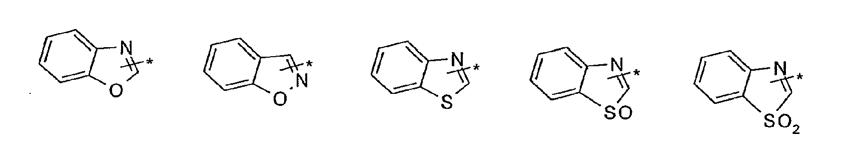 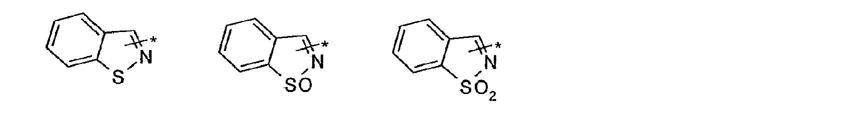 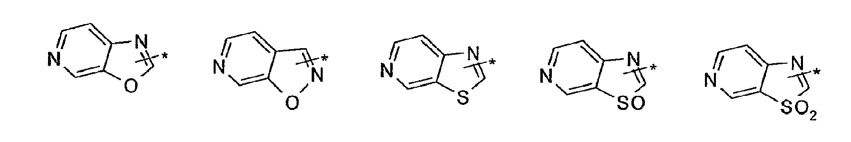 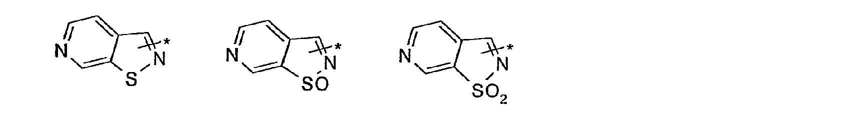 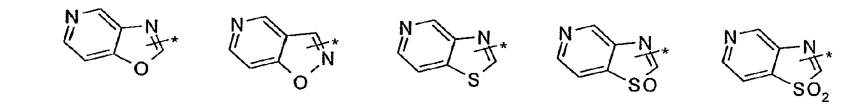 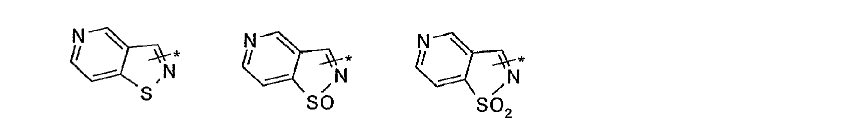 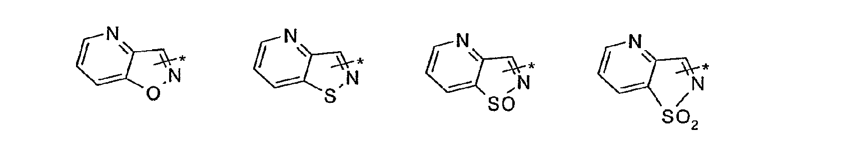 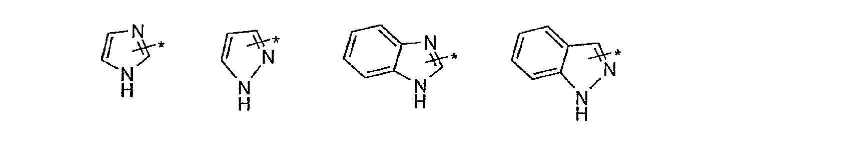 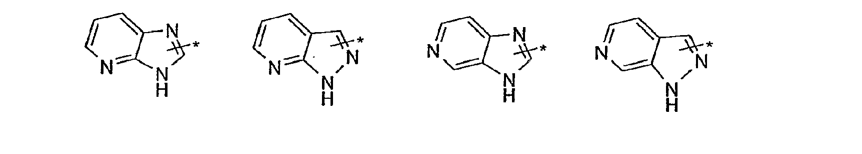 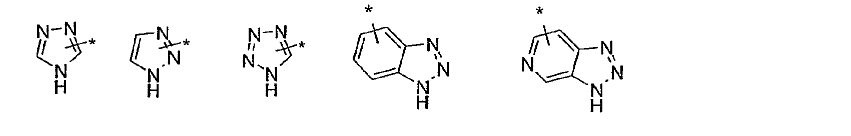 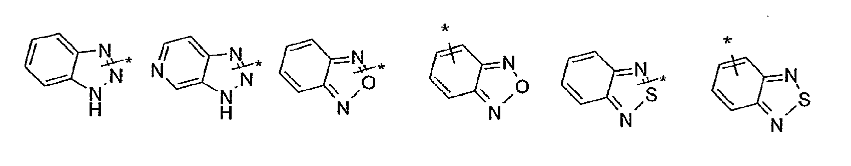 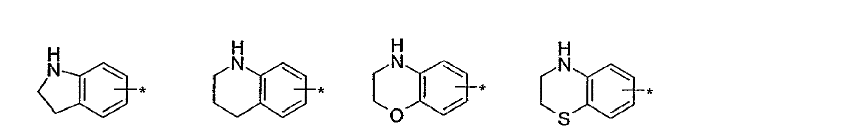 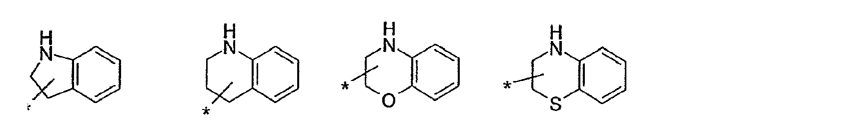 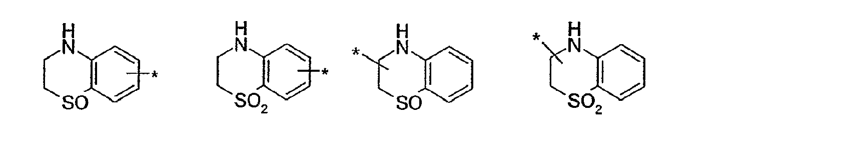 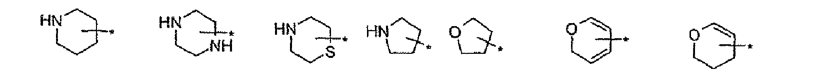 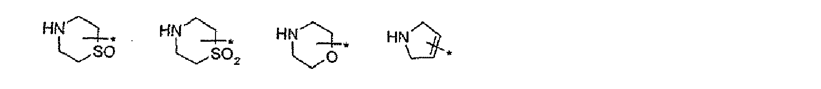 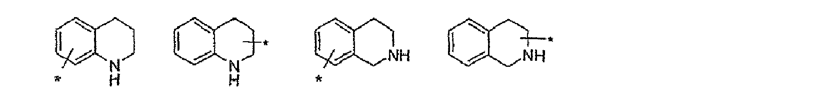 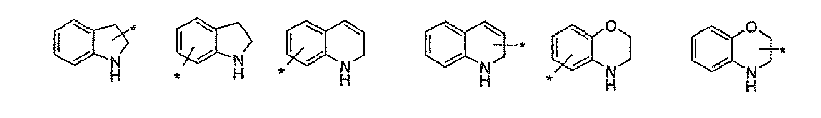 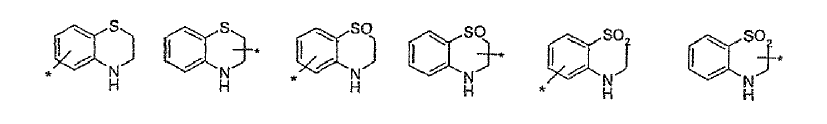 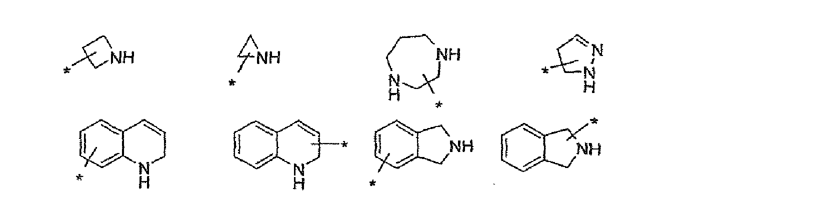 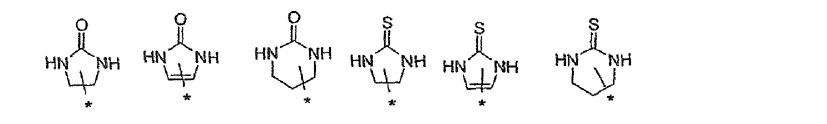 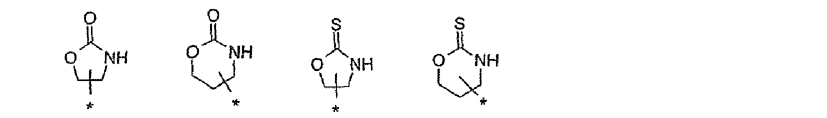 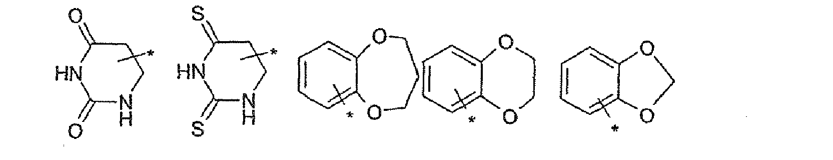 ou dans le cas où hétérocyclyle est présent dans R₃ défini comme hétérocyclyle non substitué ou substitué, hétérocyclyl-alkyle non substitué ou substitué ou hétérocyclylsulfonyle non substitué ou substitué choisi de plus parmi 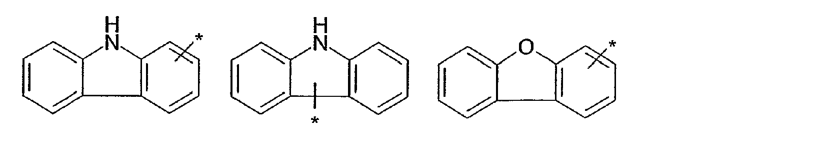 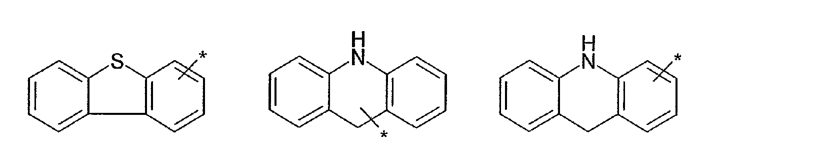 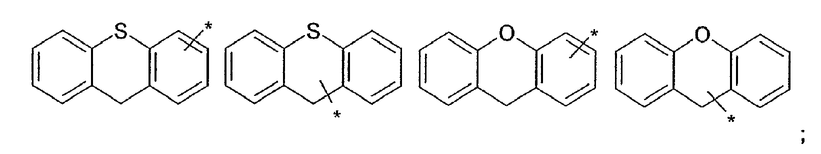 où dans chaque cas où un NH est présent - la liaison avec l'astérisque reliant le motif hétérocyclyle respectif au reste de la molécule - le H peut être remplacé par ladite liaison et/ou le H peut être remplacé par un substituant tel que défini ci-dessus,
cycloalkyle non substitué ou substitué est mono- ou polycyclique, C₃-C₁₀-cycloalkyle pouvant comporter une ou plusieurs doubles (par exemple dans cycloalcényle) et/ou triples liaisons (par exemple dans cycloalcynyle), et est non substitué ou substitué par un ou plusieurs substituants choisis indépendamment parmi ceux mentionnés ci-dessus comme substituants pour aryle ;
dans aryl-alkyle non substitué ou substitué, aryle, qui est non substitué ou substitué par un ou plusieurs substituants choisis indépendamment parmi ceux mentionnés ci-dessus comme substituants pour aryle, est tel que décrit ci-dessus pour aryle et est lié à alkyle soit au niveau d'une extrémité, soit au niveau de tout autre carbone dans la chaîne alkyle ;
dans hétérocyclyl-alkyle non substitué ou substitué, hétérocyclyle est tel que décrit ci-dessus et est non substitué ou substitué par un ou plusieurs substituants choisis indépendamment parmi ceux mentionnés ci-dessus pour aryle substitué, et hétérocyclyle est lié à alkyle, soit au niveau d'une extrémité, soit au niveau de tout autre carbone dans la chaîne alkyle ;
dans cycloalkyl-alkyle non substitué ou substitué, cycloalkyle est tel que décrit ci-dessus et est non substitué ou substitué par un ou plusieurs substituants choisis indépendamment parmi ceux mentionnés ci-dessus pour aryle substitué, et cycloalkyle est lié à alkyle, soit au niveau d'une extrémité, soit au niveau de tout autre carbone dans la chaîne alkyle ;
acyle est aryl-carbonyle ou -sulfonyle non substitué ou substitué, hétérocyclylcarbonyle ou -sulfonyle non substitué ou substitué, cycloalkylcarbonyle ou - sulfonyle non substitué ou substitué, formyle ou alkylcarbonyle ou -sulfonyle non substitué ou substitué, où aryle non substitué ou substitué, hétérocyclyle non substitué ou substitué et cycloalkyle non substitué ou substitué sont tels que définis ci-dessus et alkyle non substitué ou substitué est tel que défini ci-après ;
alkyle non substitué ou substitué est C₁-C₇-alkyle, qui est à chaîne linéaire ou ramifiée, qui est non substitué ou substitué par un ou plusieurs motifs choisis parmi aryle non substitué ou substitué tel que décrit ci-dessus, hétérocyclyle non substitué ou substitué tel que décrit ci-dessus, cycloalkyle non substitué ou substitué tel que décrit ci-dessus, C₂-C₇-alcényle, C₂-C₇-alcynyle, halogéno, hydroxy, C₁-C₇-alcoxy, halogéno-C₁-C₇-alcoxy, hydroxy-C₁-C₇-alcoxy, C₁-C₇-alcoxy-C₁-C₇-alcoxy, phényl- ou naphtyloxy, phényl- ou naphtyl-C₁-C₇-alkyloxy, C₁-C₇-alcanoyloxy, benzoyl- ou naphtoyloxy, C₁-C₇-alkylthio, halogéno-C₁-C₇-alkylthio, tel que trifluorométhylthio, hydroxy-C₁-C₇-alkylthio, C₁-C₇-alcoxy-C₁-C₇-alkylthio, phényl- ou naphtylthio, phényl- ou naphtyl-C₁-C₇-alkylthio, C₁-C₇-alcanoylthio, benzoyl- ou naphtoylthio, nitro, amino, mono- ou di(C₁-C₇-alkyle, hydroxy-C₁-C₇-alkyle et/ou C₁-C₇-alcoxy-C₁-C₇-alkyl)-amino, mono- ou di-(naphtyl- ou phényl-C₁-C₇-alkyl)-amino, C₁-C₇-alcanoylamino, benzoyl- ou naphtoylamino, C₁-C₇-alkylsulfonylamino, phényl- ou naphtylsulfonylamino où phényle ou naphtyle est non substitué ou substitué par un ou plusieurs motifs C₁-C₇-alkyle, phényl- ou naphtyl-C₁-C₇-alkylsulfonylamino, carboxyle, C₁-C₇-alkyl-carbonyle, C₁-C₇-alcoxycarbonyle, phényl- ou naphtyloxycarbonyle, phényl- ou naphtyl-C₁-C₇-alcoxycarbonyle, carbamoyle, N-mono- ou N,N-di-(C₁-C₇-alkyl)-aminocarbonyle, N-mono- ou N,N-di-(naphtyl- ou phényl-C₁-C₇-alkyl)-aminocarbonyle, N-mono- ou N,N-di-(alkyle, naphtyle, phényle, hétérocyclyle, cycloalkyle, naphtyl-, hétérocyclyl-, cycloalkyl- ou phényl-C₁-C₇-alkyl)-aminocarbonyle, cyano, C₁-C₇-alcénylène ou - alcynylène, C₁-C₇-alkylènedioxy, sulfényle, sulfinyle, C₁-C₇-alkylsulfinyle, phényl- ou naphtylsulfinyle où phényle ou naphtyle est non substitué ou substitué par un ou plusieurs motifs C₁-C₇-alkyle, cycloalkylsulfinyle, hétérocyclylsulfinyle, phényl- ou naphtyl-C₁-C₇-alkylsulfinyle, cycloalkyl-C₁-C₇-alkylsulfinyle, hétérocyclyl-C₁-C₇-alkylsulfinyle, sulfonyle, C₁-C₇-alkylsulfonyle, phényl- ou naphtylsulfonyle où phényle ou naphtyle est non substitué ou substitué par un ou plusieurs motifs C₁-C₇-alkyle, cycloalkylsulfonyle, hétérocyclylsulfonyle, phényl- ou naphtyl-C₁-C₇-alkylsulfonyle, cycloalkyl-C₁-C₇-alkylsulfonyle, hétérocyclyl-C₁-C₇-alkylsulfonyle, sulfamoyle, N-mono- ou N,N-di-(alkyle, naphtyle, phényle, hétérocyclyle, cycloalkyle, naphtyl-, hétérocyclyl-, cycloalkyl- ou phényl-C₁-C₇-alkyl)-aminosulfonyle, N-mono-, N'-mono-, N,N-di- ou N,N,N'-tri-(C₁-C₇-alkyle, hydroxy-C₁-C₇-alkyle et/ou C₁-C₇-alcoxy-C₁-C₇-alkyl)-aminocarbonylamino et N-mono-, N'-mono-, N,N-di- ou N,N,N'-tri(C₁-C₇-alkyle, hydroxy-C₁-C₇-alkyle et/ou C₁-C₇-alcoxy-C₁-C₇-alkyl)-aminosulfonylamino ;
dans alkylsulfonyle non substitué ou substitué, alkyle non substitué ou substitué est tel que défini ci-dessus pour alkyle non substitué ou substitué ;
dans arylsulfonyle non substitué ou substitué, aryle non substitué ou substitué est tel que défini ci-dessus pour aryle non substitué ou substitué ;
dans hétérocyclylsulfonyle non substitué ou substitué, hétérocyclyle non substitué ou substitué est tel que défini ci-dessus pour hétérocyclyle non substitué ou substitué ;
dans cycloalkylsulfonyle non substitué ou substitué, cycloalkyle non substitué ou substitué est tel que défini ci-dessus pour cycloalkyle non substitué ou substitué ;
lorsque R³ et R⁴ qui est alors -O- conjointement avec L qui est alors méthylène et le carbone auquel R³-L- et R⁴ sont liés forment un cycle non substitué ou substitué (ayant un ou plusieurs substituants choisis indépendamment parmi ceux mentionnés ci-dessus pour aryle, préférablement sans substituant) annelé à un aryle non substitué ou substitué, hétérocyclyle non substitué ou substitué ou cycloalkyle non substitué ou substitué, chacun d'entre eux est tel que défini ci-dessus, formant ainsi un composé spiro de formule I ;
dans imino non substitué ou substitué ainsi que lorsque des groupements NH substitués sont présents dans des hétérocycles, les substituants sont préférablement choisis parmi le groupe constitué par un substituant de formule -(C₁-C₇-alkylène)-(X)ᵣ-(C₁-C₇-alkylène)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NV-CO-, -CO-NV-, -NV-SO₂-, -SO₂-NV, -NV-CO-NV-, -NV-CO-O-, -O-CO-NV-, -NV-SO₂-NV-,
C₂-C₇-alcényle, C₂-C₇-alcynyle, cycloalkyle, phényle, naphtyle, hétérocyclyle, phényl- ou naphtyl-C₁-C₇-alkyle, cycloalkyl-C₁-C₇-alkyle, hétérocyclyl-C₁-C₇-alkyle où hétérocyclyle est tel que défini ci-après, halogéno-C₁-C₇-alkyle, phényloxy- ou naphtyloxy-C₁-C₇-alkyle, cycloalkyloxy-C₁-C₇-alkyle, hétérocyclyloxy-C₁-C₇-alkyle, phényl-C₁-C₇-alcoxy- ou naphtyl-C₁-C₇-alcoxy-C₁-C₇-alkyle, cycloalkyl-C₁-C₇-alcoxy-C₁-C₇-alkyle, hétérocyclyl-C₁-C₇-alcoxy-C₁-C₇-alkyle, di-(cycloalkyle, hétérocyclyle, naphtyle ou phényl)-amino-C₁-C₁-alkyle, di-(cycloalkyl-, hétérocyclyl-, naphtyl- ou phényl-C₁-C₇-alkyl)-amino-C₁-C₇-alkyle, benzoyl- ou naphtoylamino-C₁-C₇-alkyle, phényl- ou naphtylsulfonylamino-C₁-C₇-alkyle où phényle ou naphtyle est non substitué ou substitué par un ou plusieurs motifs C₁-C₇-alkyle, cycloalkylsulfonylamino-C₁-C₇-alkyle, hétérocyclylsulfonylamino-C₁-C₇-alkyle, phényl- ou naphtyl-C₁-C₇-alkylsulfonylamino-C₁-C₇-alkyle, cycloalkyl-C₁-C₇-alkylsulfonylamino-C₁-C₇-alkyle, hétérocyclyl-C₁-C₇-alkylsulfonylamino-C₁-C₇-alkyle, C₁-C₇-alkylcarbonyle, halogéno-C₁-C₇-alkylcarbonyle, hydroxy-C₁-C₇-alkylcarbonyle, C₁-C₇-alcoxy-C₁-C₇-alkylcarbonyle, amino-C₁-C₇-alkylcarbonyle, (N-) mono- ou (N,N-) di-(C₁-C₇-alkyl) -amino-C₁-C₇-alkylcarbonyle, C₁-C₇-alcanoylamino-C₁-C₇-alkylcarbonyle, (N,N-) di-(C₁-C₇-alkyl)-amino-C₁-C₇-alcoxycarbonyle, halogéno-C₁-C₇-alcoxycarbonyle, phényl- ou naphtyloxycarbonyle, cycloalkyloxycarbonyle, hétérocyclyloxycarbonyle, phényl- ou naphtyl-C₁-C₇-alcoxycarbonyle, cycloalkyl-C₁-C₇-alcoxycarbonyle, hétérocyclyl-C₁-C₇-alcoxycarbonyle, C₁-C₇-alkylsulfinyle, phényl- ou naphtylsulfinyle où phényle ou naphtyle est non substitué ou substitué par un ou plusieurs motifs C₁-C₇-alkyle, cycloalkylsulfinyle, hétérocyclylsulfinyle, phényl- ou naphtyl-C₁-C₇-alkylsulfinyle, cycloalkyl-C₁-C₇-alkylsulfinyle, hétérocyclyl-C₁-C₇-alkylsulfinyle, sulfonyle, C₁-C₇-alkylsulfonyle, halogéno-C₁-C₇-alkylsulfonyle, hydroxy-C₁-C₇-alkylsulfonyle, C₁-C₇-alcoxy-C₁-C₇-alkylsulfonyle, amino-C₁-C₇-alkylsulfonyle, (N,N-) di-(C₁-C₇-alkyl)-amino-C₁-C₇-alkylsulfonyle, C₁-C₇-alcanoylamino-C₁-C₇-alkylsulfonyle, phényl- ou naphtylsulfonyle où phényle ou naphtyle est non substitué ou substitué par un ou plusieurs motifs C₁-C₇-alkyle, cycloalkylsulfonyle, hétérocyclylsulfonyle, phényl- ou naphtyl-C₁-C₇-alkylsulfonyle ; cycloalkyl-C₁-C₇-alkylsulfonyle et
dans alkylcarbonyle non substitué ou substitué, alkyle non substitué ou substitué est tel que défini ci-dessus ;
dans arylcarbonyle non substitué ou substitué, hétérocyclylcarbonyle non substitué ou substitué et cycloalkylcarbonyle non substitué ou substitué, les motifs aryle, hétérocyclyle et cycloalkyle non substitués ou substitués, respectivement, sont préférablement tels que décrits pour les motifs aryle, hétérocyclyle et cycloalkyle non substitués ou substitués, respectivement ;
carboxy éthérifié est carbonyle, lié à L=oxy ou notamment imino, auquel un motif choisi parmi alkyloxy non substitué ou substitué, aryloxy non substitué ou substitué, hétérocyclyloxy non substitué ou substitué ou cycloalkyloxy non substitué ou substitué, dans chacun desquels les motifs alkyle, aryle, hétérocyclyle ou cycloalkyle non substitués ou substitués sont définis comme ci-dessus, est lié comme groupement lié ; on préfère notamment alcoxycarbonyle non substitué ou substitué, notamment C₁-C₇-alcoxycarbonyle, lié à L=imino ;
aminocarbonyle N-mono- ou N,N-di-substitué est aminocarbonyle, préférablement lié à L=oxy ou thio, qui est mono- ou di-substitué au niveau de l'azote par un ou plusieurs motifs choisis parmi alkyle non substitué ou substitué, aryle non substitué ou substitué, hétérocyclyle non substitué ou substitué ou cycloalkyle non substitué ou substitué, chacun desquels étant défini comme ci-dessus ; un exemple préféré est aryl-C₁-C₇-alkylaminocarbonyle (=aryl-C₁-C₇-NH-C(=)-), tel que benzylaminocarbonyle, lié à L=oxy ou en outre thio ; et aminosulfonyle N-mono- ou N,N-di-substitué est sulfamoyle, préférablement lié à L=imino ou notamment oxy, qui est mono- ou di-substitué au niveau de l'azote par un ou plusieurs motifs choisis parmi alkyle non substitué ou substitué, aryle non substitué ou substitué, hétérocyclyle non substitué ou substitué ou cycloalkyle non substitué ou substitué, chacun desquels étant préférablement défini comme ci-dessus ; un exemple préféré est aryl-C₁-C₇-alkylaminosulfonyle (=aryl-C₁-C₇-NH-S(=O)₂-), tel que benzylaminosulfonyle, lié à L=oxy ou en outre imino ;
ou un sel pharmaceutiquement acceptable de celui-ci.

2. Composé de formule I selon la revendication 1,
dans laquelle
R¹ est phényle ou naphtyle, chacun d'entre eux étant non substitué ou substitué par un ou plusieurs substituants choisis parmi le groupe constitué par C₁-C₇-alkyle, phényle, naphtyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, C₁-C₇-alkyl-sulfonylamino-C₁-C₇-alkyle, halogéno, hydroxy, C₁-C₇-alcoxy, hydroxy-C₁-C₇-alcoxy, C₁-C₇-alcoxy-C₁-C₇-alcoxy, phényl- ou naphtyloxy, phényl- ou naphtyl-C₁-C₇-alkyloxy, C₁-C₇-alcanoyloxy, nitro, amino, mono- ou di-(C₁-C₇-alkyl)-amino, C₁-C₇-alcanoylamino, carboxyle, C₁-C₇-alcoxycarbonyle, phényl- ou naphtyl-C₁-C₇-alcoxycarbonyle, carbamoyle, N-mono- ou N,N-di-(C₁-C₇-alkyle et/ou (phényl- ou naphtyl)-C₁-C₇-alkyl)-carbamoyle, C₁-C₇-alkylsulfonyle, phényl- ou naphtylsulfonyle non substitué ou C₁-C₇-alkyl-substitué, N-mono- ou N, N-di- (C₁-C₇-alkyle et/ou (phényl- ou naphtyl)-C₁-C₇-alkyl)-sulfamoyle et cyano ;
phényl- ou naphtyl-C₁-C₇-alkyle, où chacun parmi phényle ou naphtyle est non substitué ou substitué par un ou plusieurs substituants choisis parmi le groupe constitué par les substituants venant d'être mentionnés pour phényle ou naphtyle substitué, pyrrolyle, furanyle, thiényle, pyrimidine-2,4-dione-1-, -2-, -3- ou -5-yle et benzo[1,3]dioxalyle, chacun d'entre eux étant non substitué ou substitué par un ou plusieurs substituants choisis indépendamment parmi ceux mentionnés pour phényle ou naphtyle substitué R¹ ci-dessus ; pyrrolyl-C₁-C₇-alkyle, furanyl-C₁-C₇-alkyle, thiényl-C₁-C₇-alkyle, pyrimidine-2,4-dione-1-, -2-, -3- ou -5-yl-C₁-C₇-alkyle, indolyl-C₁-C₇-alkyle, benzofuranyl-C₁-C₇-alkyle, benzimidazolyl-C₁-C₇-alkyle, benzopyrazolyl-C₁-C₇-alkyle, quinoléinyl-C₁-C₇-alkyle, isoquinolyl-C₁-C₇-alkyle ou benzo[1,2,5]oxadiazolyl-C₁-C₇-alkyle, chacun d'entre eux étant non substitué ou substitué par un ou plusieurs substituants choisis indépendamment parmi les substituants mentionnés ci-dessus pour phényle ou naphtyle substitué R¹;
C₃-C₁₀-cycloalkyle qui est non substitué ou substitué par un ou plusieurs substituants choisis indépendamment parmi les substituants mentionnés ci-dessus pour phényl- ou naphtyl-carbonyle ou phényl- ou naphtyl-C₁-C₇-alkylcarbonyle, où chaque phényle ou naphtyle est non substitué ou substitué par un ou plusieurs substituants choisis parmi le groupe constitué par
un substituant de formule -(C₀-C₇-alkylène)-(X)ᵣ-(C₁-C₇-alkylène)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NV-CO-, -CO-NV-, -NV-SO₂-, -SO₂-NV, -NV-CO-NV-, -NV-CO-O-, -O-CO-NV-, -NV-SO₂-NV- où V est hydrogène ou alkyle non substitué ou substitué tel que défini ci-après, hydroxy-C₁-C₇-alkyle, C₁-C₇-alcoxy-C₁-C₇-alkyle, tel que 3-méthoxypropyle ou 2-méthoxyéthyle, C₁-C₇-alcoxy-C₁-C₇-alcoxy-C₁-C₇-alkyle, C₁-C₇-alcanoyloxy-C₁-C₇-alkyle, amino-C₁-C₇-alkyle, tel que aminométhyle, (N-) mono- ou (N,N-) di-(C₁-C₇-alkyl)-amino-C₁-C₇-alkyle, C₁-C₇-alcoxy-C₁-C₇-alkylamino-C₁-C₇-alkyle, mono-(naphtyl- ou phényl)-amino-C₁-C₇-alkyle, mono-(naphtyl- ou phényl-C₁-C₇-alkyl)-amino-C₁-C₇-alkyle, C₁-C₇-alcanoylamino-C₁-C₇-alkyle, C₁-C₇-alkyl-O-CO-NH-C₁-C₇-alkyle, C₁-C₇-alkylsulfonylamino-C₁-C₇-alkyle, C₁-C₇-alkyl-NH-CO-NH-C₁-C₇-alkyle, C₁-C₇-alkyl-NH-SO₂-NH-C₁-C₇-alkyle, C₁-C₇-alcoxy, hydroxy-C₁-C₇-alcoxy, C₁-C₇-alcoxy-C₁-C₇alcoxy, C₁-C₇-alcanoyloxy, mono- ou di-(C₁-C₇-alkyl)-amino, mono- di- (naphtyl- ou phényl-C₁-C₇-alkyl)-amino, N-mono-C₁-C₇-alcoxy-C₁-C₇-alkylamino, C₁-C₇-alcanoylamino, C₁-C₇-alkylsulfonylamino, C₁-C₇-alcoxycarbonyle, hydroxy-C₁-C₇-alcoxycarbonyle, C₁-C₇-alcoxy-C₁-C₇-alcoxycarbonyle, amino-C₁-C₇-alcoxycarbonyle, (N-) mono(C₁-C₇-alkyl)-amino-C₁-C₇-alcoxycarbonyle, C₁-C₇-alcanoylamino-C₁-C₇-alcoxycarbonyle, N-mono- ou N,N-di-(C₁-C₇-alkyl)-aminocarbonyle, N-C₁-C₇-alcoxy-C₁-C₇-alkylcarbamoyle ou N-mono- ou N,N-di-(C₁-C₇-alkyl)-aminosulfonyle ; ou
parmi phényl- ou naphtyl-C₁-C₇-alkyle, halogéno-C₁-C₇-alkyle, halogéno, hydroxy, phényl- ou naphtyloxy, phényl- ou naphtyl-C₁-C₇-alkyloxy, nitro, amino, amino-C₁-C₇-alkyle, carboxyle, phényl- ou naphtyl-C₁-C₇-alcoxycarbonyle, halogéno-C₁-C₇-alcoxycarbonyle, C₁-C₇-alkylsulfonyle, carbamoyle et cyano ;
hétérocyclylcarbonyle tel que chacun d'entre eux est non substitué ou substitué par un ou plusieurs substituants choisis indépendamment parmi un substituant de formule -(C₀-C₇-alkylène)-(X)ᵣ-(C₁-C₇-alkylène)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NV-CO-, -CO-NV-, -NV-SO₂-, -SO₂-NV, - NV-CO-NV-, -NV-CO-O-, -O-CO-NV-, -NV-SO₂-NV- où V est hydrogène ou alkyle non substitué ou substitué tel que défini ci-après, hydroxy-C₁-C₇-alkyle, C₁-C₇-alcoxy-C₁-C₇-alkyle, C₁-C₇-alcoxy-C₁-C₇-alcoxy-C₁-C₇-alkyle, C₁-C₇-alcanoyloxy-C₁-C₇-alkyle, amino-C₁-C₇-alkyle, (N-) mono- ou (N,N-) di-(C₁-C₇-alkyl)-amino-C₁-C₇-alkyle, C₁-C₇-alcoxy-C₁-C₇-alkylamino-C₁-C₇-alkyle, mono-(naphtyl- ou phényl)-amino-C₁-C₇-alkyle, mono- (naphtyl- ou phényl-C₁-C₇-alkyl)-amino-C₁-C₇-alkyle, C₁-C₇-alcanoylamino-C₁-C₇-alkyle, C₁-C₇-alkyl-O-CO-NH-C₁-C₇-alkyle, C₁-C₇-alkylsulfonylamino-C₁-C₇-alkyle, C₁-C₇-alkyl-NH-CO-NH-C₁-C₇-alkyle, C₁-C₇-alkyl-NH-SO₂-NH-C₁-C₇-alkyle, C₁-C₇-alcoxy, hydroxy-C₁-C₇-alcoxy, C₁-C₇-alcoxy-C₁-C₇-alcoxy, C₁-C₇-alcanoyloxy, mono- ou di-(C₁-C₇-alkyl)-amino, mono- di- (naphtyl- ou phényl-C₁-C₇-alkyl)-amino, N-mono-C₁-C₇-alcoxy-C₁-C₇-alkylamino, C₁-C₇-alcanoylamino, C₁-C₇-alkylsulfonylamino, C₁-C₇-alcoxycarbonyle, hydroxy-C₁-C₇-alcoxycarbonyle, C₁-C₇-alcoxy-C₁-C₇-alcoxycarbonyle, amino-C₁-C₇-alcoxycarbonyle, (N-) mono- (C₁-C₇-alkyl) - amino-C₁-C₇-alcoxycarbonyle, C₁-C₇-alcanoylamino-C₁-C₇-alcoxycarbonyle, N-mono- ou N,N-di-(C₁-C₇-alkyl)-aminocarbonyle, N-C₁-C₇-alcoxy-C₁-C₇-alkylcarbamoyle ou
N-mono- ou N,N-di-(C₁-C₇-alkyl)-aminosulfonyle ; ou parmi phényl- ou naphtyl-C₁-C₇-alkyle, halogéno-C₁-C₇-alkyle, halogéno, hydroxy, phényl- ou naphtyloxy, phényl- ou naphtyl-C₁-C₇-alkyloxy, nitro, amino, amino-C₁-C₇-alkyle, carboxyle, phényl- ou naphtyl-C₁-C₇-alcoxycarbonyle, halogéno-C₁-C₇-alcoxycarbonyle, C₁-C₇-alkylsulfonyle, carbamoyle et cyano ; ou
phényl- ou naphtyl-sulfonyle, où chaque phényle ou naphtyle est non substitué ou substitué par un ou plusieurs substituants choisis parmi ceux mentionnés ci-dessus pour phényle ou naphtyle substitué R¹;
R² est C₁-C₇-alkyle qui est non substitué ou substitué par un ou plusieurs substituants choisis parmi le groupe constitué par halogéno, phényl- ou naphtyle, hydroxy, C₁-C₇-alcoxy, amino, mono- ou di-(C₁-C₇-alkyl)-amino, C₁-C₇-alcanoylamino, C₁-C₇-alkyl-sulfonylamino, phényl- ou napthylsulfonylamino, phényl- ou naphtyl-C₁-C₇-alkylsulfonylamino, C₁-C₇-alcoxy-C₁-C₇-alcoxy, hydroxy-C₁-C₇-alcoxy, phényl- ou naphtyloxy, phényl- ou naphtyl-C₁-C₇-alkyloxy, C₁-C₇-alcanoyloxy, nitro, carboxyle, C₁-C₇-alcoxycarbonyle, phényl- ou naphtyl-C₁-C₇-alcoxycarbonyle, carbamoyle, N-mono- ou N,N-di-(C₁-C₇-alkyl-, phényl-, naphtyl-, phényl-C₁-C₇-alkyl- ou naphtyl-C₁-C₇-alkyl-)carbamoyle et N-mono- ou N,N-di-(C₁-C₇-alkyl-, phényl-, naphtyl-, phényl-C₁-C₇-alkyl- ou naphtyl-C₁-C₇-alkyl-)sulfamoyle ; et cyano ;
phényle ou naphtyle, chacun d'entre eux étant non substitué ou substitué par un ou plusieurs substituants choisis parmi le groupe constitué par C₁-C₇-alkyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, C₁-C₇-alkylsulfonylamino-C₁-C₇-alkyle, halogéno, hydroxy, C₁-C₇-alcoxy, C₁-C₇-alcoxy-C₁-C₇-alcoxy, hydroxy-C₁-C₇-alcoxy, phényl- ou naphtyloxy, phényl- ou naphtyl-C₁-C₇-alkyloxy, C₁-C₇-alcanoyloxy, nitro, amino, mono- ou di-(C₁-C₇-alkyl)-amino, C₁-C₇-alcanoylamino, carboxyle, C₁-C₇-alcoxycarbonyle, halogéno-C₁-C₇-alcoxycarbonyle, phényl- ou naphtyl-C₁-C₇-alcoxycarbonyle, carbamoyle, N-mono- ou N,N-di-(C₁-C₇-alkyl-, phényl-, naphtyl-, phényl-C₁-C₇-alkyl- ou naphtyl-C₁-C₇-alkyl)carbamoyle et N-mono- ou N,N-di-(C₁-C₇-alkyl-, phényl-, naphtyl-, phényl-C₁-C₇-alkyl- ou naphtyl-C₁-C₇-alkyl-)sulfamoyle et cyano ;
phényl- ou naphtyl-C₁-C₇-alkyle, où chacun parmi phényle ou naphtyle est non substitué ou substitué par un ou plusieurs substituants choisis parmi le groupe venant d'être mentionné pour phényle ou naphtyle substitué R²; C₃-C₁₀-cycloalkyle qui est non substitué ou substitué par un ou plusieurs substituants choisis parmi le groupe venant d'être mentionné pour phényle ou naphtyle substitué R² ;
C₃-C₁₀-cycloalkyl-C₁-C₇-alkyle où cycloalkyle est non substitué ou substitué par un ou plusieurs substituants choisis parmi le groupe venant d'être mentionné pour phényle ou naphtyle substitué R²; ou
pyrrolyle, furanyle ou thiényle,
ou, si L est méthylène, oxy, thio ou imino, R² est choisi parmi l'un des groupes de motifs R² venant d'être mentionnés et parmi hydrogène ;
R³ est C₁-C₇-alkyle carbamoyl-C₁-C₇-alkyle, N-mono- ou N,N-di-(C₃-C₈-cycloalkyl-, hétérocyclyl-, phényl-, naphtyl-, C₁-C₇-alkyl-, C₃-C₈-cycloalkyl-C₁-C₇-alkyle, hétérocyclyl-C₁-C₇-alkyle, phényl-C₁-C₇-alkyle et/ou naphtyl-C₁-C₇-alkyl-)aminocarbonyl-C₁-C₇-alkyle ;
phényle, naphtyle, phényl-C₁-C₇-alkyle ou naphtyl-C₁-C₇-alkyle, où chaque phényle ou naphtyle est non substitué ou substitué par un ou plusieurs substituants choisis parmi le groupe constitué par phényle, naphtyle, C₁-C₇-alkyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, C₁-C₇-alkyl-sulfonylylamino-C₁-C₇-alkyle, phényle, naphtyle, mono- ou di- (C₁-C₇-alcoxy) phényle ou -naphtyle, C₁-C₇-alkylènedioxy-phényle où les atomes d'oxy sont liés à des atomes de cycle phényle adjacents, halogéno, hydroxy, C₁-C₇-alcoxy, halogéno-C₁-C₇-alcoxy, hydroxy-C₁-C₇-alcoxy, C₁-C₇-alcoxy-C₁-C₇-alcoxy, phényl- ou naphtyloxy, phényl- ou naphtyl(non substitué ou mono-, di- ou tri-C₁-C₇-alkyl-substitué)-C₁-C₇-alkyloxy, C₁-C₇-alcanoyloxy, nitro, amino, mono- ou di-(C₁-C₇-alkyl)-amino, C₁-C₇-alcanoylamino, C₁-C₇-alkylsulfonylylamino, carboxyle, C₁-C₇-alcoxycarbonyle, phényl- ou naphtyl-C₁-C₇-alcoxycarbonyle, carbamoyle et cyano, C₁-C₇-alkylsulfonyle, C₁-C₇-alkylène qui est non substitué ou substitué par jusqu'à quatre substituants C₁-C₇-alkyle et lié à deux atomes de cycle adjacents du motif phényle ou naphtyle ; pyrrolyle, furanyle et thiényle ; phényl- ou naphtyl-sulfonyle ou phényl-C₁-C₇-alkyl- ou naphtyl-C₁-C₇-alkyl-sulfonyle, où chaque phényle ou naphtyle est non substitué ou substitué par un ou
plusieurs substituants choisis parmi le groupe venant d'être décrit pour phényle ou naphtyle substitué R³; C₃-C₁₀-cycloalkyle ou C₃-C₁₀-cycloalkyl-C₁-C₇-alkyle, où dans les deux, cycloalkyle est non substitué ou substitué par un ou plusieurs parmi les substituants venant d'être mentionnés pour phényle ou naphtyle substitué R³ ; ou
hétérocyclyle ou hétérocyclyl-C₁-C₇-alkyle où hétérocyclyle est choisi parmi pyrrolyle, furanyle, thiényle, pyrimidine-2,4-dione-1-, -3- ou -5-yle, indolyle, benzofuranyle, benzimidazolyle, benzopyrazolyle, quinoléinyle, isoquinoléinyle, méthylène-dioxy-phényle, éthylène-1,2-dioxy-phényle ou triméthylène-1,3-dioxyphényle où les groupements oxy sont liés à des atomes de cycle adjacents du cycle phényle, où chacun des motifs hétérocyclyle est non substitué ou substitué tel que mentionné ci-dessus pour phényle substitué R³;
ou, si L est imino, oxy ou thio, peut être de manière alternative phényl- ou naphtylcarbonyle, C₁-C₇-alcoxycarbonyle (signifiant C₁-C₇-alkyl-O-C(=O)-), phényloxycarbonyle, naphtyloxycarbonyle, cycloalkyloxycarbonyle, hétérocyclyloxycarbonyle, phényl-C₁-C₇-alkyloxycarbonyle, naphtyl-C₁-C₇-alkyloxycarbonyle, cycloalkyl-C₁-C₇-alcoxycarbonyle, hétérocyclyl-C₁-C₇-alcoxycarbonyle ou N-mono- ou N,N-di-(alkyle, naphtyle, phényle, hétérocyclyle, cycloalkyle, naphtyl-, hétérocyclyl-, cycloalkyl- ou phényl-C₁-C₇-alkyl)-aminocarbonyle, où dans chaque cas les cycles phényle ou naphtyle sont non substitués ou substitués tel que mentionné ci-dessus pour phényle ou naphtyle substitué R³ ; et
R⁴ est hydrogène ou hydroxy ; et
L est une liaison, méthylène (-CH₂-), oxy (-O-) ou imino (-NH-) ;
ou R³ et R⁴ qui est alors -O- conjointement avec L qui est alors méthylène et le carbone auquel R³-L- et R⁴ sont liés forment un cycle de 5 chaînons non substitué ou substitué annelé à benzo où benzo est substitué par un ou plusieurs substituants choisis parmi le groupe constitué par C₁-C₇-alkyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, halogéno, hydroxy, C₁-C₇-alcoxy, C₁-C₇-alcoxy-C₁-C₇-alcoxy, phényl- ou naphtyloxy, phényl- ou naphtyl-C₁-C₇-alkyloxy, C₁-C₇-alcanoyloxy, nitro, amino, mono- ou di-(C₁-C₇-alkyl)-amino, C₁-C₇-alcanoylamino, amino-C₁-C₇-alkyle, mono- ou di-(C₁-C₇-alkyl)-amino-C₁-C₇-alkyle, C₁-C₇-alcanoylamino-C₁-C₇-alkyle, carboxyle, C₁-C₇-alcoxycarbonyle, phényl- ou naphtyl-C₁-C₇-alcoxycarbonyle, carbamoyle et cyano, ou non substitué, formant ainsi un composé spiro de formule I, ou
R³ et R⁴ conjointement avec L forment oxo (=O) ; et
T est méthylène, carbonyle ou thiocarbonyle ;
ou un sel pharmaceutiquement acceptable de celui-ci.

3. Composé de formule I selon l'une quelconque des revendications 1 ou 2, dans laquelle
R¹ est phényl- ou naphtyl-carbonyle ou phényl- ou naphtyl-C₁-C₇-alkylcarbonyle, où chaque phényle ou naphtyle est non substitué ou substitué par un ou plusieurs substituants choisis parmi le groupe constitué par
un substituant de formule -(C₀-C₇-alkylène)-(X)ᵣ-(C₁-C₇-alkylène-)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NV-CO-, -CO-NV-, -NV-SO₂-, -SO₂-NV, -NV-CO-NV-, -NV-CO-O-, -O-CO-NV-, -NV-SO₂-NV- où V est hydrogène ou alkyle non substitué ou substitué tel que défini ci-après, hydroxy-C₁-C₇-alkyle, C₁-C₇-alcoxy-C₁-C₇-alkyle, C₁-C₇-alcoxy-C₁-C₇-alcoxy-C₁-C₇-alkyle, C₁-C₇-alcanoyloxy-C₁-C₇-alkyle, amino-C₁-C₇-alkyle, (N-) mono- ou (N,N-) di-(C₁-C₇-alkyl)-amino-C₁-C₇-alkyle, C₁-C₇-alcoxy-C₁-C₇-alkyl-amino-C₁-C₇-alkyle, mono- (naphtyl- ou phényl)-amino-C₁-C₇-alkyle, mono- (naphtyl- ou phényl-C₁-C₇-alkyl)-amino-C₁-C₇-alkyle, C₁-C₇-alcanoylamino-C₁-C₇-alkyle, C₁-C₇-alkyl-O-CO-NH-C₁-C₇-alkyle, C₁-C₇-alkylsulfonylamino-C₁-C₇-alkyle, C₁-C₇-alkyl-NH-CO-NH-Cₗ-C₇-alkyle, C₁-C₇-alkyl-NH-SO₂-NH-C₁-C₇-alkyle, C₁-C₇-alcoxy, hydroxy-C₁-C₇-alcoxy, C₁-C₇-alcoxy-C₁-C₇-alcoxy, C₁-C₇-alcanoyloxy, mono- ou di-(C₁-C₇-alkyl)-amino, mono- di- (naphtyl- ou phényl-C₁-C₇-alkyl)-amino, N-mono-C₁-C₇-alcoxy-C₁-C₇-alkylamino, C₁-C₇-alcanoylamino, C₁-C₇-alkylsulfonylamino, C₁-C₇-alcoxycarbonyle, halogéno-C₁-C₇-alcoxycarbonyle, hydroxy-C₁-C₇-alcoxycarbonyle, C₁-C₇-alcoxy-C₁-C₇-alcoxycarbonyle, amino-C₁-C₇-alcoxycarbonyle, (N-) mono-(C₁-C₇-alkyl)-amino-C₁-C₇-alcoxycarbonyle, C₁-C₇-alcanoylamino-C₁-C₇-alcoxycarbonyle, N-mono- ou N,N-di-(C₁-C₇-alkyl)-aminocarbonyle, N-C₁-C₇-alcoxy-C₁-C₇-alkylcarbamoyle ou N-mono- ou N,N-di-(C₁-C₇-alkyl)-aminosulfonyle ; ou
parmi phényl- ou naphtyl-C₁-C₇-alkyle, halogéno-C₁-C₇-alkyle, halogéno, hydroxy, phényl- ou naphtyloxy, phényl- ou naphtyl-C₁-C₇-alkyloxy, nitro, amino, amino-C₁-C₇-alkyle, carboxyle, halogéno-C₁-C₇-alcoxycarbonyle, phényl- ou naphtyl-C₁-C₇-alcoxycarbonyle, C₁-C₇-alkylsulfonyle, carbamoyle et cyano ;
pyrrolylcarbonyle, furanylcarbonyle, thiénylcarbonyle, pyrimidine-2,4-dione-1-, -2-, -3- ou -5-yl-carbonyle, indolyl-carbonyle, benzimidazolyl-carbonyle, benzopyrazolyl-carbonyle, benzofuranyl-carbonyle, quinoléinyl-carbonyle ou benzo[1,2,5]oxadiazolylcarbonyle, chacun d'entre eux étant non substitué ou substitué par un ou plusieurs substituants choisis indépendamment parmi un substituant de formule -(C₀-C₇-alkylène)-(X)ᵣ-(C₁-C₇-alkylène)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NV-CO-, -CO-NV-, -NV-SO₂-, -SO₂-NV, -NV-CO-NV-, -NV-CO-O-, -O-CO-NV-, -NV-SO₂-NV- où V est hydrogène ou alkyle non substitué ou substitué tel que défini ci-après, hydroxy-C₁-C₇-alkyle, C₁-C₇-alcoxy-C₁-C₇-alkyle, C₁-C₇-alcoxy-C₁-C₇-alcoxy-C₁-C₇-alkyle, C₁-C₇-alcanoyloxy-C₁-C₇-alkyle, amino-C₁-C₇-alkyle, (N-) mono- ou (N,N-) di-(C₁-C₇-alkyl)-amino-C₁-C₇-alkyle, C₁-C₇-alcoxy-C₁-C₇-alkylamino-C₁-C₇-alkyle, mono-(naphtyl- ou phényl)-amino-C₁-C₇-alkyle, mono-(naphtyl- ou phényl-C₁-C₇-alkyl)-amino-C₁-C₇-alkyle, C₁-C₇-alcanoylamino-C₁-C₇-alkyle, C₁-C₇-alkyl-O-CO-NH-C₁-C₇-alkyle, C₁-C₇-alkylsulfonylamino-C₁-C₇-alkyle, C₁-C₇-alkyl-NH-CO-NH-C₁-C₇-alkyle, C₁-C₇-alkyl-NH-SO₂-NH-C₁-C₇-alkyle, C₁-C₇-alcoxy, hydroxy-C₁-C₇-alcoxy, C₁-C₇-alcoxy-C₁-C₇alcoxy, C₁-C₇-alcanoyloxy, mono- ou di-(C₁-C₇-alkyl)-amino, mono- di- (naphtyl- ou phényl-C₁-C₇-alkyl)-amino, N-mono-C₁-C₇-alcoxy-C₁-C₇-alkylamino, C₁-C₇-alcanoylamino, C₁-C₇-alkylsulfonylamino, C₁-C₇-alcoxycarbonyle, hydroxy-C₁-C₇-alcoxycarbonyle, C₁-C₇-alcoxy-C₁-C₇-alcoxycarbonyle, amino-C₁-C₇-alcoxycarbonyle, (N-) mono-(C₁-C₇-alkyl)-amino-C₁-C₇-alcoxycarbonyle, C₁-C₇-alcanoylamino-C₁-C₇-alcoxycarbonyle, N-mono- ou N,N-di-(C₁-C₇-alkyl)-aminocarbonyle, N-C₁-C₇-alcoxy-C₁-C₇-alkylcarbamoyle ou N-mono- ou N,N-di-(C₁-C₇-alkyl)-aminosulfonyle ;
parmi phényl- ou naphtyl-C₁-C₇-alkyle, halogéno-C₁-C₇-alkyle, halogéno, hydroxy, phényl- ou naphtyloxy, phényl- ou naphtyl-C₁-C₇-alkyloxy, nitro, amino, amino-C₁-C₇-alkyle, carboxyle, halogéno-C₁-C₇-alcoxycarbonyle, phényl- ou naphtyl-C₁-C₇-alcoxycarbonyle, C₁-C₇-alkylsulfonyle, carbamoyle et cyano ;
R² est C₁-C₇-alkyle qui est non substitué ou substitué par un ou plusieurs substituants choisis parmi le groupe constitué par halogéno, phényl- ou naphtyle, hydroxy, C₁-C₇-alcoxy, amino, mono- ou di-(C₁-C₇-alkyl)-amino, C₁-C₇-alcanoylamino, C₁-C₇-alkyl-sulfonylamino, phényl- ou napthylsulfonylamino, phényl- ou naphtyl-C₁-C₇-alkylsulfonylamino, C₁-C₇-alcoxy-C₁-C₇-alcoxy, hydroxy-C₁-C₇-alcoxy, phényl- ou naphtyloxy, phényl- ou naphtyl-C₁-C₇-alkyloxy, C₁-C₇-alcanoyloxy, nitro, carboxyle, C₁-C₇-alcoxycarbonyle, phényl- ou naphtyl-C₁-C₇-alcoxycarbonyle, carbamoyle, N-mono- ou N,N-di-(C₁-C₇-alkyl-, phényl-, naphtyl-, phényl-C₁-C₇-alkyl- ou naphtyl-C₁-C₇-alkyl-)carbamoyle et N-mono- ou N,N-di-(C₁-C₇-alkyl-, phényl-, naphtyl-, phényl-C₁-C₇-alkyl- ou naphtyl-C₁-C₇-alkyl-)sulfamoyle ; et cyano ; C₃-C₁₀-cycloalkyle qui est non substitué ou substitué par un ou plusieurs substituants choisis indépendamment parmi les substituants mentionnés ci-dessus pour phényle ou naphtyle substitué R¹ ; ou C₃-C₁₀-cycloalkyl-C₁-C₇-alkyle où cycloalkyle est non substitué ou substitué par un ou plusieurs, par exemple jusqu'à trois, substituants choisis indépendamment parmi les substituants mentionnés ci-dessus pour phényle ou naphtyle substitué R¹ ;
et R³ et R⁴ qui est alors -O- conjointement avec L qui est alors méthylène et le carbone auquel R³-L- et R⁴ sont liés forment un cycle de 5 chaînons non substitué ou substitué annelé à benzo où benzo est substitué par un ou plusieurs substituants choisis parmi le groupe constitué par C₁-C₇-alkyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, halogéno, hydroxy, C₁-C₇-alcoxy, C₁-C₇-alcoxy-C₁-C₇-alcoxy, phényl- ou naphtyloxy, phényl- ou naphtyl-C₁-C₇-alkyloxy, C₁-C₇-alcanoyloxy, nitro, amino, mono- ou di-(C₁-C₇-alkyl)-amino, C₁-C₇-alcanoylamino, amino-C₁-C₇-alkyle, mono- ou di-(C₁-C₇-alkyl)-amino-C₁-C₇-alkyle, C₁-C₇-alcanoylamino-C₁-C₇-alkyle, carboxyle, C₁-C₇-alcoxycarbonyle, phényl- ou naphtyl-C₁-C₇-alcoxycarbonyle, carbamoyle et cyano, ou non substitué,
formant ainsi un composé spiro de formule I,
et T est carbonyle ou thiocarbonyle ou méthylène ;
ou un sel pharmaceutiquement acceptable de celui-ci.

4. Composé de formule I selon l'une quelconque des revendications 1 ou 2, dans laquelle
R¹ est phényl- ou naphtyl-carbonyle ou phényl- ou naphtyl-C₁-C₇-alkylcarbonyle, où chaque phényle ou naphtyle est non substitué ou substitué par un ou plusieurs substituants choisis parmi le groupe constitué par un substituant de formule -(C₀-C₇-alkylène)-(X)ᵣ-(C₁-C₇-alkylène)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NV-CO-, -CO-NV-, -NV-SO₂-, -SO₂-NV, -NV-CO-NV-, -NV-CO-O-, -O-CO-NV-, -NV-SO₂-NV- où V est hydrogène ou alkyle non substitué ou substitué tel que défini ci-après, hydroxy-C₁-C₇-alkyle, C₁-C₇-alcoxy-C₁-C₇-alkyle, C₁-C₇-alcoxy-C₁-C₇-alcoxy-C₁-C₇-alkyle, C₁-C₇-alcanoyloxy-C₁-C₇-alkyle, amino-C₁-C₇-alkyle, (N-) mono- ou (N,N-) di-(C₁-C₇-alkyl)-amino-C₁-C₇-alkyle, C₁-C₇-alcoxy-C₁-C₇-alkylamino-C₁-C₇-alkyle, mono-(naphtyl- ou phényl)-amino-C₁-C₇-alkyle, mono- (naphtyl- ou phényl-C₁-C₇-alkyl)-amino-C₁-C₇-alkyle, C₁-C₇-alcanoylamino-C₁-C₇-alkyle, C₁-C₇-alkyl-O-CO-NH-C₁-C₇-alkyle, C₁-C₇-alkylsulfonylamino-C₁-C₇-alkyle, C₁-C₇-alkyl-NH-CO-NH-C₁-C₇-alkyle, C₁-C₇-alkyl-NH-SO₂-NH-C₁-C₇-alkyle, C₁-C₇-alcoxy, hydroxy-C₁-C₇-alcoxy, C₁-C₇-alcoxy-C₁-C₇alcoxy, C₁-C₇-alcanoyloxy, mono- ou di-(C₁-C₇-alkyl)-amino, mono- di- (naphtyl- ou phényl-C₁-C₇-alkyl)-amino, N-mono-C₁-C₇-alcoxy-C₁-C₇-alkylamino, C₁-C₇-alcanoylamino, C₁-C₇-alkylsulfonylamino, C₁-C₇-alcoxycarbonyle, hydroxy-C₁-C₇-alcoxycarbonyle, C₁-C₇-alcoxy-C₁-C₇-alcoxycarbonyle, amino-C₁-C₇-alcoxycarbonyle, (N-) mono(C₁-C₁₇-alkyl)-amino-C₁-C₇-alcoxycarbonyle, C₁-C₇-alcanoylamino-C₁-C₇-alcoxycarbonyle, N-mono- ou N,N-di-(C₁-C₇-alkyl)-aminocarbonyle, N-C₁-C₇-alcoxy-C₁-C₇-alkylcarbamoyle ou N-mono- ou N,N-di-(C₁-C₇-alkyl)-aminosulfonyle ; ou
parmi phényl- ou naphtyl-C₁-C₇-alkyle, halogéno-C₁-C₇-alkyle, halogéno, hydroxy, phényl- ou naphtyloxy, phényl- ou naphtyl-C₁-C₇-alkyloxy, nitro, amino, amino-C₁-C₇-alkyle, carboxyle, halogéno-C₁-C₇-alcoxycarbonyle, phényl- ou naphtyl-C₁-C₇-alcoxycarbonyle, C₁-C₇-alkylsulfonyle, carbamoyle et cyano ; ou
pyrrolylcarbonyle, furanylcarbonyle, thiénylcarbonyle, pyrimidine-2,4-dione-1-, -2-, -3- ou -5-yl-carbonyle, indolyl-carbonyle, benzimidazolyl-carbonyle, benzopyrazolyl-carbonyle, benzofuranylcarbonyle, quinoléinyl-carbonyle ou benzo[1,2,5]oxadiazolylcarbonyle, chacun d'entre eux étant non substitué ou substitué par un ou plusieurs, par exemple jusqu'à trois, substituants choisis indépendamment parmi un substituant de formule -(C₀-C₇-alkylène)-(X)ᵣ-(C₁-C₇-alkylène)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NV-CO-, -CO-NV-, -NV-SO₂-, -SO₂-NV, -NV-CO-NV-, -NV-CO-O-, -O-CO-NV-, -NV-SO₂-NV- où V est hydrogène ou alkyle non substitué ou substitué tel que défini ci-après, hydroxy-C₁-C₇-alkyle, C₁-C₇-alcoxy-C₁-C₇-alkyle, tel que 3-méthoxypropyle ou 2-méthoxyéthyle, C₁-C₇-alcoxy-C₁-C₇-alcoxy-C₁-C₇-alkyle, C₁-C₇-alcanoyloxy-C₁-C₇-alkyle, amino-C₁-C₇-alkyle, (N-) mono- ou (N,N-) di-(C₁-C₇-alkyl)-amino-C₁-C₇-alkyle, C₁-C₇-alcoxy-C₁-C₇-alkylamino-C₁-C₇-alkyle, mono-(naphtyl- ou phényl)-amino-C₁-C₇-alkyle, mono- (naphtyl- ou phényl-C₁-C₇-alkyl)-amino-C₁-C₇-alkyle, C₁-C₇-alcanoylamino-C₁-C₇-alkyle, C₁-C₇-alkyl-O-CO-NH-C₁-C₇-alkyle, C₁-C₇-alkylsulfonylamino-C₁-C₇-alkyle, C₁-C₇-alkyl-NH-CO-NH-C₁-C₇-alkyle, C₁-C₇-alkyl-NH-SO₂-NH-C₁-C₇-alkyle, C₁-C₇-alcoxy, hydroxy-C₁-C₇-alcoxy, C₁-C₇-alcoxy-C₁-C₇alcoxy, C₁-C₇-alcanoyloxy, mono- ou di-(C₁-C₇-alkyl)-amino, mono- di- (naphtyl- ou phényl-C₁-C₇-alkyl)-amino, N-mono-C₁-C₇-alcoxy-C₁-C₇-alkylamino, C₁-C₇-alcanoylamino, C₁-C₇-alkylsulfonylamino, C₁-C₇-alcoxycarbonyle, hydroxy-C₁-C₇-alcoxycarbonyle, C₁-C₇-alcoxy-C₁-C₇-alcoxycarbonyle, amino-C₁-C₇-alcoxycarbonyle, (N-) mono (C₁-C₇-alkyl)-amino-C₁-C₇-alcoxycarbonyle, C₁-C₇-alcanoylamino-C₁-C₇-alcoxycarbonyle, N-mono- ou N,N-di-(C₁-C₇-alkyl)-aminocarbonyle, N-C₁-C₇-alcoxy-C₁-C₇-alkylcarbamoyle ou
N-mono- ou N,N-di-(C₁-C₇-alkyl)-aminosulfonyle ; ou parmi phényl- ou naphtyl-C₁-C₇-alkyle, halogéno-C₁-C₇-alkyle, halogéno, hydroxy, phényl- ou naphtyloxy, phényl- ou naphtyl-C₁-C₇-alkyloxy, nitro, amino, amino-C₁-C₇-alkyle, carboxyle, phényl- ou naphtyl-C₁-C₇-alcoxycarbonyle, halogéno-C₁-C₇-alcoxycarbonyle, C₁-C₇-alkylsulfonyle, carbamoyle et cyano ;
R² est C₁-C₇-alkyle qui est non substitué ou substitué par un ou plusieurs substituants choisis parmi le groupe constitué par halogéno, phényl- ou naphtyle, hydroxy, C₁-C₇-alcoxy, amino, mono- ou di-(C₁-C₇-alkyl)-amino, C₁-C₇-alcanoylamino, C₁-C₇-alkyl-sulfonylamino, phényl- ou napthylsulfonylamino, phényl- ou naphtyl-C₁-C₇-alkylsulfonylamino, C₁-C₇-alcoxy-C₁-C₇-alcoxy, hydroxy-C₁-C₇-alcoxy, phényl- ou naphtyloxy, phényl- ou naphtyl-C₁-C₇-alkyloxy, C₁-C₇-alcanoyloxy, nitro, carboxyle, C₁-C₇-alcoxycarbonyle, phényl- ou naphtyl-C₁-C₇-alcoxycarbonyle, carbamoyle, N-mono- ou N,N-di-(C₁-C₇-alkyl-, phényl-, naphtyl-, phényl-C₁-C₇-alkyl- ou naphtyl-C₁-C₇-alkyl-)carbamoyle et N-mono- ou N,N-di-(C₁-C₇-alkyl-, phényl-, naphtyl-, phényl-C₁-C₇-alkyl- ou naphtyl-C₁-C₇-alkyl-)sulfamoyle ; et cyano ; C₃-C₁₀-cycloalkyle qui est non substitué ou substitué par un ou plusieurs substituants choisis indépendamment parmi les substituants mentionnés ci-dessus pour phényle ou naphtyle substitué R¹ ; ou C₃-C₁₀-cycloalkyl-C₁-C₇-alkyle où cycloalkyle est non substitué ou substitué par un ou plusieurs substituants choisis indépendamment parmi les substituants mentionnés ci-dessus pour phényle ou naphtyle substitué R¹ ;
R³ est phényle, naphtyle, phényl-C₁-C₇-alkyle ou naphtyl-C₁-C₇-alkyle, où chaque phényle ou naphtyle est non substitué ou substitué par un ou plusieurs, par exemple jusqu'à trois, substituants choisis parmi le groupe constitué par C₁-C₇-alkyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, C₁-C₇-alkyl-sulfonylylamino-C₁-C₇-alkyle, phényle, naphtyle, mono- ou di-(C₁-C₇-alcoxy)phényle ou -naphtyle, C₁-C₇-alkylènedioxy-phényle où les atomes d'oxy sont liés à des atomes de cycle phényle adjacents, halogéno, hydroxy, C₁-C₇-alcoxy, halogéno-C₁-C₇-alcoxy, hydroxy-C₁-C₇-alcoxy, C₁-C₇-alcoxy-C₁-C₇-alcoxy, phényl- ou naphtyloxy, phényl- ou naphtyl(non substitué ou mono-, di- ou tri-C₁-C₇-alkyle substitué)-C₁-C₇-alkyloxy, C₁-C₇-alcanoyloxy, nitro, amino, mono- ou di-(C₁-C₇-alkyl)-amino, C₁-C₇-alcanoylamino C₁-C₇-alkylsulfonylylamino, carboxyle, C₁-C₇-alcoxycarbonyle, phényl- ou naphtyl-C₁-C₇-alcoxycarbonyle, carbamoyle et cyano, C₁-C₇-alkylsulfonyle, C₁-C₇-alkylène qui est non substitué ou substitué par jusqu'à quatre substituants C₁-C₇-alkyle et lié à deux atomes de cycle adjacents du motif phényle ou naphtyle ; pyrrolyle, furanyle et thiényle ;
R⁴ est hydroxy ;
L est méthylène ; et
T est carbonyle, thiocarbonyle ou préférablement méthylène ;
ou un sel pharmaceutiquement acceptable de celui-ci.

5. Composé de formule I selon l'une quelconque des revendications 1 ou 2, dans laquelle
R¹ est phényle ou naphtyle, chacun d'entre eux étant non substitué ou substitué par un ou plusieurs substituants choisis parmi le groupe constitué par C₁-C₇-alkyle, phényle, naphtyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, C₁-C₇-alkyl-sulfonylamino-C₁-C₇-alkyle, halogéno, hydroxy, C₁-C₇-alcoxy, hydroxy-C₁-C₇-alcoxy, C₁-C₇-alcoxy-C₁-C₇-alcoxy, phényl- ou naphtyloxy, phényl- ou naphtyl-C₁-C₇-alkyloxy, C₁-C₇-alcanoyloxy, nitro, amino, mono- ou di-(C₁-C₇-alkyl)-amino, C₁-C₇-alcanoylamino, carboxyle, C₁-C₇-alcoxycarbonyle, phényl- ou naphtyl-C₁-C₇-alcoxycarbonyle, carbamoyle, N-mono- ou N,N-di-(C₁-C₇-alkyle et/ou (phényl- ou naphtyl)-C₁-C₇-alkyl)-carbamoyle, C₁-C₇-alkylsulfonyle, phényl- ou naphtylsulfonyle non substitué ou C₁-C₇-alkyl-substitué, N-mono- ou N, N-di- (C₁-C₇-alkyle et/ou (phényl- ou naphtyl)-C₁-C₇-alkyl)-sulfamoyle et cyano ;
phényl- ou naphtyl-C₁-C₇-alkyle, où chacun parmi phényle ou naphtyle est non substitué ou substitué par un ou plusieurs substituants choisis parmi le groupe constitué par les substituants venant d'être mentionnés pour phényle ou naphtyle substitué, pyrrolyle, furanyle, thiényle, pyrimidine-2,4-dione-1-, -2-, -3- ou -5-yle et benzo[1,3]dioxalyle, chacun d'entre eux étant non substitué ou substitué par un ou plusieurs substituants choisis indépendamment parmi ceux mentionnés pour phényle ou naphtyle substitué R¹ ci-dessus ; pyrrolyl-C₁-C₇-alkyle, furanyl-C₁-C₇-alkyle, thiényl-C₁-C₇-alkyle, pyrimidine-2,4-dione-1-, -2-, -3- ou -5-yl-C₁-C₇-alkyle, indolyl-C₁-C₇-alkyle, benzofuranyl-C₁-C₇-alkyle, benzimidazolyl-C₁-C₇-alkyle, benzopyrazolyl-C₁-C₇-alkyle, quinoléinyl-C₁-C₇-alkyle, isoquinolyl-C₁-C₇-alkyle ou benzo[1,2,5]oxadiazolyl-C₁-C₇-alkyle, chacun d'entre eux étant non substitué ou substitué par un ou plusieurs, par exemple jusqu'à trois, substituants choisis indépendamment parmi les substituants mentionnés ci-dessus pour phényle ou naphtyle substitué R¹ ;
C₃-C₁₀-cycloalkyle qui est non substitué ou substitué par un ou plusieurs substituants choisis indépendamment parmi les substituants mentionnés ci-dessus pour phényle ou naphtyle substitué R¹ ;
C₃-C₁₀-cycloalkyl-C₁-C₇-alkyle où cycloalkyle est non substitué ou substitué par un ou plusieurs substituants choisis indépendamment parmi les substituants mentionnés ci-dessus pour phényle ou naphtyle substitué R¹ ;
phényl- ou naphtyl-carbonyle ou phényl- ou naphtyl-C₁-C₇-alkylcarbonyle, où chaque phényle ou naphtyle est non substitué ou substitué par un ou plusieurs substituants choisis parmi le groupe constitué par
un substituant de formule -(C₀-C₇-alkylène)-(X)ᵣ-(C₁-C₇-alkylène)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NV-CO-, -CO-NV-, -NV-SO₂-, -SO₂-NV, -NV-CO-NV-, -NV-CO-O-, -O-CO-NV-, -NV-SO₂-NV- où V est hydrogène ou alkyle non substitué ou substitué tel que défini ci-après ; hydroxy-C₁-C₇-alkyle, C₁-C₇-alcoxy-C₁-C₇-alkyle, C₁-C₇-alcoxy-C₁-C₇-alcoxy-C₁-C₇-alkyle, C₁-C₇-alcanoyloxy-C₁-C₇-alkyle, amino-C₁-C₇-alkyle, (N-) mono- ou (N,N-) di-(C₁-C₇-alkyl)-amino-C₁-C₇-alkyle, C₁-C₇-alcoxy-C₁-C₇-alkylamino-C₁-C₇-alkyle, mono-(naphtyl- ou phényl)-amino-C₁-C₇-alkyle, mono- (naphtyl- ou phényl-C₁-C₇-alkyl)-amino-C₁-C₇-alkyle, C₁-C₇-alcanoylamino-C₁-C₇-alkyle, C₁-C₇-alkyl-O-CO-NH-C₁-C₇-alkyle, C₁-C₇-alkylsulfonylamino-C₁-C₇-alkyle, C₁-C₇-alkyl-NH-CO-NH-C₁-C₇-alkyle, C₁-C₇-alkyl-NH-SO₂-NH-C₁-C₇-alkyle, C₁-C₇-alcoxy, hydroxy-C₁-C₇-alcoxy, C₁-C₇-alcoxy-C₁-C₇alcoxy, C₁-C₇-alcanoyloxy, mono- ou di-(C₁-C₇-alkyl)-amino, mono- di- (naphtyl- ou phényl-C₁-C₇-alkyl)-amino, N-mono-C₁-C₇-alcoxy-C₁-C₇-alkylamino, C₁-C₇-alcanoylamino, C₁-C₇-alkylsulfonylamino, C₁-C₇-alcoxycarbonyle, hydroxy-C₁-C₇-alcoxycarbonyle, C₁-C₇-alcoxy-C₁-C₇-alcoxycarbonyle, amino-C₁-C₇-alcoxycarbonyle, (N-) mono (C₁-C₇-alkyl)-amino-C₁-C₇-alcoxycarbonyle, C₁-C₇-alcanoylamino-C₁-C₇-alcoxycarbonyle, N-mono- ou N,N-di-(C₁-C₇-alkyl)-aminocarbonyle, N-C₁-C₇-alcoxy-C₁-C₇-alkylcarbamoyle ou N-mono- ou N,N-di-(C₁-C₇-alkyl)-aminosulfonyle ; ou parmi phényl- ou naphtyl-C₁-C₇-alkyle, halogéno-C₁-C₇-alkyle, halogéno, hydroxy, phényl- ou naphtyloxy, phényl- ou naphtyl-C₁-C₇-alkyloxy, nitro, amino, amino-C₁-C₇-alkyle, carboxyle, phényl- ou naphtyl-C₁-C₇-alcoxycarbonyle, halogéno-C₁-C₇-alcoxycarbonyle, C₁-C₇-alkylsulfonyle, carbamoyle et cyano ;
hétérocyclylcarbonyle tel que pyrrolylcarbonyle, furanylcarbonyle, thiénylcarbonyle, pyrimidine-2,4-dione-1-, -2-, -3- ou -5-yl-carbonyle, indolyl-carbonyle, 3-méthylindolyl-carbonyle, benzimidazolyl-carbonyle, benzopyrazolyl-carbonyle benzofuranylcarbonyle, quinoléinyl-carbonyle ou benzo[1,2,5]oxadiazolyl-carbonyle, chacun d'entre eux étant non substitué ou substitué par un ou plusieurs, par exemple jusqu'à trois, substituants choisis indépendamment parmi un substituant de formule -(C₀-C₇-alkylène)-(X)ᵣ-(C₁-C₇-alkylène)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NVCO-, -CO-NV-, -NV-SO₂-, -SO₂-NV, -NV-CO-NV-, -NV-CO-O-, -O-CO-NV-, -NV-SO₂-NV- où V est hydrogène ou alkyle non substitué ou substitué tel que défini ci-après ; hydroxy-C₁-C₇-alkyle, C₁-C₇-alcoxy-C₁-C₇-alkyle, tel que 3-méthoxypropyle ou 2-méthoxyéthyle, C₁-C₇-alcoxy-C₁-C₇-alcoxy-C₁-C₇-alkyle, C₁-C₇-alcanoyloxy-C₁-C₇-alkyle, amino-C₁-C₇-alkyle, tel que aminométhyle, (N-) mono- ou (N,N-) di- (C₁-C₇-alkyl)-amino-C₁-C₇-alkyle, C₁-C₇-alcoxy-C₁-C₇-alkylamino-C₁-C₇-alkyle, mono-(naphtyl- ou phényl)-amino-C₁-C₇-alkyle, mono- (naphtyl- ou phényl-C₁-C₇-alkyl)-amino-C₁-C₇-alkyle, C₁-C₇-alcanoylamino-C₁-C₇-alkyle, C₁-C₇-alkyl-O-CO-NH-C₁-C₇-alkyle, C₁-C₇-alkylsulfonylamino-C₁-C₇-alkyle, C₁-C₇-alkyl-NH-CO-NH-C₁-C₇-alkyle, C₁-C₇-alkyl-NH-SO₂-NH-C₁-C₇-alkyle, C₁-C₇-alcoxy, hydroxy-C₁-C₇-alcoxy, C₁-C₇-alcoxy-C₁-C₇alcoxy, C₁-C₇-alcanoyloxy, mono- ou di-(C₁-C₇-alkyl)-amino, mono- di- (naphtyl- ou phényl-C₁-C₇-alkyl)-amino, N-mono-C₁-C₇-alcoxy-C₁-C₇-alkylamino, C₁-C₇-alcanoylamino, C₁-C₇-alkylsulfonylamino, C₁-C₇-alcoxycarbonyle, hydroxy-C₁-C₇-alcoxycarbonyle, C₁-C₇-alcoxy-C₁-C₇-alcoxycarbonyle, amino-C₁-C₇-alcoxycarbonyle, (N-) mono- (C₁-C₇-alkyl)-amino-C₁-C₇-alcoxycarbonyle, C₁-C₇-alcanoylamino-C₁-C₇-alcoxycarbonyle, N-mono- ou N,N-di-(C₁-C₇-alkyl)-aminocarbonyle, N-C₁-C₇-alcoxy-C₁-C₇-alkylcarbamoyle ou N-mono- ou N,N-di-(C₁-C₇-alkyl)-aminosulfonyle ; ou
parmi phényl- ou naphtyl-C₁-C₇-alkyle, halogéno-C₁-C₇-alkyle, halogéno, hydroxy, phényl- ou naphtyloxy, phényl- ou naphtyl-C₁-C₇-alkyloxy, nitro, amino, amino-C₁-C₇-alkyle, carboxyle, phényl- ou naphtyl-C₁-C₇-alcoxycarbonyle, halogéno-C₁-C₇-alcoxycarbonyle, C₁-C₇-alkylsulfonyle, carbamoyle et cyano ;
ou phényl- ou naphtyl-sulfonyle, où chaque phényle ou naphtyle est non substitué ou substitué par un ou plusieurs substituants choisis parmi ceux mentionnés ci-dessus pour phényle ou naphtyle substitué R¹ ;
R² est C₁-C₇-alkyle qui est non substitué ou substitué par un ou plusieurs substituants choisis parmi le groupe constitué par halogéno, phényl- ou naphtyle, hydroxy, C₁-C₇-alcoxy, amino, mono- ou di-(C₁-C₇-alkyl)-amino, C₁-C₇-alcanoylamino, C₁-C₇-alkyl-sulfonylamino, phényl- ou napthylsulfonylamino, phényl- ou naphtyl-C₁-C₇-alkylsulfonylamino, C₁-C₇-alcoxy-C₁-C₇-alcoxy, hydroxy-C₁-C₇-alcoxy, phényl- ou naphtyloxy, phényl- ou naphtyl-C₁-C₇-alkyloxy, C₁-C₇-alcanoyloxy, nitro, carboxyle, C₁-C₇-alcoxycarbonyle, phényl- ou naphtyl-C₁-C₇-alcoxycarbonyle, carbamoyle, N-mono- ou N,N-di-(C₁-C₇-alkyl-, phényl-, naphtyl-, phényl-C₁-C₇-alkyl- ou naphtyl-C₁-C₇-alkyl-)carbamoyle et N-mono- ou N,N-di-(C₁-C₇-alkyl-, phényl-, naphtyl-, phényl-C₁-C₇-alkyl- ou naphtyl-C₁-C₇-alkyl-)sulfamoyle ; et cyano ;
phényle ou naphtyle, chacun d'entre eux étant non substitué ou substitué par un ou plusieurs substituants choisis parmi le groupe constitué par C₁-C₇-alkyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, C₁-C₇-alkylsulfonylamino-C₁-C₇-alkyle, halogéno, hydroxy, C₁-C₇-alcoxy, C₁-C₇-alcoxy-C₁-C₇-alcoxy, hydroxy-C₁-C₇-alcoxy, phényl- ou naphtyloxy, phényl- ou naphtyl-C₁-C₇-alkyloxy, C₁-C₇-alcanoyloxy, nitro, amino, mono- ou di-(C₁-C₇-alkyl)-amino, C₁-C₇-alcanoylamino, carboxyle, C₁-C₇-alcoxycarbonyle, halogéno-C₁-C₇-alcoxycarbonyle, phényl- ou naphtyl-C₁-C₇-alcoxycarbonyle, carbamoyle, N-mono- ou N,N-di-(C₁-C₇-alkyl-, phényl-, naphtyl-, phényl-C₁-C₇-alkyl- ou naphtyl-C₁-C₇-alkyl-)carbamoyle et N-mono- ou N,N-di-(C₁-C₇-alkyl-, phényl-, naphtyl-, phényl-C₁-C₇-alkyl- ou naphtyl-C₁-C₇-alkyl-)sulfamoyle et cyano ;
phényl- ou naphtyl-C₁-C₇-alkyle, où chacun parmi phényle ou naphtyle est non substitué ou substitué par un ou plusieurs substituants choisis parmi le groupe venant d'être mentionné pour phényle ou naphtyle substitué R² ; C₃-C₁₀-cycloalkyle qui est non substitué ou substitué par un ou plusieurs substituants choisis parmi le groupe venant d'être mentionné pour phényle ou naphtyle substitué R² ;
C₃-C₁₀-cycloalkyl-C₁-C₇-alkyle où cycloalkyle est non substitué ou substitué par un ou plusieurs, par exemple jusqu'à trois, substituants choisis parmi le groupe venant d'être mentionné pour phényle ou naphtyle substitué R² ; ou
pyrrolyle, furanyle ou thiényle ;
ou, si L est méthylène, oxy, thio ou imino, R² est choisi parmi l'un des groupes de motifs R² venant d'être mentionnés et parmi hydrogène ;
R³ est C₁-C₇-alkyle carbamoyl-C₁-C₇-alkyle, N-mono- ou N,N-di-(C₃-C₈-cycloalkyl-, hétérocyclyl-, phényl-, naphtyl-, C₁-C₇-alkyl-, C₃-C₈-cycloalkyl-C₁-C₇-alkyle, hétérocyclyl-C₁-C₇-alkyle, phényl-C₁-C₇-alkyle et/ou naphtyl-C₁-C₇-alkyl-)aminocarbonyl-C₁-C₇-alkyle ;
phényle, naphtyle, phényl-C₁-C₇-alkyle ou naphtyl-C₁-C₇-alkyle, où chaque phényle ou naphtyle est non substitué ou substitué par un ou plusieurs substituants choisis parmi le groupe constitué par phényle, naphtyle, C₁-C₇-alkyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, C₁-C₇-alkylsulfonylylamino-C₁-C₇-alkyle, phényle, naphtyle, mono- ou di- (C₁-C₇-alcoxy) phényle ou -naphtyle, C₁-C₇-alkylènedioxy-phényle où les atomes d'oxy sont liés à des atomes de cycle phényle adjacents, halogéno, hydroxy, C₁-C₇-alcoxy, halogéno-C₁-C₇-alcoxy, hydroxy-C₁-C₇-alcoxy, C₁-C₇-alcoxy-C₁-C₇-alcoxy, phényl- ou naphtyloxy, phényl- ou naphtyl(non substitué ou mono-, di- ou tri-C₁-C₇-alkyle substitué)-C₁-C₇-alkyloxy, C₁-C₇-alcanoyloxy, nitro, amino, mono- ou di-(C₁-C₇-alkyl)-amino, C₁-C₇-alcanoylamino, C₁-C₇-alkylsulfonylylamino, carboxyle, C₁-C₇-alcoxycarbonyle, phényl- ou naphtyl-C₁-C₇-alcoxycarbonyle, carbamoyle et cyano, C₁-C₇-alkylsulfonyle, C₁-C₇-alkylène qui est non substitué ou substitué par jusqu'à quatre substituants C₁-C₇-alkyle et lié à deux atomes de cycle adjacents du motif phényle ou naphtyle; pyrrolyle, furanyle et thiényle ; phényl- ou naphtyl-sulfonyle ou phényl-C₁-C₇-alkyl- ou naphtyl-C₁-C₇-alkyl-sulfonyle, où chaque phényle ou naphtyle est non substitué ou substitué par un ou plusieurs substituants choisis parmi le groupe venant d'être décrit pour phényle ou naphtyle substitué R³ ; C₃-C₁₀-cycloalkyle ou C₃-C₁₀-cycloalkyl-C₁-C₇-alkyle, où dans les deux, cycloalkyle est non substitué ou substitué par un ou plusieurs parmi les substituants venant d'être mentionnés pour phényle ou naphtyle substitué R³ ;
ou hétérocyclyle ou hétérocyclyl-C₁-C₇-alkyle où hétérocyclyle est choisi parmi pyrrolyle, furanyle, thiényle, pyrimidine-2,4-dione-1-, -3- ou -5-yle, indolyle, benzofuranyle, benzimidazolyle, benzopyrazolyle, quinoléinyle, isoquinoléinyle, méthylène-dioxy-phényle, éthylène-1,2-dioxy-phényle ou triméthylène-1,3-dioxyphényle où les groupements oxy sont liés à des atomes de cycle adjacents du cycle phényle, où chacun des motifs hétérocyclyle est non substitué ou substitué tel que mentionné ci-dessus pour phényle substitué R³ ;
ou, si L est imino, oxy ou thio, peut être de manière alternative phényl- ou naphtylcarbonyle, C₁-C₇-alcoxycarbonyle (signifiant C₁-C₇-alkyl-O-C(=O)-), phényloxycarbonyle, naphtyloxycarbonyle, cycloalkyloxycarbonyle, hétérocyclyloxycarbonyle, phényl-C₁-C₇-alkyloxycarbonyle, naphtyl-C₁-C₇-alkyloxycarbonyle, cycloalkyl-C₁-C₇-alcoxycarbonyle, hétérocyclyl-C₁-C₇-alcoxycarbonyle ou N-mono- ou N,N-di-(alkyle, naphtyle, phényle, hétérocyclyle, cycloalkyle, naphtyl-, hétérocyclyl-, cycloalkyl- ou phényl-C₁-C₇-alkyl)-aminocarbonyle, où dans chaque cas les cycles phényle ou naphtyle sont non substitués ou substitués tel que mentionné ci-dessus pour phényle ou naphtyle substitué R³ ; et
R⁴ est hydrogène ;
L est méthylène (-CH₂-), oxy (-O-) ou imino (-NH-) ; et
T est carbonyle, thiocarbonyle ou méthylène ;
ou un sel pharmaceutiquement acceptable de celui-ci.

6. Composé de formule I selon l'une quelconque des revendications 1 ou 2, dans laquelle
R¹ est phényle, naphtyle, phénylcarbonyle, naphtylcarbonyle ou phényl- ou naphtyl-C₁-C₇-alkyle, où chaque phényle ou naphtyle est non substitué ou substitué par un ou plusieurs, par exemple jusqu'à trois, substituants choisis parmi le groupe constitué par
un substituant de formule -(C₀-C₇-alkylène)-(X)ᵣ-(C₁-C₇-alkylène-)-(Y)ₛ-(C₀-C₇-alkylène)-H où C₀-alkylène signifie qu'une liaison est présente au lieu d'alkylène lié, r et s, chacun indépendamment l'un de l'autre, valent 0 ou 1 et chacun parmi X et Y, si présent et indépendamment les uns des autres, est -O-, -NV-, -S-, -O-CO-, -CO-O-, -NV-CO-, -CO-NV-, -NV-SO₂-, -SO₂-NV, -NV-CO-NV-, -NV-CO-O-, -O-CO-NV-, -NV-SO₂-NV- où V est hydrogène ou alkyle non substitué ou substitué tel que défini ci-après, choisi notamment parmi C₁-C₇-alkyle, phényle, naphtyle, phényl- ou naphtyl-C₁-C₇-alkyle et halogéno-C₁-C₇-alkyle ; par exemple C₁-C₇-alkyle, tel que méthyle, éthyle, n-propyle, isopropyle, n-butyle, isobutyle, sec-butyle ou tertio-butyle, hydroxy-C₁-C₇-alkyle, C₁-C₇-alcoxy-C₁-C₇-alkyle, tel que 3-méthoxypropyle ou 2-méthoxyéthyle, C₁-C₇-alcoxy-C₁-C₇-alcoxy-C₁-C₇-alkyle, C₁-C₇-alcanoyloxy-C₁-C₇-alkyle, amino-C₁-C₇-alkyle, tel que aminométhyle, (N-) mono- ou (N,N-) di-(C₁-C₇-alkyl)-amino-C₁-C₇-alkyle, C₁-C₇-alcoxy-C₁-C₇-alkylamino-C₁-C₇-alkyle, mono-(naphtyl- ou phényl)-amino-C₁-C₇-alkyle, mono- (naphtyl- ou phényl-C₁-C₇-alkyl)-amino-C₁-C₇-alkyle, C₁-C₇-alcanoylamino-C₁-C₇-alkyle, C₁-C₇-alkyl-O-CO-NH-C₁-C₇-alkyle, C₁-C₇-alkylsulfonylamino-C₁-C₇-alkyle, C₁-C₇-alkyl-NH-CO-NH-C₁-C₇-alkyle, C₁-C₇-alkyl-NH-SO₂-NH-C₁-C₇-alkyle, C₁-C₇-alcoxy, hydroxy-C₁-C₇-alcoxy, C₁-C₇-alcoxy-C₁-C₇alcoxy, C₁-C₇-alcanoyloxy, mono- ou di-(C₁-C₇-alkyl)-amino, mono- di- (naphtyl- ou phényl-C₁-C₇-alkyl)-amino, N-mono-C₁-C₇-alcoxy-C₁-C₇-alkylamino, C₁-C₇-alcanoylamino, C₁-C₇-alkylsulfonylamino, C₁-C₇-alcoxycarbonyle, hydroxy-C₁-C₇-alcoxycarbonyle, C₁-C₇-alcoxy-C₁-C₇-alcoxycarbonyle, amino-C₁-C₇-alcoxycarbonyle, (N-) mono (C₁-C₇-alkyl)-amino-C₁-C₇-alcoxycarbonyle, C₁-C₇-alcanoylamino-C₁-C₇-alcoxycarbonyle, N-mono- ou N,N-di-(C₁-C₇-alkyl)-aminocarbonyle, N-C₁-C₇-alcoxy-C₁-C₇-alkylcarbamoyle ou N-mono- ou N,N-di-(C₁-C₇-alkyl)-aminosulfonyle ; ou
parmi phényl- ou naphtyl-C₁-C₇-alkyle, halogéno-C₁-C₇-alkyle, halogéno, hydroxy, phényl- ou naphtyloxy, phényl- ou naphtyl-C₁-C₇-alkyloxy, nitro, amino, amino-C₁-C₇-alkyle, carboxyle, halogéno-C₁-C₇-alcoxycarbonyle, phényl- ou naphtyl-C₁-C₇-alcoxycarbonyle, C₁-C₇-alkylsulfonyle, carbamoyle et cyano ;
R² est C₁-C₇-alkyle qui est non substitué ou substitué par un ou plusieurs, par exemple jusqu'à trois, substituants choisis parmi le groupe constitué par halogéno, phényl- ou naphtyle, hydroxy, C₁-C₇-alcoxy, amino, mono- ou di-(C₁-C₇-alkyl)-amino, C₁-C₇-alcanoylamino, C₁-C₇-alkyl-sulfonylamino, phényl- ou napthylsulfonylamino, phényl- ou naphtyl-C₁-C₇-alkylsulfonylamino, C₁-C₇-alcoxy-C₁-C₇-alcoxy, hydroxy-C₁-C₇-alcoxy, phényl- ou naphtyloxy, phényl- ou naphtyl-C₁-C₇-alkyloxy, C₁-C₇-alcanoyloxy, nitro, carboxyle, C₁-C₇-alcoxycarbonyle, phényl- ou naphtyl-C₁-C₇-alcoxycarbonyle, carbamoyle, N-mono- ou N,N-di-(C₁-C₇-alkyl-, phényl-, naphtyl-, phényl-C₁-C₇-alkyl- ou naphtyl-C₁-C₇-alkyl-)carbamoyle et N-mono- ou N,N-di-(C₁-C₇-alkyl-, phényl-, naphtyl-, phényl-C₁-C₇-alkyl- ou naphtyl-C₁-C₇-alkyl-)sulfamoyle ; et cyano ;
R³ est phényle ou naphtyle, où chaque phényle ou naphtyle est non substitué ou substitué par un ou plusieurs, par exemple jusqu'à trois, substituants choisis parmi le groupe constitué par C₁-C₇-alkyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, C₁-C₇-alkyl-sulfonylylamino-C₁-C₇-alkyle, phényle, naphtyle, mono- ou di- (C₁-C₇-alcoxy)-phényle ou -naphtyle, C₁-C₇-alkylènedioxy-phényle où les atomes d'oxy sont liés à des atomes de cycle phényle adjacents, halogéno, hydroxy, C₁-C₇-alcoxy, halogéno-C₁-C₇-alcoxy, hydroxy-C₁-C₇-alcoxy, C₁-C₇-alcoxy-C₁-C₇-alcoxy, phényl- ou naphtyloxy, phényl- ou naphtyl(non substitué ou mono-, di- ou tri-C₁-C₇-alkyle substitué)-C₁-C₇-alkyloxy, C₁-C₇-alcanoyloxy, nitro, amino, mono- ou di(C₁-C₇-alkyl)-amino, C₁-C₇-alcanoylamino C₁-C₇-alkylsulfonylylamino, carboxyle, C₁-C₇-alcoxycarbonyle, phényl- ou naphtyl-C₁-C₇-alcoxycarbonyle, carbamoyle et cyano, C₁-C₇-alkylsulfonyle, C₁-C₇-alkylène qui est non substitué ou substitué par jusqu'à quatre substituants C₁-C₇-alkyle et lié à deux atomes de cycle adjacents du motif phényle ou naphtyle ; pyrrolyle, furanyle et thiényle ;
R⁴ est hydrogène ;
L est une liaison ; et
T est carbonyle, thiocarbonyle ou préférablement méthylène ;
ou un sel pharmaceutiquement acceptable de celui-ci.

7. Composé de formule I selon l'une quelconque des revendications 1 ou 2, dans laquelle
R¹ est phénylméthyle ou naphtylméthyle, où chaque phényle ou naphtyle est non substitué ou substitué par un ou plusieurs, par exemple jusqu'à trois, substituants choisis parmi le groupe constitué par C₁-C₇-alkyle, phényle, naphtyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, C₁-C₇-alkyl-sulfonylamino-C₁-C₇-alkyle, halogéno, hydroxy, C₁-C₇-alcoxy, hydroxy-C₁-C₇-alcoxy, C₁-C₇-alcoxy-C₁-C₇-alcoxy, phényl- ou naphtyloxy, phényl- ou naphtyl-C₁-C₇-alkyloxy, C₁-C₇-alcanoyloxy, nitro, amino, mono- ou di (C₁-C₇-alkyl)-amino, C₁-C₇-alcanoylamino, amino-C₁-C₇-alkyle, mono- ou di-(C₁-C₇-alkyl)-amino-C₁-C₇-alkyle, C₁-C₇-alcanoylamino-C₁-C₇-alkyle, carboxyle, C₁-C₇-alcoxycarbonyle, phényl- ou naphtyl-C₁-C₇-alcoxycarbonyle, carbamoyle, N-mono- ou N,N-di-(C₁-C₇-alkyle et/ou (phényl- ou naphtyl)-C₁-C₇-alkyl)-carbamoyle, C₁-C₇-alkylsulfonyle, phényl- ou naphtylsulfonyle non substitué ou C₁-C₇-alkyl-substitué, N-mono- ou N, N-di- (C₁-C₇-alkyle et/ou (phényl- ou naphtyl)-C₁-C₇-alkyl)-sulfamoyle et cyano ;
R² est phényle qui est non substitué ou substitué par un ou plusieurs, par exemple jusqu'à trois, substituants choisis parmi le groupe constitué par C₁-C₇-alkyle, phényle, naphtyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di-(C₁-C₇-alkyl)-amino-C₁-C₇-alkyle, C₁-C₇-alcanoylamino-C₁-C₇-alkyle, C₁-C₇-alkyl-sulfonylamino-C₁-C₇-alkyle, halogéno, hydroxy, C₁-C₇-alcoxy, hydroxy-C₁-C₇-alcoxy, C₁-C₇-alcoxy-C₁-C₇-alcoxy, phényl- ou naphtyloxy, phényl- ou naphtyl-C₁-C₇-alkyloxy, C₁-C₇-alcanoyloxy, nitro, amino, mono- ou di-(C₁-C₇-alkyl)-amino, C₁-C₇-alcanoylamino, amino-C₁-C₇-alkyle, mono- ou di-(C₁-C₇-alkyl)-amino-C₁-C₇-alkyle, C₁-C₇-alcanoylamino-C₁-C₇-alkyle, carboxyle, C₁-C₇-alcoxycarbonyle, phényl- ou naphtyl-C₁-C₇-alcoxycarbonyle, carbamoyle, N-mono- ou N,N-di-(C₁-C₇-alkyle et/ou (phényl- ou naphtyl)-C₁-C₇-alkyl)-carbamoyle, C₁-C₇-alkylsulfonyle, phényl- ou naphtylsulfonyle non substitué ou C₁-C₇-alkyl-substitué, N-mono- ou N, N-di- (C₁-C₇-alkyle et/ou (phényl- ou naphtyl)-C₁-C₇-alkyl)-sulfamoyle et cyano ;
R³ est phényle, naphtyle, phényl-C₁-C₇-alkyle ou naphtyl-C₁-C₇-alkyle, où chaque phényle ou naphtyle est non substitué ou substitué par un ou plusieurs, par exemple jusqu'à trois, substituants choisis parmi le groupe constitué par C₁-C₇-alkyle, phényl- ou naphtyl-C₁-C₇-alkyle, halogéno-C₁-C₇-alkyle, hydroxy-C₁-C₇-alkyle, C₁-C₇-alcoxy-C₁-C₇-alkyle, amino-C₁-C₇-alkyle, mono- ou di(C₁-C₇-alkyl)-amino-C₁-C₇-alkyle, C₁-C₇-alcanoylamino-C₁-C₇-alkyle, C₁-C₇-alkyl-sulfonylylamino-C₁-C₇-alkyle, phényle, naphtyle, mono- ou di-(C₁-C₇-alcoxy)phényle ou -naphtyle, C₁-C₇-alkylènedioxy-phényle où les atomes d'oxy sont liés à des atomes de cycle phényle adjacents, halogéno, hydroxy, C₁-C₇-alcoxy, halogéno-C₁-C₇-alcoxy, hydroxy-C₁-C₇-alcoxy, C₁-C₇-alcoxy-C₁-C₇-alcoxy, phényl- ou naphtyloxy, phényl- ou naphtyl(non substitué ou mono-, di- ou tri-C₁-C₇-alkyle substitué)-C₁-C₇-alkyloxy, C₁-C₇-alcanoyloxy, nitro, amino, mono- ou di-(C₁-C₇-alkyl)-amino, C₁-C₇-alcanoylamino C₁-C₇-alkylsulfonylylamino, carboxyle, C₁-C₇-alcoxycarbonyle, phényl- ou naphtyl-C₁-C₇-alcoxycarbonyle, carbamoyle et cyano, C₁-C₇-alkylsulfonyle, C₁-C₇-alkylène qui est non substitué ou substitué par jusqu'à quatre substituants C₁-C₇-alkyle et lié à deux atomes de cycle adjacents du motif phényle ou naphtyle ; pyrrolyle, furanyle et thiényle ; phényl- ou naphtyl-sulfonyle ou phényl-C₁-C₇-alkyl- ou naphtyl-C₁-C₇-alkyl-sulfonyle, où chaque phényle ou naphtyle est non substitué ou substitué par un ou plusieurs, par exemple jusqu'à trois, substituants choisis parmi le groupe venant d'être décrit pour phényle ou naphtyle substitué R³ ;
C₃-C₁₀-cycloalkyle ou C₃-C₁₀-cycloalkyl-C₁-C₇-alkyle, où dans les deux, cycloalkyle est non substitué ou substitué par un ou plusieurs parmi les substituants venant d'être mentionnés pour phényle ou naphtyle substitué R³, notamment par C₁-C₇-alkyle, phényle, naphtyle, phényl-C₁-C₇-alkyle ou naphtyl-C₁-C₇-alkyle ; ou hétérocyclyle ou hétérocyclyl-C₁-C₇-alkyle où hétérocyclyle est choisi parmi pyrrolyle, furanyle, thiényle, pyrimidine-2,4-dione-1-, -3- ou -5-yle, indolyle, benzofuranyle, benzimidazolyle, benzopyrazolyle, quinoléinyle, isoquinoléinyle, méthylène-dioxy-phényle, éthylène-1,2-dioxy-phényle ou triméthylène-1,3-dioxyphényle où les groupements oxy sont liés à des atomes de cycle adjacents du cycle phényle, où chacun des motifs hétérocyclyle est non substitué ou substitué tel que mentionné ci-dessus pour phényle substitué R³ ;
R⁴ est hydrogène ;
L est méthylène ; et
T est méthylène ;
ou un sel pharmaceutiquement acceptable de celui-ci.

8. Composé de formule I selon l'une quelconque des revendications 1 à 7, répondant à la formule IA, 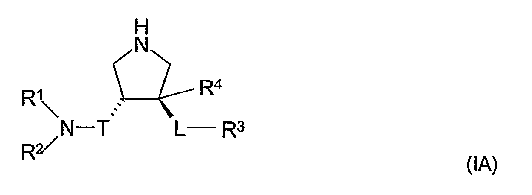 dans laquelle
R¹, R², R³, R⁴, T et L sont tels que définis selon l'une quelconque des revendications précédentes,
ou un sel pharmaceutiquement acceptable de celui-ci.

9. Composé de formule I selon l'une quelconque des revendications 1 à 7, répondant à la formule IB, 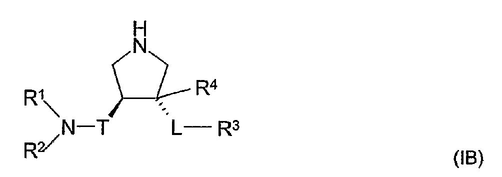 la formule IC, 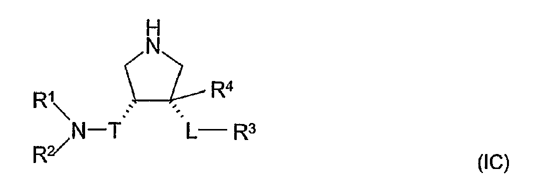 ou la formule ID, 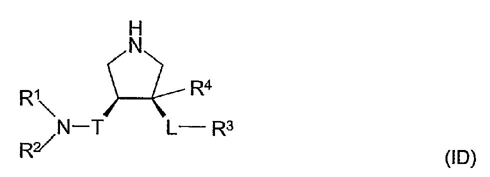 dans lesquelles
R¹, R², R³, R⁴, T et L sont tels que définis selon l'une quelconque des revendications précédentes,
ou un sel pharmaceutiquement acceptable de celui-ci.

10. Composé de formule I selon l'une quelconque des revendications précédentes, dans laquelle
R¹ est aryle non substitué ou substitué, aryl-alkyle non substitué ou substitué, ou acyle ;
R² est alkyle non substitué ou substitué, aryle non substitué ou substitué, cycloalkyle non substitué ou substitué, aryl-alkyle non substitué ou substitué, hétérocyclyl-alkyle mono- ou bicyclique non substitué ou substitué, cycloalkyl-alkyle non substitué ou substitué, à condition que si L est méthylène (-CH₂-), oxy (-O-), thio (-S-) ou imino (-NH-) non substitué ou substitué, R² est choisi parmi l'un des groupements mentionnés et parmi hydrogène ;
R³ est alkyle non substitué ou substitué, aryle non substitué ou substitué, hétérocyclyle non substitué ou substitué, cycloalkyle non substitué ou substitué, aryl-alkyle non substitué ou substitué, hétérocyclyl-alkyle non substitué ou substitué, cycloalkyl-alkyle non substitué ou substitué ou, si L est oxy ou imino non substitué ou substitué, possède l'une des significations venant d'être mentionnée ou est alkylcarbonyle non substitué ou substitué, arylcarbonyle non substitué ou substitué, hétérocyclylcarbonyle non substitué ou substitué, cycloalkylcarbonyle non substitué ou substitué, arylalkylcarbonyle non substitué ou substitué, hétérocyclyl-alkylcarbonyle non substitué ou substitué, cycloalkyl-alkylcarbonyle non substitué ou substitué, carboxy éthérifié, carbamoyle ou amino-carbonyle N-mono- ou N,N-di-substitué ; alkylsulfonyle non substitué ou substitué, arylsulfonyle non substitué ou substitué ; aryl-alkyle sulfonyle non substitué ou substitué, amino-sulfonyle N-mono- ou N,N-di-substitué ;
R⁴ est hydrogène ou hydroxy ;
L est une liaison, méthylène (-CH₂-), oxy (-O-), ou imino (-NH-) non substitué ou substitué, à condition que si L est une liaison alors R³ est l'un des motifs mentionnés pour R³ autre que alkyle substitué ;
ou R³ et R⁴ qui est alors -O- conjointement avec L qui est alors méthylène et le carbone auquel R³-L- et R⁴ sont liés forment un cycle non substitué ou substitué annelé à un aryle non substitué ou substitué, formant ainsi un composé spiro de formule I, ou
R³ et R⁴ conjointement avec L forment oxo (=O) ; et
T est méthylène ;
ou un sel de celui-ci.

11. Composé de formule I selon l'une quelconque des revendications précédentes, dans laquelle
L est méthylène ; et
R³ possède l'une des définitions suivantes (a) ou (b) :
(a) R³ est aryle non substitué ou substitué tel que défini ci-dessus,
(b) R³ est alkyle, qui est éventuellement substitué par un ou deux substituants choisis parmi le groupe constitué par O-C₁-C₄-alkyle, halogéno, hydroxy, phényle non substitué ou substitué, naphtyle non substitué ou substitué, phényl- ou naphtyloxy non substitué ou substitué, phényl- ou naphtyl-C₁-C₇-alkyloxy non substitué ou substitué, cycloalkyle non substitué ou substitué, nitro, amino, amino-C₁-C₇-alkyle, aminocarbonyle N-mono- ou N,N-di-substitué, carboxyle, et cyano.

12. Composé de formule I selon l'une quelconque des revendications précédentes, dans laquelle
L est O ; et
R³ est l'un parmi (a) à (f) suivants :
(a) aryle non substitué ou substitué où des substituants convenables sont choisis parmi le groupe constitué par C₁-C₇-alkyle, -O-C₁-C₇-alkyle, halogéno-C₁-C₇-alkyle, halogéno, hydroxy, phényl- ou naphtyloxy non substitué ou substitué, phényl- ou naphtyl-C₁-C₇-alkyloxy non substitué ou substitué, nitro, amino, amino-C₁-C₇-alkyle, carboxyle, et cyano ;
(b) arylalkyle non substitué ou substitué où des substituants convenables sont choisis parmi le groupe constitué par C₁-C₇-alkyle, -O-C₁-C₇-alkyle, halogéno-C₁-C₇-alkyle, halogéno-C₁-C₇-alkyl-O-, halogéno, hydroxy, phényle non substitué ou substitué, naphtyle non substitué ou substitué, phényl- ou naphtyloxy non substitué ou substitué, phényl- ou naphtyl-C₁-C₇-alkyloxy non substitué ou substitué, hétérocyclyle non substitué ou substitué, nitro, amino, amino-C₁-C₇-alkyle, carboxyle, et cyano, ce par quoi si phényle, naphtyle, phényl- ou naphtyloxy, phényl- ou naphtyl-C₁-C₇-alkyloxy, hétérocyclyle sont substitués, ils sont préférablement mono- ou di-substitués par C₁-C₇-alkyle, -O-C₁-C₇-alkyle, halogéno-C₁-C₇-alkyle, halogéno, hydroxy, amino, amino-C₁-C₇-alkyle, acylamino, hétérocyclyle, ou cyano ;
(c) hétérocyclyl-alkyle non substitué ou substitué, où des substituants convenables sont choisis parmi le groupe constitué par halogéno, hydroxy, phényle non substitué ou substitué, phényl- ou naphtyloxy non substitué ou substitué, phényl- ou naphtyl-C₁-C₇-alkyloxy non substitué ou substitué, nitro, amino, amino-C₁-C₇-alkyle, carboxyle, et cyano ;
(d) alkyle non substitué ou substitué, où des substituants convenables sont choisis parmi le groupe constitué par O-C₁-C₄-alkyle, halogéno, hydroxy, phényle non substitué ou substitué, naphtyle non substitué ou substitué, phényl- ou naphtyloxy non substitué ou substitué, phényl- ou naphtyl-C₁-C₇-alkyloxy non substitué ou substitué, cycloalkyle non substitué ou substitué, nitro, amino, amino-C₁-C₇-alkyle, N-mono- ou N,N-di-substitué aminocarbonyle, carboxyle, et cyano ;
(e) aminocarbonyle non substitué ou substitué qui est mono- ou di-substitué au niveau de l'azote par un ou plusieurs motifs choisis parmi alkyle non substitué ou substitué, aryle non substitué ou substitué, ou cycloalkyle non substitué ou substitué, où des substituants alkyle sont choisis parmi
(i) aryle, qui peut être non substitué ou en outre substitué par O-C₁-C₄-alkyle, halogéno, hydroxy, hétérocyclyle non substitué ou substitué, nitro, amino, acylamino, amino-C₁-C₇-alkyle, carboxyle, et cyano ;
(ii) hétérocyclyle, tel que des cycles à 5 ou 6 chaînons contenant éventuellement un atome d'azote ou d'oxygène, qui peut être non substitué ou en outre substitué par C₁-C₇-alkyle, halogéno, hydroxy, nitro, amino non substitué ou substitué, amino-C₁-C₇-alkyle non substitué ou substitué, carboxyle, et cyano ;
(iii) halogéno, hydroxy, nitro, amino, amino-C₁-C₇-alkyle, carboxyle, et cyano ;
(f) hétérocyclylcarbonyle non substitué ou substitué, où des substituants convenables sont choisis parmi le groupe constitué par C₁-C₇-alkyle, O-C₁-C₇-alkyle, halogéno-C₁-C₇-alkyle, halogéno, hydroxy, phényle ou naphtyle non substitué ou substitué, phényl- ou naphtyloxy non substitué ou substitué, phényl- ou naphtyl-C₁-C₇-alkyloxy non substitué ou substitué, nitro, amino, amino-C₁-C₇-alkyle, carboxyle, et cyano.

13. Composé de formule I selon l'une quelconque des revendications précédentes, dans laquelle
L est NH ou NH substitué, où NH substitué signifie substitué par cycloalkyle alkyle, alkyle ou par alkyle substitué par aminocarbonyle N-mono- ou N,N-di-substitué ; et
R³ est l'un parmi (a) à (m) suivants :
(a) aryl-alkyle non substitué ou substitué, où des substituants convenables sont choisis parmi le groupe constitué par -(C₀-C₇-alkylène)-(X)ᵣ-(C₁-C₇-alkylène)-(Y)ₛ-(C₀-C₇-alkylène)-H, où r et s valent 0 ou 1 et Y et X sont indépendamment O, NH ou -NH-CO-O-, -CO-NH-, NHCO, N(C₁-C₇-alkyl), halogéno-C₁-C₇-alkyle, halogéno-C₁-C₇-alkyl-O-, halogéno, hydroxy, phényl- ou naphtyloxy non substitué ou substitué, phényl- ou naphtyl-C₁-C₇-alkyloxy non substitué ou substitué, nitro, amino, amino non substitué ou substitué par N(mono ou di-CO-C₁-C₇-alkyle ou formyle), amino-C₁-C₇-alkyle non substitué ou substitué, où le motif amino peut être substitué par -C₁-C₇-alkyle, cycloalkyle, phényle ou hétérocyclyle ; carboxyle, et cyano ;
(b) hétérocyclyl-alkyle non substitué ou substitué ou hétérocyclyle non substitué ou substitué où le motif hétérocyclyle est un cycle de 5 chaînons contenant un atome d'azote ; ou un noyau bicyclique contenant un atome d'azote ou d'oxygène, où des substituants convenables sont choisis parmi le groupe constitué par C₁-C₇-alkyle, halogéno, hydroxy, phényle non substitué ou substitué, phényl- ou naphtyloxy non substitué ou substitué, phényl- ou naphtyl-C₁-C₇-alkyle non substitué ou substitué, nitro, amino, amino-C₁-C₇-alkyle, carboxyle, et cyano ;
(c) cycloalkyle non substitué ou substitué où des substituants convenables sont choisis parmi le groupe constitué par O-C₁-C₄-alkyle, halogéno, hydroxy, phényle non substitué ou substitué, naphtyle, phényl- ou naphtyloxy non substitué ou substitué, phényl- ou naphtyl-C₁-C₇-alkyloxy non substitué ou substitué, nitro, amino, amino-C₁-C₇-alkyle, carboxyle, et cyano ;
(d) cycloalkyl-alkyle non substitué ou substitué où des substituants sont choisis parmi le groupe constitué par O-C₁-C₄-alkyle, halogéno, hydroxy, phényle non substitué ou substitué, naphtyle, phényl- ou naphtyloxy non substitué ou substitué, phényl- ou naphtyl-C₁-C₇-alkyloxy non substitué ou substitué, nitro, amino non substitué ou substitué, amino-C₁-C₇-alkyle non substitué ou substitué, où le motif amino peut être substitué par -C₁-C₇-alkyle, cycloalkyle, phényle ou hétérocyclyle ; carboxyle, et cyano ;
(e) alkyle non substitué ou substitué où des substituants sont choisis parmi le groupe constitué par O-C₁-C₄-alkyle, halogéno, hydroxy, phényle non substitué ou substitué, naphtyle non substitué ou substitué, phényl- ou naphtyloxy non substitué ou substitué, phényl- ou naphtyl-C₁-C₇-alkyloxy non substitué ou substitué, cycloalkyle non substitué ou substitué, nitro, amino, amino-C₁-C₇-alkyle, aminocarbonyle N-mono- ou N,N-di-substitué, carboxyle, et cyano ;
(f) arylcarbonyle non substitué ou substitué, où des substituants sont choisis parmi le groupe constitué par -(C₀-C₇-alkylène)-(X)ᵣ-(C₁-C₇-alkylène)-(Y)ₛ-(C₀-C₇-alkylène)-H, où r et s valent 0 ou 1 et Y et X sont indépendamment O, NH ou -NH-CO-O-, -CO-NH-, NHCO, N(C₁-C₇-alkyl), halogéno-C₁-C₇-alkyle, halogéno, hydroxy, phényl- ou naphtyloxy non substitué ou substitué, phényl- ou naphtyl-C₁-C₇-alkyloxy non substitué ou substitué, nitro, amino non substitué ou substitué, N(mono ou di-CO-C₁-C₇-alkyle ou formyle)amino, amino-C₁-C₇-alkyle, carboxyle, et cyano ;
(g) alkylcarbonyle non substitué ou substitué où des substituants sont choisis parmi le groupe constitué par O-C₁-C₄-alkyle, halogéno, hydroxy, phényle ou naphtyle non substitué ou substitué, C₃-C₇-cycloalkyle non substitué ou substitué, ou hétérocyclyle substitué, nitro, amino, amino-C₁-C₇-alkyle, carboxyle, et cyano ;
(h) cycloalkyl-carbonyle non substitué ou substitué où des substituants convenables sont choisis parmi le groupe constitué par C₁-C₇-alkyle, O-C₁-C₄-alkyle, halogéno, hydroxy, phényle non substitué ou substitué, naphtyle, phényl- ou naphtyloxy non substitué ou substitué, phényl- ou naphtyl-C₁-C₇-alkyloxy non substitué ou substitué, nitro, amino, amino-C₁-C₇-alkyle, carboxyle, et cyano ;
(i) hétérocyclyl-carbonyle non substitué ou substitué où le motif hétérocyclyle comporte des cycles à 6 chaînons contenant éventuellement un atome d'oxygène ; et des noyaux bicycliques, où des substituants convenables sont choisis parmi le groupe constitué par halogéno, hydroxy, phényle non substitué ou substitué, phényl- ou naphtyloxy non substitué ou substitué, phényl- ou naphtyl-C₁-C₇-alkyle non substitué ou substitué, nitro, amino, amino-C₁-C₇-alkyle, carboxyle, et cyano ;
(j) carboxy éthérifié non substitué ou substitué auquel l'un des groupements suivants est lié :
(i) O-alkyle, qui est éventuellement substitué, où des substituants convenables sont choisis parmi le groupe constitué par O-C₁-C₄-alkyle, halogéno, hydroxy, phényle non substitué ou substitué, naphtyle non substitué ou substitué, phényl- ou naphtyloxy non substitué ou substitué, phényl- ou naphtyl-C₁-C₇-alkyloxy non substitué ou substitué, cycloalkyle non substitué ou substitué, nitro, amino, amino-C₁-C₇-alkyle, aminocarbonyle N-mono- ou N,N-di-substitué ;
(ii) O-aryle, qui peut être substitué, où des substituants convenables sont choisis parmi le groupe constitué par C₁-C₇-alkyle, O-C₁-C₄-alkyle, halogéno-C₁-C₇-alkyle, halogéno, hydroxy, amino non substitué ou substitué, acylamino, amino-C₁-C₇-alkyle non substitué ou substitué, carboxyle, et cyano ;
(iii) O-cycloalkyle, qui peut être substitué, où des substituants convenables sont choisis parmi le groupe constitué par C₁-C₇-alkyle, O-C₁-C₄-alkyle, halogéno, hydroxy, nitro, amino non substitué ou substitué, amino-C₁-C₇-alkyle non substitué ou substitué, carboxyle, et cyano ;
(k) aminocarbonyle non substitué ou substitué qui est mono- ou di-substitué au niveau de l'azote par un ou plusieurs motifs choisis parmi alkyle non substitué ou substitué, aryle non substitué ou substitué, ou cycloalkyle non substitué ou substitué, où des substituants convenables pour le motif alkyle sont aryle, et où aryle peut être non substitué ou en outre substitué par C₁-C₇-alkyle, O-C₁-C₄-alkyle, halogéno-C₁-C₇-alkyle, halogéno, hydroxy, nitro, amino, acylamino, amino-C₁-C₇-alkyle, carboxyle, et cyano ;
(l) arylsulfonyle non substitué ou substitué où des substituants sont choisis parmi le groupe constitué par C₁-C₇-alkyle, O-C₁-C₄-alkyle, halogéno-C₁-C₇-alkyle, halogéno, hydroxy, phényle non substitué ou substitué, phényl- ou naphtyloxy non substitué ou substitué, phényl- ou naphtyl-C₁-C₇-alkyle non substitué ou substitué, amino, acylamino, amino-C₁-C₇-alkyle, carboxyle, et cyano ;
(m) alkylsulfonyle non substitué ou substitué, où des substituants sont choisis parmi le groupe constitué par O-C₁-C₄-alkyle, halogéno, hydroxy, phényle ou naphtyle non substitué ou substitué, phényl- ou naphtyloxy non substitué ou substitué, phényl- ou naphtyl-C₁-C₇-alkyle non substitué ou substitué, C₃-C₇-cycloalkyle non substitué ou substitué, ou hétérocyclyle non substitué ou substitué, nitro, amino, amino-C₁-C₇-alkyle, carboxyle, et cyano.

14. Composé de formule I selon la revendication 1, choisi parmi le groupe constitué par la ((3S*,4S*)-4-benzylpyrrolidin-3-ylméthyl)-(4-chlorophényl)phénylamine ; la ((3R*,4R*)-4-benzylpyrrolidin-3-ylméthyl)-diphénylamine ; le 3-[((3R*,4R*)-4-benzylpyrrolidin-3-ylméthyl)phénylamino]phénol ; la ((3S*,4S*)-4-benzyl-pyrrolidin-3-ylméthyl)-phénéthylphénylamine ; la (3R*,4R*)-benzyl-(4-chlorophényl)-[4-(3-isopropyl-benzyl)pyrrolidin-3-ylméthyl]amine ; la (3R*,4R*)-(4-chlorophényl)-[4-(3-isopropylbenzyl)pyrrolidin-3-ylméthyl]phénylamine ; le N-((3S*,4R*)-4-benzylamino-pyrrolidin-3-ylméthyl)-N-isopropyl-4-méthoxy-3-(3-méthoxypropoxy)benzamide ; la ((3R*,4R*)-4-benzyl-pyrrolidin-3-ylméthyl)-(4-chlorophényl)cyclohexylamine ; la ((3R*,4R*)-4-benzylpyrrolidin-3-ylméthyl)-(4-chlorophényl)-((R)-1-phényléthyl)amine ; la ((3R*,4R*)-4-benzylpyrrolidin-3-ylméthyl)-(4-chloro-3-méthoxyphényl)phénylamine ; le 3-{[((3R*,4R*)-4-benzylpyrrolidin-3-ylméthyl)-(4-chlorophényl)amino]-méthyl}benzonitrile ; la benzyl((3R*,4R*)-4-benzylpyrrolidin-3-ylméthyl)-(4-chlorophényl)amine ; la benzyl-((3R,4R)-4-benzylpyrrolidin-3-ylméthyl)-(4-chlorophényl)amine ; la benzyl-((3S,4S)-4-benzyl-pyrrolidin-3-ylméthyl)-(4-chlorophényl)amine ; la ((3R*,4R*)-4-benzylpyrrolidin-3-ylméthyl)-(4-chloro-phényl)(2-méthoxybenzyl)amine ; la ((3R*,4R*)-4-benzylpyrrolidin-3-ylméthyl)-(4-chlorophényl)-(3-méthoxybenzyl)amine ; la benzyl-((3R*,4R*)-4-benzyl-pyrrolidin-3-ylméthyl)-(4-méthoxyphényl)amine ; la benzyl-((3R*,4R*)-4-benzylpyrrolidin-3-ylméthyl)-p-tolylamine ; la benzyl-((3R*,4R*)-4-benzylpyrrolidin-3-ylméthyl)-(4-fluorophényl)amine ; la ((3R*,4R*)-4-benzylpyrrolidin-3-ylméthyl)-(4-chlorophényl)naphtalén-1-ylméthylamine ; le 3-{[((3R*,4R*)-4-benzylpyrrolidin-3-ylméthyl)-(4-chlorophényl)amino]méthyl}benzamide ; la ((3R*,4R*)-4-benzylpyrrolidin-3-ylméthyl)-(4-chloro-phényl)naphtalén-2-ylméthylamine ; la ((3R*,4R*)-4-benzylpyrrolidin-3-ylméthyl)-(4-chlorophényl)-(4-propoxy-2-trifluorométhylquinoléin-6-ylméthyl)amine ; le 7-{[((3R*,4R*)-4-benzylpyrrolidin-3-ylméthyl)-(4-chlorophényl)amino]méthyl}naphtalène-2-carbonitrile ; le 7-{[((3R*,4R*)-4-benzylpyrrolidin-3-ylméthyl)-(4-chlorophényl)amino]méthyl}naphtalène-1-carbonitrile ; le 6-{[((3R*,4R*)-4-benzylpyrrolidin-3-ylméthyl)-(4-chlorophényl)amino]méthyl}naphtalène-2-carbonitrile ; la 6-{[((3R*,4R*)-4-benzylpyrrolidin-3-ylméthyl)-(4-chlorophényl)amino]méthyl}-1H-pyrimidine-2,4-dione ; le 5-{[((3R*,4R*)-4-benzylpyrrolidin-3-ylméthyl)-(4-chlorophényl)amino]méthyl}naphtalène-2-carbonitrile ; le 5-[((3R*,4R*)-4-benzylpyrrolidin-3-ylméthyl)-(4-chlorophényl)amino]méthyl)naphtalène-1-carbonitrile ; le 4-{[((3R*,4R*)-4-benzylpyrrolidin-3-ylméthyl)-(4-chlorophényl)amino]méthyl}naphtalène-1-carbonitrile ; le 4-{[((3R*,4R*)-4-benzylpyrrolidin-3-ylméthyl)-(4-chlorophényl)amino]méthyl}benzonitrile ; le 5-{[((3R*,4R*)-4-benzylpyrrolidin-3-ylméthyl)-(4-chloro-phényl)amino]méthyl}-2-fluorobenzonitrile ; le 4-[((3R*,4R*)-4-benzylpyrrolidin-3-ylméthyl)naphtalén-2-ylméthylamino]benzonitrile ; le 4-{[((3R*,4R*)-4-benzylpyrrolidin-3-ylméthyl)-(4-chlorobenzyl)-amino]méthyl}benzonitrile ; le N-((3R*,4R*)-4-benzylpyrrolidin-3-ylméthyl)-N-(3-cyanophényl)-4-méthoxy-3-(3-méthoxypropoxy)benzamide ; le chlorhydrate de N-((3R*,4R*)-4-benzylpyrrolidin-3-ylméthyl)-3,N-diphénylpropionamide ; l'acide (1R*,2R*)-2-phényl-cyclopropanecarboxylique ((3R*,4R*)-4-benzylpyrrolidin-3-ylméthyl)phénylamide ; l'acide (1R*,2R*)-2-phényl-cyclopropanecarboxylique ((3R*,4R*)-4-benzylpyrrolidin-3-ylméthyl)-(4-chlorophényl)amide ; l'acide naphtalène-2-carboxylique ((3R*,4R*)-4-benzylpyrrolidin-3-ylméthyl)-(4-chlorophényl)amide ; l'acide naphtalène-1-carboxylique ((3R*,4R*)-4-benzylpyrrolidin-3-ylméthyl)-(4-chlorophényl)amide ; le N-((3R*,4R*)-4-benzyl-pyrrolidin-3-ylméthyl)-N-(4-chlorophényl)-4-méthoxy-3-(3-méthoxypropoxy)benzamide ; le N-((3R*,4R*)-4-benzyl-pyrrolidin-3-ylméthyl)-4-méthoxy-3-(3-méthoxypropoxy)-N-phénylbenzamide ; le N-((3R*,4R*)-4-benzylpyrrolidin-3-ylméthyl)-N-(4-cyanophényl)-4-méthoxy-3-(3-méthoxy-propoxy)benzamide ; l'acide naphtalène-2-carboxylique ((3R*,4R*)-4-benzylpyrrolidin-3-ylméthyl)-(4-cyano-phényl)amide ; l'acide benzofuran-5-carboxylique ((3R*,4R*)-4-benzylpyrrolidin-3-ylméthyl)-(4-cyano-phényl)amide ; l'acide naphtalène-2-carboxylique ((3R*,4R*)-4-benzylpyrrolidin-3-ylméthyl)-(4-phénoxy-phényl)amide ; l'acide benzofuran-5-carboxylique ((3R*,4R*)-4-benzylpyrrolidin-3-ylméthyl)-(4-chlorophényl)amide ; le N-((3R*,4R*)-4-benzyl-pyrrolidin-3-ylméthyl)-N-(4-chlorophényl)-2-phénoxy-benzamide ; le N-((3R*,4R*)-4-benzylpyrrolidin-3-ylméthyl)-N-(4-chlorophényl)benzènesulfonamide ; la ((3R*,4R*)-4-benzylpyrrolidin-3-ylméthyl)-(4-chlorophényl)phénylméthylamine ; le N-((3S*,4S*)-4-benzylpyrrolidin-3-ylméthyl)-N-isopropyl-3-(3-méthoxy-propoxy)-4-méthylbenzamide ; le N-((3S*,4S*)-4-benzylpyrrolidin-3-ylméthyl)-N-isopropyl-4-méthoxy-3-(3-méthoxypropoxy)benzamide ; le N-((3S,4S)-4-benzylpyrrolidin-3-ylméthyl)-N-isopropyl-4-méthoxy-3-(3-méthoxypropoxy)benzamide ; le N-((3R,4R)-4-benzylpyrrolidin-3-ylméthyl)-N-isopropyl-4-méthoxy-3-(3-méthoxypropoxy)benzamide ; l'acide naphtalène-2-carboxylique ((3R*,4R*)-4-benzylpyrrolidin-3-ylméthyl)-isopropylamide ; le N-((3R*,4R*)-4-benzylpyrrolidin-3-ylméthyl)-4-éthyl-N-isopropyl-3-(3-méthoxypropoxy)-benzamide ; le N-((3R*,4R*)-4-benzylpyrrolidin-3-ylméthyl)-N-isopropyl-3-(3-méthoxypropoxy)benzamide ; l'acide 1-(3-méthoxypropyl)-1H-indole-2-carboxylique ((3R*,4R*)-4-benzylpyrrolidin-3-ylméthyl)isopropylamide ; le N-((3R*,4R*)-4-benzylpyrrolidin-3-ylméthyl)-N-cyclopentyl-4-méthoxy-3-(3-méthoxypropoxy)benzamide ; le N-((3R*,4R*)-4-benzylpyrrolidin-3-ylméthyl)-N-cyclopropyl-4-méthoxy-3-(3-méthoxypropoxy)benzamide ;
le N-((3R*,4R*)-4-benzylpyrrolidin-3-ylméthyl)-4-méthoxy-3-(3-méthoxypropoxy)-N-thiophén-2-ylméthyl-benzamide ; le N-((3R*,4R*)-4-benzylpyrrolidin-3-ylméthyl)-4-méthoxy-N-(2-méthoxyéthyl)-3-(3-méthoxy-propoxy)benzamide ; le N-((3R*,4R*)-4-benzylpyrrolidin-3-ylméthyl)-N-(4-chlorobenzyl)-4-méthoxy-3-(3-méthoxy-propoxy)benzamide ; le N-((3R*,4R*)-4-benzylpyrrolidin-3-ylméthyl)-4-méthoxy-3-(3-méthoxypropoxy)-N-méthyl-benzamide ; le N-((3R*,4R*)-4-benzylpyrrolidin-3-ylméthyl)-N-cyclobutyl-4-méthoxy-3-(3-méthoxypropoxy)-benzamide ; le N-((3R*,4R*)-4-benzylpyrrolidin-3-ylméthyl)-N-(1-éthylpropyl)-4-méthoxy-3-(3-méthoxypropoxy)benzamide ; le N-((3R*,4R*)-4-benzyl-pyrrolidin-3-ylméthyl)-N-cyclopropylméthyl-4-méthoxy-3-(3-méthoxypropoxy)benzamide ; le N-((3R*,4R*)-4-benzylpyrrolidin-3-ylméthyl)-4-méthoxy-3-(3-méthoxy-propoxy)-N-(2,2,2-trifluoroéthyl)benzamide ; le N-((3R*,4R*)-4-benzylpyrrolidin-3-ylméthyl)-N-(4-cyanobenzyl)-4-méthoxy-3-(3-méthoxypropoxy)benzamide ; le N-((3R*,4R*)-4-benzylpyrrolidin-3-ylméthyl)-N-(1,2-diméthylpropyl)-4-méthoxy-3-(3-méthoxypropoxy)-benzamide ; le N-((3R*,4R*)-4-benzylpyrrolidin-3-ylméthyl)-N-(4-chlorobenzyl)-2-phénoxybenzamide ; le N-((3R*,4R*)-4-benzylpyrrolidin-3-ylméthyl)-N-(4-chlorobenzyl)-3-phénoxybenzamide ; le N-((3R*,4R*)-4-benzylpyrrolidin-3-ylméthyl)-N-(4-chlorobenzyl)-4-phénoxybenzamide ; la ((3S*,4S*)-4-benzylpyrrolidin-3-ylméthyl)(4-chlorophényl)-[4-méthoxy-3-(3-méthoxy-propoxy)benzyl]amine ; l'acide benzofuran-5-carboxylique ((3R*,4R*)-4-benzylpyrrolidin-3-ylméthyl)-(4-chlorobenzyl)amide ; le N-((3R*,4R*)-4-benzylpyrrolidin-3-ylméthyl)-4-méthoxy-3-(3-méthoxypropoxy)-N-(3-phénylpropyl)benzamide ; le N-((3R*,4R*)-4-benzylpyrrolidin-3-ylméthyl)-4-méthoxy-3-(3-méthoxypropoxy)-N-phénéthylbenzamide ; le N-((3R*,4R*)-4-benzylpyrrolidin-3-ylméthyl)-4-méthoxy-3-(3-méthoxypropoxy)-N-(4-phénylbutyl)benzamide ; l'acide naphtalène-2-carboxylique (4-benzyloxyphényl)-((3S*,4S*)-4-benzylpyrrolidin-3-ylméthyl)amide ; la (3-aminométhylbenzyl)-((3R*,4R*)-4-benzylpyrrolidin-3-ylméthyl)-(4-chlorophényl)amine ; la (8-aminométhylnaphtalén-2-ylméthyl)-((3R*,4R*)-4-benzylpyrrolidin-3-ylméthyl)-(4-chlorophényl)amine ; la (4-aminométhylphényl)benzyl-((3R*,4R*)-4-benzylpyrrolidin-3-ylméthyl)amine ; la (7-aminométhylnaphtalén-2-ylméthyl)-((3R*,4R*)-4-benzylpyrrolidin-3-ylméthyl)-(4-chlorophényl)amine ; la (4-aminométhylnaphtalén-1-ylméthyl)((3R*,4R*)-4-benzylpyrrolidin-3-ylméthyl)-(4-chlorophényl)amine ; le N-(3-{[((3R*,4R*)-4-benzylpyrrolidin-3-ylméthyl)-(4-chlorophényl)amino]méthyl}benzyl)acétamide ; le N-(3{[((3R*,4R*)-4-benzylpyrrolidin-3-ylméthyl)-(4-chlorophényl)amino]méthyl}benzyl)formamide ; la ((3R*,4R*)-4-benzylpyrrolidin-3-ylméthyl)-(4-chlorophényl)-(3-diméthylaminométhylbenzyl)amine ; la ((3R*,4R*)-4-benzylpyrrolidin-3-ylméthyl)-(4-chlorophényl)-(3-aminobenzyl)amine ; la ((3R*4R*)-4-benzylpyrrolidin-3-ylméthyl)isopropyl-[4-méthoxy-3-(3-méthoxypropoxy)benzyl]amine ; le N-((3R*,4R*)-4-benzylpyrrolidin-3-ylméthyl)-N-isopropyl-4-méthoxy-3-(2-méthoxyéthoxy)benzamide ; la ((3R*,4R*)-4-benzylpyrrolidin-3-ylméthyl)-(4-chlorophényl)-[2-(3-méthoxy-propoxy)benzyl]amine ; la ((3R*,4R*)-4-benzylpyrrolidin-3-ylméthyl)-(4-chlorophényl)-[2-(2-méthoxy-éthoxy)benzyl]amine ; le 2-{[((3R*,4R*)-4-benzylpyrrolidin-3-ylméthyl)(4-chlorophényl)amino]méthyl}-phénol ; l'acide 1-(3-méthoxypropyl)-1H-indole-6-carboxylique ((3R*,4R*)-4-benzylpyrrolidin-3-ylméthyl)-isopropylamide ; l'acide 1-(2-méthoxyéthyl)-1H-indole-6-carboxylique ((3R*,4R*)-4-benzylpyrrolidin-3-ylméthyl)isopropylamide ; la benzyl-(4-chlorophényl)-((3R*,4R*)-4-phénéthylpyrrolidin-3-ylméthyl)amine ; la ((3S*,4R*)-4-benzylpyrrolidin-3-ylméthyl)-(4-chlorophényl)phénylamine ; le N-((3S*,4S*)-4-benzyloxypyrrolidin-3-ylméthyl)-N-isopropyl-4-méthoxy-3-(3-méthoxypropoxy)benzamide ; le N-((3S*,4R*)-4-benzyloxypyrrolidin-3-ylméthyl)-N-isopropyl-4-méthoxy-3-(3-méthoxypropoxy)benzamide ; le N-((3S*,4S*)-4-hydroxypyrrolidin-3-ylméthyl)-N-isopropyl-4-méthoxy-3-(3-méthoxypropoxy)benzamide ; le N-((3S*,4R*)-4-hydroxypyrrolidin-3-ylméthyl)-N-isopropyl-4-méthoxy-3-(3-méthoxypropoxy)benzamide ; la benzyl-((3S*,4R*)-4-benzyloxypyrrolidin-3-ylméthyl)-(4-chlorophényl)amine ; le N-isopropyl-4-méthoxy-3-(3-méthoxypropoxy)-N-[(3S*,4S*)-4-(4-trifluorométhylphénoxy)pyrrolidin-3-ylméthyl]benzamide ; le N-isopropyl-4-méthoxy-3-(3-méthoxypropoxy)-N-[(3S*,4R*)-4-(4-trifluorométhylphénoxy)pyrrolidin-3-ylméthyl]benzamide ; la benzyl(4-chlorophényl)-[(3S*,4R*)-4-(4-trifluorométhylphénoxy)-pyrrolidin-3-ylméthyl]amine ; la (3R*,4R*)-(4-chlorophényl)-[4-(3-isopropylphénoxy)pyrrolidin-3-ylméthyl]phénylamine ; la (3R*,4R*)-benzyl-(4-chlorophényl)-[4-(3-isopropylphénoxy)pyrrolidin-3-ylméthyl]amine ; la (3R*,4R*)-(4-chlorophényl)-[4-(3-isopropylphénoxy)pyrrolidin-3-ylméthyl]-(2-méthoxy-benzyl)amine ; la (3R*,4R*)-(4-chlorophényl)-[4-(3-isopropylphénoxy)pyrrolidin-3-ylméthyl]-(3-méthoxy-benzyl)amine ; la (3R*,4R*)-benzo[1,2,5]oxadiazol-5-ylméthyl-(4-chlorophényl)-[4-(3-isopropylphénoxy)-pyrrolidin-3-ylméthyl]amine ; le N-((3S*,4R*)-4-benzyl-4-hydroxypyrrolidin-3-ylméthyl)-N-isopropyl-4-méthoxy-3-(3-méthoxypropoxy)benzamide ; le N-[(3S*,4R*)-4-(2-fluorobenzyl)-4-hydroxypyrrolidin-3-ylméthyl]-N-isopropyl-4-méthoxy-3-(3-méthoxypropoxy)benzamide ; le N-isopropyl-4-méthoxy-3-(3-méthoxypropoxy)-N-((S)-4-oxopyrrolidin-3-ylméthyl)benzamide ; le N-((3R*,4R*)-4-CClohexylaminopyrrolidin-3-ylméthyl)-N-isopropyl-4-méthoxy-3-(3-méthoxypropoxy)benzamide ; le N-isopropyl-N-((3R*,4R*)-4-isopropylaminopyrrolidin-3-ylméthyl)-4-méthoxy-3-(3-méthoxypropoxy)benzamide ; le N-isopropyl-4-méthoxy-3-(3-méthoxypropoxy)-N-[(3R*,4R*)-4-(toluène-4-sulfonylamino)pyrrolidin-3-ylméthyl]benzamide ; le N-isopropyl-4-méthoxy-N-[(3R*,4R*)-4-(4-méthoxybenzènesulfonylamino)pyrrolidin-3-ylméthyl]-3-(3-méthoxypropoxy)benzamide ; le N-isopropyl-4-méthoxy-N-[(3R*,4R*)-4-(3-méthoxybenzènesulfonylamino)pyrrolidin-3-ylméthyl]-3-(3-méthoxypropoxy)benzamide ; le N-isopropyl-4-méthoxy-3-(3-méthoxypropoxy)-N-[(3R*,4R*)-4-(naphtalène-2-sulfonylamino)pyrrolidin-3-ylméthyl]-benzamide ; le N-isopropyl-4-méthoxy-3-(3-méthoxypropoxy)-N-((3R*,4R*)-4-phénylméthanesulfonylaminopyrrolidin-3-ylméthyl)benzamide ; le N-((3R*,4R*)-4-benzylaminopyrrolidin-3-ylméthyl)-N-isopropyl-4-méthoxy-3-(3-méthoxypropoxy)benzamide ; l'ester isopropylique de l'acide [(3R*,4R*)-4-({isopropyl-[4-méthoxy-3-(3-méthoxypropoxy)benzoyl]amino}méthyl)-pyrrolidin-3-yl]carbamique ; le N-[(3S*,4S*)-4-(cyclopropylcarbamoylméthylamino)pyrrolidin-3-ylméthyl]-N-isopropyl-4-méthoxy-3-(3-méthoxypropoxy)-benzamide ; le N-((3R*,4R*)-4-benzylpyrrolidin-3-ylméthyl)-N-isopropyl-4-méthoxy-3-(4-méthoxybutyl)-benzamide ; le N-((3R*,4R*)-4-benzylpyrrolidin-3-ylméthyl)-N-isopropyl-4-méthoxy-3-(3-méthoxypropylamino)benzamide ; l'acide 1-(3-méthoxypropyl)-3-méthyl-1H-indole-6-carboxylique ((3R,4R)-4-benzylpyrrolidin-3-ylméthyl)isopropylamide ; le 3-benzyloxy-N-((3S*,4S*)-4-benzylpyrrolidin-3-ylméthyl)-N-isopropyl-4-méthoxybenzamide ; le N-isopropyl-4-méthoxy-3-(3-méthoxypropoxy)-N-pyrrolidin-3-ylméthylbenzamide ; le N-(2-{[((3R*,4R*)-4-benzylpyrrolidin-3-ylméthyl)-(4-chlorophényl)amino]méthyl}phényl)acétamide ; le N-((3R*,4R*)-4-benzylpyrrolidin-3-ylméthyl)-3-(2-éthoxyéthoxy)-N-isopropyl-4-méthoxybenzamide ; le N-((3S*,4S*)-4-benzylpyrrolidin-3-ylméthyl)-N-isopropyl-4-méthoxy-3-(2-méthoxyéthoxyméthyl)benzamide ; le N-((3R*,4R*)-4-benzylpyrrolidin-3-ylméthyl)-3-(3-hydroxypropoxy)-N-isopropyl-4-méthoxybenzamide ; l'acide 1-(3-méthoxypropyl)-1H-indole-5-carboxylique ((3R*,4R*)-4-benzylpyrrolidin-3-ylméthyl)isopropylamide ; l'acide 1-(2-méthoxy-éthyl)-1H-indole-5-carboxylique ((3R*,4R*)-4-benzylpyrrolidin-3-ylméthyl)isopropylamide ; la (3R*,4R*)-(4-chlorophényl)-[4-(3-isopropylphényl)-pyrrolidin-3-ylméthyl]phénylamine ; la (3R*,4R*)-benzyl-(4-chloro-3-méthoxyphényl)-[4-(3-isopropylphényl)pyrrolidin-3-ylméthyl]amine ; la (3R*,4R*)-(4-chloro-3-méthoxyphényl)-[4-(3-isopropylphényl)-pyrrolidin-3-ylméthyl]phénylamine ; le N-((3R*,4S*)-4-benzylpyrrolidin-3-ylméthyl)-N-isopropyl-4-méthoxy-3-(3-méthoxypropoxy)benzamide ; le N-isopropyl-4-méthoxy-N-[(3S*,4S*)-4-(3-méthoxybenzyloxy)pyrrolidin-3-ylméthyl]-3-(3-méthoxypropoxy)benzamide ; le N-isopropyl-4-méthoxy-3-(3-méthoxypropoxy)-N-[(3S*,4S*)-4-(naphtalén-2-ylméthoxy)pyrrolidin-3-ylméthyl]-benzamide ; le N-[(3S*,4S*)-4-(3,5-diméthoxybenzyloxy)pyrrolidin-3-ylméthyl]-N-isopropyl-4-méthoxy-3-(3-méthoxypropoxy)benzamide ; le N-[(3S*,4S*)-4-(3-éthoxybenzyloxy)pyrrolidin-3-ylméthyl]-N-isopropyl-4-méthoxy-3-(3-méthoxypropoxy)benzamide ; le N-[(3S*,4S*)-4-(3-isopropoxybenzyloxy)pyrrolidin-3-ylméthyl]-N-isopropyl-4-méthoxy-3-(3-méthoxypropoxy)-benzamide ; le N-[(3S*,4S*)-4-(3-cyanobenzyloxy)pyrrolidin-3-ylméthyl]-N-isopropyl-4-méthoxy-3-(3-méthoxypropoxy)benzamide ; le N-isopropyl-4-méthoxy-3-(3-méthoxypropoxy)-N-[(3S,4S)-4-(3-trifluorométhoxybenzyloxy)pyrrolidin-3-ylméthyl]benzamide ; le N-isopropyl-4-méthoxy-3-(3-méthoxypropoxy)-N-[(3S,4S)-4-(3-propoxybenzyloxy)pyrrolidin-3-ylméthyl]benzamide ;
le N-[(3S,4S)-4-(biphényl-3-ylméthoxy)pyrrolidin-3-ylméthyl]-N-isopropyl-4-méthoxy-3-(3-méthoxypropoxy)-benzamide ; le N-isopropyl-4-méthoxy-3-(3-méthoxypropoxy)-N-[(3S,4S)-4-(3-phénoxybenzyloxy)pyrrolidin-3-ylméthyl]benzamide ; le N-[(3S*,4S*)-4-(3,5-diéthoxybenzyloxy)pyrrolidin-3-ylméthyl]-N-isopropyl-4-méthoxy-3-(3-méthoxypropoxy)benzamide ; le N-isopropyl-4-méthoxy-3-(3-méthoxypropoxy)-N-[(3S*,4S*)-4-(5,5,8,8-tétraméthyl-5,6,7,8-tétrahydronaphtalén-2-ylméthoxy)-pyrrolidin-3-ylméthyl]benzamide ; le N-[(3S*,4S*)-4-(3,4-dihydro-2H-benzo[b][1,4]dioxépin-7-ylméthoxy)-pyrrolidin-3-ylméthyl]-N-isopropyl-4-méthoxy-3-(3-méthoxypropoxy)benzamide ; le N-[(3S*,4S*)-4-(7-cyanonaphtalén-2-ylméthoxy)pyrrolidin-3-ylméthyl]-N-isopropyl-4-méthoxy-3-(3-méthoxypropoxy)benzamide ; le N-[(3S*,4S*)-4-(2,3-diméthoxybenzyloxy)pyrrolidin-3-ylméthyl]-N-isopropyl-4-méthoxy-3-(3-méthoxypropoxy)-benzamide ; le N-[(3S*,4S*)-4-(3-benzyloxybenzyloxy)pyrrolidin-3-ylméthyl]-N-isopropyl-4-méthoxy-3-(3-méthoxypropoxy)benzamide ; le N-isopropyl-4-méthoxy-3-(3-méthoxypropoxy)-N-[(3S*,4S*)-4-(3-pyrrol-1-ylbenzyloxy)pyrrolidin-3-ylméthyl]benzamide ; le N-[(3S*,4S*)-4-(benzofuran-5-ylméthoxy)pyrrolidin-3-ylméthyl]-N-isopropyl-4-méthoxy-3-(3-méthoxypropoxy)-benzamide ; le N-[(3S*,4S*)-4-(2,3-dihydrobenzo-[1,4]dioxin-5-ylméthoxy)pyrrolidin-3-ylméthyl]-N-isopropyl-4-méthoxy-3-(3-méthoxypropoxy)benzamide ; le N-[(3S*,4S*)-4-(3,4-diméthylbenzyloxy)pyrrolidin-3-ylméthyl]-N-isopropyl-4-méthoxy-3-(3-méthoxypropoxy)-benzamide ; le N-[(3S*,4S*)-4-(3,4-dihydro-2H-benzo[b]-[1,4]dioxépin-6-ylméthoxy)pyrrolidin-3-ylméthyl]-N-isopropyl-4-méthoxy-3-(3-méthoxypropoxy)benzamide ; le N-isopropyl-4-méthoxy-3-(3-méthoxypropoxy)-N-[(3S*,4S*)-4-(naphtalén-1-ylméthoxy)pyrrolidin-3-ylméthyl]benzamide ; le N-isopropyl-4-méthoxy-N-{(3S,4S)-4-[3-(4-méthoxyphénoxy)benzyloxy]pyrrolidin-3-ylméthyl}-3-(3-méthoxypropoxy)benzamide ; le N-[(3S*,4S*)-4-(3-benzo[1,3]dioxol-5-ylbenzyloxy)-pyrrolidin-3-ylméthyl]-N-isopropyl-4-méthoxy-3-(3-méthoxypropoxy)benzamide ; le N-isopropyl-4-méthoxy-N-[(3S,4S)-4-(2'-méthoxybiphényl-3-ylméthoxy)pyrrolidin-3-ylméthyl]-3-(3-méthoxypropoxy)benzamide ; le N-[4-(2-chloro-6-fluorobenzyl)-4-hydroxypyrrolidin-3-ylméthyl]-N-isopropyl-4-méthoxy-3-(3-méthoxypropoxy)benzamide ;
le N-((3R*,4R*)-4-benzylaminopyrrolidin-3-ylméthyl)-N-isopropyl-4-méthoxy-3-(3-méthoxypropoxy)benzamide ; le N-isopropyl-4-méthoxy-3-(3-méthoxypropoxy)-N-[(3R*,4R*)-4-(1-phényléthylamino)pyrrolidin-3-ylméthyl]benzamide ; le N-((3R*,4R*)-4-isobutylaminopyrrolidin-3-ylméthyl)-N-isopropyl-4-méthoxy-3-(3-méthoxypropoxy)benzamide ; le N-((3R*,4R*)-4-CClobutylaminopyrrolidin-3-ylméthyl)-N-isopropyl-4-méthoxy-3-(3-méthoxypropoxy)benzamide ; le N-((3R*,4R*)-4-cyclopentylaminopyrrolidin-3-ylméthyl)-N-isopropyl-4-méthoxy-3-(3-méthoxypropoxy)benzamide ; le N-[(3R*,4R*)-4-(cyclopentylméthylamino)pyrrolidin-3-ylméthyl]-N-isopropyl-4-méthoxy-3-(3-méthoxypropoxy)-benzamide ; le N-isopropyl-4-méthoxy-3-(3-méthoxypropoxy)-N-((3R*,4R*)-4-phénéthylaminopyrrolidin-3-ylméthyl)benzamide ; et le N-isopropyl-4-méthoxy-3-(3-méthoxypropoxy)-N-{(3R*,4R*)-4-[(naphtalén-2-ylméthyl)-amino]pyrrolidin-3-ylméthyl}benzamide ;
ou à partir des composés de formules 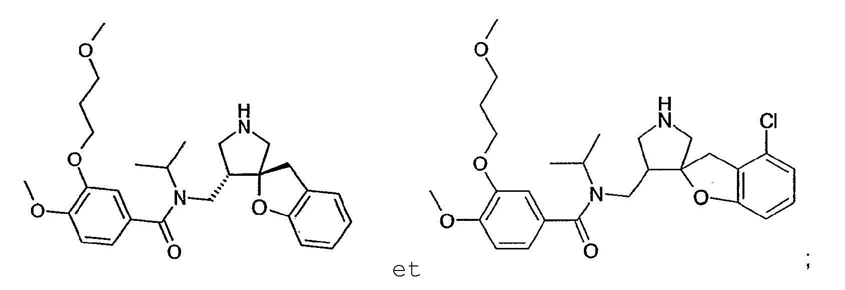 ou un sel pharmaceutiquement acceptable de celui-ci.

15. Composé de formule I selon la revendication 1, choisi parmi le groupe des composés constitué par
le (3S*,4S*)-4-({isopropyl-[4-méthoxy-3-(3-méthoxypropoxy)benzoyl]amino}méthyl)pyrrolidin-3-ylester de l'acide benzylcarbamique ; et
le N-isopropyl-4-méthoxy-3-(3-méthoxypropoxy)-N-[(3R*,4R*)-4-(naphtalène-2-sulfonylamino)pyrrolidin-3-ylméthyl]-benzamide ;
ou un sel pharmaceutiquement acceptable de celui-ci.

16. Composé de formule I, ou sel pharmaceutiquement acceptable de celui-ci, selon l'une quelconque des revendications 1 à 15, pour une utilisation dans le diagnostic ou le traitement thérapeutique d'un animal à sang chaud.

17. Composé de formule I, ou sel pharmaceutiquement acceptable de celui-ci, selon l'une quelconque des revendications 1 à 15, pour une utilisation selon la revendication 14 dans le traitement d'une maladie (= trouble) qui dépend de l'activité de la rénine.

18. Utilisation d'un composé de formule I, ou d'un sel pharmaceutiquement acceptable de celui-ci, selon l'une quelconque des revendications 1 à 15, pour la fabrication d'une composition pharmaceutique destinée au traitement d'une maladie qui dépend de l'activité de la rénine.

19. Formulation pharmaceutique, comprenant un composé de formule I, ou un sel pharmaceutiquement acceptable de celui-ci, selon l'une quelconque des revendications 1 à 15 et au moins un matériau véhicule pharmaceutiquement acceptable.

20. Procédé de fabrication d'un composé de formule I, ou d'un sel pharmaceutiquement acceptable de celui-ci, selon l'une quelconque des revendications 1 à 15, comprenant
A) pour la synthèse d'un composé de formule I dans laquelle T est méthylène, carbonyle ou thiocarbonyle et R¹, R², R³, R⁴ et T revêtent les significations données ci-dessus ou ci-après pour un composé de formule I, la réaction d'un acide de formule II, 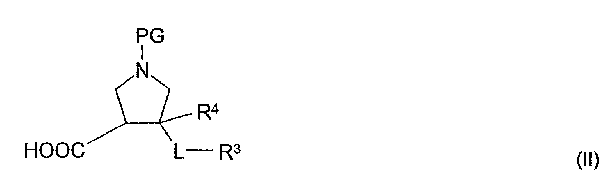 ou d'un dérivé réactif de celui-ci, où R³, R⁴ et L sont tels que définis pour un composé de formule I et PG est un groupement protecteur, avec
(i) un composé amino de formule III,
R¹R²NH (III)
dans laquelle R¹ et R² sont tels que définis pour un composé de formule I, dans des conditions de condensation et
(a) afin d'obtenir un composé de formule I dans laquelle T est carbonyle et dans laquelle R¹, R², R³, R⁴, L et PG sont tels que définis pour des composés de formule I, l'élimination des groupements protecteurs ou (b), si on le souhaite, la réduction du groupement carbonyle dans le composé obtenable de formule IV (un composé particulier de formule I), 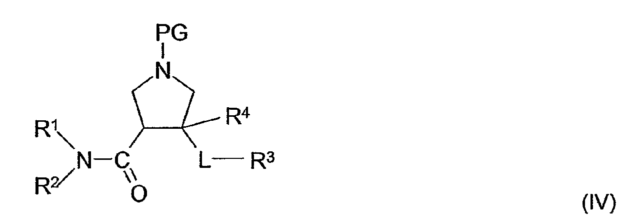 dans laquelle R¹, R², R³, R⁴, L et PG sont tels que définis pour des composés de formule II et III, en un groupement méthylène, et, afin d'obtenir un composé de formule I dans laquelle R¹, R², R³, R⁴, L et PG sont tels que définis pour des composés de formule I et T est méthylène, l'élimination des groupements protecteurs ; ou
(ii) avec un composé amino de formule V,
R²-NH₂ (V)
dans laquelle R¹ est tel que défini pour un composé de formule I, afin de donner un composé de formule VI, 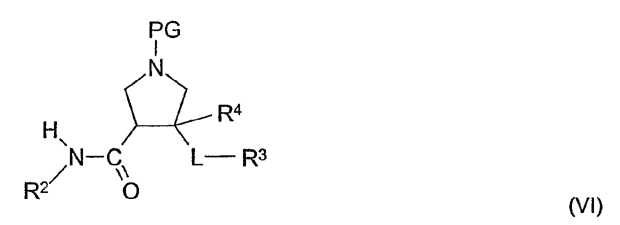 dans laquelle R², R³, R⁴ et L sont tels que définis pour un composé de formule I et PG est un groupement protecteur, et soit
(a) la réduction du groupement carbonyle, ce par quoi on obtient un composé de formule VII 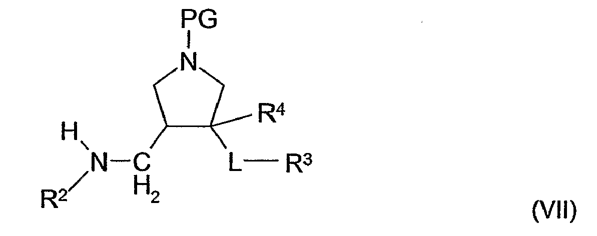 dans laquelle R², R³, R⁴, L et PG sont tels que définis pour un composé de formule VI, et la réaction du composé de formule VII avec un composé de formule VIII,
R¹-Z (VIII)
dans laquelle R¹ est tel que défini pour un composé de formule I et Z est un groupement partant, et,
afin d'obtenir un composé de formule I dans laquelle T est méthylène et R¹, R², R³, R⁴ et L sont tels que définis pour un composé correspondant de formule I, l'élimination des groupements protecteurs ;
soit (b) la réaction du composé de formule VI avec un composé de formule VIII tel que défini ci-dessus et, afin d'obtenir un composé de formule I dans laquelle T est carbonyle et R¹, R², R³, R⁴ et L sont tels que définis pour un composé de formule I, l'élimination des groupements protecteurs ;
B) pour la synthèse d'un composé de formule I dans laquelle T est méthylène et R¹, R², R³, R⁴ et T revêtent les significations données ci-dessus ou ci-après pour un composé de formule I, la réaction d'un aldéhyde de formule IX, 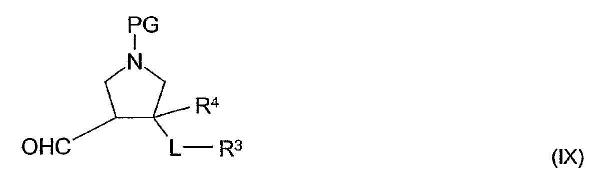 dans laquelle R³, R⁴ et L sont tels que définis pour un composé de formule I et PG est un groupement protecteur, soit
(i) avec un composé amino de formule III tel que défini ci-dessus dans des conditions d'amination réductrice et, afin d'obtenir un composé de formule I dans laquelle R¹, R², R³, R⁴ et L sont tels que définis pour un composé de formule I et T est méthylène, l'élimination des groupements protecteurs ;
soit (ii) avec un composé amino de formule V tel que défini ci-dessus, ce par quoi on obtient un composé de formule X 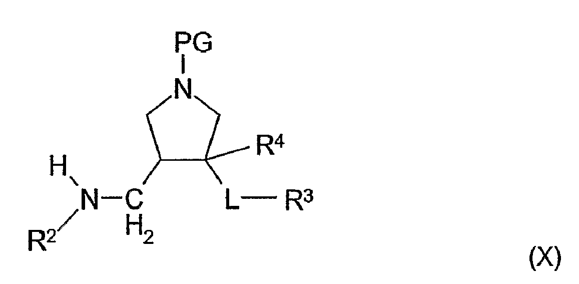 dans laquelle R², R³, R⁴ et L sont tels que définis pour un composé de formule I et PG est un groupement protecteur, dans des conditions d'amination réductrice puis la réaction du composé de formule X
(I) avec un composé de formule VIII tel que défini ci-dessus ou
(II) pour l'introduction d'un motif R¹ lié via un groupement méthylène faisant partie dudit R¹, avec un aldéhyde de formule VIII*
R¹*-CHO (VIII*)
dans laquelle R¹* est un motif complémentant le motif R¹-CH₂- ainsi obtenable en un motif correspondant R¹ dans le composé résultant, dans des conditions d'amination réductrice, et, afin d'obtenir un composé de formule I dans laquelle T est méthylène et R¹, R², R³, R⁴ et L sont tels que définis pour un composé de formule I, l'élimination des groupements protecteurs ;
C) pour la synthèse d'un composé de formule I dans laquelle R¹, R² et T sont tels que définis pour un composé de formule I, R³ est alkyle non substitué ou substitué, aryle non substitué ou substitué, hétérocyclyle non substitué ou substitué, cycloalkyle non substitué ou substitué, aryl-alkyle non substitué ou substitué, hétérocyclyl-alkyle non substitué ou substitué, cycloalkyl-alkyle non substitué ou substitué, alkylsulfonyle non substitué ou substitué, arylsulfonyle non substitué ou substitué, hétérocyclylsulfonyle non substitué ou substitué, cycloalkylsulfonyle non substitué ou substitué, alkylcarbonyle non substitué ou substitué, arylcarbonyle non substitué ou substitué, hétérocyclylcarbonyle non substitué ou substitué, carboxy éthérifié ou aminosulfonyle N-mono- ou N,N-disubstitué, R⁴ est hydrogène et L est oxy, thio ou imino non substitué ou substitué, un composé de formule XI, 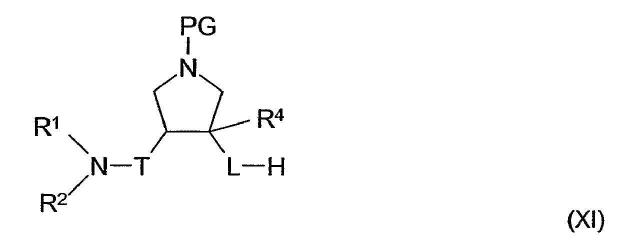 dans laquelle R¹, R², R⁴ et T sont tels que venant d'être définis, PG est un groupement protecteur et L est oxy, thio ou imino non substitué ou substitué, est réagi
(i) avec un composé de formule XII,
R³-Z (XII)
dans laquelle Z est un groupement partant et R³ est tel que venant d'être défini, ou
(ii) dans le cas où L est imino ou imino monosubstitué, dans des conditions d'amination réductrice avec un aldéhyde de formule XIIA
R³*-CHO (XIIA)
dans laquelle R³* est un motif complétant un motif R³*-CH₂ ainsi obtenable en un motif correspondant R³ dans le composé résultant,
et, afin d'obtenir un composé correspondant de formule I, l'élimination des groupements protecteurs ;
D) pour la préparation d'un composé de formule I dans laquelle R¹, R² et T sont tels que définis dans la formule I et R³ et R⁴ conjointement avec L forment oxo, thioxo ou imino non substitué ou substitué, l'oxydation d'un composé de formule XI tel que défini ci-dessus mais où L est oxy en un composé oxo correspondant de formule XIII, 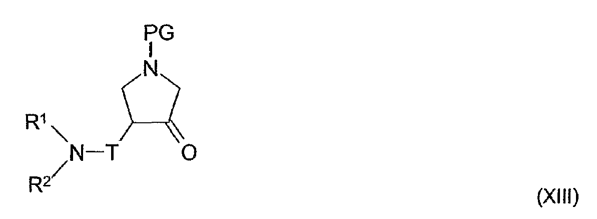 dans laquelle R¹, R² et T sont tels que définis selon la formule I et, si on le souhaite, la conversion du groupement oxo en un groupement thioxo ou imino non substitué ou substitué, et, afin d'obtenir un composé correspondant de formule I, l'élimination du ou des groupements protecteurs ;
E) pour la synthèse d'un composé de formule I, où R¹, R², L et T sont tels que définis pour un composé de formule I, R³ est alkyle non substitué ou substitué, aryle non substitué ou substitué, hétérocyclyle non substitué ou substitué, cycloalkyle non substitué ou substitué, aryl-alkyle non substitué ou substitué, hétérocyclyl-alkyle non substitué ou substitué ou cycloalkyl-alkyle non substitué ou substitué, et R⁴ est hydroxy, la réaction d'un composé de formule XIII tel que défini ci-dessus avec un réactif métallique de formule XIV,
R³-L-Mg-Hal (XIV)
dans laquelle R³ est tel que venant d'être défini et Hal est halogéno, et, afin d'obtenir un composé correspondant de formule I, l'élimination des groupements protecteurs ;
F) pour la synthèse d'un composé spiro de formule I dans laquelle R¹, R² et T sont tels que définis pour un composé de formule I et R³ et R⁴ qui est alors -O-conjointement avec L qui est alors méthylène et le carbone auquel R³-L- et R⁴ sont liés forment un cycle non substitué ou substitué annelé à un aryle non substitué ou substitué, hétérocyclyle non substitué ou substitué ou cycloalkyle non substitué ou substitué, la réaction d'un composé de formule XV, 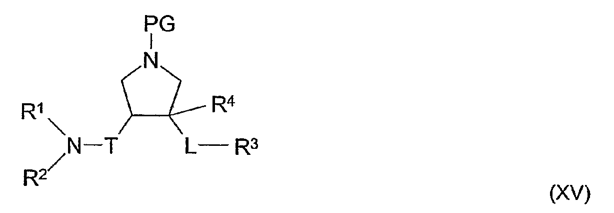 dans laquelle R¹, R² et T sont tels que définis pour un composé de formule I, R³ est aryle non substitué ou substitué, hétérocyclyle non substitué ou substitué, ou cycloalkyle non substitué ou substitué, chacun d'entre eux portant un groupement partant, L est méthylène et R⁴ est hydroxy, en présence d'une base forte afin d'obtenir un composé spiro correspondant de formule I, l'élimination des groupements protecteurs ;
G) pour la synthèse d'un composé de formule I dans laquelle R¹, R² et L sont tels que définis pour un composé de formule I, R⁴ est hydrogène, L est oxy, thio ou imino et R³ est amino-carbonyle N-mono-substitué, la réaction d'un composé de formule XI tel qu'illustré ci-dessus dans C) dans laquelle L est oxy, thio ou imino et les autres motifs sont tels que décrits ci-dessus, avec un composé d'isocyanate de formule XIB,
R³**-NCO (XIIB)
dans laquelle R³** est un substituant complétant l'amino-carbonyle N-mono-substitué correspondant, et l'élimination des groupements protecteurs afin d'obtenir le composé correspondant de formule I ; ou
H) pour la synthèse d'un composé de formule I dans laquelle R¹, R² et T sont tels que définis pour un composé de formule I, L est oxy, thio ou imino non substitué ou substitué et R³ tel que défini ci-dessus, la réaction d'un dérivé réactif d'un composé de formule XI tel que défini ci-dessus dans C), où au lieu de -L-H, un groupement partant est présent, R⁴ est hydrogène et les autres motifs sont tels que définis dans C), avec un composé de formule XIIC,
R³-L-H (XIIC)
dans laquelle R³ est tel que défini pour un composé de formule I et L est oxy, thio ou imino non substitué ou substitué, et l'élimination des groupements protecteurs afin d'obtenir le composé correspondant de formule I ;
et, si on le souhaite, après un ou plusieurs des processus mentionnés dans (A) à (H) la conversion d'un composé obtenable de formule I ou d'une forme protégée de celui-ci en un composé différent de formule I, la conversion d'un sel d'un composé obtenable de formule I en composé libre ou un sel différent, la conversion d'un composé libre obtenable de formule I en un sel de celui-ci, et/ou la séparation d'un mélange obtenable d'isomères d'un composé de formule I en isomères individuels ;
où dans l'un quelconque des produits de départ, outre les groupements protecteurs spécifiques mentionnés, d'autres groupements protecteurs peuvent être présents, et les groupements protecteurs quelconques sont éliminés au niveau d'un stade approprié afin d'obtenir le composé correspondant de formule I, ou un sel de celui-ci.
